(19) Europäisches Patentamt
European Patent Office
Office européen des brevets

(11) EP 1 888 785 B1

(12) **EUROPEAN PATENT SPECIFICATION**

(45) Date of publication and mention of the grant of the patent:
**04.03.2015 Bulletin 2015/10**

(21) Application number: **06770766.1**

(22) Date of filing: **18.05.2006**

(51) Int Cl.:
***C12Q 1/68*** *(2006.01)* ***G01N 33/53*** *(2006.01)*

(86) International application number:
**PCT/US2006/019615**

(87) International publication number:
**WO 2006/127537 (30.11.2006 Gazette 2006/48)**

# (54) THYROID FINE NEEDLE ASPIRATION MOLECULAR ASSAY

MOLEKULARER THYROID-ASSAY MIT ABSAUGEN ÜBER FEINE NADEL

ANALYSE MOLÉCULAIRE DE LA THYROIDE PAR ASPIRATION À L'AIGUILLE

(84) Designated Contracting States:
**AT BE BG CH CY CZ DE DK EE ES FI FR GB GR HU IE IS IT LI LT LU LV MC NL PL PT RO SE SI SK TR**

(30) Priority: **20.05.2005 US 683173 P**

(43) Date of publication of application:
**20.02.2008 Bulletin 2008/08**

(60) Divisional application:
**12166440.3 / 2 518 166**

(73) Proprietor: **Janssen Diagnostics, LLC**
**Raritan, NJ 08869 (US)**

(72) Inventors:
- **JIANG, Yuqiu**
  **San Diego, CA 92130 (US)**
- **BACKUS, John W.**
  **Ontario, NY 14519 (US)**
- **MAZUMDER, Abhijit**
  **Basking Ridge, NJ 07970 (US)**
- **CHOWDARY, Dondapati**
  **Princeton Junction, NJ 08550 (US)**
- **YANG, Fei**
  **San Diego, CA 92130 (US)**
- **WANG, Yixin**
  **San Diego, CA 92130 (US)**
- **JATKOE, Timothy**
  **San Diego, CA 92122 (US)**

(74) Representative: **Goodfellow, Hugh Robin**
**Carpmaels & Ransford LLP**
**One Southampton Row**
**London WC1B 5HA (GB)**

(56) References cited:
**US-A1- 2002 102 531** **US-A1- 2003 219 760**
**US-A1- 2003 225 526** **US-B1- 6 436 642**

- **"[HG-U133A] Affymetrix Human Genome U133A Array" GEO,, 11 March 2002 (2002-03-11), XP002527544**
- **KHOLOVÁ I ET AL: "Diagnostic role of markers dipeptidyl peptidase IV and thyroid peroxidase in thyroid tumors" ANTICANCER RESEARCH, INTERNATIONAL INSTITUTE OF ANTICANCER RESEARCH, GR, vol. 23, no. 2A, 1 March 2003 (2003-03-01) , pages 871-875, XP009125721 ISSN: 0250-7005**
- **MAZZANTI C ET AL: "Using gene expression profiling to differentiate benign versus malignant thyroid tumors" CANCER RESEARCH, AMERICAN ASSOCIATION FOR CANCER RESEARCH, BALTIMORE, MD., US, vol. 64, 15 April 2004 (2004-04-15), pages 2898-2903, XP002343973 ISSN: 0008-5472**
- **JARZAB B ET AL: "Gene expression profile of papillary thyroid cancer: sources of variability and diagnostic implications" CANCER RESEARCH, AMERICAN ASSOCIATION FOR CANCER RESEARCH, BALTIMORE, MD., US, vol. 65, no. 4, 15 February 2005 (2005-02-15), pages 1587-1597, XP002465072 ISSN: 0008-5472**
- **FINLEY DAVID J ET AL: "Discrimination of benign and malignant thyroid nodules by molecular profiling" ANNALS OF SURGERY, J.B. LIPPINCOTT COMPANY, PHILADELPHIA, US, vol. 240, no. 3, 1 September 2004 (2004-09-01), pages 425-436, XP002444871 ISSN: 0003-4932**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



**(Cont. next page)**

- TAKANO T ET AL: "Gene expression profiles in thyroid carcinomas" BRITISH JOURNAL OF CANCER, vol. 83, no. 11, December 2000 (2000-12), pages 1495-1502, XP002555968 ISSN: 0007-0920
- POLEEV ANDREJ ET AL: "PAX8, a human paired box gene: Isolation and expression in developing thyroid, kidney and Wilms' tumors" DEVELOPMENT (CAMBRIDGE), vol. 116, no. 3, 1992, pages 611-623, XP002555969 ISSN: 0950-1991
- PAWITAN Y ET AL: "Gene expression profiling for prognosis using Cox regression" STATISTICS IN MEDICINE, vol. 23, no. 11, 15 June 2004 (2004-06-15) , pages 1767-1780, XP002555970 ISSN: 0277-6715
- CHEN G ET AL: "Discordant protein and mRNA expression in lung adenocarcinomas", MOLECULAR & CELLULAR PROTEOMICS, AMERICAN SOCIETY FOR BIOCHEMISTRY AND MOLECULAR BIOLOGY, INC, US, vol. 1, no. 4, 1 April 2002 (2002-04-01), pages 304-313, XP008123587, ISSN: 1535-9476, DOI: 10.1074/MCP.M200008-MCP200 [retrieved on 2002-03-12]

## Description

There are approximately 25,600 new cases of thyroid carcinoma diagnosed in the United States each year, and 1,400 patients will die of the disease. About 75% of all thyroid cancers belong to the papillary thyroid carcinoma type. The rest consist of 10% follicular carcinoma, 5% to 9% medullary thyroid cancer, 1% to 2% anaplastic cancer, 1% to 3% lymphoma, and less than 1% sarcoma and other rare tumors. Usually a lump (nodule) in the thyroid is the first sign of thyroid cancer. There are 10 to 18 million people in US with a single thyroid nodule, and approximately 490,000 become clinically apparent each year. Fortunately only about 5% of these nodules are cancerous.

The commonly used method for thyroid cancer diagnosis is fine needle aspiration (FNA) biopsy. FNA samples are examined cytologically to determine whether the nodules are benign or cancerous. The sensitivity and specificity of FNA range from 68% to 98%, and 72% to 100% respectively, depending on institutions and doctors. Unfortunately, in 25% of the cases the specimens are either inadequate for diagnosis or indeterminable by cytology. In current medical practice, patients with indeterminate results are sent to surgery, with consequence that only 25% have cancer and 75% end up with unnecessary surgery. A molecular assay with high sensitivity and a better specificity (higher than 25%) would greatly improve current diagnostic accuracy of thyroid cancer, and omit unnecessary surgery for non-cancerous patients.

Comparative genomic hybridization (CGH), serial analysis of gene expression (SAGE), and DNA microarray have been used to identify genetic events occurring in thyroid cancers such as loss of heterozygosity, up and down gene regulation, and genetic rearrangements. PAX8 and PPAR$\gamma$ genetic rearrangement event has been demonstrated to be associated with follicular thyroid cancer (FTC). Rearrangement of the ret proto-oncogene is related to papillary thyroid cancer (PTC). Down-regulation of thyroid peroxidase (TPO) gene is observed in both FTC and PTC. Galectin-3 was reported to be a candidate marker to differentiate malignant thyroid neoplasms from benign lesions. However, there are other studies demonstrating that Galectin-3 is not a cancer-specific marker. Many genes purported to be useful in thyroid cancer diagnosis lack the sensitivity and specificity required for an accurate molecular assay.

> Kholova et al. (Anticancer Res. 2003 Mar-Apr;23(2A):871-5.) describes a study to evaluate dipeptidyl peptidase IV (DPP IV) as a marker of malignancy in FNAC. We tested 254 thyroid specimens (intraoperative imprint smears) for DPP IV.
>
> Mazzanti et al. (Cancer Res. 2004 Apr 15;64(8):2898-903) discuss a 10 and 6 gene model which is used to differentiate benign versus malignant thyroid tumors.
>
> Jarzab et al. (Cancer Res. 2005 Feb 15;65(4): 1587-97) teaches a study which looked for an optimal set of genes differentiating between papillary thyroid cancer (PTC) and normal thyroid tissue and assessed the sources of variability in gene expression profiles.
>
> Finley et al. (Ann Surg. 2004 Sep;240(3):425-36; discussion 436-7) assessed whether molecular profiling can discriminate between benign and malignant thyroid nodules with the necessary sensitivity and specificity required of a screening test.
>
> Takano et al. (Br J Cancer. 2000 December; 83(11): 1495-1502.) teaches gene expression profiles of human thyroid carcinomas which were analysed by serial analysis of gene expression (SAGE).

## SUMMARY OF THE INVENTION

The present invention encompasses methods of diagnosing thyroid cancer in a biological sample obtained from a patient comprising measuring the expression levels in the sample of genes corresponding to SEQ ID NOs: 36, 53, 76, 211 and 242; where the gene expression levels above or below pre-determined cut-off levels are indicative of thyroid cancer; wherein gene expression is (a) measured on a microarray or gene chip; or (b) determined by nucleic acid amplification conducted by polymerase chain reaction (PCR) of RNA extracted from the sample.

The present disclosure encompasses methods of differentiating between thyroid carcinoma and benign thyroid diseases by obtaining a sample from a patient; and measuring the expression levels in the sample of genes selected from the group consisting of those encoding mRNA: corresponding to SEQ ID NOs: 36, 53, 76, 211 and 242; and/or corresponding to SEQ ID NOs: 199, 207, 255 and 354; or recognized specifically by the probe sets selected from the group consisting of psids corresponding to SEQ ID NOs: 36, 53, 76, 211 and 242 as depicted in Table 25; and/or recognized specifically by the probe sets selected from the group consisting of psids corresponding to SEQ ID NOs: 199, 207, 255 and 354 as depicted in Table 25; where the gene expression levels above or below pre-determined cut-off levels are indicative of thyroid carcinoma.

The present disclosure encompasses methods of testing indeterminate thyroid fine needle aspirate (FNA) thyroid nodule samples by: obtaining a sample from a patient; and measuring the expression levels in the sample of genes selected from the group consisting of those encoding mRNA: corresponding to SEQ ID NOs: 36, 53, 76, 211 and 242; and/or corresponding to SEQ ID NOs: 199, 207, 255 and 354; or recognized specifically by the probe sets selected from the group consisting of psids corresponding to SEQ ID NOs: 36, 53, 76, 211 and 242 as depicted in Table 25; and/or recognized specifically by the probe sets selected from the group consisting of psids corresponding to SEQ ID NOs: 199, 207, 255 and 354 as depicted in Table 25; where the gene expression levels above or below pre-determined cut-off levels are indicative of thyroid cancer.

The present disclosure encompasses methods of determining thyroid cancer patient treatment protocol by: obtaining a biological sample from a thyroid cancer patient; and measuring the expression levels in the sample of genes selected from the group consisting of those encoding mRNA: corresponding to SEQ ID NOs: 36, 53, 76, 211 and 242; and/or corresponding to SEQ ID NOs: 199, 207, 255 and 354; or recognized specifically by the probe sets selected from the group consisting of psids corresponding to SEQ ID NOs: 36, 53, 76, 211 and 242 as depicted in Table 25; and/or recognized specifically by the probe sets selected from the group consisting of psids corresponding to SEQ ID NOs: 199, 207, 255 and 354 as depicted in Table 25; where the gene expression levels above or below pre-determined cut-off levels are sufficiently indicative of cancer to enable a physician to determine the type of surgery and/or therapy recommend to treat the disease.

The present disclosure encompasses methods of treating a thyroid cancer patient by obtaining a biological sample from a thyroid cancer patient; and measuring the expression levels in the sample of genes selected from the group consisting of those encoding mRNA: corresponding to SEQ ID NOs: 36, 53, 73, 211 and 242; and/or corresponding to SEQ ID NOs: 199, 207, 255 and 354; or recognized specifically by the probe sets selected from the group consisting of psids corresponding to SEQ ID NOs: 36, 53, 73, 211 and 242 as depicted in Table 25; and/or recognized specifically by the probe sets selected from the group consisting of psids corresponding to SEQ ID NOs: 199, 207, 255 and 354 as depicted in Table 25; where the gene expression levels above or below pre-determined cut-off levels are indicative of cancer; and treating the patient with a thyroidectomy if they are cancer positive.

The present disclosure encompasses methods of cross validating a gene expression profile for thyroid carcinoma patients by: a. obtaining gene expression data from a statistically significant number of patient biological samples; b. randomizing sample order; c. setting aside data from about 10% - 50% of samples; d. computing, for the remaining samples, for factor of interest on all variables and selecting variables that meet a p-value cutoff (p); e. selecting variables that fit a prediction model using a forward search and evaluating the training error until it hits a predetermined error rate; f. testing the prediction model on the left-out 10-50% of samples; g. repeating steps c., -g. with a new set of samples removed; and h. continuing steps c) -g) until 100% of samples have been tested and record classification performance.

The present disclosure encompasses methods of independently validating a gene expression profile and gene profiles obtained thereby for thyroid carcinoma patients by obtaining gene expression data from a statistically significant number of patient biological samples; normalizing the source variabilities in the gene expression data; computing for factor of interest on all variables that were selected previously; and testing the prediction model on the sample and record classification performance.

The present disclosure encompasses a method of generating a posterior probability score to enable diagnosis of thyroid carcinoma patients by: obtaining gene expression data from a statistically significant number of patient biological samples; applying linear discrimination analysis to the data to obtain selected genes; and applying weighted expression levels to the selected genes with discriminate function factor to obtain a prediction model that can be applied as a posterior probability score.

The present disclosure encompasses methods of generating a thyroid carcinoma prognostic patient report and reports obtained thereby, by obtaining a biological sample from the patient; measuring gene expression of the sample; applying a posterior probability thereto; and using the results obtained thereby to generate the report.

The present invention encompasses compositions consisting of one probe set consisting of: SEQ ID NOs: 36, 53, 76, 211 and 242.

The present invention encompasses kits for conducting an assay to determine thyroid carcinoma diagnosis in a biological sample as defined in the claims consisting of materials for detecting isolated nucleic acid sequences, their complements, or portions thereof of a combination of genes encoding mRNA: corresponding to SEQ ID NOs: 36, 53, 76, 211 and 242.

The present disclosure encompasses articles for assessing thyroid carcinoma status containing: materials for detecting isolated nucleic acid sequences, their complements, or portions thereof of a combination of genes selected from the group consisting of those encoding mRNA: corresponding to SEQ ID NOs: 36, 53, 76, 211 and 242; and/or corresponding to SEQ ID NOs: 199, 207, 255 and 354; or recognized specifically by the probe sets selected from the group consisting of psids corresponding to SEQ ID NOs: 36, 53, 76, 211 and 242 as depicted in Table 25; and/or recognized specifically by the probe sets selected from the group consisting of psids corresponding to SEQ ID NOs: 199, 207, 255 and 354 as depicted in Table 25.

The present invention encompasses the use of microarrays or gene chips for performing the methods provided herein, as defined in the claims.

The present disclosure encompasses diagnostic/prognostic portfolios containing isolated nucleic acid sequences, their complements, or portions thereof of a combination of genes selected from the group consisting of those encoding mRNA: corresponding to SEQ ID NOs: 36, 53, 76, 211 and 242; and/or corresponding to SEQ ID NOs: 199, 207, 255 and 354; or recognized specifically by the probe sets selected from the group consisting of psids corresponding to SEQ ID NOs: 36, 53, 76, 211 and 242 as depicted in Table 25; and/or recognized specifically by the probe sets selected from the group consisting of psids corresponding to SEQ ID NOs: 199, 207, 255 and 354 as depicted in Table 25 where the combination is sufficient to characterize thyroid carcinoma status or risk of relapse in a biological sample.

## BRIEF DESCRIPTION OF THE DRAWINGS

Figure 1 is an ROC curve of the LOOCV of the 4-gene signature in 98 training samples.

Figure 2 is an ROC curve of the 5-gene signature in 98 training samples.

Figure 3a is an ROC curve of the 4-gene signature in 74 independent validation samples; 3b is an ROC curve of the 5-gene signature in 74 independent validation samples.

Figure 4a is an ROC curve of the 4-gene signature that is normalized to the three-thyroid control genes; 4b is an ROC curve of the 5-gene signature that is normalized to the three-thyroid control genes.

Figure 5a is an ROC curve of the 4-gene signature with one-round amplification in 47 thyroid samples; 5b is an ROC curve of the 4-gene signature with two-round amplification in 47 thyroid samples; 5c is an ROC curve of the 5-gene signature with one-round amplification in 47 thyroid samples; 5d is an ROC curve of the 5-gene signature with two-round amplification in 47 thyroid samples.

Figures 6a and 6b depict the ROC curves for cross validation with the 83 independent fresh frozen thyroid samples.

Figures 7a and 7b depict the ROC curves for signature validation with the 47 fine needle aspirate (FNA) thyroid samples.

Figures 8a and 8b depict the ROC curves for signature performance in 28 paired fresh frozen and FNA thyroid samples.

## DETAILED DESCRIPTION

In this study the goal was to identify signatures that can be used in assays such as DNA chip-based assay to differentiate thyroid carcinomas from benign thyroid diseases. 31 primary papillary thyroid tumors, 21 follicular thyroid cancers, 33 follicular adenoma samples, and 13 benign thyroid diseases were analyzed by using the Affymetrix human U133A Gene Chip. Comparison of gene expression profiles between thyroid cancers and benign tissues has enabled us to identify two signatures: a 5-gene signature identified by percentile analysis and manual selection, and a 4-gene signature selected by Linear Discrimination Analysis (LDA) approach. These two signatures have the performance of sensitivity/specificity 92%/70% and 92%/61%, respectively, and have been validated in 74 independent thyroid samples. The results presented herein demonstrate that these candidate signatures facilitate the diagnosis of thyroid cancers with better sensitivity and specificity than currently available diagnostic procedures. These two signatures are suitable for use in testing indeterminate FNA samples.

By performing gene profiling on 98 representative thyroid benign and tumor samples on Affymetrix U133a chips, we have selected two gene signatures, a 5-gene signature and a 4-gene signature, for thyroid FNA molecular assay. Signatures were selected to achieve the best sensitivity of the assay at a close to 95%. Except for fibronectin and thyroid peroxidase, the other seven genes from the two signatures have not been implicated previously in thyroid tumorogenesis. Both signatures have been validated with an independent 74 thyroid samples, and achieved performance that is equivalent to the one in the 98 training samples. The performances of the two gene signatures are 92% sensitivity and 70%/61% specificity, respectively. When these two signatures are normalized to the specific thyroid control genes the performances are improved relative to the ones of the non-normalized signatures. Furthermore, the signatures performed equivalently with two different target preparations, namely one-round amplification and two-round amplifications. This validation is extremely important for thyroid assays that are FNA samples, which usually contain limited numbers of thyroid cells.

The mere presence or absence of particular nucleic acid sequences in a tissue sample has only rarely been found to have diagnostic or prognostic value. Information about the expression of various proteins, peptides or mRNA, on the other hand, is increasingly viewed as important. The mere presence of nucleic acid sequences having the potential to express proteins, peptides, or mRNA (such sequences referred to as "genes") within the genome by itself is not determinative of whether a protein, peptide, or mRNA is expressed in a given cell. Whether or not a given gene capable of expressing proteins, peptides, or mRNA does so and to what extent such expression occurs, if at all, is determined by a variety of complex factors. Irrespective of difficulties in understanding and assessing these factors, assaying gene expression can provide useful information about the occurrence of important events such as tumorogenesis, metastasis, apoptosis, and other clinically relevant phenomena. Relative indications of the degree to which genes are active or inactive can be found in gene expression profiles. The gene expression profiles of this invention are used to provide a diagnosis and treat patients for thyroid cancer.

Sample preparation requires the collection of patient samples. Patient samples used in the inventive method are those that are suspected of containing diseased cells such as cells taken from a nodule in a fine needle aspirate (FNA) of thyroid tissue. Bulk tissue preparation obtained from a biopsy or a surgical specimen and laser capture microdissection are also suitable for use. Laser Capture Microdissection (LCM) technology is one way to select the cells to be studied, minimizing variability caused by cell type heterogeneity. Consequently, moderate or small changes in gene expression between normal or benign and cancerous cells can be readily detected. Samples can also comprise circulating epithelial cells extracted from peripheral blood. These can be obtained according to a number of methods but the most preferred method is the magnetic separation technique described in U.S. Patent 6,136,182. Once the sample containing the cells of interest has been obtained, RNA is extracted and amplified and a gene expression profile is obtained, preferably via microarray, for genes in the appropriate portfolios.

The present invention encompasses methods of diagnosing thyroid cancer in a biological sample obtained from a patient comprising measuring the expression levels in the sample of genes from those encoding mRNA corresponding to SEQ ID NOs: 36, 53, 76, 211 and 242; where the gene expression levels above or below pre-determined cut-off levels are indicative of thyroid cancer; wherein gene expression is (a) measured on a microarray or gene chip; or (b) determined by nucleic acid amplification conducted by polymerase chain reaction (PCR) of RNA extracted from the sample.

The present disclosure encompasses methods of differentiating between thyroid carcinoma and benign thyroid diseases by obtaining a sample from a patient; and measuring the expression levels in the sample of genes from those encoding mRNA: corresponding to SEQ ID NOs: 36, 53, 76, 211 and 242; and/or corresponding to SEQ ID NOs: 199, 207, 255 and 354; or recognized specifically by the probe sets from psids corresponding to SEQ ID NOs: 36, 53, 76, 211 and 242 as depicted in Table 25; and/or recognized specifically by the probe sets from psids corresponding to SEQ ID NOs: 199, 207, 255 and 354 as depicted in Table 25; where the gene expression levels above or below pre-determined cut-off levels are indicative of thyroid carcinoma.

The present disclosure encompasses methods of testing indeterminate thyroid fine needle aspirate (FNA) thyroid nodule samples by: obtaining a sample from a patient; and measuring the expression levels in the sample of genes from those encoding mRNA: corresponding to SEQ ID NOs: 36, 53, 76, 211 and 242; and/or corresponding to SEQ ID NOs: 199, 207, 255 and 354; or recognized specifically by the probe sets from psids corresponding to SEQ ID NOs: 36, 53, 76, 211 and 242 as depicted in Table 25; and/or recognized specifically by the probe sets from psids corresponding to SEQ ID NOs: 199, 207, 255 and 354 as depicted in Table 25; where the gene expression levels above or below pre-determined cut-off levels are indicative of thyroid cancer.

The present disclosure encompasses methods of determining thyroid cancer patient treatment protocol by: obtaining a biological sample from a thyroid cancer patient; and measuring the expression levels in the sample of genes from those encoding mRNA: corresponding to SEQ ID NOs: 36, 53, 73, 211 and 242; and/or corresponding to SEQ ID NOs: 199, 207, 255 and 354; or recognized specifically by the probe sets from psids corresponding to SEQ ID NOs: 36, 53, 73, 211 and 242 as depicted in Table 25; and/or recognized specifically by the probe sets from psids corresponding to SEQ ID NOs: 199, 207, 255 and 354 as depicted in Table 25; where the gene expression levels above or below pre-determined cut-off levels are sufficiently indicative of cancer to enable a physician to determine the type of surgery and/or therapy recommend to treat the disease.

The present disclosure encompasses methods of treating a thyroid cancer patient by obtaining a biological sample from a thyroid cancer patient; and measuring the expression levels in the sample of genes from those encoding mRNA: corresponding to SEQ ID NOs: 36, 53, 76, 211 and 242; and/or corresponding to SEQ ID NOs: 199, 207, 255 and 354; or recognized specifically by the probe sets from psids corresponding to SEQ ID NOs: 36, 53, 76, 211 and 242 as depicted in Table 25; and/or recognized specifically by the probe sets from psids corresponding to SEQ ID NOs: 199, 207, 255 and 354 as depicted in Table 25; where the gene expression levels above or below pre-determined cut-off levels are indicative of cancer; and treating the patient with thyroidectomy if they are cancer positive.

The SEQ ID NOs in the above methods can be 36, 53, 73, 211 and 242 or 199, 207, 255 and 354, or 45, 215, 65, 29, 190, 199, 207, 255 and 354.

The invention also encompasses the above methods containing the steps of further measuring the expression level of at least one gene encoding mRNA: corresponding to SEQ ID NOs: 142, 219 and 309; and/or corresponding to SEQ ID NOs: 9, 12 and 18, as defined in the claims. The invention also encompasses the above methods containing the steps of further measuring the expression level of at least one gene constitutively expressed in the sample, as defined in the claims.

Cadherin 3, type 1 (SEQ ID NO: 53) is mentioned in US20030194406; US 20050037439; and US 20040137539.

Fibronectin (SEQ ID NO: 242) is mentioned in US6436642 and US20030104419. Secretory granule, neuroendocrine protein 1 (SEQ ID NO: 76) is mentioned in US20030232350; and US20040002067. Testican-1 (SEQ ID NO: 36) is mentioned in US20030108963; and US20050037463. Thyroid peroxidase (SEQ ID NO: 211) is mentioned in US6066449, US20030118553; US20030054571; WO9102061; and WO9856953. Chemokine C (C-C) motif ligand 18 (SEQ ID NO: 354) is mentioned in WO2005005601 and US20020114806. Pulmonary surfactant-associated protein B (SEQ ID NO: 355) is mentioned in US20030219760; and US20030232350. K+ channel beta subunit (SEQ ID NO: 207) is mentioned in US20030096782; and US 20020168638. Putative prostate cancer suppressor (SEQ ID NO: 178) is mentioned in WO2005020784. Bone marrow stromal cell antigen 1 (SEQ ID NO: 142) is mentioned in WO2004040014; and WO2005020784. Leucocyte immunoglobulin-like receptor-6b (SEQ ID NO: 219) is mentioned in US20030060614. Bridging integrator 2 (SEQ ID NO: 309) is mentioned in EP1393776; WO02057414; WO0116158 and US6831063. Cysteine-rich, angiogenic inducer, 61 (SEQ ID NO: 9) is mentioned in WO2004030615; and WO9733995. Selenoprotein P, Plasma 1 (SEQ ID NO: 12) is mentioned in US20040241653 and WO2005015236. Insulin-like growth factor-binding protein 4 (SEQ ID NO: 18) is mentioned in WO2005015236; WO9203469; WO9203152; and EP0546053.

In this invention, the most preferred method for analyzing the gene expression pattern of a patient in the methods provided herein is through the use of a linear discrimination analysis program. The present disclosure encompasses a method of generating a posterior probability score to enable diagnosis of thyroid carcinoma patients by: obtaining gene expression data from a statistically significant number of patient biological samples; applying linear discrimination analysis to the data to obtain selected genes; and applying weighted expression levels to the selected genes with discriminate function factor to obtain a prediction model that can be applied as a posterior probability score. Other analytical tools can also be used to answer the same question such as, logistic regression and neural network approaches.

For instance, the following can be used for linear discriminant analysis:

$$p_{(CP)} = \frac{e^{d_{(CP)}-d_{(CN)}}}{1+e^{d_{(CP)}-d_{(CN)}}}$$

$$p_{(CN)} = \frac{1}{1+e^{d_{(CP)}-d_{(CN)}}}$$

where,

| | |
|---|---|
| $I_{(psid)}$ | = The log base 2 intensity of the probe set enclosed in parenthesis. |
| $d_{(CP)}$ | = The discriminant function for the cancer positive class |
| $d_{(CN)}$ | = The discriminant function for the cancer negative class |
| $P_{(CP)}$ | = The posterior p-value for the cancer positive class |
| $P_{(CN)}$ | = The posterior p-value for the cancer negative class |

Numerous other well-known methods of pattern recognition are available. The following references provide some examples: Weighted Voting: Golub et al. (1999); Support Vector Machines: Su et al. (2001); and Ramaswamy et al. (2001); K-nearest Neighbors: Ramaswamy (2001); and Correlation Coefficients: van't Veer et al. (2002).

Preferably, portfolios are established such that the combination of genes in the portfolio exhibit improved sensitivity and specificity relative to individual genes or randomly selected combinations of genes. In the context of the instant invention, the sensitivity of the portfolio can be reflected in the fold differences exhibited by a gene's expression in the diseased state relative to the normal state. Specificity can be reflected in statistical measurements of the correlation of the signaling of gene expression with the condition of interest. For example, standard deviation can be a used as such a measurement. In considering a group of genes for inclusion in a portfolio, a small standard deviation in expression measurements correlates with greater specificity. Other measurements of variation such as correlation coefficients can also be used in this capacity. The invention also encompasses the above methods where the specificity is at least about 40%, at least about 50% and at least about 60%. The invention also encompasses the above methods where the sensitivity is at least at least about 90% and at least about 92%.

The invention also encompasses the above methods where the comparison of expression patterns is conducted with pattern recognition methods. One method of the invention involves comparing gene expression profiles for various genes (or portfolios) to ascribe diagnoses. The gene expression profiles of each of the genes comprising the portfolio are fixed in a medium such as a computer readable medium. This can take a number of forms. For example, a table can be established into which the range of signals (e.g., intensity measurements) indicative of disease is input. Actual patient data can then be compared to the values in the table to determine whether the patient samples are normal, benign or diseased. In a more sophisticated embodiment, patterns of the expression signals (e.g., fluorescent intensity) are recorded digitally or graphically. The gene expression patterns from the gene portfolios used in conjunction with patient samples are then compared to the expression patterns.

Pattern comparison software can then be used to determine whether the patient samples have a pattern indicative of the disease. Of course, these comparisons can also be used to determine whether the patient is not likely to experience the disease. The expression profiles of the samples are then compared to the portfolio of a control cell. If the sample expression patterns are consistent with the expression pattern for cancer then (in the absence of countervailing medical considerations) the patient is treated as one would treat a thyroid cancer patient. If the sample expression patterns are consistent with the expression pattern from the normal/control cell then the patient is diagnosed negative for cancer.

Preferably, levels of up and down regulation are distinguished based on fold changes of the intensity measurements of hybridized microarray probes. A 1.5 fold difference is preferred for making such distinctions (or a p-value less than 0.05). That is, before a gene is said to be differentially expressed in diseased versus normal cells, the diseased cell is found to yield at least about 1.5 times more, or 1.5 times less intensity than the normal cells. The greater the fold difference, the more preferred is use of the gene as a diagnostic or prognostic tool. Genes selected for the gene expression profiles of this invention have expression levels that result in the generation of a signal that is distinguishable from those of the normal or non-modulated genes by an amount that exceeds background using clinical laboratory instrumentation.

Statistical values can be used to confidently distinguish modulated from non-modulated genes and noise. Statistical tests find the genes most significantly different between diverse groups of samples. The Student's T-test is an example of a robust statistical test that can be used to find significant differences between two groups. The lower the p-value, the more compelling the evidence that the gene is showing a difference between the different groups. Nevertheless, since microarrays measure more than one gene at a time, tens of thousands of statistical tests may be asked at one time. Because of this, one is unlikely to see small p-values just by chance and adjustments for this using a Sidak correction as well as a randomization/permutation experiment can be made. A p-value less than 0.05 by the T-test is evidence that the gene is significantly different. More compelling evidence is a p-value less then 0.05 after the Sidak correction is factored in. For a large number of samples in each group, a p-value less than 0.05 after the randomization/permutation test is the most compelling evidence of a significant difference.

The present invention encompasses the use of microarrays or gene chips for performing the methods provided herein, as defined in the claims. The microarrays can contain isolated nucleic acid sequences, their complements, or portions thereof of a combination of genes from those encoding mRNA: corresponding to SEQ ID NOs: 36, 53, 76, 211 and 242; or recognized specifically by the probe sets from psids corresponding to SEQ ID NOs: 36, 53, 76, 211 and 242 as depicted in Table 25. The microarray preferably measures or characterizes at least about 1.5-fold over- or under-expression, provides a statistically significant p-value over- or under-expression, or a p-value is less than 0.05. Preferably, the microarray contains a cDNA array or an oligonucleotide array and may contain one or more internal control reagents. One preferred internal control reagent is a method of detecting PAX8 gene expression which can be measured using SEQ ID NOs: 409-411.

Preferably, an oligonucleotide in the array corresponds to the 3' non-coding region of the gene the expression of which is being measured.

Another parameter that can be used to select genes that generate a signal that is greater than that of the non-modulated gene or noise is the use of a measurement of absolute signal difference. Preferably, the signal generated by the modulated gene expression is at least 20% different than those of the normal or non-modulated gene (on an absolute basis). It is even more preferred that such genes produce expression patterns that are at least 30% different than those of normal or non-modulated genes.

Preferred methods for establishing gene expression profiles include determining the amount of RNA that is produced by a gene that can code for a protein or peptide. This is accomplished by reverse transcriptase PCR (RT-PCR), competitive RT-PCR, real time RT-PCR, differential display RT-PCR, Northern Blot analysis and other related tests. While it is possible to conduct these techniques using individual PCR reactions, it is best to amplify complementary DNA (cDNA) or complementary RNA (cRNA) produced from mRNA and analyze it via microarray. A number of different array configurations and methods for their production are known to those of skill in the art and are described in U.S. Patents such as: 5,445,934; 5,532,128; 5,556,752; 5,242,974; 5,384,261; 5,405,783; 5,412,087; 5,424,186; 5,429,807; 5,436,327; 5,472,672; 5,527,681; 5,529,756; 5,545,531; 5,554,501; 5,561,071; 5,571,639; 5,593,839; 5,599,695; 5,624,711; 5,658,734; and 5,700,637.

Microarray technology allows for the measurement of the steady-state mRNA level of thousands of genes simultaneously thereby presenting a powerful tool for identifying effects such as the onset, arrest, or modulation of uncontrolled cell proliferation. Two microarray technologies are currently in wide use. The first are cDNA arrays and the second are oligonucleotide arrays. Although differences exist in the construction of these chips, essentially all downstream data analysis and output are the same. The product of these analyses are typically measurements of the intensity of the signal received from a labeled probe used to detect a cDNA sequence from the sample that hybridizes to a nucleic acid sequence at a known location on the microarray. Typically, the intensity of the signal is proportional to the quantity of cDNA, and thus mRNA, expressed in the sample cells. A large number of such techniques are available and useful. Preferred methods for determining gene expression can be found in US Patents 6,271,002; 6,218,122; 6,218,114; and 6,004,755.

Analysis of the expression levels is conducted by comparing such signal intensities. This is best done by generating a ratio matrix of the expression intensities of genes in a test sample versus those in a control sample. For instance, the gene expression intensities from a diseased tissue can be compared with the expression intensities generated from benign or normal tissue of the same type. A ratio of these expression intensities indicates the fold-change in gene expression between the test and control samples.

Gene expression profiles can also be displayed in a number of ways. The most common method is to arrange raw fluorescence intensities or ratio matrix into a graphical dendogram where columns indicate test samples and rows indicate genes. The data are arranged so genes that have similar expression profiles are proximal to each other. The expression ratio for each gene is visualized as a color. For example, a ratio less than one (indicating down-regulation) may appear in the blue portion of the spectrum while a ratio greater than one (indicating up-regulation) may appear as a color in the red portion of the spectrum. Commercially available computer software programs are available to display such data including "GENESPRING" from Silicon Genetics, Inc. and "DISCOVERY" and "INFER" software from Partek, Inc.

Modulated genes used in the methods of the invention are described in the Examples. The genes that are differentially expressed are either up regulated or down regulated in patients with thyroid cancer relative to those with benign thyroid diseases. Up regulation and down regulation are relative terms meaning that a detectable difference (beyond the contribution of noise in the system used to measure it) is found in the amount of expression of the genes relative to some baseline. In this case, the baseline is the measured gene expression of a benign disease patient. The genes of interest in the diseased cells are then either up regulated or down regulated relative to the baseline level using the same measurement method. Diseased, in this context, refers to an alteration of the state of a body that interrupts or disturbs, or has the potential to disturb, proper performance of bodily functions as occurs with the uncontrolled proliferation of cells. Someone is diagnosed with a disease when some aspect of that person’s genotype or phenotype is consistent with the presence of the disease. However, the act of conducting a diagnosis or prognosis includes the determination of disease/status issues such as determining the likelihood of relapse, type of therapy and therapy monitoring. In therapy monitoring, clinical judgments are made regarding the effect of a given course of therapy by comparing the expression of genes over time to determine whether the gene expression profiles have changed or are changing to patterns more consistent with normal tissue.

Genes can be grouped so that information obtained about the set of genes in the group provides a sound basis for making a clinically relevant judgment such as a diagnosis, prognosis, or treatment choice. As with most diagnostic markers, it is often desirable to use the fewest number of markers sufficient to make a correct medical judgment. This prevents a delay in treatment pending further analysis as well unproductive use of time and resources.

One method of establishing gene expression portfolios is through the use of optimization algorithms such as the mean variance algorithm widely used in establishing stock portfolios. This method is described in detail in US patent publication number 20030194734. Essentially, the method calls for the establishment of a set of inputs (stocks in financial applications, expression as measured by intensity here) that will optimize the return (e.g., signal that is generated) one receives for using it while minimizing the variability of the return. Many commercial software programs are available to conduct such operations. "Wagner Associates Mean-Variance Optimization Application," referred to as "Wagner Software" throughout this specification, is preferred. This software uses functions from the "Wagner Associates Mean-Variance Optimization Library" to determine an efficient frontier and optimal portfolios in the Markowitz sense is preferred. Use of this type of software requires that microarray data be transformed so that it can be treated as an input in the way stock return and risk measurements are used when the software is used for its intended financial analysis purposes.

The process of selecting a portfolio can also include the application of heuristic rules. Preferably, such rules are formulated based on biology and an understanding of the technology used to produce clinical results. More preferably, they are applied to output from the optimization method. For example, the mean variance method of portfolio selection can be applied to microarray data for a number of genes differentially expressed in subjects with cancer. Output from the method would be an optimized set of genes that could include some genes that are expressed in peripheral blood as well as in diseased tissue. If samples used in the testing method are obtained from peripheral blood and certain genes differentially expressed in instances of cancer could also be differentially expressed in peripheral blood, then a heuristic rule can be applied in which a portfolio is selected from the efficient frontier excluding those that are differentially expressed in peripheral blood. Of course, the rule can be applied prior to the formation of the efficient frontier by, for example, applying the rule during data pre-selection.

Other heuristic rules can be applied that are not necessarily related to the biology in question. For example, one can apply a rule that only a prescribed percentage of the portfolio can be represented by a particular gene or group of genes. Commercially available software such as the Wagner Software readily accommodates these types of heuristics. This can be useful, for example, when factors other than accuracy and precision (e.g., anticipated licensing fees) have an impact on the desirability of including one or more genes.

The gene expression profiles of this disclosure can also be used in conjunction with other non-genetic diagnostic methods useful in cancer diagnosis, prognosis, or treatment monitoring. For example, in some circumstances it is beneficial to combine the diagnostic power of the gene expression based methods described above with data from conventional markers such as serum protein markers (e.g., Cancer Antigen 27.29 ("CA 27.29")). A range of such markers exists including such analytes as CA 27.29. In one such method, blood is periodically taken from a treated patient and then subjected to an enzyme immunoassay for one of the serum markers described above. When the concentration of the marker suggests the return of tumors or failure of therapy, a sample source amenable to gene expression analysis is taken. Where a suspicious mass exists, a fine needle aspirate (FNA) is taken and gene expression profiles of cells taken from the mass are then analyzed as described above. Alternatively, tissue samples may be taken from areas adjacent to the tissue from which a tumor was previously removed. This approach can be particularly useful when other testing produces ambiguous results.

The present disclosure encompasses methods of cross validating a gene expression profile and the profiles thus obtained, for thyroid carcinoma patients by: a. obtaining gene expression data from a statistically significant number of patient biological samples; b. randomizing sample order; c. setting aside data from about 10% - 50% of samples; d. computing, for the remaining samples, for factor of interest on all variables and selecting variables that meet a p-value cutoff (p); e. selecting variables that fit a prediction model using a forward search and evaluating the training error until it hits a predetermined error rate; f. testing the prediction model on the left-out 10-50% of samples; g. repeating steps c., -g. with a new set of samples removed; and h. continuing steps c) -g) until 100% of samples have been tested and record classification performance. In this method, preferably, the gene expression data obtained in step h. is represented by genes from those encoding mRNA: corresponding to SEQ ID NOs: 1, 4, 7, 8, 10-11, 13-17, 19-24, 26-27, 29-31, 33-35, 37-38, 40-52, 54-72, 75-82, 84-135, 138-141, 144-151, 153-159, 161-162, 164, 166-173, 176-198, 200-201, 203-206, 208-209, 212-213, 215-218, 220-221, 223, 227-233, 235-241, 243-244, 246-249, 251, 253-254, 256-263, 265-289, 291-293, 295-308, 310-331, 333-341, 343-345, 347-348, 350-353 and 355-363; or recognized specifically by the probe sets from psids in Table 25 corresponding to SEQ ID NOs: 1, 4, 7, 8, 10-11, 13-17, 19-24, 26-27, 29-31, 33-35, 37-38, 40-52, 54-72, 75-82, 84-135, 138-141, 144-151, 153-159, 161-162, 164, 166-173, 176-198, 200-201, 203-206, 208-209, 212-213, 215-218, 220-221, 223, 227-233, 235-241, 243-244, 246-249, 251, 253-254, 256-263, 265-289, 291-293, 295-308, 310-331, 333-341, 343-345, 347-348, 350-353 and 355-363.

The present disclosure encompasses methods of independently validating a gene expression profile and the profiles thus obtained, for thyroid cancer patients by obtaining gene expression data from a statistically significant number of patient biological samples; normalizing the source variabilities in the gene expression data; computing for factor of interest on all variables that were selected previously; and testing the prediction model on the sample and record classification performance. In this method, preferably, the gene expression data obtained in step d. is represented by genes from those encoding mRNA: corresponding to SEQ ID NOs: 1, 4, 7, 8, 10-11, 13-17, 19-24, 26-27, 29-31, 33-35, 37-38, 40-52, 54-72, 75-82, 84-135, 138-141, 144-151, 153-159, 161-162, 164, 166-173, 176-198, 200-201, 203-206, 208-209, 212-213, 215-218, 220-221, 223, 227-233, 235-241, 243-244, 246-249, 251, 253-254, 256-263, 265-289, 291-293, 295-308, 310-331, 333-341, 343-345, 347-348, 350-353 and 355-363; or recognized specifically by the probe sets from psids in Table 25 corresponding to SEQ ID NOs: 1, 4, 7, 8, 10-11, 13-17, 19-24, 26-27, 29-31, 33-35, 37-38, 40-52, 54-72, 75-82, 84-135, 138-141, 144-151, 153-159, 161-162, 164, 166-173, 176-198, 200-201, 203-206, 208-209, 212-213, 215-218, 220-221, 223, 227-233, 235-241, 243-244, 246-249, 251, 253-254, 256-263, 265-289, 291-293, 295-308, 310-331, 333-341, 343-345, 347-348, 350-353 and 355-363.

The present disclosure encompasses methods of generating a posterior probability to enable diagnosis of thyroid carcinoma patients by obtaining gene expression data from a statistically significant number of patient biological samples; applying linear discrimination analysis to the data to obtain selected genes; applying weighted expression levels to the selected genes with discriminate function factor to obtain a prediction model that can be applied as a posterior probability score. For instance, the following can be used for Linear Discriminant Analysis:

$$p_{(CP)} = \frac{e^{d_{(CP)} - d_{(CN)}}}{1 + e^{d_{(CP)} - d_{(CN)}}}$$

$$p_{(CN)} = \frac{1}{1 + e^{d_{(CP)} - d_{(CN)}}}$$

where,

$I_{(psid)}$ = The log base 2 intensity of the probe set enclosed in parenthesis.
$d_{(CP)}$ = The discriminant function for the cancer positive class
$d_{(CN)}$ = The discriminant function for the cancer negative class
$P_{(CP)}$ = The posterior p-value for the cancer positive class
$P_{(CN)}$ = The posterior p-value for the cancer negative class

The present disclosure encompasses methods of generating a thyroid carcinoma diagnostic patient report and reports obtained thereby, by obtaining a biological sample from the patient; measuring gene expression of the sample; applying a posterior probability score thereto; and using the results obtained thereby to generate the report. The report can also contain an assessment of patient outcome and/or probability of risk relative to the patient population.

The present invention encompasses compositions consisting of one probe set consisting of SEQ ID NOs: 36, 53, 76, 211 and 242.

The present invention encompasses kits for conducting an assay to determine thyroid carcinoma diagnosis in a biological sample consisting of materials for detecting isolated nucleic acid sequences, their complements, or portions thereof of a combination of genes encoding mRNA corresponding to SEQ ID NOs: 36, 53, 76, 211 and 242.

Kits made according to the invention include formatted assays for determining the gene expression profiles. These can include all or some of the materials needed to conduct the assays such as reagents and instructions and a medium through which nucleic acid sequences, their complements, or portions thereof are assayed.

Articles of this disclosure include representations of the gene expression profiles useful for treating, diagnosing, prognosticating, and otherwise assessing diseases. These profile representations are reduced to a medium that can be automatically read by a machine such as computer readable media (magnetic, optical, and the like). The articles can also include instructions for assessing the gene expression profiles in such media. For example, the articles may comprise a CD ROM having computer instructions for comparing gene expression profiles of the portfolios of genes described above. The articles may also have gene expression profiles digitally recorded therein so that they may be compared with gene expression data from patient samples. Alternatively, the profiles can be recorded in different representational format. A graphical recordation is one such format. Clustering algorithms such as those incorporated in "DISCOVERY" and "INFER" software from Partek, Inc. mentioned above can best assist in the visualization of such data.

Different types of articles of manufacture according to the disclosure are media or formatted assays used to reveal gene expression profiles. These can comprise, for example, microarrays in which sequence complements or probes are affixed to a matrix to which the sequences indicative of the genes of interest combine creating a readable determinant of their presence. Alternatively, articles according to the disclosure can be fashioned into reagent kits for conducting hybridization, amplification, and signal generation indicative of the level of expression of the genes of interest for detecting cancer.

The present disclosure encompasses articles for assessing thyroid carcinoma status containing: materials for detecting isolated nucleic acid sequences, their complements, or portions thereof of a combination of genes from those encoding mRNA: corresponding to SEQ ID NOs: 36, 53, 76, 211 and 242; and/or corresponding to SEQ ID NOs: 199, 207, 255 and 354; or recognized specifically by the probe sets from psids corresponding to SEQ ID NOs: 36, 53, 76, 211 and 242 as depicted in Table 25; and/or recognized specifically by the probe sets from psids corresponding to SEQ ID NOs: 199, 207, 255 and 354 as depicted in Table 25. The SEQ ID NOs. can be 36, 53, 76, 211 and 242; 199, 207, 255 and 354; or 45, 215, 65, 29, 190, 199, 207, 255 and 354.

The present disclosure encompasses diagnostic/prognostic portfolios containing isolated nucleic acid sequences, their complements, or portions thereof of a combination of genes from those encoding mRNA: corresponding to SEQ ID NOs: 36, 53, 76, 211 and 242; and/or corresponding to SEQ ID NOs: 199, 207, 255 and 354; or recognized specifically by the probe sets from psids corresponding to SEQ ID NOs: 36, 53, 76, 211 and 242 as depicted in Table 25; and/or recognized specifically by the probe sets from psids corresponding to SEQ ID NOs: 199, 207, 255 and 354 as depicted in Table 25 where the combination is sufficient to characterize thyroid carcinoma status or risk of relapse in a biological sample. Preferably, the portfolio measures or characterizes at least about 1.5-fold over- or under-expression or provides a statistically significant p-value over- or under-expression. Preferably, the p-value is less than 0.05.

The following examples are provided to illustrate but not limit the claimed invention.

**Example 1**

**Materials and Methods**

Tissue samples

Fresh frozen thyroid benign diseases, follicular adenoma, follicular carcinoma, and papillary carcinoma samples were obtained from different commercial vendors including Genomics Collaborative, Inc. (Cambridge, MA), Asterand (Detroit, MI), and Proteogenex (Los Angeles, CA). All samples were collected according to an Institutional Review Board approval protocol. Patients demographic and pathology information were also collected. The histopathological features of each sample were reviewed to confirm diagnosis, estimate sample preservation and tumor content.

RNA isolation

Standard TriZol protocol was used for all the RNA isolations. Tissue was homogenized in TriZol reagent (Invitrogen, Carlsbad, CA). Total RNA was isolated from TriZol and precipitated at -20°C with isopropyl alcohol. RNA pellets were washed with 75% ethanol, dissolved in water and stored at -80°C until use. RNA integrity was examined with Agilent 2100 Bioanalyser RNA 6000 NanoAssay (Agilent Technologies, Palo Alto, CA).

Linear Discrimination Analysis

Linear Discriminant Analysis was performed using these steps: calculation of a common (pooled) covariance matrix and within-group means; calculation of the set of linear discriminant functions from the common covariance and the within-group means; and classification using the linear discriminant functions.

Plugging the chip intensity readings for each probe into the following equation can be used to derive the posterior probability of an unknown thyroid sample as either cancer positive or negative. For example, if a thyroid sample is tested with the assay and gives a $p_{(CP)} > 0.5$ this sample will be classified as thyroid cancer.
For the 4 gene signature:

$$d_{(CP)} = -50.9964 + 0.220424(I_{(32128_at)}) + 1.520185(I_{(209810_at)}) + 3.09431(I_{(210078_s_at)}) + 6.11283(I_{(213423_x_at)})$$

$$d_{(CN)} = -46.7445 + 0.374751(I_{(32128_at)}) + 1.010852(I_{(209810_at)}) + 3.645515(I_{(210078_s_at)}) + 5.337296(I_{(213423_x_at)})$$

For the 5 gene signature:

$$d_{(CP)} = -135.931 + 4.737838(I_{(202363_at)}) - 1.23763(I_{(203256_at)}) + 1.984148(I_{(203889_at)}) + 6.638082(I(_{210342_s_at}) + 10.7704(I_{(212464_s_at)})$$

$$d_{(CN)} = -128.978 + 4.610498\ (I_{(202363_at)}) - 1.28685\ (I_{(203256_at)}) + 1.656772\ (I_{(203889_at)}) + 6.859133\ (I(_{210342_s_at}) + 10.24482\ (I_{(212464_s_at)})$$

$$p_{(CP)} = \frac{e^{d_{(CP)}-d_{(CN)}}}{1+e^{d_{(CP)}-d_{(CN)}}}$$

$$p_{(CN)} = \frac{1}{1 + e^{d_{(CP)} - d_{(CN)}}}$$

where,

$I_{(psid)}$ = The log base 2 intensity of the probe set enclosed in parenthesis.
$d_{(CP)}$ = The discriminant function for the cancer positive class
$d_{(CN)}$ = The discriminant function for the cancer negative class
$P_{(CP)}$ = The posterior p-value for the cancer positive class
$P_{(CN)}$ = The posterior p-value for the cancer negative class

Two-round aRNA amplification

aRNA was amplified from 10 ng total RNA using the RiboBeast 2-Round Aminoallyl-aRNA Amplification kit (Epicentre, WI), a T7 based RNA linear amplification protocol, with some modifications. Total RNA was reverse transcribed using an oligo(dT) primer containing a T7 RNA polymerase promoter sequence and Superscript III RT. The second-strand synthesis was carried out using Bst DNA polymerase. An extra step of incubation with an exonuclease mix of Exo I and Exo VII was performed to reduce background. The double-stranded cDNA served as the template for T7-mediated linear amplification by in vitro transcription. For the second round of amplification, instead of using the RiboBeast reagents, the ENZO BioArray HighYield RNA Transcript Labeling kit (Affymetrix, CA) was used in place of the in vitro transcription step of Aminoallyl-aRNA. The aRNA was quantified by Agilent Nano Chip technology.

**Example 2**

**Microarray analysis**

Labeled cRNA was prepared and hybridized with the high-density oligonucleotide array Hu133A Gene Chip (Affymetrix, Santa Clara, CA) containing a total of 22,000 probe sets. Hybridization was performed according to a standard protocol provided by the manufacturer. Arrays were scanned using Affymetrix protocols and scanners. For subsequent analysis, each probe set was considered as an independent gene. Expression values for each gene were calculated by using Affymetrix Gene Chip analysis software MAS 5.0. All chips met the following quality control standards: the percentage of "presence" call, the scaling factor, the background level, and the noise level have to be within the range of mean plus or minus 3 standard deviation. All chips used for subsequent analysis have passed these quality control criteria. Sample collection for signature selection and independent validation is summarized in Table 1.

Table 1. Sample collection for signature training and validation

| Training Sample Set | |
|---|---|
| Category | Number of Samples |
| Follicular Adenoma (FA) | 33 |
| Follicular Carcinoma (FC) | 21 |
| Benign Diseases (BN) | 13 |
| Papillary Carcinoma (PC) | 31 |
| Validation Sample Set | |
| Category | Number of Samples |
| Follicular Adenoma (FA) | 38 |
| Follicular Carcinoma (FC) | 5 |
| Follicular Variant of Papillary Carcinoma (FVPTC) | 11 |
| Papillary Carcinoma (PC) | 20 |

**Example 3**

**Results Signature Identification**

A. Gene Selection

A total of 98 samples including 31 primary papillary thyroid tumors, 21 follicular thyroid cancers, 33 follicular adenoma, and 13 benign thyroid tissues were analyzed by using Affymetrix human U133A gene chips. Five gene selection criteria were applied to the entire data set to obtain a limited number of genes for subsequent gene marker or signature identification:

1. Genes with at least one "Present Call" in this sample set were considered.
2. Genes with more than one "Present Call" in 12 PBL samples were excluded.
3. Only genes with chip intensity larger than 200 in all samples were selected.
4. Using genes that passed the above three criteria, we performed a variety of analyses, as listed in Table 2, to identify genes that are either up-regulated or down-regulated in thyroid tumors.
5. Finally, genes with expression change greater than 1.4-fold were selected.

Table 2. Summary of different types of percentile analyses

| | Type of Percentile Analysis |
|---|---|
| 1 | 20% FC vs 100% Benign |
| 2 | 30% FC vs 90% Benign |
| 3 | 30% PC vs 90% Benign |
| 4 | 70% FC vs 50% Benign |
| 5 | 70% PC vs 50% Benign |
| 6 | 90% Benign vs 30% FC |
| 7 | 90% Benign vs 30% PC |

The final number of selected genes for signature identification is 322, described in Table 25, SEQ ID NOs: 1, 4, 7, 8, 10-11, 13-17, 19-24, 26-27, 29-31, 33-35, 37-38, 40-52, 54-72, 75-82, 84-135, 138-141, 144-151, 153-159, 161-162, 164, 166-173, 176-198, 200-201, 203-206, 208-209, 212-213, 215-218, 220-221, 223, 227-233, 235-241, 243-244, 246-249, 251, 253-254, 256-263, 265-289, 291-293, 295-308, 310-331, 333-341, 343-345, 347-348, 350-353 and 355-363. The data obtained from the 322 selected genes are provided in Table 3 and summarized in Table 4.

Table 3

3a

| SEQ ID NO: | 800TT | 801TT | 802TT | 804TT | 805TT | 806TT | 807TT | 818TT | 819TT |
|---|---|---|---|---|---|---|---|---|---|
| 354 | 10235.3 | 163.8 | 12.2 | 99.1 | 111.6 | 2506.3 | 1780.7 | 29.3 | 4.1 |
| 355 | 508.5 | 150.8 | 31.5 | 83.8 | 63.1 | 457.5 | 189.9 | 48.2 | 45.3 |
| 356 | 82.7 | 754 | 707.3 | 477.3 | 1120.7 | 827.5 | 246.3 | 1068.3 | 960.1 |
| 357 | 834 | 758.9 | 718 | 249.4 | 883.6 | 554.5 | 377.5 | 616.1 | 706.9 |
| 358 | 2490.4 | 593.3 | 565.5 | 540.3 | 1132.7 | 750.4 | 803.7 | 377.4 | 534.6 |
| 359 | 533.5 | 342 | 412 | 409.4 | 413.3 | 396.5 | 309.5 | 352.8 | 317.7 |
| 362 | 136.4 | 550 | 399.6 | 653.8 | 573.4 | 322.4 | 539.5 | 317.5 | 324.9 |
| 360 | 476.2 | 324.5 | 352.5 | 348.2 | 473.3 | 342.9 | 170.3 | 190.1 | 279.4 |
| 361 | 196.4 | 995.8 | 839.3 | 382.6 | 926.9 | 480.8 | 305.3 | 913.9 | 1123.4 |
| 363 | 642.7 | 392.9 | 410.2 | 742.3 | 447.2 | 504.9 | 457.6 | 397 | 490.3 |
| 1 | 57.5 | 694.1 | 410.9 | 413.8 | 950.3 | 946.6 | 351.2 | 317.7 | 346.4 |
| 2 | 854 | 3127.2 | 2912.3 | 675.3 | 4484 | 3866 | 950.5 | 2514.3 | 2438.1 |
| 7 | 6.6 | 584 | 1380.1 | 315.4 | 543 | 393.6 | 303.9 | 713.9 | 821.6 |
| 8 | 6606.3 | 941.2 | 1104.5 | 736.7 | 749.7 | 1740.5 | 1083.6 | 423.7 | 583.3 |
| 9 | 1852.9 | 10149.7 | 6856.2 | 2912.1 | 6808.7 | 1233.4 | 3010.6 | 3890.5 | 2225.4 |
| 10 | 213.9 | 56.5 | 66.8 | 70.8 | 121.8 | 102.5 | 426.1 | 58.3 | 149.9 |
| 11 | 463.3 | 163.4 | 131.5 | 187.4 | 350.7 | 210.3 | 129.1 | 340.2 | 336.3 |
| 13 | 286.2 | 180.9 | 111.8 | 233.1 | 197.5 | 335.1 | 173.8 | 70.7 | 108.3 |
| 14 | 1590.2 | 437.8 | 323.5 | 558.1 | 897.5 | 1161.3 | 642.8 | 105.9 | 170.6 |
| 15 | 9293.6 | 467.4 | 332.9 | 823.3 | 3718.3 | 204 | 959.7 | 506.8 | 670.5 |
| 16 | 180 | 1093.1 | 911.1 | 674.5 | 960.2 | 1600.8 | 467 | 1244.5 | 995.6 |
| 17 | 850.8 | 1221.7 | 2320.1 | 687.9 | 1614.9 | 1605.7 | 404.6 | 1911.4 | 2114 |

| | | | | | | | | | |
|---|---|---|---|---|---|---|---|---|---|
| 19 | 411.1 | 1415.6 | 184.1 | 723.5 | 1035.5 | 370.5 | 548.9 | 147 | 192.6 |
| 20 | 733.9 | 1.1 | 1.1 | 74.5 | 256.4 | 1.1 | 326.7 | 1.1 | 1.2 |
| 21 | 72.3 | 200.7 | 12.9 | 81.5 | 88.3 | 242.5 | 67 | 11.5 | 37.4 |
| 22 | 3094.8 | 9706.3 | 7329.5 | 3225.3 | 6075.3 | 6859.5 | 2406.5 | 6706.8 | 8884.7 |
| 23 | 4749.1 | 837.4 | 1.1 | 835.6 | 2674.6 | 364.3 | 1127.5 | 1.1 | 31.7 |
| 24 | 965.6 | 221 | 39.2 | 361.9 | 738.2 | 167.3 | 169.5 | 71.9 | 121.6 |
| 26 | 7416.9 | 7158.3 | 127.4 | 6838.8 | 8238 | 2252.3 | 5033.6 | 65.1 | 119.9 |
| 27 | 58.1 | 722.5 | 759 | 815.4 | 651.8 | 689.9 | 359.8 | 573 | 381.9 |
| 29 | 215.3 | 111.1 | 55.4 | 103.7 | 90.2 | 70.5 | 65.9 | 199.3 | 105.7 |
| 30 | 5508.4 | 259.4 | 11.7 | 410.1 | 853.5 | 143.9 | 596.3 | 20.7 | 45.8 |
| 31 | 141.5 | 311.1 | 66.8 | 198.7 | 183.8 | 290.6 | 464.1 | 50.5 | 108.6 |
| 33 | 20935.4 | 341.8 | 144.8 | 828.3 | 7608.5 | 425 | 359.1 | 89.7 | 315.9 |
| 34 | 82.5 | 4387.6 | 4002.6 | 2634.3 | 4784.6 | 4798 | 2420.6 | 3104.2 | 3558 |
| 35 | 289.7 | 142.4 | 32.9 | 436.5 | 220.8 | 143.9 | 838.9 | 24.4 | 27.4 |
| 36 | 760.5 | 205.8 | 124 | 156.6 | 338.1 | 167.8 | 148.6 | 42.9 | 140.8 |
| 37 | 339.4 | 237.6 | 117.2 | 340.6 | 200.9 | 294.7 | 332.7 | 183.3 | 276.3 |
| 38 | 15634.5 | 361.2 | 546.7 | 505.1 | 4617.1 | 382.4 | 256.9 | 914.7 | 676.5 |
| 40 | 622.8 | 479.8 | 437.7 | 682.8 | 423.9 | 523.7 | 477.9 | 481.4 | 742.7 |
| 41 | 217.2 | 359.7 | 234.4 | 221.2 | 757.4 | 528.7 | 139.2 | 226.1 | 164.4 |
| 42 | 219.8 | 238.2 | 203.5 | 163.5 | 270.5 | 212.1 | 176.6 | 169.7 | 210.4 |
| 43 | 234.7 | 301 | 366.1 | 430.4 | 277 | 390.7 | 298.8 | 366.6 | 431.1 |
| 44 | 1194.4 | 428.6 | 352.7 | 678.6 | 1038.4 | 590.6 | 502.4 | 312.5 | 412.6 |
| 45 | 93.2 | 348.4 | 537.2 | 498.2 | 362.4 | 526.8 | 286.3 | 593.7 | 721.8 |
| 46 | 353.4 | 362.7 | 228.1 | 486.3 | 390 | 348.8 | 264.3 | 223.9 | 308.2 |
| 47 | 1440 | 122.7 | 195.5 | 267.7 | 384.4 | 279.8 | 279.8 | 186 | 120.1 |
| 48 | 199.4 | 203.4 | 16.7 | 1371.4 | 795.6 | 261.9 | 2274.7 | 36.6 | 21 |
| 49 | 195.9 | 337.7 | 268 | 285.8 | 341.3 | 420.2 | 392.8 | 175.7 | 207.1 |
| 50 | 8413 | 277.9 | 72.9 | 481 | 1790.1 | 240.3 | 276.3 | 19.6 | 144.4 |
| 51 | 548.4 | 42 | 29 | 436.1 | 66.9 | 35.2 | 265.4 | 38.8 | 35.4 |
| 52 | 189.3 | 199.3 | 189.3 | 144.9 | 217.9 | 179.8 | 189.7 | 265.6 | 263.1 |
| 53 | 90.2 | 79.3 | 46.2 | 51 | 36.3 | 81.2 | 109.4 | 83.5 | 33.9 |
| 54 | 879.2 | 383 | 1133.4 | 796.7 | 378.8 | 853 | 508.4 | 1350.7 | 462.2 |
| 55 | 69.1 | 130.4 | 81.7 | 126.9 | 65.7 | 123.7 | 96.6 | 86.1 | 118.9 |
| 56 | 29.9 | 53.2 | 83.6 | 82.1 | 125.5 | 80.4 | 57 | 64.5 | 59.6 |
| 57 | 5589.3 | 468.9 | 208.8 | 519 | 338.3 | 2738.1 | 2814.6 | 95.3 | 120 |
| 58 | 25483 | 492.1 | 294.6 | 702.4 | 495.2 | 2804.2 | 3544.8 | 95.7 | 164.7 |
| 59 | 57.6 | 1695.3 | 1278.9 | 884.1 | 1841.6 | 2339.6 | 560.2 | 1800.2 | 2204 |
| 60 | 308 | 154.2 | 148.1 | 188 | 151.4 | 261 | 115.3 | 120.2 | 177.2 |
| 61 | 193.7 | 168.2 | 134.2 | 228 | 153.6 | 138.7 | 132.1 | 83.1 | 176.1 |
| 62 | 119.4 | 478.9 | 203.5 | 1627.2 | 1442.2 | 1810.1 | 903.6 | 75 | 2303.9 |
| 63 | 98.7 | 380.9 | 470 | 197.8 | 346.6 | 224.8 | 242.2 | 297.5 | 192 |
| 64 | 143.1 | 133.4 | 210.8 | 393.3 | 197.5 | 194.5 | 165.5 | 154.4 | 209.9 |
| 65 | 328.5 | 124 | 64.1 | 172.8 | 348.1 | 151.2 | 192.8 | 101.1 | 127.9 |
| 66 | 249.1 | 732 | 1332.3 | 450.2 | 923.7 | 721.7 | 524.1 | 1163.3 | 1056 |
| 67 | 293.1 | 31554.3 | 31050.5 | 34557.4 | 35093.9 | 33778.1 | 21708.9 | 33200.2 | 37492 |
| 68 | 63 | 2405 | 2694.9 | 2196.1 | 5092.2 | 382 | 1972.5 | 2314.2 | 2245.9 |
| 69 | 756.5 | 5955.8 | 7077.6 | 5234.2 | 6301.1 | 1133.5 | 5870 | 6218.4 | 8519.4 |
| 70 | 424.6 | 2064.6 | 2173.1 | 4141.4 | 1212.7 | 1829.6 | 2418 | 1388.8 | 1591.4 |
| 71 | 499.3 | 545.4 | 159.2 | 507.5 | 2187.3 | 258.8 | 298.2 | 106.5 | 73.5 |
| 72 | 431.6 | 355.2 | 472.7 | 512.9 | 768.2 | 712.7 | 665.7 | 1451.1 | 410 |
| 73 | 272.6 | 264.5 | 266.7 | 235 | 473.2 | 341 | 218 | 433.5 | 361.9 |
| 75 | 120.4 | 364.8 | 176 | 279 | 491.9 | 225.9 | 244.3 | 377.5 | 440.7 |
| 76 | 153.3 | 176.5 | 46 | 143.8 | 95.5 | 498 | 182.1 | 2 | 34.8 |
| 77 | 102.1 | 3322.2 | 3704.4 | 3549.3 | 7422.7 | 3279.7 | 2337.5 | 2786.8 | 6811 |
| 78 | 297.4 | 973.1 | 1410.8 | 487 | 1074.9 | 411.4 | 315 | 1025.7 | 834.1 |
| 79 | 126.7 | 120.4 | 132.9 | 181.3 | 166.4 | 87.6 | 155.4 | 114.9 | 113.9 |
| 80 | 153.8 | 328.9 | 160.6 | 571.7 | 267 | 307.1 | 430.9 | 345.1 | 451.1 |
| 81 | 179.7 | 181.8 | 101.5 | 996.9 | 161.1 | 619.2 | 1156.4 | 73 | 105.5 |

| | | | | | | | | | |
|---|---|---|---|---|---|---|---|---|---|
| 82 | 54.9 | 362.8 | 385.4 | 367 | 264.5 | 251.3 | 241.3 | 414.2 | 220 |
| 84 | 101.2 | 291.6 | 129.7 | 162.2 | 330.9 | 286.7 | 196.3 | 75.3 | 77.5 |
| 85 | 558.7 | 171.1 | 121.8 | 116.1 | 116.9 | 188.5 | 368.6 | 191 | 161.4 |
| 86 | 100.5 | 90.5 | 67.6 | 100.4 | 71.3 | 78.5 | 104.6 | 80.5 | 101.4 |
| 87 | 144.9 | 375.2 | 299.1 | 190 | 267 | 227.3 | 94.1 | 70.7 | 488.1 |
| 88 | 136.1 | 4022.9 | 4905.4 | 2121.4 | 4345.4 | 4155.7 | 1621.6 | 5239.4 | 7618.2 |
| 89 | 361.1 | 158.9 | 159.8 | 335 | 218.5 | 244.6 | 224.8 | 136.2 | 163 |
| 90 | 1105.6 | 113.4 | 92.7 | 212.6 | 301.1 | 131.7 | 229.6 | 81.7 | 75.7 |
| 91 | 41283.8 | 405.2 | 83.4 | 373.4 | 101.5 | 3129.9 | 2037.3 | 10.9 | 73.8 |
| 92 | 125.5 | 4171.4 | 3562.5 | 2666.8 | 2820.2 | 7133.6 | 1929 | 11013.3 | 6783.5 |
| 93 | 102.9 | 336.1 | 636.6 | 108.4 | 214.1 | 218.9 | 125.8 | 330.1 | 222.8 |
| 94 | 173.3 | 927 | 600.9 | 384.5 | 1060.6 | 985.2 | 285.3 | 819.3 | 759 |
| 95 | 43.6 | 796 | 225.2 | 699.2 | 229.7 | 1129 | 264.2 | 589.6 | 347.5 |
| 96 | 166.4 | 173.4 | 234 | 108.8 | 198.5 | 171.6 | 90.4 | 154.2 | 139.9 |
| 97 | 28.3 | 196.3 | 256.1 | 110.4 | 302.3 | 307.3 | 112.4 | 520.9 | 415.1 |
| 98 | 33 | 47.3 | 44.5 | 6.1 | 33.2 | 34.4 | 62.9 | 9.7 | 34 |
| 99 | 315.9 | 219.6 | 221.2 | 281.5 | 254.6 | 306.8 | 218.9 | 200 | 240.6 |
| 100 | 40.3 | 12275.1 | 7465.8 | 4158.8 | 5537.2 | 19961.1 | 1057.9 | 1835.4 | 28160.9 |
| 101 | 169.6 | 185 | 264.2 | 312.3 | 225.5 | 277.8 | 165.2 | 258.7 | 276.2 |
| 102 | 208.7 | 160.9 | 193 | 308.2 | 155.3 | 201.8 | 154.2 | 171.3 | 189.5 |
| 103 | 402 | 302.9 | 300.1 | 382.9 | 342.4 | 374.1 | 315.3 | 292.4 | 280.2 |
| 104 | 25.2 | 569 | 354.3 | 402.2 | 554.5 | 1086.3 | 407.5 | 469.3 | 840.9 |
| 105 | 46.4 | 550.9 | 418 | 437.1 | 724.9 | 1141 | 481.6 | 596 | 1001.3 |
| 106 | 15.2 | 622.8 | 398.8 | 419.8 | 577.5 | 1074.2 | 377.8 | 444.5 | 794.4 |
| 107 | 6337.1 | 255.7 | 210 | 448.7 | 353.8 | 639.6 | 315 | 91.3 | 155.9 |
| 108 | 167.9 | 316.2 | 436.9 | 315 | 429.5 | 269.2 | 221.7 | 417.3 | 467 |
| 109 | 64.8 | 1110.3 | 875.6 | 160.6 | 318.4 | 942.9 | 215.8 | 1481.1 | 53.8 |
| 110 | 1.1 | 1261.5 | 1589.1 | 263.4 | 923.8 | 4084.9 | 415 | 4417 | 6.3 |
| 111 | 33.1 | 1767.8 | 1964.9 | 1872.4 | 427.3 | 1690.7 | 1292.2 | 749.5 | 1008.9 |
| 112 | 1.3 | 407.2 | 104.1 | 401.9 | 271.4 | 454.9 | 209.1 | 219.9 | 30.2 |
| 113 | 139 | 51.3 | 189.9 | 275.6 | 197.3 | 169.2 | 225.7 | 104.1 | 185.9 |
| 114 | 268.1 | 679 | 853.3 | 378.5 | 772.9 | 883.3 | 311 | 353.1 | 443.1 |
| 115 | 357.8 | 569.5 | 376 | 383.7 | 574.8 | 380.6 | 337.8 | 221.5 | 323.5 |
| 116 | 291.2 | 223 | 163.2 | 334.1 | 242.5 | 288.6 | 199.1 | 177.2 | 195.2 |
| 117 | 289.8 | 5414.4 | 4654.4 | 1394.2 | 3310.6 | 2815.7 | 721.7 | 3557.6 | 5696.6 |
| 118 | 92 | 533.6 | 90.4 | 527.7 | 275.1 | 665.7 | 306.8 | 60.3 | 99.6 |
| 119 | 114.7 | 226.3 | 336.7 | 194.9 | 102.7 | 168.3 | 397.7 | 81.7 | 65 |
| 120 | 78.6 | 116.4 | 118.2 | 137 | 338.5 | 139 | 164.3 | 51.8 | 73.3 |
| 121 | 161.2 | 788.8 | 997.1 | 260.6 | 745.8 | 1450.3 | 199.5 | 403.7 | 483.3 |
| 122 | 90.3 | 263.5 | 89.9 | 186.6 | 106.4 | 226.4 | 124.7 | 51.1 | 205 |
| 123 | 172.4 | 136.9 | 175 | 193.8 | 162.9 | 133.6 | 111.5 | 92.3 | 143.1 |
| 124 | 182.9 | 130.4 | 129.2 | 229.5 | 137.2 | 159 | 182.4 | 106.9 | 127 |
| 125 | 771.9 | 885.8 | 846.9 | 1521.7 | 915.3 | 963.4 | 856.9 | 836.5 | 775.4 |
| 126 | 25.4 | 1583.4 | 1549.9 | 728 | 2100.4 | 1526.4 | 558.8 | 1490.2 | 1266 |
| 127 | 101.3 | 121.8 | 97.6 | 163.5 | 99.6 | 101.1 | 101.1 | 74.1 | 160.2 |
| 128 | 346 | 234 | 238.5 | 469 | 239.1 | 325 | 198.4 | 245.9 | 311.8 |
| 129 | 4494.1 | 248.8 | 234 | 274.6 | 301.2 | 383.9 | 270 | 72.7 | 148.1 |
| 130 | 117.9 | 100.2 | 138.4 | 121.9 | 98.9 | 104 | 97.7 | 33 | 120.6 |
| 131 | 95.6 | 67.8 | 71.9 | 133.7 | 83.3 | 81.3 | 92 | 127.2 | 160.1 |
| 132 | 232 | 188.4 | 200.5 | 251 | 174 | 266.8 | 171.5 | 186.5 | 257.7 |
| 133 | 31.9 | 91.9 | 61.8 | 130.6 | 56.7 | 175.5 | 98.6 | 96.9 | 108.1 |
| 134 | 68.7 | 109.8 | 181.9 | 126.1 | 162.2 | 149.1 | 117.5 | 169.9 | 162.7 |
| 135 | 342.4 | 599 | 423.7 | 336.6 | 593.1 | 454.2 | 196.2 | 246.7 | 390.2 |
| 138 | 376.2 | 149.8 | 97.5 | 172.2 | 167.1 | 141.1 | 170.9 | 102.7 | 126.1 |
| 139 | 109.4 | 107.8 | 120.5 | 114.4 | 131 | 110 | 134.2 | 47.6 | 78.6 |
| 140 | 50.4 | 4099.6 | 2561.4 | 1129.9 | 2225.8 | 4646.8 | 179.3 | 3587 | 3225.4 |
| 141 | 28.7 | 1684.3 | 2067.9 | 1954.8 | 1743.8 | 2519.7 | 838.9 | 2235.9 | 1940.3 |
| 144 | 530.5 | 489.8 | 450.9 | 680.6 | 482.9 | 521.4 | 406.9 | 3706.9 | 433.4 |

| | | | | | | | | | |
|---|---|---|---|---|---|---|---|---|---|
| 145 | 237.2 | 196.3 | 199.6 | 376.6 | 227.5 | 244 | 164 | 175.5 | 217.2 |
| 146 | 240 | 207.7 | 196.5 | 287 | 216.2 | 221.3 | 171.1 | 170.3 | 240.1 |
| 147 | 34.8 | 59.1 | 32.6 | 71.1 | 106.8 | 38 | 76.9 | 66.7 | 44.2 |
| 148 | 677.5 | 348.2 | 249.8 | 461.1 | 251.5 | 501.4 | 616.9 | 292.6 | 327.2 |
| 149 | 35.2 | 397.1 | 361.6 | 203.1 | 742.4 | 464.8 | 110.1 | 706.6 | 571 |
| 150 | 205.4 | 160.9 | 229.9 | 251 | 200.4 | 189.2 | 332.7 | 156.6 | 501.8 |
| 151 | 173.8 | 466.2 | 220 | 285.9 | 235.8 | 499.7 | 139.8 | 284.8 | 258.5 |
| 153 | 96.7 | 149.4 | 109.5 | 120 | 139.6 | 106.3 | 110.4 | 101.5 | 94.3 |
| 154 | 353.7 | 288.8 | 62.8 | 174.6 | 358.1 | 579.2 | 122.3 | 45.3 | 359.9 |
| 155 | 497.4 | 136.2 | 109.5 | 189.4 | 118.5 | 147.5 | 131.9 | 115.5 | 137.5 |
| 156 | 73 | 97.7 | 84.6 | 132.7 | 100.1 | 118.9 | 87 | 64.6 | 73 |
| 157 | 153 | 149.2 | 105 | 143.1 | 123.7 | 160.2 | 159 | 151.6 | 147.2 |
| 158 | 1.3 | 8.9 | 102.4 | 99.5 | 1.1 | 23.1 | 74.8 | 7.9 | 93.2 |
| 159 | 33 | 2887.3 | 3372.9 | 2710.7 | 3432.1 | 3979.2 | 1070.5 | 3809.9 | 4336.8 |
| 161 | 1.3 | 134.2 | 113.3 | 263.6 | 128.2 | 276.1 | 557.1 | 118.4 | 2.9 |
| 162 | 1735.6 | 170 | 24.1 | 264.5 | 702.3 | 122.3 | 365 | 31.1 | 85.1 |
| 164 | 94.6 | 209.1 | 2869.2 | 348.2 | 520.8 | 2102.6 | 33.2 | 1670.7 | 436.5 |
| 166 | 700.4 | 6433.5 | 9921.6 | 4090.9 | 7108.3 | 3609.5 | 3473.4 | 7815.7 | 5478.4 |
| 167 | 1.2 | 2320.4 | 1318 | 1994.5 | 3064.9 | 2291.7 | 729.2 | 3184.3 | 2565.5 |
| 168 | 190 | 593.2 | 775.5 | 598.1 | 769.2 | 610.1 | 414.8 | 1931.6 | 1123.8 |
| 169 | 512.2 | 4400.2 | 2669.8 | 2975.6 | 2812.9 | 6215.7 | 3878.6 | 1890.1 | 829.9 |
| 170 | 73.5 | 1014.4 | 2146.7 | 775.8 | 1454.9 | 1033.1 | 409.3 | 2535.1 | 867.2 |
| 171 | 5104.5 | 1332.2 | 272.6 | 2234.5 | 4145.4 | 565.7 | 3636.7 | 1.3 | 1.2 |
| 172 | 697.5 | 650.4 | 797.2 | 721.1 | 621.5 | 792.4 | 573.5 | 666.6 | 657 |
| 173 | 117 | 169.1 | 136.9 | 226.4 | 229 | 245.8 | 226.4 | 407.6 | 310.5 |
| 176 | 1.2 | 1.2 | 3906.6 | 275.7 | 74.8 | 809.2 | 3.4 | 2700.3 | 62.8 |
| 177 | 101 | 617.1 | 461.8 | 602.7 | 515.7 | 623.3 | 414 | 876.6 | 749.6 |
| 178 | 167.1 | 612.8 | 1577.1 | 197.3 | 112.9 | 554.5 | 318.8 | 310.2 | 64.7 |
| 179 | 153.4 | 58.6 | 90.9 | 90 | 66.8 | 93.5 | 114.4 | 42.3 | 93.2 |
| 180 | 501.7 | 70.9 | 72.4 | 141.5 | 208.3 | 85.6 | 126.3 | 91.4 | 102.8 |
| 181 | 624.8 | 12366.2 | 9356.2 | 8736.7 | 10384.9 | 9225.7 | 6284 | 9589 | 10736.6 |
| 182 | 416.7 | 513.2 | 472.4 | 300.9 | 658.9 | 537.7 | 246.8 | 533.1 | 527.2 |
| 183 | 2674.8 | 1193.3 | 1.1 | 989.7 | 2589.1 | 336.1 | 836.3 | 1.1 | 10.3 |
| 184 | 424.7 | 204.6 | 210.9 | 228.6 | 394.8 | 275.7 | 205.5 | 185.6 | 179.6 |
| 185 | 1535 | 1703.8 | 1133.3 | 2133.8 | 2209.6 | 911.3 | 1201.8 | 1162.9 | 1148.4 |
| 186 | 489.1 | 547.1 | 422.7 | 473 | 508.7 | 434.6 | 349.5 | 507.5 | 315 |
| 187 | 1394 | 260.6 | 57.1 | 168.9 | 226.7 | 84.5 | 424 | 34.5 | 60.7 |
| 188 | 257.6 | 162.3 | 197.5 | 380.9 | 488.7 | 103.9 | 313.1 | 102.5 | 160.7 |
| 189 | 66.4 | 86.3 | 121.9 | 244.8 | 239.7 | 78.6 | 190.3 | 75.4 | 112.2 |
| 190 | 78.1 | 83.5 | 429.5 | 280.5 | 102.5 | 357.6 | 91.9 | 97.6 | 84.8 |
| 191 | 5186 | 883.9 | 1804.8 | 892.9 | 1302.6 | 986.6 | 662.9 | 1537.4 | 1728.1 |
| 192 | 183.6 | 117.1 | 105.4 | 207.3 | 160.4 | 130.7 | 133.3 | 86.8 | 109.4 |
| 193 | 75.2 | 1034.1 | 1775.6 | 473 | 1246.2 | 728.5 | 400.4 | 2163.8 | 1704.8 |
| 194 | 35.4 | 141 | 109.3 | 203.6 | 162.4 | 216.6 | 81.4 | 282.5 | 166.3 |
| 195 | 166.4 | 286.5 | 375.7 | 319.3 | 152.4 | 274.8 | 209.4 | 184.4 | 172.6 |
| 196 | 3.1 | 6834.9 | 4672.9 | 5030.2 | 2309.2 | 6216.8 | 4103.7 | 6137.9 | 6356.5 |
| 197 | 99.9 | 104 | 74.8 | 123.9 | 144.8 | 74.6 | 78.7 | 42.6 | 76.1 |
| 198 | 64.1 | 63.1 | 53.4 | 170.6 | 81.1 | 107.7 | 53.7 | 58.8 | 105 |
| 199 | 831.8 | 240 | 58.3 | 107.9 | 91.4 | 602.1 | 232 | 56.8 | 73.6 |
| 200 | 526.8 | 384.3 | 120.9 | 388.4 | 798.8 | 293.1 | 355.9 | 148.2 | 153 |
| 201 | 8960.4 | 165 | 22.2 | 144.7 | 80.9 | 2818 | 2028.7 | 40.3 | 67.2 |
| 203 | 81.3 | 472.4 | 610.9 | 91.8 | 451.2 | 472.3 | 101.5 | 229.9 | 173.5 |
| 204 | 111.5 | 557.7 | 815 | 343.5 | 524.4 | 484.1 | 188.1 | 623.8 | 532.3 |
| 205 | 110.1 | 6177.9 | 4916.2 | 3074.2 | 4380.7 | 5662.9 | 2513.5 | 6031.4 | 4413.3 |
| 206 | 327.4 | 232 | 221.4 | 2279.4 | 1294.7 | 242.3 | 23853.3 | 182.8 | 201.2 |
| 207 | 140.6 | 1170.8 | 430.3 | 478 | 1596.2 | 1348.5 | 347.2 | 2523.2 | 2245.3 |
| 208 | 102.4 | 611.4 | 208 | 94.9 | 323 | 317.4 | 205.4 | 75 | 371.1 |
| 209 | 19.6 | 3466.7 | 4058.3 | 3621 | 1656.7 | 3787.3 | 1837.3 | 3395.9 | 2777.2 |

| | | | | | | | | | |
|---|---|---|---|---|---|---|---|---|---|
| 211 | 68.5 | 13748.3 | 30156 | 19158.6 | 20412.6 | 11472.5 | 5548.4 | 13311.6 | 18245.4 |
| 212 | 97.3 | 492.1 | 523.3 | 1191.7 | 274.3 | 490.1 | 563.4 | 430.6 | 523.7 |
| 213 | 118.2 | 98.7 | 138 | 131.3 | 79.4 | 72.3 | 107.5 | 56.1 | 87.5 |
| 215 | 74.5 | 1676.2 | 558.9 | 803.5 | 1642.3 | 2856.5 | 476.4 | 4663.2 | 3720.1 |
| 216 | 75.1 | 912.5 | 1082.4 | 602 | 874 | 1309.1 | 366.3 | 1248.6 | 1513.4 |
| 217 | 253.9 | 338.8 | 345.6 | 232.8 | 478.1 | 255.5 | 260.8 | 183.8 | 153.8 |
| 218 | 217.6 | 281.1 | 360.1 | 283.7 | 411.2 | 483.8 | 185.6 | 541.5 | 461.7 |
| 220 | 116.2 | 103.9 | 66.9 | 97.5 | 83.3 | 99.7 | 69.5 | 88.4 | 87.1 |
| 221 | 587 | 961.9 | 558.8 | 297.6 | 954.6 | 365.8 | 346.2 | 594 | 456.9 |
| 223 | 239.4 | 255 | 135.7 | 374.9 | 272.4 | 283.3 | 188.8 | 205.6 | 338.9 |
| 227 | 16526.9 | 687.5 | 402.1 | 801.2 | 8633.7 | 549.7 | 683 | 662.9 | 533.2 |
| 228 | 109.5 | 590.7 | 434.3 | 616.8 | 410.6 | 745.3 | 291.4 | 596.4 | 397.3 |
| 229 | 238.4 | 255.8 | 193.3 | 117.2 | 266.5 | 145.6 | 228.4 | 102.2 | 491.9 |
| 230 | 226.6 | 172.7 | 87.5 | 128.9 | 178.7 | 109 | 152.9 | 81.6 | 97.2 |
| 231 | 164.7 | 239.9 | 241 | 177.8 | 240.8 | 204.3 | 82.9 | 126.8 | 110.2 |
| 232 | 726.2 | 6011.4 | 69.4 | 4687.8 | 4109.5 | 1565.9 | 3685.2 | 55.1 | 121.4 |
| 233 | 1921.3 | 10660.6 | 3028 | 2711.7 | 7662.7 | 7862.6 | 1829 | 3283.3 | 5519.5 |
| 235 | 1985.5 | 721.4 | 238.4 | 931.8 | 2117.4 | 492.2 | 1437 | 77 | 96.1 |
| 236 | 111.5 | 389.5 | 124.3 | 281.9 | 194.3 | 199.7 | 268.5 | 87 | 132.1 |
| 237 | 678.2 | 2297.4 | 1864.1 | 1203 | 1986.1 | 1743 | 1173.9 | 1963.8 | 2872.9 |
| 238 | 100.7 | 1551.2 | 1160.8 | 572.2 | 1309.9 | 1228.5 | 451 | 1210.1 | 642.7 |
| 239 | 332.1 | 763.7 | 202 | 225.7 | 393.5 | 575.5 | 339 | 267.4 | 73.6 |
| 240 | 2452.6 | 853.2 | 251.5 | 167.9 | 951.3 | 599.5 | 378.3 | 208.9 | 113.2 |
| 241 | 3976.2 | 1815.3 | 487.3 | 320 | 1245.6 | 1299.5 | 844.5 | 423.8 | 113.2 |
| 242 | 22247.9 | 1204.2 | 676.7 | 2441.5 | 3900.1 | 2073.4 | 2060.1 | 1433.3 | 2211.8 |
| 243 | 500.8 | 602.3 | 147.7 | 417.4 | 460.7 | 206.9 | 420.7 | 210.1 | 264.1 |
| 244 | 408.8 | 194.7 | 250 | 139.3 | 215 | 163.3 | 362.3 | 191.9 | 185.1 |
| 246 | 346.4 | 311.5 | 153.6 | 129.5 | 426.7 | 193.8 | 297.6 | 237.1 | 115.3 |
| 247 | 228 | 128.5 | 128.2 | 180.1 | 149.8 | 122.1 | 119 | 95 | 82.6 |
| 248 | 84.9 | 198.9 | 142.5 | 115.1 | 171.7 | 266.5 | 76.9 | 113.4 | 199.3 |
| 249 | 1035.7 | 288.9 | 2257.9 | 857.3 | 1164 | 1466.9 | 537.1 | 513.5 | 696.2 |
| 251 | 264 | 88.8 | 78.2 | 123 | 232.6 | 138 | 74.6 | 121.3 | 124.6 |
| 253 | 108.7 | 314.2 | 326.6 | 251.4 | 350.9 | 226.2 | 192.2 | 227.9 | 226.6 |
| 254 | 141.8 | 397.1 | 285.7 | 91.7 | 668.4 | 932.2 | 144.2 | 644.2 | 128.5 |
| 255 | 158.7 | 631.9 | 1918.6 | 177.7 | 94.3 | 560.5 | 328.5 | 275.4 | 74 |
| 256 | 28050 | 525.2 | 120.3 | 424.5 | 151.7 | 1961.1 | 1539.7 | 156.2 | 137.1 |
| 257 | 114.5 | 99.8 | 83 | 100.6 | 201.5 | 126.4 | 86.7 | 157.4 | 157.6 |
| 258 | 158.6 | 49.9 | 1.2 | 230.7 | 87 | 162.9 | 312.2 | 9.5 | 188.6 |
| 259 | 1056.7 | 678.8 | 74.6 | 435 | 975.6 | 278.5 | 381.2 | 78.8 | 19.9 |
| 260 | 181.2 | 203.6 | 112.1 | 206.3 | 156.5 | 223.4 | 178.5 | 122.7 | 146.6 |
| 261 | 102.9 | 308.1 | 236.7 | 105 | 398.6 | 247.2 | 179 | 878.1 | 456.3 |
| 263 | 715.4 | 341.5 | 448.7 | 152.3 | 586.5 | 224.7 | 469.7 | 458.1 | 389 |
| 263 | 497.6 | 226.9 | 137.3 | 149.6 | 251 | 166.4 | 214.5 | 85.4 | 135.2 |
| 265 | 94.8 | 312.7 | 829.9 | 450.4 | 436.1 | 369.5 | 258.6 | 592.9 | 638.8 |
| 266 | 265.8 | 327 | 344.5 | 288.3 | 382.4 | 344.9 | 252.4 | 143.5 | 246 |
| 256 | 250.1 | 118.4 | 95.6 | 163.6 | 115.3 | 138.5 | 119.7 | 91.9 | 107.1 |
| 268 | 9.8 | 979.7 | 661.1 | 894.3 | 411.9 | 1696.9 | 366.1 | 409.7 | 1018.8 |
| 269 | 54 | 49.9 | 40.5 | 67.3 | 34.6 | 39.1 | 37.9 | 27 | 41.9 |
| 270 | 250 | 173.8 | 181.8 | 187.3 | 170 | 224.5 | 142.3 | 228.7 | 279.3 |
| 271 | 480.9 | 213.9 | 276.2 | 361 | 316 | 273.2 | 153.9 | 288.4 | 298 |
| 272 | 848.6 | 504.7 | 506.3 | 704.7 | 547.1 | 495.8 | 552.5 | 478.3 | 596.9 |
| 273 | 1.2 | 2102.3 | 4434.2 | 854.4 | 4432 | 2983.7 | 1404 | 2522.8 | 2416.9 |
| 274 | 1684.7 | 1062 | 218.2 | 668.5 | 812.7 | 391.8 | 925.2 | 159.9 | 290.7 |
| 275 | 287.7 | 447.3 | 282.7 | 622.1 | 481 | 245.2 | 328.9 | 276.9 | 234.7 |
| 276 | 24704.9 | 1039.3 | 565 | 1641.6 | 11368.8 | 805.3 | 1068.9 | 882.8 | 583 |
| 277 | 242 | 197.4 | 155.1 | 247.2 | 180.4 | 234.4 | 184.7 | 192.8 | 192.5 |
| 278 | 1231.4 | 973 | 145.3 | 901.4 | 1474.2 | 594.6 | 925.8 | 126 | 131.6 |
| 279 | 17.3 | 2232.4 | 2050.1 | 1268 | 1303.2 | 2923.4 | 900.4 | 2377.3 | 1977.2 |

| | | | | | | | | | |
|---|---|---|---|---|---|---|---|---|---|
| 280 | 59.4 | 2645.5 | 2730.2 | 1625.9 | 2545.4 | 3355.1 | 1233.4 | 2801.5 | 3378.5 |
| 281 | 11.1 | 384.8 | 65 | 38.7 | 71.4 | 100.9 | 103.7 | 14.1 | 17.9 |
| 282 | 608.4 | 296.3 | 256.6 | 463.7 | 205.6 | 325.1 | 415.2 | 194.5 | 210.3 |
| 283 | 332.9 | 264.5 | 234.2 | 544.1 | 264.3 | 374.3 | 400.4 | 291.5 | 271.4 |
| 284 | 78.3 | 67.8 | 69.4 | 78.9 | 68 | 92 | 71.1 | 59.9 | 88.3 |
| 285 | 128.1 | 76.9 | 110.6 | 115.5 | 106.6 | 107.8 | 83.1 | 89.2 | 95.8 |
| 286 | 136.9 | 882.8 | 1547.8 | 797.2 | 698 | 1112.3 | 458.4 | 673.7 | 251.9 |
| 287 | 133.4 | 121.2 | 112.9 | 117.4 | 91.9 | 158.2 | 87 | 70.4 | 94.5 |
| 288 | 171.7 | 149.6 | 158.2 | 178.1 | 157.8 | 201 | 117 | 123.4 | 124.4 |
| 289 | 2065.2 | 477.9 | 207.9 | 783.6 | 1036.1 | 420.3 | 2425.5 | 18.4 | 1.3 |
| 291 | 133.5 | 64.4 | 97.5 | 72.7 | 132.7 | 73.5 | 87.3 | 58.9 | 49.4 |
| 292 | 584.5 | 63.2 | 25 | 154.1 | 1191.9 | 660.3 | 757.5 | 27.5 | 40.9 |
| 293 | 85.6 | 186.6 | 107 | 288.9 | 87.8 | 189 | 235.3 | 128.1 | 55.8 |
| 295 | 99.7 | 791.3 | 242.3 | 325.4 | 327.2 | 175.4 | 230.6 | 133.1 | 144.9 |
| 296 | 676.2 | 918.1 | 709.4 | 934.2 | 1877.1 | 1141.7 | 589.6 | 753.4 | 857.2 |
| 297 | 208.1 | 61.9 | 45 | 125.1 | 78.1 | 67 | 128.8 | 33.5 | 56.2 |
| 298 | 486.9 | 293.3 | 320.6 | 257.4 | 410.5 | 356.9 | 211.9 | 196.2 | 400.4 |
| 299 | 238.1 | 923.6 | 448.9 | 1006.6 | 1054.5 | 910 | 660.9 | 696.1 | 686.7 |
| 300 | 215.4 | 203.8 | 395.4 | 255.6 | 312.3 | 188.4 | 202.2 | 244.5 | 347.9 |
| 301 | 212.9 | 290 | 820.4 | 372.3 | 708.3 | 367.5 | 860.1 | 1368.3 | 1185.7 |
| 302 | 29.5 | 25.8 | 10.5 | 19 | 34.5 | 45.7 | 38.4 | 9 | 11.2 |
| 303 | 244.8 | 782.2 | 902.3 | 349.1 | 907.8 | 713.9 | 323.7 | 823.8 | 976.6 |
| 304 | 1861.6 | 166.7 | 207.3 | 308.3 | 367.6 | 378.4 | 394.1 | 160.5 | 243.8 |
| 305 | 175.3 | 159.5 | 1205.8 | 156.7 | 144.6 | 223.3 | 176.9 | 191.7 | 180 |
| 306 | 257.6 | 154.3 | 146.1 | 240.3 | 149 | 206.8 | 136.3 | 126 | 182.9 |
| 307 | 140.9 | 142.6 | 126.5 | 207 | 130.3 | 179.3 | 122.6 | 127.1 | 144.6 |
| 308 | 51.2 | 1843.1 | 1030.9 | 1212.9 | 1456.7 | 2208.3 | 994.9 | 1319.8 | 3640.6 |
| 310 | 56.1 | 389.3 | 310.4 | 316.3 | 466.8 | 385.1 | 278.8 | 726.7 | 401.6 |
| 311 | 95.8 | 109.3 | 154.9 | 90 | 107.1 | 120.1 | 96.3 | 40.5 | 202.1 |
| 312 | 1.1 | 193.8 | 599 | 390.5 | 611.6 | 728.7 | 365.5 | 1657.6 | 289.8 |
| 313 | 114.1 | 119.7 | 208.9 | 142.2 | 124.9 | 120.7 | 102.2 | 171.4 | 137.5 |
| 314 | 228.4 | 414.8 | 146.7 | 189.2 | 419.3 | 985 | 268.1 | 164.1 | 805.7 |
| 315 | 106.4 | 758.2 | 540.2 | 634.9 | 351.4 | 466.9 | 317.2 | 369.3 | 322.4 |
| 316 | 87.3 | 363.4 | 151 | 353 | 425.1 | 312.8 | 284.4 | 214.7 | 159.2 |
| 317 | 349.5 | 178.8 | 142.4 | 246 | 187.7 | 213.8 | 173.4 | 172.5 | 170.8 |
| 318 | 41 | 165 | 47.1 | 88.3 | 62.8 | 174.6 | 58.9 | 29.9 | 36.8 |
| 319 | 434.9 | 249.1 | 85.8 | 420.7 | 189.3 | 189.1 | 249.8 | 96.7 | 149.6 |
| 320 | 107.4 | 586.7 | 418.3 | 403.8 | 488.4 | 523.9 | 404.2 | 689.4 | 494.1 |
| 321 | 543.8 | 319.1 | 469.7 | 514.1 | 488.3 | 438.7 | 255 | 599.8 | 413.8 |
| 322 | 143.6 | 271.4 | 269.5 | 298.2 | 237 | 237.4 | 211.8 | 133.2 | 239 |
| 323 | 70.7 | 3285.6 | 3624.3 | 1105.3 | 4318.5 | 2487.4 | 569 | 2285.5 | 3132.6 |
| 324 | 83.1 | 109.6 | 285.4 | 143.7 | 116.7 | 132.5 | 85.8 | 120.8 | 77.4 |
| 325 | 110.9 | 251.9 | 316.5 | 290 | 236.5 | 383.2 | 230.7 | 313.5 | 303.7 |
| 326 | 135.5 | 3242.5 | 8125.6 | 4127.5 | 5469.6 | 4502.9 | 1608.5 | 3693.9 | 2895.8 |
| 327 | 58.5 | 45 | 1.2 | 25 | 59.9 | 35 | 17.5 | 37.7 | 1.1 |
| 328 | 65.8 | 655.8 | 464.1 | 320.5 | 288.4 | 542.9 | 197.8 | 325.2 | 508.2 |
| 329 | 80 | 64.2 | 69 | 112.3 | 56 | 94.8 | 79.6 | 45.7 | 59.5 |
| 330 | 40.5 | 80.1 | 60.7 | 58 | 25.8 | 201.8 | 52.3 | 15.2 | 35 |
| 331 | 432.9 | 313.6 | 325.8 | 596 | 282.5 | 296.4 | 296.4 | 323.7 | 289.3 |
| 333 | 130.6 | 705.5 | 966.7 | 555.7 | 561.6 | 839.7 | 421.8 | 765.6 | 867.7 |
| 334 | 249.3 | 142.3 | 160.4 | 202.9 | 176.2 | 182.3 | 124.3 | 206.3 | 169.1 |
| 335 | 82.5 | 80.4 | 83 | 109.4 | 123.6 | 101.9 | 119.4 | 73.9 | 145.7 |
| 336 | 190.4 | 460 | 647.9 | 449.1 | 599.6 | 811.8 | 357.9 | 794.4 | 514 |
| 337 | 325.2 | 155.4 | 169.1 | 87.4 | 565.9 | 331.4 | 139.4 | 121 | 218.6 |
| 338 | 456.9 | 208.6 | 167.7 | 232 | 253.9 | 347.6 | 196.9 | 230.3 | 166.2 |
| 339 | 286.9 | 555.1 | 405.8 | 559 | 379.8 | 628.6 | 310.9 | 351.5 | 538.6 |
| 340 | 512.3 | 229.9 | 206.7 | 351.4 | 259.7 | 284.5 | 241.8 | 186.5 | 258.4 |
| 341 | 149.5 | 767.9 | 579.4 | 500.3 | 838.9 | 598.2 | 472.1 | 804.7 | 865.1 |

| | | | | | | | | | |
|---|---|---|---|---|---|---|---|---|---|
| 343 | 152.9 | 40.5 | 579 | 1479.8 | 52.4 | 120.2 | 411.9 | 326.7 | 85 |
| 344 | 233.7 | 1144.2 | 879.2 | 1306.4 | 1034.5 | 1267 | 1054.1 | 1516.9 | 1049.3 |
| 345 | 371.6 | 243.5 | 219 | 304.9 | 271.1 | 232.5 | 238.5 | 219.9 | 295.3 |
| 347 | 98.2 | 3633.1 | 2744.6 | 2838.4 | 3198.4 | 4569.8 | 1600.1 | 1448.8 | 3315.8 |
| 348 | 78.5 | 6124.8 | 6613.5 | 4006.5 | 4485.3 | 7289.4 | 3180.7 | 2561.5 | 5272.3 |
| 349 | 582.7 | 260 | 240.8 | 164.6 | 205.7 | 203.8 | 225.1 | 195.3 | 203.1 |
| 350 | 355.9 | 281.6 | 330.4 | 285.5 | 454.2 | 255 | 105.2 | 161.7 | 183.4 |
| 351 | 732.9 | 348.3 | 47.4 | 261.9 | 443.7 | 347.8 | 457.3 | 73.6 | 169.9 |
| 352 | 162.3 | 297 | 339.9 | 197.7 | 488.4 | 390.6 | 178.5 | 201.7 | 300.5 |
| 353 | 186.4 | 405.1 | 211.5 | 354.7 | 282.6 | 265.3 | 148.4 | 203 | 378.9 |

3b

| SEQ ID NO: | 820TT | 821TT | 822TT | 823TT | 824TT | 825TT | 827TT | 828TT | 829TT |
|---|---|---|---|---|---|---|---|---|---|
| 354 | 101.8 | 32.1 | 469 | 263.8 | 1085.8 | 22 | 126.1 | 35 | 1609.8 |
| 355 | 50.1 | 45.1 | 504.2 | 78.3 | 2521.5 | 71 | 173 | 79.8 | 1017.3 |
| 356 | 697.4 | 939.2 | 1065.9 | 788.9 | 148.7 | 218.1 | 645.8 | 867.3 | 539.6 |
| 357 | 470.4 | 512.9 | 724.1 | 516.5 | 395.9 | 181 | 909.4 | 596 | 552.3 |
| 358 | 572.8 | 424.1 | 483.2 | 901.7 | 829.4 | 659.8 | 1454.2 | 582.7 | 1006.9 |
| 359 | 347.5 | 382.6 | 400.4 | 351.2 | 192.3 | 373.5 | 325.1 | 300.2 | 434.7 |
| 362 | 518.5 | 320.1 | 492.7 | 704.3 | 136.5 | 151.1 | 355.4 | 543.3 | 388.6 |
| 360 | 319.8 | 228.6 | 329.3 | 403.7 | 774.2 | 179.6 | 366.3 | 240.3 | 358.5 |
| 361 | 480.9 | 1229.9 | 857.7 | 620.4 | 288.1 | 256 | 1180.4 | 566.6 | 668.1 |
| 363 | 418.7 | 427.1 | 382.7 | 545 | 359 | 1019.2 | 338.9 | 487.8 | 695 |
| 1 | 693.8 | 684 | 807.9 | 656.3 | 335.6 | 360.3 | 549.1 | 242.6 | 662.7 |
| 2 | 1188.2 | 4262.8 | 2973.5 | 2345.3 | 485.6 | 836.8 | 2548.2 | 5155 | 2907.2 |
| 7 | 285 | 570.3 | 706.5 | 933.7 | 231.3 | 398 | 465.3 | 243.7 | 598.5 |
| 8 | 772 | 941.1 | 585.4 | 1410 | 767.6 | 899.7 | 976.4 | 715.3 | 1364.2 |
| 9 | 6790.1 | 6640.5 | 7386.3 | 3225.8 | 5435.7 | 126.3 | 4807.5 | 1611.9 | 3114.7 |
| 10 | 127.8 | 73.6 | 88.9 | 165.8 | 1940.4 | 149.4 | 211.5 | 105.7 | 161.7 |
| 11 | 219.2 | 282.4 | 325.1 | 111.4 | 558 | 78.5 | 557.8 | 260.1 | 213.2 |
| 13 | 97.3 | 102.3 | 156.9 | 158.8 | 964.8 | 160.6 | 157.6 | 106.2 | 231.8 |
| 14 | 280.4 | 281.5 | 511.4 | 335.9 | 1351.5 | 111.1 | 708.2 | 213 | 543.4 |
| 15 | 407.6 | 174.9 | 613.5 | 2405.8 | 5398.8 | 42.5 | 1013.9 | 261.5 | 818.7 |
| 16 | 996 | 867.4 | 1065.2 | 1313.3 | 639.8 | 600.3 | 893.2 | 655.3 | 1176.4 |
| 17 | 905 | 1286.8 | 1004.5 | 1341.4 | 3130.7 | 458.2 | 571.6 | 1357.1 | 567.7 |
| 19 | 176.2 | 160.5 | 2137 | 215.5 | 362.5 | 258.3 | 1756.9 | 171.6 | 407.1 |
| 20 | 2.8 | 1.1 | 5.6 | 55.2 | 3077.9 | 1.1 | 341.3 | 1.2 | 120.3 |
| 21 | 198.4 | 8.3 | 87.4 | 53.3 | 32.8 | 37.4 | 1005.1 | 37.9 | 87.5 |
| 22 | 6631.1 | 1931.6 | 3566.8 | 5632 | 5189.5 | 1534.9 | 4150.7 | 7366.1 | 5102.6 |
| 23 | 182.8 | 21.2 | 532.3 | 340.7 | 1711.8 | 1.1 | 1721.4 | 40.1 | 764.8 |
| 24 | 77.8 | 33.2 | 358.6 | 267.6 | 422.3 | 74.6 | 370.7 | 37.1 | 206.7 |
| 26 | 295.9 | 149.3 | 5582.1 | 822 | 2610.5 | 73.9 | 6672.8 | 337.8 | 2827.2 |
| 27 | 919.8 | 264.6 | 443.1 | 611.7 | 683.8 | 209.2 | 1842.7 | 221 | 356.4 |
| 29 | 70.7 | 70.7 | 74.8 | 144.2 | 604.4 | 984.4 | 261 | 138.9 | 191.1 |
| 30 | 121.9 | 25.4 | 650.1 | 375.7 | 4468.9 | 46.5 | 561.6 | 45.4 | 242.8 |
| 31 | 5697.5 | 123.5 | 224.1 | 147.9 | 1115.1 | 83.1 | 610.3 | 182 | 319.6 |
| 33 | 468.4 | 153 | 716.1 | 13385.7 | 6334.6 | 140.7 | 1847 | 224.8 | 1175.1 |
| 34 | 1720.2 | 2051.3 | 3037.5 | 1584.2 | 336.1 | 3724.7 | 2119 | 5586.2 | 2611 |
| 35 | 244.1 | 41.7 | 84.2 | 62.7 | 824.1 | 52.2 | 178.2 | 17.4 | 1328.5 |
| 36 | 119.6 | 118 | 169.3 | 230.6 | 1819.9 | 929.8 | 683 | 492.9 | 214.8 |
| 37 | 155.7 | 184.2 | 228.5 | 240.8 | 319.5 | 467.7 | 184.9 | 199.9 | 387.7 |
| 38 | 585.2 | 232.7 | 573.6 | 6068.7 | 4340.6 | 74.3 | 1214.6 | 360.9 | 671.8 |
| 40 | 591.8 | 535.9 | 446.1 | 487.3 | 508.4 | 640.3 | 459 | 382.6 | 707.6 |
| 41 | 125.3 | 451.1 | 286.6 | 226.1 | 178.8 | 297.4 | 551.6 | 110.9 | 268.1 |
| 42 | 208.6 | 409.7 | 329.7 | 258.1 | 211.2 | 288.8 | 404.4 | 466.9 | 264.3 |

| | | | | | | | | | |
|---|---|---|---|---|---|---|---|---|---|
| 43 | 1026.6 | 218.9 | 326.6 | 574.3 | 230.1 | 671.1 | 309 | 239.3 | 385 |
| 44 | 615.3 | 369.3 | 535.2 | 505 | 392.7 | 412.7 | 711.3 | 532.7 | 637 |
| 45 | 520.7 | 482.1 | 383.7 | 299.9 | 78.6 | 389.4 | 649.9 | 316.5 | 478.7 |
| 46 | 320.2 | 300.6 | 305.2 | 401.7 | 263.7 | 512.6 | 527.8 | 382.7 | 432.7 |
| 47 | 139.1 | 150.5 | 229.5 | 149.7 | 531.9 | 197.1 | 197.3 | 127 | 210.4 |
| 48 | 1.3 | 21.1 | 476.7 | 92.9 | 168.2 | 12.9 | 551.2 | 39.1 | 389.5 |
| 49 | 166.1 | 218.5 | 298.7 | 280.5 | 457.8 | 255.1 | 900.8 | 192.7 | 389.9 |
| 50 | 347.9 | 99.6 | 314.6 | 484.2 | 1930.4 | 50 | 732 | 80.5 | 471.8 |
| 51 | 242.8 | 1.5 | 41.1 | 67.7 | 580.8 | 328.7 | 355.4 | 25.4 | 109.3 |
| 52 | 192.8 | 248.6 | 243.7 | 205.8 | 370.9 | 381.6 | 276.2 | 205.6 | 177.2 |
| 53 | 96.2 | 87.9 | 70.9 | 47.3 | 43.3 | 73.7 | 337.6 | 95.3 | 151.9 |
| 54 | 407.4 | 1349.6 | 375.9 | 248.1 | 781 | 299.7 | 1508.8 | 490.7 | 389.2 |
| 55 | 121.8 | 106.2 | 97.6 | 87.5 | 86 | 144.3 | 103.2 | 108.1 | 117.5 |
| 56 | 45.3 | 148.5 | 66.5 | 67 | 89.3 | 49.7 | 49.3 | 42.5 | 86.4 |
| 57 | 676 | 281.8 | 885.5 | 948.4 | 804.4 | 353.9 | 504.6 | 82.9 | 3332.6 |
| 58 | 778.6 | 312.3 | 1214.5 | 1060.8 | 1872 | 293.1 | 691.4 | 84.7 | 3886.7 |
| 59 | 1030.8 | 2434.1 | 2195.8 | 1055.8 | 320 | 324.2 | 1155.1 | 1409.3 | 875.3 |
| 60 | 135.3 | 164.3 | 164.2 | 188.6 | 621.3 | 165.8 | 256 | 106.3 | 154.4 |
| 61 | 162.8 | 141.6 | 158.3 | 159.3 | 275.5 | 228.7 | 281.2 | 168.6 | 177.2 |
| 62 | 4949.9 | 24.3 | 607 | 1227.9 | 429.7 | 1067.9 | 763.2 | 300.6 | 2120.4 |
| 63 | 484.5 | 316.4 | 328.9 | 307.7 | 341.1 | 48.2 | 473.5 | 163.4 | 154.3 |
| 64 | 204.6 | 186.8 | 246.6 | 401.8 | 70.3 | 580.2 | 1442.7 | 100.3 | 242.1 |
| 65 | 65.5 | 179.7 | 174.9 | 187.7 | 722.1 | 50 | 248.4 | 72.4 | 115.2 |
| 66 | 773.7 | 1116.3 | 780.2 | 512.2 | 301 | 358.5 | 869.5 | 898.9 | 607.4 |
| 67 | 28999.8 | 29358.2 | 32402.2 | 37818.6 | 3337.4 | 25748.4 | 22954.2 | 36641 | 37486.1 |
| 68 | 416.7 | 3006.9 | 3943.7 | 3062.4 | 153 | 2880.9 | 1180.9 | 1284.8 | 2957.1 |
| 69 | 1079.4 | 7132.7 | 8048.4 | 4826.8 | 649.2 | 8495.4 | 5023.1 | 8078.4 | 7915.3 |
| 70 | 6077.8 | 1872.9 | 984 | 682.7 | 249.3 | 3261.4 | 4520.3 | 1401.4 | 911.7 |
| 71 | 132.3 | 112.4 | 422.9 | 210.1 | 2216.8 | 82.9 | 814.1 | 97.5 | 287.6 |
| 72 | 490.8 | 1451.8 | 1261.7 | 235.1 | 471.1 | 74.1 | 306.4 | 974.8 | 1198.6 |
| 73 | 348.5 | 369.6 | 410.2 | 281.9 | 216.9 | 255.6 | 357.1 | 221.4 | 359.5 |
| 75 | 419.5 | 82.9 | 224.3 | 363.6 | 248.7 | 233.6 | 379.3 | 555 | 287.7 |
| 76 | 11.9 | 119.6 | 100.8 | 434.7 | 2481.2 | 1185 | 1252.2 | 685.6 | 485.4 |
| 77 | 4580.1 | 3450.4 | 5301.7 | 6090.7 | 261.8 | 3157.9 | 2146 | 4545.6 | 3500.3 |
| 78 | 1117.1 | 545.1 | 716.9 | 909.9 | 254.8 | 255.9 | 868 | 1420.7 | 711.9 |
| 79 | 530 | 164.2 | 183.5 | 180.9 | 240.5 | 155.8 | 185.8 | 142.8 | 115.9 |
| 80 | 603.5 | 1468.6 | 828.2 | 186.8 | 153.5 | 1495.3 | 273.8 | 761.5 | 324.2 |
| 81 | 1295.1 | 100.2 | 158.7 | 133.6 | 628.6 | 130 | 314.3 | 106.5 | 383.7 |
| 82 | 337 | 330.3 | 245.9 | 228.3 | 61.7 | 460.2 | 141 | 303.9 | 207.5 |
| 84 | 106.4 | 568.8 | 287.1 | 105.9 | 114.1 | 486.2 | 497.7 | 114.6 | 176.9 |
| 85 | 172.3 | 125.3 | 120 | 175.2 | 169 | 193.1 | 218.4 | 131.9 | 194 |
| 86 | 87 | 87.2 | 53.6 | 86.3 | 89.1 | 131.6 | 85 | 639.6 | 137 |
| 87 | 214.5 | 168.9 | 252.9 | 285.8 | 1403.4 | 202.4 | 230.4 | 184.8 | 289.6 |
| 88 | 1660.9 | 2332 | 6323.6 | 5821 | 275.2 | 1272.1 | 4080.3 | 7866.9 | 5791.5 |
| 89 | 206 | 177.6 | 198 | 327.1 | 1314.5 | 479.1 | 253.4 | 182.8 | 368.1 |
| 90 | 115.4 | 102.5 | 134.4 | 235.2 | 2070.3 | 140.2 | 141 | 111.9 | 226.2 |
| 91 | 502.7 | 105.1 | 1042 | 284.8 | 1947.8 | 244.5 | 511.1 | 18.4 | 4872 |
| 92 | 6425 | 5848 | 4350.4 | 2893.7 | 766.3 | 1143 | 8256.4 | 4985.1 | 2390.7 |
| 93 | 205.6 | 230.3 | 307.5 | 325 | 218.5 | 186.3 | 253.4 | 252 | 154.8 |
| 94 | 357.1 | 1091.1 | 1411.5 | 311.1 | 158.3 | 46.1 | 509.8 | 632.4 | 557.7 |
| 95 | 241.3 | 283.6 | 505 | 576.3 | 62.8 | 172.8 | 115.5 | 216.1 | 532 |
| 96 | 280.5 | 181.9 | 147 | 142.4 | 142.7 | 170.7 | 216.5 | 116.6 | 156.5 |
| 97 | 466.6 | 344 | 322 | 221.4 | 70.5 | 218.7 | 225.5 | 294.9 | 178.2 |
| 98 | 23.3 | 32.5 | 29.3 | 22.1 | 31.8 | 317.2 | 35.8 | 22 | 26.8 |
| 99 | 200.6 | 212.8 | 216.1 | 258.2 | 243.6 | 366.3 | 323 | 184.2 | 327.9 |
| 100 | 1421.1 | 302.2 | 3029.1 | 5780.9 | 673.2 | 1623.5 | 2932.3 | 3304.8 | 11319.8 |
| 101 | 272 | 218 | 267.6 | 467.2 | 55.2 | 386.1 | 265.8 | 234.8 | 421.9 |
| 102 | 152.6 | 160.8 | 134.1 | 185.4 | 141.5 | 525.5 | 8297.9 | 131.6 | 193.5 |

| | | | | | | | | | |
|---|---|---|---|---|---|---|---|---|---|
| 103 | 330.3 | 366.8 | 262.3 | 438.6 | 370.5 | 687.6 | 328.6 | 308.6 | 379.9 |
| 104 | 678.2 | 302.2 | 864.8 | 623.8 | 62.2 | 907.1 | 333.9 | 1068.4 | 672.3 |
| 105 | 495.9 | 329.5 | 905.2 | 487.5 | 77.9 | 996.4 | 425 | 1136.1 | 616.5 |
| 106 | 612.3 | 277.5 | 775 | 557.3 | 34.6 | 814.9 | 280 | 957.1 | 583.4 |
| 107 | 308.9 | 188.7 | 250.9 | 591.6 | 1517.4 | 241.8 | 305 | 161.8 | 1102.6 |
| 108 | 388.5 | 475.8 | 557.1 | 574.6 | 533.2 | 365.7 | 509.2 | 634 | 321.6 |
| 109 | 47.9 | 211.4 | 334.1 | 275.6 | 373.3 | 113.2 | 1573 | 359.4 | 1203.1 |
| 110 | 1.1 | 367.7 | 820.1 | 614 | 750.8 | 2.3 | 2128.4 | 450.7 | 2481.1 |
| 111 | 105.4 | 111.5 | 1115.1 | 1085.4 | 31.9 | 1134.1 | 919.7 | 2507.5 | 1720.3 |
| 112 | 62.4 | 132.4 | 118.7 | 104.6 | 124.9 | 88.9 | 1090.7 | 12.5 | 512.4 |
| 113 | 149.1 | 66.5 | 145.6 | 225.3 | 1395.8 | 112.7 | 316.5 | 230.1 | 145.4 |
| 114 | 929.6 | 594 | 672 | 804.4 | 155.5 | 238.7 | 339.5 | 618.6 | 378.6 |
| 115 | 473.6 | 276.3 | 355.8 | 431 | 779.1 | 286.2 | 826.4 | 464.8 | 562.1 |
| 116 | 228.8 | 183.6 | 205.8 | 214.2 | 113.4 | 286 | 233.8 | 157.3 | 281.6 |
| 117 | 1259.3 | 645.9 | 2714.7 | 1854.7 | 284.3 | 889.6 | 776.5 | 5906.8 | 1981.4 |
| 118 | 165 | 99.4 | 148.2 | 154 | 104.5 | 952.7 | 926.2 | 63.2 | 218.8 |
| 119 | 74.1 | 55.5 | 95.2 | 192.8 | 129.2 | 122.6 | 138.9 | 69 | 252.5 |
| 120 | 154.7 | 88.6 | 159.7 | 229.3 | 533 | 78.8 | 126.8 | 101.7 | 185.5 |
| 121 | 470.8 | 523.4 | 1077.7 | 284.1 | 270.2 | 79.9 | 1935.6 | 440.9 | 773.2 |
| 122 | 139.1 | 32 | 51.8 | 203.5 | 559 | 116.7 | 160.9 | 98.4 | 167.8 |
| 123 | 127.6 | 127.8 | 146.6 | 223 | 86.8 | 290.5 | 147 | 161 | 152.7 |
| 124 | 323.6 | 121.6 | 123 | 147.7 | 138.1 | 317.3 | 2203.4 | 125.6 | 200.2 |
| 125 | 909.9 | 886.8 | 770.6 | 1100 | 653.6 | 1873.2 | 823.8 | 980.3 | 1308.7 |
| 126 | 865.4 | 1870.3 | 1455.2 | 950.6 | 132.1 | 353.6 | 443.8 | 1299.3 | 756.7 |
| 127 | 146.8 | 89.1 | 116.4 | 108.3 | 102.8 | 176.3 | 89.7 | 112.1 | 164.4 |
| 128 | 199.2 | 243.1 | 243.5 | 292.8 | 184.9 | 435.5 | 169371.4 | 255.3 | 380.5 |
| 129 | 201 | 129.2 | 160.1 | 346.8 | 964 | 143.6 | 229.7 | 180.9 | 349.9 |
| 130 | 436.5 | 199.9 | 143.8 | 114.3 | 61.5 | 134.4 | 300.8 | 73.2 | 120.4 |
| 131 | 133.9 | 92.3 | 74.4 | 88.5 | 83.4 | 229.7 | 410.5 | 60.2 | 101.7 |
| 132 | 170.5 | 183.6 | 217.5 | 279 | 177.9 | 291.2 | 172.6 | 176.3 | 285.9 |
| 133 | 76.5 | 158.5 | 71.8 | 115.2 | 44.6 | 280.4 | 133.9 | 76.6 | 58.9 |
| 134 | 177.7 | 117.6 | 92.6 | 87.1 | 47.4 | 135.3 | 194.3 | 131.1 | 125.4 |
| 135 | 668.9 | 332.2 | 491.2 | 604 | 177.1 | 258 | 426.2 | 374.2 | 376.3 |
| 138 | 89.5 | 107.6 | 139.2 | 104.1 | 252.7 | 204.2 | 408.1 | 146.3 | 173.8 |
| 139 | 94.5 | 61.7 | 100.1 | 154 | 66.7 | 109.2 | 217.6 | 69.7 | 69.7 |
| 140 | 488.1 | 5040.7 | 5478 | 884.2 | 19.9 | 864.2 | 414.1 | 2686.3 | 2320.6 |
| 141 | 1820.2 | 2069.9 | 1896.4 | 1208 | 195.6 | 666 | 1637.7 | 1312.8 | 1389.1 |
| 144 | 389.9 | 2941 | 519.1 | 509.5 | 290.3 | 566.4 | 3501.1 | 2333.5 | 594.6 |
| 145 | 176.7 | 233.5 | 205.8 | 273.5 | 163.7 | 364.5 | 169 | 239.5 | 307.6 |
| 146 | 180.4 | 184.7 | 182 | 241.3 | 165.7 | 371.9 | 208.9 | 218.9 | 287.1 |
| 147 | 60.6 | 67.5 | 36.5 | 35.9 | 45 | 33 | 48.8 | 39.7 | 84.2 |
| 148 | 218 | 311.7 | 680.6 | 305.9 | 613.6 | 301.8 | 333.6 | 170.7 | 855.4 |
| 149 | 389.2 | 615.3 | 612.9 | 411.2 | 46.9 | 38.6 | 377.1 | 483.8 | 290.4 |
| 150 | 292.4 | 123.3 | 170.9 | 249.3 | 169.9 | 382.6 | 1326 | 246.2 | 272.1 |
| 151 | 172.2 | 109.5 | 207.1 | 176.5 | 173 | 197.7 | 201.3 | 53.4 | 217.8 |
| 153 | 100.2 | 143.8 | 139 | 113.1 | 97.6 | 119 | 115.6 | 97.7 | 102.5 |
| 154 | 965.5 | 130.7 | 240.3 | 422.3 | 239.6 | 103.1 | 344.9 | 332 | 536.6 |
| 155 | 150.4 | 145.1 | 158.1 | 122 | 114.6 | 231.1 | 116.7 | 136.3 | 190.3 |
| 156 | 72.3 | 57.3 | 76.9 | 97.8 | 60.5 | 106.5 | 85.4 | 49.1 | 91.7 |
| 157 | 92.1 | 101.4 | 129.5 | 97.6 | 197.6 | 183.4 | 266.9 | 102.3 | 169 |
| 158 | 105.7 | 18.1 | 95.3 | 31 | 1.1 | 1.5 | 124 | 10.3 | 17.7 |
| 159 | 3118.9 | 2438.5 | 2254 | 4357.3 | 169.7 | 2658.7 | 1376.2 | 3763.9 | 2939.4 |
| 161 | 5.6 | 102 | 74.9 | 43.7 | 13.3 | 206.3 | 5.9 | 19.5 | 43 |
| 162 | 86.4 | 52.8 | 243.7 | 212.1 | 2021.6 | 52.6 | 430 | 59.9 | 191.7 |
| 164 | 75.9 | 635.3 | 125.4 | 46.6 | 62.7 | 90.5 | 4939.7 | 520.4 | 106.5 |
| 166 | 8478.4 | 8844.4 | 7517.8 | 9251.3 | 668.6 | 5048 | 2532 | 11114.9 | 5155.6 |
| 167 | 944.4 | 2088.2 | 2168.9 | 3078.3 | 35.4 | 2685.3 | 569.9 | 438.5 | 2149.5 |
| 168 | 981.2 | 741.9 | 536.9 | 381.5 | 101.8 | 263.9 | 1589.1 | 1090 | 1157.8 |

| | | | | | | | | | |
|---|---|---|---|---|---|---|---|---|---|
| 169 | 998.2 | 828.4 | 1558.6 | 3346.8 | 4596.6 | 373.2 | 2769.4 | 732.9 | 18910.1 |
| 170 | 1690.4 | 2036.1 | 1458.2 | 1172.6 | 1929.3 | 472.1 | 1536.6 | 1074.1 | 748.2 |
| 171 | 35.6 | 1.2 | 1357.1 | 179.9 | 2986.6 | 1.2 | 1399.7 | 39.7 | 3790.7 |
| 172 | 660.5 | 696.5 | 574.5 | 822 | 549.6 | 679.2 | 517.6 | 539 | 873.1 |
| 173 | 172.3 | 120.5 | 182.9 | 172.3 | 441.6 | 91 | 875.5 | 186.4 | 445.7 |
| 176 | 1.1 | 32.1 | 22.8 | 1.1 | 1.1 | 269.9 | 3233.2 | 1.1 | 160.9 |
| 177 | 675.5 | 641.9 | 541.6 | 1175.8 | 196.5 | 1163.3 | 437.1 | 345.5 | 599.4 |
| 178 | 144.2 | 121.8 | 72.4 | 360.4 | 1481.8 | 1025 | 2178.6 | 88.7 | 313.7 |
| 179 | 52.1 | 87.1 | 93.5 | 143.3 | 81.8 | 124.1 | 75.4 | 43.4 | 153.8 |
| 180 | 95.7 | 66.1 | 137.1 | 160.5 | 253.7 | 190.5 | 496.2 | 69.1 | 199.3 |
| 181 | 8377.6 | 9185.5 | 10162.2 | 11258.5 | 1081.7 | 11429.6 | 5621.9 | 12118.4 | 10006.8 |
| 182 | 458.5 | 488.6 | 593.1 | 336.2 | 742.2 | 148.2 | 383.4 | 287.6 | 393.9 |
| 183 | 41.7 | 8.1 | 711.7 | 110.5 | 624.4 | 1.2 | 1507 | 41.7 | 551.1 |
| 184 | 241.8 | 165.4 | 281.2 | 458.9 | 346.3 | 189.5 | 244.1 | 175.4 | 339.3 |
| 185 | 1648.2 | 527.4 | 1463 | 1633.2 | 3854.7 | 591.4 | 1805.1 | 1354.3 | 962 |
| 186 | 852.3 | 370.9 | 554.5 | 405.8 | 293.7 | 714.5 | 452.3 | 164.8 | 319.5 |
| 187 | 81.4 | 76.3 | 149.6 | 122.4 | 192.9 | 83.5 | 4783 | 75.8 | 136.5 |
| 188 | 325.5 | 64.9 | 281 | 153.1 | 548.5 | 72.7 | 239.9 | 148.5 | 260.2 |
| 189 | 179.1 | 36.6 | 199.4 | 99.5 | 226.7 | 74.1 | 111.4 | 100 | 151.7 |
| 190 | 65.2 | 56.7 | 57.5 | 206 | 147 | 170.8 | 473.9 | 107.2 | 158.5 |
| 191 | 1282.4 | 180.6 | 706.5 | 991.9 | 1859.6 | 193.4 | 671.8 | 1181.2 | 884.9 |
| 192 | 133.1 | 59.7 | 97.6 | 101 | 117.7 | 186.9 | 1346.8 | 80.1 | 111.3 |
| 193 | 2817 | 1551 | 1158.4 | 1627.3 | 211 | 677.6 | 728.5 | 2190.6 | 745.3 |
| 194 | 224.1 | 133.7 | 191.1 | 211.3 | 94.6 | 57.1 | 198.5 | 79.7 | 112.4 |
| 195 | 210.1 | 378.3 | 415.7 | 457.7 | 91.8 | 391.3 | 167.4 | 250.9 | 161.2 |
| 196 | 2059.1 | 6068.9 | 8250.3 | 6100.2 | 260.4 | 9272.9 | 4421.5 | 2709.1 | 7446.4 |
| 197 | 116.3 | 74.9 | 72.5 | 69.9 | 1094.7 | 92.5 | 126.6 | 97.4 | 137.7 |
| 198 | 69.1 | 54.5 | 79.3 | 90.4 | 64.8 | 142.2 | 658.4 | 74.2 | 125.9 |
| 199 | 73.1 | 63 | 674.4 | 113.7 | 3555.6 | 87.8 | 295.6 | 125.9 | 1666.8 |
| 200 | 152.4 | 193.6 | 440 | 136.7 | 1166.9 | 233.9 | 450.3 | 193.7 | 343.6 |
| 201 | 101.2 | 31 | 560.4 | 302.8 | 1214.5 | 17.7 | 90 | 30.4 | 1612.8 |
| 203 | 703.7 | 907.4 | 1270.8 | 514 | 92.1 | 126.9 | 1145.2 | 1552.7 | 207.6 |
| 204 | 1285.1 | 377.7 | 403 | 705.2 | 88.6 | 338.5 | 595.2 | 922.9 | 476.3 |
| 205 | 2463.4 | 3054.5 | 3730.8 | 4980.4 | 361.5 | 572.3 | 3463.4 | 6141.2 | 3833.2 |
| 206 | 187.4 | 167.3 | 168.6 | 267.3 | 1097.9 | 453.7 | 228.3 | 296.2 | 3177.1 |
| 207 | 288.4 | 3037.3 | 2123.7 | 206.1 | 61.4 | 218.8 | 485.6 | 2438.3 | 1258.7 |
| 208 | 190.3 | 97.4 | 336.5 | 323 | 109.9 | 9.2 | 306.2 | 227.8 | 548.3 |
| 209 | 3259.2 | 3551.6 | 5575.6 | 2237.4 | 150.6 | 4727.5 | 1832.2 | 732.2 | 3005.4 |
| 211 | 8683.6 | 22986.2 | 20317.7 | 33405.4 | 565 | 7384.7 | 9662.3 | 29932.5 | 20413.1 |
| 212 | 1572 | 515.7 | 339.7 | 208.5 | 80.1 | 1016.3 | 1086.1 | 339.2 | 299.9 |
| 213 | 94.5 | 90 | 92.1 | 102.5 | 61.4 | 224.9 | 80.9 | 66.5 | 121.6 |
| 215 | 366.7 | 5578.5 | 4327 | 285.6 | 78.2 | 266 | 478.1 | 3071.2 | 1903.3 |
| 216 | 633.5 | 670 | 697.1 | 660.4 | 547 | 267.5 | 566.7 | 785.5 | 412.5 |
| 217 | 235.7 | 147.3 | 281.6 | 384 | 479.9 | 98.7 | 277.7 | 221.7 | 271.5 |
| 218 | 191.6 | 481.1 | 295.1 | 384 | 112.2 | 335.8 | 274 | 687.3 | 319.7 |
| 220 | 74 | 79.4 | 131.9 | 86.9 | 109.2 | 139.9 | 74.3 | 78.7 | 98.2 |
| 221 | 739.7 | 775 | 1236 | 738.2 | 1516.5 | 159.5 | 796.8 | 459.3 | 439.4 |
| 223 | 226.1 | 262.3 | 230.6 | 220.4 | 235.4 | 419.6 | 210.6 | 191.4 | 360.2 |
| 227 | 784.7 | 236.7 | 712.8 | 3864 | 4971.9 | 50.2 | 2112.1 | 483.2 | 1731 |
| 228 | 427.4 | 369.6 | 410.7 | 756.3 | 119 | 453 | 308.8 | 321.2 | 777.9 |
| 229 | 121.4 | 178.2 | 180.7 | 386.6 | 270.9 | 497.6 | 489 | 353.6 | 172.9 |
| 230 | 85 | 63.3 | 105.6 | 210.3 | 1243.7 | 40.9 | 331.4 | 106.7 | 325.5 |
| 231 | 337.9 | 417.5 | 650 | 305 | 129.2 | 163.1 | 486.4 | 499 | 193.8 |
| 232 | 213 | 116.6 | 3960.5 | 604.6 | 1673.4 | 54.4 | 4508.4 | 185.8 | 2113.5 |
| 233 | 2564 | 1822.6 | 6196.9 | 2781.4 | 3667.3 | 1118.9 | 3084 | 6379.7 | 4116.8 |
| 235 | 137.6 | 63.4 | 580.8 | 361.2 | 1679.5 | 92.8 | 833 | 86.2 | 2441.7 |
| 236 | 160.2 | 120 | 182.2 | 264.3 | 417.6 | 254.8 | 288.4 | 125.5 | 310.6 |
| 237 | 2392.5 | 1737.2 | 1778.8 | 1851.2 | 3102.3 | 638.8 | 2558.1 | 2048.7 | 1204.1 |

| | | | | | | | | | |
|---|---|---|---|---|---|---|---|---|---|
| 238 | 934.8 | 1575.1 | 1087.9 | 1078.8 | 215.5 | 179.1 | 1369.6 | 2083.3 | 807.8 |
| 239 | 256.3 | 59.9 | 259.2 | 442 | 208.4 | 101.6 | 275.9 | 58.6 | 485 |
| 240 | 316.9 | 27.3 | 330.2 | 381.2 | 683 | 52.8 | 381.6 | 41.4 | 575.1 |
| 241 | 602.7 | 36.7 | 562.8 | 985.9 | 886.1 | 83.4 | 734.2 | 126.7 | 1214 |
| 242 | 2714.7 | 325 | 2278 | 12129 | 22000.8 | 874.8 | 2544.9 | 845.1 | 6760 |
| 243 | 477.1 | 168.4 | 373 | 453.4 | 7356.1 | 182.8 | 441.2 | 223 | 259.3 |
| 244 | 137.2 | 198.3 | 172.5 | 137.8 | 308.4 | 111.1 | 215.7 | 213.9 | 147.9 |
| 246 | 262 | 306.4 | 301.9 | 426.2 | 407.7 | 346.6 | 609.8 | 874.2 | 145.9 |
| 247 | 117.5 | 142.6 | 77 | 156.9 | 119.1 | 289.1 | 120.2 | 120.9 | 213.4 |
| 248 | 216.4 | 124.1 | 74.2 | 115.8 | 92.4 | 107.5 | 317.2 | 126.7 | 106.4 |
| 249 | 180 | 341.2 | 320.1 | 176.7 | 185.5 | 885.1 | 399.6 | 131.7 | 791.8 |
| 251 | 63.8 | 107.9 | 106.3 | 84.5 | 742.7 | 44.5 | 117.5 | 94.5 | 111.7 |
| 253 | 190.7 | 244.8 | 204.4 | 375.3 | 374.5 | 218.4 | 376.5 | 486.5 | 269.6 |
| 254 | 145.4 | 1957.1 | 1159.5 | 243.2 | 123.7 | 228.2 | 1365 | 142.3 | 312.9 |
| 255 | 170.9 | 82.9 | 51.9 | 379.1 | 1787.5 | 1107.5 | 2481.4 | 91.2 | 318.4 |
| 256 | 517.8 | 132.4 | 995.4 | 353.1 | 1340.6 | 331.8 | 499.8 | 141.8 | 3605.8 |
| 257 | 124.3 | 174.8 | 165.9 | 97.2 | 172.3 | 54 | 209.3 | 115.4 | 173.8 |
| 258 | 24 | 1.2 | 253.4 | 53.4 | 345.6 | 104.4 | 131.7 | 3.9 | 456.5 |
| 259 | 692.2 | 65.2 | 512.5 | 236.2 | 422.2 | 1.2 | 669.1 | 79.7 | 638.4 |
| 260 | 153.5 | 168.4 | 123.3 | 295.4 | 151.6 | 561.5 | 224.9 | 163.9 | 266.2 |
| 261 | 197.8 | 221.1 | 313.1 | 301.5 | 77.8 | 119.5 | 496.8 | 1094.7 | 301.6 |
| 263 | 698.9 | 553.8 | 407.2 | 370.6 | 1231.9 | 141.3 | 387.3 | 692.4 | 333.4 |
| 263 | 135.5 | 78.4 | 109.7 | 225.9 | 1211 | 201.2 | 434.5 | 96.2 | 404.4 |
| 265 | 362.9 | 618.8 | 519.4 | 342.3 | 102 | 258.8 | 178.2 | 477.2 | 588.4 |
| 266 | 246.8 | 201.9 | 307.4 | 607.3 | 257.6 | 820.3 | 300.7 | 537.1 | 367 |
| 256 | 115.8 | 100.6 | 138.3 | 134.8 | 325.1 | 134.2 | 161.2 | 75.9 | 165.2 |
| 268 | 209.2 | 366.9 | 653.7 | 922.5 | 10 | 480.6 | 333.1 | 299.5 | 775.9 |
| 269 | 40.7 | 48.5 | 42.3 | 72.2 | 126 | 90.5 | 30 | 60.5 | 91.4 |
| 270 | 466.8 | 148.2 | 169.3 | 215.5 | 180.7 | 290.2 | 237 | 153.1 | 262.6 |
| 271 | 340.4 | 250.6 | 292 | 363.1 | 191.9 | 147.4 | 231.1 | 338.8 | 278.4 |
| 272 | 419.7 | 600.7 | 550.3 | 549.6 | 590.7 | 925.3 | 506 | 506.9 | 675.1 |
| 273 | 1329.6 | 4064.8 | 5723.5 | 4200.8 | 53.9 | 141.9 | 2361.5 | 2682.8 | 4782.9 |
| 274 | 572.9 | 159.6 | 459.6 | 777.2 | 2112.5 | 590 | 1292.6 | 264.9 | 670.4 |
| 275 | 400.8 | 151.3 | 339.1 | 398 | 1769 | 221.5 | 415.8 | 329.2 | 264.8 |
| 276 | 1260.7 | 304 | 1311.2 | 5788.9 | 9405.7 | 142.2 | 3231.8 | 599.6 | 2504.9 |
| 277 | 175.4 | 164.3 | 192.2 | 231.9 | 150.5 | 254.7 | 152.2 | 197.2 | 281.6 |
| 278 | 1103 | 87.3 | 544.7 | 209.6 | 729.1 | 38.2 | 801.4 | 62.2 | 875.4 |
| 279 | 1853 | 3010.3 | 1676.6 | 3236.4 | 131.7 | 425.7 | 2072.3 | 2745.7 | 1987 |
| 280 | 2108.7 | 4444.5 | 2655.3 | 3526 | 338.9 | 391.3 | 2886.5 | 4540.8 | 2685.8 |
| 281 | 18.4 | 25 | 53.6 | 118.3 | 67.5 | 103.2 | 129.1 | 36 | 49.1 |
| 282 | 208.4 | 189.9 | 229.3 | 225.9 | 218 | 274.6 | 281.5 | 269.8 | 574.2 |
| 283 | 226.2 | 239 | 236.5 | 269.1 | 179.9 | 647.8 | 210.9 | 202.5 | 338.1 |
| 284 | 76.5 | 83 | 85.5 | 84.8 | 61.1 | 154.1 | 83.4 | 51.9 | 87.9 |
| 285 | 83.4 | 81.8 | 98.2 | 109.9 | 70.2 | 153.3 | 87 | 104.4 | 126.6 |
| 286 | 2043.9 | 638.5 | 1891.2 | 121.4 | 602.8 | 343.8 | 417.5 | 688.5 | 824.4 |
| 287 | 102.2 | 122.8 | 119.2 | 106.4 | 113.9 | 133.5 | 95.5 | 144.2 | 149.6 |
| 288 | 142 | 147.4 | 138.8 | 152.9 | 154.3 | 269 | 139.9 | 140.5 | 203.7 |
| 289 | 673.8 | 1.2 | 569.8 | 199.4 | 1260.8 | 269.4 | 831.4 | 65.9 | 2672.1 |
| 291 | 209.6 | 44 | 112.8 | 181.3 | 341.7 | 44 | 78.9 | 99.5 | 126.6 |
| 292 | 62.9 | 40.3 | 181.8 | 49.2 | 103.4 | 47.8 | 786.4 | 36.9 | 412.2 |
| 293 | 489.3 | 121.1 | 95.1 | 72.6 | 52.1 | 99.7 | 409.7 | 50.8 | 113 |
| 295 | 527.5 | 128.5 | 168 | 466.6 | 768.9 | 354.3 | 354.7 | 222.9 | 256.1 |
| 296 | 773.6 | 924.1 | 1329.9 | 843.8 | 1063 | 956.8 | 1070.2 | 873.2 | 688.9 |
| 297 | 52 | 51.9 | 85.2 | 60.1 | 2284.1 | 95.4 | 70.8 | 52.2 | 96.9 |
| 298 | 295.2 | 198.2 | 300.3 | 295.1 | 502.3 | 161.6 | 292.8 | 372.6 | 290 |
| 299 | 1170.6 | 1365.1 | 561.9 | 926.9 | 160.3 | 431.4 | 1139.9 | 604 | 851.1 |
| 300 | 79.1 | 638.7 | 458 | 158.4 | 272.1 | 104.6 | 617.9 | 383.6 | 205.3 |
| 301 | 1366.1 | 435.2 | 1418.5 | 798.3 | 317.5 | 423.3 | 664.2 | 750.6 | 2119.8 |

| | | | | | | | | | |
|---|---|---|---|---|---|---|---|---|---|
| 302 | 14.8 | 21.6 | 21.1 | 13.6 | 121.5 | 39.5 | 24.7 | 9.5 | 34.9 |
| 303 | 1272.6 | 1149.6 | 761.8 | 834.5 | 159.9 | 477.7 | 449.7 | 1072.6 | 432.1 |
| 304 | 1151.3 | 128.6 | 230.5 | 335.2 | 454.3 | 186.6 | 1221.7 | 235 | 379.7 |
| 305 | 151.1 | 238.9 | 140.3 | 950.2 | 110.9 | 1077 | 1043.9 | 166.7 | 211.5 |
| 306 | 136.4 | 144.5 | 117.6 | 155.2 | 192.6 | 291.2 | 118.6 | 150.5 | 228.4 |
| 307 | 132.8 | 177.7 | 207.1 | 196.3 | 120 | 249.1 | 112.4 | 122.8 | 281.5 |
| 308 | 2569.4 | 1758.7 | 2241.2 | 2859.3 | 230.6 | 2770.2 | 677.2 | 2894.9 | 1090.1 |
| 310 | 980 | 334 | 456.4 | 390 | 114.5 | 278.1 | 997.8 | 654.8 | 472.6 |
| 311 | 142.6 | 73.1 | 84.3 | 121.4 | 78.4 | 146.9 | 148.3 | 200.6 | 115.7 |
| 312 | 2560.4 | 57.2 | 426.8 | 89.2 | 18.4 | 574.8 | 506.6 | 402.8 | 956.5 |
| 313 | 209.5 | 119.1 | 121.2 | 222.7 | 91.7 | 201.4 | 478.6 | 92.9 | 117.2 |
| 314 | 158.8 | 162.7 | 249.8 | 188.5 | 309.4 | 89.2 | 547.1 | 122.9 | 576.3 |
| 315 | 702.8 | 557.9 | 900 | 546.6 | 104 | 247.6 | 529.5 | 657.3 | 404.1 |
| 316 | 250.6 | 332.6 | 259.8 | 311.9 | 69.6 | 518.6 | 460.2 | 314.7 | 248.1 |
| 317 | 150.5 | 169.2 | 177 | 177.5 | 133.8 | 233 | 171.7 | 173.6 | 261.6 |
| 318 | 40.7 | 117.2 | 213.8 | 44.5 | 47.3 | 18.6 | 57.8 | 59.8 | 9.1 |
| 319 | 309.7 | 173 | 162.4 | 217 | 97.8 | 1038.4 | 828.1 | 117.5 | 167.2 |
| 320 | 822.5 | 511 | 664.1 | 815.9 | 185 | 371.3 | 845 | 592.1 | 437.2 |
| 321 | 629.1 | 258.6 | 321.1 | 511.8 | 309.4 | 452.1 | 355 | 573.3 | 321.3 |
| 322 | 195.9 | 274 | 200.6 | 228.2 | 502.2 | 565.2 | 318 | 241 | 250.5 |
| 323 | 5483.1 | 3828.6 | 3464.5 | 1733.4 | 233.8 | 941.9 | 1329.5 | 2376 | 2289.1 |
| 324 | 224.9 | 61 | 44.8 | 85.4 | 32 | 56.6 | 246 | 103.9 | 70.2 |
| 325 | 342.8 | 379 | 339.3 | 282.9 | 230.2 | 339.8 | 313.3 | 331.3 | 331 |
| 326 | 2895.3 | 6732.3 | 6959.5 | 716.8 | 385.8 | 689.6 | 1354.3 | 3462.4 | 810.2 |
| 327 | 14 | 26.8 | 9 | 107.3 | 745.3 | 31.8 | 92.3 | 30.5 | 40.5 |
| 328 | 281.4 | 393 | 463 | 538.5 | 44.8 | 160.9 | 160.6 | 240 | 510.8 |
| 329 | 83.3 | 80 | 72.1 | 51.7 | 315.4 | 197.3 | 209.5 | 65.5 | 97.5 |
| 330 | 51.4 | 40.9 | 62.5 | 38.8 | 64.6 | 86 | 49.8 | 45.3 | 75.4 |
| 331 | 310.9 | 265.9 | 292.9 | 290.5 | 301.2 | 1151 | 377.7 | 262.6 | 363.2 |
| 333 | 770.8 | 635.6 | 1012.3 | 910.6 | 190.7 | 762.8 | 346 | 908.1 | 711.7 |
| 334 | 207.8 | 219.4 | 166 | 215.4 | 137.5 | 112.8 | 565.2 | 135.4 | 170 |
| 335 | 155.6 | 80.9 | 114.5 | 131.9 | 90.7 | 718.1 | 215.7 | 922.8 | 155.9 |
| 336 | 340.7 | 427.2 | 563.8 | 689.1 | 96.3 | 436.7 | 487.1 | 782.5 | 499 |
| 337 | 2849 | 76.8 | 200.7 | 463 | 932.4 | 112.2 | 638.2 | 474.2 | 194.5 |
| 338 | 177.2 | 183.9 | 190.4 | 175.1 | 273.4 | 59.6 | 256.4 | 142.1 | 229.6 |
| 339 | 395.4 | 525.8 | 437.1 | 581 | 225 | 445.4 | 326.7 | 362.3 | 485.5 |
| 340 | 223.5 | 251.1 | 290.8 | 318.2 | 197.1 | 253.7 | 187.5 | 285.3 | 397.3 |
| 341 | 473 | 874 | 1273.4 | 721.4 | 508.7 | 1207.3 | 566.8 | 660.8 | 584.1 |
| 343 | 469.4 | 161.5 | 78.2 | 124.9 | 366 | 1807.9 | 1281 | 53.8 | 117.8 |
| 344 | 1783.5 | 1064.1 | 1156.4 | 1751.4 | 281.3 | 986.5 | 1814.7 | 1291.3 | 1259.5 |
| 345 | 244.8 | 232.7 | 258.2 | 265.4 | 279 | 383.5 | 225.4 | 192.1 | 278.1 |
| 347 | 939.3 | 1308.9 | 2012 | 2968.2 | 324.4 | 1367.9 | 2442.7 | 1031.3 | 2634.6 |
| 348 | 1619 | 2746.3 | 3836 | 4352.3 | 628.4 | 2022.4 | 5951.2 | 4142.1 | 3535.5 |
| 349 | 208.6 | 294.7 | 249.4 | 334.4 | 413.9 | 154.9 | 222.4 | 262.8 | 343 |
| 350 | 265 | 151.9 | 221.4 | 354.3 | 1016.4 | 1.2 | 367.5 | 179 | 283.6 |
| 351 | 121.2 | 184 | 512.2 | 66.7 | 230.2 | 220.7 | 229.7 | 69.7 | 320.1 |
| 352 | 468.7 | 405.1 | 432.1 | 461.6 | 124.7 | 253.5 | 262.2 | 439 | 377.6 |
| 353 | 422.8 | 154.1 | 375.8 | 272.6 | 206.3 | 272.5 | 306.9 | 342 | 275.3 |

3c

| SEQ ID NO: | 830TT | 831TT | 832TT | 834TT | 835TT | 836TT | 837TT | 838TT | 839TT |
|---|---|---|---|---|---|---|---|---|---|
| 354 | 1607.7 | 1269.4 | 69.2 | 410.9 | 2189.2 | 23.1 | 352.6 | 432.9 | 560.6 |
| 355 | 1226.2 | 9155.4 | 818.2 | 3259.7 | 4192.9 | 60.2 | 6955.8 | 2712.6 | 387 |
| 356 | 589.5 | 420.2 | 584 | 481.7 | 485.2 | 628.1 | 781.8 | 356.3 | 432.6 |
| 357 | 639.1 | 496.4 | 606.3 | 873.1 | 453.3 | 762.3 | 556.9 | 747.8 | 483.8 |

| | | | | | | | | | |
|---|---|---|---|---|---|---|---|---|---|
| 358 | 1141.1 | 1537.2 | 2093.3 | 1332.4 | 1450.6 | 785 | 1727.4 | 1490.9 | 867.6 |
| 359 | 410.8 | 324.8 | 302.5 | 275.6 | 347.4 | 372 | 401.6 | 274.5 | 352.8 |
| 362 | 433.7 | 1188.9 | 809.4 | 714.8 | 722 | 536.8 | 1286.6 | 790.9 | 408.6 |
| 360 | 355.3 | 785.8 | 548.6 | 473.7 | 527.2 | 237.8 | 648 | 589.2 | 258.9 |
| 361 | 669.1 | 117.2 | 502.4 | 332.7 | 153.1 | 491.8 | 673.8 | 155.9 | 634.6 |
| 363 | 624.8 | 516.7 | 452.9 | 386.1 | 575.4 | 348.2 | 555.6 | 423.3 | 578.6 |
| 1 | 681.6 | 1339.4 | 926.7 | 1627.5 | 1378.3 | 825.9 | 1180.5 | 1556.8 | 483 |
| 2 | 2308.2 | 2517.8 | 2273.1 | 5347 | 1263.4 | 2813.7 | 1449.6 | 2931.3 | 2339.6 |
| 7 | 760.1 | 396.2 | 691.3 | 1209.2 | 602.6 | 682.5 | 797.8 | 924.6 | 504.6 |
| 8 | 1540.3 | 559.2 | 932.1 | 520.1 | 496.7 | 1011.1 | 563.4 | 319.8 | 942.6 |
| 9 | 2980.9 | 1665.3 | 5198.9 | 2703.9 | 1212.7 | 4689.7 | 7367.2 | 4021.7 | 2722.5 |
| 10 | 122.4 | 197.5 | 65.2 | 110.5 | 139.5 | 59.4 | 99.3 | 137.7 | 236.6 |
| 11 | 376.8 | 703.1 | 345.6 | 651.8 | 578.7 | 208.4 | 539.6 | 862.8 | 174 |
| 13 | 181.2 | 792.6 | 515 | 259.6 | 570.1 | 291.6 | 711.6 | 596.7 | 310.5 |
| 14 | 618.3 | 2196.3 | 1802 | 1423.9 | 1281.3 | 1502.7 | 2410.1 | 2087.7 | 533.5 |
| 15 | 807.1 | 1589.6 | 1001.5 | 828.3 | 1241.5 | 307.4 | 2913.4 | 4498.6 | 625.1 |
| 16 | 1484.5 | 3932 | 1622.5 | 2238.6 | 3413.6 | 1419.6 | 2274.4 | 2625.1 | 831.6 |
| 17 | 699.7 | 876.3 | 1400.3 | 1300.9 | 988.9 | 1281 | 1049.5 | 2078.4 | 694.1 |
| 19 | 482.6 | 465.6 | 2749.2 | 461.1 | 810 | 290.3 | 1716.7 | 1582.7 | 1076.2 |
| 20 | 84 | 450.8 | 263.1 | 290.5 | 360 | 1.1 | 182.4 | 324.9 | 165.7 |
| 21 | 91.3 | 3467.2 | 514.9 | 1814.1 | 3225.9 | 499.2 | 2094.7 | 1840.3 | 181.3 |
| 22 | 3156.1 | 4671.9 | 5203.4 | 6611.6 | 3435 | 2468.1 | 9279.3 | 6745.6 | 4447.3 |
| 23 | 495.3 | 1234.6 | 1083.4 | 798.1 | 1519.6 | 356.2 | 1191.8 | 2950.2 | 871.6 |
| 24 | 274.4 | 317.3 | 405.7 | 185.5 | 432.2 | 235.1 | 795.2 | 871.3 | 137.7 |
| 26 | 2752.2 | 4302.1 | 8189.5 | 1864.3 | 3917.4 | 2490.7 | 6974 | 5535.2 | 4706 |
| 27 | 424.6 | 749.9 | 1258.8 | 1611.9 | 871.3 | 505 | 1476.7 | 1146.7 | 506.1 |
| 29 | 165.7 | 162.7 | 75.4 | 90.5 | 104.2 | 94.3 | 114.4 | 146.2 | 101 |
| 30 | 312.4 | 592.5 | 409.5 | 348 | 418.1 | 136.7 | 1284.6 | 1307.6 | 408.1 |
| 31 | 331.7 | 12536.5 | 1834 | 9042.7 | 16967 | 303.8 | 5146.5 | 6843.3 | 352.9 |
| 33 | 1256.2 | 4507.9 | 1474.3 | 2328.6 | 4981.8 | 451.7 | 4542.5 | 11875.5 | 544.7 |
| 34 | 2831.9 | 492 | 3213.2 | 870 | 569.7 | 1873.2 | 2278.6 | 454.6 | 2785.1 |
| 35 | 1217 | 1751.1 | 260.4 | 933.6 | 1418.4 | 120.2 | 423.2 | 768.6 | 680.3 |
| 36 | 145.6 | 258.1 | 974.8 | 3626.3 | 913.3 | 524.5 | 893.8 | 1391.4 | 185.2 |
| 37 | 286.6 | 268.9 | 312.8 | 203.1 | 264.8 | 203 | 424.3 | 264.8 | 270.4 |
| 38 | 709.7 | 3086.6 | 1199.9 | 882.2 | 1931.9 | 384.4 | 1810.3 | 7524 | 348.4 |
| 40 | 603.3 | 849.4 | 466.7 | 631.9 | 695.6 | 357.4 | 654.4 | 633.1 | 633.2 |
| 41 | 285.5 | 513.6 | 628.4 | 721.9 | 2482 | 1004.7 | 621.2 | 2126 | 360.3 |
| 42 | 229.9 | 548.9 | 402.2 | 516.1 | 450.5 | 519.8 | 332.6 | 612.9 | 222.9 |
| 43 | 390.1 | 489.1 | 363.3 | 192.2 | 399.6 | 388.1 | 369 | 222.3 | 314.1 |
| 44 | 652.8 | 952.5 | 925.3 | 1044.2 | 930.7 | 784.2 | 962.5 | 1223.4 | 468 |
| 45 | 691.2 | 1619.8 | 791.5 | 1433.2 | 1616.2 | 618.5 | 1030.5 | 1531.9 | 335.2 |
| 46 | 351.6 | 412.4 | 339 | 330.5 | 453.3 | 276.5 | 345.7 | 346.9 | 376.6 |
| 47 | 256.3 | 423.2 | 349.8 | 448.9 | 546.2 | 138.6 | 305.9 | 635.7 | 188.2 |
| 48 | 297.1 | 112.1 | 708.1 | 397.5 | 367.9 | 134.4 | 1885.5 | 680.1 | 1204.7 |
| 49 | 497.2 | 2981.6 | 1179.9 | 712 | 5248.7 | 553.9 | 1047.2 | 640.1 | 381.8 |
| 50 | 350.3 | 659.2 | 627.4 | 427.2 | 665.1 | 230.3 | 1754.6 | 2654 | 228.2 |
| 51 | 80.2 | 1018.5 | 175.1 | 392.4 | 669.4 | 374.5 | 326.8 | 448.4 | 77.7 |
| 52 | 219.7 | 261.6 | 189.5 | 266.9 | 275.7 | 214.6 | 245.4 | 224.4 | 208.5 |
| 53 | 97.5 | 1385.3 | 1668.8 | 2690.6 | 1207.6 | 509 | 1096 | 2281.1 | 132.5 |
| 54 | 401.9 | 1101.6 | 1148.7 | 1527.2 | 880.3 | 1550.3 | 682.6 | 1648.3 | 318.8 |
| 55 | 87.2 | 79.5 | 120.6 | 81 | 64 | 126 | 101.5 | 117.8 | 130.8 |
| 56 | 41.5 | 356.3 | 290.8 | 220 | 321.6 | 255.3 | 116.9 | 178.9 | 114.6 |
| 57 | 4709.8 | 826 | 3670.5 | 13995.1 | 2735.5 | 1208.7 | 2495.3 | 8987.2 | 1600.1 |
| 58 | 5493.8 | 942.8 | 3824 | 13347.2 | 3431.8 | 1473.6 | 2604.6 | 9793.3 | 1896.9 |
| 59 | 990.8 | 4570.9 | 2467.2 | 2820.8 | 3584.1 | 1578.8 | 2600.2 | 2511.9 | 1043.9 |
| 60 | 86.6 | 409 | 328.3 | 697.7 | 313.2 | 195.4 | 394 | 769.5 | 205.8 |
| 61 | 148.6 | 102.4 | 113.5 | 119.1 | 96.3 | 180.5 | 151.5 | 144.5 | 90.7 |
| 62 | 2552.4 | 3419 | 2087.8 | 1947.4 | 3982.4 | 632.7 | 4686.3 | 1552 | 893.8 |

| | | | | | | | | | |
|---|---|---|---|---|---|---|---|---|---|
| 63 | 138.1 | 500.7 | 504.9 | 621.5 | 477.8 | 416.2 | 765.6 | 623.8 | 200.9 |
| 64 | 194.3 | 430.2 | 1152 | 334.2 | 1238.6 | 157.1 | 225.5 | 301.4 | 184.8 |
| 65 | 119.3 | 275.4 | 393.9 | 220.9 | 330.6 | 146.6 | 362.6 | 472.9 | 192.6 |
| 66 | 644.5 | 1716.5 | 799.8 | 2089.9 | 1882.3 | 860.9 | 884.9 | 1790.8 | 494.1 |
| 67 | 33664.7 | 18698 | 25385.3 | 29495.1 | 22746.1 | 32326.6 | 35830.4 | 25673.3 | 30440.8 |
| 68 | 4863.4 | 757.4 | 639.2 | 500.3 | 628.5 | 1067 | 877.5 | 537.2 | 2729.1 |
| 69 | 7538.6 | 1746.3 | 2633.5 | 2161.4 | 1446.6 | 3285.4 | 3619.9 | 2224.2 | 3873.7 |
| 70 | 1167.7 | 4005.5 | 2775 | 4480.9 | 5935.2 | 2888.4 | 2408.7 | 3524.2 | 1112.2 |
| 71 | 331.3 | 7296.2 | 3308.3 | 4722.7 | 6620.9 | 641.7 | 3323 | 7752.1 | 521.9 |
| 72 | 867.2 | 2722 | 587.7 | 1727.1 | 2480.6 | 715.1 | 1071.1 | 2441.2 | 729.3 |
| 73 | 248.7 | 702.4 | 513.9 | 998.3 | 674.5 | 351.8 | 560.2 | 646.8 | 291.3 |
| 75 | 191.9 | 299.8 | 1021.2 | 2627.9 | 481 | 184.1 | 684.5 | 3040.4 | 241.4 |
| 76 | 602.9 | 419.6 | 424.1 | 687.6 | 576.5 | 1929.4 | 374.1 | 1713.5 | 249.7 |
| 77 | 3969.7 | 1046.9 | 2803 | 1502.3 | 1123.7 | 3525.2 | 3967.9 | 610.4 | 3028 |
| 78 | 461 | 260.7 | 681.4 | 434.3 | 255.4 | 719 | 658.2 | 440.9 | 425.5 |
| 79 | 112.1 | 359.7 | 118.8 | 269.2 | 354.3 | 241.7 | 238 | 391.9 | 129.7 |
| 80 | 211.4 | 800.8 | 565.5 | 1265 | 877.2 | 479.3 | 445.8 | 1163.2 | 181.6 |
| 81 | 381.4 | 8963.9 | 1038.9 | 7150.5 | 8082.3 | 289.1 | 2966.6 | 5268.9 | 339.8 |
| 82 | 204.4 | 189.8 | 246.1 | 203.2 | 216.8 | 260.9 | 253.2 | 152.4 | 345.8 |
| 84 | 167 | 297.3 | 276.3 | 381.1 | 417.7 | 274.7 | 303.1 | 497.5 | 238.9 |
| 85 | 186.4 | 1174.1 | 201.5 | 1066.1 | 1674.8 | 143.6 | 434.1 | 306 | 203.8 |
| 86 | 75.7 | 748.2 | 119 | 174.2 | 654.4 | 84.9 | 219.2 | 164.9 | 102.9 |
| 87 | 252.7 | 2338.9 | 668.2 | 445 | 1007.4 | 249.1 | 1199.6 | 589.5 | 215 |
| 88 | 6167.9 | 470.3 | 4262.6 | 1023.8 | 548.1 | 5577.9 | 4604.6 | 683.8 | 3916.7 |
| 89 | 267 | 506.7 | 193 | 233.1 | 541.1 | 192.3 | 266.6 | 262.9 | 321.8 |
| 90 | 190.7 | 110.5 | 214.3 | 244.9 | 280 | 124.7 | 260 | 290 | 113.5 |
| 91 | 7102.8 | 1126.2 | 1543.8 | 6389.7 | 2050.8 | 529.2 | 760.6 | 7087.2 | 1108.2 |
| 92 | 1861.9 | 9980.6 | 9744 | 7280.9 | 5486.5 | 7913.2 | 7006.7 | 5412 | 2498.8 |
| 93 | 163.2 | 108.9 | 245.5 | 157.5 | 112.4 | 254.2 | 250.4 | 119 | 193 |
| 94 | 839.4 | 577 | 1488.5 | 1006.1 | 405.5 | 1082.5 | 1126.2 | 964.8 | 634.9 |
| 95 | 515.4 | 1234.6 | 807.9 | 454 | 959.8 | 864.4 | 770.5 | 494.4 | 662.2 |
| 96 | 144.9 | 212.1 | 199 | 351.5 | 306.3 | 223.8 | 227.7 | 302.5 | 175 |
| 97 | 130.8 | 471.3 | 333.4 | 484 | 448.6 | 329.5 | 480.1 | 568.4 | 147.1 |
| 98 | 15.8 | 32.4 | 37.6 | 34.4 | 51.3 | 39 | 38.4 | 65.9 | 38.2 |
| 99 | 318.3 | 435.4 | 289.7 | 489.3 | 399.5 | 198.2 | 351.7 | 473.1 | 298 |
| 100 | 12916 | 51.7 | 13251.4 | 1990.7 | 423.3 | 4429.8 | 13164.2 | 230 | 5552.1 |
| 101 | 532.8 | 290.8 | 329.4 | 342.8 | 316.8 | 363.7 | 300.1 | 234.7 | 333.5 |
| 102 | 229.2 | 186.8 | 199.6 | 138.3 | 206.2 | 149.6 | 226.5 | 175.3 | 202.1 |
| 103 | 342.5 | 406.4 | 434 | 355.9 | 371.1 | 306.7 | 526.4 | 435.6 | 362.1 |
| 104 | 596.1 | 90.6 | 698.7 | 267.8 | 65.4 | 947.3 | 366 | 148.9 | 436.1 |
| 105 | 533.9 | 86.9 | 770.1 | 333.9 | 108.7 | 831.3 | 274.7 | 239.4 | 443.9 |
| 106 | 573.6 | 70.7 | 537.2 | 270.6 | 112.2 | 911.2 | 341.9 | 194.3 | 486.5 |
| 107 | 1027.4 | 294.7 | 183.4 | 222.5 | 716.1 | 250.8 | 263.1 | 280.6 | 335.9 |
| 108 | 241.6 | 145 | 283.6 | 211.1 | 169.4 | 333 | 276.2 | 235.5 | 308.9 |
| 109 | 1066.5 | 2807.4 | 2388.4 | 1135.5 | 1428.2 | 2931.4 | 1315.4 | 2683.7 | 95.3 |
| 110 | 2960.7 | 7199 | 6101.1 | 2633.5 | 3373.8 | 5614 | 5706.1 | 6310.8 | 130.7 |
| 111 | 2125.4 | 189.5 | 742.5 | 359.1 | 253 | 1180.3 | 925.2 | 140.9 | 1262.1 |
| 112 | 523.3 | 2050.6 | 1308.3 | 1693.4 | 1084.8 | 1386.3 | 718.3 | 1809.1 | 394.9 |
| 113 | 74.8 | 326 | 591.7 | 308.2 | 365.7 | 404.6 | 253 | 437.5 | 190.6 |
| 114 | 494.6 | 580.3 | 510.6 | 830.3 | 436.6 | 489.3 | 361.3 | 460.5 | 588.4 |
| 115 | 501.5 | 966.3 | 740.2 | 1215.5 | 1149 | 363.5 | 1225 | 1118.5 | 466.4 |
| 116 | 279 | 621.8 | 666.9 | 515.9 | 520.1 | 199.1 | 1091 | 891.9 | 310.3 |
| 117 | 1708.9 | 122.3 | 1731.3 | 467 | 162.6 | 817.7 | 1395.5 | 183.9 | 1888.5 |
| 118 | 136.1 | 129.6 | 287.9 | 133.8 | 97 | 154.8 | 231.6 | 230.9 | 391.1 |
| 119 | 250.9 | 375.2 | 298.4 | 289.2 | 272.3 | 298.3 | 279.5 | 171.8 | 367.9 |
| 120 | 135 | 98.5 | 266 | 117.9 | 142.2 | 119.8 | 265.8 | 210.2 | 170.8 |

| | | | | | | | | | |
|---|---|---|---|---|---|---|---|---|---|
| 121 | 925.3 | 1636.7 | 2341 | 5981.4 | 2305 | 1239.6 | 3217.7 | 4469.4 | 424.6 |
| 122 | 146.6 | 403.5 | 321.9 | 294.5 | 387.4 | 169.6 | 475.8 | 415.8 | 99.5 |
| 123 | 185.4 | 288.7 | 188.9 | 285.9 | 269.3 | 240.8 | 291.1 | 457.9 | 164 |
| 124 | 178.1 | 141 | 152.7 | 97.1 | 156.7 | 104.4 | 167.4 | 144.7 | 176.4 |
| 125 | 1161.1 | 1141.8 | 2587.8 | 5502.6 | 1399.3 | 689 | 1600.7 | 2696.8 | 1040.4 |
| 126 | 656.8 | 38.1 | 771.3 | 130.6 | 17.2 | 1187.1 | 477 | 64 | 905 |
| 127 | 102.9 | 244.7 | 132 | 189 | 183 | 113.8 | 209.9 | 130.6 | 133 |
| 128 | 387 | 69514.5 | 255.7 | 68429 | 281 | 269 | 317.9 | 214941 | 338.3 |
| 129 | 357 | 1983.4 | 2117 | 559.5 | 1969.4 | 382.4 | 899 | 1412 | 221.4 |
| 130 | 80.2 | 488.5 | 349.7 | 368.2 | 469.6 | 255.7 | 169.9 | 378.5 | 109 |
| 131 | 97.6 | 577.1 | 413.3 | 535.1 | 592.4 | 154 | 308.1 | 504.5 | 115.2 |
| 132 | 259 | 267.5 | 225.2 | 362.6 | 285.3 | 231.6 | 294.2 | 316.4 | 233.1 |
| 133 | 62 | 284.1 | 126.4 | 152.8 | 224.7 | 170.9 | 160.7 | 281.1 | 70.9 |
| 134 | 129.3 | 363.2 | 256.8 | 186.3 | 308.2 | 228.3 | 147.2 | 117.2 | 137.1 |
| 135 | 398.3 | 284.6 | 523.7 | 620.2 | 375.8 | 623.2 | 554.6 | 638.2 | 372.3 |
| 138 | 150.3 | 205.6 | 166.6 | 165.6 | 244.7 | 180.3 | 238.1 | 192.4 | 141.7 |
| 139 | 87.2 | 82.8 | 324.7 | 238 | 168 | 198.4 | 204.8 | 178.3 | 147.6 |
| 140 | 2117.8 | 107.3 | 1157 | 1151.6 | 68.3 | 2112.4 | 1062.3 | 199.7 | 2021.8 |
| 141 | 1371.7 | 677 | 2205.3 | 517.1 | 203.1 | 1460.2 | 1313.5 | 110 | 1344.6 |
| 144 | 487.8 | 6712.6 | 9651.1 | 13878.1 | 3393.4 | 9082.5 | 5985.5 | 22188.6 | 513.2 |
| 145 | 262 | 429.3 | 287.6 | 252.2 | 268.9 | 217 | 336.2 | 203.2 | 226.4 |
| 146 | 194.8 | 463.3 | 178.9 | 314.7 | 540.9 | 163.7 | 240.6 | 520.1 | 224.1 |
| 147 | 59.7 | 56.9 | 119.9 | 366.3 | 152 | 50.5 | 148.4 | 263.9 | 103.4 |
| 148 | 821.7 | 868.3 | 548.5 | 469.3 | 1125.9 | 334.8 | 1013.8 | 676.8 | 741 |
| 149 | 242.9 | 174.5 | 271.1 | 252.6 | 219.5 | 363.8 | 434.4 | 169 | 218.3 |
| 150 | 183.1 | 227.8 | 233.6 | 259.9 | 424.1 | 141.2 | 359.8 | 423 | 249.3 |
| 151 | 211.4 | 1131.6 | 570 | 576.6 | 580.9 | 369.8 | 475.6 | 262.8 | 327.6 |
| 153 | 81.4 | 122.6 | 274.3 | 196.9 | 115.5 | 453.6 | 156.4 | 725.7 | 123.9 |
| 154 | 687.5 | 4823.4 | 1405.3 | 5742.3 | 4211.6 | 942.8 | 2873.5 | 9112.2 | 480.4 |
| 155 | 139.2 | 1013.1 | 289.7 | 320.7 | 766.9 | 289.3 | 405.9 | 264.7 | 116.9 |
| 156 | 77.8 | 638.3 | 346.5 | 465.6 | 740.7 | 128.6 | 262.3 | 511.3 | 127.6 |
| 157 | 210.4 | 137.4 | 176.3 | 144 | 198 | 97.2 | 246 | 151.5 | 150.2 |
| 158 | 14.7 | 1191.6 | 243.3 | 631.9 | 852.7 | 303.1 | 733.1 | 924.1 | 12.1 |
| 159 | 3335.3 | 1313.5 | 2648.7 | 1400.3 | 1293.6 | 2577.5 | 2106.8 | 1174.1 | 3077.4 |
| 161 | 58.4 | 6972 | 2447.5 | 5870.8 | 5806.5 | 3314.6 | 521.9 | 4390.4 | 98.3 |
| 162 | 145 | 582 | 315.4 | 818.2 | 813.3 | 91.8 | 982.1 | 2165.6 | 316.3 |
| 164 | 75.4 | 1096.7 | 5139.7 | 1786.4 | 674.5 | 836.8 | 3086.4 | 577.7 | 211.6 |
| 166 | 5882.4 | 1758.7 | 3139.9 | 1274 | 662.7 | 3213.1 | 4469.7 | 1150.4 | 3810.8 |
| 167 | 2593.9 | 1006.1 | 2947.1 | 987 | 1134.4 | 2040.2 | 2587.3 | 1035.8 | 2569 |
| 168 | 1092.3 | 472.4 | 620.1 | 812.6 | 879.6 | 719.1 | 508.4 | 613 | 801.1 |
| 169 | 16935.6 | 11084.6 | 7798.3 | 11788.6 | 6525.4 | 4941.2 | 10219 | 13134.4 | 4408.7 |
| 170 | 640.4 | 2234.2 | 2081.1 | 2242.8 | 2170.8 | 2086 | 1389.5 | 2450 | 684.4 |
| 171 | 4002.8 | 860 | 1755.1 | 540.2 | 1265.9 | 373.5 | 3814.4 | 1969.2 | 2887.5 |
| 172 | 871.6 | 562.5 | 496.9 | 524.4 | 536.8 | 741.7 | 618.8 | 487.5 | 795.1 |
| 173 | 575.8 | 843.7 | 419.7 | 1448.7 | 916.9 | 512.9 | 1159.7 | 2172.2 | 361 |
| 176 | 217.5 | 1069.1 | 1369.4 | 1083.4 | 461.5 | 2385.1 | 823 | 271.7 | 71.1 |
| 177 | 712.3 | 592 | 497.4 | 495.9 | 559.8 | 651.8 | 489.3 | 372.4 | 574 |
| 178 | 333 | 1722 | 1370.5 | 2400 | 1863.3 | 1852.3 | 1112.4 | 2050.7 | 135.6 |
| 179 | 148.9 | 1165.2 | 914 | 1121.1 | 1438.7 | 84.3 | 739.1 | 1727.8 | 116.9 |
| 180 | 178 | 394.9 | 592.7 | 482.6 | 468.6 | 261.9 | 679.3 | 584.8 | 124.9 |
| 181 | 8559.6 | 3563.7 | 9515.2 | 5496.6 | 3150.6 | 6350.8 | 8535 | 5034.9 | 9024.4 |
| 182 | 303.1 | 363.2 | 607 | 437.8 | 284.5 | 355 | 547.5 | 405.8 | 347.6 |
| 183 | 463.4 | 743.1 | 1416.6 | 409.4 | 692.8 | 313.6 | 1066.3 | 1567.7 | 752.9 |
| 184 | 321.8 | 771.5 | 17054.7 | 1022.1 | 346 | 4736.3 | 714.2 | 527.3 | 221.3 |
| 185 | 858.5 | 7670.7 | 3976.3 | 3238.8 | 6074.4 | 967.8 | 6474.3 | 5307.2 | 1275.6 |
| 186 | 422.8 | 223.9 | 502 | 187.1 | 245.8 | 411.1 | 334.5 | 155.1 | 496.2 |
| 187 | 130.6 | 301.4 | 247.9 | 382.4 | 430.9 | 98 | 251.4 | 429.4 | 499.9 |

| | | | | | | | | | |
|---|---|---|---|---|---|---|---|---|---|
| 188 | 231.3 | 443.1 | 490.2 | 209.5 | 672.4 | 207.6 | 460.1 | 490.2 | 230.3 |
| 189 | 186.2 | 290.4 | 253.1 | 112 | 418.8 | 162.1 | 356.5 | 338.2 | 129.7 |
| 190 | 115.7 | 719.2 | 278 | 442.3 | 529.6 | 419.8 | 501.5 | 634 | 110.1 |
| 191 | 762.2 | 2944.3 | 1482.9 | 1491 | 1153.8 | 593.3 | 2253.3 | 2972.3 | 855.8 |
| 192 | 90.9 | 135.5 | 169.1 | 82.5 | 159.1 | 122.1 | 164.6 | 95.6 | 111.4 |
| 193 | 830.6 | 156.9 | 650.7 | 332 | 73.5 | 1566.3 | 778.2 | 246.1 | 749.5 |
| 194 | 116.9 | 307.8 | 210.6 | 367 | 243.7 | 248.1 | 220.6 | 242.1 | 121.1 |
| 195 | 167.1 | 213.3 | 230.4 | 93.7 | 205.8 | 349.5 | 194.4 | 147.8 | 226.8 |
| 196 | 8492.8 | 4271.5 | 4837.9 | 4245 | 4837.6 | 5613.6 | 5166.8 | 2741.2 | 7143.4 |
| 197 | 126 | 137.6 | 88 | 126 | 163.1 | 62.4 | 170 | 153.7 | 99.7 |
| 198 | 77.9 | 1237.6 | 107.3 | 1714.1 | 2069.7 | 160.7 | 379.3 | 3850.1 | 42.8 |
| 199 | 1975.9 | 15850.1 | 1067.1 | 3670.6 | 6331.3 | 92.9 | 10936.9 | 4209.3 | 574.9 |
| 200 | 442.4 | 367.6 | 806.6 | 641.4 | 731.8 | 272.8 | 852.3 | 608.7 | 378.9 |
| 201 | 1717.3 | 1466.3 | 62.7 | 409 | 2205.6 | 81.5 | 409.1 | 388.1 | 531.5 |
| 203 | 185.5 | 1325.5 | 1994.8 | 2659.6 | 1146.7 | 553 | 426.7 | 1650.6 | 294.4 |
| 204 | 640.5 | 373.8 | 990 | 1221.5 | 639.1 | 971.7 | 716.9 | 673.8 | 413.2 |
| 205 | 3638.5 | 3132.4 | 5021.1 | 4836.2 | 2548.8 | 4867.6 | 5773.1 | 5664.6 | 4500.1 |
| 206 | 4305.4 | 266.2 | 226.9 | 194.4 | 2367.5 | 186.1 | 1565.6 | 1933.1 | 13603.8 |
| 207 | 991.4 | 24.6 | 637.6 | 252.9 | 75.4 | 1349.4 | 407.4 | 119.4 | 589.5 |
| 208 | 589.9 | 1696 | 384.8 | 2423.4 | 990.6 | 458.5 | 465 | 2841.2 | 361.9 |
| 209 | 3044.4 | 1250.1 | 3110.3 | 684.4 | 220.2 | 3097.8 | 4613.7 | 225.9 | 2810 |
| 211 | 21702.9 | 163 | 6106.7 | 1726.7 | 272.3 | 20338.2 | 14214.4 | 218.1 | 13108.3 |
| 212 | 363.1 | 1146.1 | 984.2 | 1161.8 | 1648.5 | 1165.9 | 720.9 | 1062.1 | 324.3 |
| 213 | 117.5 | 85.6 | 93.1 | 80.5 | 140.7 | 104 | 160.1 | 103.1 | 133.2 |
| 215 | 1547.8 | 71 | 895.2 | 308.8 | 53.3 | 2847.9 | 790 | 201.2 | 863.8 |
| 216 | 466.2 | 184 | 743.2 | 255.8 | 157.4 | 648.4 | 522.1 | 231.6 | 528.4 |
| 217 | 253.9 | 1156.6 | 956.5 | 1388.7 | 916.7 | 655.7 | 993.9 | 1488 | 283.6 |
| 218 | 273.9 | 156.2 | 297.3 | 168.4 | 134.5 | 253.5 | 206.7 | 182.5 | 309 |
| 220 | 111.8 | 108 | 178.4 | 87.6 | 87.4 | 78.9 | 114.5 | 73.1 | 95 |
| 221 | 401.7 | 980.8 | 956.6 | 1765.3 | 1118.9 | 944.1 | 2190.1 | 2120.8 | 463.1 |
| 223 | 313.8 | 693.9 | 257 | 526.7 | 856.5 | 230.4 | 725.6 | 679.9 | 230.5 |
| 227 | 1309.4 | 2741.4 | 829.9 | 1455.7 | 2656.4 | 513.5 | 2644.7 | 7955 | 811.4 |
| 228 | 788.4 | 341.7 | 662.1 | 341 | 400 | 605.3 | 554.8 | 321.3 | 480.1 |
| 229 | 165.7 | 231.4 | 242.7 | 245.2 | 169.4 | 132.9 | 206.6 | 199.8 | 256.2 |
| 230 | 378.3 | 3159.3 | 602.9 | 2495.6 | 2807.6 | 427.9 | 1760.9 | 2607.2 | 253.5 |
| 231 | 179.7 | 503.8 | 1002.1 | 932.9 | 465.2 | 263.4 | 286.4 | 683.6 | 168.8 |
| 232 | 2120.5 | 2917.6 | 6467.5 | 1163.8 | 3285.8 | 1825.2 | 5213.4 | 4175.9 | 3969.4 |
| 233 | 3812.2 | 8994.9 | 10159.2 | 16687 | 8022.5 | 3071.4 | 10101.6 | 14509.8 | 5294.2 |
| 235 | 2787.4 | 609.3 | 1195.2 | 414.8 | 721.3 | 253.4 | 2159.8 | 921 | 1447.2 |
| 236 | 205.5 | 420.9 | 541.4 | 310.6 | 425.8 | 168.2 | 568.3 | 431.3 | 257.5 |
| 237 | 1075.9 | 1872.5 | 2343.1 | 2170.9 | 1947 | 1599.7 | 1714 | 1850.9 | 1457.9 |
| 238 | 595.7 | 686.4 | 1928.8 | 1067.3 | 636.7 | 1278.6 | 1084.7 | 907.4 | 839.5 |
| 239 | 408.9 | 910 | 1240.6 | 343.7 | 979.2 | 698.3 | 1207.4 | 1043.8 | 297.9 |
| 240 | 439.8 | 896.6 | 923.9 | 386.1 | 1012.3 | 671.2 | 1029.8 | 1706.7 | 346.5 |
| 241 | 1039.9 | 2207 | 2261 | 786.1 | 1746.5 | 1431 | 2286 | 2914.4 | 614.4 |
| 242 | 6785 | 25733 | 9423.8 | 18037.6 | 23053.8 | 1044.8 | 22442.7 | 27313.9 | 3641 |
| 243 | 172.2 | 719.2 | 374.5 | 427.7 | 727.7 | 198.8 | 793.6 | 730.7 | 379 |
| 244 | 117.1 | 158.2 | 359.4 | 1692.5 | 225.5 | 224.6 | 205.9 | 781.3 | 255.6 |
| 246 | 205.2 | 1124.9 | 754.6 | 1864.1 | 1124.1 | 998.2 | 993.1 | 2067 | 192.7 |
| 247 | 158.1 | 122.8 | 98.9 | 90.5 | 167.3 | 80.1 | 192 | 119.8 | 170.2 |
| 248 | 93.3 | 964 | 2395.2 | 4780.4 | 1534.5 | 1401.2 | 1160.4 | 3767.9 | 132.8 |
| 249 | 628.3 | 275.9 | 966.5 | 609.4 | 278.5 | 1711.8 | 1017 | 717.3 | 746.2 |
| 251 | 86.7 | 1115.8 | 467.4 | 572.2 | 1064.5 | 196 | 697.9 | 947.7 | 78.7 |
| 253 | 180.8 | 267.8 | 313.8 | 390.7 | 438.7 | 298.7 | 304.7 | 693.5 | 229.6 |
| 254 | 323.1 | 411.8 | 1816.5 | 1559.8 | 527.5 | 2075 | 1017.1 | 1776.1 | 350.4 |
| 255 | 307.7 | 1612.2 | 1295.2 | 2395.7 | 1944.5 | 1881.3 | 1003.5 | 2216.6 | 117.3 |
| 256 | 4984 | 885.5 | 1242.8 | 5330.6 | 1414.4 | 583.9 | 717.6 | 2463.5 | 988.4 |

| | | | | | | | | | |
|---|---|---|---|---|---|---|---|---|---|
| 257 | 229.6 | 347.5 | 146.2 | 392.4 | 283.4 | 105.2 | 231.5 | 331.8 | 93.6 |
| 258 | 467.5 | 1932.3 | 306.9 | 580.9 | 1669.9 | 50.7 | 1440.1 | 419.2 | 185.1 |
| 259 | 656.1 | 739.8 | 1239.2 | 816.5 | 649.5 | 213.6 | 1710.4 | 612.8 | 550.2 |
| 260 | 211.1 | 149.6 | 119.9 | 165.1 | 155.4 | 190.5 | 169.4 | 142.4 | 201.5 |
| 261 | 304.8 | 92.6 | 456.1 | 380.7 | 123.1 | 205.9 | 183.4 | 133.3 | 296.1 |
| 263 | 416.5 | 696.6 | 508.2 | 595.2 | 545.5 | 479.7 | 789.5 | 725.8 | 408.1 |
| 263 | 565.6 | 2856 | 535.6 | 2244.5 | 2669.9 | 321.7 | 1544.8 | 2324 | 280 |
| 265 | 470.5 | 69.8 | 209.8 | 116.1 | 97.9 | 338.5 | 324.8 | 100.9 | 325.4 |
| 266 | 256 | 195.2 | 345.7 | 246.5 | 313.5 | 349.5 | 292.8 | 235.5 | 347.8 |
| 256 | 173.1 | 291.4 | 341.1 | 568.6 | 490.5 | 185.1 | 300.4 | 785.2 | 162.7 |
| 268 | 527.3 | 595.7 | 2023.3 | 935.8 | 545 | 1146.6 | 808.3 | 526.4 | 721.4 |
| 269 | 32.6 | 266.5 | 71.6 | 170.1 | 494.5 | 52.6 | 298.3 | 240.8 | 65.9 |
| 270 | 279.9 | 197.9 | 274.9 | 183 | 205.6 | 244.4 | 305.9 | 202.5 | 162.7 |
| 271 | 242.7 | 1141.1 | 567 | 357.7 | 1086 | 274.5 | 625.4 | 1269.3 | 250.7 |
| 272 | 700.4 | 967.8 | 477.4 | 599.8 | 826.1 | 428.3 | 698 | 698.6 | 480 |
| 273 | 4697.9 | 211 | 2811.9 | 916.2 | 234.7 | 3500.5 | 4802.8 | 1550 | 1104.3 |
| 274 | 603.8 | 1411.8 | 1737.5 | 867.4 | 1091 | 321.9 | 1389.7 | 1216 | 664.2 |
| 275 | 225.9 | 3619.5 | 1297.8 | 806.9 | 2484.1 | 321.7 | 2165.1 | 1639.8 | 396.9 |
| 276 | 2732 | 4767.8 | 2036.4 | 2069.3 | 5010.2 | 844 | 6695.5 | 12351.9 | 1238.7 |
| 277 | 255.2 | 264.7 | 225.8 | 355.7 | 325.1 | 200.6 | 250.2 | 259 | 206.6 |
| 278 | 765.8 | 1291.5 | 1132.1 | 1008.7 | 1492.3 | 255.4 | 1550.7 | 1198.8 | 598.7 |
| 279 | 2233.9 | 1837 | 1527.8 | 2169.7 | 1934.2 | 2261.9 | 1828.4 | 2407.2 | 2843.7 |
| 280 | 3349.3 | 2489.9 | 1693.7 | 3130.8 | 2269.8 | 3147.5 | 2337 | 3287.7 | 2513.8 |
| 281 | 37.7 | 385.1 | 326.7 | 815.7 | 570.2 | 488.3 | 255.1 | 762.4 | 74 |
| 282 | 523 | 447 | 277.8 | 1273.6 | 749.8 | 235.4 | 421.2 | 3058.5 | 317.7 |
| 283 | 447.5 | 340.8 | 310 | 351.6 | 440.4 | 246.8 | 393 | 386.9 | 348.1 |
| 284 | 74.4 | 83 | 130.8 | 103.8 | 100.8 | 71.2 | 141.1 | 145.8 | 89.2 |
| 285 | 116 | 109.8 | 107.8 | 96.2 | 136.8 | 95 | 105.2 | 105.6 | 93.8 |
| 286 | 1056.7 | 63.5 | 148.9 | 68.8 | 54.6 | 307.6 | 176.7 | 60.5 | 344.1 |
| 287 | 126.9 | 81.8 | 136.5 | 115.2 | 128.5 | 110 | 153.7 | 95.5 | 99.4 |
| 288 | 172.6 | 145.5 | 166.4 | 146.9 | 195.1 | 144.4 | 165.3 | 173.2 | 184 |
| 289 | 2816.8 | 4208.5 | 1023.7 | 2660.2 | 4312.6 | 404.1 | 3191.6 | 3539 | 1715.5 |
| 291 | 120 | 338.3 | 132.7 | 186.1 | 234.4 | 129.8 | 203 | 425.5 | 113 |
| 292 | 473.6 | 820.1 | 4181.1 | 6680.4 | 826.4 | 110.4 | 8212.2 | 16919.5 | 977.5 |
| 293 | 120 | 1421.4 | 1229.9 | 592.8 | 1834.6 | 509 | 674.6 | 986.7 | 171.8 |
| 295 | 341.6 | 1948.4 | 1254.9 | 2043.1 | 2299.8 | 1064.4 | 1671.9 | 2245.9 | 229.1 |
| 296 | 552.7 | 1443.5 | 1644.1 | 1603.5 | 1908.8 | 822.2 | 1505.8 | 1432.6 | 753.8 |
| 297 | 45.3 | 90.8 | 94.6 | 50.3 | 139 | 53.5 | 88.5 | 154.3 | 116.4 |
| 298 | 440.1 | 517.5 | 501.4 | 487.3 | 470.9 | 471.4 | 489.6 | 506.6 | 219.8 |
| 299 | 725.6 | 819.4 | 1275.9 | 4386.6 | 3103.3 | 1663.7 | 910.5 | 4069.9 | 1147.8 |
| 300 | 177.4 | 1033.2 | 826.4 | 1230.7 | 1114.8 | 748.3 | 543.4 | 1306.1 | 250.1 |
| 301 | 2124.1 | 211.2 | 358.6 | 194.9 | 158 | 913.5 | 300.7 | 228.4 | 485.7 |
| 302 | 37.2 | 267 | 6.5 | 240.6 | 211.9 | 26.1 | 121.2 | 193.6 | 46.4 |
| 303 | 324.2 | 270.8 | 472.7 | 273.6 | 263 | 997.6 | 487 | 248.9 | 581.6 |
| 304 | 344.9 | 585.6 | 322.9 | 461.8 | 884.2 | 214.1 | 543 | 356.5 | 364.9 |
| 305 | 175.5 | 190.4 | 287.7 | 598.2 | 791.2 | 551.1 | 859.4 | 2550.8 | 158.7 |
| 306 | 189.2 | 803.1 | 191.1 | 557.8 | 620.8 | 163.1 | 322.9 | 218.6 | 195.4 |
| 307 | 302 | 238.1 | 145.1 | 553.9 | 295.1 | 159.8 | 280.9 | 449.1 | 226.3 |
| 308 | 1190.6 | 794.8 | 1165.2 | 705.2 | 515.1 | 1800.1 | 914.4 | 520.9 | 1641.8 |
| 310 | 362.3 | 134.8 | 505.7 | 103.4 | 125.4 | 229.5 | 318.3 | 62.5 | 326.1 |
| 311 | 62.7 | 499.8 | 263.8 | 932.2 | 246.8 | 162.7 | 224.5 | 1036.4 | 115.8 |
| 312 | 902.2 | 50.7 | 593.6 | 151.6 | 54.4 | 843.6 | 810.1 | 19.4 | 523.1 |
| 313 | 132.9 | 517.9 | 687.5 | 313.3 | 244.1 | 457.1 | 385.1 | 576.9 | 131.9 |
| 314 | 522.6 | 650.8 | 980.1 | 1264.1 | 1058 | 586.8 | 1856.2 | 1354.1 | 372 |
| 315 | 391.8 | 154.3 | 554.7 | 155.4 | 153.4 | 591.4 | 395.7 | 235.9 | 458 |
| 316 | 210.3 | 558.3 | 491 | 565.6 | 525.6 | 484.4 | 470.4 | 509 | 234 |
| 317 | 314.7 | 271 | 195.4 | 240.9 | 303.1 | 168.8 | 387.8 | 1026.2 | 214.7 |
| 318 | 45.9 | 383.2 | 425.9 | 527.9 | 428.5 | 97 | 264.8 | 364.3 | 127.7 |

| | | | | | | | | | |
|---|---|---|---|---|---|---|---|---|---|
| 319 | 174.9 | 528.4 | 259 | 454.7 | 535.4 | 302.5 | 225.5 | 319.9 | 223.3 |
| 320 | 572.9 | 159.3 | 518.2 | 278.5 | 211.4 | 569.8 | 518.8 | 239.3 | 464.9 |
| 321 | 307.2 | 232.7 | 332.4 | 169.5 | 222.3 | 311.8 | 367.9 | 203.1 | 354.6 |
| 322 | 164.7 | 350.9 | 214.6 | 345.1 | 354.5 | 264.7 | 257.5 | 318.3 | 243.3 |
| 323 | 2580.9 | 1349.5 | 3402.6 | 2592.2 | 1137.5 | 3939.8 | 2125.9 | 1848.5 | 1426.6 |
| 324 | 69 | 597.2 | 475.4 | 645.4 | 435.8 | 233.3 | 224 | 688.1 | 72.4 |
| 325 | 317.1 | 681.6 | 505.4 | 818.9 | 491.5 | 395.8 | 558.8 | 605.8 | 254.6 |
| 326 | 790 | 1838.6 | 2873.2 | 3607 | 2251.9 | 4068.7 | 6144 | 3552.4 | 3136 |
| 327 | 6.7 | 409.3 | 78.4 | 615.9 | 402.1 | 10 | 18.7 | 342.6 | 30.3 |
| 328 | 751.2 | 151 | 376.6 | 307 | 255.2 | 542.9 | 560.4 | 300.2 | 662.8 |
| 329 | 85.3 | 109.9 | 91 | 74 | 63 | 94.7 | 77.9 | 45.9 | 80.6 |
| 330 | 54.9 | 951 | 436.1 | 1119 | 408.8 | 55.7 | 678 | 877.4 | 141.4 |
| 331 | 403.9 | 382.1 | 215 | 302.1 | 371.7 | 380.3 | 325.4 | 324.5 | 332 |
| 333 | 571.4 | 156 | 462.8 | 160.5 | 140.1 | 1058.8 | 443.4 | 150.5 | 603.1 |
| 334 | 195.4 | 500.9 | 1027.6 | 250.9 | 655 | 196.6 | 332.8 | 432.2 | 161.6 |
| 335 | 155.1 | 109.1 | 732.5 | 572.2 | 165.5 | 62 | 194.8 | 923.7 | 127.6 |
| 336 | 668 | 210.4 | 480.1 | 280.3 | 175.5 | 389.2 | 344.2 | 252.7 | 445.1 |
| 337 | 197.1 | 220.6 | 207.5 | 266.7 | 387.8 | 309.2 | 374 | 240.3 | 256.6 |
| 338 | 217.2 | 890.9 | 464.6 | 1427.6 | 896.6 | 189.4 | 1039 | 1728.1 | 231.7 |
| 339 | 377 | 346.8 | 345.1 | 332.7 | 324.7 | 435.1 | 457.4 | 277.5 | 454.9 |
| 340 | 330.6 | 95874.2 | 237.5 | 123339.8 | 92245.2 | 222.4 | 696.6 | 74884.9 | 304.2 |
| 341 | 657.2 | 434.5 | 521.8 | 461 | 442.6 | 785.8 | 552.9 | 485.6 | 713.7 |
| 343 | 109.4 | 4793.6 | 207.9 | 601.9 | 2590 | 773.6 | 1138 | 1171.9 | 57.1 |
| 344 | 1167 | 249.7 | 893.9 | 545.2 | 581.2 | 963.4 | 1019.3 | 554.7 | 1114.2 |
| 345 | 252.6 | 620.5 | 437.9 | 408.2 | 509.3 | 204.8 | 544.8 | 404.8 | 294.1 |
| 347 | 2279.3 | 1845.7 | 6453.3 | 3490 | 1999.5 | 3041.5 | 2644 | 1859.9 | 2280.8 |
| 348 | 2856.1 | 2533.8 | 7636.1 | 6329.8 | 3556.3 | 5813.9 | 5133.1 | 4694.9 | 5037.6 |
| 349 | 353.3 | 256.7 | 290.3 | 316.7 | 301.8 | 407.4 | 319.1 | 485.8 | 258.4 |
| 350 | 386 | 804.7 | 473.6 | 475.2 | 611.1 | 257.1 | 657.4 | 638.3 | 199.2 |
| 351 | 308.4 | 2206.4 | 1213.1 | 2566.3 | 1996.9 | 246.9 | 1150.3 | 2079.9 | 292.1 |
| 352 | 355.6 | 83.4 | 314.2 | 140.3 | 111.5 | 364 | 265.6 | 231 | 294.5 |
| 353 | 232.6 | 402.1 | 295 | 294.2 | 423.7 | 229.4 | 384.5 | 414.6 | 243.7 |

3d

| SEQ ID NO: | 840TT | 842TT | 862TT | 863TT | 864TT | 865TT | 866TT | 867TT | 869TT |
|---|---|---|---|---|---|---|---|---|---|
| 354 | 413.4 | 103.1 | 28.5 | 89.1 | 50.8 | 119.4 | 632.2 | 9.2 | 48.7 |
| 355 | 518.1 | 63.6 | 16 | 83.3 | 29 | 114.5 | 55.8 | 49.5 | 44 |
| 356 | 617.8 | 1163.1 | 1507.8 | 789.4 | 1313.2 | 1067.3 | 1224.9 | 824.3 | 740.8 |
| 357 | 750.6 | 663.3 | 1662.6 | 876.7 | 839.2 | 1660 | 1243 | 1043.5 | 843.5 |
| 358 | 647.8 | 509.5 | 567.7 | 1088.7 | 1049.5 | 795 | 1349.4 | 535 | 1699.5 |
| 359 | 330.5 | 214.7 | 472.1 | 313.5 | 373.5 | 481.3 | 371.9 | 361.8 | 235.3 |
| 362 | 288.4 | 245.9 | 172.7 | 589.6 | 377.5 | 866.5 | 666.6 | 454.5 | 954 |
| 360 | 434.1 | 305.6 | 182.5 | 297.3 | 370.2 | 347.1 | 290.1 | 218.1 | 444.6 |
| 361 | 682.3 | 740.4 | 589.9 | 765.7 | 495.7 | 894.5 | 527.5 | 1472.7 | 327.2 |
| 363 | 435.5 | 328.8 | 483.4 | 387 | 360.5 | 673 | 502.1 | 431.2 | 367 |
| 1 | 532.1 | 658.9 | 827.5 | 640.1 | 768.3 | 484.6 | 619.1 | 510.9 | 964.5 |
| 2 | 4059.8 | 3830.7 | 4516.5 | 3770.5 | 2512.9 | 3756.6 | 2448.4 | 3524.2 | 3003.6 |
| 7 | 483.8 | 209.7 | 470.6 | 825.4 | 484.2 | 153.7 | 393.7 | 965 | 873.7 |
| 8 | 1199.1 | 1312 | 934.6 | 1643.2 | 1176.8 | 871.7 | 1342.3 | 768.5 | 732 |
| 9 | 8223.9 | 2596.3 | 7524.8 | 6220.9 | 3456.3 | 6027 | 7023.5 | 7427.5 | 3975.6 |
| 10 | 51.2 | 96.2 | 67.9 | 101.6 | 111.8 | 139.3 | 75.7 | 46.4 | 157.4 |
| 11 | 171.2 | 198.5 | 495.5 | 218.3 | 206 | 597.6 | 634.9 | 288.5 | 318.8 |
| 13 | 537.4 | 265 | 36.3 | 777.1 | 442.1 | 95.7 | 614.9 | 65.2 | 238.9 |
| 14 | 1233.6 | 1157.9 | 134.4 | 2731 | 1781.1 | 837.6 | 2365 | 195.6 | 747.9 |
| 15 | 699.2 | 133.5 | 191.7 | 417.5 | 408.8 | 260 | 185.5 | 349.2 | 539.4 |

| | | | | | | | | | |
|---|---|---|---|---|---|---|---|---|---|
| 16 | 662.4 | 1257.3 | 1518.2 | 1301 | 966.8 | 1066.2 | 1385 | 714.3 | 1347.5 |
| 17 | 1194.5 | 1224.2 | 2909.6 | 1355.9 | 1395.5 | 1413.2 | 1584.1 | 1543.7 | 1301.1 |
| 19 | 1305.5 | 169.4 | 164.2 | 167.4 | 153.8 | 335.4 | 192.4 | 217.8 | 159 |
| 20 | 11.1 | 1.1 | 55 | 1.1 | 1.2 | 69.6 | 36.1 | 1.7 | 2.7 |
| 21 | 240.8 | 87.2 | 45.6 | 290.6 | 14.9 | 100.6 | 193.6 | 1.2 | 665.2 |
| 22 | 9947.1 | 7799.8 | 11063.4 | 5454.8 | 5808.5 | 5943.2 | 5158.2 | 8719.7 | 4509.2 |
| 23 | 956.7 | 1.1 | 48.1 | 157.6 | 1.1 | 4.3 | 28.8 | 77.3 | 62.7 |
| 24 | 218 | 30.6 | 818.7 | 223.9 | 55.2 | 225 | 83.6 | 138.2 | 313.6 |
| 26 | 9557.1 | 76.6 | 228.7 | 772.1 | 58.3 | 169.6 | 349.8 | 241.6 | 342.1 |
| 27 | 776.7 | 416.1 | 446.1 | 321.7 | 837 | 194.8 | 624.3 | 214.8 | 248.9 |
| 29 | 59.3 | 307.6 | 113.8 | 111.9 | 151.8 | 93.7 | 155.2 | 94.7 | 165.9 |
| 30 | 1162 | 61.1 | 36.1 | 74 | 37.7 | 338 | 95.9 | 132.1 | 81.4 |
| 31 | 656.3 | 70.7 | 13.1 | 264 | 24.8 | 107.5 | 243.8 | 63.6 | 1804.1 |
| 33 | 863 | 622.5 | 646.3 | 338.4 | 225.9 | 1364.6 | 528.1 | 298.3 | 824.1 |
| 34 | 4801 | 4062.8 | 7041.9 | 2172.2 | 2709.9 | 7154.6 | 4874 | 3483.4 | 1898.9 |
| 35 | 349.5 | 48.6 | 21 | 69.1 | 220.8 | 140.3 | 127.8 | 42.4 | 29.6 |
| 36 | 247.6 | 112.1 | 70.6 | 952.1 | 127.4 | 437.9 | 132.7 | 244.9 | 2851.6 |
| 37 | 359.5 | 154.3 | 129.5 | 201 | 163.4 | 356.6 | 166.6 | 166.1 | 147.5 |
| 38 | 818.9 | 343.8 | 681 | 579.2 | 1066.9 | 635.4 | 454.1 | 452.9 | 944.3 |
| 40 | 584.7 | 371.3 | 337.3 | 437.1 | 313.3 | 551.1 | 403.2 | 378 | 412.8 |
| 41 | 247.9 | 211.8 | 28 | 324.9 | 206.9 | 141.2 | 142.1 | 246.9 | 1045.2 |
| 42 | 201.2 | 77.8 | 271.3 | 370.1 | 220.5 | 370.8 | 243.3 | 311.3 | 477.7 |
| 43 | 319.5 | 237 | 319.5 | 348.9 | 367.9 | 367.4 | 376.6 | 359.5 | 514.5 |
| 44 | 340.2 | 338.9 | 375.8 | 466.7 | 295.2 | 1064.8 | 611.2 | 385.6 | 862.4 |
| 45 | 387.6 | 484.6 | 587.1 | 607.2 | 566.7 | 339.3 | 634 | 461.7 | 686.8 |
| 46 | 312.8 | 273 | 211.3 | 310.6 | 243.5 | 430.8 | 275.4 | 232.3 | 243.1 |
| 47 | 267.1 | 166.9 | 95.5 | 209.9 | 200.1 | 356 | 306.7 | 200 | 468.1 |
| 48 | 586.4 | 32.7 | 36.8 | 85.4 | 15.6 | 170.4 | 44.6 | 128.7 | 52.4 |
| 49 | 559.1 | 182.8 | 87.8 | 323.1 | 320.3 | 292.7 | 479.7 | 171.1 | 244 |
| 50 | 183 | 51.5 | 313.7 | 151.2 | 54.7 | 307.2 | 159.4 | 147.7 | 107.5 |
| 51 | 50.2 | 57.3 | 25.1 | 53.5 | 27.6 | 365.3 | 326.7 | 129.5 | 249.3 |
| 52 | 195.7 | 196.6 | 173.8 | 234.1 | 186.2 | 225.4 | 159.6 | 208.9 | 221.8 |
| 53 | 82.5 | 34.1 | 142.8 | 294.3 | 73 | 168.9 | 158.4 | 50.3 | 1001.9 |
| 54 | 497.5 | 1164.8 | 197.8 | 1471.8 | 1002.7 | 709.6 | 1166.2 | 163.1 | 1728.2 |
| 55 | 119.7 | 111.7 | 100 | 104.8 | 90.9 | 118.7 | 116 | 94.5 | 92.2 |
| 56 | 50.4 | 78.9 | 15.7 | 207.8 | 125.4 | 69.1 | 86.5 | 91.8 | 155 |
| 57 | 913.7 | 120.1 | 39.3 | 348.7 | 96 | 112.4 | 648.3 | 63.7 | 1449.6 |
| 58 | 937.3 | 144.4 | 33.7 | 395.5 | 111.4 | 501.8 | 1139.1 | 62.2 | 1741.7 |
| 59 | 1524.7 | 2456.9 | 2314.6 | 1572.7 | 1908.7 | 1437.1 | 1816.2 | 851.3 | 1581 |
| 60 | 239.9 | 177.8 | 101.9 | 208.9 | 122.9 | 164.2 | 115.8 | 114.4 | 341.6 |
| 61 | 138.8 | 102.4 | 167.6 | 195.1 | 161.4 | 602.8 | 171.7 | 220.1 | 121.5 |
| 62 | 3218.4 | 1230.8 | 93.3 | 144.4 | 1893.7 | 776.3 | 1111.1 | 1116.3 | 275.7 |
| 63 | 476.2 | 251.9 | 390.8 | 305.5 | 390.5 | 446.7 | 360.3 | 186.2 | 416 |
| 64 | 260 | 108.5 | 72.5 | 133 | 186.3 | 125.9 | 184.1 | 121.4 | 174.1 |
| 65 | 130.1 | 114.1 | 115.9 | 113.8 | 100.7 | 57.6 | 84 | 103.6 | 167.3 |
| 66 | 736.8 | 1620 | 528.8 | 1200.9 | 1060.8 | 922.4 | 1062.5 | 613.3 | 1936.4 |
| 67 | 36967.4 | 32327.9 | 35416.1 | 33262.7 | 33086.4 | 50663.1 | 33055.8 | 33098.2 | 33842.1 |
| 68 | 3604.6 | 765.6 | 2711 | 2612.8 | 1433.6 | 3309.4 | 1090.5 | 3575.3 | 2217.7 |
| 69 | 4370.2 | 2244.8 | 8123.8 | 4669.2 | 2913.3 | 9134.7 | 2674.3 | 9458.6 | 4732.4 |
| 70 | 2223.5 | 1376.4 | 751.7 | 2378 | 1890.8 | 2166.2 | 2162.9 | 1456.9 | 3220.2 |
| 71 | 1018.7 | 90.7 | 156 | 1072.2 | 120.5 | 96.6 | 145.6 | 125.6 | 898.5 |
| 72 | 552.9 | 1523.3 | 537.5 | 355.6 | 575.3 | 229.2 | 397.7 | 248.8 | 869.3 |
| 73 | 447.7 | 234.9 | 263.5 | 297.8 | 293.7 | 265.4 | 305 | 231.6 | 465.4 |
| 75 | 260.1 | 159 | 459.2 | 194.5 | 212.8 | 460 | 245.4 | 508.2 | 472.1 |
| 76 | 273.6 | 135.4 | 1.1 | 2171.1 | 611.1 | 559.9 | 220.4 | 108.3 | 1748.8 |
| 77 | 4806.2 | 4952.7 | 7485.3 | 2585.4 | 4993.9 | 9359.9 | 6144.5 | 5753.5 | 2422 |
| 78 | 623.9 | 418.4 | 269 | 898.4 | 765.7 | 441 | 601.4 | 703 | 1341.8 |
| 79 | 121.6 | 161.5 | 129.6 | 160.7 | 133.3 | 143.3 | 133.6 | 130.7 | 177.4 |

| | | | | | | | | | |
|---|---|---|---|---|---|---|---|---|---|
| 80 | 132.5 | 767.8 | 1091.7 | 413.8 | 130.3 | 423 | 489.5 | 238.5 | 763.7 |
| 81 | 512.5 | 282.4 | 123.8 | 218.8 | 242.4 | 297 | 1287.3 | 81.3 | 806.9 |
| 82 | 309.9 | 198.2 | 168.6 | 248.4 | 267 | 111.1 | 195.8 | 299.2 | 260.3 |
| 84 | 387.2 | 136.7 | 73.1 | 108.8 | 68.4 | 133.5 | 96.7 | 102.1 | 324.1 |
| 85 | 248.9 | 142.3 | 90.9 | 119.5 | 146.4 | 305.5 | 231.4 | 148.7 | 109.4 |
| 86 | 128.2 | 81.2 | 328 | 73.6 | 54.4 | 101.3 | 98.7 | 95.6 | 101 |
| 87 | 389.7 | 249.9 | 208.9 | 161 | 233.2 | 395.2 | 213.6 | 276.9 | 167.9 |
| 88 | 3944.8 | 2735.3 | 8374.9 | 5614.3 | 5926.1 | 7145.8 | 6096.2 | 9499.1 | 4340.8 |
| 89 | 354 | 171.2 | 148.1 | 205.8 | 141.7 | 276.5 | 145.7 | 180.3 | 128.5 |
| 90 | 115.9 | 91.9 | 105.1 | 103.9 | 93 | 163.4 | 108.3 | 110.6 | 69.3 |
| 91 | 1164.3 | 208.6 | 1.2 | 284.2 | 96.6 | 444.2 | 1453.8 | 67 | 464.2 |
| 92 | 4085 | 6778.2 | 2708.1 | 9676.9 | 5168.6 | 3758.8 | 6575.2 | 2375.7 | 10249.7 |
| 93 | 301.2 | 226.8 | 421.5 | 194.9 | 232.2 | 375.5 | 171.1 | 428.1 | 163.7 |
| 94 | 995.1 | 541.1 | 897.8 | 657.7 | 595.7 | 381.9 | 809 | 335 | 1275.8 |
| 95 | 1045.2 | 582.5 | 118.6 | 543.6 | 452.1 | 179.3 | 209.1 | 188.8 | 345.6 |
| 96 | 151.5 | 196 | 167.6 | 244 | 181.7 | 245.6 | 205.7 | 171.9 | 230.8 |
| 97 | 166.6 | 276.5 | 308 | 289.5 | 386.8 | 228.9 | 347 | 249.1 | 374.6 |
| 98 | 60.4 | 31.5 | 27 | 23.1 | 9.2 | 31.1 | 20 | 38.2 | 21.7 |
| 99 | 253.6 | 187.7 | 194.3 | 246 | 183.1 | 311.2 | 227.1 | 164.5 | 250.1 |
| 100 | 19287.6 | 6400.4 | 8181.3 | 2605.3 | 10229.3 | 7403 | 9672.3 | 11255.4 | 738.2 |
| 101 | 266.7 | 227.3 | 183.5 | 187 | 210.8 | 246.9 | 215.4 | 175.2 | 303.8 |
| 102 | 171.5 | 108.4 | 168.3 | 145.6 | 139.2 | 216.4 | 161.6 | 192.6 | 129.1 |
| 103 | 278.8 | 262.5 | 300.2 | 251.6 | 305.7 | 614.4 | 341.7 | 296.7 | 354.3 |
| 104 | 443.1 | 801.2 | 207.5 | 609.2 | 639.4 | 662.8 | 235.7 | 480.5 | 507.8 |
| 105 | 463.1 | 1053.8 | 415.1 | 584.5 | 740.7 | 772.3 | 384.2 | 617 | 387.3 |
| 106 | 518.9 | 705.8 | 207.9 | 540.7 | 585.7 | 524.4 | 227 | 513.5 | 481.3 |
| 107 | 364.9 | 105 | 41.3 | 163.2 | 64.4 | 139.2 | 185.1 | 113.3 | 132.5 |
| 108 | 330.2 | 330.9 | 645.2 | 275.1 | 408 | 425.5 | 324.6 | 567.1 | 309.2 |
| 109 | 67.3 | 6034.5 | 117 | 7025.2 | 3555.9 | 130.1 | 4629.2 | 56.5 | 4997.5 |
| 110 | 28.9 | 11743.3 | 96.5 | 11643.7 | 6913.6 | 1.2 | 6469 | 1.1 | 12381.4 |
| 111 | 1421.1 | 1571 | 367.8 | 1848.8 | 3058.4 | 428.3 | 1123.4 | 1923.1 | 1485.6 |
| 112 | 527 | 115.6 | 213 | 921.9 | 582.1 | 50.6 | 951.8 | 441.7 | 1495.5 |
| 113 | 455.9 | 246.9 | 53.3 | 182.6 | 309 | 119 | 257 | 158.9 | 473.4 |
| 114 | 507.9 | 943.6 | 1193 | 488.7 | 600.7 | 470.7 | 525.8 | 646.5 | 1445.6 |
| 115 | 579.2 | 287 | 238.3 | 468.6 | 331.5 | 339.6 | 406.9 | 347.8 | 648.7 |
| 116 | 323 | 237.9 | 245 | 203.6 | 210.8 | 323.5 | 238.4 | 227.4 | 251.7 |
| 117 | 2477.5 | 2049 | 5384.6 | 1932.8 | 2356.6 | 2151 | 1724 | 4147.6 | 916.2 |
| 118 | 442.9 | 1260.5 | 217.3 | 146.6 | 481 | 247.5 | 132.8 | 138.7 | 375.2 |
| 119 | 196.2 | 294.2 | 59.3 | 102.7 | 207.4 | 22.6 | 112.9 | 103.5 | 412.3 |
| 120 | 173.7 | 63.5 | 76.2 | 116 | 85.4 | 51.5 | 83.3 | 88.4 | 65.8 |
| 121 | 572.4 | 1198.8 | 854.2 | 827.2 | 1024.8 | 671 | 1242.7 | 359.7 | 1485.9 |
| 122 | 290.1 | 202.3 | 237.7 | 134.1 | 153.2 | 200.7 | 314.3 | 99.7 | 125.7 |
| 123 | 49.5 | 102.7 | 113.5 | 109.8 | 125.4 | 167.4 | 124.8 | 161 | 127.5 |
| 124 | 122 | 156.7 | 104.5 | 141 | 126.9 | 183.5 | 129.5 | 127.1 | 87.6 |
| 125 | 901.7 | 798.3 | 548.8 | 603.5 | 613.7 | 1170.8 | 823 | 708 | 576.3 |
| 126 | 1558.5 | 2022.3 | 1906.6 | 1073.1 | 1537.3 | 1698.3 | 1203.7 | 1574 | 734.6 |
| 127 | 67.5 | 94.2 | 121.1 | 129.3 | 177.1 | 229.6 | 142.1 | 113.2 | 149.3 |
| 128 | 270.3 | 227.2 | 174 | 236.1 | 175.5 | 523.6 | 265.8 | 219.6 | 215.4 |
| 129 | 252.6 | 119.8 | 55.2 | 205.4 | 120.9 | 238.2 | 283.6 | 118.6 | 158.2 |
| 130 | 71 | 74.9 | 81.3 | 173.4 | 64.2 | 151.7 | 101.2 | 72.2 | 206.8 |
| 131 | 99.7 | 56.1 | 56.9 | 101.9 | 71.1 | 71.4 | 85.6 | 39.6 | 410 |
| 132 | 179.1 | 167.2 | 163.5 | 226.3 | 168.3 | 291.1 | 198.8 | 168.5 | 289.6 |
| 133 | 65.3 | 135.6 | 129.8 | 134.1 | 108.6 | 90.5 | 155.9 | 90.6 | 247.7 |
| 134 | 141.8 | 67.6 | 374.3 | 220.5 | 242.9 | 331.8 | 297.9 | 192.8 | 232 |
| 135 | 323.6 | 502.2 | 986.3 | 429.9 | 398.9 | 720.6 | 576.6 | 551 | 856.1 |
| 138 | 179.5 | 85.7 | 141.7 | 111.3 | 90.4 | 263.4 | 150 | 100.7 | 95.9 |
| 139 | 106.9 | 129.9 | 131.6 | 116.9 | 120.4 | 167.8 | 111.3 | 119.2 | 214.6 |
| 140 | 1721.8 | 3036.3 | 2185.5 | 2508.8 | 4344.2 | 551 | 489.1 | 3284.4 | 2492 |

| | | | | | | | | | |
|---|---|---|---|---|---|---|---|---|---|
| 141 | 2410.6 | 3225.5 | 1665.6 | 1916.7 | 2935.3 | 1825.5 | 2844.6 | 1474.7 | 1924.7 |
| 144 | 467.5 | 2221 | 378.9 | 13755 | 448 | 595.3 | 8268.8 | 333.6 | 24504.7 |
| 145 | 184.9 | 199.7 | 201.3 | 201.7 | 182.6 | 355.7 | 205.2 | 181.5 | 193.7 |
| 146 | 199.9 | 232.9 | 156.3 | 218.2 | 193.1 | 318.3 | 215.5 | 183.7 | 234.1 |
| 147 | 39 | 23.2 | 35 | 23.8 | 39.4 | 81 | 29.4 | 8.2 | 43.7 |
| 148 | 623.9 | 168.1 | 255.4 | 231.2 | 256.6 | 443 | 311.3 | 211.5 | 208.8 |
| 149 | 298.4 | 748.7 | 946.9 | 488 | 781.4 | 682.8 | 843.3 | 449.2 | 418 |
| 150 | 271.8 | 256.3 | 118.7 | 145.6 | 239.5 | 283.8 | 224.4 | 131.5 | 178.9 |
| 151 | 386 | 739.9 | 194.8 | 394.3 | 448.1 | 199.1 | 376.1 | 165.6 | 142 |
| 153 | 101.1 | 82.9 | 117.5 | 170.7 | 94.7 | 158.3 | 93.7 | 98.7 | 124.8 |
| 154 | 696.5 | 806.1 | 188.7 | 316.4 | 89.7 | 503.2 | 343.3 | 153.6 | 615.8 |
| 155 | 126.3 | 91.9 | 134.1 | 121.4 | 87.9 | 243.6 | 235.9 | 104.5 | 96.7 |
| 156 | 119.2 | 40.6 | 68.7 | 94.7 | 77.3 | 67 | 131.6 | 62.7 | 173.3 |
| 157 | 77.5 | 165.7 | 132.1 | 100.7 | 88 | 103.8 | 133.9 | 113.1 | 166.4 |
| 158 | 30.3 | 9.6 | 1.4 | 158.3 | 97.2 | 41.9 | 159.9 | 1.2 | 1019.8 |
| 159 | 3785.9 | 3917 | 4901.3 | 3467.1 | 3669.5 | 3653.4 | 3209.2 | 3643.5 | 3005.3 |
| 161 | 90.5 | 73.4 | 50 | 2779.2 | 176 | 170.6 | 3362.6 | 1.2 | 4114.9 |
| 162 | 165.6 | 48.8 | 50.3 | 49 | 23.6 | 75.1 | 52.8 | 83.8 | 63.7 |
| 164 | 274.7 | 1048.7 | 159.5 | 2491.4 | 3031 | 446.5 | 2413.5 | 213.4 | 2997.7 |
| 166 | 5679.8 | 5894 | 6666.6 | 4629.2 | 8306.1 | 5972 | 3859.7 | 5542.7 | 4096.6 |
| 167 | 2803.8 | 865.7 | 583.6 | 1447.2 | 1517.1 | 468.1 | 1020.9 | 895.3 | 1756 |
| 168 | 664.5 | 1070.8 | 946.4 | 1307.6 | 1154.7 | 804.8 | 787.3 | 1131.5 | 1135.4 |
| 169 | 4025.8 | 9912.9 | 783 | 3510.5 | 4082.6 | 1690.6 | 3122.8 | 3606.3 | 3273.9 |
| 170 | 630.2 | 1665.5 | 1244.3 | 2308 | 1487.7 | 895.5 | 1906.2 | 884.1 | 2192.6 |
| 171 | 1597.9 | 18.2 | 1.3 | 52.3 | 123.4 | 3 | 174 | 103.7 | 50.6 |
| 172 | 648.7 | 336.4 | 566.3 | 568.5 | 546.7 | 747 | 570.3 | 569.6 | 634.3 |
| 173 | 301.5 | 351.8 | 502.4 | 419.8 | 242.7 | 82.9 | 106.8 | 377.3 | 1153.3 |
| 176 | 205 | 103.6 | 37.4 | 2372.3 | 1244.7 | 431 | 2046.7 | 1.1 | 6658.9 |
| 177 | 657.2 | 340.2 | 225.5 | 561.7 | 411 | 195 | 319.8 | 255.7 | 474.3 |
| 178 | 133.2 | 340.8 | 44 | 588.7 | 77.1 | 236.2 | 156 | 90.1 | 2026.1 |
| 179 | 114.7 | 48.7 | 65.5 | 86.7 | 56.7 | 89.4 | 59.6 | 95.5 | 53.8 |
| 180 | 85.1 | 88.5 | 71.9 | 296.6 | 369.5 | 193.3 | 269.4 | 71.5 | 700 |
| 181 | 13466.7 | 11481.6 | 14492 | 10957.2 | 9935.2 | 21897 | 10184 | 12851.6 | 7312.1 |
| 182 | 654.9 | 653.9 | 427.9 | 406.4 | 491.3 | 347.1 | 403.3 | 562.8 | 449.5 |
| 183 | 1741.8 | 1.2 | 72 | 216.6 | 1.5 | 13.4 | 127.3 | 74.7 | 60.8 |
| 184 | 210.8 | 167 | 156.2 | 4707.4 | 163.4 | 275.2 | 340.4 | 183.2 | 408.7 |
| 185 | 2742.8 | 559.8 | 331.2 | 1091.6 | 875.4 | 2220.6 | 1051 | 563.7 | 1819.8 |
| 186 | 540.1 | 408.4 | 119.6 | 379 | 511.7 | 293.6 | 378.6 | 390.8 | 334.8 |
| 187 | 461 | 78.3 | 76.6 | 73.6 | 56.4 | 324 | 75.8 | 168 | 671.7 |
| 188 | 583.1 | 57.1 | 288.4 | 239 | 128.3 | 224 | 113.7 | 356.9 | 137.6 |
| 189 | 194.8 | 29.2 | 85.5 | 119.5 | 62.4 | 143.8 | 67.8 | 131.3 | 68 |
| 190 | 123.9 | 206.8 | 248.5 | 85.4 | 260.6 | 70.6 | 194.8 | 276 | 275.5 |
| 191 | 1270.2 | 2209.5 | 1240.3 | 741.8 | 1132.9 | 3308.2 | 2393.3 | 694.6 | 828.7 |
| 192 | 119.5 | 81 | 147 | 79.8 | 63.3 | 171.5 | 100.5 | 89 | 245.3 |
| 193 | 572 | 747.2 | 2493.9 | 1776.2 | 1406.7 | 2749.4 | 771.4 | 2411.6 | 1560.3 |
| 194 | 156.3 | 196.3 | 74.6 | 184.9 | 231.7 | 72.8 | 155.2 | 181 | 247.3 |
| 195 | 308.3 | 259.1 | 115.5 | 265.8 | 182.4 | 266.7 | 269.9 | 321.4 | 172.6 |
| 196 | 8888.9 | 1967.3 | 1862.4 | 4252.6 | 4150.7 | 1524.3 | 2417.5 | 2838 | 4062.6 |
| 197 | 123.1 | 56.4 | 64.3 | 55.9 | 63.4 | 61 | 92.7 | 88.5 | 67.4 |
| 198 | 72.7 | 46.7 | 59.1 | 128.1 | 86.2 | 134.7 | 76 | 89.2 | 1231.8 |
| 199 | 715.5 | 149.9 | 77.4 | 200.9 | 99.1 | 117.1 | 93.7 | 83.2 | 58.1 |
| 200 | 436.8 | 119 | 110 | 279 | 144.7 | 161.1 | 188.8 | 132.1 | 183.8 |
| 201 | 420.4 | 136.7 | 34.5 | 76 | 82.5 | 105.4 | 714.7 | 46.9 | 48.3 |
| 203 | 216.9 | 328.6 | 478.5 | 1783 | 339.6 | 318.5 | 429.8 | 215.3 | 696.8 |
| 204 | 424.2 | 534.9 | 2224.1 | 826.2 | 1409.9 | 1071.2 | 1167.6 | 730.8 | 1179.7 |
| 205 | 5342.3 | 6991.4 | 6610.9 | 7662.4 | 6557.2 | 4601.1 | 6434.7 | 5994.9 | 5922.6 |
| 206 | 196.9 | 136.2 | 256 | 226 | 167.3 | 461.3 | 381.3 | 255.3 | 194 |
| 207 | 856.9 | 2195.1 | 498.1 | 3374 | 2428.9 | 655.5 | 1311 | 1015.2 | 1559.2 |

| | | | | | | | | |
|---|---|---|---|---|---|---|---|---|
| 208 | 394.5 | 627.5 | 228.8 | 462.5 | 481.5 | 375.3 | 249.2 | 214.1 | 366.3 |
| 209 | 4454.3 | 1325.4 | 400 | 2326.1 | 2927.3 | 297.9 | 2136.1 | 2044.2 | 4109.1 |
| 211 | 9691.9 | 10693.8 | 23350.1 | 14624.2 | 22270.2 | 30888 | 17057.7 | 24224.7 | 20568.5 |
| 212 | 572.3 | 271.2 | 156.5 | 584.8 | 425.7 | 440.5 | 458.5 | 393.6 | 829.4 |
| 213 | 67 | 114 | 61.5 | 73.5 | 71.3 | 151.9 | 87.1 | 81 | 82.8 |
| 215 | 822.1 | 2240.3 | 284 | 3771.2 | 3251.7 | 364.9 | 1154.1 | 891.6 | 1823.7 |
| 216 | 1084.8 | 1208.4 | 774.3 | 825.1 | 931.3 | 1019 | 1459.9 | 886.9 | 394.2 |
| 217 | 263.5 | 128.1 | 178 | 388.9 | 253.4 | 229.3 | 192.2 | 287.8 | 619.7 |
| 218 | 247 | 369 | 430.6 | 309.9 | 285.4 | 378.5 | 371.4 | 431.1 | 300.2 |
| 220 | 192.1 | 65.1 | 72.8 | 82.9 | 64.6 | 195.1 | 82 | 97.9 | 152.1 |
| 221 | 874.9 | 273.4 | 348.7 | 610.2 | 398.6 | 589.1 | 442 | 576.1 | 781.3 |
| 223 | 203.5 | 216.6 | 257.1 | 227.9 | 193 | 239.1 | 307.3 | 269.5 | 181.6 |
| 227 | 1491.4 | 508.8 | 973.3 | 663 | 611.5 | 1289.2 | 657.2 | 715.4 | 1113.4 |
| 228 | 479.7 | 346.7 | 305.5 | 399.5 | 432.4 | 168.3 | 334.5 | 406.8 | 398 |
| 229 | 344.9 | 179.3 | 263.5 | 202.7 | 135.8 | 258.4 | 146.7 | 307 | 178.3 |
| 230 | 408 | 173.8 | 53.4 | 230 | 72.6 | 146.6 | 89.4 | 72.6 | 1044.4 |
| 231 | 60.9 | 169.5 | 179.7 | 565.7 | 166.8 | 279.1 | 189.2 | 170.4 | 295.4 |
| 232 | 7655.5 | 69.6 | 144.4 | 522.1 | 6.3 | 156.1 | 223.7 | 143.5 | 237.3 |
| 233 | 10823.9 | 8532.5 | 7380 | 6335.2 | 4653 | 14890.4 | 6151.6 | 6357.9 | 8900.2 |
| 235 | 964.3 | 116.8 | 99.5 | 118.8 | 232 | 172.3 | 135.3 | 109.6 | 68.6 |
| 236 | 284.7 | 110.1 | 85.2 | 190 | 113.2 | 251.9 | 125 | 160.6 | 109 |
| 237 | 2149.2 | 2342.6 | 1981.7 | 1910 | 1532.6 | 1925.6 | 1630.1 | 2187 | 2001.9 |
| 238 | 1376.8 | 1774.9 | 1294.3 | 1639.9 | 1873.9 | 1331 | 1069.9 | 1075.9 | 920.9 |
| 239 | 677.1 | 102.3 | 55.7 | 598 | 88.8 | 415.3 | 790.4 | 144.5 | 446.3 |
| 240 | 910 | 202.5 | 215.3 | 1413.1 | 146.1 | 590.9 | 1856.1 | 149.8 | 658.9 |
| 241 | 1746.2 | 288.3 | 229.3 | 1845.3 | 213.4 | 1394.4 | 2581 | 292.1 | 1300.3 |
| 242 | 3459.7 | 3959.5 | 1573.1 | 930.4 | 1419.2 | 4708.4 | 2169.8 | 1081.9 | 3241.5 |
| 243 | 789.8 | 205.4 | 201.9 | 189.4 | 191.5 | 359.6 | 385.6 | 187.1 | 231.5 |
| 244 | 166.8 | 289.4 | 346.4 | 297.2 | 266.9 | 336.1 | 253.7 | 298.8 | 291.7 |
| 246 | 735.5 | 339.5 | 415.4 | 754.5 | 305.6 | 434.1 | 389.1 | 182.4 | 1612.5 |
| 247 | 152.5 | 91.8 | 114.1 | 105.7 | 117.4 | 162.4 | 111.1 | 109 | 77.5 |
| 248 | 113.4 | 311.4 | 156.1 | 827.2 | 163.8 | 295.1 | 550.6 | 57.7 | 969 |
| 249 | 546.3 | 2609.6 | 77.3 | 1171.4 | 2406.9 | 1453.1 | 2505.9 | 1690.1 | 2558.1 |
| 251 | 114.6 | 75.4 | 190.3 | 144.5 | 67.4 | 139.8 | 164.5 | 86.3 | 272.4 |
| 253 | 343.5 | 454 | 621.6 | 336.8 | 375.4 | 294.5 | 353 | 308 | 370.8 |
| 254 | 634.2 | 878.2 | 135.8 | 1836.5 | 1289.6 | 195.3 | 904.4 | 151.2 | 1561.8 |
| 255 | 104.9 | 294.1 | 41.8 | 691.5 | 87 | 372.2 | 205.6 | 116.5 | 1995.4 |
| 256 | 859.7 | 310.3 | 98.5 | 401.3 | 162.4 | 559.4 | 1488.4 | 155 | 514.5 |
| 257 | 100.3 | 86.9 | 144.3 | 100.1 | 101.5 | 226.9 | 206.1 | 125.7 | 172.8 |
| 258 | 150.3 | 110.9 | 556.9 | 31 | 96.4 | 364.7 | 435.1 | 1.3 | 56.5 |
| 259 | 1252.6 | 22.5 | 14.5 | 71.4 | 242.1 | 53.8 | 318.2 | 52.7 | 72.5 |
| 260 | 153.5 | 162.6 | 101.6 | 142.8 | 117.7 | 170.7 | 162.1 | 100.8 | 140.1 |
| 261 | 284.9 | 426 | 473.6 | 367.3 | 285.2 | 584.1 | 539.9 | 954.5 | 991.9 |
| 263 | 387.1 | 519.8 | 1024.7 | 612.1 | 575.4 | 914.8 | 292.9 | 533.1 | 500.2 |
| 263 | 443.5 | 164.6 | 135.3 | 170.4 | 102.9 | 275.3 | 155 | 114.2 | 855 |
| 265 | 418.7 | 323.5 | 784.9 | 1333.6 | 878.4 | 655 | 510.2 | 758 | 222 |
| 266 | 365.9 | 238.9 | 328.9 | 335.6 | 303.6 | 404.8 | 506.9 | 500.5 | 362.2 |
| 256 | 232 | 48.3 | 68.8 | 144.5 | 86.5 | 161 | 74.1 | 81 | 263.7 |
| 268 | 789.4 | 359.6 | 223.2 | 619.4 | 600 | 188.7 | 380.9 | 385.6 | 616.2 |
| 269 | 29.5 | 23.7 | 45.2 | 37.7 | 42.9 | 141.6 | 73.5 | 56.7 | 27.4 |
| 270 | 318.9 | 330.1 | 251.6 | 249.2 | 249.1 | 310.4 | 140.9 | 168.8 | 208.5 |
| 271 | 239.6 | 226.6 | 272.6 | 240.3 | 278.2 | 416.5 | 239.5 | 262.2 | 282.4 |
| 272 | 529.2 | 443 | 502.9 | 351.9 | 421.7 | 790.6 | 423.8 | 540.7 | 390.3 |
| 273 | 2412.2 | 538 | 5359.4 | 2677 | 2738.3 | 2513.7 | 2568.7 | 3343.1 | 6538 |
| 274 | 860.6 | 187.6 | 415.1 | 523.9 | 197.7 | 408.9 | 297.8 | 848.1 | 225.6 |
| 275 | 810.8 | 120.9 | 107.2 | 223.4 | 149.2 | 360.4 | 213.5 | 132.2 | 481.2 |
| 276 | 3000.6 | 430.1 | 824.6 | 833 | 608.9 | 992.7 | 603.1 | 628.9 | 1368.2 |
| 277 | 143.4 | 150.6 | 195.4 | 175.5 | 161.1 | 288.2 | 217.2 | 177.8 | 321.7 |

| | | | | | | | | | |
|---|---|---|---|---|---|---|---|---|---|
| 278 | 1181.4 | 13.4 | 74.4 | 130.2 | 391.9 | 174.1 | 641.2 | 103.4 | 104.3 |
| 279 | 3167.2 | 3036.4 | 3286.5 | 3373.9 | 2195.2 | 1542 | 1783.5 | 1888.5 | 2287.8 |
| 280 | 2799.6 | 4372.9 | 8233.1 | 4783.5 | 3720.7 | 5792.3 | 3596.6 | 2627.6 | 3750.3 |
| 281 | 198.3 | 45.4 | 21.1 | 878.5 | 124.3 | 131.5 | 432.7 | 26.2 | 503.4 |
| 282 | 262.4 | 214.2 | 212.7 | 210.6 | 257.2 | 367.2 | 315.7 | 214.2 | 206 |
| 283 | 200.9 | 307.7 | 203.2 | 180 | 205.1 | 205.9 | 289.5 | 199.1 | 361.3 |
| 284 | 86.1 | 79 | 44.8 | 62.9 | 58.3 | 68.8 | 54.7 | 64.2 | 67.5 |
| 285 | 87.3 | 96.6 | 120.5 | 99.3 | 90.3 | 149.5 | 127.6 | 90 | 82.7 |
| 286 | 786.2 | 1509.2 | 1245.6 | 480.5 | 1419.1 | 1355.4 | 1398.3 | 986.2 | 463.7 |
| 287 | 130.9 | 88.7 | 115.7 | 133.4 | 101 | 206.6 | 124.4 | 129.4 | 95.2 |
| 288 | 121.5 | 110.5 | 133.2 | 130.9 | 129.7 | 239.3 | 147.4 | 130.8 | 98.9 |
| 289 | 817 | 97.8 | 1.2 | 264.2 | 130.1 | 209 | 209.4 | 93.1 | 162 |
| 291 | 128.3 | 84.3 | 184.7 | 105.8 | 127.5 | 140.4 | 106.4 | 110.2 | 175.4 |
| 292 | 325.5 | 712.8 | 127.1 | 172.1 | 269.8 | 126.4 | 1240.7 | 58 | 79 |
| 293 | 169.4 | 70.3 | 44.4 | 339.3 | 157.8 | 14.6 | 329.2 | 57 | 289.7 |
| 295 | 1114.8 | 74.6 | 91 | 388.6 | 176.3 | 215.7 | 303 | 129.7 | 708.9 |
| 296 | 1100.3 | 1475.6 | 747.1 | 869.3 | 1179.1 | 893.3 | 1093.5 | 664 | 1046.4 |
| 297 | 80.1 | 103.7 | 60.5 | 51.8 | 26.9 | 86 | 54.9 | 51.9 | 60.9 |
| 298 | 262.5 | 378 | 262.4 | 322.1 | 347.3 | 553.8 | 442.5 | 361.5 | 482.8 |
| 299 | 1244.6 | 880.6 | 660.4 | 1666.7 | 1114.4 | 999.2 | 1190.8 | 835.1 | 1409.5 |
| 300 | 221.5 | 287.8 | 78.8 | 1021.2 | 396.7 | 142.9 | 552.3 | 600.2 | 1201.1 |
| 301 | 345.6 | 176.4 | 916.7 | 791.8 | 996.1 | 2261.6 | 747.6 | 1879.6 | 867.3 |
| 302 | 30.1 | 11.5 | 9.6 | 1.2 | 9.5 | 52.1 | 8.2 | 31.1 | 20.4 |
| 303 | 659.7 | 792.4 | 1951.7 | 791.9 | 1024.8 | 1077.6 | 1052.7 | 1025.2 | 591.6 |
| 304 | 338.3 | 113.8 | 145.9 | 218.3 | 159.1 | 368.5 | 315.3 | 213.9 | 279.5 |
| 305 | 126.3 | 179.5 | 829.3 | 410.1 | 205.7 | 213 | 328.4 | 168.9 | 2353 |
| 306 | 137.6 | 129.3 | 119.7 | 111.1 | 136.2 | 360.3 | 171.7 | 145.8 | 121.9 |
| 307 | 116.1 | 98.9 | 137.8 | 143.6 | 128.3 | 221.3 | 155.3 | 111.8 | 121.6 |
| 308 | 1839.8 | 1691.4 | 3877.1 | 1787.2 | 1771.6 | 990 | 1269.4 | 1210.9 | 1188.7 |
| 310 | 746.5 | 649 | 365.2 | 639.4 | 842.4 | 331.6 | 428.2 | 770.7 | 283.9 |
| 311 | 148.4 | 156.4 | 215.3 | 228.8 | 178.6 | 94.4 | 194.5 | 255.6 | 341 |
| 312 | 960.4 | 1273.3 | 387.3 | 768.1 | 1668 | 504.5 | 546.2 | 772.8 | 843.7 |
| 313 | 116.8 | 110.3 | 118.2 | 691.9 | 301.6 | 156.3 | 349.8 | 120.8 | 1297 |
| 314 | 813.7 | 765 | 104 | 454.5 | 738.3 | 343 | 480.5 | 177.9 | 358 |
| 315 | 511.2 | 104.2 | 79.8 | 249.6 | 127.6 | 186.6 | 134.3 | 235.8 | 364 |
| 316 | 333.7 | 362.7 | 292.4 | 420 | 274.7 | 331.6 | 438.9 | 346.4 | 445.6 |
| 317 | 135.8 | 140 | 147.1 | 152.2 | 131.7 | 314.3 | 145.4 | 127.2 | 161.3 |
| 318 | 177.4 | 80.7 | 163.7 | 110.3 | 146.9 | 54.5 | 212.4 | 38.7 | 48.6 |
| 319 | 137.7 | 159.9 | 208.9 | 347.1 | 302.7 | 212.6 | 492.3 | 225 | 299.2 |
| 320 | 786.7 | 569.4 | 419.9 | 576.6 | 664 | 261.9 | 358.7 | 564.8 | 362.1 |
| 321 | 430.1 | 418.3 | 554.5 | 345.7 | 495.1 | 766.8 | 520.2 | 522.1 | 417.8 |
| 322 | 313.5 | 258.9 | 206 | 327.9 | 350.1 | 237.1 | 317.3 | 284.4 | 302.2 |
| 323 | 1640.1 | 3893.2 | 6623 | 4933.3 | 4083.6 | 4146.3 | 4123.6 | 2877.7 | 8765.3 |
| 324 | 191.6 | 52.6 | 89.7 | 451.6 | 184.5 | 161.8 | 224.9 | 94.8 | 485.6 |
| 325 | 252.3 | 394.8 | 205 | 336.6 | 342.2 | 306 | 382.9 | 200 | 472.4 |
| 326 | 6503.5 | 4515.1 | 5659.1 | 3116.9 | 4025.8 | 4887.6 | 5753.4 | 5207.4 | 3124.3 |
| 327 | 32.7 | 114.3 | 47.6 | 44.2 | 34.9 | 28.1 | 45.1 | 32.7 | 122 |
| 328 | 572.1 | 311.6 | 141.9 | 444 | 207.9 | 70.7 | 242.6 | 305.2 | 432.5 |
| 329 | 55.7 | 56.6 | 98.4 | 87.9 | 54.4 | 253.9 | 95 | 87.8 | 62.1 |
| 330 | 248.9 | 21.4 | 18.4 | 33.3 | 71.6 | 70.8 | 50.4 | 26.1 | 41.6 |
| 331 | 262.5 | 211 | 258.2 | 272.6 | 238.9 | 488.3 | 289.6 | 242.5 | 276.5 |
| 333 | 527 | 469.3 | 712.6 | 701.7 | 769.3 | 702.2 | 868.3 | 980.3 | 1069.6 |
| 334 | 178.8 | 178 | 143.4 | 202.5 | 157.4 | 217.1 | 144 | 129.4 | 147.4 |
| 335 | 109.7 | 80.4 | 65 | 59.9 | 71 | 237.8 | 69.3 | 69.9 | 140.5 |
| 336 | 478.1 | 1866 | 439.4 | 413 | 717.7 | 515.6 | 725.6 | 440.9 | 491.5 |
| 337 | 250.5 | 86.1 | 605.5 | 216 | 112.1 | 769 | 301 | 201.7 | 436.9 |
| 338 | 207.4 | 381.7 | 182.5 | 222.8 | 212.2 | 291.2 | 398.5 | 122.7 | 422.9 |
| 339 | 491.6 | 370.5 | 242.7 | 371.6 | 357.1 | 291.6 | 301 | 329.6 | 333.1 |

| | | | | | | | | | |
|---|---|---|---|---|---|---|---|---|---|
| 340 | 262.2 | 228.9 | 221.7 | 277.5 | 209.8 | 433.8 | 234.6 | 244 | 238.8 |
| 341 | 553.7 | 859.2 | 680.7 | 749.1 | 833.5 | 945.2 | 877.8 | 1040.5 | 836.3 |
| 343 | 82.4 | 129.6 | 166.7 | 112.2 | 227 | 121.6 | 417.5 | 99.3 | 261.1 |
| 344 | 1494.7 | 1234.1 | 923 | 1174 | 1290.7 | 695.2 | 1049.8 | 1243.6 | 776.1 |
| 345 | 229.2 | 243.3 | 208 | 263.4 | 197.6 | 424 | 253.1 | 228.2 | 190 |
| 347 | 2522.7 | 2002 | 1475.1 | 3154.4 | 2419.4 | 3643.7 | 3353.4 | 2213 | 2257.1 |
| 348 | 5357.3 | 8676.5 | 4846.9 | 7151 | 6595.2 | 9416.2 | 8225.1 | 8754.7 | 7625.2 |
| 349 | 199 | 234.6 | 342.6 | 366.5 | 251 | 342.4 | 222.3 | 330.6 | 366.8 |
| 350 | 425.9 | 228.7 | 186 | 194.8 | 307.5 | 295.9 | 344.4 | 186.6 | 478.3 |
| 351 | 639.7 | 438.8 | 120.8 | 247.4 | 113.3 | 139.6 | 323 | 154.5 | 311.5 |
| 352 | 383.1 | 438.1 | 904.1 | 394.6 | 424.3 | 618.5 | 412.3 | 428.9 | 250.7 |
| 353 | 440.7 | 413.1 | 270.3 | 281.2 | 203 | 315.4 | 237.6 | 265.9 | 206.9 |

3e

| SEQ ID NO: | 871TT | 879TT | 881TT | 882TT | 883TT | 884TT | 885TT | 886TT |
|---|---|---|---|---|---|---|---|---|
| 354 | 116.3 | 981.8 | 502.2 | 324.4 | 156.7 | 786.8 | 376.6 | 642.7 |
| 355 | 66.1 | 8420.7 | 2645.1 | 6152 | 13553.8 | 2564.7 | 3412.8 | 12344.6 |
| 356 | 338.5 | 1495.5 | 623.7 | 501.4 | 743.6 | 903.3 | 1811.4 | 741.6 |
| 357 | 443.4 | 1347.3 | 566 | 608.2 | 679.7 | 484.6 | 1559 | 983.6 |
| 358 | 700.6 | 1921.2 | 2381.5 | 2373.2 | 1674.3 | 2027.6 | 3756.9 | 1590.4 |
| 359 | 376 | 185.1 | 264.6 | 206.9 | 308.2 | 331.9 | 281.8 | 247.9 |
| 362 | 655.5 | 1294.9 | 729.9 | 1088.1 | 864.4 | 1725.1 | 1214.9 | 1246.6 |
| 360 | 266.2 | 611.8 | 464.8 | 543.6 | 452.1 | 537.3 | 693.4 | 474.9 |
| 361 | 152.3 | 149.9 | 338.7 | 186.5 | 167.3 | 256.5 | 235.5 | 312.1 |
| 363 | 570.7 | 493.8 | 575.1 | 513.3 | 517.2 | 615.3 | 759.7 | 454.4 |
| 1 | 130.2 | 622.4 | 1944.7 | 1584.5 | 1981.6 | 1283.3 | 420.9 | 1894.3 |
| 2 | 411.7 | 1993.4 | 3797.1 | 2512.7 | 4511.4 | 2897.7 | 1254 | 3862.9 |
| 7 | 372.9 | 193.8 | 680.5 | 596.1 | 614.8 | 129.5 | 2.7 | 504.2 |
| 8 | 2363.3 | 511.5 | 342.5 | 363.9 | 358 | 497.4 | 221.6 | 355.6 |
| 9 | 381.6 | 1198.7 | 1487.5 | 781.6 | 985.4 | 2024.6 | 3933.7 | 1448.2 |
| 10 | 171.5 | 160.4 | 133.2 | 137.6 | 129.1 | 154.3 | 182.4 | 107 |
| 11 | 324 | 858.2 | 937 | 906.4 | 953.3 | 1879.7 | 1481 | 2251.2 |
| 13 | 126.1 | 303.3 | 531.1 | 319.9 | 664.9 | 496 | 223.2 | 472.7 |
| 14 | 295.2 | 932.1 | 1713.8 | 1521.7 | 2129.6 | 2134.5 | 1840.1 | 2225.4 |
| 15 | 95.6 | 2064.6 | 1333.1 | 540.9 | 265.7 | 569.7 | 709.8 | 1795.9 |
| 16 | 511.2 | 3919.7 | 2909.7 | 3131.8 | 3773.6 | 3125.2 | 3619.3 | 3331.5 |
| 17 | 214.6 | 800.5 | 1475.1 | 1075.3 | 1483.6 | 1162 | 1482.8 | 1281.8 |
| 19 | 198 | 274.5 | 768.1 | 268.8 | 208 | 509.4 | 276.1 | 570.1 |
| 20 | 1988.3 | 739.3 | 319.5 | 316.5 | 192 | 142.3 | 242.8 | 575.4 |
| 21 | 34.6 | 2492 | 1542.8 | 2495.1 | 1851.5 | 2668.6 | 1631.9 | 2770.3 |
| 22 | 1078.1 | 4211.3 | 4116 | 3543.7 | 3242.1 | 4723.4 | 3467.8 | 4881.8 |
| 23 | 1.1 | 825.2 | 1432.3 | 370.9 | 163.9 | 1192.4 | 221.9 | 1517 |
| 24 | 74.6 | 391.1 | 433 | 388.2 | 211.7 | 258.7 | 300.5 | 572.7 |
| 26 | 48.4 | 921.9 | 4466.3 | 587.5 | 349.2 | 1513.7 | 644.9 | 1870.3 |
| 27 | 495.1 | 654.3 | 1266.2 | 1076 | 1461.5 | 696.9 | 334.7 | 1531.4 |
| 29 | 381.5 | 152.8 | 91.8 | 35.9 | 162.7 | 135.1 | 152 | 238.5 |
| 30 | 31.3 | 686.6 | 444 | 223.3 | 222 | 321.8 | 356.5 | 825.6 |
| 31 | 635.6 | 18297.7 | 14048.6 | 8744.1 | 15394.7 | 17798.2 | 16472.5 | 12208.2 |
| 33 | 439.9 | 7923.9 | 4124.5 | 3804.6 | 3932.5 | 2361.7 | 3149.1 | 7173.6 |
| 34 | 3213.6 | 825.9 | 1104.9 | 697 | 285.8 | 603.4 | 436.7 | 1025.5 |
| 35 | 22.6 | 2143.6 | 1770.3 | 1187.9 | 1220.4 | 1620.3 | 1651.3 | 529 |
| 36 | 492.1 | 322.5 | 1635.1 | 387.3 | 554.6 | 818.8 | 268.4 | 1021.4 |
| 37 | 429.6 | 140.1 | 245.5 | 191.1 | 224.1 | 241.8 | 207.1 | 178.5 |
| 38 | 237.9 | 1844.2 | 2220.8 | 794.4 | 644.7 | 984 | 292.1 | 3477.4 |
| 40 | 451.5 | 617.3 | 495.8 | 1186.4 | 725.4 | 800.6 | 880.8 | 1036.1 |

| | | | | | | | | |
|---|---|---|---|---|---|---|---|---|
| 41 | 252.1 | 1258.8 | 943 | 4956.1 | 2394.2 | 566.6 | 987.6 | 3025.4 |
| 42 | 579.4 | 532.8 | 258.1 | 1104 | 396.5 | 335.9 | 491.3 | 504 |
| 43 | 1029 | 384.9 | 273.6 | 344 | 416.5 | 403.9 | 517.2 | 325.7 |
| 44 | 565.3 | 1802.4 | 1501.6 | 1596.1 | 1100.9 | 1275.9 | 1583.2 | 1199 |
| 45 | 1500.8 | 1346.9 | 1175.4 | 1573.6 | 2053.9 | 971.8 | 332 | 1783.9 |
| 46 | 307.7 | 539 | 343 | 380.7 | 553.4 | 414.6 | 398.2 | 340.8 |
| 47 | 67.8 | 1213 | 521.9 | 742.8 | 498.1 | 507 | 1389.1 | 810.8 |
| 48 | 6.2 | 645.9 | 561.5 | 325.6 | 92.6 | 189.4 | 537.5 | 190.6 |
| 49 | 192.1 | 1573.1 | 2319.6 | 1719.2 | 1210.4 | 6469.1 | 1691.6 | 1799.3 |
| 50 | 2 | 1030.9 | 954.7 | 379.1 | 309.7 | 799.1 | 597.4 | 2116.3 |
| 51 | 772.5 | 259.7 | 304 | 170.3 | 108.1 | 1357.1 | 408.8 | 394.9 |
| 52 | 193.2 | 298.8 | 343.6 | 347.9 | 276 | 289.4 | 348.2 | 335.9 |
| 53 | 153.5 | 1632.6 | 2144.7 | 2293.9 | 2026.5 | 1732.8 | 446.5 | 1982.9 |
| 54 | 809.7 | 1147.3 | 1387.6 | 2014.6 | 1631.7 | 1524.6 | 1231.9 | 2093.8 |
| 55 | 147.7 | 86.2 | 101.7 | 108.3 | 85.2 | 103.3 | 116.6 | 62.5 |
| 56 | 130 | 94.8 | 277.7 | 121.8 | 341.8 | 98.5 | 180.9 | 241.4 |
| 57 | 777.3 | 1976.6 | 14161.4 | 3909.2 | 3643.6 | 667.8 | 1053.7 | 5922.8 |
| 58 | 1225.7 | 5418.7 | 14855.8 | 6952.4 | 4309.6 | 3516.6 | 6571.6 | 9242 |
| 59 | 271.6 | 3644.3 | 3754.4 | 4801.4 | 3817.3 | 6657.4 | 4444.2 | 3360.8 |
| 60 | 159.2 | 218.9 | 601.1 | 529.4 | 311.4 | 171.9 | 231.9 | 506.7 |
| 61 | 138.9 | 159.4 | 118.4 | 73.6 | 104.8 | 116.1 | 164.5 | 81.1 |
| 62 | 3244.1 | 5818 | 1646.1 | 2645.7 | 3666.2 | 3358 | 2535.7 | 4029.7 |
| 63 | 157.2 | 628.9 | 667.9 | 804.6 | 516.3 | 778 | 703.2 | 578.3 |
| 64 | 994.5 | 119.1 | 251.9 | 202.5 | 258.2 | 236.6 | 151.1 | 722.1 |
| 65 | 35.2 | 209.3 | 234.7 | 217.1 | 113.7 | 118.8 | 146.7 | 305.4 |
| 66 | 437.5 | 2105.5 | 1849.4 | 2646.8 | 2322.7 | 1754.7 | 1801.8 | 1830.2 |
| 67 | 13299.5 | 26691.3 | 29363.4 | 21948.4 | 30861.2 | 27520.3 | 32791 | 27456.3 |
| 68 | 1527.1 | 379.2 | 840.8 | 286.8 | 238.8 | 383.7 | 53.6 | 2104.6 |
| 69 | 5084.1 | 1418.5 | 1727.3 | 964.1 | 782.1 | 2036.1 | 478 | 3532.1 |
| 70 | 9067.8 | 5289.8 | 5151.5 | 5378.2 | 4923.7 | 7248.8 | 5765.7 | 6685.7 |
| 71 | 356.7 | 3218.2 | 6059.4 | 4739.2 | 7588 | 3114 | 2518 | 4665.5 |
| 72 | 126.9 | 2024.9 | 3174.2 | 2185.2 | 2596.3 | 2512.4 | 2145.9 | 1991.3 |
| 73 | 163.4 | 917.4 | 942.3 | 785.8 | 642.5 | 1021.7 | 1109.2 | 1059.2 |
| 75 | 234.3 | 480.2 | 1234.8 | 2124.7 | 1532.8 | 329.3 | 830.5 | 2611.1 |
| 76 | 1819.9 | 1037.3 | 1473 | 1162.8 | 2498.4 | 959.8 | 921.6 | 1764.5 |
| 77 | 5259 | 3101.8 | 1035.9 | 1814.1 | 885.5 | 1999.1 | 3528.7 | 1319.6 |
| 78 | 568.7 | 520.5 | 454.4 | 245.9 | 225.2 | 250.6 | 256.7 | 390.8 |
| 79 | 402.6 | 303.3 | 288.4 | 416.4 | 496.9 | 377.6 | 309.8 | 241.7 |
| 80 | 141.1 | 1486.2 | 1527.9 | 1160.9 | 747.9 | 1195.7 | 1756.4 | 792.8 |
| 81 | 189.9 | 13371 | 7841.3 | 9074.6 | 16493.4 | 8121 | 5097.9 | 11272.1 |
| 82 | 435.3 | 141.8 | 224.9 | 197.1 | 249.7 | 31 | 6.8 | 170.9 |
| 84 | 71.2 | 138.6 | 259.2 | 151.7 | 138.9 | 415.1 | 303 | 326.8 |
| 85 | 187.8 | 1315 | 612.4 | 336.4 | 240.8 | 318.3 | 399.8 | 1550.2 |
| 86 | 93.5 | 90.2 | 120.7 | 294.3 | 724.1 | 302.7 | 89.1 | 261.7 |
| 87 | 539.5 | 1122.1 | 581.5 | 1395.1 | 1476 | 1619.4 | 1043.7 | 2328.9 |
| 88 | 3161.6 | 561.1 | 1162.2 | 632.3 | 466 | 750.6 | 597.3 | 1050.6 |
| 89 | 317.9 | 237.2 | 318.3 | 196.3 | 337.5 | 343.7 | 255.7 | 239.7 |
| 90 | 111.8 | 266.6 | 953.6 | 334.9 | 123.2 | 226 | 325.2 | 129.6 |
| 91 | 1023.4 | 3728.1 | 4506.6 | 4033.4 | 2043.2 | 2095 | 2191.9 | 3082.3 |
| 92 | 2374.9 | 4865.1 | 6296.6 | 8454.9 | 11642.6 | 11587.7 | 9943.6 | 10568 |
| 93 | 103.8 | 133.1 | 109.8 | 121.6 | 41.7 | 69.9 | 17.1 | 166 |
| 94 | 59.2 | 296.8 | 784.9 | 944.1 | 600.5 | 776.8 | 481.1 | 1257.8 |
| 95 | 69 | 126.3 | 450.5 | 453.8 | 310.8 | 189.3 | 165.4 | 142.4 |
| 96 | 147.8 | 682.4 | 387.8 | 387.3 | 404.3 | 357.9 | 565.3 | 420.4 |
| 97 | 204.3 | 883.1 | 454.1 | 708.8 | 421.4 | 468 | 686.4 | 732.8 |
| 98 | 728.4 | 10.5 | 42.6 | 72.2 | 43.5 | 29.7 | 53 | 36.4 |
| 99 | 210.6 | 821.1 | 369.4 | 814.2 | 595.6 | 485.9 | 696.6 | 541.1 |
| 100 | 995.9 | 102.8 | 3585.7 | 89.5 | 34.3 | 2798.5 | 112 | 3456 |

| | | | | | | | | |
|---|---|---|---|---|---|---|---|---|
| 101 | 133.4 | 146.6 | 270.3 | 174.9 | 310.3 | 198.2 | 121.7 | 192.7 |
| 102 | 190.6 | 110.7 | 244 | 152.9 | 172.3 | 180.7 | 158 | 123.8 |
| 103 | 379.6 | 430.7 | 535.3 | 399.6 | 560.3 | 572.8 | 586.9 | 406.8 |
| 104 | 252 | 69.6 | 93.2 | 189.4 | 28.6 | 67.4 | 16.2 | 92.3 |
| 105 | 363.6 | 168.9 | 116.4 | 217.8 | 70.2 | 146.4 | 118.2 | 171.8 |
| 106 | 442.3 | 95.1 | 136.6 | 154 | 56.3 | 79.8 | 12.9 | 109.4 |
| 107 | 301 | 538.7 | 356.6 | 408.6 | 301.5 | 322.4 | 420.8 | 596.8 |
| 108 | 255.8 | 236.3 | 168 | 137.3 | 106.8 | 179.7 | 237 | 170.7 |
| 109 | 169.9 | 1482.1 | 544.7 | 1597 | 294.1 | 1205.6 | 1691 | 683.9 |
| 110 | 322.7 | 1903.7 | 1404.2 | 4278.2 | 691.9 | 2791.8 | 2817.9 | 2504.6 |
| 111 | 252.7 | 25.4 | 233.3 | 24.4 | 63.4 | 101.2 | 47.1 | 118.3 |
| 112 | 633.2 | 1314.2 | 1405.6 | 2124.5 | 1859.3 | 1085.3 | 587.6 | 1601.9 |
| 113 | 164.4 | 330.6 | 333.5 | 268.5 | 218.6 | 285.2 | 237.3 | 350.6 |
| 114 | 88.6 | 309.3 | 1164.6 | 333.2 | 513.7 | 630.7 | 664.7 | 778.3 |
| 115 | 326.5 | 1299.5 | 1101.1 | 1198.8 | 1051.2 | 817.4 | 1102.2 | 1084.6 |
| 116 | 252.3 | 360.5 | 666.1 | 1347.3 | 817.6 | 327.9 | 554.6 | 825.9 |
| 117 | 42.1 | 101.1 | 493.9 | 114.6 | 74 | 399.7 | 133.8 | 234.5 |
| 118 | 1176.7 | 116.7 | 300.6 | 130.5 | 200.8 | 470.8 | 207.3 | 112.5 |
| 119 | 98.3 | 184.2 | 281.1 | 245.5 | 317.6 | 213 | 191.2 | 336 |
| 120 | 99.5 | 98.8 | 155.8 | 124.7 | 95.6 | 98.9 | 123.9 | 78.9 |
| 121 | 234.8 | 2992.4 | 4320.2 | 4055.8 | 3020 | 5336.5 | 3158.4 | 5803.1 |
| 122 | 645.9 | 446.7 | 248.2 | 356.4 | 312.1 | 428.7 | 329.5 | 346.1 |
| 123 | 300.8 | 313.4 | 352.6 | 504.1 | 413.5 | 386.8 | 444 | 417.5 |
| 124 | 4390.2 | 96.1 | 118.6 | 125.8 | 161.7 | 134.4 | 182.6 | 94.9 |
| 125 | 1484.8 | 805.4 | 27732 | 982.2 | 7375.5 | 984.2 | 1310.9 | 807.1 |
| 126 | 278.6 | 56.9 | 192.9 | 24.9 | 31.8 | 77.8 | 104.9 | 66.8 |
| 127 | 114.5 | 332.1 | 225.5 | 171.6 | 237.5 | 193.8 | 339.3 | 225.1 |
| 128 | 318.6 | 181.2 | 386.4 | 213 | 69850.3 | 108521.8 | 356.1 | 93954.5 |
| 129 | 176.1 | 1916.3 | 1120.7 | 2375.9 | 2049.1 | 1464.3 | 1602.6 | 1660 |
| 130 | 114.6 | 735.8 | 381 | 595.7 | 599.3 | 446.9 | 434.4 | 456.6 |
| 131 | 236.1 | 156.9 | 297.1 | 426.8 | 385.2 | 268.2 | 149.3 | 279.8 |
| 132 | 169.8 | 303.3 | 464.3 | 423.7 | 368.6 | 256 | 428.6 | 304.5 |
| 133 | 102.5 | 245.3 | 224.6 | 394 | 186.7 | 265.2 | 300 | 269.3 |
| 134 | 175.1 | 516.6 | 316.3 | 534.3 | 524 | 626.6 | 679.7 | 687.4 |
| 135 | 170 | 964.7 | 971.6 | 756.7 | 562.2 | 683.1 | 1544.5 | 931 |
| 138 | 145.9 | 173.1 | 201.3 | 127.5 | 189.1 | 187.4 | 173.9 | 166 |
| 139 | 66.8 | 286.4 | 170 | 167 | 270.8 | 159 | 281 | 340.1 |
| 140 | 26 | 16.5 | 135.6 | 163.6 | 1.2 | 183.8 | 98.2 | 1064.1 |
| 141 | 407.3 | 178 | 380.3 | 594.8 | 275 | 1127.7 | 586.1 | 346.3 |
| 144 | 370.2 | 23200.8 | 7070.4 | 20031.2 | 20055.7 | 5695.1 | 21008 | 16416.4 |
| 145 | 203 | 197.5 | 215.6 | 241.1 | 234 | 295.8 | 284.7 | 197.3 |
| 146 | 299.6 | 606.7 | 853.8 | 1054.9 | 249.3 | 683.3 | 966.5 | 411.5 |
| 147 | 34.5 | 138.1 | 361.4 | 219.1 | 120.1 | 69 | 110.2 | 51.6 |
| 148 | 200.3 | 1072.9 | 641.5 | 371.8 | 309.3 | 1298 | 734.4 | 563.6 |
| 149 | 147.1 | 811.8 | 330.1 | 243.5 | 389.9 | 584.1 | 960.6 | 374.5 |
| 150 | 243.6 | 194.2 | 614.8 | 339.8 | 227.4 | 390.5 | 510.2 | 508.8 |
| 151 | 103.4 | 200.9 | 536 | 659.6 | 1097.9 | 831.2 | 572.5 | 1463 |
| 153 | 102.7 | 134.4 | 117.8 | 319.9 | 659.8 | 147 | 158.2 | 276.8 |
| 154 | 64.7 | 5340.9 | 7684.3 | 4496.6 | 6819 | 3595.6 | 1313.4 | 7444 |
| 155 | 155.1 | 604.1 | 224.8 | 527.6 | 443.5 | 980.8 | 352 | 708.4 |
| 156 | 117.9 | 162.1 | 382.4 | 213.5 | 503.7 | 174 | 219.3 | 257.1 |
| 157 | 134.1 | 215.4 | 212.5 | 227.7 | 257.4 | 258.4 | 309.6 | 331.4 |
| 158 | 857.5 | 149.9 | 598.8 | 544.5 | 850.2 | 506.6 | 301.4 | 728.3 |
| 159 | 1385.8 | 1366 | 1517.3 | 1101.7 | 1387.1 | 1799.8 | 2020.5 | 1476 |
| 161 | 950.1 | 6758.6 | 7657.2 | 10885 | 16984 | 4705.2 | 7562.3 | 6065.7 |
| 162 | 12.5 | 256.2 | 584.1 | 235.7 | 456.1 | 369.3 | 231.2 | 451.5 |
| 164 | 47.5 | 347.7 | 1040.5 | 4140.2 | 1526.9 | 2205.5 | 1430.7 | 7468.9 |
| 166 | 4175.2 | 1086.4 | 1301.1 | 700.5 | 641.4 | 1360.7 | 585.8 | 1191.3 |

| | | | | | | | | |
|---|---|---|---|---|---|---|---|---|
| 167 | 545.5 | 254.9 | 1335.1 | 355.9 | 1787.4 | 118.5 | 231.5 | 771.1 |
| 168 | 140.6 | 1535.5 | 1213 | 549.9 | 594.1 | 1616.8 | 2190.3 | 639.2 |
| 169 | 582.9 | 12292.6 | 13216.5 | 15240.1 | 14867.1 | 7648.8 | 9941.3 | 13159.9 |
| 170 | 857.8 | 2119.5 | 2344.7 | 3361.9 | 2260.9 | 2642.5 | 1920 | 3323.2 |
| 171 | 1.1 | 1063.9 | 967.8 | 79.8 | 105 | 514 | 339.5 | 801 |
| 172 | 489.3 | 575.3 | 552.3 | 483.9 | 608.6 | 582.7 | 664.1 | 514.2 |
| 173 | 168.3 | 410.3 | 1713.9 | 878.7 | 690.8 | 1123.5 | 254.3 | 2373.9 |
| 176 | 50.7 | 173.8 | 993.1 | 155.4 | 1107.2 | 1814.1 | 400.9 | 1423.1 |
| 177 | 610.5 | 234 | 350.8 | 201.7 | 455.2 | 147.8 | 113.6 | 315.4 |
| 178 | 670.2 | 457.3 | 1924 | 1409.8 | 1033.8 | 1365.3 | 281.5 | 700.6 |
| 179 | 188.9 | 540.8 | 1136 | 1826.7 | 836.8 | 964.6 | 165.7 | 1383.2 |
| 180 | 208.1 | 750.5 | 880.8 | 997.3 | 460.6 | 628.8 | 717.2 | 618.1 |
| 181 | 9102.8 | 4805.7 | 6228.9 | 4111.8 | 3157.6 | 5112.8 | 5426.8 | 4235.7 |
| 182 | 244.4 | 524.8 | 434.5 | 395.3 | 395.9 | 340.8 | 570.4 | 455 |
| 183 | 1.1 | 286.3 | 920.2 | 156 | 69.6 | 540.1 | 211.4 | 691 |
| 184 | 202.5 | 365.3 | 361.1 | 2170.5 | 3042.7 | 341.2 | 479.3 | 909.8 |
| 185 | 707.4 | 2338.2 | 5269.7 | 5295.8 | 3116 | 6542.9 | 3238.5 | 6677.1 |
| 186 | 1338.6 | 98.2 | 262.6 | 165.5 | 183.3 | 170.6 | 131.9 | 218.6 |
| 187 | 301.2 | 421.4 | 792.2 | 247.6 | 123.3 | 291.1 | 366.9 | 720.2 |
| 188 | 54.4 | 507.9 | 399.8 | 687.8 | 469.2 | 348.4 | 605.1 | 684.6 |
| 189 | 68.7 | 209 | 287.2 | 379.3 | 305.1 | 161.4 | 160.7 | 404.9 |
| 190 | 199.8 | 242.2 | 483.5 | 455.5 | 631.9 | 241 | 71.1 | 375.7 |
| 191 | 1726.7 | 4039 | 3532.7 | 2210.1 | 1524.2 | 4609 | 6889.4 | 3737.3 |
| 192 | 89.6 | 89.6 | 157.2 | 211.4 | 92.6 | 233.7 | 145.8 | 105.4 |
| 193 | 85 | 216.9 | 219.4 | 119.4 | 69.2 | 392.2 | 176.5 | 239.5 |
| 194 | 49.9 | 282 | 251.7 | 297.8 | 199.9 | 135 | 172.5 | 243.3 |
| 195 | 712.1 | 117.7 | 174.3 | 152.9 | 85.3 | 174.3 | 73.7 | 152.1 |
| 196 | 2873.3 | 845.8 | 1650.6 | 1499.5 | 1608.9 | 962 | 584.5 | 1404 |
| 197 | 483.3 | 253.5 | 190.9 | 146 | 112.4 | 139 | 144.8 | 141.2 |
| 198 | 126.6 | 4231 | 1176.9 | 1159.9 | 1263.8 | 999 | 925 | 2035.1 |
| 199 | 87.2 | 9967.3 | 4014.3 | 8315.9 | 19492.1 | 4175.4 | 4540.2 | 17229.4 |
| 200 | 344.7 | 460.3 | 1008.1 | 823.2 | 437.1 | 394.5 | 655.2 | 945.4 |
| 201 | 122.7 | 1085.1 | 434.8 | 378.2 | 229.8 | 766.3 | 506.3 | 515.7 |
| 203 | 678.5 | 1088.3 | 1322.9 | 1133.7 | 3167.7 | 436.2 | 563 | 3185.7 |
| 204 | 634.2 | 3003.4 | 1397.7 | 1339.9 | 1300.3 | 1299.2 | 2900.2 | 1073 |
| 205 | 946.3 | 5589.7 | 3415.4 | 3931.9 | 4602.2 | 5034.5 | 4943.8 | 6059.1 |
| 206 | 278.8 | 299.7 | 268.4 | 293.8 | 350.2 | 398.3 | 635.4 | 241.1 |
| 207 | 125.7 | 79.6 | 212.3 | 46.6 | 106.6 | 58.1 | 57.3 | 679.7 |
| 208 | 5.4 | 3861.8 | 679.2 | 3099.7 | 462.3 | 711 | 799 | 604.6 |
| 209 | 648.2 | 26.6 | 610.8 | 71.6 | 104.3 | 77.3 | 72 | 161.4 |
| 211 | 5817.8 | 134.3 | 575.9 | 84.6 | 135.1 | 1200.4 | 233.2 | 1507.1 |
| 212 | 1458.6 | 689.4 | 1135 | 1092.6 | 988.6 | 986.3 | 460.6 | 1341.5 |
| 213 | 7576.6 | 117.6 | 121.9 | 100.6 | 96.3 | 151.6 | 183.1 | 136.2 |
| 215 | 71.8 | 73.2 | 323.2 | 71.1 | 161.2 | 100.5 | 30.8 | 676.7 |
| 216 | 415.9 | 276.5 | 268.1 | 173.7 | 199.3 | 277.1 | 434.2 | 282.3 |
| 217 | 78.7 | 959.5 | 2746.7 | 1024.9 | 2616.6 | 671.5 | 359.6 | 850.8 |
| 218 | 268.3 | 172.1 | 161.1 | 189.2 | 123.2 | 160.7 | 128.5 | 186.8 |
| 220 | 134.5 | 72.8 | 184.9 | 96.1 | 84.3 | 650.4 | 100.4 | 81.9 |
| 221 | 207.5 | 1438 | 1268.5 | 2111.3 | 963.7 | 1155.3 | 1048.4 | 1581.4 |
| 223 | 308.1 | 673.6 | 497.3 | 535.5 | 748.5 | 534.7 | 710.2 | 869.9 |
| 227 | 474 | 3740.6 | 3123.8 | 1451.8 | 740.1 | 1207.8 | 1221.4 | 3703.7 |
| 228 | 553.9 | 202.8 | 342.4 | 247.9 | 322.9 | 245.3 | 315.9 | 260.5 |
| 229 | 978.6 | 365 | 204.1 | 229.3 | 104.9 | 176.7 | 197.5 | 149.4 |
| 230 | 157 | 4381.3 | 4364.6 | 3469 | 4117.2 | 2984.9 | 4092.3 | 2935.6 |
| 231 | 299.2 | 338.1 | 519.6 | 436.9 | 1304.6 | 261.4 | 183.1 | 1209.9 |
| 232 | 1.2 | 479.7 | 2967.9 | 397.7 | 283.8 | 1161.6 | 447.9 | 1442.7 |
| 233 | 1408.3 | 17920.3 | 13126.5 | 7241.8 | 19318.8 | 13487.2 | 18348.9 | 15152.5 |
| 235 | 22.6 | 659.3 | 624.9 | 172.2 | 155.8 | 342.8 | 152.7 | 485.3 |

| | | | | | | | | |
|---|---|---|---|---|---|---|---|---|
| 236 | 244.8 | 153.5 | 397.3 | 304.6 | 449.7 | 331.3 | 200.6 | 282.5 |
| 237 | 841.8 | 1675 | 2348.2 | 1931.5 | 1633.1 | 2253.2 | 749.1 | 1687.1 |
| 238 | 606.8 | 847.7 | 670.6 | 582.6 | 625.6 | 723.4 | 726.5 | 667.2 |
| 239 | 455.9 | 304.4 | 1179.8 | 492.8 | 201.3 | 464.9 | 311.9 | 376.9 |
| 240 | 756.3 | 795.3 | 1590.4 | 747.3 | 330.3 | 1193.3 | 846 | 709.6 |
| 241 | 1604.8 | 957.3 | 2984.8 | 1412.5 | 619.3 | 2269.1 | 981.8 | 1321 |
| 242 | 13722.5 | 46283.3 | 32477.3 | 33142.3 | 38026.7 | 42087.5 | 49249 | 39172.5 |
| 243 | 322.6 | 586.6 | 483.3 | 483.6 | 499.4 | 723.7 | 534 | 603.1 |
| 244 | 80.8 | 1208.5 | 771.3 | 1636.9 | 1032.2 | 928.2 | 507.9 | 553.8 |
| 246 | 167.7 | 2506.3 | 1683.7 | 2500.3 | 1675.8 | 557.3 | 1133.6 | 1677.5 |
| 247 | 116.8 | 115.5 | 128.4 | 104.8 | 125.8 | 135.7 | 147.3 | 96.5 |
| 248 | 309.3 | 1728.9 | 1916.3 | 3434.3 | 3874.7 | 1004.6 | 2401.7 | 5089.3 |
| 249 | 432 | 681.6 | 804.8 | 479.8 | 417.3 | 1208.2 | 1265.1 | 742.5 |
| 251 | 134 | 671 | 368.8 | 2493.8 | 784.3 | 1646.7 | 587.9 | 1794.9 |
| 253 | 375.8 | 544.2 | 623.3 | 589.2 | 333.1 | 383.4 | 859.9 | 310.8 |
| 254 | 716.8 | 989.8 | 1029.3 | 1067.7 | 790.5 | 817 | 991.7 | 1396.7 |
| 255 | 777.9 | 706 | 2286.6 | 1766.1 | 1100.5 | 1765.9 | 286 | 948.3 |
| 256 | 645.4 | 3136 | 3129.7 | 3456.4 | 1905.4 | 1995.5 | 3000.3 | 2611 |
| 257 | 107.8 | 348.2 | 360.1 | 289.9 | 300.9 | 240.8 | 403 | 750 |
| 258 | 83.1 | 3074.5 | 844.5 | 2046.8 | 1476.7 | 3628.7 | 3511.2 | 2536.1 |
| 259 | 1.2 | 668.5 | 1027.2 | 351 | 524.2 | 1117.3 | 356.7 | 500 |
| 260 | 185.4 | 108.7 | 158.6 | 251.8 | 168.6 | 182 | 256.7 | 116.2 |
| 261 | 73.8 | 130.7 | 187.3 | 61.6 | 210.2 | 242.6 | 130.1 | 258.1 |
| 263 | 919.6 | 1233.9 | 819.4 | 1299.4 | 572.7 | 912.1 | 1233 | 872.7 |
| 263 | 199.8 | 4626.1 | 3402.3 | 3087.9 | 3813.2 | 3582 | 6403.5 | 3036.9 |
| 265 | 310 | 125 | 156 | 135.2 | 128.1 | 234.7 | 96.8 | 155.8 |
| 266 | 381.6 | 899.6 | 433.3 | 530.4 | 242.9 | 358.6 | 641.2 | 241.1 |
| 256 | 130.8 | 141.8 | 211.4 | 344.3 | 408.2 | 195.6 | 211 | 388.4 |
| 268 | 155.9 | 135.7 | 783.8 | 251.7 | 990.9 | 145.6 | 77.9 | 262.5 |
| 269 | 70.4 | 137.9 | 127.4 | 87.5 | 920.7 | 144.7 | 106.4 | 123.1 |
| 270 | 906 | 197.1 | 177.3 | 148.3 | 194.1 | 214.8 | 161.2 | 198.9 |
| 271 | 197.7 | 411.2 | 613.2 | 278.3 | 1153.3 | 2568.1 | 824.5 | 1254.6 |
| 272 | 530.2 | 865.4 | 861.9 | 814.5 | 776.6 | 929.7 | 1402.4 | 1012 |
| 273 | 2.1 | 207.7 | 884.3 | 165.7 | 2070.8 | 969.7 | 200.6 | 1967.3 |
| 274 | 1050.2 | 932.1 | 1127.6 | 1151.3 | 1481.1 | 2100.5 | 1603.4 | 1010.4 |
| 275 | 277.8 | 674 | 1507.8 | 1511.2 | 956.4 | 1603.8 | 960.9 | 2338.9 |
| 276 | 496.5 | 4907.8 | 5071 | 1824.8 | 921.9 | 1584.4 | 1043 | 6199.7 |
| 277 | 159.6 | 251.2 | 318.9 | 445.6 | 355.2 | 318.1 | 503.6 | 277.7 |
| 278 | 15.3 | 1224.6 | 1434.2 | 602.7 | 1133.3 | 1898 | 1401.1 | 984.1 |
| 279 | 678.5 | 1074.4 | 1101.7 | 1030.5 | 1878 | 1425 | 1541.1 | 2160.3 |
| 280 | 1096.5 | 3203.9 | 2152.5 | 2126.2 | 3768.5 | 4309.6 | 7516.9 | 4768.7 |
| 281 | 38.3 | 590.9 | 605.4 | 938.5 | 832.1 | 257.9 | 315.3 | 385.4 |
| 282 | 407.5 | 416.5 | 704.2 | 1079.1 | 1604.5 | 485.4 | 482.3 | 1146.7 |
| 283 | 603.1 | 605 | 593.6 | 521.9 | 638.2 | 488.6 | 789.7 | 516.5 |
| 284 | 131.4 | 59.7 | 136.2 | 49.9 | 188.8 | 106.8 | 101.5 | 85 |
| 285 | 154.1 | 117 | 110.9 | 109.1 | 135.7 | 131.9 | 163 | 116 |
| 286 | 530.4 | 43.8 | 59.5 | 27.7 | 22.9 | 109.8 | 66.7 | 34.6 |
| 287 | 102.4 | 116.9 | 133.6 | 99.1 | 113.6 | 295 | 171.3 | 79.7 |
| 288 | 160.1 | 126.6 | 179.5 | 153.3 | 164.7 | 202.8 | 216.9 | 139.7 |
| 289 | 1536.2 | 4488.8 | 4944.9 | 1532.8 | 3646.8 | 5911.3 | 4344.5 | 8246.5 |
| 291 | 102.7 | 248.4 | 286.1 | 208.5 | 228.9 | 130.1 | 253.2 | 374 |
| 292 | 42.7 | 732.8 | 8793.1 | 18089.3 | 3318.5 | 3787.1 | 10588.9 | 8088.8 |
| 293 | 271.4 | 344.8 | 740.3 | 493 | 462.9 | 499.8 | 453.7 | 383.2 |
| 295 | 727.5 | 890.4 | 2593.2 | 1314 | 3115.5 | 310.9 | 729.5 | 1143.9 |
| 296 | 887.9 | 2346.4 | 1719.1 | 1863.5 | 1576.8 | 2166.6 | 2085.1 | 1930.4 |
| 297 | 96.5 | 59.7 | 110.1 | 83.1 | 71.1 | 60.1 | 112 | 82.2 |
| 298 | 546.1 | 730.6 | 785.4 | 968.7 | 730.8 | 563 | 1034.8 | 859.8 |
| 299 | 691.2 | 3305.3 | 2824.9 | 5439.5 | 1735.1 | 1756.8 | 2682.4 | 4873.4 |

| | | | | | | | | |
|---|---|---|---|---|---|---|---|---|
| 300 | 323.5 | 1392.4 | 991.4 | 1256.6 | 1607.8 | 1164.4 | 1068.3 | 1558.2 |
| 301 | 728.3 | 209.7 | 186.7 | 221.8 | 162.3 | 294.8 | 281.5 | 306.3 |
| 302 | 7.7 | 347.9 | 205.9 | 264.9 | 249.4 | 213.4 | 340.1 | 128.4 |
| 303 | 488.4 | 309.5 | 299.3 | 350 | 256.8 | 366.4 | 288.4 | 319.4 |
| 304 | 190.4 | 376.7 | 480.7 | 406.4 | 314.9 | 1106.8 | 764.9 | 973.2 |
| 305 | 478.8 | 246.1 | 628.7 | 2896.4 | 633.1 | 802.4 | 305.6 | 892.8 |
| 306 | 159.1 | 618.4 | 450.3 | 475.8 | 439.9 | 1419.5 | 763.8 | 450.3 |
| 307 | 132.8 | 254.7 | 425.8 | 462.3 | 354.6 | 254.6 | 307.1 | 237 |
| 308 | 5822.9 | 744.5 | 524.5 | 787 | 589.2 | 606.8 | 354.7 | 424.9 |
| 310 | 1068.6 | 151.4 | 133.4 | 116.4 | 77.7 | 177.3 | 68 | 124.3 |
| 311 | 36.2 | 641.7 | 499 | 622.8 | 349.4 | 351.8 | 356 | 454.7 |
| 312 | 4778.5 | 245 | 143.2 | 236.8 | 4.3 | 250.7 | 224.5 | 335 |
| 313 | 136.4 | 173 | 383.6 | 419 | 537.8 | 354.8 | 230.1 | 549.2 |
| 314 | 485.9 | 926.3 | 1589 | 956.6 | 1048.3 | 1458.6 | 629.4 | 877.4 |
| 315 | 125.9 | 82.4 | 187.2 | 88 | 58.7 | 77 | 93.2 | 43.3 |
| 316 | 367.6 | 574.4 | 633.7 | 462.8 | 337.8 | 583 | 763.4 | 450.7 |
| 317 | 145.2 | 202 | 1956.5 | 215.8 | 199.9 | 250.1 | 242.6 | 647.4 |
| 318 | 22.4 | 277.6 | 212 | 450.5 | 289.2 | 439.1 | 285.9 | 458.3 |
| 319 | 1838.4 | 512.3 | 276.4 | 733 | 268.8 | 487.6 | 670 | 354.9 |
| 320 | 477 | 181.1 | 267.8 | 128.4 | 160.8 | 244 | 142.2 | 214.4 |
| 321 | 718.7 | 163.3 | 292.4 | 134.5 | 159.2 | 263 | 227.2 | 257.6 |
| 322 | 440.4 | 379.6 | 285.7 | 360.6 | 238.3 | 342.7 | 305 | 208.4 |
| 323 | 1582.3 | 3954.1 | 3885.8 | 3447.2 | 3490.5 | 3121.3 | 3460.9 | 3724.3 |
| 324 | 112.2 | 445.5 | 257.9 | 704.3 | 519.5 | 235.2 | 528.1 | 737.5 |
| 325 | 190.1 | 374.9 | 430.9 | 503.9 | 541.5 | 326.1 | 367.3 | 610.4 |
| 326 | 422.9 | 4053.9 | 6121.7 | 4456.6 | 1364.3 | 5707.5 | 3498.2 | 4848.9 |
| 327 | 5.6 | 292.7 | 203.1 | 609.7 | 707.7 | 133.8 | 263.6 | 273.5 |
| 328 | 72.2 | 52 | 299.8 | 94.6 | 299.9 | 87.1 | 27.6 | 114.9 |
| 329 | 216.1 | 65.5 | 49.1 | 96.4 | 60.5 | 118.7 | 102.7 | 76.4 |
| 330 | 74.1 | 438.1 | 1060 | 824 | 632 | 896 | 708.8 | 558.4 |
| 331 | 783.2 | 297.5 | 466.2 | 372.3 | 422.4 | 374.2 | 562 | 297.2 |
| 333 | 216.1 | 167.4 | 206.9 | 194.6 | 174.5 | 38.1 | 190.6 | 149.2 |
| 334 | 107.8 | 126.8 | 470.1 | 153.1 | 513.7 | 220 | 141.5 | 193.3 |
| 335 | 570.7 | 191.9 | 697.3 | 783.9 | 505.5 | 208 | 243.3 | 414.7 |
| 336 | 491.6 | 342.6 | 210.4 | 272.6 | 218 | 256.8 | 428.3 | 243 |
| 337 | 127.4 | 619.5 | 632.8 | 711.3 | 511.9 | 972.3 | 606.6 | 804.8 |
| 338 | 180.6 | 1559 | 1838 | 1292.5 | 2158.9 | 837.9 | 1460.8 | 1632.3 |
| 339 | 303.9 | 217.3 | 347.8 | 220 | 329.4 | 258.1 | 219.3 | 184.2 |
| 340 | 185.5 | 137573.8 | 177311.9 | 54500.9 | 184533.6 | 59673.9 | 314613.6 | 86723.1 |
| 341 | 412.2 | 585 | 708 | 530.7 | 576.4 | 437.9 | 557.3 | 684.6 |
| 343 | 7951.1 | 2880.9 | 1575.6 | 1520.5 | 3477.9 | 2425 | 2379.2 | 1087.2 |
| 344 | 1755.5 | 649.1 | 436.9 | 434.5 | 285.2 | 463.7 | 452.7 | 599.1 |
| 345 | 236.2 | 296.5 | 511.3 | 662 | 580.8 | 429.8 | 424.2 | 488.6 |
| 347 | 821.3 | 1063 | 3642.7 | 1447.8 | 4119.2 | 1163.9 | 1210.2 | 1782.6 |
| 348 | 1679 | 4474.7 | 7575.3 | 4999.7 | 6225.7 | 3803.1 | 3979.1 | 4537.5 |
| 349 | 256.8 | 526.7 | 376.5 | 477.4 | 483.9 | 423.5 | 503.2 | 641.4 |
| 350 | 210.5 | 768.8 | 682.2 | 677.9 | 628.8 | 579 | 671.7 | 500.6 |
| 351 | 119.8 | 929.5 | 1553 | 2794.5 | 1558.8 | 1728.2 | 788.7 | 1446.6 |
| 352 | 435.7 | 264.1 | 190.3 | 177.4 | 180.3 | 233.4 | 304.2 | 190.7 |
| 353 | 385 | 500.7 | 566.6 | 574.8 | 346.1 | 382.1 | 487.3 | 473 |

3f

| SEQ ID NO: | 892TT | 893TT | 894TT | 896TT | 898TT | 899TT | 900TT | 901TT |
|---|---|---|---|---|---|---|---|---|
| 354 | 838.5 | 571.4 | 438.3 | 31.6 | 78.4 | 438.1 | 332.3 | 995.7 |
| 355 | 5801.6 | 1326.3 | 4267.6 | 187.6 | 121 | 2990.3 | 3907.9 | 3595.2 |

| | | | | | | | | |
|---|---|---|---|---|---|---|---|---|
| 356 | 672.9 | 416.1 | 1076.3 | 2648.9 | 200.3 | 682.2 | 309.3 | 200.9 |
| 357 | 815.8 | 844.6 | 869 | 1303.9 | 212 | 719.3 | 729.3 | 131.3 |
| 358 | 1742.6 | 1693.2 | 2440.3 | 1481.7 | 366.4 | 2077.8 | 2415.2 | 704.2 |
| 359 | 186 | 222.6 | 304.4 | 405.5 | 466.7 | 343.4 | 245.5 | 236.6 |
| 362 | 2087.5 | 1335.3 | 1239.7 | 326.8 | 171.4 | 870.6 | 879.2 | 101.1 |
| 360 | 472.7 | 502.8 | 559.4 | 1042.8 | 290.1 | 549.2 | 506 | 174.5 |
| 361 | 390.4 | 80 | 162.4 | 329.8 | 212.1 | 162.1 | 138.5 | 149.9 |
| 363 | 335.6 | 549.7 | 748.6 | 666.9 | 742.2 | 461 | 356.5 | 506.8 |
| 1 | 1364 | 864.1 | 2300.1 | 1111.5 | 725.5 | 1891.2 | 735.8 | 1211.8 |
| 2 | 8223.2 | 2863.6 | 1612.1 | 3492.1 | 669.6 | 1116 | 3477.7 | 10076.1 |
| 7 | 621.3 | 68.9 | 83.5 | 28.3 | 339.5 | 548.2 | 237.7 | 108.6 |
| 8 | 434.3 | 297.2 | 236.7 | 314.4 | 1057.9 | 469 | 735.8 | 1382.2 |
| 9 | 2209.5 | 1428.7 | 2874.5 | 9019.8 | 219.4 | 1483.2 | 931.8 | 299 |
| 10 | 87.4 | 318.6 | 123 | 113.9 | 359.9 | 132.2 | 797.4 | 116.1 |
| 11 | 2437.4 | 1234.9 | 867.7 | 1328.7 | 332.7 | 722 | 898.4 | 645.5 |
| 13 | 397 | 162.2 | 347.3 | 290.4 | 118.5 | 313.3 | 718 | 1070.4 |
| 14 | 2294.4 | 977.8 | 1525.3 | 1055.3 | 256.2 | 1105.9 | 2608.5 | 4093.2 |
| 15 | 828.3 | 416.9 | 482.6 | 728.2 | 16.4 | 502.2 | 3166.5 | 179.1 |
| 16 | 3234 | 2475.7 | 4710 | 1027.1 | 239.9 | 3055.6 | 4422.5 | 721.2 |
| 17 | 2718.1 | 742.6 | 607.2 | 522 | 240.9 | 762.6 | 1568.9 | 129.7 |
| 19 | 1086.6 | 248.6 | 333.5 | 226.7 | 290.8 | 444.8 | 7182.8 | 1716.2 |
| 20 | 192.8 | 641.7 | 12.5 | 39 | 1.2 | 792.4 | 372.3 | 36.3 |
| 21 | 1826.1 | 4120.6 | 1923.8 | 941.2 | 238.4 | 2518.7 | 2236.8 | 94.6 |
| 22 | 8192.1 | 2215.6 | 1368.7 | 1592.1 | 753.1 | 5336.9 | 2477.2 | 299.2 |
| 23 | 687.5 | 415.7 | 227.3 | 1.1 | 1.1 | 709.6 | 2476.1 | 179.7 |
| 24 | 326.7 | 113.9 | 163.5 | 127.1 | 110.4 | 165 | 803.4 | 105 |
| 26 | 1128.9 | 408.7 | 398.2 | 63.7 | 106.1 | 1433.6 | 5983.2 | 1234.8 |
| 27 | 1213 | 865.2 | 488.6 | 772.5 | 725.7 | 1107.3 | 619.8 | 58.3 |
| 29 | 134.2 | 85.5 | 245.2 | 242.8 | 215.1 | 196.6 | 356.2 | 198.7 |
| 30 | 362.2 | 248.6 | 151.7 | 102.9 | 16.7 | 261.5 | 1169.1 | 236 |
| 31 | 9692.5 | 20154 | 18540.6 | 1550.9 | 250.2 | 15669.5 | 3557.5 | 5.2 |
| 33 | 1841.1 | 2277.7 | 1079.8 | 442.8 | 137.2 | 3329.2 | 14318.3 | 588.8 |
| 34 | 826.4 | 839.3 | 441.4 | 624 | 4011.4 | 566.1 | 579.8 | 2078.9 |
| 35 | 1512.1 | 1217.1 | 1163.2 | 194.2 | 132.8 | 1055.6 | 1451.8 | 26.9 |
| 36 | 2188 | 735.7 | 379.8 | 646.9 | 507.5 | 1041.8 | 675.2 | 8116.1 |
| 37 | 133.7 | 152.8 | 191.6 | 188 | 737.6 | 240.4 | 318.6 | 263.7 |
| 38 | 776.1 | 895.4 | 248.4 | 519.4 | 99.4 | 1003.5 | 6688.2 | 339.4 |
| 40 | 910.9 | 789.8 | 648.8 | 518.5 | 810.2 | 652.7 | 553.1 | 417.3 |
| 41 | 922.5 | 3053 | 1250.6 | 119.4 | 254.6 | 779.1 | 3979.3 | 333.2 |
| 42 | 343.8 | 889 | 400.7 | 444.1 | 260.1 | 318.1 | 535.4 | 200.6 |
| 43 | 219.4 | 422 | 417 | 1244.1 | 1305.2 | 310.9 | 593.5 | 1335.2 |
| 44 | 1388 | 1454.8 | 1175.7 | 1009.9 | 401.4 | 945.2 | 1312.6 | 316.6 |
| 45 | 1335.6 | 1007.6 | 839.8 | 405.9 | 730.7 | 1723.4 | 1554 | 740.1 |
| 46 | 281.1 | 267.6 | 425 | 376.4 | 402.6 | 326.4 | 1021.7 | 863 |
| 47 | 739.5 | 340.9 | 354.1 | 543.9 | 308.4 | 485.9 | 555.9 | 1064.5 |
| 48 | 570.5 | 23 | 230 | 40.3 | 27.6 | 142.1 | 56.8 | 273.5 |
| 49 | 2690.8 | 7059.8 | 2365.9 | 162.5 | 247.7 | 3550.1 | 610.3 | 195.8 |
| 50 | 582.4 | 319.5 | 412.6 | 124.5 | 80 | 377.8 | 1255 | 230 |
| 51 | 530 | 1015.7 | 325.9 | 138.5 | 2289.9 | 333.2 | 623.9 | 65.3 |
| 52 | 333.7 | 327.9 | 286.2 | 281.6 | 329.4 | 221.1 | 347.5 | 493.3 |
| 53 | 3598.9 | 1864.6 | 1743.5 | 365.8 | 89.1 | 1567.9 | 1670 | 73.1 |
| 54 | 1988 | 1754.5 | 1175.5 | 286.1 | 91.4 | 1181.1 | 1478.9 | 2364.4 |
| 55 | 85.9 | 96.8 | 95.9 | 124.3 | 145 | 85.6 | 80 | 140.6 |
| 56 | 213.9 | 163.6 | 156.6 | 39 | 62.8 | 229.7 | 214.4 | 57.5 |
| 57 | 6568.1 | 82.1 | 618.1 | 951.4 | 256.7 | 1930 | 764.7 | 814 |
| 58 | 10305.1 | 843.3 | 1639.6 | 3341.1 | 274.3 | 2514.5 | 1398 | 1879.6 |
| 59 | 4632.7 | 8361.8 | 4125.6 | 1656.3 | 319.1 | 4755.6 | 2037.8 | 1675.3 |
| 60 | 510.1 | 221.7 | 136.9 | 117.4 | 148.1 | 354.9 | 336.7 | 187.1 |

| | | | | | | | | |
|---|---|---|---|---|---|---|---|---|
| 61 | 98.3 | 111.4 | 123.3 | 179.6 | 275.5 | 108.5 | 72.9 | 74.6 |
| 62 | 5423.2 | 3854.1 | 2373.9 | 8567.7 | 2395.3 | 3812.3 | 2208.6 | 1481 |
| 63 | 830.4 | 1299.6 | 461.4 | 605.2 | 69.5 | 803.8 | 256.1 | 37.7 |
| 64 | 479.7 | 114.8 | 188.8 | 126.9 | 488.9 | 197.6 | 329.8 | 245.9 |
| 65 | 389.5 | 110.3 | 87.8 | 50.1 | 50.1 | 176 | 383.4 | 101.2 |
| 66 | 1831.2 | 3206.9 | 2835.9 | 601 | 634.9 | 2474.7 | 1318.9 | 103.3 |
| 67 | 26156.4 | 24869.8 | 31314 | 34527.1 | 10176.9 | 25736.9 | 1905.1 | 173.4 |
| 68 | 821.8 | 40.7 | 2360.3 | 789.4 | 2432.7 | 943.9 | 68.9 | 1.1 |
| 69 | 2579.5 | 781.6 | 1976.3 | 1894.3 | 5728.2 | 2305.4 | 397.5 | 111.3 |
| 70 | 5688.9 | 8491.7 | 8887.2 | 2267.9 | 5973.9 | 8571 | 4098.7 | 128.4 |
| 71 | 3496.6 | 5511.6 | 2751.2 | 100.6 | 65.6 | 3748.2 | 10193.5 | 626.2 |
| 72 | 3296 | 2026.7 | 1766.1 | 530.8 | 106.1 | 922 | 2699.1 | 99.9 |
| 73 | 763.6 | 839.9 | 660.7 | 520.4 | 264.6 | 888.6 | 359.2 | 419.4 |
| 75 | 4452.7 | 1125.7 | 275.2 | 310.3 | 145 | 804.4 | 156.6 | 359.9 |
| 76 | 2427.6 | 2043.5 | 940.2 | 329.2 | 2388.1 | 841.4 | 3184.4 | 22133.7 |
| 77 | 563.4 | 2247.6 | 3062.3 | 6825.9 | 2972.1 | 1722.2 | 280.8 | 2325.7 |
| 78 | 623.5 | 124.1 | 227.5 | 554.4 | 340.2 | 302.3 | 207.6 | 170.9 |
| 79 | 297.9 | 552.3 | 257.1 | 484.8 | 185.7 | 287.4 | 938.8 | 230.9 |
| 80 | 2294.3 | 1015.3 | 932.1 | 949.4 | 267.8 | 937.2 | 1004.6 | 2105.3 |
| 81 | 8681.4 | 6802.8 | 13448.3 | 318.1 | 159.4 | 16138.8 | 9121 | 168.3 |
| 82 | 128.2 | 99.7 | 135 | 270.9 | 561.7 | 187.3 | 146.7 | 52.5 |
| 84 | 308.9 | 120.8 | 130.3 | 138.1 | 893.7 | 256.5 | 336 | 131.7 |
| 85 | 410.7 | 2865.5 | 317.4 | 179.7 | 186.4 | 225.1 | 1173 | 198.2 |
| 86 | 71.5 | 641.8 | 296.9 | 391.1 | 257.7 | 123.2 | 2621.8 | 23448.8 |
| 87 | 689 | 3701.3 | 2676.7 | 616.5 | 261 | 857 | 2313.9 | 324 |
| 88 | 712.7 | 622.2 | 840 | 1105.6 | 1000.6 | 639.5 | 272.4 | 1569.4 |
| 89 | 295 | 186 | 158.6 | 179.6 | 961.8 | 277.3 | 453.9 | 155.9 |
| 90 | 215.5 | 68.7 | 167.8 | 107.4 | 122.9 | 164.7 | 1000.2 | 289 |
| 91 | 7519.7 | 924.8 | 1647.3 | 799.8 | 217.9 | 817.9 | 2001.9 | 2046.3 |
| 92 | 11830.9 | 13031.1 | 13383.8 | 7238.6 | 302.1 | 3811.1 | 5046.1 | 174.3 |
| 93 | 115.5 | 87.8 | 34 | 89.4 | 211.9 | 78.4 | 109.2 | 75.8 |
| 94 | 767.1 | 1362.1 | 410.1 | 191.2 | 97.7 | 553.9 | 380.2 | 39.9 |
| 95 | 299.5 | 345.9 | 216.6 | 55.5 | 193.5 | 512 | 708 | 62.4 |
| 96 | 353.2 | 438.1 | 419.9 | 460.6 | 210.2 | 326 | 144.2 | 304.2 |
| 97 | 808.2 | 693.9 | 524.2 | 2786.4 | 252.2 | 405.5 | 344.9 | 189.9 |
| 98 | 20.1 | 38.9 | 44.1 | 21.6 | 22.7 | 45.4 | 14.9 | 853 |
| 99 | 1151.9 | 1198.4 | 389.3 | 273.9 | 337.4 | 383.1 | 950.5 | 207.7 |
| 100 | 152.9 | 90.9 | 122.7 | 552.8 | 61.1 | 313.3 | 41.7 | 36022.7 |
| 101 | 260.8 | 135.8 | 150.6 | 140.2 | 234.4 | 326.1 | 275.4 | 121.1 |
| 102 | 117.3 | 164.9 | 193.6 | 214.6 | 452.9 | 190.4 | 161.9 | 15084.6 |
| 103 | 359.7 | 366.5 | 553.1 | 618.6 | 458.1 | 556.4 | 352.4 | 349.3 |
| 104 | 72.3 | 85.7 | 95.1 | 84.2 | 660.1 | 174.1 | 49.8 | 301.7 |
| 105 | 76.8 | 146 | 192.9 | 201.7 | 396.4 | 190.8 | 55 | 576.1 |
| 106 | 80.9 | 148.1 | 99.6 | 97.2 | 589.1 | 212.8 | 79.7 | 301 |
| 107 | 207.3 | 184.2 | 599.8 | 177.7 | 156.9 | 201.1 | 710.6 | 224.5 |
| 108 | 163.3 | 204.4 | 177.6 | 565.4 | 277.2 | 123.7 | 285.7 | 396 |
| 109 | 1770.9 | 611.2 | 2356.1 | 78.1 | 65.3 | 737.6 | 812.4 | 1225.9 |
| 110 | 3284.9 | 1445.7 | 5145.6 | 1.2 | 1.3 | 2012.1 | 2144.5 | 3019.9 |
| 111 | 125.1 | 46 | 62.7 | 29.8 | 254.8 | 274.1 | 161.3 | 39.9 |
| 112 | 616 | 1456.3 | 1415 | 289.8 | 56.8 | 1850.3 | 2408.5 | 94.2 |
| 113 | 484.5 | 533.3 | 277.4 | 98.1 | 241.5 | 317.7 | 339.8 | 66.4 |
| 114 | 600.1 | 371.3 | 562.4 | 290 | 103.7 | 987.5 | 502.2 | 156.9 |
| 115 | 1041.7 | 1742.3 | 1015.4 | 1023 | 384.6 | 1053.1 | 733.8 | 268.3 |
| 116 | 932 | 296 | 381 | 243.7 | 236.9 | 432.4 | 1273.7 | 147.7 |
| 117 | 339.7 | 124.5 | 167.1 | 263.6 | 190.4 | 177.4 | 107.7 | 55.5 |
| 118 | 69.7 | 220.3 | 177.8 | 130.5 | 3113.8 | 104.8 | 101.3 | 865.7 |
| 119 | 298.6 | 219.6 | 301.2 | 94.8 | 80.9 | 379.1 | 460.6 | 53 |
| 120 | 98.3 | 53.4 | 99.6 | 177.4 | 150.1 | 98.1 | 1050.9 | 126.1 |

| | | | | | | | | |
|---|---|---|---|---|---|---|---|---|
| 121 | 5866 | 1900 | 3972.7 | 505.4 | 92.2 | 6630 | 207 | 2844.8 |
| 122 | 322.1 | 911.3 | 404.3 | 385.8 | 240.5 | 366.3 | 568.3 | 63.3 |
| 123 | 404.9 | 634.2 | 345.1 | 149 | 392 | 340.4 | 234.3 | 311.6 |
| 124 | 131.4 | 129.1 | 160.1 | 787.8 | 2835.4 | 130.4 | 121.4 | 150.7 |
| 125 | 723.6 | 926.1 | 1036 | 939.9 | 2182.9 | 947.8 | 12974.8 | 522.7 |
| 126 | 141.1 | 30.5 | 89 | 159 | 1503.2 | 35.6 | 3.1 | 355.9 |
| 127 | 191.9 | 184.3 | 258.1 | 319.5 | 139.6 | 201.4 | 155.4 | 104.9 |
| 128 | 179 | 235943.8 | 208.7 | 264 | 359.9 | 236127.3 | 90211.9 | 230929.7 |
| 129 | 1267.5 | 3432.6 | 1214.7 | 246.9 | 149.3 | 1110.6 | 5445.8 | 153.6 |
| 130 | 540.5 | 632.3 | 587.9 | 375.8 | 113.6 | 347.2 | 1074.4 | 82.4 |
| 131 | 856.8 | 607.9 | 246.2 | 90.3 | 123.9 | 357 | 480 | 54.8 |
| 132 | 387.6 | 420.8 | 334.4 | 228.4 | 227.3 | 360.2 | 314.6 | 225.9 |
| 133 | 237.3 | 363.1 | 302.2 | 130.6 | 230.6 | 311.5 | 218 | 603.9 |
| 134 | 594.4 | 1140 | 498.1 | 384 | 104 | 345.6 | 401.6 | 224.8 |
| 135 | 1075.3 | 533.8 | 887.3 | 1588.4 | 220 | 677.5 | 175.4 | 301.4 |
| 138 | 231.7 | 110.1 | 175.8 | 232.6 | 169.6 | 171 | 141.3 | 203.3 |
| 139 | 298.9 | 162.7 | 156.5 | 459.8 | 119.7 | 182.1 | 162 | 227.8 |
| 140 | 762.9 | 725.5 | 154.5 | 31.8 | 1.2 | 92.1 | 35.4 | 1.1 |
| 141 | 621.6 | 930.7 | 688.4 | 687.8 | 23 | 420.1 | 36.5 | 666.8 |
| 144 | 37803 | 9899.9 | 22316.3 | 645.5 | 490.7 | 13084 | 9054.7 | 316.7 |
| 145 | 177 | 368.3 | 269.9 | 232.7 | 310.9 | 289.2 | 250 | 219.2 |
| 146 | 243 | 250.9 | 892.8 | 185.8 | 329 | 1096 | 513.5 | 231 |
| 147 | 120.4 | 64.3 | 79.7 | 78.1 | 34.2 | 140.2 | 94 | 23.6 |
| 148 | 677.9 | 631.3 | 630.4 | 298.9 | 225.4 | 335.1 | 307.8 | 192.7 |
| 149 | 398.6 | 217.3 | 602.8 | 1656.9 | 31.3 | 377.5 | 134 | 54.5 |
| 150 | 374.1 | 454.4 | 196.3 | 972.4 | 157.6 | 149.2 | 283.3 | 5740.8 |
| 151 | 1052 | 1195 | 656.8 | 163.7 | 191.8 | 924.2 | 636.7 | 216.6 |
| 153 | 139.4 | 146.7 | 146.2 | 319.5 | 194.7 | 88.4 | 724.1 | 99.2 |
| 154 | 8645.2 | 16591.3 | 5176.3 | 424.8 | 109.1 | 3577 | 4066.1 | 75.6 |
| 155 | 246.6 | 937.6 | 813.8 | 187.8 | 175.2 | 624.4 | 381.4 | 146.7 |
| 156 | 295.3 | 187.6 | 212 | 76.1 | 53.7 | 543.4 | 250.4 | 52.4 |
| 157 | 223.9 | 203.1 | 318.4 | 199.3 | 184.1 | 199.3 | 122.6 | 224.2 |
| 158 | 537.6 | 821.6 | 265.2 | 1.4 | 27 | 713.1 | 498.5 | 9.4 |
| 159 | 1344.7 | 1973.3 | 1599.7 | 3234 | 904.2 | 1579.4 | 405.5 | 1.3 |
| 161 | 3301.2 | 7782.7 | 14054.5 | 63.2 | 66.7 | 8990.6 | 4295.2 | 228.4 |
| 162 | 701.6 | 131.2 | 171.7 | 65.8 | 32 | 330.5 | 1161.7 | 70.7 |
| 164 | 8016.9 | 3022.2 | 1600.5 | 253.8 | 89.7 | 771.6 | 851.6 | 88.2 |
| 166 | 1326.9 | 1067.9 | 409.9 | 3604 | 3611.9 | 1114.7 | 1254.5 | 258.6 |
| 167 | 457.9 | 95.7 | 482.2 | 1592.1 | 1309.9 | 959.2 | 81.5 | 1.2 |
| 168 | 1090.3 | 935.1 | 1205.2 | 793.6 | 90.8 | 552 | 89.1 | 34.2 |
| 169 | 13698.9 | 8961.4 | 4979.8 | 1588.3 | 221.9 | 15295.7 | 6424.9 | 919.2 |
| 170 | 2889.3 | 2125.1 | 1875.6 | 2018.5 | 38 | 2738.1 | 2166.4 | 1941.3 |
| 171 | 575.6 | 86.9 | 234.3 | 1.3 | 1.2 | 326.8 | 1162.6 | 272.5 |
| 172 | 480.7 | 479.3 | 537.5 | 625.7 | 696.9 | 476.9 | 451.9 | 506.7 |
| 173 | 1793.3 | 647.9 | 338.5 | 142.8 | 256.6 | 1057.8 | 862.7 | 492.4 |
| 176 | 1468.4 | 1365.5 | 272.9 | 1.2 | 75.3 | 744.9 | 117.3 | 10085.2 |
| 177 | 275.1 | 98.7 | 141 | 183.5 | 592.4 | 278.4 | 235.9 | 131.1 |
| 178 | 1912.6 | 1129.2 | 203.6 | 86.2 | 728 | 1472.4 | 2389 | 12289 |
| 179 | 485.6 | 1376.2 | 243.4 | 60 | 153.3 | 1065.3 | 3098.7 | 53.9 |
| 180 | 1011.7 | 464.5 | 526.5 | 263.1 | 154.4 | 957.8 | 1085 | 118.1 |
| 181 | 5130.4 | 3292.8 | 3724 | 13102.1 | 11246.2 | 5277.8 | 2419.3 | 389.1 |
| 182 | 530.4 | 324.9 | 404 | 767.8 | 204.4 | 388.4 | 384.7 | 169.2 |
| 183 | 447.6 | 199 | 135.7 | 1.1 | 1.1 | 259.9 | 1258.2 | 281.4 |
| 184 | 620 | 357.8 | 340.4 | 291.6 | 192 | 428.8 | 14293.9 | 682.8 |
| 185 | 3627.9 | 10011.6 | 2963.4 | 729 | 355.6 | 10614.7 | 3033.4 | 880 |
| 186 | 173.9 | 184.8 | 258.3 | 1106 | 716.2 | 173.4 | 287.8 | 2619 |
| 187 | 333.3 | 525.2 | 136.5 | 200.2 | 239.2 | 314.3 | 689.4 | 176.4 |
| 188 | 435.3 | 350.4 | 378.3 | 132.3 | 145.2 | 161.9 | 1419.6 | 133.9 |

| | | | | | | | | |
|---|---|---|---|---|---|---|---|---|
| 189 | 181.3 | 130.5 | 201.3 | 91.3 | 144.2 | 92.7 | 674.1 | 79.5 |
| 190 | 359.9 | 238.5 | 312 | 108.8 | 110.1 | 485.5 | 163.7 | 58.4 |
| 191 | 5707 | 3740.1 | 3029.6 | 3256.5 | 886.2 | 2447.8 | 3778.5 | 97.4 |
| 192 | 150.4 | 95.8 | 139.2 | 130.9 | 129.3 | 109.1 | 506.3 | 2753.6 |
| 193 | 361.7 | 114.3 | 88.9 | 6063.3 | 105.9 | 289.6 | 160.1 | 46.2 |
| 194 | 261.5 | 249.3 | 203.6 | 200.9 | 134.1 | 251.6 | 372.2 | 471.3 |
| 195 | 132 | 110.9 | 158.3 | 96.7 | 440.4 | 150 | 189.2 | 297.3 |
| 196 | 1327.6 | 1039.5 | 1104 | 311.8 | 7746.9 | 3084.9 | 1670.6 | 448.2 |
| 197 | 132.2 | 98.3 | 139.2 | 194 | 218.3 | 141.3 | 868.5 | 520.5 |
| 198 | 532.6 | 1476.5 | 1624.7 | 83.8 | 94.6 | 2377.6 | 755.4 | 70.4 |
| 199 | 7184.6 | 2171.3 | 7934.6 | 482.3 | 52.5 | 4537.3 | 5623.9 | 4564.9 |
| 200 | 975.6 | 370.7 | 300.2 | 182.2 | 210.4 | 482.6 | 427.5 | 1652.6 |
| 201 | 957 | 604.8 | 467.3 | 35.1 | 11.1 | 420.7 | 334 | 1094.3 |
| 203 | 3711.3 | 1570.8 | 1229.6 | 5308.7 | 248.9 | 965.6 | 1358.9 | 47.1 |
| 204 | 1750.8 | 1898.9 | 2112.9 | 4011.7 | 204.1 | 1174.1 | 290.9 | 27.1 |
| 205 | 6039 | 4435.4 | 3880.5 | 6493.9 | 2337.3 | 4612.6 | 1166.9 | 3.2 |
| 206 | 163.8 | 374.4 | 608.3 | 472.7 | 308.5 | 390.5 | 250 | 327.8 |
| 207 | 480.6 | 47.7 | 74.7 | 206.6 | 34.9 | 103.4 | 58.1 | 59.8 |
| 208 | 585.4 | 1688.4 | 1780.1 | 191.9 | 58.9 | 866.4 | 278.1 | 1.6 |
| 209 | 197.5 | 4.4 | 60.6 | 353.2 | 5030.7 | 400.5 | 66.4 | 207.9 |
| 211 | 1550.5 | 219.1 | 1271.8 | 1356.7 | 942.5 | 164.7 | 120.1 | 87.4 |
| 212 | 1426 | 1312.8 | 870.1 | 225.4 | 1412.1 | 2035.3 | 1106.7 | 52.1 |
| 213 | 104 | 191.1 | 118.8 | 124.5 | 3087.3 | 123.3 | 121.9 | 77.7 |
| 215 | 475.1 | 55.9 | 84.6 | 15 | 9 | 131.4 | 77.7 | 93 |
| 216 | 397.6 | 259.8 | 310.5 | 423.2 | 377.9 | 179.3 | 226.1 | 222.2 |
| 217 | 1130.5 | 517.6 | 725 | 187.5 | 173.5 | 2109.1 | 1336.7 | 189.2 |
| 218 | 159.4 | 224.9 | 205.2 | 353.5 | 294.1 | 236.6 | 199.5 | 89 |
| 220 | 79.6 | 70 | 204.6 | 1293.4 | 119 | 128.5 | 270.9 | 68484.3 |
| 221 | 2459.4 | 1795.5 | 739.3 | 429.3 | 254.4 | 1453.4 | 860.5 | 219.7 |
| 223 | 981.9 | 680.4 | 687.3 | 439.1 | 267.8 | 500.2 | 459.3 | 290.4 |
| 227 | 1489.9 | 1104.5 | 879.4 | 1033.7 | 822.7 | 1224.7 | 5386.3 | 435.5 |
| 228 | 186.1 | 178.2 | 271.2 | 198.1 | 433.9 | 318.4 | 220.6 | 177.1 |
| 229 | 180.5 | 334 | 201.9 | 302.5 | 172.6 | 189.2 | 253.5 | 582.6 |
| 230 | 3245.4 | 2835.9 | 3394.8 | 163.3 | 99.6 | 3274.1 | 4388 | 147.1 |
| 231 | 1258.8 | 702.6 | 436 | 985.6 | 204.9 | 418.8 | 591.9 | 75.2 |
| 232 | 704.3 | 266.5 | 271.6 | 59.5 | 31.8 | 1000 | 4104.9 | 782 |
| 233 | 15997.8 | 9251.8 | 21829 | 5239.6 | 15022 | 18292.9 | 3490 | 3738.3 |
| 235 | 405.2 | 135 | 205.3 | 218.8 | 77.9 | 242.7 | 688.4 | 216.2 |
| 236 | 345.4 | 176.8 | 244.3 | 51.8 | 289.1 | 251.1 | 569.9 | 355.3 |
| 237 | 2087.1 | 2074.2 | 977.3 | 667.1 | 647.6 | 2376.1 | 1252 | 2105 |
| 238 | 1029.7 | 709.6 | 957.5 | 1263.7 | 456.5 | 1002.9 | 733.2 | 417.9 |
| 239 | 398.5 | 111.4 | 252.1 | 162.3 | 97.3 | 806.1 | 929.5 | 1253.4 |
| 240 | 590.8 | 219.9 | 517.4 | 355.8 | 74.6 | 973.1 | 1561.1 | 1235 |
| 241 | 1224.2 | 353.4 | 781.2 | 558.8 | 62.3 | 2210.8 | 2833.8 | 2772.1 |
| 242 | 31599.6 | 34700.3 | 41193.8 | 1998.1 | 39786.8 | 38117 | 29127.6 | 4032.5 |
| 243 | 496 | 1167.3 | 574.5 | 293.2 | 363.2 | 674.9 | 937.7 | 478.8 |
| 244 | 619.3 | 993.1 | 600.4 | 303.2 | 63.1 | 271.1 | 782.3 | 352.7 |
| 246 | 1856.6 | 1243.2 | 1238.1 | 641.8 | 111.2 | 2226.5 | 1235.4 | 797.6 |
| 247 | 90.8 | 89.3 | 158.9 | 200.2 | 539.1 | 130.5 | 88.4 | 2091.6 |
| 248 | 3748.4 | 2617 | 1198.3 | 214.5 | 80.7 | 1744 | 935.6 | 108.1 |
| 249 | 2042.6 | 468.9 | 513.5 | 50.4 | 326.4 | 336.8 | 254.7 | 971.7 |
| 251 | 595 | 1004.5 | 592 | 56.1 | 35 | 1047.9 | 2445.5 | 71.5 |
| 253 | 465.8 | 497.1 | 469.6 | 410 | 220.1 | 449.3 | 295.9 | 531.1 |
| 254 | 2043.5 | 910.3 | 939.7 | 363.4 | 86 | 1038.8 | 861.8 | 107 |
| 255 | 2304.8 | 1354.8 | 344.7 | 122.3 | 762.2 | 1679.9 | 2631.1 | 14516.5 |
| 256 | 7209.5 | 1050.3 | 1841.6 | 927.6 | 282.3 | 701.3 | 1193.8 | 1494.3 |
| 257 | 856.2 | 248.4 | 216.9 | 662.9 | 150 | 341.6 | 378.8 | 234.1 |
| 258 | 1862.6 | 2711.5 | 2864.4 | 273.4 | 23.2 | 1388.3 | 3786.6 | 38.9 |

| | | | | | | | | |
|---|---|---|---|---|---|---|---|---|
| 259 | 690.8 | 1255 | 315.5 | 8.9 | 8.9 | 666.1 | 567.4 | 35.6 |
| 260 | 171.5 | 136.4 | 233.1 | 141.7 | 251.1 | 174 | 130.8 | 576.4 |
| 261 | 647.4 | 192.8 | 70.8 | 118.2 | 87.7 | 108 | 72.5 | 91.1 |
| 263 | 797.9 | 1445.4 | 889.1 | 1265 | 3327 | 572.6 | 1048.4 | 4844.5 |
| 263 | 2900.5 | 3388 | 4354 | 382 | 258.7 | 2740.8 | 3471.4 | 176.5 |
| 265 | 89.2 | 115.6 | 179.2 | 394.1 | 219 | 100.9 | 131.6 | 85.6 |
| 266 | 213.6 | 237.5 | 772.5 | 877 | 652.8 | 477.8 | 159.6 | 124 |
| 256 | 293.8 | 159.8 | 170.3 | 96.7 | 137.6 | 266.6 | 612.1 | 111.9 |
| 268 | 427.2 | 47.3 | 133.1 | 64.4 | 531.8 | 497.3 | 80.3 | 18.9 |
| 269 | 144.1 | 171.6 | 184 | 64.4 | 137.5 | 256.6 | 159.2 | 47.1 |
| 270 | 149.1 | 221.6 | 249.7 | 540.1 | 727.4 | 204.9 | 126.7 | 413 |
| 271 | 928.9 | 1477.3 | 659.3 | 228.8 | 157 | 361 | 939.9 | 257.9 |
| 272 | 2065.5 | 769.5 | 961.5 | 709.2 | 889.7 | 644.3 | 583.3 | 1336.4 |
| 273 | 460 | 254.4 | 1932.6 | 1972 | 55.7 | 1557.3 | 1.2 | 1.1 |
| 274 | 1272 | 1303.2 | 959.1 | 381.8 | 692.6 | 817.7 | 3288.6 | 1936.2 |
| 275 | 1067.2 | 3809.3 | 819 | 189.7 | 89.6 | 3279.9 | 984.3 | 183.6 |
| 276 | 1560.5 | 1665.4 | 856.1 | 437.3 | 864.3 | 1770.9 | 8639.5 | 830.5 |
| 277 | 420.2 | 373.4 | 347.1 | 255.6 | 230.4 | 352.6 | 225.8 | 197.8 |
| 278 | 1479.6 | 2479.2 | 413.9 | 38.1 | 16.1 | 1327.7 | 1485.3 | 1104.2 |
| 279 | 4418.5 | 1252.4 | 1647.9 | 1229.2 | 826.2 | 1907.3 | 690.5 | 1.2 |
| 280 | 6980.6 | 3103.2 | 5603.5 | 9202.3 | 973.7 | 3117.3 | 1054 | 1.4 |
| 281 | 893.5 | 469.5 | 390.8 | 50.9 | 76.1 | 645.6 | 28.3 | 2949.8 |
| 282 | 436.5 | 866.2 | 712.4 | 604.9 | 432.1 | 429.3 | 4533.3 | 239.2 |
| 283 | 378.5 | 494.1 | 618.3 | 438.7 | 610.6 | 397.3 | 345.7 | 2247.9 |
| 284 | 86.5 | 78.4 | 84.7 | 59.9 | 151.4 | 121.6 | 104 | 90 |
| 285 | 100.1 | 125 | 140.6 | 171.6 | 130.3 | 107.2 | 95.4 | 109.5 |
| 286 | 21.9 | 47 | 34.4 | 361.5 | 622.2 | 17.4 | 51 | 14.1 |
| 287 | 85 | 103.5 | 147.3 | 266.3 | 140.8 | 110.4 | 90.8 | 52593.6 |
| 288 | 106.7 | 108.9 | 226 | 255.8 | 302 | 141.1 | 141.3 | 161.5 |
| 289 | 16536.4 | 1225.1 | 3454 | 1845.6 | 187.6 | 1317.1 | 1594.5 | 336.7 |
| 291 | 197.9 | 131.8 | 167.2 | 170.5 | 27.6 | 264.2 | 372 | 222.7 |
| 292 | 10808.4 | 363.8 | 202.4 | 174.8 | 66 | 1720 | 15444.1 | 227.7 |
| 293 | 409.1 | 361.7 | 288.2 | 206.3 | 82.6 | 711.2 | 306.9 | 55.7 |
| 295 | 812 | 283.8 | 1193.9 | 554.6 | 477.9 | 2370 | 701.1 | 344.9 |
| 296 | 2134.1 | 3003.7 | 1806.5 | 1160.2 | 1121 | 1902.2 | 1362.4 | 253.2 |
| 297 | 38.5 | 59.7 | 102.3 | 75.6 | 2740.8 | 70.7 | 113.3 | 39.2 |
| 298 | 610.2 | 990 | 998 | 952 | 469.4 | 525.7 | 390.2 | 600.9 |
| 299 | 2108.9 | 4536.4 | 3222.2 | 2152.4 | 419.3 | 4258 | 1379.7 | 77.2 |
| 300 | 1679.8 | 1409.5 | 1107.2 | 90.1 | 244.2 | 1361.8 | 952.6 | 239.4 |
| 301 | 221.2 | 271 | 284.8 | 845.7 | 261 | 211.4 | 223.5 | 162.3 |
| 302 | 216.8 | 246.1 | 174.8 | 34.5 | 40.3 | 171.5 | 310.6 | 21.7 |
| 303 | 266.3 | 414.9 | 259.1 | 1838.2 | 984.5 | 263.8 | 215.7 | 52.1 |
| 304 | 1007.4 | 636.1 | 791.1 | 1190.1 | 791 | 396.5 | 803.9 | 2631.2 |
| 305 | 2780.8 | 1913 | 203.5 | 627.5 | 451.4 | 213.2 | 4287.8 | 28703.6 |
| 306 | 600.9 | 1369.7 | 510.6 | 266.5 | 278.1 | 451.2 | 469.4 | 196.1 |
| 307 | 427.2 | 183.4 | 239.2 | 150 | 194.3 | 189.6 | 485.2 | 145.3 |
| 308 | 296.6 | 711.6 | 941.5 | 559.6 | 3583.4 | 734 | 515.2 | 1057.1 |
| 310 | 71.1 | 164.3 | 307 | 1005.9 | 1091.8 | 92.5 | 194.1 | 204.9 |
| 311 | 819.5 | 309.6 | 177.4 | 119.9 | 48.5 | 805.6 | 148.5 | 343.4 |
| 312 | 35.7 | 488.7 | 553.3 | 5485.3 | 1957.5 | 229.2 | 282.3 | 1084.7 |
| 313 | 624.7 | 219.7 | 192.2 | 247.3 | 117.1 | 393.7 | 632.5 | 548.7 |
| 314 | 1060.4 | 958.7 | 775.5 | 190.2 | 164.3 | 1171 | 521.3 | 302.7 |
| 315 | 208.4 | 52.1 | 72 | 131.8 | 117.4 | 149.4 | 73.1 | 78.3 |
| 316 | 967.1 | 497.3 | 525.1 | 285.3 | 123.4 | 509.3 | 313.1 | 1093.3 |
| 317 | 1983.8 | 165 | 184.2 | 186.9 | 201.3 | 230.5 | 235.8 | 160.7 |
| 318 | 535.8 | 1159.4 | 335.7 | 57.8 | 34.2 | 275.6 | 327.6 | 202.1 |
| 319 | 303 | 615.9 | 648.6 | 565.7 | 660.6 | 420.7 | 486.2 | 82.1 |
| 320 | 250.3 | 171.3 | 229.3 | 898.4 | 581.8 | 283.3 | 182.8 | 91.2 |

| | | | | | | | | |
|---|---|---|---|---|---|---|---|---|
| 321 | 153.9 | 170.4 | 222.1 | 487.3 | 395.4 | 195.2 | 275.7 | 152.1 |
| 322 | 272.1 | 366 | 269.8 | 234.9 | 505.1 | 327 | 291.7 | 323.8 |
| 323 | 4149 | 2529.2 | 4790.2 | 11767.2 | 291.7 | 2101.2 | 1031.5 | 36.9 |
| 324 | 597.9 | 1026.6 | 425.6 | 216.6 | 66.4 | 520.2 | 374.4 | 38.3 |
| 325 | 600.8 | 475.6 | 489.7 | 427.7 | 333.3 | 614.4 | 461.4 | 102.3 |
| 326 | 6135.2 | 4645.8 | 2435.9 | 4623.7 | 489.7 | 3385.8 | 361.8 | 54.9 |
| 327 | 137 | 39.2 | 140.2 | 11.6 | 18.1 | 772 | 1166.8 | 47 |
| 328 | 139.5 | 41.9 | 42 | 35.3 | 115.4 | 264.6 | 82.5 | 28.1 |
| 329 | 45.1 | 69.1 | 52.6 | 106.4 | 190.4 | 59.3 | 53.4 | 158.6 |
| 330 | 1850.5 | 187.5 | 197.4 | 57.5 | 63.1 | 611.9 | 92.7 | 48.8 |
| 331 | 306.7 | 290.7 | 381.2 | 458.9 | 680.9 | 301.1 | 355.5 | 305.4 |
| 333 | 168.8 | 202.7 | 113.7 | 654.4 | 440.1 | 99.6 | 101.7 | 733.2 |
| 334 | 157.4 | 151.2 | 147.9 | 185.5 | 154.5 | 491.6 | 227.7 | 175.6 |
| 335 | 355 | 141.1 | 215.2 | 128.2 | 621.4 | 273.8 | 122.1 | 95.7 |
| 336 | 199.6 | 235 | 357 | 392.5 | 544.7 | 194.9 | 201.4 | 419.4 |
| 337 | 529.5 | 776.2 | 670.2 | 5029.2 | 12.6 | 247.4 | 1259.7 | 37.2 |
| 338 | 2393.6 | 467.5 | 1714.9 | 222.6 | 93.8 | 1228.2 | 573.6 | 80.8 |
| 339 | 268.9 | 201 | 224.4 | 249 | 562.8 | 317.5 | 248.1 | 316.4 |
| 340 | 108216.2 | 107282.5 | 151414.7 | 284.8 | 351.6 | 104374 | 125245.9 | 415.7 |
| 341 | 557.6 | 400 | 640.4 | 937.5 | 796.5 | 538.1 | 314.8 | 530.6 |
| 343 | 4739.2 | 4407 | 2670 | 1153.2 | 3880.9 | 3677.6 | 847.9 | 199.5 |
| 344 | 535.4 | 284.2 | 615.6 | 2191.3 | 1660.9 | 465.9 | 413.9 | 155.3 |
| 345 | 437.9 | 418.5 | 469.5 | 233.5 | 318 | 503.8 | 379.4 | 297.9 |
| 347 | 2204.6 | 607.2 | 2427.6 | 366.1 | 1391.9 | 1925.4 | 461.5 | 76.1 |
| 348 | 7719.8 | 2494 | 5392.3 | 378.6 | 2314.6 | 5267.1 | 1183.1 | 125.8 |
| 349 | 570.2 | 609 | 636 | 458.1 | 172.2 | 320.7 | 665.1 | 493.5 |
| 350 | 497.3 | 566.4 | 585.7 | 901.1 | 105.7 | 491.3 | 539.6 | 37.9 |
| 351 | 1925.2 | 1358.6 | 1065.7 | 133.7 | 96.1 | 1962.8 | 717.3 | 32.9 |
| 352 | 177.5 | 197.1 | 290.5 | 1585.5 | 569.8 | 165.4 | 101.5 | 302.3 |
| 353 | 734.5 | 602 | 399.5 | 357.6 | 924 | 415.5 | 202.7 | 175.3 |

3g

| SEQ ID NO: | 902TT | 903TT | 904TT | 907TT | 908TT | 909TT | 910TT | 913TT |
|---|---|---|---|---|---|---|---|---|
| 354 | 1260.9 | 3539.8 | 3818.1 | 9.2 | 180.3 | 221.3 | 64.7 | 837.9 |
| 355 | 83.5 | 5857.1 | 8246.9 | 48.4 | 247 | 60.3 | 53.8 | 151.4 |
| 356 | 105.3 | 500.4 | 419.6 | 781.6 | 797.8 | 102.4 | 278.5 | 575.7 |
| 357 | 249.4 | 689.9 | 517.8 | 798.6 | 569.9 | 361.7 | 600.7 | 655.3 |
| 358 | 975.3 | 2387.6 | 1281.2 | 823.5 | 857.3 | 1419.9 | 874.2 | 849.4 |
| 359 | 419.6 | 370.8 | 337.1 | 471.8 | 328.4 | 383.9 | 347.2 | 356.6 |
| 362 | 373.2 | 1448.2 | 359.4 | 401 | 473.1 | 781.6 | 303.4 | 262.5 |
| 360 | 268 | 446.7 | 558.4 | 242 | 366.4 | 1651.7 | 276.5 | 241.5 |
| 361 | 172.3 | 318.3 | 567.6 | 1196.1 | 670.7 | 316.5 | 236.7 | 426.2 |
| 363 | 1097.1 | 422.7 | 450.5 | 468.3 | 468.2 | 570.7 | 425.1 | 561.6 |
| 1 | 242.7 | 1509.1 | 565.3 | 1975.2 | 843.7 | 742.5 | 644.2 | 516.1 |
| 2 | 425.6 | 4753.1 | 2426.5 | 2640.2 | 3242 | 1866.5 | 1738.3 | 2465.6 |
| 7 | 96.7 | 1020.9 | 322.7 | 1442.2 | 913.4 | 2.1 | 688.8 | 390.1 |
| 8 | 1309.4 | 1177 | 1828.8 | 572.9 | 1092.8 | 1041.7 | 904 | 1781.9 |
| 9 | 496.7 | 2818.2 | 4872.6 | 1566 | 6508.7 | 2940.7 | 542 | 8678 |
| 10 | 150.7 | 81.4 | 218 | 97.4 | 90.5 | 118.5 | 139 | 57.6 |
| 11 | 623.6 | 578.7 | 535.7 | 283.7 | 341.2 | 219.9 | 168.8 | 310 |
| 13 | 173.4 | 526.7 | 1528.1 | 95.2 | 286 | 227.3 | 1018.4 | 322 |
| 14 | 424.5 | 1784.7 | 2754 | 255.4 | 584.8 | 1148.7 | 2517.6 | 721.5 |
| 15 | 192.6 | 231.1 | 6970.4 | 559.7 | 866.8 | 2354.9 | 135.8 | 2247.9 |
| 16 | 814.4 | 2337.1 | 1020.3 | 571 | 1207.1 | 216.6 | 1179.9 | 947 |
| 17 | 417 | 1095.8 | 1202.6 | 1279.3 | 1125.9 | 1162.1 | 297.9 | 1237.4 |

| | | | | | | | | |
|---|---|---|---|---|---|---|---|---|
| 19 | 320.6 | 619.6 | 2250.9 | 274.2 | 4651.6 | 16678.5 | 159.1 | 586.3 |
| 20 | 41.5 | 263.6 | 1643.5 | 94.1 | 70.4 | 35.5 | 1.3 | 42.3 |
| 21 | 128 | 2017.2 | 451.4 | 191.3 | 326.9 | 52.2 | 97.2 | 172.4 |
| 22 | 2089.5 | 4271.9 | 6617.2 | 4571.3 | 6361.4 | 2003.9 | 1809.4 | 7708.1 |
| 23 | 1.1 | 427.6 | 5067.6 | 455.9 | 966.7 | 2190.6 | 113.7 | 2551.2 |
| 24 | 140.7 | 218.4 | 963.7 | 245.9 | 328.3 | 456.7 | 114.4 | 572.7 |
| 26 | 110.4 | 1938 | 14240.4 | 1024 | 7609.6 | 17839.9 | 409.3 | 5874.4 |
| 27 | 736 | 1194.6 | 387.1 | 374.1 | 866.3 | 660.5 | 336.8 | 622.3 |
| 29 | 1746.3 | 207.1 | 150.7 | 96.2 | 129.5 | 375.2 | 87.3 | 132.6 |
| 30 | 60.4 | 204.1 | 7348.8 | 247 | 268.9 | 1830.2 | 51.2 | 900.4 |
| 31 | 250.5 | 4640.9 | 1272.4 | 54.2 | 361.6 | 246.2 | 125.6 | 11511.9 |
| 33 | 726.1 | 1138.2 | 22827.9 | 1763 | 1784.5 | 2090.6 | 172.6 | 8499.2 |
| 34 | 1224.5 | 1241.4 | 1284.7 | 2615.8 | 4040.9 | 1589 | 2170.1 | 3921.1 |
| 35 | 265.2 | 1740 | 1410.3 | 169.9 | 408.1 | 178.3 | 49.7 | 274.5 |
| 36 | 306.6 | 2846 | 753.6 | 141.1 | 241.3 | 516.5 | 112.2 | 307.4 |
| 37 | 441.8 | 182.5 | 666.4 | 164.8 | 262.4 | 355.8 | 217.6 | 253.3 |
| 38 | 390.4 | 378.8 | 13610.5 | 1317 | 946.6 | 761.7 | 296.8 | 5444.7 |
| 40 | 678 | 819.1 | 540.9 | 3014.7 | 512.9 | 629.2 | 400 | 556.2 |
| 41 | 644.1 | 499.6 | 729.1 | 155 | 425.8 | 379.6 | 508.2 | 453.1 |
| 42 | 291 | 418.9 | 301 | 279.7 | 205.7 | 234 | 453.1 | 140.6 |
| 43 | 2139.6 | 206.7 | 327.3 | 675.6 | 397 | 893.4 | 362.6 | 369.2 |
| 44 | 737 | 1115 | 1144.4 | 735.2 | 636.8 | 1352.5 | 377.3 | 421.9 |
| 45 | 1766.4 | 1105.2 | 390.4 | 587.3 | 440.9 | 35.5 | 1234.1 | 598.6 |
| 46 | 871.9 | 344.7 | 382.8 | 237.1 | 318 | 362.4 | 260.5 | 359.7 |
| 47 | 157.7 | 796 | 425.9 | 228.6 | 277.4 | 458.4 | 103.4 | 356.2 |
| 48 | 100.3 | 309.2 | 2616.3 | 124.4 | 634.6 | 1634.5 | 65.7 | 281.8 |
| 49 | 270.1 | 6992.3 | 2626.6 | 317.7 | 377.3 | 697.2 | 177.2 | 387.6 |
| 50 | 165.6 | 199.9 | 3637.3 | 652.6 | 305.4 | 478.1 | 71.2 | 1362.6 |
| 51 | 678.9 | 706.6 | 252.1 | 581.3 | 55.1 | 329.7 | 103.8 | 97.2 |
| 52 | 336 | 238.2 | 225.3 | 231.8 | 202.9 | 192.7 | 152.9 | 196.9 |
| 53 | 318.6 | 1664.1 | 277 | 119.1 | 102.4 | 73.8 | 168.1 | 127.6 |
| 54 | 230.9 | 1387 | 467.5 | 296.1 | 274.5 | 242.3 | 1255.6 | 479.9 |
| 55 | 100.5 | 82.4 | 82.1 | 91.1 | 106 | 121.4 | 99.6 | 99.4 |
| 56 | 18.8 | 321.8 | 83.3 | 126.2 | 51 | 49.7 | 267 | 79.7 |
| 57 | 2055.1 | 6894.7 | 1056.4 | 181 | 1040.6 | 898.5 | 320.9 | 6449.5 |
| 58 | 3611 | 7181.8 | 1246 | 192.6 | 956.8 | 1419.1 | 257.5 | 8484.9 |
| 59 | 652.5 | 2674.1 | 1061 | 813.9 | 1612.9 | 824.9 | 505.6 | 837.7 |
| 60 | 153.4 | 426.9 | 592.3 | 113.9 | 288.1 | 186.2 | 123.5 | 328.9 |
| 61 | 248.3 | 108 | 110.1 | 157.8 | 116.1 | 86.1 | 104.3 | 147.4 |
| 62 | 892.6 | 4240.6 | 2145.8 | 4532 | 1616.2 | 180.2 | 167.4 | 1671.5 |
| 63 | 43.1 | 696.1 | 228.6 | 152.7 | 248 | 203.2 | 222.5 | 1249.7 |
| 64 | 754.3 | 484.8 | 96.8 | 136.8 | 172.4 | 8787.9 | 99.3 | 336.8 |
| 65 | 83.2 | 218.3 | 730.9 | 106.4 | 207.9 | 403.2 | 75.9 | 215.4 |
| 66 | 493.5 | 1210 | 670.5 | 495.6 | 581.9 | 1176.5 | 951.4 | 686.8 |
| 67 | 10133.8 | 33324.8 | 24242 | 34583.3 | 33362.6 | 3068.3 | 31350 | 37066.4 |
| 68 | 2389.5 | 1201.4 | 1568 | 9401.9 | 2614.5 | 46.9 | 4918.3 | 1993.1 |
| 69 | 6293.3 | 2834.3 | 3650.8 | 13600.4 | 4671 | 198 | 6730.8 | 2110.2 |
| 70 | 7326.8 | 5218.4 | 1465.6 | 2301.9 | 1698 | 1562.6 | 3047.7 | 1292.1 |
| 71 | 109.3 | 5006.6 | 1167.7 | 193.9 | 743.9 | 3907.9 | 456.8 | 458.4 |
| 72 | 207.7 | 2313.9 | 1722.7 | 291.3 | 396.6 | 781 | 109 | 349.6 |
| 73 | 194.2 | 657.8 | 526.2 | 409.4 | 339.5 | 863 | 203.3 | 363.5 |
| 75 | 76.6 | 1227.8 | 331.8 | 313 | 290.4 | 824.9 | 86.8 | 175.5 |
| 76 | 2053.9 | 1453.3 | 198.2 | 1.4 | 166 | 90.4 | 4285.2 | 181.1 |
| 77 | 1780 | 1586 | 1427 | 6365.7 | 5262.6 | 227.6 | 2010.2 | 3182.5 |
| 78 | 481.4 | 317.2 | 437.6 | 789.5 | 715.2 | 564.6 | 535.8 | 202.4 |
| 79 | 461.6 | 214.6 | 214.2 | 96.5 | 110.5 | 1271 | 235.9 | 128.6 |
| 80 | 121.1 | 746.1 | 294 | 272.1 | 130.4 | 80.8 | 247.6 | 349.5 |
| 81 | 125.1 | 6473 | 2165.1 | 112.4 | 380.6 | 161.3 | 327.9 | 257.7 |

| | | | | | | | | |
|---|---|---|---|---|---|---|---|---|
| 82 | 704.3 | 216.5 | 171 | 431.3 | 318.7 | 149.5 | 340 | 308.5 |
| 84 | 221.9 | 611.2 | 399.9 | 105.1 | 228.8 | 2138.2 | 106 | 204.7 |
| 85 | 269.2 | 233.6 | 1982.8 | 169 | 149.9 | 211.7 | 137.7 | 320.8 |
| 86 | 135.5 | 108.9 | 106.9 | 95.7 | 105.4 | 93.8 | 73.7 | 117.7 |
| 87 | 127.6 | 465.3 | 323.2 | 95.3 | 403.9 | 62.8 | 157.6 | 1429.6 |
| 88 | 1283.6 | 2522.4 | 1743.7 | 11815.2 | 3616.8 | 446.2 | 3870.7 | 3025.4 |
| 89 | 256.7 | 275.1 | 477.6 | 154.3 | 289.3 | 541.1 | 134.8 | 307.1 |
| 90 | 116.5 | 131.5 | 622.1 | 230.7 | 165.3 | 129.6 | 92.9 | 171.2 |
| 91 | 1291.4 | 6737.9 | 681 | 75.2 | 942 | 526 | 142.5 | 8893.2 |
| 92 | 127.7 | 8921.7 | 3533.8 | 2067.5 | 4220.7 | 3041.6 | 9733.8 | 1770.2 |
| 93 | 120.4 | 181.9 | 160.9 | 277.6 | 309.8 | 97.6 | 147.2 | 189.7 |
| 94 | 1.3 | 950.7 | 333.4 | 626.4 | 1123.6 | 927.6 | 636 | 725.2 |
| 95 | 63.1 | 671.5 | 509.6 | 309 | 1194.2 | 87.6 | 1502.3 | 486 |
| 96 | 163.4 | 343.5 | 183.1 | 221 | 134.2 | 332.6 | 215.1 | 172.7 |
| 97 | 174.6 | 412.9 | 129.6 | 240.3 | 192.1 | 109.1 | 232 | 131.9 |
| 98 | 269 | 46.9 | 23.6 | 5.9 | 40.3 | 461.4 | 247.8 | 29.4 |
| 99 | 317.3 | 330.4 | 345.2 | 231.4 | 230 | 327.3 | 224.5 | 258.3 |
| 100 | 278.9 | 5487.5 | 4658.3 | 2977.6 | 17708.1 | 533.6 | 704.6 | 14117.2 |
| 101 | 148.9 | 470.7 | 172.2 | 240.4 | 366.1 | 137.6 | 433.1 | 297.3 |
| 102 | 267.2 | 130.3 | 143.5 | 163 | 138 | 256.5 | 184.4 | 174.4 |
| 103 | 345.9 | 454.6 | 332.2 | 322.3 | 296 | 331.9 | 319.9 | 317.1 |
| 104 | 620.8 | 254.8 | 155.1 | 500.3 | 426.6 | 538.2 | 415.4 | 490.4 |
| 105 | 676.6 | 264.2 | 231.1 | 551.9 | 371.6 | 831.1 | 467.2 | 475.1 |
| 106 | 832.7 | 268.5 | 316.9 | 494.6 | 516.3 | 767.4 | 474.1 | 471.9 |
| 107 | 214.1 | 396.6 | 1610.7 | 203.7 | 296.7 | 656.5 | 164.1 | 329.5 |
| 108 | 430.9 | 225.7 | 420.7 | 408.9 | 306.7 | 300.6 | 149.3 | 296.7 |
| 109 | 144.5 | 5293.3 | 3445.6 | 49.3 | 99.8 | 63.9 | 4951.6 | 88.8 |
| 110 | 156.2 | 13367.4 | 4936.4 | 1.2 | 133.1 | 66.6 | 10420.4 | 80.6 |
| 111 | 31.1 | 449 | 319.1 | 972.4 | 1746.3 | 72.4 | 634.6 | 1582.3 |
| 112 | 1208.7 | 915.8 | 358.6 | 513 | 445.2 | 79.8 | 1002.1 | 451.1 |
| 113 | 257 | 526.1 | 411.1 | 260 | 302.4 | 165.9 | 228.3 | 166.5 |
| 114 | 219.5 | 492.8 | 315.8 | 675.8 | 574.1 | 743.9 | 193.5 | 408.5 |
| 115 | 266.9 | 1153.1 | 535 | 357.9 | 489.5 | 1534.5 | 313.6 | 476.9 |
| 116 | 239.1 | 559.7 | 345 | 285.6 | 292.6 | 498.3 | 203 | 270.4 |
| 117 | 451.4 | 852.5 | 1167.1 | 3758.5 | 2636.4 | 220 | 694.6 | 1851.1 |
| 118 | 691.9 | 236.5 | 502.4 | 98.3 | 512.6 | 440.9 | 312.8 | 488.6 |
| 119 | 125.2 | 496.5 | 189.8 | 66 | 278.8 | 218.8 | 77.4 | 289.7 |
| 120 | 643.2 | 122 | 264.2 | 153.9 | 186.9 | 624.7 | 139.7 | 154 |
| 121 | 749.5 | 2913.7 | 860.2 | 336.8 | 764 | 1394.6 | 129.9 | 463.5 |
| 122 | 460.8 | 265.7 | 184.8 | 120.8 | 246 | 58.9 | 86.9 | 235.1 |
| 123 | 207.9 | 251.7 | 204.8 | 140.6 | 122.2 | 149.6 | 111.5 | 124.4 |
| 124 | 5670.9 | 155.5 | 148 | 368.2 | 165.8 | 183.3 | 107.3 | 167.1 |
| 125 | 1479.5 | 3998.5 | 753.1 | 813.4 | 967.4 | 1104.9 | 793.8 | 1004.9 |
| 126 | 762.9 | 199.8 | 558.9 | 519.5 | 1369.1 | 191.1 | 682.8 | 864.4 |
| 127 | 111.6 | 161.4 | 71.8 | 154.2 | 116.4 | 116.1 | 96.1 | 113.7 |
| 128 | 270.9 | 268.1 | 265.5 | 253.8 | 194.8 | 249.5 | 193.3 | 261.6 |
| 129 | 178.9 | 974.6 | 1280 | 192.4 | 245.5 | 216.2 | 155.5 | 1485 |
| 130 | 93.9 | 415 | 135.9 | 64 | 98.9 | 84.4 | 187.3 | 78.1 |
| 131 | 79.9 | 649 | 99.1 | 56.7 | 110.5 | 120.9 | 82.5 | 81.5 |
| 132 | 246.1 | 248.4 | 231.5 | 176.3 | 238.7 | 261 | 172.1 | 211 |
| 133 | 132.4 | 196 | 55.9 | 78.8 | 66.9 | 67.3 | 116.7 | 83.1 |
| 134 | 80.7 | 357.4 | 130 | 244.7 | 105.4 | 224.6 | 226.1 | 57.7 |
| 135 | 206.7 | 527.6 | 330.5 | 525.9 | 476.5 | 254.4 | 334.4 | 358.2 |
| 138 | 146.5 | 176.3 | 495.1 | 127.6 | 180.2 | 387 | 83.2 | 571.2 |
| 139 | 125.6 | 176.9 | 93.3 | 107.1 | 113.1 | 161 | 90.5 | 99 |
| 140 | 1.1 | 2124.9 | 375.1 | 3699.3 | 699.4 | 58.6 | 710.1 | 1623.8 |
| 141 | 39.2 | 1376.8 | 980.8 | 1472.7 | 1866.8 | 95.6 | 1265 | 1674.3 |
| 144 | 438.1 | 9635.2 | 440.9 | 420.6 | 469.1 | 364.9 | 21511.5 | 484.8 |

| | | | | | | | | |
|---|---|---|---|---|---|---|---|---|
| 145 | 295.9 | 239.9 | 182.7 | 181.3 | 218.7 | 151.5 | 152.5 | 257.7 |
| 146 | 298.9 | 424.8 | 178.8 | 181 | 250.5 | 196.8 | 180.3 | 248.8 |
| 147 | 48.8 | 115.3 | 160 | 38.6 | 86.5 | 34.7 | 27.4 | 52.8 |
| 148 | 301.5 | 1046.2 | 1135.9 | 223.2 | 407.2 | 165.1 | 181.9 | 387.1 |
| 149 | 47.3 | 241.2 | 214.2 | 458.8 | 455.6 | 29.3 | 104.5 | 303.3 |
| 150 | 170 | 537.9 | 266.4 | 204.9 | 218.7 | 213.6 | 102.9 | 241.9 |
| 151 | 89.2 | 551.3 | 254.9 | 165.8 | 534.6 | 208.7 | 145 | 347.2 |
| 153 | 86.5 | 128.6 | 144.8 | 102.4 | 114 | 131.9 | 482.5 | 102.5 |
| 154 | 89.9 | 4360.8 | 958.8 | 781.1 | 686.3 | 354.2 | 99.8 | 1301.7 |
| 155 | 231.3 | 491.3 | 148.5 | 113.4 | 129.9 | 135.7 | 457.7 | 250.3 |
| 156 | 106.4 | 562.9 | 116.7 | 63.7 | 114.5 | 94.8 | 103.6 | 100.3 |
| 157 | 215.1 | 148.4 | 161 | 134.5 | 165.7 | 219 | 111.3 | 154.4 |
| 158 | 32.9 | 1321.3 | 40.5 | 1.2 | 17.3 | 60.4 | 325.4 | 9.7 |
| 159 | 599.7 | 2783.1 | 1317.4 | 2648.2 | 4295.6 | 54 | 3352.4 | 1844.5 |
| 161 | 46.4 | 2664.3 | 142.6 | 105.4 | 37 | 79.3 | 12174.8 | 49 |
| 162 | 70.2 | 205.9 | 2154.4 | 120.2 | 230.1 | 302.5 | 26.7 | 575.3 |
| 164 | 97.2 | 4519.6 | 284.1 | 80.3 | 241.1 | 136.1 | 4278.6 | 145.2 |
| 166 | 1463.3 | 1937 | 2689 | 6646.8 | 6511.9 | 2436.2 | 4573.3 | 3272.4 |
| 167 | 213.4 | 1628.1 | 728.3 | 2340.5 | 4783.1 | 39.7 | 2419 | 1862.8 |
| 168 | 46.2 | 1573.6 | 390.5 | 1142.4 | 452.1 | 470.9 | 298.3 | 741.5 |
| 169 | 5537.1 | 13680.4 | 14101.2 | 2418 | 4999.2 | 901.3 | 1483.3 | 4751.3 |
| 170 | 246.1 | 2210.5 | 661.2 | 1664.4 | 916.3 | 216.4 | 2618.5 | 589.3 |
| 171 | 163.7 | 592.2 | 6989.4 | 403.5 | 2812.8 | 5944.7 | 1.2 | 2024.1 |
| 172 | 503.1 | 636.3 | 612.7 | 637.8 | 678.8 | 382.4 | 649.1 | 592.8 |
| 173 | 438.7 | 1492.9 | 435.8 | 581.6 | 451.6 | 406.1 | 375.7 | 321.6 |
| 176 | 1.2 | 4078.3 | 151.3 | 721.4 | 41.7 | 21.5 | 659.5 | 126.2 |
| 177 | 504.8 | 573.3 | 573.5 | 497.6 | 548.9 | 188.7 | 524.1 | 641 |
| 178 | 763.2 | 2581.3 | 274.3 | 130.1 | 130.5 | 395.3 | 1441.3 | 106 |
| 179 | 143.7 | 1098.5 | 182.3 | 83.9 | 71.1 | 89.7 | 62.5 | 528.9 |
| 180 | 146.2 | 930.7 | 373.3 | 106 | 151.6 | 300.6 | 164 | 186.4 |
| 181 | 9206.5 | 6564.9 | 6165.7 | 13167.3 | 11548.7 | 1304.8 | 7115.7 | 9061 |
| 182 | 263.8 | 376.5 | 668.2 | 414.6 | 507 | 1400.6 | 232.9 | 393.9 |
| 183 | 1.2 | 395.2 | 3392.4 | 216.2 | 953.9 | 3944.8 | 144.7 | 1249.2 |
| 184 | 228.5 | 3067.2 | 599.8 | 207 | 334.4 | 400.4 | 10162.8 | 380.1 |
| 185 | 931 | 4108 | 3749.7 | 1981.6 | 1615.9 | 3634.4 | 834.2 | 6945.4 |
| 186 | 493.3 | 324.2 | 396.2 | 656.7 | 709.1 | 1183.3 | 453.3 | 462 |
| 187 | 96.3 | 258.3 | 1535.7 | 409.2 | 314.3 | 2757.2 | 67.4 | 650.9 |
| 188 | 66.9 | 405.5 | 832.9 | 151.3 | 247.2 | 306.9 | 89.3 | 144.4 |
| 189 | 70.6 | 239.2 | 289.3 | 112 | 199.7 | 237.6 | 59 | 78.6 |
| 190 | 1.3 | 1113.6 | 243.5 | 38 | 52 | 103.1 | 101.1 | 159.9 |
| 191 | 898.6 | 2228 | 2806.6 | 1788.2 | 1533.1 | 466 | 498.6 | 1912.7 |
| 192 | 103.7 | 377 | 267 | 65.3 | 134.7 | 128.9 | 66.1 | 119.5 |
| 193 | 140.5 | 752.9 | 580.6 | 2185.8 | 876.8 | 67.5 | 271.5 | 561.8 |
| 194 | 60.2 | 320.3 | 198.5 | 159.6 | 173.5 | 96 | 126.9 | 140.9 |
| 195 | 308.8 | 210.3 | 178.6 | 401.4 | 266.1 | 175.7 | 367.5 | 230.8 |
| 196 | 5889.2 | 6514.4 | 4504.5 | 4416.3 | 7685 | 183.7 | 6854.2 | 7068.9 |
| 197 | 374.5 | 81.9 | 129.2 | 108.8 | 122 | 174.9 | 68.1 | 178.1 |
| 198 | 95.3 | 300 | 73.2 | 42.5 | 55.8 | 86.5 | 39.2 | 77.1 |
| 199 | 87.1 | 7825.9 | 10594.6 | 51.3 | 344.6 | 74.9 | 63 | 274.1 |
| 200 | 130.8 | 511 | 514 | 170.4 | 544.7 | 475.6 | 154.9 | 259.7 |
| 201 | 1208.7 | 3427.4 | 3404.4 | 38.8 | 160.1 | 168.6 | 42.3 | 918.4 |
| 203 | 6000.1 | 982.6 | 359.1 | 96.8 | 204.8 | 46.1 | 331.8 | 188.3 |
| 204 | 206 | 700.4 | 262.9 | 650.9 | 591.3 | 846.3 | 674.6 | 437.2 |
| 205 | 1762.1 | 5866.2 | 3715.3 | 2132.7 | 5805.3 | 149.5 | 5381.1 | 2955.4 |
| 206 | 597.2 | 208.1 | 13097.8 | 301.2 | 1950.7 | 2675.5 | 252.6 | 329.8 |
| 207 | 77.6 | 602.1 | 745.6 | 352.7 | 484.2 | 308.4 | 1182.9 | 575 |
| 208 | 283.8 | 306.2 | 451.2 | 156.5 | 329.6 | 1044.1 | 156.9 | 149.8 |
| 209 | 254.3 | 1864.3 | 892.2 | 4970.4 | 5671.9 | 5534 | 2026.3 | 2662.4 |

| | | | | | | | | |
|---|---|---|---|---|---|---|---|---|
| 211 | 262.3 | 3358.1 | 4615 | 29093.4 | 15999.8 | 393.6 | 7278.5 | 12689.2 |
| 212 | 1608.1 | 1431.5 | 332.2 | 551.6 | 514.2 | 482.4 | 786.4 | 353.9 |
| 213 | 3281.6 | 97.3 | 108.3 | 107.5 | 105.4 | 160 | 99.5 | 104.5 |
| 215 | 89.6 | 862.3 | 617 | 432.2 | 796.7 | 441.3 | 1738.3 | 806.2 |
| 216 | 333.1 | 284 | 385.1 | 651 | 910.8 | 264.4 | 588 | 601.9 |
| 217 | 224.3 | 1692 | 513.5 | 290.3 | 372.2 | 448.4 | 238.7 | 272.8 |
| 218 | 271.4 | 191 | 194.3 | 230.9 | 353.1 | 288.6 | 279.7 | 263.7 |
| 220 | 26824.2 | 132 | 73.9 | 124.1 | 470.8 | 129.2 | 103.4 | 350.5 |
| 221 | 215 | 1009.5 | 780.2 | 674.9 | 632.3 | 1487 | 406.7 | 578.3 |
| 223 | 352.8 | 388.2 | 438 | 286.9 | 203.4 | 160.1 | 124.3 | 1200.6 |
| 227 | 565.2 | 503.9 | 16406 | 1473.6 | 1813.5 | 2226.4 | 314.3 | 5151.9 |
| 228 | 624.4 | 455.2 | 331.2 | 808.6 | 799.2 | 205.8 | 683.1 | 604 |
| 229 | 1426.6 | 209.6 | 275.1 | 972.8 | 180.9 | 357.1 | 249.2 | 538.6 |
| 230 | 281.7 | 1542.1 | 611.5 | 209 | 207.8 | 215.8 | 97 | 574 |
| 231 | 2885.2 | 380.6 | 200 | 67.8 | 164.2 | 95.5 | 166.1 | 101.5 |
| 232 | 58 | 1351.9 | 13448.1 | 771.8 | 6104.8 | 19484.1 | 294.7 | 4379.3 |
| 233 | 2461.3 | 14608.9 | 11362.4 | 5629.1 | 11330.5 | 8712.7 | 1500.9 | 5870.1 |
| 235 | 317.4 | 465.1 | 5668.6 | 250.5 | 1690.5 | 2382.3 | 71.8 | 932.8 |
| 236 | 179.8 | 493.3 | 282.1 | 229.7 | 368.1 | 466.5 | 98.5 | 194.2 |
| 237 | 734 | 1979.9 | 2226.6 | 2347 | 1794.9 | 1757.7 | 790 | 2552.2 |
| 238 | 269.2 | 1084.1 | 803.1 | 718.3 | 1198.8 | 688.1 | 706.9 | 861.9 |
| 239 | 101.2 | 896.8 | 1025.8 | 156.8 | 1210.1 | 429.8 | 176 | 647.7 |
| 240 | 117.5 | 1116.1 | 1919.1 | 210.2 | 1169.1 | 420.8 | 189.3 | 1162.5 |
| 241 | 173.9 | 2109.5 | 3105.6 | 444 | 2419 | 956.7 | 341 | 2001 |
| 242 | 43673.6 | 18485.8 | 19181.5 | 4555.3 | 1630.4 | 3478.5 | 1273.2 | 10481.9 |
| 243 | 157.3 | 341.9 | 1232.7 | 219.9 | 483 | 699.2 | 82.1 | 1003.1 |
| 244 | 115 | 746.8 | 224.6 | 148.2 | 196.6 | 321 | 261 | 166.4 |
| 246 | 153.2 | 1046.6 | 224.3 | 103.9 | 143.7 | 173.8 | 1360.9 | 276.9 |
| 247 | 446 | 126.1 | 129.3 | 102.9 | 139 | 134.8 | 77.7 | 138.1 |
| 248 | 202.4 | 3011.8 | 309.9 | 182.5 | 128.8 | 133.9 | 1016.4 | 127.7 |
| 249 | 196.3 | 2527.3 | 1726.5 | 1395.5 | 337.1 | 271.3 | 233.7 | 469.7 |
| 251 | 386.5 | 1104.2 | 346.8 | 127.5 | 90.8 | 190.6 | 175.2 | 116.5 |
| 253 | 173.5 | 286.7 | 365.9 | 263 | 403.6 | 544.3 | 306.8 | 269.1 |
| 254 | 121 | 1348.6 | 394.8 | 73 | 434.2 | 259.8 | 554.3 | 264.1 |
| 255 | 953.8 | 2792.9 | 253.9 | 137.5 | 120.4 | 451.3 | 1497.2 | 98.4 |
| 256 | 1230.9 | 5411.2 | 704.5 | 85.2 | 918.6 | 465.8 | 224.1 | 7960.4 |
| 257 | 196 | 284.4 | 209 | 154.9 | 200.8 | 57.7 | 75.2 | 165.8 |
| 258 | 596.3 | 1068.8 | 1617.2 | 24.7 | 209.4 | 33.6 | 45.7 | 129.9 |
| 259 | 3.3 | 934.8 | 2120.9 | 131.2 | 687.3 | 806.3 | 46 | 531.9 |
| 260 | 248.3 | 182.9 | 167.4 | 127.1 | 173.3 | 209.8 | 128.2 | 198.6 |
| 261 | 19 | 401.9 | 345.1 | 1066.3 | 184.5 | 629.8 | 66.6 | 326.5 |
| 263 | 3859.5 | 516.2 | 662.8 | 577.5 | 720.7 | 535.7 | 488.6 | 407 |
| 263 | 418.6 | 1350.1 | 772.4 | 214.4 | 221.7 | 353.8 | 173.2 | 599.8 |
| 265 | 369.8 | 167.3 | 372.9 | 453.3 | 248.6 | 93.4 | 268.9 | 414.8 |
| 266 | 1779.8 | 263 | 318.1 | 407 | 491.3 | 511.1 | 236.7 | 401.1 |
| 256 | 117.2 | 391.4 | 130.1 | 92.5 | 181.8 | 142.5 | 146 | 152 |
| 268 | 120.1 | 1119 | 310.7 | 819.2 | 1655.3 | 121.7 | 645.8 | 1264.1 |
| 269 | 333.7 | 121.5 | 82.3 | 48 | 46.9 | 55.7 | 27.4 | 63.7 |
| 270 | 521.6 | 206.4 | 175.2 | 475.9 | 301.9 | 165.7 | 234.5 | 239.6 |
| 271 | 251.7 | 2043.7 | 362.9 | 255.5 | 197.7 | 227 | 225.6 | 192.4 |
| 272 | 620.4 | 563.5 | 600.9 | 411.3 | 451 | 639.9 | 426.2 | 524.5 |
| 273 | 136.7 | 2008 | 783.2 | 9402.2 | 3170.7 | 37.5 | 2967.5 | 1970.3 |
| 274 | 436.6 | 1279.4 | 1048.7 | 803.4 | 803.2 | 1605 | 278.3 | 616.8 |
| 275 | 267.1 | 1400.8 | 971.4 | 450.3 | 462.3 | 1341.1 | 238.2 | 2631.7 |
| 276 | 711.3 | 764 | 25930 | 2051.7 | 3808.8 | 5060.2 | 400.3 | 7898.3 |
| 277 | 188.1 | 213.8 | 210 | 151.7 | 217.1 | 196.7 | 161.8 | 199.4 |
| 278 | 65.7 | 1341 | 3512.1 | 262.6 | 1386.1 | 710.4 | 64.3 | 510.5 |
| 279 | 577.5 | 3255 | 2105.6 | 1256.3 | 3760.9 | 49.6 | 3058.3 | 2014.5 |

| | | | | | | | | |
|---|---|---|---|---|---|---|---|---|
| 280 | 876.1 | 4218.8 | 2673.7 | 1739.2 | 3129.6 | 176.5 | 3883.1 | 2142.3 |
| 281 | 33.4 | 653.2 | 169.7 | 48.5 | 118.7 | 39 | 363.8 | 289 |
| 282 | 387.6 | 472.5 | 331.7 | 252.3 | 284.5 | 371.6 | 219.7 | 694.4 |
| 283 | 584.8 | 297.3 | 224 | 322.6 | 357.9 | 395.3 | 382.4 | 244 |
| 284 | 124.9 | 130.1 | 89.2 | 53.1 | 74.7 | 68.2 | 61.3 | 104.6 |
| 285 | 154 | 100.9 | 114.9 | 90.5 | 98.7 | 114.7 | 98.4 | 113.9 |
| 286 | 14.5 | 91.5 | 472.1 | 1032.5 | 277 | 1968 | 1525.5 | 442.2 |
| 287 | 744.2 | 114.2 | 131.4 | 94.7 | 147.1 | 127.5 | 65.1 | 206.5 |
| 288 | 245.8 | 103.9 | 143.6 | 161.5 | 162.8 | 177.7 | 112.5 | 161.4 |
| 289 | 231.3 | 5107.2 | 4629.4 | 93.7 | 1685.9 | 4764.5 | 141.9 | 2073.7 |
| 291 | 67.2 | 135.6 | 201.2 | 185.4 | 144.5 | 245.6 | 73.8 | 128.4 |
| 292 | 43.6 | 1910.5 | 2535.8 | 72.3 | 371.6 | 4192.7 | 50.7 | 206.7 |
| 293 | 63.9 | 1213.8 | 284.1 | 54.5 | 117 | 102.3 | 188.8 | 561 |
| 295 | 329 | 1994.3 | 668.6 | 211.8 | 267.3 | 97.1 | 684.5 | 3454 |
| 296 | 1451.1 | 1263.3 | 1341.5 | 761.3 | 1067.5 | 1967.3 | 758.9 | 687.7 |
| 297 | 593 | 73.8 | 109.1 | 49.4 | 123.5 | 88 | 42.9 | 364.8 |
| 298 | 360 | 444.6 | 275.9 | 300.9 | 273.2 | 358.5 | 306.5 | 436 |
| 299 | 1094.3 | 2223.7 | 794.8 | 1242.1 | 1051.7 | 1871.6 | 1233.7 | 605.2 |
| 300 | 186 | 1200.8 | 322.3 | 408.7 | 137.5 | 150.8 | 1157.7 | 233.1 |
| 301 | 250.2 | 525.4 | 365.9 | 4246.8 | 594.8 | 155.1 | 545.6 | 329.5 |
| 302 | 28.2 | 82.4 | 44.3 | 22.7 | 36.1 | 25.7 | 6.3 | 27.7 |
| 303 | 1397.3 | 375.7 | 390.8 | 921 | 836.9 | 531.6 | 689.2 | 589 |
| 304 | 485.2 | 1102.9 | 799.5 | 659.6 | 239.7 | 1106.9 | 120.6 | 501.1 |
| 305 | 1523.9 | 661.1 | 201.8 | 144.2 | 192.3 | 247.8 | 195.8 | 173 |
| 306 | 222.8 | 363.6 | 184.3 | 150.2 | 197.8 | 184.8 | 128.9 | 208.3 |
| 307 | 180.1 | 571.1 | 188 | 169.2 | 150.6 | 127.7 | 165.3 | 198 |
| 308 | 3026.7 | 857.8 | 1219.5 | 1191.4 | 1726.2 | 64.1 | 1855.4 | 1201.9 |
| 310 | 932.8 | 136.9 | 271.1 | 1036.8 | 498.7 | 99 | 290.2 | 343.2 |
| 311 | 48.5 | 360 | 99.1 | 180.9 | 152.4 | 76.1 | 167.7 | 104.5 |
| 312 | 4628 | 5 | 236.7 | 2257.7 | 1039.8 | 66 | 643.8 | 643 |
| 313 | 126.7 | 420.1 | 135.6 | 121 | 78.1 | 101 | 622.3 | 132.7 |
| 314 | 119.1 | 1765 | 872.1 | 133.6 | 629.1 | 211.4 | 112.9 | 473 |
| 315 | 141.7 | 212.9 | 441.6 | 286.4 | 519.9 | 1377.8 | 271.7 | 192.2 |
| 316 | 119.8 | 562.5 | 354.9 | 220.7 | 270.5 | 441.8 | 189.1 | 436.3 |
| 317 | 178.4 | 694.3 | 216.3 | 177.3 | 204.7 | 218.1 | 156.9 | 178.7 |
| 318 | 28.3 | 646.1 | 105.3 | 30.6 | 143.4 | 43.8 | 88.5 | 101.1 |
| 319 | 1682.7 | 339.1 | 154 | 339.5 | 190.3 | 129.4 | 447.1 | 293.2 |
| 320 | 816.1 | 326.7 | 425.5 | 913.6 | 680.7 | 459.9 | 482.1 | 501.5 |
| 321 | 360.2 | 245.2 | 811.4 | 486.9 | 413.3 | 931.3 | 260.1 | 454.2 |
| 322 | 604.1 | 279.1 | 205.3 | 203 | 203.2 | 198.1 | 215.6 | 180.1 |
| 323 | 620.6 | 4592.1 | 1478.5 | 13669.8 | 1883 | 297 | 3464 | 1551 |
| 324 | 106.1 | 478.7 | 118.1 | 131.8 | 77.6 | 74.8 | 294.8 | 56.2 |
| 325 | 255.2 | 515.4 | 238.4 | 250.8 | 279.1 | 142.4 | 597.5 | 247.7 |
| 326 | 318.5 | 3462.4 | 2655.2 | 2527.1 | 3852.6 | 220.7 | 891.6 | 2803.7 |
| 327 | 64.1 | 44.3 | 86.6 | 1.3 | 52 | 25.3 | 11.5 | 100.1 |
| 328 | 58.8 | 534.2 | 264.2 | 543.1 | 719.4 | 95.4 | 544.4 | 515.6 |
| 329 | 122.4 | 66.8 | 60.1 | 62.8 | 59.5 | 91.3 | 45.4 | 74.1 |
| 330 | 49 | 1131.8 | 126.4 | 41.5 | 57.5 | 131.5 | 31.9 | 64 |
| 331 | 632.1 | 258 | 263 | 307.2 | 275.1 | 480 | 248.3 | 284.1 |
| 333 | 147 | 285.7 | 369.3 | 854.9 | 519 | 285.3 | 603 | 413.2 |
| 334 | 129 | 302.9 | 300.5 | 121 | 170.3 | 145.9 | 129.4 | 211.5 |
| 335 | 215.5 | 628.5 | 174.5 | 108 | 107.2 | 135.9 | 51.5 | 116.8 |
| 336 | 358.7 | 245.7 | 230.7 | 382.1 | 500.4 | 819.1 | 394.5 | 415.4 |
| 337 | 105.8 | 1112.7 | 1023.2 | 358 | 235.6 | 1517.6 | 97.2 | 364 |
| 338 | 134.6 | 567.8 | 499.9 | 131.2 | 238.2 | 332.7 | 132 | 278.1 |
| 339 | 335.4 | 352.2 | 311 | 385 | 625.4 | 323.2 | 314.7 | 428.2 |
| 340 | 303.2 | 343.2 | 263.9 | 231 | 263.3 | 223.5 | 177.2 | 315.9 |
| 341 | 408.4 | 464.7 | 438.1 | 1146.6 | 803.2 | 349.7 | 511.1 | 757.8 |

| | | | | | | | | |
|---|---|---|---|---|---|---|---|---|
| 343 | 1287 | 814.6 | 228 | 123.5 | 59.8 | 75.2 | 658.4 | 97.9 |
| 344 | 2683.4 | 670.4 | 822.4 | 1937.2 | 1561.2 | 1319.5 | 1187.9 | 1110.2 |
| 345 | 270.8 | 356.3 | 270.7 | 213.6 | 219.2 | 281.7 | 199.4 | 223.6 |
| 347 | 321.3 | 3161.8 | 1516.8 | 2286.9 | 3928.3 | 682.1 | 1998.6 | 3960.1 |
| 348 | 592.8 | 6727.1 | 3096.6 | 4465.5 | 7652.3 | 3345.7 | 4616.8 | 6087.1 |
| 349 | 213.8 | 458.9 | 441.6 | 165.6 | 239.7 | 177.8 | 310.8 | 270.7 |
| 350 | 139.5 | 477.9 | 625.9 | 282.7 | 381.8 | 1996.1 | 328.4 | 149.7 |
| 351 | 45.6 | 2277.1 | 515.7 | 158 | 453.8 | 105.4 | 294.7 | 668.6 |
| 352 | 470.6 | 167.1 | 213.9 | 516.8 | 305.7 | 2359 | 246.1 | 210.6 |
| 353 | 792.6 | 308.9 | 297.2 | 170.8 | 260.9 | 153.9 | 208.2 | 337.5 |

3h

| SEQ ID NO: | 914TT | 915TT | 917TT | 918TT | 919TT | 920TT | 921TT | 922TT |
|---|---|---|---|---|---|---|---|---|
| 354 | 71.9 | 52.3 | 102.4 | 6332.8 | 339.5 | 28.3 | 586.4 | 1.1 |
| 355 | 77.9 | 90.7 | 59.8 | 865.8 | 218.8 | 75.4 | 827.6 | 77.1 |
| 356 | 309.5 | 389.3 | 791.7 | 762.2 | 694.7 | 578.2 | 1581.5 | 949.2 |
| 357 | 238.7 | 565.6 | 645.2 | 593.2 | 611.7 | 800.3 | 666.9 | 768.9 |
| 358 | 812.3 | 933.3 | 620.4 | 729.2 | 758.3 | 710.1 | 556.9 | 571.9 |
| 359 | 399.3 | 508 | 376.9 | 330 | 320.8 | 353.6 | 299.6 | 506 |
| 362 | 527.8 | 508.3 | 517.3 | 1171.8 | 669.6 | 758.3 | 407.4 | 431.6 |
| 360 | 287.6 | 262.1 | 276.8 | 458.5 | 477.6 | 256.8 | 248.9 | 297.3 |
| 361 | 153.4 | 816.8 | 853.1 | 453.3 | 532.5 | 1170.9 | 1300 | 542.2 |
| 363 | 631.4 | 726.4 | 557.1 | 448.6 | 429.4 | 599.4 | 429.9 | 590.7 |
| 1 | 770.6 | 362.7 | 797.9 | 661.6 | 799.3 | 756.1 | 848.9 | 856.2 |
| 2 | 481 | 3910.3 | 1810.9 | 1338.3 | 3134.1 | 3947 | 4719 | 2783.8 |
| 7 | 299.1 | 2389.1 | 790.1 | 343.4 | 419.8 | 1494.7 | 829.3 | 845.9 |
| 8 | 614.3 | 810.2 | 763.6 | 1902.7 | 1081.7 | 629.1 | 831.6 | 938.2 |
| 9 | 524.5 | 1890.1 | 9055.3 | 9890.7 | 8192.7 | 2264.6 | 3685.8 | 10424.8 |
| 10 | 169.8 | 317.6 | 42 | 79.2 | 58.4 | 136.2 | 120 | 40.5 |
| 11 | 146 | 141.2 | 351.8 | 282.6 | 234.9 | 269.5 | 274.6 | 155.7 |
| 13 | 104.4 | 96.3 | 115.4 | 412.7 | 501.8 | 91 | 136.4 | 182.4 |
| 14 | 150.5 | 149.2 | 276.7 | 1839.8 | 1222 | 149.8 | 335.3 | 427.4 |
| 15 | 83.6 | 639.9 | 331.3 | 203.1 | 1168.9 | 304.5 | 277.5 | 582.7 |
| 16 | 834 | 740.5 | 1080.2 | 1040.9 | 977.4 | 761 | 1018.5 | 1197.7 |
| 17 | 95.8 | 775.8 | 1324.9 | 1290.5 | 1245.3 | 1170.3 | 1306.3 | 917.3 |
| 19 | 261.4 | 404.1 | 541.1 | 316.8 | 4263.1 | 155.9 | 150.6 | 3899.9 |
| 20 | 1.3 | 44.2 | 15.6 | 83.6 | 83 | 31 | 1.4 | 1.1 |
| 21 | 383.4 | 16.8 | 145.6 | 554.6 | 432 | 35.5 | 42.5 | 144.6 |
| 22 | 483.4 | 7044.6 | 9872.2 | 5621.2 | 6671.8 | 7593.9 | 4117.5 | 6209.5 |
| 23 | 1.2 | 758 | 271.8 | 277.7 | 2394.8 | 1.1 | 325.3 | 627.9 |
| 24 | 29 | 304.7 | 103.5 | 271.5 | 469.3 | 49.8 | 178.9 | 196.8 |
| 26 | 161.3 | 2148.7 | 2238.7 | 435.1 | 15713.7 | 60.3 | 774.4 | 7022.3 |
| 27 | 2027.3 | 253.6 | 510.6 | 701 | 703.8 | 324.3 | 324 | 728.4 |
| 29 | 167.6 | 74.4 | 68.6 | 190.8 | 125.6 | 73.1 | 84.8 | 170.1 |
| 30 | 1.1 | 246 | 188 | 525.7 | 759.9 | 74.5 | 129.7 | 286.6 |
| 31 | 256.3 | 146 | 449.7 | 1402.9 | 328.4 | 110.8 | 218.2 | 222.7 |
| 33 | 69.4 | 1248.9 | 534.4 | 1635.5 | 1154 | 322.1 | 691.6 | 434.8 |
| 34 | 2249.5 | 2411.8 | 4154.5 | 3039.8 | 4215.7 | 4564.8 | 2908.5 | 3979 |
| 35 | 118.8 | 296.6 | 55.5 | 1050.9 | 413 | 100.2 | 126.5 | 95 |
| 36 | 230.8 | 313.4 | 160.9 | 183.5 | 250.9 | 153.4 | 140.4 | 242.6 |
| 37 | 313.2 | 317.7 | 214.6 | 190.8 | 269.8 | 183.3 | 173.6 | 197.9 |
| 38 | 61.3 | 851.9 | 351.3 | 1044.4 | 981.7 | 465.1 | 612.9 | 276.6 |
| 40 | 719.3 | 813.3 | 549.5 | 535.4 | 513 | 829.2 | 442.3 | 543.7 |
| 41 | 172.6 | 171 | 190 | 284.7 | 581.1 | 145.2 | 362.8 | 549.8 |
| 42 | 343.9 | 273.7 | 144 | 130.6 | 210.4 | 229.7 | 202.5 | 160 |

| | | | | | | | | |
|---|---|---|---|---|---|---|---|---|
| 43 | 1155.5 | 721.6 | 513.5 | 373.6 | 260 | 617.8 | 269.7 | 355.8 |
| 44 | 467.8 | 606.3 | 633.6 | 727.9 | 790.8 | 640 | 420.7 | 335.6 |
| 45 | 1102.5 | 638.1 | 519.3 | 594.7 | 368.8 | 478.2 | 608.5 | 404.8 |
| 46 | 443.3 | 467.6 | 402.9 | 285.6 | 318.8 | 295.6 | 260.9 | 360.4 |
| 47 | 246.9 | 143.9 | 102.7 | 241.9 | 285.2 | 145.3 | 226 | 182.5 |
| 48 | 48.2 | 197.7 | 222.2 | 49.7 | 1024.9 | 23 | 23 | 465.8 |
| 49 | 234.5 | 355.6 | 207.3 | 1446.9 | 736.1 | 344.1 | 252.2 | 364.3 |
| 50 | 42 | 399.9 | 174.3 | 301.5 | 536.5 | 71.4 | 207.3 | 223.1 |
| 51 | 444.2 | 59.2 | 128.9 | 228.8 | 73.7 | 65.9 | 19.4 | 52.1 |
| 52 | 203.3 | 187.7 | 224 | 202.7 | 200.7 | 180.8 | 198.3 | 190.4 |
| 53 | 186.9 | 1.2 | 213.7 | 202.1 | 69.2 | 75.4 | 108.4 | 128.1 |
| 54 | 684.7 | 417.1 | 501.3 | 720.5 | 410.5 | 294 | 509.8 | 366.4 |
| 55 | 129.4 | 96.5 | 115.6 | 98.9 | 115.5 | 100.7 | 94.9 | 72.2 |
| 56 | 247.2 | 93.8 | 50 | 62 | 65.7 | 100.5 | 74 | 62.3 |
| 57 | 713.4 | 745.5 | 417.1 | 4396.2 | 674.3 | 127.1 | 1311.9 | 226 |
| 58 | 679.3 | 672.7 | 334.5 | 5289.1 | 905 | 113.7 | 1260.3 | 158.8 |
| 59 | 1114.3 | 488.5 | 1847.4 | 1440.1 | 1675.2 | 906.2 | 2348 | 1103.1 |
| 60 | 160.5 | 166.8 | 216.5 | 231.6 | 179.7 | 142.2 | 165.1 | 203.4 |
| 61 | 293.8 | 239.7 | 136.1 | 128 | 145.9 | 155.4 | 122.5 | 116.8 |
| 62 | 2975 | 2562.6 | 1652.4 | 1873.2 | 1527 | 526 | 60.4 | 3206.2 |
| 63 | 144.6 | 161.3 | 490.3 | 578.9 | 239.3 | 242 | 239.6 | 251.9 |
| 64 | 1905.1 | 179 | 138 | 290.9 | 184.1 | 124 | 138.2 | 156.2 |
| 65 | 54 | 106 | 187.6 | 155.7 | 243.6 | 75 | 166.3 | 146.9 |
| 66 | 676.3 | 527.1 | 840.5 | 1026.2 | 765.5 | 627.5 | 1284.4 | 290.3 |
| 67 | 33082 | 52249.5 | 37966 | 30570.8 | 32361.9 | 42996.5 | 29286 | 39242.9 |
| 68 | 4579.1 | 7275.8 | 2857.3 | 1137.2 | 4016.2 | 7822.9 | 2361.2 | 3436.9 |
| 69 | 7472.6 | 15249.7 | 5078.7 | 2335.4 | 5252.6 | 10446.3 | 7213.9 | 6679.9 |
| 70 | 9607.7 | 1458 | 3227.3 | 1337.8 | 1473.1 | 1664.6 | 444 | 1375.9 |
| 71 | 234 | 129.9 | 307.4 | 332.1 | 2083.3 | 87 | 105.8 | 493.5 |
| 72 | 117.7 | 616.5 | 195.6 | 1937.6 | 446.3 | 359.7 | 707 | 166.5 |
| 73 | 299.5 | 310.6 | 290.6 | 264.1 | 473 | 267.6 | 370.2 | 457.7 |
| 75 | 167.6 | 215.7 | 252.1 | 193 | 312.5 | 406 | 77.3 | 143.2 |
| 76 | 987.1 | 33.3 | 18.9 | 74.9 | 191.4 | 9.9 | 122.1 | 134.1 |
| 77 | 3486.8 | 9405.2 | 5866.2 | 3917.2 | 3054.2 | 10710.9 | 3845.4 | 7445 |
| 78 | 322.9 | 806.6 | 1125.4 | 721.3 | 791.7 | 1090.1 | 887.2 | 548.7 |
| 79 | 156.3 | 75.4 | 84.6 | 199.2 | 122.3 | 105 | 76.6 | 133.8 |
| 80 | 577.4 | 115.1 | 104.4 | 407.6 | 226.3 | 186.6 | 758.9 | 124.9 |
| 81 | 120.4 | 121.1 | 187.8 | 1222.5 | 315.8 | 185 | 177.3 | 358 |
| 82 | 480.2 | 436.7 | 398.1 | 285.9 | 256.1 | 480.6 | 350.3 | 306.7 |
| 84 | 122.6 | 108.4 | 185.4 | 180.2 | 503.7 | 117.8 | 89.6 | 217.3 |
| 85 | 172.1 | 158.7 | 146.6 | 1050.6 | 166.4 | 134.4 | 197.3 | 142.6 |
| 86 | 99.3 | 87.7 | 80.9 | 78.1 | 93.6 | 56 | 77.2 | 98.4 |
| 87 | 346.4 | 214.6 | 478.3 | 693.6 | 282.7 | 172 | 329.4 | 231.5 |
| 88 | 2119.7 | 12099.4 | 6271.8 | 3285.9 | 4646.7 | 8968.2 | 1915.3 | 9385.7 |
| 89 | 308.6 | 290.4 | 303 | 232.4 | 299.7 | 233.4 | 163.8 | 190.9 |
| 90 | 98.7 | 279.4 | 108.4 | 89 | 113.4 | 81.7 | 100.6 | 149.5 |
| 91 | 582.6 | 482.3 | 275 | 6847.8 | 528.1 | 61.1 | 1397.9 | 71.5 |
| 92 | 2641.5 | 1419.6 | 2112.8 | 3432 | 3134.2 | 1442.4 | 4677.3 | 2547 |
| 93 | 88.7 | 211.3 | 338.9 | 402.7 | 236.9 | 286.5 | 297.3 | 310.6 |
| 94 | 89.7 | 1072.4 | 937.7 | 516.7 | 1228 | 974.1 | 1427 | 1237.6 |
| 95 | 510.2 | 756.1 | 652.5 | 461.5 | 647.6 | 779.5 | 500.1 | 747.8 |
| 96 | 142.3 | 129.6 | 170.2 | 169.9 | 161.5 | 179.1 | 155.6 | 168.2 |
| 97 | 169.9 | 186.3 | 239.2 | 240.4 | 173.7 | 176.8 | 317.9 | 243.4 |
| 98 | 37 | 25.2 | 39.9 | 87.1 | 52.8 | 25.5 | 28.4 | 39.9 |
| 99 | 287.4 | 268.3 | 235.3 | 210.6 | 248.3 | 226.3 | 210.8 | 292.3 |
| 100 | 289.9 | 8016.1 | 3817.9 | 5438.1 | 6332.1 | 6494.4 | 1146 | 13390 |
| 101 | 271.7 | 593.1 | 444.8 | 210.9 | 231.5 | 571.7 | 327.5 | 268.8 |
| 102 | 244.7 | 270.9 | 155.8 | 164.2 | 185.3 | 197.2 | 148.5 | 149 |

| | | | | | | | | |
|---|---|---|---|---|---|---|---|---|
| 103 | 359.8 | 424.3 | 368.8 | 345.3 | 325.6 | 402.3 | 249.8 | 390.8 |
| 104 | 582.6 | 395.2 | 757 | 373.7 | 448.9 | 715.7 | 422.8 | 1046.9 |
| 105 | 605.4 | 439.1 | 711.2 | 498.5 | 563 | 713.5 | 478.7 | 844.5 |
| 106 | 573.3 | 421.3 | 707.9 | 394.3 | 496.4 | 727.1 | 411.8 | 1158.6 |
| 107 | 342.9 | 313.4 | 232.5 | 633.3 | 349.9 | 97.4 | 256.3 | 159.1 |
| 108 | 374.4 | 509.1 | 404.5 | 447.5 | 424 | 395.3 | 507 | 313.7 |
| 109 | 70.1 | 48.4 | 100.4 | 1071.3 | 216 | 52.3 | 605.8 | 112.2 |
| 110 | 1.3 | 1.1 | 56.4 | 2298.1 | 314.9 | 11.2 | 1496.5 | 175 |
| 111 | 618.4 | 1832.7 | 1179.5 | 1928 | 929.6 | 2094.9 | 301.9 | 2011.4 |
| 112 | 925.8 | 306.9 | 118.6 | 634.8 | 404.2 | 186.5 | 159.1 | 438 |
| 113 | 222.3 | 166.6 | 157.8 | 240.9 | 191.7 | 283.5 | 59.8 | 260.2 |
| 114 | 192.2 | 388.8 | 475 | 452.6 | 701.1 | 833.9 | 536.1 | 428.3 |
| 115 | 744.1 | 403.4 | 380.9 | 477.3 | 722 | 354.5 | 316 | 403.1 |
| 116 | 74 | 256.2 | 288.5 | 251.7 | 354.9 | 244.7 | 200.7 | 290.2 |
| 117 | 124.2 | 3117.3 | 3370.9 | 1392.1 | 2479.3 | 3139.4 | 3082.9 | 1506.6 |
| 118 | 45.9 | 127.9 | 220.2 | 543.8 | 654.1 | 106.6 | 76 | 302.5 |
| 119 | 73.6 | 109.6 | 79.5 | 187.2 | 253.7 | 129 | 89.7 | 180.1 |
| 120 | 63 | 151.9 | 118.9 | 83.3 | 329.6 | 139.5 | 96.5 | 196 |
| 121 | 49.1 | 198.1 | 1209.5 | 1344.6 | 731.2 | 553.5 | 930.8 | 1026.3 |
| 122 | 325.5 | 74.7 | 168.8 | 279.7 | 220 | 128.3 | 73.4 | 226.7 |
| 123 | 191.5 | 158.6 | 161.6 | 149.7 | 200.5 | 180.6 | 123.1 | 147.8 |
| 124 | 208.1 | 325.2 | 147.4 | 136 | 144.3 | 215.6 | 157.8 | 152.9 |
| 125 | 1661.5 | 1623.3 | 1124.6 | 933.3 | 856.1 | 1323.7 | 974.7 | 1239.4 |
| 126 | 336.6 | 998.6 | 1518 | 1111.4 | 1195.1 | 749.3 | 1734.3 | 1307.5 |
| 127 | 114.1 | 160.9 | 127 | 108.1 | 99.9 | 108.8 | 94.3 | 121.3 |
| 128 | 377.5 | 502 | 304.4 | 273.4 | 240.5 | 360.7 | 256.7 | 248.3 |
| 129 | 152 | 156.1 | 228.4 | 614.3 | 248.8 | 187.1 | 182.1 | 174.4 |
| 130 | 201.8 | 125.2 | 123.1 | 91.1 | 86.5 | 146.4 | 126 | 102.5 |
| 131 | 171.6 | 96.4 | 92.7 | 100.4 | 74.1 | 81.2 | 64.7 | 62 |
| 132 | 194.7 | 290.5 | 224 | 204.2 | 202.2 | 246.9 | 198.3 | 213.9 |
| 133 | 185.3 | 28.5 | 90.5 | 95.6 | 99.8 | 53.6 | 115.1 | 67.8 |
| 134 | 108.7 | 105.8 | 146.2 | 129.8 | 136.1 | 152.6 | 101.4 | 104.9 |
| 135 | 329.3 | 300.4 | 422.9 | 350.2 | 459.4 | 315.2 | 405.3 | 583.7 |
| 138 | 141.3 | 138.1 | 148 | 138 | 261.5 | 129.3 | 112.3 | 140.1 |
| 139 | 64.7 | 72.7 | 91.7 | 113 | 89.4 | 72.3 | 78.5 | 70.3 |
| 140 | 12.8 | 3594.2 | 546.1 | 1731 | 1815.6 | 2735.6 | 7476.3 | 1203.1 |
| 141 | 765 | 2165 | 2264.2 | 1694.9 | 2113.3 | 2212.9 | 1964.9 | 2552.8 |
| 144 | 557.3 | 658.9 | 531.9 | 445.9 | 506.1 | 576 | 448.1 | 498.5 |
| 145 | 293.6 | 414.8 | 253.6 | 213.1 | 181.7 | 238.7 | 218 | 282.1 |
| 146 | 346.7 | 292.7 | 269.6 | 217.1 | 213.1 | 208.1 | 211.4 | 258.4 |
| 147 | 62.7 | 79.5 | 46.1 | 53.8 | 81.9 | 58.5 | 49.8 | 63.7 |
| 148 | 269.9 | 289.4 | 334.4 | 865.9 | 508.3 | 260.4 | 407.4 | 281.3 |
| 149 | 97.4 | 236.4 | 437.2 | 418.5 | 394.6 | 307.1 | 973.7 | 534.2 |
| 150 | 137.6 | 243.5 | 188.6 | 496 | 186.8 | 175.5 | 160 | 154.5 |
| 151 | 127.6 | 135.7 | 166.9 | 264 | 342.2 | 154.3 | 155.1 | 238.1 |
| 153 | 123.6 | 100.3 | 115.7 | 116.3 | 102.4 | 89.5 | 160.7 | 101.3 |
| 154 | 85 | 197.4 | 1113.2 | 453.2 | 438.4 | 272.8 | 68.6 | 476.9 |
| 155 | 278.9 | 193.9 | 179.6 | 197.2 | 142.9 | 121 | 155.5 | 125.7 |
| 156 | 223.2 | 67.3 | 125 | 73.3 | 134.8 | 87.9 | 62.7 | 94.5 |
| 157 | 191.4 | 141 | 80.7 | 108 | 126.5 | 132.4 | 139.9 | 153.4 |
| 158 | 120.4 | 27.9 | 18.7 | 22.2 | 13.5 | 8.3 | 48.3 | 1.1 |
| 159 | 2173.3 | 3351 | 4458.5 | 2838.9 | 3155.5 | 4574.9 | 3436 | 5276.1 |
| 161 | 321.7 | 4.9 | 48.5 | 38.3 | 51.1 | 73.2 | 32.3 | 115.5 |
| 162 | 57.7 | 109.5 | 90.4 | 127 | 451.2 | 53.5 | 97.4 | 109.9 |
| 164 | 18.3 | 107.3 | 133.5 | 945.5 | 294.7 | 100.8 | 471.3 | 145 |
| 166 | 1789.6 | 8438 | 8113.2 | 9464 | 4481.5 | 10514.2 | 6218.7 | 8586.9 |
| 167 | 1221.4 | 1687.4 | 2694.8 | 870.2 | 2235.2 | 3402.4 | 2967 | 3999.6 |
| 168 | 1134.1 | 1571.9 | 233.8 | 389.6 | 470.2 | 1162.3 | 676.1 | 517.9 |

| | | | | | | | | |
|---|---|---|---|---|---|---|---|---|
| 169 | 1299.1 | 3874.8 | 2937.3 | 20149.3 | 4579.7 | 3975.5 | 5507.1 | 3449.1 |
| 170 | 496.6 | 1040.8 | 993.1 | 1421.7 | 1194.6 | 1747 | 1725.6 | 975.7 |
| 171 | 1.7 | 726.5 | 381.8 | 1172.2 | 3538.7 | 1.7 | 311.2 | 923.1 |
| 172 | 787.9 | 831.5 | 671.7 | 556.8 | 576.2 | 725.4 | 702.2 | 758.7 |
| 173 | 325.2 | 174.5 | 231.2 | 453.8 | 404 | 104.2 | 388.1 | 164.7 |
| 176 | 1.1 | 61.7 | 1.1 | 901.4 | 45.4 | 116.1 | 25.7 | 13.1 |
| 177 | 1168.1 | 892.5 | 744.8 | 380 | 424.7 | 794 | 561 | 663.6 |
| 178 | 1040.7 | 147.4 | 125.3 | 225.6 | 118 | 65.1 | 137.1 | 105.3 |
| 179 | 175.2 | 142.5 | 80.7 | 229.8 | 86.5 | 162.9 | 95.4 | 82.1 |
| 180 | 219.9 | 159.1 | 69.7 | 168.4 | 130.9 | 108.4 | 102 | 102.9 |
| 181 | 7935.3 | 13498.8 | 13730.6 | 11985.5 | 10419.5 | 12804.4 | 11323.8 | 12979.3 |
| 182 | 150.3 | 447.5 | 499.1 | 465.6 | 614.8 | 394.1 | 697.3 | 535.6 |
| 183 | 1.2 | 302 | 315.6 | 55.7 | 3038.8 | 1.1 | 111.6 | 870.4 |
| 184 | 164.6 | 304.5 | 220.8 | 214.1 | 342.7 | 208.6 | 235.9 | 282.7 |
| 185 | 339.5 | 1227.3 | 1316 | 2812.4 | 2324.1 | 1291.1 | 801.3 | 951.8 |
| 186 | 1007.5 | 586.1 | 520.9 | 357.3 | 592.2 | 596 | 427.6 | 531 |
| 187 | 105.8 | 150.7 | 121.4 | 138.6 | 346.7 | 114.5 | 71.7 | 179.7 |
| 188 | 90.9 | 117.6 | 131.9 | 111.7 | 401.8 | 185.4 | 95.3 | 186.5 |
| 189 | 44.7 | 89.1 | 89.1 | 75.5 | 222.5 | 76.8 | 62.8 | 140 |
| 190 | 387.4 | 103.3 | 160.9 | 193.8 | 110.4 | 132.3 | 17.1 | 290.6 |
| 191 | 1052.5 | 301.2 | 1095.5 | 2271.6 | 1145.2 | 677.5 | 637 | 486.6 |
| 192 | 630.6 | 99.9 | 111.6 | 214 | 209.1 | 70.9 | 85.5 | 102.3 |
| 193 | 74.3 | 2980.5 | 1451.8 | 1464.1 | 967.9 | 3258.6 | 1246.7 | 1115.3 |
| 194 | 103.9 | 95.6 | 148.6 | 197.7 | 173.4 | 182.1 | 182.9 | 151.6 |
| 195 | 232 | 517.9 | 233.4 | 227.6 | 231.8 | 371.1 | 311.1 | 252.3 |
| 196 | 14368.7 | 9559.4 | 6515.4 | 4725.2 | 6534.5 | 6168.5 | 3594.5 | 10426.2 |
| 197 | 174.7 | 101.2 | 74.1 | 54.7 | 170.5 | 108.4 | 82.8 | 122.4 |
| 198 | 91 | 135.6 | 56.6 | 60.3 | 89.4 | 59.6 | 45.6 | 59.4 |
| 199 | 61 | 139.7 | 76.3 | 1103.8 | 315.1 | 93.4 | 1137.5 | 145 |
| 200 | 174.8 | 227.8 | 166 | 143.6 | 811.9 | 124 | 150.7 | 494.8 |
| 201 | 97.8 | 40.6 | 125.1 | 6018.8 | 363.4 | 16.6 | 745.3 | 54 |
| 203 | 248.8 | 51.5 | 202.6 | 274 | 233 | 114.9 | 678.6 | 137.4 |
| 204 | 355.2 | 445.7 | 563 | 594.4 | 551.8 | 780.5 | 259.1 | 741.3 |
| 205 | 3081.7 | 3669.5 | 4544.7 | 4530.2 | 5821 | 4180.6 | 2864.1 | 6443.9 |
| 206 | 635.1 | 335.2 | 212.1 | 203.2 | 2002.9 | 327.3 | 212.8 | 296.9 |
| 207 | 108.8 | 465.2 | 1154 | 1017.8 | 856.5 | 253.4 | 2406.9 | 982.7 |
| 208 | 376.1 | 87.8 | 163.3 | 235.1 | 433.7 | 162 | 209.8 | 341.4 |
| 209 | 4098 | 6563.6 | 5001.8 | 2151.6 | 3441.1 | 5849.7 | 5350.8 | 5424.3 |
| 211 | 13293.2 | 56352.5 | 27543 | 16362.2 | 12402.5 | 30128.4 | 25478.3 | 26582.6 |
| 212 | 3366.9 | 504.5 | 937.3 | 451.1 | 493.9 | 512.1 | 182 | 356.5 |
| 213 | 557 | 72.1 | 102.2 | 72.7 | 87.2 | 125.6 | 68.6 | 84.7 |
| 215 | 69.6 | 611.1 | 1358.5 | 1255.1 | 1026.1 | 288 | 5253.8 | 1589.5 |
| 216 | 307.4 | 418 | 1035.4 | 885.1 | 913.2 | 784.3 | 771.5 | 663.5 |
| 217 | 122.9 | 221.4 | 276.8 | 282.9 | 382.1 | 276 | 250.6 | 310.7 |
| 218 | 405.2 | 348.1 | 325 | 389.5 | 308.3 | 374.2 | 366.4 | 279.9 |
| 220 | 133.1 | 120.1 | 104.4 | 90.8 | 74 | 87.2 | 60.6 | 104.9 |
| 221 | 202.1 | 462 | 707.8 | 738.8 | 744.5 | 462.6 | 507.6 | 536.4 |
| 223 | 401.3 | 290.1 | 265 | 373.6 | 219.2 | 205.5 | 250.1 | 279.2 |
| 227 | 96.8 | 1403.6 | 588.9 | 742.9 | 1379 | 767.3 | 484.7 | 435.2 |
| 228 | 744.1 | 1944.7 | 680.2 | 365.7 | 418 | 1097.5 | 375 | 675.9 |
| 229 | 192.8 | 268 | 148.6 | 179.1 | 300.2 | 245.5 | 147 | 234.8 |
| 230 | 587.1 | 143.8 | 108 | 519.8 | 327.3 | 137.2 | 90.6 | 168.9 |
| 231 | 159.4 | 112.3 | 101.9 | 123.3 | 147.3 | 95.5 | 310.1 | 105.2 |
| 232 | 86 | 1502.8 | 1501.4 | 235.4 | 15668.2 | 38.8 | 605.2 | 4886.3 |
| 233 | 813.8 | 1781.7 | 6349.7 | 5834.7 | 8518.5 | 4215.2 | 3924.8 | 4770.7 |
| 235 | 19.1 | 393.8 | 248.5 | 975.6 | 1843.2 | 72.6 | 302.8 | 584.4 |
| 236 | 135.6 | 172.6 | 172 | 178.6 | 303.1 | 218.4 | 107.7 | 240.4 |
| 237 | 1106.1 | 1789 | 2038.4 | 1890.3 | 2100.8 | 1798.9 | 1370.4 | 1717.2 |

| | | | | | | | | |
|---|---|---|---|---|---|---|---|---|
| 238 | 366.2 | 375.6 | 1127.2 | 1261.7 | 1343.5 | 899.5 | 859 | 1414.4 |
| 239 | 61.2 | 182.2 | 230.2 | 474.9 | 731.7 | 98.3 | 176.9 | 251.2 |
| 240 | 91.7 | 193.4 | 362.9 | 596.3 | 923.8 | 202.3 | 144.4 | 289.6 |
| 241 | 26.6 | 324.2 | 559.9 | 1218.5 | 1761.7 | 170.8 | 321.8 | 584.8 |
| 242 | 4172.9 | 1891.1 | 1654.2 | 11791 | 2788.1 | 2452.4 | 1809.2 | 1758.5 |
| 243 | 248.1 | 152.6 | 323.5 | 711.2 | 510.7 | 217.7 | 135.2 | 244.7 |
| 244 | 68.8 | 98.9 | 183.4 | 204.9 | 179.5 | 139.4 | 209.7 | 132.7 |
| 246 | 385.3 | 80.7 | 187.9 | 227.3 | 218.7 | 122.1 | 230.3 | 250.4 |
| 247 | 115.2 | 187.8 | 109.8 | 134.8 | 162.4 | 139.5 | 154.3 | 176.4 |
| 248 | 175.8 | 124.7 | 211.6 | 157.6 | 122.1 | 213.7 | 119.3 | 151 |
| 249 | 78.8 | 1316.1 | 1303.2 | 1406.5 | 646.5 | 1094 | 89.7 | 316.7 |
| 251 | 41.9 | 62.5 | 102.3 | 171.4 | 129 | 87.1 | 73 | 44.9 |
| 253 | 297.4 | 222.6 | 147.6 | 339.3 | 377.1 | 276.6 | 218.4 | 296.8 |
| 254 | 170 | 132 | 351.7 | 258.6 | 501.3 | 45.2 | 529.9 | 460.2 |
| 255 | 1140 | 47 | 85.6 | 207.2 | 108 | 89.8 | 121.1 | 51.6 |
| 256 | 509.5 | 559.2 | 396.4 | 6461.4 | 596.9 | 143.8 | 1128.1 | 72.8 |
| 257 | 111.3 | 113 | 208.1 | 145.8 | 103.3 | 155.1 | 204.9 | 122 |
| 258 | 373.9 | 20.9 | 71.2 | 831.4 | 206.8 | 7.3 | 19.2 | 134.7 |
| 259 | 1.2 | 346.7 | 153.9 | 329.1 | 1431.2 | 189.1 | 51.2 | 376.3 |
| 260 | 269.3 | 188.5 | 157.8 | 133.8 | 179.4 | 168.2 | 152.1 | 211.1 |
| 261 | 363.1 | 886.3 | 408.2 | 266.3 | 337.5 | 580.5 | 238.9 | 222.6 |
| 263 | 350.7 | 311.2 | 391.8 | 381.3 | 340.2 | 408 | 301.8 | 534.9 |
| 263 | 560.3 | 220 | 122.6 | 491.3 | 334.4 | 187.2 | 129.9 | 227 |
| 265 | 305.3 | 771.9 | 487.2 | 449.1 | 359.5 | 453.8 | 572.3 | 448.6 |
| 266 | 587.1 | 510.1 | 304.8 | 271.5 | 354.2 | 346.8 | 203.3 | 606.6 |
| 256 | 110.1 | 118.2 | 141.4 | 183.8 | 193.4 | 156.1 | 104.6 | 141.6 |
| 268 | 829.5 | 440.5 | 993.8 | 821.4 | 1312.7 | 938.4 | 407.2 | 1476.7 |
| 269 | 58.4 | 43.6 | 32.7 | 52.3 | 48.9 | 47.7 | 35.4 | 31.4 |
| 270 | 414.3 | 315 | 208 | 162.5 | 188.1 | 275.9 | 132.5 | 378.4 |
| 271 | 184.7 | 216.5 | 242.4 | 350 | 268.7 | 323.9 | 246.1 | 211.3 |
| 272 | 673.6 | 752.9 | 549.8 | 450 | 534.1 | 526.4 | 507.2 | 463.9 |
| 273 | 667.9 | 4430.8 | 2398.6 | 2278.9 | 2835.2 | 5763.4 | 3170.9 | 10524 |
| 274 | 309.4 | 563.1 | 382 | 416.7 | 1158.9 | 634.4 | 198.9 | 501.8 |
| 275 | 150.9 | 343.1 | 349.7 | 862.7 | 617.9 | 370.3 | 217.2 | 282.6 |
| 276 | 144.2 | 2577.7 | 914.9 | 1262.7 | 2706.1 | 873.2 | 822.4 | 731.1 |
| 277 | 221 | 264.6 | 204.6 | 186.4 | 182 | 199.3 | 212.4 | 205.9 |
| 278 | 5.1 | 445.5 | 286.4 | 951.3 | 2040.1 | 322.7 | 97.9 | 556.9 |
| 279 | 1964.2 | 1929.5 | 1874.1 | 1425.7 | 2873.1 | 2411.1 | 2753 | 4215.2 |
| 280 | 1725.8 | 1807.2 | 1790 | 2026.6 | 3105.8 | 3426.9 | 3401.6 | 4521.1 |
| 281 | 299.6 | 10.6 | 61.7 | 159.3 | 165.8 | 22.7 | 10.9 | 56.8 |
| 282 | 253.1 | 287.7 | 240.7 | 553.9 | 252 | 245.3 | 295.4 | 310.6 |
| 283 | 451.8 | 310.2 | 223.6 | 224.6 | 261.3 | 262.4 | 300.3 | 265.8 |
| 284 | 107.2 | 65 | 79.3 | 94.4 | 77.1 | 95.2 | 88.3 | 68.2 |
| 285 | 126.9 | 108.2 | 89.2 | 90.8 | 95.7 | 83.3 | 101.1 | 56.8 |
| 286 | 207.5 | 742.2 | 937.4 | 1260.1 | 840.3 | 441.7 | 1221.8 | 518 |
| 287 | 147.3 | 167.1 | 130.2 | 109.1 | 90 | 112.1 | 102.3 | 113 |
| 288 | 171.6 | 203.3 | 136.7 | 135.2 | 166.9 | 154.6 | 140.7 | 158 |
| 289 | 371.3 | 670.5 | 83.5 | 1902.3 | 2036.5 | 48 | 228 | 264.6 |
| 291 | 80.2 | 126.4 | 72.5 | 78.8 | 132.2 | 210.1 | 63.6 | 112.7 |
| 292 | 38.1 | 110.7 | 46.9 | 472.6 | 1250.2 | 27.4 | 126.2 | 66.3 |
| 293 | 222.5 | 84 | 110.2 | 187.1 | 124 | 59.9 | 126.5 | 88.8 |
| 295 | 1213.6 | 281.8 | 785.8 | 379.3 | 174.4 | 535.7 | 147 | 292.6 |
| 296 | 898.3 | 582 | 827.5 | 972.1 | 1280.1 | 738.6 | 1194.8 | 908.9 |
| 297 | 58.4 | 68.5 | 79.3 | 128 | 150.2 | 55.4 | 43.3 | 58.5 |
| 298 | 389.2 | 195.7 | 352.2 | 318 | 292.9 | 275.4 | 295.4 | 345.4 |
| 299 | 1008.9 | 952.4 | 435.5 | 935.9 | 1207.2 | 1276.8 | 1004.8 | 874.4 |
| 300 | 616.3 | 416.1 | 93.2 | 235.8 | 253 | 285.2 | 321.8 | 218.3 |
| 301 | 787.4 | 7692.9 | 760.9 | 597.6 | 582 | 4161.5 | 866.6 | 1551.4 |

| | | | | | | | | |
|---|---|---|---|---|---|---|---|---|
| 302 | 8.2 | 20.2 | 20.5 | 26.4 | 38.3 | 11.5 | 48.2 | 19.3 |
| 303 | 667.1 | 584.1 | 822.2 | 624 | 674.3 | 794.9 | 891.8 | 698 |
| 304 | 184.1 | 237.5 | 283.8 | 497 | 280.3 | 200.5 | 163.9 | 170.7 |
| 305 | 158.6 | 187.4 | 179.4 | 240.3 | 128.9 | 137.5 | 171.4 | 144 |
| 306 | 177.6 | 272.7 | 152.9 | 164.2 | 148.6 | 151.8 | 118.4 | 196.2 |
| 307 | 232.6 | 294 | 179.7 | 177.7 | 166.7 | 143.1 | 269.6 | 156.1 |
| 308 | 3916.9 | 1024.8 | 1901.6 | 978.1 | 2018.2 | 1367 | 1084.7 | 2030.4 |
| 310 | 840.8 | 1027 | 434.4 | 345.5 | 351.7 | 775.7 | 403.9 | 604 |
| 311 | 1.4 | 100.4 | 111.4 | 207.4 | 110.6 | 112.7 | 85.5 | 48.2 |
| 312 | 3441.6 | 2815.6 | 597.5 | 664 | 496.5 | 1479.8 | 176.1 | 1442.5 |
| 313 | 100.5 | 123.2 | 112.4 | 128.1 | 112.7 | 146.2 | 83.4 | 132.5 |
| 314 | 271.3 | 291.9 | 302.3 | 543.9 | 514 | 181.8 | 157.4 | 408.4 |
| 315 | 194.3 | 265 | 553.7 | 442.1 | 676.9 | 352.4 | 338.1 | 580.4 |
| 316 | 190.3 | 130.5 | 393.5 | 448.9 | 461.5 | 184.7 | 184 | 326.6 |
| 317 | 187.8 | 207.7 | 143.5 | 1020.4 | 167.2 | 168.1 | 180.4 | 189.7 |
| 318 | 41.3 | 18.4 | 63.6 | 115.2 | 161.2 | 34.4 | 15 | 117.9 |
| 319 | 860 | 151.7 | 173.4 | 206.1 | 185.7 | 184.2 | 193 | 172.4 |
| 320 | 509.6 | 854.4 | 499.3 | 516.6 | 643.1 | 697.6 | 512 | 842.6 |
| 321 | 856.4 | 633.2 | 596.3 | 403.9 | 477.8 | 545.6 | 282.5 | 377.9 |
| 322 | 433.2 | 202.4 | 278.6 | 249.3 | 234 | 227.3 | 234.3 | 199.3 |
| 323 | 841.5 | 2787.9 | 2937.2 | 1793.4 | 1324.1 | 4240.9 | 2486 | 2525.8 |
| 324 | 34.6 | 20.8 | 38.8 | 166.5 | 195.2 | 110.1 | 85.5 | 75.9 |
| 325 | 493.2 | 226.8 | 266.8 | 301.9 | 256.4 | 292.2 | 312.9 | 251.6 |
| 326 | 107.7 | 1500 | 4118.2 | 5094.1 | 4992.3 | 1863.8 | 3521.7 | 2850 |
| 327 | 8.4 | 1.2 | 6.1 | 58.1 | 53.1 | 10.1 | 1.1 | 19.4 |
| 328 | 448.4 | 1313.7 | 690.6 | 383.9 | 558.3 | 678.1 | 451.7 | 643.7 |
| 329 | 242.5 | 67.9 | 67.9 | 52 | 61.7 | 53.9 | 71.2 | 50.2 |
| 330 | 17.8 | 47.2 | 39.7 | 282.1 | 70.4 | 42.8 | 119.6 | 47.7 |
| 331 | 733.3 | 495.8 | 278.9 | 232 | 273.6 | 280.6 | 257 | 242.5 |
| 333 | 302.3 | 1043.4 | 1067 | 536.5 | 739.2 | 789 | 589.6 | 857.9 |
| 334 | 135 | 171 | 185.4 | 142.8 | 175.4 | 125.4 | 150.7 | 191.2 |
| 335 | 114.3 | 112 | 135.6 | 198.2 | 140.2 | 65.3 | 82.4 | 146.1 |
| 336 | 273.5 | 427.6 | 549 | 929.9 | 527.7 | 486.6 | 685.7 | 484.4 |
| 337 | 127.6 | 450.5 | 304.7 | 308.8 | 228.9 | 259.2 | 192 | 337.1 |
| 338 | 122 | 131.1 | 153.1 | 314.9 | 206.1 | 109.7 | 203.8 | 162.9 |
| 339 | 589.7 | 576.7 | 629.8 | 367.4 | 474.8 | 496.3 | 533.8 | 528.2 |
| 340 | 345.7 | 362 | 289.7 | 277.4 | 243.4 | 291.8 | 246.1 | 272.2 |
| 341 | 601 | 1030.1 | 896.3 | 495.4 | 536.5 | 1091.4 | 1222.4 | 1064.8 |
| 343 | 4100.7 | 199.4 | 431 | 1240.1 | 83 | 30.4 | 75.2 | 123.1 |
| 344 | 1624.5 | 2249.2 | 1044.5 | 1143.8 | 1222.6 | 1571.5 | 1112.9 | 1923.9 |
| 345 | 238.1 | 412.6 | 253.9 | 217.5 | 231.2 | 189.6 | 247.2 | 211.3 |
| 347 | 2041.8 | 1350.1 | 3062.9 | 2896.2 | 3974.1 | 2601.7 | 1109.2 | 3899.9 |
| 348 | 2985.8 | 3296.8 | 7137.1 | 7441.2 | 9015.9 | 4507.6 | 2225.5 | 6141.5 |
| 349 | 242.2 | 171.3 | 270.3 | 274.6 | 214.3 | 206.7 | 232 | 284.3 |
| 350 | 145.3 | 165.3 | 253.4 | 465.2 | 473.9 | 247 | 260.1 | 349.3 |
| 351 | 221.5 | 71.2 | 134 | 484.9 | 440.9 | 92.2 | 237.3 | 182.5 |
| 352 | 302.8 | 377.2 | 355.1 | 306.4 | 343.9 | 332.8 | 471.2 | 707.4 |
| 353 | 485.1 | 155.6 | 246.1 | 298.4 | 282.3 | 160.5 | 283 | 245.4 |

3i

| SEQ ID NO: | 923TT | 924TT | 925TT | 926TT | 927TT | 928TT | 932TT | 933TT |
|---|---|---|---|---|---|---|---|---|
| 354 | 4999.2 | 948.9 | 8.5 | 8107.3 | 357.6 | 741.1 | 2903.8 | 582.1 |
| 355 | 1542.9 | 255.1 | 105.5 | 4694.4 | 98.3 | 91.6 | 1287.2 | 466 |
| 356 | 955.2 | 717.8 | 1055 | 1279.5 | 479.1 | 524.8 | 624.3 | 654.8 |
| 357 | 742.1 | 866.6 | 793.6 | 981.1 | 533.1 | 757.7 | 765.3 | 734.4 |

| | | | | | | | | |
|---|---|---|---|---|---|---|---|---|
| 358 | 619.2 | 1731.9 | 778.3 | 770.2 | 675 | 559.4 | 2309.3 | 1072.7 |
| 359 | 393.4 | 300.3 | 283.4 | 311.1 | 437.6 | 326 | 298.3 | 314.7 |
| 362 | 382.2 | 940.4 | 368.8 | 968.1 | 747.1 | 473.3 | 3226.3 | 666.7 |
| 360 | 305.2 | 656.7 | 364.5 | 362.7 | 233.2 | 217.2 | 638.3 | 448 |
| 361 | 455.4 | 543 | 660.9 | 437.4 | 376.4 | 1699.9 | 116.7 | 462.7 |
| 363 | 425.6 | 414.8 | 478.3 | 461 | 574.2 | 432.5 | 334.7 | 437.1 |
| 1 | 730.2 | 1032.9 | 682 | 844.1 | 393.9 | 674.5 | 1344.9 | 1001.2 |
| 2 | 3449 | 1961.4 | 3686.9 | 2642.7 | 1807.9 | 3449 | 6464 | 2719.4 |
| 7 | 354 | 463.2 | 545 | 364.5 | 928.7 | 902.8 | 291.2 | 1104.3 |
| 8 | 2391.2 | 881.8 | 994.1 | 2512.8 | 1239.1 | 1078.9 | 1105.4 | 1030.3 |
| 9 | 7066.7 | 3621.1 | 8955.9 | 10216 | 6146 | 1554.6 | 3073.1 | 2224.4 |
| 10 | 76.1 | 133.7 | 48.5 | 112.3 | 70.9 | 40.5 | 125.8 | 84.2 |
| 11 | 423.1 | 502.3 | 256.7 | 620.1 | 215.5 | 224.5 | 1442.7 | 450.7 |
| 13 | 208.3 | 631.4 | 132.2 | 270.3 | 127.5 | 202.1 | 244.8 | 524.1 |
| 14 | 1772 | 3284.9 | 595.1 | 1382.8 | 378.2 | 703.9 | 1281.9 | 1396.6 |
| 15 | 273.3 | 253.1 | 691.6 | 629.7 | 624.5 | 137.9 | 1375.3 | 988.3 |
| 16 | 1155.7 | 1826.6 | 1298.2 | 1503.2 | 766.7 | 976.8 | 2803.3 | 1587.5 |
| 17 | 981 | 1525 | 1084 | 1110.8 | 713.8 | 1879.1 | 2006.1 | 958.6 |
| 19 | 295.3 | 325.4 | 2332.9 | 154.5 | 766.2 | 143 | 5450.6 | 1516 |
| 20 | 5.6 | 231.8 | 1.1 | 406.2 | 93 | 7.7 | 717.2 | 285 |
| 21 | 216.9 | 1426.9 | 466.2 | 1058.3 | 233.1 | 89.3 | 2805.3 | 820.1 |
| 22 | 5541.1 | 5019.9 | 7633.3 | 7736.7 | 4422.4 | 4997.7 | 6683.9 | 5281.2 |
| 23 | 418.4 | 128.7 | 924.7 | 522.4 | 263.1 | 95.8 | 1540.6 | 1482.2 |
| 24 | 124.7 | 262.2 | 466.7 | 332.9 | 277.9 | 112.7 | 502.5 | 370 |
| 26 | 2003.6 | 326.4 | 9052 | 447.2 | 1915.3 | 199.6 | 1793.9 | 5637.4 |
| 27 | 903.7 | 1325.6 | 731.2 | 620.3 | 523.4 | 499.5 | 1436.8 | 1142.6 |
| 29 | 77.9 | 153.4 | 131.3 | 265.8 | 339.2 | 91 | 278.2 | 113.3 |
| 30 | 244.5 | 111.3 | 342.6 | 1082.1 | 219.4 | 136 | 880.6 | 493.2 |
| 31 | 717.2 | 5693.4 | 185.8 | 2809.1 | 627.1 | 214.1 | 13720.9 | 4115 |
| 33 | 942.9 | 1061.8 | 704.6 | 3949.8 | 244.3 | 466.4 | 5262.7 | 2453.2 |
| 34 | 3136.1 | 702.3 | 4502.9 | 3384.3 | 2498.9 | 1817.4 | 204.4 | 1637.3 |
| 35 | 823.2 | 709.6 | 179 | 1649.8 | 528.8 | 173.2 | 2586.2 | 722.3 |
| 36 | 196 | 1335.4 | 173.1 | 183.3 | 431.8 | 189.1 | 339.1 | 986.2 |
| 37 | 190.3 | 178.3 | 281.4 | 264.8 | 256.3 | 166 | 142.8 | 210.3 |
| 38 | 764.6 | 1248.7 | 543.5 | 2191.9 | 232.8 | 763.6 | 2898.9 | 1375.1 |
| 40 | 454.8 | 644.3 | 481.5 | 529.5 | 897.3 | 467 | 541 | 618.6 |
| 41 | 324.4 | 539.9 | 467.9 | 199.6 | 143.9 | 2803.9 | 331.2 | 668.8 |
| 42 | 245.1 | 482.5 | 178 | 170.4 | 246.6 | 572.5 | 528.4 | 345.2 |
| 43 | 312.4 | 237 | 363 | 359 | 522.3 | 337.5 | 229 | 305.9 |
| 44 | 485.5 | 1587.5 | 666.9 | 778.7 | 788.5 | 427.7 | 1308.4 | 1173.2 |
| 45 | 523.4 | 1739.2 | 564.2 | 954.7 | 723.9 | 426.5 | 1423.9 | 1045.9 |
| 46 | 219.6 | 289.5 | 291.3 | 270.7 | 363.6 | 297.2 | 236.2 | 308.8 |
| 47 | 358.3 | 1203.3 | 261.6 | 420.8 | 219.8 | 231.9 | 811 | 565.7 |
| 48 | 132.2 | 32.9 | 529.4 | 17.2 | 481.9 | 15 | 768.8 | 1447.3 |
| 49 | 502.3 | 3238.8 | 380.6 | 5448 | 761.8 | 211.7 | 13066 | 3743 |
| 50 | 203.5 | 179.7 | 458.7 | 481.9 | 247 | 130.4 | 1359.3 | 491.3 |
| 51 | 36.1 | 495.5 | 36.6 | 477.9 | 354.1 | 190.1 | 860.5 | 367.9 |
| 52 | 217.6 | 305.8 | 203.1 | 178.1 | 145.5 | 199.2 | 266.4 | 254.3 |
| 53 | 135.2 | 1943.2 | 130.6 | 584.4 | 98 | 144.2 | 2628.1 | 714.3 |
| 54 | 980.6 | 1795 | 433.4 | 795.1 | 591.6 | 1643.8 | 1486.9 | 1394.9 |
| 55 | 106.3 | 85.4 | 98.8 | 76.5 | 97.2 | 90.8 | 86.4 | 81.5 |
| 56 | 72.3 | 168.2 | 54.5 | 77.8 | 70.1 | 175.9 | 120.4 | 338.8 |
| 57 | 4530.2 | 5218.3 | 281.2 | 4508.1 | 1636.4 | 1547.5 | 4191.4 | 4170.2 |
| 58 | 8556.8 | 6205.1 | 264.9 | 5634.2 | 1765.7 | 2033.7 | 8482.4 | 4433.9 |
| 59 | 1700.3 | 4628.3 | 2056.3 | 1693.4 | 800.8 | 1336.9 | 5450.1 | 2540.6 |
| 60 | 185.2 | 235.6 | 229.2 | 272.6 | 145.4 | 296.3 | 224.9 | 319 |
| 61 | 115.2 | 98.8 | 149 | 130.4 | 193.6 | 134.3 | 90.8 | 139.6 |
| 62 | 3145.7 | 4454.6 | 2756.3 | 2855 | 5821 | 142.2 | 5129.2 | 2442.2 |

| | | | | | | | | |
|---|---|---|---|---|---|---|---|---|
| 63 | 379.2 | 744.4 | 325.8 | 938.2 | 356.1 | 502 | 1395.4 | 539.9 |
| 64 | 238.6 | 3544.9 | 113.1 | 512.4 | 242.4 | 124.5 | 661.9 | 573.5 |
| 65 | 113.4 | 390.1 | 172.8 | 433.7 | 95.5 | 201.5 | 506.6 | 282.3 |
| 66 | 712.9 | 1199.2 | 529.5 | 816.9 | 392.7 | 1636.5 | 1229.2 | 1158.8 |
| 67 | 29201.1 | 24240.4 | 35951.3 | 30300.5 | 39570.8 | 34590.3 | 12924.3 | 33523.7 |
| 68 | 369.9 | 258.4 | 3692.3 | 955.1 | 3718.5 | 2125.3 | 13.8 | 2527.3 |
| 69 | 1786.8 | 1413.7 | 5838.9 | 2685.9 | 9047.2 | 4524.2 | 193.4 | 5503.7 |
| 70 | 1299.1 | 4941.4 | 1670.8 | 1399.3 | 4204.2 | 2761.2 | 1891.3 | 3217.7 |
| 71 | 319.8 | 3969.9 | 1361.7 | 304 | 214.9 | 196.2 | 7882.8 | 2439.7 |
| 72 | 858.1 | 2076.7 | 424 | 1838.1 | 291.2 | 256.6 | 4680.6 | 1518.6 |
| 73 | 330.7 | 633.1 | 342.2 | 295.9 | 422.6 | 202.3 | 561.1 | 512.8 |
| 75 | 160.9 | 1925.8 | 332.6 | 236.1 | 224.7 | 510.6 | 2680.7 | 1504.4 |
| 76 | 186.2 | 576.1 | 105.9 | 163.7 | 609.8 | 358.6 | 809.9 | 826.1 |
| 77 | 3237.7 | 1390.9 | 5841.2 | 3756.2 | 8578.9 | 3419.8 | 278.9 | 3072.7 |
| 78 | 390.6 | 630.4 | 1085.3 | 608.2 | 792.8 | 957.2 | 640.8 | 631.7 |
| 79 | 149 | 230.2 | 167.8 | 234.3 | 145.3 | 153.8 | 436.8 | 205.9 |
| 80 | 335 | 1124.8 | 139.3 | 306 | 338 | 751.9 | 1832.2 | 582.1 |
| 81 | 756.3 | 1099.9 | 314 | 2079.9 | 1185.6 | 169.3 | 5642.3 | 1783.2 |
| 82 | 204.4 | 219.8 | 346.6 | 218.1 | 417.4 | 382.9 | 97.4 | 242.2 |
| 84 | 161.4 | 305.1 | 329.2 | 121.2 | 177.5 | 155.6 | 567.2 | 276 |
| 85 | 268.5 | 148.9 | 131.5 | 9338.9 | 157.7 | 130.4 | 841 | 209.5 |
| 86 | 63.7 | 60.1 | 90.7 | 94.7 | 77.3 | 63.7 | 128.2 | 210.3 |
| 87 | 336.8 | 563.4 | 358.3 | 784 | 227.4 | 345.8 | 6260.3 | 351.1 |
| 88 | 2065.9 | 1415.1 | 5272.5 | 2763 | 4806.2 | 6097.4 | 444.1 | 3528.8 |
| 89 | 170 | 217.9 | 167.4 | 265.8 | 193.2 | 219.7 | 218.7 | 301.1 |
| 90 | 125.6 | 103.3 | 107.3 | 195.6 | 166.8 | 106.5 | 188.5 | 236.2 |
| 91 | 14152.6 | 4927.9 | 66.9 | 6763.6 | 1057.9 | 1430.5 | 7192.3 | 2761 |
| 92 | 4155.7 | 9975.4 | 3017.4 | 2430.3 | 1461.8 | 5499.5 | 10750.2 | 6764.7 |
| 93 | 253.7 | 200.3 | 376.1 | 458.2 | 173.2 | 390.5 | 123.5 | 213.3 |
| 94 | 2143.2 | 1723 | 1215.4 | 1528.1 | 389.5 | 566.5 | 1579.9 | 1152.9 |
| 95 | 493.2 | 509.2 | 582.1 | 343.1 | 199.5 | 575.4 | 147.5 | 827.4 |
| 96 | 154 | 286.2 | 193.1 | 193.6 | 157.8 | 242.7 | 283.8 | 221.5 |
| 97 | 289.4 | 1005.4 | 259.4 | 282.6 | 167.4 | 291 | 674.7 | 490.2 |
| 98 | 16.8 | 18.8 | 39.8 | 36.2 | 26.9 | 32.4 | 20.3 | 129.5 |
| 99 | 254.8 | 406.1 | 225.7 | 255.7 | 266 | 219.6 | 745 | 306.1 |
| 100 | 12961.3 | 633.2 | 23136.5 | 13157.8 | 4258.2 | 18457 | 92.1 | 9157.9 |
| 101 | 199.2 | 366.4 | 205.1 | 247.6 | 410.2 | 348.6 | 240.2 | 460.8 |
| 102 | 169.7 | 159.1 | 125.6 | 126.2 | 215.3 | 150.2 | 91.5 | 222 |
| 103 | 283.5 | 423.8 | 297.5 | 295.3 | 439.2 | 325.4 | 387.5 | 411.3 |
| 104 | 480.4 | 470.1 | 825.8 | 374.7 | 462.5 | 333.1 | 196.7 | 465.7 |
| 105 | 619.1 | 605.5 | 855.3 | 445.7 | 366.9 | 360.7 | 367.7 | 428.9 |
| 106 | 431 | 505.5 | 776.2 | 415.5 | 433 | 341.7 | 213.9 | 403.2 |
| 107 | 543.7 | 258.5 | 162.4 | 1100.5 | 444.6 | 341 | 846.1 | 327.4 |
| 108 | 369.2 | 171.8 | 356.8 | 273.4 | 382.3 | 416.2 | 184.3 | 269.6 |
| 109 | 2753.8 | 628.9 | 228.3 | 4738.3 | 100.4 | 1962.4 | 446.6 | 1981.7 |
| 110 | 4958.7 | 1350.1 | 414.3 | 7933.5 | 114.9 | 2744.5 | 1064.5 | 3917.5 |
| 111 | 1702 | 404.9 | 1150.9 | 1477.8 | 1041.7 | 814.8 | 162.5 | 988.1 |
| 112 | 1008.4 | 1893 | 534.7 | 114 | 706.3 | 402.2 | 1536.4 | 1239.5 |
| 113 | 151.5 | 782 | 135.7 | 174.6 | 265.6 | 218.3 | 1021.9 | 721.6 |
| 114 | 530.5 | 317.9 | 811.7 | 453.1 | 335.8 | 512.2 | 419.4 | 481.1 |
| 115 | 303.3 | 874.1 | 578.9 | 468.1 | 458.5 | 324.9 | 1401.9 | 823.6 |
| 116 | 268 | 457.4 | 242.4 | 317.8 | 206.9 | 239.4 | 1630.2 | 331.4 |
| 117 | 2483.3 | 467.5 | 3516.8 | 1446.4 | 1615.1 | 4189.5 | 228.4 | 1398 |
| 118 | 165.1 | 112 | 513.5 | 378.2 | 458.6 | 641.7 | 191.7 | 241.8 |
| 119 | 231.3 | 494.5 | 171.7 | 189.4 | 195.2 | 333.6 | 346.5 | 301.3 |
| 120 | 87.4 | 99.3 | 267.3 | 72.4 | 98.9 | 75.7 | 52 | 227.3 |
| 121 | 967.4 | 3533.9 | 789.3 | 1797 | 301.9 | 588.6 | 4329.5 | 1463.8 |
| 122 | 139.2 | 473.8 | 265.9 | 361.2 | 315 | 76.3 | 341.7 | 238.2 |

| | | | | | | | | |
|---|---|---|---|---|---|---|---|---|
| 123 | 119.5 | 215.8 | 183.1 | 128.8 | 119.8 | 265.2 | 182.7 | 263.7 |
| 124 | 109.4 | 166.6 | 139.1 | 148.2 | 604.8 | 130.2 | 108.7 | 169.3 |
| 125 | 722.7 | 763.1 | 1037.4 | 1010.3 | 1402.8 | 848.6 | 637.5 | 908.8 |
| 126 | 1296.7 | 159.8 | 1627.8 | 874.6 | 884.3 | 818.1 | 110.9 | 767.8 |
| 127 | 104.8 | 194.8 | 105 | 95.2 | 146.2 | 98.1 | 305.7 | 145.8 |
| 128 | 224.9 | 308 | 285.9 | 277.2 | 363.9 | 241.5 | 136.6 | 243.7 |
| 129 | 545.4 | 2482.1 | 213.2 | 2357.2 | 237.7 | 1139.4 | 5618.8 | 1929.5 |
| 130 | 76.7 | 408.1 | 75.2 | 90.5 | 165.7 | 222.2 | 490.8 | 419.5 |
| 131 | 85.8 | 1368.9 | 53.3 | 125.2 | 103.8 | 83.8 | 726.3 | 483.3 |
| 132 | 172.5 | 245.6 | 197.9 | 213 | 230.3 | 179.6 | 212.1 | 210.3 |
| 133 | 146.7 | 108.3 | 66.2 | 57.7 | 106.6 | 66.4 | 177.9 | 79.6 |
| 134 | 143.3 | 290.6 | 125.8 | 87.5 | 146 | 152.7 | 489.7 | 203.5 |
| 135 | 483 | 571.2 | 802.1 | 590.4 | 416.8 | 293.2 | 388.6 | 465.7 |
| 138 | 139.2 | 119.6 | 184.4 | 178 | 169.6 | 75.2 | 370.9 | 179.5 |
| 139 | 121.1 | 452.7 | 115.4 | 122.7 | 113.3 | 162.6 | 239 | 194 |
| 140 | 4146.1 | 1876 | 1951.6 | 1338.3 | 211.9 | 1.2 | 179.7 | 833.9 |
| 141 | 1804.4 | 1208.1 | 1688 | 1567 | 2363.3 | 1017 | 699.3 | 1291.8 |
| 144 | 380.1 | 9550.3 | 459.6 | 472.6 | 561 | 7295.8 | 9513.3 | 10388.7 |
| 145 | 194.4 | 2091 | 162.4 | 172 | 259.9 | 188.3 | 229 | 274.5 |
| 146 | 205.4 | 202 | 199.5 | 239.1 | 259 | 184.3 | 149.4 | 241.4 |
| 147 | 52 | 107.5 | 75.7 | 55.9 | 66.3 | 46.2 | 53.6 | 102.4 |
| 148 | 718.7 | 437 | 450.1 | 1743.3 | 388 | 408.3 | 1716 | 259.8 |
| 149 | 513.6 | 418 | 556.5 | 723.8 | 170.1 | 284.3 | 345.2 | 370.6 |
| 150 | 340.8 | 469.1 | 189.2 | 647.8 | 337.7 | 133.8 | 357.6 | 199.7 |
| 151 | 378.3 | 501.3 | 350.8 | 284.8 | 186.5 | 295.1 | 1725 | 566.4 |
| 153 | 135.8 | 159.4 | 124.9 | 120.8 | 128.2 | 109.1 | 403.4 | 1078.5 |
| 154 | 315.7 | 3650.2 | 708.6 | 1687.8 | 203.3 | 594.7 | 7527.9 | 3225.5 |
| 155 | 173.6 | 320.6 | 127.8 | 180.3 | 169.7 | 101.4 | 200.9 | 264.6 |
| 156 | 45 | 254 | 61.8 | 80.7 | 117.5 | 103.3 | 127.8 | 570.5 |
| 157 | 136.9 | 129.1 | 120.6 | 168.2 | 166.4 | 137.2 | 114.9 | 166.3 |
| 158 | 1.2 | 666.7 | 6.7 | 71.9 | 42.6 | 71.2 | 1801.9 | 533.3 |
| 159 | 1953.4 | 2285.3 | 3885.5 | 2682.2 | 3034.8 | 3946.6 | 1345.5 | 2577.3 |
| 161 | 319.4 | 1302 | 72.9 | 3.4 | 29.5 | 683.5 | 715.7 | 2665.9 |
| 162 | 110.3 | 41 | 257 | 370 | 123.6 | 69.8 | 481.8 | 259.5 |
| 164 | 1671 | 9328.7 | 183.5 | 423.7 | 105.2 | 5095.9 | 2963.9 | 3993.3 |
| 166 | 4942.9 | 5074.8 | 7381.4 | 6040.8 | 6386.6 | 3311.8 | 2123.6 | 2331.2 |
| 167 | 749.2 | 988.4 | 3046.3 | 1523.6 | 2291.6 | 1961.5 | 174.8 | 2118.3 |
| 168 | 751.1 | 1098.9 | 495.4 | 268.2 | 581.5 | 558 | 495.7 | 1402.7 |
| 169 | 7503.7 | 2997.6 | 1624.4 | 7311.5 | 4532.9 | 8835.6 | 8236.4 | 8878.2 |
| 170 | 1092.7 | 3984.7 | 914.4 | 1353.1 | 1072.7 | 3583.1 | 6780.1 | 2356.3 |
| 171 | 1091.9 | 103.2 | 2345.5 | 1683.7 | 1336.1 | 67.4 | 1413.4 | 1527 |
| 172 | 647.2 | 545.2 | 686.6 | 676.8 | 882.2 | 643.6 | 381.4 | 537.6 |
| 173 | 310.1 | 807.9 | 316.1 | 454.7 | 125 | 570.2 | 2254.5 | 686.8 |
| 176 | 352.8 | 3659.5 | 161.7 | 682 | 66.9 | 511.8 | 1820.1 | 1938.3 |
| 177 | 266.4 | 422.7 | 627.2 | 464.7 | 698.3 | 660 | 182.2 | 629.9 |
| 178 | 78.1 | 2301.7 | 104.6 | 251.2 | 360.2 | 1258.1 | 2294.6 | 1651.5 |
| 179 | 128.9 | 1233.1 | 66.2 | 511.4 | 130.9 | 58.8 | 1647.2 | 1137.8 |
| 180 | 105.8 | 710.8 | 107 | 181.6 | 146.8 | 78.7 | 1093 | 420.8 |
| 181 | 9757 | 7620 | 13164.3 | 9078.6 | 11540.2 | 7914.9 | 4064.3 | 8345.1 |
| 182 | 496.8 | 473.5 | 600.9 | 574.6 | 265.4 | 353.5 | 605.3 | 428.7 |
| 183 | 392.6 | 39.9 | 1783.8 | 80.3 | 243.4 | 23.2 | 486.1 | 1115.1 |
| 184 | 259.1 | 22002.2 | 275.3 | 392.4 | 236.8 | 191.2 | 3061.9 | 2469.9 |
| 185 | 1311.7 | 3109.9 | 1695.2 | 2606.4 | 1042.9 | 801.2 | 1663.7 | 6383.1 |
| 186 | 309.2 | 248.2 | 731.9 | 456.2 | 752.1 | 448.3 | 238.7 | 330.6 |
| 187 | 360.2 | 80.5 | 282.7 | 112.1 | 108.2 | 52.7 | 216.2 | 310.5 |
| 188 | 146.5 | 209 | 366.3 | 96.4 | 142.6 | 103.3 | 898.6 | 358.8 |
| 189 | 84.1 | 133.6 | 168.1 | 73.6 | 133 | 48.1 | 304.3 | 209.3 |
| 190 | 66 | 370 | 79.8 | 99.8 | 90.5 | 387.4 | 665.7 | 640.7 |

| | | | | | | | | |
|---|---|---|---|---|---|---|---|---|
| 191 | 1054.3 | 1687.1 | 1089.6 | 4008.9 | 457.1 | 1051.3 | 4042.4 | 1869.4 |
| 192 | 101.9 | 78.1 | 134.8 | 200.9 | 179.1 | 65.7 | 775.6 | 145.6 |
| 193 | 815.2 | 514.5 | 1367.9 | 1552.3 | 1059.7 | 2629.4 | 354.5 | 944.3 |
| 194 | 138.2 | 264.9 | 193.5 | 255.5 | 125.4 | 157.6 | 270.8 | 185.4 |
| 195 | 269.8 | 168.4 | 314.1 | 235.5 | 344.1 | 291 | 109.8 | 242.7 |
| 196 | 1835 | 1891 | 3022.5 | 2154.7 | 3025 | 5691.1 | 1224 | 6848.2 |
| 197 | 78 | 67 | 146.6 | 89.4 | 111.8 | 94.4 | 72.1 | 99.4 |
| 198 | 59.7 | 349.1 | 117.3 | 72.4 | 77.7 | 65.9 | 89.4 | 217.4 |
| 199 | 2202.9 | 301.5 | 120.6 | 6209.3 | 190.5 | 91 | 1705.6 | 642.7 |
| 200 | 369.9 | 467.8 | 572.4 | 164.5 | 285 | 358.3 | 533 | 694.8 |
| 201 | 4783.4 | 1093.3 | 42.1 | 7327.6 | 380.1 | 865.7 | 2830.3 | 670.3 |
| 203 | 229.2 | 565.9 | 244.9 | 314.3 | 126.5 | 350.8 | 4025.7 | 1159.8 |
| 204 | 1028.9 | 1353.2 | 742.4 | 630.8 | 467.1 | 794.8 | 1093.2 | 723.2 |
| 205 | 4521.1 | 3738.9 | 6165.2 | 5757.6 | 2714.1 | 3696 | 4479 | 5109.5 |
| 206 | 286.5 | 137.7 | 822.1 | 155.9 | 838.5 | 245.4 | 1616.9 | 203.7 |
| 207 | 1415.9 | 489.1 | 680.7 | 771.5 | 469.4 | 789.4 | 93.7 | 464.5 |
| 208 | 1235.8 | 212.4 | 285.9 | 104.7 | 204.8 | 139.6 | 283.2 | 359.2 |
| 209 | 833.2 | 1350.4 | 3254.1 | 1747.4 | 5771.7 | 1318.9 | 86.5 | 2452.8 |
| 211 | 11370.8 | 683.4 | 10746.5 | 11683.6 | 36602.3 | 14294.3 | 496.2 | 11150.1 |
| 212 | 417.3 | 1679.4 | 495.3 | 397.9 | 1235.8 | 814.5 | 486.7 | 989.5 |
| 213 | 80.3 | 68.5 | 84.3 | 144.4 | 80.3 | 59 | 74.4 | 100.7 |
| 215 | 1765.1 | 754.5 | 1147.4 | 1032.5 | 842.1 | 953 | 108 | 565.1 |
| 216 | 976.1 | 385.3 | 1162.1 | 844.6 | 488.1 | 945.3 | 574.9 | 513.2 |
| 217 | 246.7 | 1309.8 | 268.5 | 563.9 | 267.1 | 1014.6 | 687.1 | 752.5 |
| 218 | 309.8 | 166.4 | 348.5 | 328.9 | 276.7 | 314 | 111.9 | 166.4 |
| 220 | 75.3 | 89.1 | 81.1 | 68.8 | 102.4 | 64.6 | 58.5 | 66.7 |
| 221 | 711.2 | 968.6 | 587.7 | 780.3 | 463.3 | 617.2 | 1979.7 | 1046.5 |
| 223 | 360.9 | 409.7 | 327.9 | 542.2 | 323.6 | 203.4 | 535.9 | 305.2 |
| 227 | 720 | 681.3 | 761.1 | 2095.7 | 501.1 | 532.7 | 2050.6 | 2295.1 |
| 228 | 319.6 | 300.8 | 802.4 | 439.9 | 916.7 | 403.2 | 157.7 | 507 |
| 229 | 228.5 | 277.6 | 251.1 | 185.2 | 164.1 | 200.4 | 180.1 | 258.6 |
| 230 | 277.5 | 1169.1 | 166.3 | 609.9 | 138.4 | 112.4 | 1313.4 | 1176.2 |
| 231 | 146.9 | 281.7 | 111 | 166 | 120.6 | 124.9 | 1309.9 | 513.5 |
| 232 | 1325.8 | 198 | 6317 | 350.3 | 1427 | 140.7 | 1196.8 | 3680.1 |
| 233 | 6765.2 | 7965.7 | 6520.5 | 7610.7 | 2523.6 | 3946.5 | 15970.8 | 5207.9 |
| 235 | 798.9 | 137.6 | 1126.6 | 2196.9 | 740.6 | 73.9 | 877 | 866.4 |
| 236 | 109 | 495.7 | 368.7 | 241.9 | 407.9 | 187.5 | 496.9 | 426 |
| 237 | 1643.3 | 2222.9 | 1846 | 1647.1 | 1467.4 | 1755.2 | 1820.3 | 2097.6 |
| 238 | 1432.8 | 1140.6 | 1085.2 | 1024.6 | 592.9 | 1468.4 | 1314.4 | 1287.1 |
| 239 | 83.3 | 421.5 | 345.8 | 665.5 | 279.4 | 372.4 | 776.5 | 766.3 |
| 240 | 140.9 | 406.6 | 453 | 953.8 | 285.9 | 481.1 | 1780.9 | 951.5 |
| 241 | 234.7 | 932.4 | 931.7 | 1944 | 457.3 | 892.7 | 2797.1 | 2091.7 |
| 242 | 3314 | 21699.6 | 1079.3 | 21653.5 | 3263.3 | 3729.4 | 21913.7 | 24536 |
| 243 | 354 | 246.6 | 412.2 | 1107.5 | 269.7 | 217.5 | 691.3 | 311.1 |
| 244 | 222 | 388.3 | 205 | 223.8 | 105.6 | 325.5 | 474.4 | 399.6 |
| 246 | 472.3 | 1406 | 305.7 | 434.6 | 199.4 | 177.4 | 1088.7 | 901.8 |
| 247 | 92.8 | 98.8 | 151 | 126.2 | 202.2 | 107.4 | 83.4 | 102.1 |
| 248 | 212.2 | 3753.3 | 158.2 | 227.2 | 186.1 | 449.4 | 2480 | 3964.5 |
| 249 | 1409.4 | 1281.8 | 405.1 | 1043 | 649.5 | 1270.8 | 1958 | 1894.7 |
| 251 | 99.7 | 776.5 | 96.3 | 222.9 | 77.6 | 219.8 | 1995.8 | 859.5 |
| 253 | 275 | 293.5 | 302.1 | 128.1 | 252.8 | 311 | 316.4 | 291.9 |
| 254 | 695.9 | 1525.8 | 629.1 | 370.5 | 121.7 | 2570 | 1228.6 | 1090.4 |
| 255 | 60.9 | 2489.7 | 89.7 | 245.4 | 289 | 1251.3 | 2613.6 | 1698.6 |
| 256 | 12009.8 | 3823 | 76.1 | 6109 | 998.6 | 1154.1 | 6963.6 | 1470.4 |
| 257 | 181.8 | 218.1 | 208 | 382.8 | 124.9 | 107 | 369.9 | 216.5 |
| 258 | 864.1 | 663.9 | 212.2 | 1288.3 | 130 | 47.2 | 2891.5 | 350.9 |
| 259 | 235.3 | 658.4 | 1209.1 | 373.2 | 212.5 | 143.5 | 1662.8 | 1191.8 |
| 260 | 151.4 | 161.7 | 168.5 | 138.6 | 240.5 | 128.7 | 127.1 | 189.1 |

| | | | | | | | | |
|---|---|---|---|---|---|---|---|---|
| 261 | 200.6 | 567.6 | 409.5 | 332.1 | 282.3 | 256.2 | 194 | 303.8 |
| 263 | 524.9 | 637.2 | 549.3 | 697.3 | 354.8 | 473.9 | 797.2 | 691.2 |
| 263 | 262.8 | 1110.6 | 213 | 541.8 | 164.6 | 154.5 | 1268.5 | 980.9 |
| 265 | 278.9 | 105.6 | 510.8 | 504.8 | 664 | 337.3 | 59.8 | 364 |
| 266 | 414.8 | 163.6 | 326.2 | 220.7 | 601.9 | 433.1 | 158.4 | 283.7 |
| 256 | 126.6 | 169.2 | 102.6 | 129.7 | 138.6 | 131.2 | 232.7 | 376.5 |
| 268 | 455 | 1366.4 | 1736.7 | 757.1 | 890.5 | 1003.2 | 289.5 | 1318 |
| 269 | 29.7 | 103.9 | 37 | 84.9 | 68.8 | 46 | 87.8 | 154.9 |
| 270 | 198.8 | 186.3 | 209.5 | 175.8 | 440 | 234.1 | 176.8 | 287.7 |
| 271 | 239.3 | 1388 | 167.3 | 2027.5 | 279.7 | 256.3 | 3061.5 | 829.7 |
| 272 | 368 | 514.5 | 501.9 | 443.2 | 535.3 | 513.2 | 464.6 | 370.6 |
| 273 | 3575.1 | 1142.1 | 4935.8 | 2965.1 | 4032.3 | 5489.5 | 628.9 | 5515.9 |
| 274 | 369 | 1242.3 | 929 | 638.7 | 910.6 | 393.1 | 3068.9 | 1428 |
| 275 | 331.8 | 1186.4 | 419.7 | 790.1 | 385.7 | 210.6 | 387.1 | 2099.4 |
| 276 | 1037.3 | 821.9 | 994.3 | 2971.4 | 802.9 | 655.6 | 3409.5 | 3911.4 |
| 277 | 154 | 235.3 | 180.6 | 192.9 | 222 | 146.1 | 209.9 | 210.9 |
| 278 | 393.7 | 2185.4 | 1095.9 | 1106.9 | 475.5 | 287.1 | 2762.2 | 1290.3 |
| 279 | 1931.9 | 1574.6 | 2162.6 | 1696.4 | 1482 | 1484.1 | 2472.1 | 2330 |
| 280 | 2954.9 | 2033.6 | 2673.9 | 2531.2 | 1688.4 | 1936.3 | 3691.5 | 2628.7 |
| 281 | 76.5 | 295.7 | 134.9 | 81.7 | 87.5 | 993.9 | 793 | 372.3 |
| 282 | 295.3 | 214.1 | 295 | 3209.3 | 379.2 | 245.1 | 981.6 | 261.1 |
| 283 | 249.5 | 269.5 | 265.3 | 232.1 | 431.8 | 208.7 | 205.3 | 271.7 |
| 284 | 73.5 | 112.2 | 61.9 | 75.7 | 76.4 | 97.7 | 57.1 | 92.7 |
| 285 | 111 | 99.4 | 75.2 | 101.1 | 94.7 | 68.6 | 93.9 | 98 |
| 286 | 629.8 | 52.2 | 799.8 | 1994.7 | 465.5 | 828.9 | 53.1 | 264.8 |
| 287 | 115.5 | 113 | 87.7 | 122.5 | 134.4 | 100.9 | 81.5 | 95.9 |
| 288 | 109.4 | 141.8 | 130.1 | 140.7 | 149.8 | 126.1 | 108.4 | 112.5 |
| 289 | 1666 | 1269.4 | 941.6 | 5416.8 | 893.2 | 209.9 | 5258.5 | 2320.1 |
| 291 | 79.6 | 122.1 | 96 | 292.2 | 114.6 | 97.9 | 167.1 | 160.9 |
| 292 | 226.5 | 5489.8 | 317.3 | 841.6 | 96.1 | 81.2 | 4786.4 | 2366.7 |
| 293 | 173.9 | 986.8 | 92 | 624.7 | 212.3 | 203.9 | 667.1 | 929.4 |
| 295 | 312.6 | 1375.5 | 229.5 | 1024.5 | 598 | 569.3 | 688.5 | 1755 |
| 296 | 1129.7 | 1734.4 | 1277.5 | 815.2 | 602.9 | 561.5 | 2271.7 | 1202.6 |
| 297 | 81.6 | 53.9 | 53.8 | 152.2 | 91.5 | 62.5 | 118.6 | 45.6 |
| 298 | 394.3 | 619.7 | 407.5 | 404.3 | 288.6 | 242.2 | 506.9 | 419.7 |
| 299 | 869.8 | 1276.7 | 1226.2 | 959.5 | 865.1 | 1714.9 | 2366.2 | 1985.2 |
| 300 | 273.3 | 1444.8 | 240.7 | 364.3 | 206.4 | 660 | 1568.7 | 998 |
| 301 | 354.5 | 293.6 | 797 | 439.2 | 5202.8 | 496.9 | 215.5 | 810.2 |
| 302 | 43 | 69.4 | 22.9 | 53.4 | 24.2 | 8.5 | 116.5 | 61.8 |
| 303 | 635.7 | 390 | 1008.6 | 773.9 | 568.9 | 855.4 | 413.7 | 576.5 |
| 304 | 565.4 | 436.2 | 225.3 | 950.6 | 633.7 | 252.3 | 868 | 425.2 |
| 305 | 213.5 | 1945 | 86.7 | 250.1 | 230.8 | 450.4 | 693.3 | 661.4 |
| 306 | 150.6 | 895.3 | 136.6 | 156.5 | 236.5 | 135.1 | 1745.4 | 158.5 |
| 307 | 127.6 | 203.9 | 134.7 | 192.6 | 264.9 | 138.8 | 211.5 | 228.6 |
| 308 | 1248.1 | 607.6 | 1715.7 | 815.5 | 1207.6 | 1667 | 526.5 | 1140.3 |
| 310 | 323.3 | 105.3 | 561.9 | 412.8 | 554.5 | 441.2 | 95.6 | 355.5 |
| 311 | 187.1 | 409 | 134.4 | 246.2 | 66.9 | 232.4 | 864.3 | 176.7 |
| 312 | 215.6 | 35.7 | 964.5 | 512.9 | 1442.3 | 743.1 | 1.2 | 712.1 |
| 313 | 102.1 | 614.8 | 77.9 | 187.7 | 226.5 | 400.1 | 210.6 | 454.4 |
| 314 | 681 | 1706.8 | 671.1 | 751.7 | 185.6 | 303.4 | 684.7 | 792.1 |
| 315 | 211.5 | 216.7 | 421.9 | 234 | 416.7 | 500.7 | 245.9 | 280 |
| 316 | 435.5 | 619.5 | 376.2 | 536.9 | 395.4 | 330.2 | 704.9 | 460.6 |
| 317 | 168.8 | 203.4 | 167.5 | 2721.9 | 212.3 | 142.8 | 142.3 | 295.9 |
| 318 | 129.1 | 300.4 | 132.8 | 150.6 | 54.1 | 39.7 | 1463.9 | 297.9 |
| 319 | 438.9 | 357.5 | 187.5 | 180.3 | 354.2 | 257.4 | 855.9 | 322.5 |
| 320 | 533.7 | 201.6 | 562 | 465.6 | 667.1 | 655.6 | 266.9 | 475.1 |
| 321 | 320.6 | 174.3 | 471.9 | 402.1 | 561.7 | 371.6 | 180.9 | 319.4 |
| 322 | 257.1 | 275.3 | 249 | 193.6 | 264.7 | 232 | 267.4 | 249.9 |

| | | | | | | | | |
|---|---|---|---|---|---|---|---|---|
| 323 | 2838.3 | 3740 | 2267.7 | 2105.4 | 2020.1 | 7965.7 | 2455.6 | 6172.4 |
| 324 | 139.6 | 720.8 | 116.1 | 87.9 | 66.3 | 225.9 | 269 | 421.6 |
| 325 | 325.1 | 538.7 | 346.6 | 353 | 241.9 | 342.5 | 547.1 | 466.2 |
| 326 | 6936.9 | 3406 | 3724.5 | 6176 | 2949 | 3655.6 | 1280.4 | 2870.4 |
| 327 | 29.3 | 28.4 | 40.5 | 34.8 | 1.1 | 43.1 | 50.5 | 85.2 |
| 328 | 323.8 | 284.6 | 596.2 | 451.7 | 857.7 | 576.7 | 72.6 | 488.6 |
| 329 | 76 | 83.5 | 69.9 | 71.3 | 81 | 78.7 | 65.1 | 41.9 |
| 330 | 148.3 | 861.9 | 37.4 | 251.5 | 19.5 | 53.5 | 1666.2 | 255.1 |
| 331 | 232.2 | 284.6 | 291.4 | 279.4 | 449.1 | 243.7 | 257.3 | 263.5 |
| 333 | 420 | 491.9 | 777.5 | 589.6 | 728.3 | 591.1 | 250.1 | 419.1 |
| 334 | 179.2 | 509.5 | 135.4 | 165.9 | 136.1 | 152.8 | 170.2 | 551.5 |
| 335 | 109 | 243.2 | 144.4 | 104.1 | 452.1 | 72.9 | 710.8 | 914.6 |
| 336 | 765.5 | 230.7 | 670.7 | 465 | 499.8 | 294.9 | 129.3 | 318.8 |
| 337 | 312.9 | 1159 | 419.7 | 497.2 | 178 | 165.9 | 990.9 | 1049.7 |
| 338 | 577.4 | 617.7 | 185.9 | 401.6 | 177.5 | 251.9 | 956.2 | 491.5 |
| 339 | 392.3 | 332.8 | 470.3 | 340.9 | 615.4 | 377.2 | 170.7 | 322.8 |
| 340 | 246.7 | 275.9 | 213.8 | 279.9 | 271.3 | 234.8 | 170.6 | 251.1 |
| 341 | 539.3 | 411.3 | 601 | 608.3 | 542.6 | 515.2 | 389.8 | 567.6 |
| 343 | 244.1 | 385 | 83 | 562.5 | 1435.6 | 259.1 | 2195.3 | 417.3 |
| 344 | 1212.4 | 452.6 | 1195.1 | 997.3 | 1945.3 | 1246.2 | 594.8 | 932 |
| 345 | 264.5 | 585.5 | 216.6 | 239.2 | 204.1 | 247.3 | 398.5 | 204.5 |
| 347 | 1738 | 3503.5 | 5107.2 | 2563.1 | 2564.3 | 2751.2 | 1484 | 4149.2 |
| 348 | 3976.7 | 7721.4 | 6875.5 | 4637.6 | 3708.3 | 6099.2 | 5834.6 | 7036.6 |
| 349 | 273.4 | 377.5 | 176.5 | 381.9 | 233.3 | 415.6 | 597.8 | 380.1 |
| 350 | 321.2 | 741.3 | 399.7 | 390.9 | 121.3 | 191.6 | 653.8 | 456.2 |
| 351 | 524.9 | 2215.5 | 274.7 | 977.6 | 159.5 | 507.2 | 2220.4 | 809.3 |
| 352 | 503.6 | 131.4 | 464 | 399.1 | 339.9 | 347.1 | 171.6 | 202.5 |
| 353 | 362.5 | 223.7 | 256.6 | 221.7 | 248.9 | 379.2 | 318.9 | 224.1 |

3j

| SEQ ID NO: | 938TT | 940TT | 941TT | 945TT | 946TT | 947TT | 948TT | 954TT |
|---|---|---|---|---|---|---|---|---|
| 354 | 124.7 | 131.5 | 68.5 | 1831.3 | 2991.1 | 1970.3 | 4151.3 | 697.9 |
| 355 | 60.5 | 58.5 | 18.7 | 651 | 2959.8 | 3221.3 | 3037.9 | 91.9 |
| 356 | 930.6 | 668.1 | 621.5 | 1481.2 | 524 | 619.9 | 852.9 | 343.7 |
| 357 | 668.5 | 1037.6 | 614.1 | 992.7 | 744.6 | 692.6 | 698.9 | 500.3 |
| 358 | 534.1 | 2157.9 | 778.4 | 766.2 | 2015.3 | 1757.9 | 1431.4 | 322.3 |
| 359 | 630.5 | 285.6 | 377.6 | 284.2 | 331.2 | 319.5 | 309.2 | 503.3 |
| 362 | 416.8 | 682.5 | 687 | 883.5 | 1080.8 | 836.2 | 746 | 476.8 |
| 360 | 275.7 | 208.2 | 221.9 | 220.7 | 571.6 | 596.9 | 804.3 | 159.4 |
| 361 | 583.8 | 838.6 | 283.7 | 134.5 | 371.1 | 271.3 | 137.7 | 156.1 |
| 363 | 425 | 358.8 | 438.2 | 340.4 | 460.3 | 536.2 | 369.7 | 849.5 |
| 1 | 912.1 | 505.2 | 651.7 | 1316.8 | 1552.6 | 1201.3 | 1465.6 | 256.1 |
| 2 | 2300.4 | 2928.6 | 1830.4 | 985.5 | 3226.8 | 1970 | 1555.6 | 876.4 |
| 7 | 656.3 | 686.7 | 1076.6 | 47.5 | 1068.9 | 536 | 365.4 | 1299.1 |
| 8 | 1044.1 | 2152.8 | 933.8 | 991 | 820.3 | 808.2 | 739.8 | 1547.8 |
| 9 | 4509.2 | 1083.6 | 1954.2 | 1300.1 | 2078.3 | 3908 | 1283.1 | 685.2 |
| 10 | 140.1 | 88.1 | 122.4 | 1873.2 | 92.1 | 150.4 | 164.5 | 113.4 |
| 11 | 70.2 | 163.3 | 140.5 | 707 | 443.9 | 558.3 | 863.6 | 82.9 |
| 13 | 151.7 | 615.2 | 230.4 | 207 | 349.6 | 491.1 | 268.6 | 107.8 |
| 14 | 292.7 | 1519.9 | 379.4 | 942.7 | 1077.9 | 1723.4 | 1237 | 119.8 |
| 15 | 287.3 | 218.1 | 204.7 | 3524.8 | 341.4 | 2287.5 | 744.4 | 147.9 |
| 16 | 1093.9 | 1353.6 | 1394.3 | 376.6 | 2242.4 | 2345.1 | 4508.1 | 532.5 |
| 17 | 1167.2 | 1155.9 | 933 | 517 | 1588.4 | 984.5 | 1068.3 | 312.3 |
| 19 | 241.1 | 159.7 | 215.2 | 431.1 | 311 | 2812 | 648.8 | 285.5 |
| 20 | 1.2 | 13.8 | 1.1 | 238.8 | 388.1 | 452.5 | 759.1 | 1.2 |

| | | | | | | | | |
|---|---|---|---|---|---|---|---|---|
| 21 | 13.1 | 453.2 | 114.2 | 12979.2 | 1497.1 | 2096.8 | 4503 | 98.9 |
| 22 | 3034.3 | 2692.9 | 7458.4 | 7149.4 | 3919.7 | 5688.1 | 5625.7 | 1444.4 |
| 23 | 240.2 | 50.1 | 1.1 | 2306.1 | 246.9 | 2461.4 | 936.2 | 1.1 |
| 24 | 167.4 | 59.1 | 67 | 383.4 | 283.5 | 552.3 | 205.8 | 59.9 |
| 26 | 1363.1 | 333 | 155 | 3026.1 | 1238.7 | 9164 | 2694.5 | 66.1 |
| 27 | 664.2 | 691.5 | 441.3 | 957.5 | 1357 | 954.3 | 1589.7 | 352.8 |
| 29 | 89.7 | 109.7 | 122.7 | 3754.7 | 40.3 | 136.4 | 202.8 | 163.6 |
| 30 | 132.2 | 43.3 | 144.7 | 1236.6 | 203.1 | 1185.3 | 426.3 | 73.8 |
| 31 | 70.5 | 216.2 | 85.5 | 16661.7 | 8943.3 | 13168.1 | 16458.7 | 70.6 |
| 33 | 754.7 | 291.7 | 496 | 10017.8 | 2060.8 | 7990.6 | 3081.2 | 83.8 |
| 34 | 3799.4 | 1463.1 | 2114.5 | 557.9 | 529.3 | 1398.3 | 512.3 | 4496.7 |
| 35 | 49.6 | 245.8 | 449.4 | 283.1 | 1235.6 | 706.1 | 239.4 | 71 |
| 36 | 319.6 | 311.9 | 235.6 | 621.1 | 2677.9 | 398 | 273.3 | 356.8 |
| 37 | 165.2 | 175.1 | 219.1 | 198.1 | 231.1 | 292.9 | 220.8 | 375.8 |
| 38 | 588.7 | 850.6 | 583.8 | 5243.7 | 1146.5 | 3990 | 1615.2 | 79.3 |
| 40 | 474.3 | 418.3 | 456.1 | 5232.8 | 561 | 592.8 | 854.7 | 557.2 |
| 41 | 145.4 | 200.5 | 95.4 | 209.9 | 739.6 | 1408.3 | 703.3 | 263.3 |
| 42 | 259.7 | 422.1 | 296.8 | 134.4 | 388.8 | 299.2 | 320 | 346.4 |
| 43 | 312.7 | 469.7 | 413.4 | 844.8 | 254.3 | 342.2 | 278.6 | 1487.4 |
| 44 | 513.5 | 424.1 | 470.7 | 554 | 1223.3 | 1138.7 | 885.5 | 455.5 |
| 45 | 890.2 | 736.7 | 547.2 | 940.6 | 1426.2 | 1512.1 | 1930.9 | 1179.9 |
| 46 | 245 | 310.6 | 350.6 | 275.4 | 289.5 | 350 | 292.2 | 348.2 |
| 47 | 282.9 | 220.4 | 144.2 | 180.6 | 703.3 | 454.3 | 694.6 | 183.6 |
| 48 | 65.9 | 25.3 | 41.1 | 147.2 | 354.7 | 1468.6 | 413 | 15.7 |
| 49 | 257.1 | 1250.1 | 224.5 | 12537.4 | 5118.7 | 2265.3 | 4031 | 294.4 |
| 50 | 174.5 | 90.2 | 118.5 | 1385.9 | 212.4 | 962.9 | 427.9 | 32.6 |
| 51 | 95.1 | 552.7 | 56.4 | 170.8 | 478 | 419.9 | 827.7 | 730.2 |
| 52 | 197 | 196.4 | 147.1 | 401.4 | 251.1 | 232.6 | 352.8 | 240.3 |
| 53 | 48.5 | 381.9 | 96.1 | 1013.6 | 2192.5 | 931.3 | 1091.5 | 1.7 |
| 54 | 826.4 | 1710.8 | 514.8 | 1477.3 | 1185.6 | 797.4 | 1536.4 | 340.4 |
| 55 | 115.1 | 111.9 | 94.4 | 164.7 | 94 | 80.6 | 89.7 | 130.4 |
| 56 | 64 | 268.4 | 79.2 | 90.4 | 219.3 | 188.7 | 175.5 | 63.7 |
| 57 | 617.9 | 1038.1 | 375.9 | 868.2 | 12415.3 | 3983 | 1493.6 | 579.1 |
| 58 | 584.4 | 972.6 | 292.8 | 2931.7 | 11316.3 | 5093.9 | 2162.2 | 939.4 |
| 59 | 1552.8 | 1358.2 | 1824.4 | 3299.3 | 3044.8 | 3483.9 | 4854.3 | 122.2 |
| 60 | 123.4 | 154 | 130.4 | 239.2 | 412.9 | 378.4 | 248.4 | 139.6 |
| 61 | 193 | 106.4 | 98 | 402.3 | 130.9 | 79 | 142.9 | 155.7 |
| 62 | 1365.1 | 178 | 2305.2 | 2981.4 | 3752.1 | 2025.7 | 3563.2 | 4345.7 |
| 63 | 145.2 | 365.5 | 174.3 | 650.2 | 742.9 | 588.7 | 744.3 | 153.8 |
| 64 | 143.1 | 168.7 | 146.9 | 198.1 | 479.3 | 303.3 | 288.3 | 1482.7 |
| 65 | 149.3 | 80.8 | 54.7 | 446.7 | 254.3 | 265.4 | 155.3 | 56.9 |
| 66 | 834.1 | 822.9 | 752.9 | 332.7 | 1793.6 | 1625.8 | 2019.9 | 272.4 |
| 67 | 35954.4 | 28315.7 | 36691.2 | 2509.3 | 29331.4 | 28689.2 | 19265.7 | 24099.5 |
| 68 | 2348.5 | 1225 | 3289.6 | 23.4 | 760.4 | 1000 | 260.1 | 1110.3 |
| 69 | 3844.9 | 1911.7 | 4757.3 | 675.4 | 2667.4 | 3541.2 | 1114.3 | 7548.2 |
| 70 | 2817.2 | 5474 | 2510.6 | 6068.1 | 4627.5 | 5853 | 4221.9 | 8610 |
| 71 | 194.3 | 578.8 | 125.9 | 216.5 | 9508.5 | 3820.8 | 1470.2 | 84.3 |
| 72 | 588.1 | 1284.2 | 401.1 | 327.2 | 2278.5 | 1343.5 | 1418 | 131 |
| 73 | 258 | 239.7 | 218.8 | 232.7 | 766.7 | 694.8 | 726.4 | 198.6 |
| 75 | 252.1 | 212.9 | 239.3 | 123.9 | 1227.9 | 463.3 | 938.9 | 272.6 |
| 76 | 2962.6 | 845.5 | 915.9 | 198.8 | 1587.9 | 1178 | 574.9 | 3611 |
| 77 | 16342.3 | 2666.5 | 15026.8 | 503.1 | 1235 | 1954.2 | 2093.6 | 4483.2 |
| 78 | 729.3 | 762.4 | 748.4 | 157.2 | 758.2 | 290.5 | 576.4 | 1050.6 |
| 79 | 174.2 | 122.2 | 114.9 | 257.1 | 232.1 | 270.7 | 344.5 | 138 |
| 80 | 1248 | 787.5 | 1009.5 | 282.2 | 770.1 | 649.9 | 1098.8 | 266.3 |
| 81 | 115.6 | 272.3 | 127.3 | 229.2 | 5071.1 | 11031.3 | 7637.7 | 43.7 |
| 82 | 610.7 | 314.6 | 210.5 | 232 | 257.3 | 258.9 | 135.4 | 1823.3 |
| 84 | 94.2 | 94.6 | 92.6 | 83 | 304.7 | 340 | 208.7 | 87.3 |

| | | | | | | | | |
|---|---|---|---|---|---|---|---|---|
| 85 | 140.2 | 169.4 | 138.1 | 2066.1 | 1108.1 | 1254 | 441.8 | 428.8 |
| 86 | 39.9 | 71.9 | 99.7 | 82.2 | 84.5 | 383.5 | 141 | 293.5 |
| 87 | 298.4 | 166.5 | 180.3 | 11204.7 | 1172.7 | 669.1 | 1630.9 | 199.4 |
| 88 | 5196.8 | 3340.8 | 5827.4 | 127.5 | 1059.3 | 1704.6 | 635.3 | 1431.5 |
| 89 | 235.6 | 196.8 | 215.8 | 267.6 | 325.5 | 267.5 | 617.5 | 237 |
| 90 | 115.4 | 85.7 | 96.9 | 368.5 | 152.8 | 292.9 | 344.8 | 75.4 |
| 91 | 522 | 538.6 | 254.8 | 3408.3 | 6047.2 | 2646.8 | 1460.5 | 1153.4 |
| 92 | 3787.5 | 7863.1 | 2264 | 316.1 | 7435.1 | 5854.5 | 5932.4 | 213.6 |
| 93 | 338.7 | 224 | 230.8 | 159.4 | 208.2 | 159.2 | 161.6 | 162.1 |
| 94 | 635.6 | 617.5 | 996.8 | 254.1 | 860.9 | 768 | 1265.5 | 29.8 |
| 95 | 699.7 | 1108.4 | 462.4 | 81.3 | 719 | 569.5 | 312.2 | 76.1 |
| 96 | 333 | 207.6 | 214.4 | 289.7 | 323.1 | 290.3 | 295.4 | 231.9 |
| 97 | 416.2 | 279.5 | 425.1 | 563.7 | 405.6 | 387 | 496.6 | 170.1 |
| 98 | 27.3 | 37.6 | 37.7 | 36.2 | 18.7 | 37.2 | 39.6 | 211.1 |
| 99 | 301.5 | 228.7 | 224.2 | 752.5 | 370.7 | 398.9 | 427.9 | 210 |
| 100 | 2314.7 | 3267.4 | 16920.7 | 300.1 | 254.3 | 4199.9 | 860.3 | 2812.9 |
| 101 | 330.3 | 321.7 | 727.2 | 74.4 | 500.6 | 272 | 225.2 | 171.9 |
| 102 | 169.4 | 127.4 | 144.6 | 162.3 | 150.2 | 178.5 | 170.9 | 171.9 |
| 103 | 381.1 | 314.1 | 488 | 277.5 | 491.6 | 397.1 | 366.3 | 377.4 |
| 104 | 692.9 | 415.8 | 921.4 | 315.6 | 185.7 | 234.9 | 58.6 | 310.2 |
| 105 | 372.6 | 381.6 | 637 | 159.3 | 149.3 | 300.4 | 79.5 | 455.3 |
| 106 | 646.1 | 411.7 | 1024.2 | 213.3 | 167.6 | 292.1 | 69.4 | 362 |
| 107 | 267 | 257.5 | 123.5 | 748.7 | 451.6 | 527.6 | 584.4 | 230.2 |
| 108 | 573.6 | 195.9 | 332.9 | 184.1 | 209.2 | 219.8 | 263 | 536.8 |
| 109 | 61.9 | 1019.9 | 56.4 | 186.2 | 3392.9 | 1439.4 | 1742.7 | 27.8 |
| 110 | 25.1 | 2062.4 | 22.9 | 215.1 | 7704.2 | 3177 | 4011.8 | 1.1 |
| 111 | 143.5 | 479.3 | 2111.4 | 41.4 | 269.7 | 337.1 | 266.3 | 190.6 |
| 112 | 551.8 | 742.9 | 559 | 155 | 1022.1 | 1281.4 | 1187.5 | 1294.4 |
| 113 | 99.7 | 563.8 | 103.4 | 189.8 | 444.1 | 262.8 | 316.1 | 223.5 |
| 114 | 503.8 | 603.2 | 350.4 | 104.9 | 714.7 | 677 | 361.5 | 212.4 |
| 115 | 401.8 | 436 | 616.1 | 1714.8 | 863.7 | 903.2 | 724.2 | 444.3 |
| 116 | 209.7 | 203.7 | 150.5 | 2167.8 | 763.4 | 502.8 | 591.3 | 201.2 |
| 117 | 3806.6 | 1057.1 | 3146.1 | 179.1 | 561.9 | 533.2 | 197.2 | 320 |
| 118 | 138.9 | 49.5 | 85.3 | 477.1 | 265.1 | 194.4 | 119.2 | 509.1 |
| 119 | 81.2 | 371.2 | 135.5 | 216 | 567.1 | 144.4 | 163.5 | 165.4 |
| 120 | 160.6 | 114.3 | 71.9 | 92.6 | 176.6 | 216.8 | 101.4 | 84.2 |
| 121 | 164.4 | 411.2 | 437.2 | 213.3 | 3724.5 | 1512.3 | 3717.5 | 67.3 |
| 122 | 98.8 | 80.1 | 243.2 | 1399.7 | 264.3 | 428.5 | 386 | 304.3 |
| 123 | 228.3 | 146.3 | 183.8 | 86 | 273.3 | 303.5 | 160.2 | 337.2 |
| 124 | 151.3 | 149.7 | 152.5 | 120.2 | 120.6 | 139.9 | 109.1 | 1219.8 |
| 125 | 909.1 | 689.8 | 819.3 | 575.9 | 33514.1 | 1041.2 | 939.8 | 1157.9 |
| 126 | 1425.9 | 1166.4 | 900.7 | 103.3 | 147.1 | 234.7 | 109.9 | 694.2 |
| 127 | 99.1 | 86.5 | 106.2 | 122.9 | 202.2 | 163.7 | 251.1 | 114.5 |
| 128 | 231.7 | 208 | 253.1 | 214 | 261.3 | 71697.2 | 219.5 | 291.6 |
| 129 | 202.2 | 353.5 | 133.8 | 1125.3 | 2440.8 | 1025.8 | 1063.6 | 165.3 |
| 130 | 345.4 | 395.1 | 121 | 69 | 388.5 | 391.7 | 446.8 | 121.4 |
| 131 | 168.1 | 194.1 | 81.9 | 126.4 | 965.8 | 403.9 | 495.8 | 134.7 |
| 132 | 181 | 188.5 | 164.5 | 532.9 | 286.9 | 263.7 | 252.1 | 213.2 |
| 133 | 101.3 | 107.4 | 143.6 | 234.5 | 193.6 | 164.6 | 236.4 | 134.6 |
| 134 | 208.4 | 308.8 | 149 | 144 | 300.6 | 320.7 | 552.5 | 107.9 |
| 135 | 238.9 | 233.2 | 326.6 | 225.1 | 687.6 | 468.7 | 352.5 | 301.7 |
| 138 | 96.3 | 108.4 | 108 | 222.7 | 408.8 | 249.3 | 192.4 | 170.3 |
| 139 | 165.6 | 99.9 | 76.6 | 136.8 | 269.3 | 296.3 | 159 | 95.6 |
| 140 | 2802.5 | 2059.9 | 1086.6 | 36.8 | 599.5 | 750.1 | 666.1 | 1.3 |
| 141 | 2290.9 | 2263.4 | 2358.9 | 108.9 | 934.1 | 627.7 | 325.8 | 732.5 |
| 144 | 7698.2 | 8602.5 | 444.5 | 323.9 | 12464.5 | 11467.6 | 7038 | 368 |
| 145 | 193.7 | 146.1 | 183.3 | 157.9 | 225.5 | 228.7 | 268.8 | 245.8 |
| 146 | 240.8 | 175.6 | 204.2 | 249.1 | 261 | 358.4 | 743.9 | 242.2 |

| | | | | | | | | |
|---|---|---|---|---|---|---|---|---|
| 147 | 47.8 | 28.4 | 34.3 | 55 | 200.2 | 100.2 | 51 | 64.4 |
| 148 | 229.4 | 308.8 | 238.2 | 263.9 | 480.4 | 561.5 | 358.6 | 145.1 |
| 149 | 551.3 | 349.3 | 339.3 | 928.4 | 251.7 | 301.5 | 518.5 | 137 |
| 150 | 164.4 | 207.3 | 163.4 | 141.1 | 291.4 | 208.5 | 289.6 | 323.1 |
| 151 | 126.6 | 323.1 | 102.9 | 132 | 599.9 | 423.7 | 877.9 | 89.4 |
| 153 | 115.7 | 114.6 | 91 | 123.5 | 136.1 | 115.3 | 108.6 | 154.4 |
| 154 | 96.3 | 720.7 | 304.7 | 130.9 | 1984.8 | 5390.1 | 6547.4 | 79.7 |
| 155 | 97.9 | 124.5 | 101.4 | 112.9 | 272.1 | 587.2 | 922.9 | 167.6 |
| 156 | 87.7 | 90.5 | 95.8 | 49 | 682.8 | 491.1 | 331.1 | 59.2 |
| 157 | 111.6 | 117.5 | 128.1 | 151.8 | 208 | 161.9 | 186.4 | 149.3 |
| 158 | 65.6 | 1732.5 | 86.6 | 324.9 | 473.6 | 737.6 | 1773.8 | 15.3 |
| 159 | 3116.8 | 3452 | 5239.7 | 900.3 | 2646.8 | 1464.2 | 1902.5 | 980 |
| 161 | 14481.9 | 1741.5 | 2880.9 | 1.1 | 4831.7 | 8199.7 | 3949.6 | 50.7 |
| 162 | 89.7 | 55.3 | 28.4 | 589.7 | 490 | 589.7 | 221.5 | 35.9 |
| 164 | 177.5 | 8435.5 | 104.7 | 76.7 | 5265.1 | 293.6 | 4062.9 | 88.1 |
| 166 | 4359.3 | 2839.1 | 6163 | 1137.7 | 1081.2 | 2208.2 | 2366.9 | 3751.1 |
| 167 | 1749.1 | 1818.8 | 3470.5 | 1.2 | 1723.1 | 1291.3 | 832.1 | 565.3 |
| 168 | 710.2 | 1123.1 | 423.8 | 59.3 | 1688.4 | 894.7 | 1924 | 122.3 |
| 169 | 1524.8 | 2200.3 | 2148.9 | 1401.8 | 11518.5 | 12728.9 | 6382.1 | 1374.4 |
| 170 | 1062.4 | 2335.7 | 758 | 1597.8 | 2005.8 | 2041.3 | 3494.1 | 467.3 |
| 171 | 3638.6 | 58 | 1497.1 | 1638.6 | 545 | 2916.5 | 723.7 | 1.4 |
| 172 | 663.9 | 679.9 | 727.5 | 420.9 | 685.3 | 596.1 | 496.1 | 610.3 |
| 173 | 416.9 | 738.3 | 140.5 | 328.5 | 1336.6 | 772.4 | 1320.3 | 267.7 |
| 176 | 1.1 | 6520.4 | 150.5 | 99.2 | 3722.2 | 368.3 | 1845.4 | 29.3 |
| 177 | 545.8 | 562.4 | 1099.7 | 185.2 | 574 | 488 | 384.2 | 598 |
| 178 | 1775.4 | 1955 | 376.9 | 512.6 | 2127.5 | 1048.9 | 1852.4 | 1565.3 |
| 179 | 180.3 | 138.6 | 117.4 | 2012.3 | 1196.7 | 828.9 | 1163 | 106.1 |
| 180 | 74.4 | 892.7 | 105.8 | 237.5 | 803.4 | 419.6 | 473.4 | 114.3 |
| 181 | 9404.2 | 6484.3 | 10300.3 | 941.7 | 4892.7 | 6450.6 | 4354.9 | 9168.5 |
| 182 | 372 | 308.8 | 208.5 | 248.4 | 424.6 | 399.7 | 405 | 287.1 |
| 183 | 264 | 49.6 | 27.6 | 849.9 | 182.8 | 2050.1 | 619.5 | 1.1 |
| 184 | 340.2 | 3360.4 | 202.1 | 1220.7 | 1934.2 | 441.7 | 306.9 | 181.9 |
| 185 | 955.1 | 847.9 | 908.6 | 8586.1 | 5990 | 6583.4 | 4156.5 | 642.7 |
| 186 | 377.1 | 397.9 | 305.4 | 331.6 | 290.1 | 282.2 | 230.3 | 953.8 |
| 187 | 63 | 83.5 | 54.1 | 487.3 | 334.1 | 525.2 | 118.2 | 375.2 |
| 188 | 135.2 | 163.5 | 93.8 | 278.1 | 334.8 | 593.6 | 565 | 40.8 |
| 189 | 97.4 | 79.7 | 73.5 | 170.6 | 247.6 | 288.5 | 313.7 | 63.8 |
| 190 | 71.9 | 271.7 | 441.9 | 766.9 | 331.8 | 397.9 | 570.8 | 70.8 |
| 191 | 1184 | 1205.3 | 431.3 | 1127.3 | 2057.1 | 2357.6 | 1735.3 | 1441.4 |
| 192 | 637.6 | 81.1 | 90.9 | 98.5 | 97.8 | 124.5 | 152.4 | 136.2 |
| 193 | 1083.7 | 1465.1 | 2071.9 | 376.7 | 704.1 | 341.3 | 284.4 | 528.8 |
| 194 | 105.9 | 174.3 | 134.6 | 104.3 | 225.4 | 234.8 | 239.8 | 67.9 |
| 195 | 513.2 | 534.1 | 382 | 172.8 | 221 | 202.4 | 154.9 | 532.6 |
| 196 | 6150.6 | 6442.8 | 10030.2 | 655.3 | 4512.8 | 2383.7 | 1783.6 | 3777.8 |
| 197 | 119.9 | 70.8 | 66.6 | 405.3 | 140.1 | 126.5 | 270.4 | 248.4 |
| 198 | 86.4 | 67.1 | 56.7 | 167.2 | 622.1 | 1324.8 | 1830.6 | 65.7 |
| 199 | 63.7 | 49.5 | 36.5 | 946.4 | 3952.8 | 4557 | 3993.9 | 70 |
| 200 | 141.2 | 131.1 | 93.9 | 326.4 | 480.8 | 559.4 | 384.1 | 118.8 |
| 201 | 99.9 | 125.1 | 64.3 | 1769.6 | 3178.7 | 2044.9 | 3554.3 | 746.9 |
| 203 | 205.7 | 2333.5 | 101.7 | 55 | 1633.6 | 575.7 | 143.9 | 68.5 |
| 204 | 536.9 | 539.6 | 677.1 | 33.9 | 1308 | 734.6 | 891.1 | 781.5 |
| 205 | 2519.6 | 5145.4 | 5497.1 | 266.3 | 3930.6 | 3699 | 1755.2 | 3034.5 |
| 206 | 161.9 | 155.6 | 164.4 | 948.8 | 1321.7 | 387.6 | 243.4 | 466.4 |
| 207 | 632.9 | 3069.3 | 716.5 | 82.8 | 354.7 | 315 | 82.2 | 99.4 |
| 208 | 414.1 | 294.1 | 69.8 | 102.5 | 523.4 | 2129.8 | 361.4 | 69 |
| 209 | 2353.2 | 3015.8 | 3230.6 | 167.6 | 1434.9 | 1049.6 | 343.5 | 3631.8 |
| 211 | 25849.9 | 14301.8 | 31677 | 367.5 | 805.9 | 1538.7 | 536.8 | 17099.2 |
| 212 | 806.2 | 1682.7 | 669.3 | 1766.7 | 1465.3 | 1682.1 | 1111.3 | 2465.1 |

| | | | | | | | | |
|---|---|---|---|---|---|---|---|---|
| 213 | 95.8 | 142 | 90.3 | 97.7 | 93.5 | 71.1 | 127 | 3700.6 |
| 215 | 908.3 | 4183.7 | 1058.1 | 54.1 | 546.4 | 345.5 | 127.3 | 143.3 |
| 216 | 474.8 | 805.2 | 456.5 | 290.5 | 173.7 | 292.6 | 242.1 | 234.8 |
| 217 | 310.4 | 448.5 | 198 | 253 | 2353.6 | 1241.6 | 1337.2 | 128.5 |
| 218 | 356.8 | 253.7 | 304.9 | 121 | 189.9 | 218 | 303.7 | 349.5 |
| 220 | 88.7 | 59 | 60.1 | 118.1 | 89.5 | 87.6 | 93.5 | 429 |
| 221 | 480.5 | 659.6 | 319.4 | 233.3 | 1004.2 | 1199.6 | 880.1 | 240.6 |
| 223 | 244.2 | 220.3 | 254.2 | 2169.6 | 392.2 | 560.5 | 572.7 | 233.1 |
| 227 | 757 | 501.6 | 622.8 | 6541.8 | 749.4 | 5527.9 | 2138.8 | 93.6 |
| 228 | 608.7 | 551 | 1481.7 | 131.1 | 431.4 | 377.7 | 265 | 587.1 |
| 229 | 493.1 | 133.9 | 211.8 | 452.4 | 129.8 | 269.4 | 179.7 | 599 |
| 230 | 161.6 | 103.2 | 123.8 | 1187.9 | 2084.8 | 2370.1 | 4149.4 | 75.2 |
| 231 | 118.2 | 789.1 | 176.3 | 80 | 752.2 | 257.9 | 129.9 | 148.9 |
| 232 | 941.1 | 198.3 | 129.7 | 1787.8 | 868.5 | 6851.2 | 1950.1 | 5.6 |
| 233 | 7265.5 | 4514.5 | 2133.7 | 1354.4 | 13569.2 | 13328.5 | 4088.9 | 897.7 |
| 235 | 824 | 100.3 | 178.6 | 1170.4 | 445.6 | 1811 | 569.9 | 75.9 |
| 236 | 205.3 | 143.9 | 239.7 | 348.8 | 434.4 | 422.2 | 446.1 | 266.4 |
| 237 | 1942.7 | 1890.5 | 1088.6 | 2229.9 | 2092.3 | 1996.6 | 2088.9 | 774.4 |
| 238 | 939.7 | 1507.6 | 905.5 | 237.4 | 920 | 814.4 | 934.8 | 213.8 |
| 239 | 155.3 | 1246.4 | 106.2 | 990 | 1304.2 | 963.5 | 415.2 | 125.6 |
| 240 | 105.3 | 1516.5 | 105.5 | 2154.1 | 1232.9 | 1172.8 | 394.5 | 111.2 |
| 241 | 298.2 | 3242 | 161.3 | 3194.2 | 2781.8 | 2574.4 | 962 | 156 |
| 242 | 6684 | 765 | 2229.5 | 13301.5 | 22076.9 | 31987.1 | 27579.2 | 969.6 |
| 243 | 154.1 | 116 | 133.6 | 2751 | 287.9 | 733.2 | 891.1 | 152.3 |
| 244 | 177 | 177.5 | 176.5 | 466.1 | 826.4 | 342.9 | 309.4 | 68.7 |
| 246 | 291.6 | 200.2 | 283.7 | 627.1 | 1322 | 1294.2 | 1779 | 143.8 |
| 247 | 98.8 | 89.9 | 132.5 | 88.7 | 163 | 133.7 | 73.4 | 283.9 |
| 248 | 126.4 | 1414.5 | 201.1 | 404 | 3465.6 | 1012.6 | 1020 | 178.9 |
| 249 | 782.4 | 3162 | 1403.3 | 76.1 | 1097.8 | 767.3 | 537.1 | 537.9 |
| 251 | 161.5 | 155.1 | 63.5 | 1785.1 | 627.7 | 1481.5 | 1000.6 | 82.3 |
| 253 | 264.5 | 218 | 442.9 | 168.6 | 424.5 | 446.9 | 362.6 | 279.9 |
| 254 | 105.6 | 2093.1 | 81.7 | 82.5 | 1493.7 | 652.8 | 622.8 | 398.9 |
| 255 | 1747.2 | 2263.9 | 395 | 659.6 | 2271.9 | 1148.5 | 1978.8 | 1845.7 |
| 256 | 435.9 | 539.6 | 279.6 | 2547.3 | 3827.2 | 1885.7 | 1217.9 | 1110.2 |
| 257 | 56 | 110.7 | 80.2 | 207.2 | 269.3 | 316 | 390.9 | 8.1 |
| 258 | 9.3 | 1.3 | 118.2 | 870 | 206.6 | 1331.3 | 3545.5 | 38.6 |
| 259 | 108.7 | 29.1 | 63 | 254.6 | 602.2 | 1965.4 | 1032.4 | 1.2 |
| 260 | 164.7 | 153.3 | 244.8 | 156.4 | 212.5 | 156.2 | 106.4 | 401.2 |
| 261 | 991.9 | 382.7 | 312.5 | 75.3 | 207.2 | 161.7 | 238.6 | 71.9 |
| 263 | 714.7 | 331.8 | 539.2 | 1150 | 469.9 | 540.1 | 765.1 | 394.2 |
| 263 | 186.9 | 138 | 97.6 | 1287.2 | 1762.6 | 2495.8 | 3807.6 | 183.3 |
| 265 | 788.6 | 425.8 | 473.1 | 76.4 | 87.4 | 170.1 | 56.1 | 398 |
| 266 | 353.2 | 233.3 | 346.6 | 277.1 | 270.4 | 331.4 | 210.9 | 954.6 |
| 256 | 72.3 | 174.2 | 105.6 | 365 | 383.6 | 281.3 | 202.2 | 129.4 |
| 268 | 281.1 | 1707.5 | 870.9 | 706.9 | 2068.2 | 688.8 | 318 | 283.3 |
| 269 | 39.1 | 44.6 | 40 | 54.8 | 173.2 | 430.9 | 328.8 | 58.9 |
| 270 | 215.2 | 272.7 | 184.8 | 275.7 | 242.6 | 247.3 | 201.7 | 957.5 |
| 271 | 235.7 | 337.1 | 250.4 | 160.2 | 871.9 | 511.6 | 907.5 | 198.1 |
| 272 | 413.3 | 461.6 | 314.7 | 697 | 411.8 | 753.7 | 501.7 | 539.4 |
| 273 | 1403.7 | 2489.3 | 5667.8 | 24.3 | 1615.9 | 2911.3 | 1683 | 814.1 |
| 274 | 614.7 | 410.3 | 702.2 | 2259.7 | 916.5 | 1540.8 | 1573.5 | 855 |
| 275 | 249.7 | 229.2 | 228.5 | 8716.5 | 2253.7 | 2452.5 | 1476.9 | 255.5 |
| 276 | 1102.2 | 729.4 | 823 | 8564.6 | 1180 | 8452.9 | 3389.5 | 78.2 |
| 277 | 166.3 | 154.9 | 175.4 | 443.3 | 274.9 | 287.7 | 236.4 | 171 |
| 278 | 409.1 | 57.7 | 133.6 | 461.1 | 1337.1 | 1922.6 | 1313.9 | 21.4 |
| 279 | 3251.5 | 3522.3 | 3542.3 | 82.4 | 1789.5 | 2134.1 | 1300.8 | 1253.5 |
| 280 | 3966.4 | 3899.2 | 3833.5 | 208.1 | 1888.5 | 3042.7 | 1982.8 | 1794.8 |
| 281 | 19.5 | 547.7 | 210.9 | 28.9 | 707.3 | 438.3 | 349.4 | 158 |

| | | | | | | | | |
|---|---|---|---|---|---|---|---|---|
| 282 | 204.3 | 211.2 | 288.1 | 298.4 | 427.8 | 422.4 | 236.1 | 272.7 |
| 283 | 238.5 | 204.2 | 254 | 221.9 | 351.9 | 341.6 | 284.8 | 456.3 |
| 284 | 74.4 | 60.6 | 95.4 | 58.4 | 2258.3 | 84.1 | 82.5 | 93.7 |
| 285 | 116.8 | 93.3 | 101.5 | 95.5 | 93.9 | 98.8 | 108.7 | 120.7 |
| 286 | 151.8 | 621.9 | 136.9 | 73 | 31 | 112.7 | 64.6 | 214.1 |
| 287 | 106.5 | 73.4 | 112.1 | 121 | 113.4 | 88.9 | 92.4 | 149.7 |
| 288 | 165.7 | 147.9 | 139.4 | 188.3 | 157.7 | 159.2 | 153.4 | 210.6 |
| 289 | 361.8 | 386.8 | 175.9 | 1115.3 | 4422.6 | 4190.9 | 1131.9 | 143.3 |
| 291 | 48.4 | 122.1 | 79.7 | 80.2 | 146.2 | 305.5 | 248.8 | 57.2 |
| 292 | 105.8 | 222.9 | 15.8 | 2034.2 | 2474.9 | 1817.6 | 494.7 | 16.9 |
| 293 | 79.2 | 522.4 | 59 | 729.5 | 1441.8 | 926 | 922.7 | 76.1 |
| 295 | 337.6 | 458.1 | 827.4 | 131.1 | 2369 | 2332.1 | 1528.5 | 122.4 |
| 296 | 383.8 | 625.7 | 393.8 | 207.2 | 1441.2 | 1567.1 | 2234 | 1401.5 |
| 297 | 53 | 59 | 28.1 | 437.4 | 57.3 | 124.9 | 58.8 | 72.5 |
| 298 | 391.9 | 287.2 | 290.1 | 297.6 | 584.3 | 544.1 | 371.3 | 310.6 |
| 299 | 923.9 | 1489.6 | 612.9 | 234.9 | 1909.5 | 2964.3 | 2109.9 | 599.9 |
| 300 | 418.5 | 788.5 | 191.3 | 336 | 1170.1 | 795.1 | 1388 | 351.3 |
| 301 | 991 | 481.9 | 1125.5 | 359.4 | 219.4 | 308.6 | 234.6 | 849 |
| 302 | 23.5 | 10.4 | 20.2 | 207 | 135.3 | 153.1 | 376.5 | 9.2 |
| 303 | 1127.8 | 805.7 | 1035.8 | 76.4 | 260.7 | 316 | 336 | 830.1 |
| 304 | 168.9 | 328.4 | 141.9 | 2398 | 458.1 | 665 | 1628.3 | 347.3 |
| 305 | 615.6 | 310.9 | 1835.6 | 139.9 | 1686.4 | 577.1 | 314.2 | 187.1 |
| 306 | 157 | 151.3 | 142.3 | 1385.7 | 410.9 | 361.9 | 901.3 | 131.6 |
| 307 | 130.6 | 104.8 | 192.1 | 1073.9 | 343.2 | 232.4 | 160.4 | 141.2 |
| 308 | 2650.8 | 1762.2 | 3182.2 | 208.5 | 559.7 | 737.4 | 291.1 | 2599.7 |
| 310 | 283.4 | 464.8 | 253.6 | 85.6 | 100.7 | 156.8 | 136.2 | 1022.4 |
| 311 | 179.3 | 173.4 | 57.1 | 111.3 | 657.6 | 321.6 | 401.6 | 52.1 |
| 312 | 292.8 | 760.1 | 295.9 | 36.9 | 34.2 | 289.2 | 131.1 | 2976.1 |
| 313 | 430.3 | 1430.7 | 249 | 92.5 | 552.3 | 277.7 | 267 | 157.2 |
| 314 | 226.3 | 371.8 | 186.9 | 342.8 | 2406.4 | 1007.7 | 840.8 | 106.5 |
| 315 | 309.4 | 351.9 | 325.9 | 30.1 | 395.5 | 278.5 | 274.6 | 535.1 |
| 316 | 209.5 | 432.1 | 259.5 | 113.1 | 414.1 | 508.4 | 795.4 | 296.6 |
| 317 | 144.3 | 130.5 | 141.8 | 227.4 | 261.4 | 240 | 204 | 160.6 |
| 318 | 107.5 | 220.6 | 62.4 | 340.5 | 222.2 | 300.4 | 303.5 | 33.3 |
| 319 | 498.2 | 410.5 | 297.8 | 132.9 | 340.8 | 346.8 | 430.1 | 820.9 |
| 320 | 581.2 | 637.7 | 414.9 | 113.7 | 262.1 | 263.9 | 280.4 | 504.6 |
| 321 | 733.9 | 546.4 | 690.4 | 441.5 | 201.4 | 365.2 | 236.3 | 491.1 |
| 322 | 217.9 | 299 | 263.9 | 192.8 | 327.7 | 307.9 | 337.2 | 518.8 |
| 323 | 3187.2 | 4533.5 | 2223.1 | 758 | 4003.9 | 2801.5 | 1477.2 | 1113.1 |
| 324 | 69.4 | 506.8 | 68.6 | 123.4 | 493.6 | 420.7 | 324.7 | 47.1 |
| 325 | 450.9 | 358.4 | 218.3 | 215.6 | 509.5 | 446.4 | 622 | 185.7 |
| 326 | 2420.8 | 2944.2 | 761.2 | 377.6 | 2798.7 | 2211.2 | 3775.5 | 127.9 |
| 327 | 67.4 | 1.1 | 3.5 | 294.9 | 23.8 | 104.1 | 48.9 | 1.1 |
| 328 | 488.9 | 519.1 | 682.6 | 28.9 | 478.5 | 335.1 | 132.2 | 219.6 |
| 329 | 22.8 | 62 | 67.7 | 356.2 | 65 | 51.2 | 64.3 | 179.2 |
| 330 | 40.2 | 11.3 | 38.3 | 50 | 722.3 | 567.2 | 650.1 | 45.5 |
| 331 | 273.9 | 221.2 | 263.6 | 301.3 | 394.4 | 336.3 | 281.2 | 667.1 |
| 333 | 901.4 | 799.4 | 1778.7 | 71.1 | 251.5 | 317.3 | 197.7 | 472.4 |
| 334 | 142.6 | 391.3 | 161.2 | 132.6 | 683.2 | 297.3 | 816.3 | 123.4 |
| 335 | 92.1 | 83.6 | 139.7 | 122.3 | 635.2 | 109.4 | 93 | 650.1 |
| 336 | 415.5 | 361.2 | 472.6 | 55.1 | 215.3 | 220 | 144.3 | 242.6 |
| 337 | 248.1 | 1107.3 | 362.9 | 456.2 | 546.9 | 381.8 | 2046.3 | 137 |
| 338 | 116.6 | 210.1 | 98.7 | 249.2 | 759.2 | 1019.7 | 678.9 | 129.6 |
| 339 | 410.3 | 355.2 | 381.9 | 207.7 | 358.5 | 303.8 | 255.2 | 422.3 |
| 340 | 222.1 | 232.5 | 256.4 | 303.1 | 602.9 | 95894.7 | 123847.4 | 268.6 |
| 341 | 340.4 | 368.3 | 779 | 229.7 | 428.7 | 419.5 | 322 | 798.8 |
| 343 | 351.3 | 283.2 | 67.9 | 210.7 | 808.4 | 2720.6 | 7228.2 | 3446.2 |
| 344 | 1130.7 | 1272.9 | 909.5 | 155.6 | 531.7 | 706.2 | 595.7 | 1725.9 |

| | | | | | | | | |
|---|---|---|---|---|---|---|---|---|
| 345 | 214.4 | 187.9 | 216.5 | 326.2 | 289.7 | 349.2 | 223.2 | 308.6 |
| 347 | 780 | 4322.6 | 1880.3 | 3519.1 | 4904.6 | 2435.8 | 1042.8 | 1057 |
| 348 | 2033.1 | 8452.1 | 3722.6 | 8444.3 | 8098.7 | 3669.4 | 2324.6 | 2993.4 |
| 349 | 414.1 | 298.8 | 450 | 384.7 | 420.6 | 314.2 | 331.6 | 323.1 |
| 350 | 193.6 | 155.1 | 166.7 | 164.9 | 603.4 | 717.3 | 1008.9 | 102.4 |
| 351 | 149.8 | 87.7 | 51.7 | 177.8 | 1787.4 | 975.6 | 863.9 | 113 |
| 352 | 305.1 | 222.3 | 426.3 | 134.8 | 158.9 | 214.2 | 127.4 | 1132 |
| 353 | 228.2 | 245.9 | 178.8 | 150.1 | 197.2 | 301.1 | 428.1 | 413 |

3j

| SEQ ID NO: | 955TT | 956TT | 957TT | 959TT | 960TT | 961TT | 962TT |
|---|---|---|---|---|---|---|---|
| 354 | 87.9 | 200.9 | 8.7 | 55.2 | 27.7 | 427 | 68.1 |
| 355 | 94.6 | 90.3 | 49.7 | 131.5 | 43.1 | 452.4 | 87.9 |
| 356 | 581.7 | 511.3 | 566.1 | 708.3 | 716.3 | 1212.4 | 709.2 |
| 357 | 586.6 | 1247.1 | 595.1 | 664.9 | 783 | 891.1 | 787.2 |
| 358 | 714.8 | 1339.8 | 621 | 733.9 | 978.3 | 526.4 | 1369.9 |
| 359 | 451.3 | 315.7 | 386.8 | 424.9 | 420.5 | 309.2 | 381.1 |
| 362 | 290 | 512.7 | 459.8 | 835.1 | 394.4 | 252.8 | 419.6 |
| 360 | 221.5 | 346.6 | 132.9 | 201.9 | 228.2 | 220.9 | 348.6 |
| 361 | 182.4 | 412 | 1902 | 403.3 | 846.8 | 964.3 | 1117.4 |
| 363 | 416.2 | 327.3 | 406.2 | 502.2 | 548.9 | 506.1 | 522 |
| 1 | 675.4 | 869.8 | 676.6 | 267.5 | 452.3 | 717.4 | 496.3 |
| 2 | 1844.7 | 3591.1 | 3028.1 | 3641.6 | 3980.3 | 3803.6 | 3593.4 |
| 7 | 309.8 | 581.3 | 550.1 | 630.4 | 1040.2 | 1288.9 | 1720.8 |
| 8 | 1337.3 | 1229.5 | 1489.8 | 865.2 | 743.2 | 1080.8 | 1023.5 |
| 9 | 2938.3 | 3522 | 7292.5 | 1627.8 | 13667.8 | 4833.1 | 816.2 |
| 10 | 58.5 | 57.9 | 102.2 | 97.4 | 77.9 | 90.8 | 91 |
| 11 | 237.4 | 291.8 | 198.6 | 338.4 | 478.9 | 402.4 | 127 |
| 13 | 245.4 | 217.8 | 83.9 | 97 | 110.5 | 141.1 | 325.4 |
| 14 | 1390 | 1022.1 | 201.2 | 221.1 | 188.4 | 343.3 | 1201.7 |
| 15 | 92.3 | 394.2 | 192.7 | 775.3 | 322.5 | 337.5 | 375.9 |
| 16 | 1190 | 2000.9 | 686.2 | 1137.7 | 815.9 | 1366.2 | 1403.6 |
| 17 | 298.5 | 665.4 | 989 | 1727.8 | 1644 | 1382.7 | 773.2 |
| 19 | 176.4 | 249.5 | 161.8 | 245.1 | 209.8 | 154.9 | 170.7 |
| 20 | 1.1 | 58.3 | 1.4 | 133.8 | 1.1 | 2.2 | 1.1 |
| 21 | 354 | 1554.7 | 106.9 | 1.3 | 129.6 | 94.6 | 88.3 |
| 22 | 1772.4 | 3116.7 | 5851.7 | 6314.1 | 10192.9 | 7077.5 | 2802.8 |
| 23 | 44.4 | 809.8 | 34.3 | 489.7 | 33.2 | 163 | 1.1 |
| 24 | 86.3 | 453.2 | 147.6 | 353 | 90.7 | 121.5 | 117.5 |
| 26 | 645.4 | 2180.5 | 47.8 | 1478.1 | 169.6 | 380.5 | 74.7 |
| 27 | 278.1 | 1212.1 | 159.1 | 236.2 | 335.3 | 339.4 | 261.4 |
| 29 | 78.4 | 81.9 | 23.5 | 82.1 | 53.5 | 68.7 | 59.6 |
| 30 | 71.2 | 234.9 | 129.8 | 239.5 | 51.6 | 114 | 15.5 |
| 31 | 294.9 | 587.2 | 48.2 | 138.9 | 86 | 174.5 | 43.6 |
| 33 | 162.4 | 998.1 | 428.5 | 1117.4 | 279.9 | 251.3 | 313.6 |
| 34 | 3053 | 2498.1 | 3095.7 | 2119.5 | 3047.9 | 2373.9 | 1094.7 |
| 35 | 181.9 | 491.2 | 74 | 441.4 | 22.7 | 965.8 | 17 |
| 36 | 127.6 | 209.5 | 637.3 | 2400.3 | 139.2 | 157.6 | 1485.1 |
| 37 | 166.9 | 139.5 | 190.8 | 131.7 | 237.7 | 198.7 | 233.9 |
| 38 | 130.1 | 678 | 413.5 | 1057.4 | 365.3 | 316.8 | 662.7 |
| 40 | 257 | 450.6 | 6557.8 | 482.9 | 628.5 | 522.6 | 591.2 |
| 41 | 134.2 | 455.4 | 123.4 | 163.6 | 368.6 | 605.8 | 254.3 |
| 42 | 257.3 | 576.9 | 200.4 | 398.3 | 154.3 | 212 | 244.1 |
| 43 | 446 | 239.2 | 748.7 | 367 | 323.5 | 371.3 | 664.8 |
| 44 | 546.1 | 682.4 | 841.1 | 846.6 | 457.9 | 413.3 | 620.1 |

| | | | | | | | |
|---|---|---|---|---|---|---|---|
| 45 | 1085.8 | 897.1 | 601.8 | 484.8 | 436.6 | 816.9 | 845.3 |
| 46 | 294.3 | 214.6 | 267 | 376.1 | 303.2 | 283 | 320 |
| 47 | 96.5 | 206 | 140.7 | 192.8 | 168.4 | 247 | 213.3 |
| 48 | 18 | 313.8 | 21.5 | 378.3 | 15 | 41.1 | 1.5 |
| 49 | 303.8 | 472.1 | 236.5 | 221.8 | 366.2 | 411.7 | 363.7 |
| 50 | 78.2 | 293.2 | 108 | 497.3 | 147.7 | 127.2 | 66.9 |
| 51 | 342.3 | 124.4 | 234.4 | 45.5 | 54.4 | 36.3 | 156.3 |
| 52 | 194.3 | 158.4 | 311.6 | 182.3 | 216.4 | 212.3 | 171.6 |
| 53 | 153.6 | 347.3 | 19 | 83.6 | 112.1 | 196.9 | 251.8 |
| 54 | 1255 | 1165.2 | 316.5 | 359.8 | 277.1 | 598.1 | 1248 |
| 55 | 116.8 | 117.3 | 120.3 | 89.7 | 118.2 | 109.3 | 88.7 |
| 56 | 219.8 | 182.9 | 88.1 | 65.6 | 49.5 | 53.3 | 172.8 |
| 57 | 257.9 | 611.8 | 345 | 831.2 | 168 | 1240.7 | 159.2 |
| 58 | 242.6 | 710 | 490.1 | 2548.4 | 205.5 | 1436.5 | 82.1 |
| 59 | 1047.2 | 2248.5 | 1125 | 813.5 | 918.1 | 1999 | 1120.9 |
| 60 | 97 | 300.2 | 154 | 85.5 | 160.5 | 117.3 | 185.6 |
| 61 | 90.4 | 103.2 | 161.9 | 145.1 | 203.2 | 134.9 | 108.2 |
| 62 | 300.7 | 1868.2 | 953.7 | 574.1 | 642.8 | 1633.8 | 139 |
| 63 | 297 | 536.7 | 193.6 | 228.4 | 311.9 | 261.2 | 255.9 |
| 64 | 109.8 | 100.1 | 170.4 | 227.4 | 176.6 | 190.7 | 115.1 |
| 65 | 87.5 | 158.6 | 150.9 | 81.8 | 71.6 | 67.2 | 102 |
| 66 | 1355.7 | 951.2 | 564.5 | 797.6 | 309.4 | 509.5 | 1530.7 |
| 67 | 34223.4 | 31838 | 24742.4 | 36682.2 | 36457.8 | 32604.1 | 43684.2 |
| 68 | 3348.2 | 1692.5 | 1286.8 | 3946 | 5683.4 | 2624.1 | 1368.7 |
| 69 | 7947.5 | 4162 | 3970 | 11781.2 | 10279.8 | 7055 | 5076.1 |
| 70 | 4104.3 | 2568.6 | 4964 | 2477.4 | 881.6 | 933.2 | 2792.2 |
| 71 | 619.9 | 681.8 | 113.5 | 372.3 | 137.1 | 72 | 319.9 |
| 72 | 229.3 | 609.4 | 1269.4 | 639.7 | 229.4 | 694.7 | 159.4 |
| 73 | 260.4 | 365.9 | 207.3 | 238.1 | 275.9 | 264.9 | 416.7 |
| 75 | 149.7 | 302.2 | 171.8 | 538.4 | 453.7 | 249.2 | 115.4 |
| 76 | 2162.3 | 1321.7 | 189 | 2328.1 | 12.2 | 54.4 | 1010.4 |
| 77 | 5244.7 | 2705.9 | 4944.6 | 4760.4 | 4771.9 | 3213.4 | 3514.8 |
| 78 | 310.5 | 589.3 | 1329.2 | 1771 | 866.2 | 805.1 | 952.5 |
| 79 | 231.8 | 135.6 | 383 | 134.3 | 114.6 | 119.6 | 116.4 |
| 80 | 667.1 | 355.6 | 404.3 | 450.5 | 85 | 239.4 | 268.7 |
| 81 | 1253.3 | 1566.6 | 84.2 | 100.9 | 194.3 | 384.9 | 263.5 |
| 82 | 333.1 | 228.4 | 350.7 | 215.3 | 247.9 | 520.6 | 252.5 |
| 84 | 147 | 325.5 | 277 | 141.8 | 114.9 | 106 | 153.2 |
| 85 | 163.8 | 102.1 | 111.6 | 119.6 | 149.3 | 210.2 | 180.5 |
| 86 | 193.8 | 55.9 | 95.3 | 298.1 | 298.3 | 70.2 | 71.1 |
| 87 | 156.9 | 158.6 | 353.7 | 164.2 | 293.6 | 482.8 | 160.9 |
| 88 | 3325 | 4358.2 | 4541.7 | 6014.1 | 7677.1 | 10815.9 | 5006 |
| 89 | 140.5 | 120.8 | 185.6 | 259 | 178.4 | 199.4 | 228.6 |
| 90 | 68.4 | 170.5 | 91.9 | 205.9 | 87.5 | 144.7 | 114 |
| 91 | 249.8 | 383.1 | 110.8 | 1269.1 | 70.9 | 1429.6 | 73.4 |
| 92 | 4953.4 | 6959.3 | 1774.3 | 2917.7 | 2117.8 | 7949.3 | 7560.6 |
| 93 | 201 | 209.6 | 400.5 | 170.5 | 283.1 | 293.5 | 163.4 |
| 94 | 260.4 | 3158.7 | 52.2 | 867.5 | 660.8 | 606.6 | 836.7 |
| 95 | 593.4 | 622.7 | 577.7 | 104.7 | 388.1 | 646.7 | 1203.2 |
| 96 | 165.4 | 233.3 | 200.5 | 123.7 | 184.7 | 171 | 372 |
| 97 | 273.3 | 336.6 | 287.9 | 296.3 | 132.3 | 317 | 370.1 |
| 98 | 70.8 | 41.8 | 25.4 | 16.5 | 25.6 | 32.7 | 30 |
| 99 | 164.8 | 189.4 | 116 | 167.2 | 280.8 | 245.9 | 257.1 |
| 100 | 610.8 | 12721.1 | 16769.4 | 9378.2 | 4271.4 | 15550.1 | 1798.6 |
| 101 | 232.4 | 238.6 | 434.5 | 383.4 | 434.1 | 504.6 | 729.3 |
| 102 | 147.5 | 132.7 | 112.2 | 180.7 | 209.4 | 215.6 | 169.1 |
| 103 | 309.3 | 255.1 | 195.4 | 418.3 | 293.4 | 404.3 | 381.3 |
| 104 | 631.5 | 573.5 | 522.3 | 415.2 | 370.9 | 830.3 | 589 |

| | | | | | | | |
|---|---|---|---|---|---|---|---|
| 105 | 655.3 | 488.2 | 398.8 | 564 | 421.8 | 540.9 | 516.4 |
| 106 | 562.5 | 491 | 440.4 | 361 | 470.2 | 736.1 | 469 |
| 107 | 170.3 | 157.5 | 207.8 | 405.5 | 147.6 | 558.5 | 118.6 |
| 108 | 260.5 | 430.5 | 385.5 | 555.3 | 419 | 273.4 | 323.9 |
| 109 | 6189.1 | 2697.1 | 42.6 | 65.5 | 78.8 | 577.2 | 6878.3 |
| 110 | 10703.6 | 3965.4 | 1.2 | 28.1 | 19.7 | 1384.5 | 13039 |
| 111 | 1743.4 | 1684.3 | 944.4 | 1390.4 | 2754.7 | 1147.9 | 3105.8 |
| 112 | 823.1 | 805.8 | 41.7 | 109.8 | 237.7 | 59.8 | 620.1 |
| 113 | 246 | 445 | 478 | 246 | 90.9 | 253.2 | 214.1 |
| 114 | 239.9 | 397.1 | 452.5 | 688.8 | 1076.1 | 566 | 830.3 |
| 115 | 441.3 | 600.8 | 435.6 | 627.2 | 408 | 348.9 | 523.2 |
| 116 | 223.5 | 218.9 | 200.8 | 251.8 | 236.2 | 182.6 | 216.1 |
| 117 | 413 | 582.6 | 4938.9 | 5309.2 | 2714.3 | 3839.8 | 902.6 |
| 118 | 203.1 | 268.2 | 141 | 148.8 | 118.4 | 34.2 | 105.3 |
| 119 | 45.1 | 322.8 | 110.9 | 119.5 | 75.9 | 246.3 | 233 |
| 120 | 63.4 | 106.6 | 118.6 | 184.1 | 122.3 | 82.1 | 114.7 |
| 121 | 461.9 | 1324 | 1289.4 | 211.1 | 422.1 | 737.7 | 749.7 |
| 122 | 243.1 | 213.9 | 52.5 | 140.8 | 84.5 | 274.4 | 144.3 |
| 123 | 189.9 | 195.8 | 480.9 | 135.7 | 146.6 | 163.4 | 216.1 |
| 124 | 130.1 | 117 | 258.6 | 193.4 | 182.9 | 170.1 | 178 |
| 125 | 984.3 | 573.5 | 539.9 | 1065.4 | 967.5 | 1025.7 | 1172 |
| 126 | 1322.2 | 965.1 | 1360 | 738.2 | 631.3 | 1011.4 | 682.8 |
| 127 | 131.7 | 96.4 | 132 | 117.3 | 113.4 | 116.7 | 127.7 |
| 128 | 194.1 | 182.2 | 236.4 | 276.3 | 285.9 | 310.1 | 367.6 |
| 129 | 130.1 | 229.7 | 252.6 | 224.8 | 171.9 | 522.6 | 200.2 |
| 130 | 185.4 | 150.7 | 162.7 | 99 | 93.7 | 89.6 | 207.8 |
| 131 | 95.8 | 152.7 | 148.3 | 47.3 | 74.7 | 96.5 | 94.2 |
| 132 | 163.2 | 168.1 | 192.6 | 193.3 | 258.1 | 217.7 | 272.4 |
| 133 | 204.5 | 249.2 | 84.8 | 160.3 | 70.8 | 65.2 | 75.8 |
| 134 | 287.4 | 159.6 | 98.4 | 197.6 | 118.4 | 129.9 | 184.5 |
| 135 | 373.8 | 551.6 | 416.6 | 392.1 | 459.3 | 328.2 | 406.9 |
| 138 | 137.1 | 115.6 | 126.5 | 195 | 133.1 | 149.9 | 117.4 |
| 139 | 116.4 | 142.3 | 79.7 | 71.6 | 80.2 | 87.7 | 77.6 |
| 140 | 74.5 | 3607.6 | 2148.2 | 1275.7 | 3106.6 | 2235.3 | 1653.3 |
| 141 | 1566.1 | 2429.4 | 761.5 | 1182 | 2077.2 | 1681.8 | 1605.6 |
| 144 | 12425.9 | 7539.3 | 395.2 | 417 | 450.5 | 512.8 | 7238.7 |
| 145 | 160.5 | 137.2 | 206.9 | 220 | 255.1 | 198.3 | 239.2 |
| 146 | 182.1 | 147.4 | 169.7 | 202.3 | 262.8 | 225.5 | 211.9 |
| 147 | 28.6 | 43.9 | 91 | 66.4 | 26.5 | 59.8 | 20 |
| 148 | 205.3 | 553.1 | 180.2 | 198 | 270.3 | 668.6 | 306.4 |
| 149 | 325 | 326.5 | 236.6 | 362.1 | 407.1 | 765.1 | 342.1 |
| 150 | 197.1 | 234.6 | 172.3 | 171.9 | 270.8 | 246.9 | 159.2 |
| 151 | 123.1 | 264.2 | 95.7 | 127.8 | 158.8 | 148.1 | 257.2 |
| 153 | 103.8 | 118.2 | 112.8 | 119.6 | 110.6 | 115.4 | 113.9 |
| 154 | 304.4 | 1676.6 | 2431.9 | 408.8 | 152.5 | 2641.1 | 142.4 |
| 155 | 498.7 | 158.7 | 136.4 | 138 | 136.6 | 144.9 | 139.3 |
| 156 | 95.4 | 89.8 | 63.6 | 51.8 | 74.1 | 120.8 | 65.3 |
| 157 | 178 | 115.2 | 155.5 | 132.3 | 138.2 | 159.9 | 181.3 |
| 158 | 283.4 | 1174.2 | 19.8 | 16.2 | 1.2 | 13.6 | 578.5 |
| 159 | 1776.8 | 2641.6 | 1398.3 | 1601.9 | 3134.9 | 3315.7 | 3396 |
| 161 | 9817.8 | 2623.3 | 177.1 | 129.7 | 6.7 | 152.9 | 1887.4 |
| 162 | 45.7 | 101.6 | 27.6 | 150.8 | 40.3 | 76.2 | 46.9 |
| 164 | 78.1 | 707 | 463.5 | 110.3 | 193.8 | 333.7 | 3770.9 |
| 166 | 6384 | 2992.4 | 4919.5 | 5671.4 | 6028.1 | 6429.2 | 3769.1 |
| 167 | 1191.3 | 1999.3 | 1864 | 686.4 | 1834.7 | 2207.6 | 2362.6 |
| 168 | 1167.3 | 843.3 | 2714.3 | 1862.4 | 1004 | 922.5 | 1487.6 |
| 169 | 3437.7 | 4365.5 | 1880.1 | 3159.8 | 1921.4 | 5836 | 1307.9 |
| 170 | 1488.1 | 2441.6 | 1019.6 | 1307.1 | 1150.4 | 2496.9 | 1953.8 |

| | | | | | | | |
|---|---|---|---|---|---|---|---|
| 171 | 1.2 | 722.2 | 1.2 | 760.4 | 1.2 | 725.9 | 1.2 |
| 172 | 590.7 | 573.3 | 719.5 | 631.6 | 816.5 | 666.4 | 904.9 |
| 173 | 408.8 | 355.1 | 171 | 213.4 | 406.9 | 481.6 | 564.2 |
| 176 | 1175.5 | 1461.1 | 175.7 | 8.4 | 704.9 | 850.3 | 2537.2 |
| 177 | 536.9 | 672.1 | 597.5 | 376.7 | 544 | 817.5 | 635.8 |
| 178 | 385.8 | 730.5 | 91.3 | 209.5 | 184.7 | 233 | 1073.8 |
| 179 | 74.1 | 84.7 | 60.3 | 70.3 | 71.4 | 127.1 | 145.7 |
| 180 | 156.8 | 498.8 | 119.5 | 132.1 | 72.9 | 109.4 | 609.9 |
| 181 | 8682 | 7928.2 | 10784.9 | 10442 | 14140.4 | 12487.9 | 12014.6 |
| 182 | 323.1 | 400.6 | 375.2 | 349.8 | 535.4 | 405.4 | 334.3 |
| 183 | 128.7 | 461.7 | 1.2 | 388.5 | 15.5 | 53.1 | 1.3 |
| 184 | 279.4 | 565.1 | 227.8 | 327.3 | 211.1 | 272.9 | 2642.9 |
| 185 | 1142.8 | 848.2 | 1463.7 | 1098.5 | 1043 | 1044.1 | 911.4 |
| 186 | 444.5 | 436.7 | 360.3 | 324.6 | 419 | 403 | 314.3 |
| 187 | 214.8 | 123.4 | 366.1 | 170.7 | 79.5 | 90.4 | 76.6 |
| 188 | 94.7 | 141.3 | 105.2 | 357.2 | 157 | 110 | 170.5 |
| 189 | 49.2 | 74.3 | 80 | 146.6 | 60.9 | 66.5 | 88 |
| 190 | 267.8 | 422 | 71.2 | 95.9 | 85.5 | 102.1 | 152.5 |
| 191 | 825.2 | 845.3 | 1874.8 | 739.8 | 734.8 | 1020.6 | 520.6 |
| 192 | 67.8 | 308.7 | 77.4 | 94.4 | 80.7 | 77.4 | 79.8 |
| 193 | 695 | 1573.5 | 2485.3 | 1849.3 | 2885.4 | 3460.8 | 1615.5 |
| 194 | 173.6 | 299.7 | 106.6 | 134.3 | 150.7 | 122.6 | 175.9 |
| 195 | 248.2 | 343.9 | 371.7 | 347.9 | 320.2 | 194.4 | 297.7 |
| 196 | 7207.4 | 7285.3 | 5142.4 | 2474.2 | 6626.6 | 8775.3 | 11352.4 |
| 197 | 50.6 | 93.6 | 113.8 | 142.3 | 96.4 | 86.5 | 58 |
| 198 | 56.8 | 44.6 | 36.9 | 51.1 | 66.8 | 47.9 | 128.9 |
| 199 | 135.2 | 104.6 | 57.8 | 204.7 | 35.2 | 599.8 | 103.1 |
| 200 | 110.8 | 310.7 | 104.4 | 327.5 | 121.9 | 121.6 | 147.5 |
| 201 | 125.9 | 200.1 | 46.9 | 69.2 | 25.3 | 505.3 | 54.7 |
| 203 | 163.6 | 319 | 130.2 | 85 | 252 | 312.1 | 621.1 |
| 204 | 826.2 | 879.7 | 567.6 | 726.8 | 704.1 | 434 | 980.1 |
| 205 | 4963.1 | 7695 | 2785.7 | 6249.9 | 6069.1 | 6172.2 | 6469.8 |
| 206 | 384 | 1284 | 196.8 | 1397.4 | 323.3 | 282.2 | 224.9 |
| 207 | 1180.7 | 1623.7 | 402.1 | 2190.2 | 453.5 | 1652.9 | 1647 |
| 208 | 495.8 | 653.7 | 150.2 | 227.7 | 257 | 340.5 | 253.6 |
| 209 | 1608.4 | 2981.5 | 2844.2 | 445.8 | 5286.9 | 4175.6 | 6653.1 |
| 211 | 7731.4 | 15392.6 | 3699.2 | 33617.1 | 23929.6 | 28201.9 | 37299.8 |
| 212 | 911.5 | 692 | 1485 | 440.7 | 207.7 | 396.6 | 971.4 |
| 213 | 58.3 | 77.2 | 150.2 | 91.5 | 119.6 | 83.2 | 84.4 |
| 215 | 1364.9 | 1835.2 | 477.2 | 1858.8 | 394.4 | 2522.1 | 2910.2 |
| 216 | 540.7 | 636.6 | 665 | 441.7 | 610.8 | 635.7 | 494.3 |
| 217 | 196.1 | 494.8 | 258 | 300.8 | 260 | 97.9 | 424.1 |
| 218 | 359.4 | 357 | 308.6 | 572.5 | 327.9 | 346.3 | 318.6 |
| 220 | 87.6 | 66.5 | 71.4 | 89.9 | 106 | 86.7 | 88.5 |
| 221 | 195.8 | 787.9 | 397 | 518.1 | 651.9 | 372.5 | 497.7 |
| 223 | 248.3 | 279.5 | 456 | 247.2 | 226.7 | 308.9 | 202.1 |
| 227 | 326.2 | 1074.9 | 579.8 | 1616.2 | 517.9 | 516.4 | 447.3 |
| 228 | 377.5 | 502.7 | 583.4 | 486.6 | 764.6 | 521.7 | 871.2 |
| 229 | 311.2 | 113.4 | 1212.9 | 377.4 | 152.7 | 93.4 | 149 |
| 230 | 417.2 | 297.5 | 120.9 | 131.8 | 135.8 | 242.5 | 285.4 |
| 231 | 108.5 | 189.8 | 88.2 | 67.4 | 140.1 | 160.7 | 309.4 |
| 232 | 465.5 | 1573.3 | 41.4 | 973.5 | 118.3 | 273.3 | 78.3 |
| 233 | 728.9 | 3634.1 | 7030.2 | 1396.1 | 3819.1 | 5175.1 | 2235.4 |
| 235 | 37.3 | 474.7 | 88.2 | 526 | 120.4 | 662.1 | 42.3 |
| 236 | 199.7 | 143.3 | 108.3 | 196.9 | 220 | 171.3 | 142.5 |
| 237 | 1614.8 | 1725.3 | 2189.4 | 1342.6 | 2031.3 | 1672.9 | 1475 |
| 238 | 823.6 | 1424 | 1041.5 | 1173.3 | 691.2 | 1142.2 | 1169.5 |
| 239 | 56.1 | 641.7 | 183 | 140.5 | 95 | 88 | 683.1 |

| | | | | | | | |
|---|---|---|---|---|---|---|---|
| 240 | 98.3 | 853.8 | 155.4 | 330.5 | 155.1 | 106.4 | 574.8 |
| 241 | 84.4 | 1914.8 | 289.8 | 519.5 | 207.1 | 95 | 1367 |
| 242 | 2194.8 | 989.6 | 2107.8 | 1991.2 | 1042.5 | 1474.6 | 688.1 |
| 243 | 89.9 | 263.3 | 247.9 | 268.6 | 679.7 | 287.2 | 89 |
| 244 | 209.3 | 288.3 | 89.9 | 209.1 | 146.5 | 131 | 131 |
| 246 | 1348 | 810.8 | 270.7 | 434.5 | 85.9 | 160.3 | 487.6 |
| 247 | 113.4 | 92.8 | 68 | 145.3 | 125.8 | 139.4 | 150.2 |
| 248 | 503.9 | 571.2 | 120.3 | 199.5 | 153.6 | 226.1 | 529.7 |
| 249 | 650 | 1958.3 | 945.4 | 442.8 | 746.9 | 674.2 | 1861.5 |
| 251 | 149.3 | 159.2 | 98.7 | 67 | 54.5 | 98.7 | 133.7 |
| 253 | 458.5 | 369.7 | 392.2 | 512.7 | 189.7 | 171.8 | 255.1 |
| 254 | 759.1 | 1048.2 | 56.3 | 201.5 | 144.1 | 322.5 | 1876.1 |
| 255 | 395 | 847.1 | 92.8 | 241.2 | 190.6 | 204 | 937.8 |
| 256 | 497.3 | 504.3 | 202.9 | 1243.9 | 138.2 | 1112.8 | 153.2 |
| 257 | 96.8 | 170.3 | 61.6 | 110.7 | 240.3 | 224.1 | 83.2 |
| 258 | 80.1 | 318.2 | 1.1 | 4 | 1.2 | 194.6 | 1.3 |
| 259 | 31.3 | 422.4 | 35.1 | 291.4 | 170 | 194.9 | 6.1 |
| 260 | 144.2 | 118.9 | 152.1 | 196.1 | 181.1 | 162.3 | 192.9 |
| 261 | 72 | 118.5 | 599.4 | 788.2 | 759.5 | 233.2 | 707.9 |
| 263 | 414.9 | 536.2 | 500.4 | 605.6 | 316.7 | 492.7 | 460.5 |
| 263 | 550.2 | 391.2 | 170.3 | 206.7 | 218 | 261.4 | 299 |
| 265 | 334.6 | 245.7 | 405.7 | 534.5 | 619.5 | 1119 | 574.8 |
| 266 | 546.7 | 433.1 | 317.8 | 513.8 | 421.9 | 266.6 | 397.7 |
| 256 | 69.3 | 168.9 | 137.2 | 94.2 | 115.1 | 101 | 97.3 |
| 268 | 570.4 | 855.7 | 334.9 | 287.8 | 801.2 | 1200.2 | 1032.6 |
| 269 | 17.8 | 25.5 | 51.2 | 32.6 | 33.8 | 37.9 | 56.8 |
| 270 | 313.9 | 169.7 | 378.4 | 245.5 | 183.8 | 220.5 | 267 |
| 271 | 197.8 | 175.4 | 205.9 | 317.3 | 297.9 | 247.6 | 263.9 |
| 272 | 537.8 | 303.4 | 368 | 416.8 | 596.6 | 461.6 | 589.2 |
| 273 | 2896.5 | 2422.4 | 2113.9 | 4788.3 | 2936.5 | 2726.7 | 6835.9 |
| 274 | 736 | 340.7 | 226.1 | 863.7 | 465 | 215.6 | 180.7 |
| 275 | 312 | 220.3 | 428 | 292.8 | 254 | 301.5 | 300 |
| 276 | 290.2 | 1528.1 | 727.2 | 2093.9 | 539.5 | 692.9 | 622.8 |
| 277 | 171.5 | 177.3 | 190.4 | 200.2 | 228.4 | 201.6 | 245.6 |
| 278 | 57 | 615.1 | 90.6 | 495.4 | 201.9 | 350.4 | 20.5 |
| 279 | 2553.9 | 3673.6 | 1781.1 | 3550.4 | 3051.6 | 3161.9 | 3164.5 |
| 280 | 4112.4 | 4496.7 | 2693.5 | 6671.4 | 4029.9 | 3715.1 | 3402.8 |
| 281 | 420.9 | 478.9 | 381.7 | 99.3 | 24.7 | 76.5 | 829.5 |
| 282 | 284.1 | 237.5 | 220.4 | 467.3 | 243.1 | 183.4 | 280.3 |
| 283 | 320.7 | 247.3 | 262.2 | 267.1 | 257.1 | 287.9 | 255.5 |
| 284 | 86.1 | 60.6 | 66.9 | 82.5 | 68 | 91 | 102.6 |
| 285 | 74.5 | 84.5 | 78.8 | 98.8 | 132.4 | 97.7 | 89.8 |
| 286 | 140.3 | 125.8 | 856.5 | 479.2 | 1253 | 1123.8 | 337.3 |
| 287 | 82.7 | 68.3 | 104.9 | 131 | 118.4 | 100.2 | 124.1 |
| 288 | 112.6 | 131.8 | 109.3 | 132.3 | 203.1 | 166.3 | 157.2 |
| 289 | 329.1 | 523 | 74.6 | 1912.1 | 5 | 1194.8 | 79.2 |
| 291 | 72.9 | 135.9 | 147.6 | 116.3 | 111.7 | 106 | 91.2 |
| 292 | 61.3 | 181.2 | 49.9 | 155.8 | 43.9 | 982.9 | 77.4 |
| 293 | 210.2 | 267.7 | 100.5 | 70.6 | 87.4 | 91.2 | 418.7 |
| 295 | 880.3 | 560.6 | 520.9 | 266.4 | 534.9 | 307.3 | 261.4 |
| 296 | 1067.2 | 1234.6 | 688.7 | 782.9 | 718.7 | 604.5 | 724.8 |
| 297 | 47.9 | 64.8 | 51.5 | 87.2 | 38.6 | 69.1 | 58.3 |
| 298 | 551.5 | 344.5 | 386.7 | 324.7 | 241.8 | 269 | 394.7 |
| 299 | 1468.8 | 1771.8 | 787.5 | 1048 | 935.5 | 572.9 | 948.9 |
| 300 | 817.3 | 680.9 | 461.3 | 248.2 | 137.3 | 294.2 | 748.4 |
| 301 | 608.4 | 1624.7 | 1490.7 | 3382.6 | 4444 | 1930.7 | 1769 |
| 302 | 10.4 | 26.6 | 30.4 | 33.2 | 26.3 | 35.7 | 24.2 |
| 303 | 710.1 | 705.1 | 1706.6 | 859.3 | 577.5 | 788.3 | 425.1 |

| | | | | | | | |
|---|---|---|---|---|---|---|---|
| 304 | 95.7 | 466.8 | 190.4 | 187.2 | 124.8 | 202.8 | 143.5 |
| 305 | 157.1 | 529.4 | 493.4 | 795.6 | 163.1 | 69.8 | 249 |
| 306 | 131.8 | 108.5 | 180.6 | 143.8 | 154.4 | 173 | 206 |
| 307 | 191.4 | 207.2 | 84.5 | 181.7 | 133.4 | 205.1 | 178.9 |
| 308 | 2703.4 | 1485.8 | 1302 | 2184.2 | 756.3 | 1330.1 | 1074.7 |
| 310 | 549.2 | 417.5 | 612 | 811.3 | 723.1 | 576 | 409.8 |
| 311 | 145 | 132 | 126 | 159.1 | 101.9 | 148.1 | 113.9 |
| 312 | 1216.9 | 574.9 | 992.1 | 892.8 | 612.7 | 695.2 | 1340.7 |
| 313 | 307.7 | 190.1 | 158.9 | 201.8 | 115.6 | 112.7 | 1437.5 |
| 314 | 416.9 | 725.7 | 134.8 | 157 | 168.8 | 428.1 | 448.6 |
| 315 | 192.5 | 412.2 | 496 | 467.2 | 366.2 | 506.8 | 341.4 |
| 316 | 444 | 470.1 | 95.3 | 286 | 281.8 | 131.4 | 291.2 |
| 317 | 157.9 | 142 | 150.3 | 197.9 | 191.9 | 177.2 | 201.1 |
| 318 | 140.5 | 334.7 | 56.9 | 29.1 | 73.1 | 87 | 78.1 |
| 319 | 706.9 | 280.3 | 127.7 | 267.6 | 70.7 | 164.5 | 199.4 |
| 320 | 535.8 | 590.3 | 887.9 | 568 | 592.8 | 742.2 | 577.9 |
| 321 | 340.3 | 326.5 | 372 | 793.8 | 460.1 | 414.9 | 375.2 |
| 322 | 352.2 | 258.3 | 185.4 | 237.2 | 248.6 | 152.6 | 280.9 |
| 323 | 2455.7 | 4246.4 | 3485.2 | 2786.2 | 4469.1 | 2754.2 | 4942.2 |
| 324 | 348.9 | 411 | 169.2 | 70 | 73 | 87.9 | 460 |
| 325 | 449.5 | 519.2 | 168 | 216.7 | 282.5 | 299.4 | 469.4 |
| 326 | 1603.6 | 3167 | 3571.4 | 404.9 | 4260.9 | 3099.9 | 1692.6 |
| 327 | 26.8 | 44 | 10.3 | 12.2 | 25.7 | 20.6 | 8.2 |
| 328 | 277.9 | 499.5 | 114.4 | 194 | 624.6 | 445.4 | 617.5 |
| 329 | 63.1 | 51.9 | 64 | 54.1 | 56.3 | 67.2 | 81.9 |
| 330 | 26.8 | 398.3 | 51.1 | 38.3 | 53.4 | 44.1 | 28.4 |
| 331 | 353.6 | 211.8 | 246.9 | 326.3 | 313.7 | 256.2 | 346.2 |
| 333 | 628.2 | 817.1 | 534.6 | 764.7 | 963.2 | 1039.8 | 993.5 |
| 334 | 129 | 131.6 | 121.3 | 127.9 | 171.5 | 135.9 | 210.2 |
| 335 | 61.5 | 83.3 | 276.3 | 550.9 | 108.1 | 127.9 | 116.4 |
| 336 | 877.3 | 835 | 262.7 | 397.8 | 457.2 | 330 | 598.7 |
| 337 | 119 | 244.5 | 349.6 | 426.7 | 114.8 | 364.1 | 225.7 |
| 338 | 213.7 | 307.5 | 152.1 | 190 | 160 | 264.1 | 213.2 |
| 339 | 337.9 | 298.3 | 253.4 | 278.6 | 421.1 | 490.3 | 476.5 |
| 340 | 161.4 | 161 | 248.4 | 316.2 | 277.8 | 273.6 | 290.1 |
| 341 | 575.9 | 645.8 | 726.6 | 782.5 | 1057.6 | 767.6 | 465.1 |
| 343 | 1203 | 450.3 | 740.9 | 103.8 | 137.1 | 178 | 105 |
| 344 | 1275 | 1111.4 | 1819.2 | 1393.5 | 1260.3 | 1521.1 | 1289.7 |
| 345 | 164.6 | 211.9 | 210.1 | 208.7 | 288.4 | 221.3 | 290.7 |
| 347 | 2603 | 3049.5 | 1135.9 | 1212.8 | 2982.6 | 3471.6 | 2440.3 |
| 348 | 4636 | 4968.1 | 2088.3 | 2517.8 | 4998.7 | 6143.9 | 5642.9 |
| 349 | 231.2 | 321.5 | 329.2 | 238.9 | 199 | 275.7 | 273.9 |
| 350 | 306.8 | 357 | 165.3 | 139.9 | 214.3 | 201.7 | 315.7 |
| 351 | 661.6 | 248.9 | 70.2 | 75.7 | 64.2 | 140 | 76.9 |
| 352 | 228.8 | 337.7 | 313.8 | 812.7 | 340.3 | 238.1 | 322 |
| 353 | 268 | 118.5 | 158.9 | 211.1 | 171 | 284.3 | 124.5 |

3k

| SEQ ID NO: | 967TT | 968TT | 969TT | 970TT | 982TT | 983TT |
|---|---|---|---|---|---|---|
| 354 | 23.2 | 55.9 | 169.3 | 22.6 | 1.2 | 136.2 |
| 355 | 28.5 | 50.2 | 1052.4 | 81.6 | 25.9 | 96.4 |
| 356 | 625.8 | 410.2 | 2722.4 | 190.1 | 528.5 | 449.5 |
| 357 | 301.6 | 385.5 | 990 | 299.7 | 781 | 321.6 |
| 358 | 855.6 | 468.3 | 570.2 | 419.9 | 530.5 | 768 |
| 359 | 336.5 | 327.4 | 464.6 | 420.5 | 326.9 | 556.2 |

| | | | | | | |
|---|---|---|---|---|---|---|
| 362 | 247.8 | 386.1 | 248.5 | 248 | 355.4 | 570.5 |
| 360 | 219.2 | 183.9 | 501.1 | 198.9 | 170.3 | 376.9 |
| 361 | 248.2 | 544 | 730.9 | 108.3 | 746.6 | 231.5 |
| 363 | 716.9 | 541.2 | 438.2 | 3964.4 | 373.4 | 824.2 |
| 1 | 469.7 | 405.7 | 1234.4 | 133.8 | 970 | 527.9 |
| 2 | 135.1 | 2759.1 | 3994.4 | 239.3 | 3662.3 | 264.1 |
| 7 | 1.3 | 402.8 | 129 | 94.8 | 1127.5 | 460.2 |
| 8 | 1162.1 | 811.3 | 967.4 | 854.4 | 644.1 | 915.3 |
| 9 | 2702.9 | 1018.8 | 4839.3 | 217.3 | 1823.6 | 997.2 |
| 10 | 190.7 | 504.6 | 237.6 | 195.3 | 126.1 | 271.3 |
| 11 | 1104.8 | 1435.3 | 2566.4 | 297.7 | 275.4 | 422.7 |
| 13 | 198.3 | 136 | 54.3 | 73.1 | 68.5 | 266.9 |
| 14 | 545.3 | 572.6 | 232.1 | 73.2 | 69.4 | 676 |
| 15 | 82.9 | 465.9 | 409.7 | 155.7 | 537.1 | 134.5 |
| 16 | 981.5 | 987.6 | 1149.6 | 167.1 | 888.7 | 1443.1 |
| 17 | 178.5 | 639 | 1677.1 | 125.2 | 2064.5 | 437.9 |
| 19 | 289.1 | 542.1 | 212.7 | 359 | 106.4 | 291.4 |
| 20 | 1.1 | 118.7 | 73.8 | 1.2 | 19.5 | 6.1 |
| 21 | 928.1 | 1108.3 | 1144.3 | 77 | 10.6 | 499.2 |
| 22 | 1063.2 | 3119.6 | 4358 | 872.9 | 8269.8 | 577.8 |
| 23 | 1.2 | 379.9 | 35.2 | 1.2 | 1.1 | 61.4 |
| 24 | 73.7 | 59 | 226.7 | 57.2 | 297.5 | 65.5 |
| 26 | 117.4 | 453.2 | 143.8 | 58.4 | 89 | 440 |
| 27 | 1699.9 | 830.8 | 811.4 | 403.5 | 511.2 | 509.3 |
| 29 | 594.6 | 288 | 325.3 | 378 | 112.4 | 982.9 |
| 30 | 86.9 | 82.7 | 124.5 | 10.9 | 45 | 97 |
| 31 | 1573.7 | 38.8 | 497.9 | 471.5 | 34.3 | 72.6 |
| 33 | 441.8 | 872.9 | 1130.8 | 75.9 | 411.7 | 352 |
| 34 | 1018.8 | 2164.1 | 2701.3 | 2309.9 | 2634.9 | 4374.3 |
| 35 | 99.6 | 70.4 | 393.2 | 80.2 | 13.2 | 133.7 |
| 36 | 167.8 | 137.3 | 152.5 | 203.8 | 1201.5 | 111.3 |
| 37 | 329.9 | 181.7 | 190.5 | 777.4 | 172.2 | 464.8 |
| 38 | 240.2 | 1359.2 | 1110.5 | 89.7 | 1069.5 | 51.1 |
| 40 | 666.4 | 451.5 | 462.6 | 593.8 | 349.6 | 703.8 |
| 41 | 180.9 | 1696.6 | 333.6 | 178.9 | 730 | 231.9 |
| 42 | 176.5 | 403.8 | 214 | 306.2 | 308.2 | 287.7 |
| 43 | 821.9 | 483.9 | 321 | 1489 | 232.8 | 1352.9 |
| 44 | 467.5 | 380 | 471.5 | 555.7 | 360.3 | 455 |
| 45 | 770.8 | 434.4 | 558.1 | 1817 | 1071 | 1373.8 |
| 46 | 411.4 | 330.8 | 255 | 409.7 | 273.6 | 401.2 |
| 47 | 166.6 | 296.7 | 247.1 | 277.4 | 321.6 | 130.9 |
| 48 | 1.2 | 116.9 | 1.2 | 69 | 28.9 | 111 |
| 49 | 627.1 | 965.5 | 130.2 | 225.8 | 251.8 | 772.3 |
| 50 | 142.5 | 216.3 | 192.3 | 15.1 | 77.1 | 161.5 |
| 51 | 57.4 | 149.4 | 38.2 | 2101.5 | 1490.9 | 1190.6 |
| 52 | 308.7 | 413.6 | 298.7 | 307.9 | 217.7 | 234.6 |
| 53 | 84.9 | 100.2 | 159.9 | 93.3 | 102 | 217.6 |
| 54 | 870.3 | 607.4 | 345.8 | 269.3 | 669.6 | 529 |
| 55 | 161.6 | 94.8 | 98.6 | 115.2 | 110.3 | 144.3 |
| 56 | 106.7 | 36.5 | 54 | 42.1 | 150.1 | 33.6 |
| 57 | 343.8 | 1228.1 | 103 | 770.3 | 171.3 | 966.7 |
| 58 | 392.8 | 1366.3 | 238.3 | 1256 | 263.5 | 1149.6 |
| 59 | 605.2 | 1677.6 | 2094 | 285.7 | 1088 | 1273.8 |
| 60 | 202.7 | 198 | 164.7 | 124.8 | 168.1 | 155.5 |
| 61 | 542.2 | 245.6 | 124.9 | 288 | 103.9 | 201.2 |
| 62 | 1650.5 | 7509.1 | 3612.1 | 1170.1 | 318.8 | 9459.8 |
| 63 | 259.6 | 202.4 | 365.9 | 187.3 | 388.3 | 316.8 |
| 64 | 540.3 | 166.5 | 122.8 | 1276.6 | 123.7 | 1611 |

| | | | | | | |
|---|---|---|---|---|---|---|
| 65 | 88.4 | 78.4 | 66.1 | 90.1 | 66.4 | 98.4 |
| 66 | 351.3 | 424.5 | 843.2 | 182.5 | 1223.8 | 103.2 |
| 67 | 8409.7 | 26223.6 | 34005.1 | 9041.4 | 32601.5 | 3094.7 |
| 68 | 2810.9 | 3260.1 | 2929.3 | 4961.6 | 4818.8 | 513.1 |
| 69 | 11767.3 | 6789.1 | 10957 | 11079.3 | 10388.2 | 3855.9 |
| 70 | 2845.6 | 1603 | 1889.8 | 8120.7 | 1927.8 | 1628.7 |
| 71 | 268.6 | 92.8 | 207.5 | 74.8 | 92.7 | 322.2 |
| 72 | 139.6 | 120.7 | 919.8 | 113.4 | 282.2 | 275.2 |
| 73 | 237.7 | 278.3 | 786.2 | 192.5 | 388.7 | 263.9 |
| 75 | 91.9 | 141.7 | 446.1 | 57.6 | 157.7 | 455.9 |
| 76 | 554.2 | 24.1 | 14.5 | 498.3 | 903.9 | 2000.7 |
| 77 | 2410.2 | 1463.1 | 4948 | 4569.6 | 2755.1 | 5975.3 |
| 78 | 315.7 | 431.5 | 516.1 | 324.3 | 1188.7 | 81 |
| 79 | 162.4 | 146 | 171.7 | 204.3 | 106.5 | 265.2 |
| 80 | 100.7 | 790.8 | 491.3 | 95.1 | 372.8 | 933.3 |
| 81 | 2776.2 | 78.6 | 226.5 | 49.7 | 116.4 | 232.7 |
| 82 | 1230.5 | 231.4 | 120 | 1479.5 | 468.6 | 3720.2 |
| 84 | 308.9 | 961.7 | 101 | 78.7 | 204.4 | 653.1 |
| 85 | 350.3 | 125.3 | 159.1 | 154.7 | 129 | 484.4 |
| 86 | 91 | 85.7 | 73.9 | 126.3 | 52.9 | 189.6 |
| 87 | 296.2 | 320.3 | 1151.2 | 607.3 | 338 | 598.1 |
| 88 | 1909 | 1350 | 5707.2 | 1415.8 | 10923.2 | 860.8 |
| 89 | 206.8 | 203.5 | 145.5 | 160.9 | 154.6 | 303.9 |
| 90 | 95.9 | 93.9 | 95.8 | 99.7 | 108.7 | 108.2 |
| 91 | 362 | 346.8 | 219.6 | 491.8 | 78.8 | 550.5 |
| 92 | 3647.7 | 833.5 | 7305.9 | 83.4 | 3870.6 | 3178.4 |
| 93 | 158.2 | 341.1 | 321.3 | 94.2 | 217.3 | 459 |
| 94 | 199.5 | 488 | 854.3 | 71.2 | 251.2 | 114.4 |
| 95 | 90.1 | 99 | 154.9 | 131 | 686.8 | 242.9 |
| 96 | 152.2 | 124.4 | 172.1 | 130.6 | 192.9 | 111.2 |
| 97 | 251.1 | 342.4 | 429.8 | 95.2 | 143.5 | 354.9 |
| 98 | 240.9 | 37.1 | 30.6 | 67.9 | 14.8 | 257.9 |
| 99 | 321.4 | 281.7 | 244.4 | 316 | 192.3 | 311.7 |
| 100 | 288.5 | 356.2 | 13918.1 | 777.1 | 7145.1 | 1884.5 |
| 101 | 158.2 | 192.6 | 116.4 | 133.6 | 117.1 | 224.1 |
| 102 | 353.9 | 153.6 | 142.8 | 363.4 | 159 | 275.5 |
| 103 | 351.3 | 300.2 | 333.9 | 367.9 | 291.8 | 152.5 |
| 104 | 274.1 | 45.1 | 405.3 | 200.1 | 1282.8 | 873.3 |
| 105 | 449.3 | 51.2 | 538 | 259.7 | 701.6 | 1037.6 |
| 106 | 297.6 | 32.8 | 390.4 | 273.1 | 945.8 | 933.9 |
| 107 | 169.5 | 176.1 | 188.6 | 213.6 | 121.6 | 426.3 |
| 108 | 160.8 | 676.4 | 409.7 | 356.4 | 505.9 | 328.9 |
| 109 | 3423.5 | 49.2 | 4125.3 | 130.2 | 29.4 | 301.6 |
| 110 | 5966.7 | 10.7 | 4569.2 | 200.4 | 25.8 | 956.3 |
| 111 | 253.1 | 609.4 | 71.4 | 158.5 | 134.5 | 223.5 |
| 112 | 1062.8 | 94.2 | 773.3 | 1195.8 | 507.3 | 1.2 |
| 113 | 221.5 | 123.7 | 67.4 | 210.1 | 274.1 | 7.7 |
| 114 | 123.6 | 108.3 | 382.3 | 250.2 | 272.6 | 105.3 |
| 115 | 397.9 | 320.1 | 369.8 | 242.8 | 282.1 | 292 |
| 116 | 279.4 | 395 | 237.5 | 165.5 | 184.5 | 311.1 |
| 117 | 200.4 | 1406.9 | 3545.8 | 607.8 | 2324.7 | 349.8 |
| 118 | 336.5 | 116.3 | 86.4 | 5808.9 | 64.9 | 2556.4 |
| 119 | 365.6 | 76.7 | 50.2 | 122.9 | 83.3 | 80.8 |
| 120 | 48.1 | 344 | 49.8 | 99.6 | 3044.7 | 110.5 |
| 121 | 259.6 | 336.7 | 403.1 | 87.6 | 1213.5 | 143.5 |
| 122 | 292.8 | 185.3 | 455.1 | 158.5 | 34.9 | 507.8 |
| 123 | 139.7 | 152.8 | 81.1 | 352.1 | 126 | 366.5 |
| 124 | 172.2 | 143.6 | 148.2 | 5160.7 | 124 | 172 |

| | | | | | | |
|---|---|---|---|---|---|---|
| 125 | 1555.3 | 978.9 | 878.3 | 1620.9 | 867.1 | 1453.5 |
| 126 | 87.5 | 59.3 | 1558.6 | 257.7 | 1842.3 | 258.4 |
| 127 | 140.3 | 83.5 | 117.6 | 156 | 105 | 192 |
| 128 | 320 | 249.4 | 167.1 | 365.5 | 265.9 | 418.6 |
| 129 | 189.6 | 189.4 | 161.7 | 86 | 198.2 | 224.2 |
| 130 | 122.9 | 84.1 | 71.2 | 115.6 | 87.4 | 72 |
| 131 | 140.1 | 39.7 | 182.4 | 236.8 | 71.2 | 535 |
| 132 | 243.2 | 184 | 208.8 | 237.5 | 218.6 | 256.7 |
| 133 | 174.8 | 338.7 | 113.2 | 82.8 | 67.1 | 321.5 |
| 134 | 182.1 | 160.9 | 497.9 | 87.7 | 278.3 | 109.1 |
| 135 | 286.8 | 205.4 | 723.3 | 307 | 241.7 | 336.9 |
| 138 | 146.4 | 136.3 | 144.8 | 124 | 114.4 | 176.2 |
| 139 | 202.8 | 189.9 | 152 | 72.1 | 169.5 | 132.4 |
| 140 | 18.2 | 1010.6 | 2927.9 | 23.6 | 1469.1 | 1.5 |
| 141 | 178.6 | 283.5 | 2257.9 | 3.4 | 1606.2 | 310 |
| 144 | 6356.1 | 261.5 | 311.4 | 419.2 | 349.3 | 527.5 |
| 145 | 298.7 | 186.1 | 180.4 | 255.9 | 190 | 279.2 |
| 146 | 272 | 229.4 | 195.1 | 295.7 | 196 | 343.1 |
| 147 | 42.5 | 44.6 | 35.8 | 58.6 | 44.5 | 29.3 |
| 148 | 357.4 | 192.1 | 831.9 | 156.5 | 464.4 | 1068.9 |
| 149 | 272.6 | 184 | 1807.9 | 33 | 284.7 | 171 |
| 150 | 182.2 | 502.5 | 356 | 148.7 | 526.3 | 948.6 |
| 151 | 265.1 | 174.8 | 181.4 | 138.7 | 143.7 | 162.4 |
| 153 | 132.4 | 185.7 | 157.8 | 103.5 | 113.3 | 218.6 |
| 154 | 145.6 | 46.6 | 390.9 | 121.8 | 68.4 | 160.8 |
| 155 | 226.6 | 139.4 | 130.3 | 224.6 | 96.3 | 238.7 |
| 156 | 81.5 | 50.5 | 20.5 | 169.1 | 107.5 | 88.7 |
| 157 | 226.7 | 170.6 | 136.4 | 131.5 | 112.2 | 189.1 |
| 158 | 236.4 | 237.1 | 1.1 | 202.4 | 39.1 | 650.6 |
| 159 | 1520.9 | 4126.2 | 3171.7 | 755.4 | 4600.4 | 3258.9 |
| 161 | 31.6 | 790.1 | 3.5 | 12.1 | 2.5 | 1.3 |
| 162 | 62.1 | 71.5 | 45.6 | 40.8 | 21.6 | 57.1 |
| 164 | 391.7 | 235.7 | 1236.8 | 91.4 | 215.8 | 40.9 |
| 166 | 4422.1 | 4880.3 | 7064.9 | 2557.6 | 3776.6 | 8467.5 |
| 167 | 544.2 | 1623 | 684.2 | 1035.6 | 3875 | 2178.2 |
| 168 | 2410.7 | 82.3 | 985.8 | 19.1 | 523.6 | 43 |
| 169 | 485.1 | 1840.5 | 7446.2 | 1266.9 | 1301.4 | 2686.1 |
| 170 | 971.7 | 643 | 1478.2 | 166.9 | 2916.1 | 760.7 |
| 171 | 38.2 | 233.9 | 733 | 1.2 | 1.2 | 503.5 |
| 172 | 492.3 | 472 | 656 | 483.1 | 510.4 | 757.2 |
| 173 | 542.8 | 375.8 | 794.6 | 114.4 | 595 | 647.8 |
| 176 | 10.6 | 32.1 | 1.2 | 69.1 | 991.6 | 1.2 |
| 177 | 224.2 | 1237.4 | 236.6 | 1044.6 | 789.8 | 993.8 |
| 178 | 1934.6 | 409 | 470.7 | 650.5 | 1387 | 941.3 |
| 179 | 145.3 | 412.9 | 60.7 | 161.2 | 85.7 | 245.7 |
| 180 | 161 | 92.4 | 67.4 | 128.2 | 94.3 | 131.7 |
| 181 | 9117.6 | 8152.5 | 11035.8 | 13798.5 | 15093.4 | 8202.9 |
| 182 | 189.4 | 445.2 | 574.2 | 128.1 | 524.1 | 227.5 |
| 183 | 1.1 | 64.7 | 11.7 | 1.2 | 1.1 | 72.1 |
| 184 | 215 | 541.8 | 290 | 167.9 | 286.9 | 190 |
| 185 | 973.9 | 711.7 | 1014 | 679.8 | 914.9 | 1030.3 |
| 186 | 377 | 560.6 | 395.4 | 1755.4 | 302.8 | 732.2 |
| 187 | 53.8 | 67.3 | 41.8 | 81.4 | 33.1 | 104.6 |
| 188 | 43.6 | 33.7 | 103.2 | 61.3 | 1218.5 | 94.9 |
| 189 | 86.4 | 37.9 | 69.2 | 84.5 | 695.6 | 108.4 |
| 190 | 407.6 | 372.7 | 283.5 | 135.6 | 90.2 | 162.9 |
| 191 | 359.8 | 2605 | 2070.6 | 919.3 | 2961.2 | 4325.2 |
| 192 | 856.8 | 383.6 | 183.3 | 146.2 | 69.3 | 827.1 |

| | | | | | | |
|---|---|---|---|---|---|---|
| 193 | 75.8 | 1538.4 | 2686.5 | 638.6 | 2367.3 | 33.3 |
| 194 | 121.6 | 135.5 | 230 | 28.4 | 238.5 | 127.6 |
| 195 | 159.5 | 372.6 | 180 | 614.6 | 333.1 | 355.6 |
| 196 | 7404.9 | 4791.5 | 952.5 | 7933.6 | 7823.2 | 8113.2 |
| 197 | 133.6 | 44.6 | 130.5 | 218.5 | 71.7 | 113.9 |
| 198 | 129.4 | 323.2 | 103.3 | 138.9 | 56.8 | 123.9 |
| 199 | 105.7 | 36 | 1584.1 | 2.7 | 42.7 | 162 |
| 200 | 251.1 | 102.9 | 160.6 | 270.3 | 374.1 | 170.5 |
| 201 | 70.3 | 49.7 | 172.9 | 58.1 | 1.6 | 125.6 |
| 203 | 155.2 | 84.1 | 172.2 | 202.8 | 393.4 | 343.4 |
| 204 | 258.9 | 711.5 | 1093.8 | 268.3 | 710.3 | 515.6 |
| 205 | 1215.1 | 1501.8 | 7081.6 | 570.2 | 5009.6 | 1308.5 |
| 206 | 328 | 173 | 298.2 | 310.4 | 185.1 | 597.2 |
| 207 | 172.9 | 68.6 | 1428.1 | 46.5 | 2541.6 | 144.3 |
| 208 | 1492.3 | 7 | 24.8 | 10.1 | 783.8 | 45.3 |
| 209 | 667.1 | 1776.2 | 311.6 | 1203.9 | 2745 | 1499.4 |
| 211 | 1193.2 | 2651.6 | 12245 | 343.1 | 25458 | 17479.9 |
| 212 | 837.5 | 433 | 245 | 2353.6 | 512.9 | 728.8 |
| 213 | 203.3 | 87 | 71.4 | 3629.9 | 93.5 | 468.6 |
| 215 | 176 | 71.1 | 554.7 | 57.5 | 3381.9 | 202 |
| 216 | 611.8 | 308.3 | 915.4 | 490.4 | 896.8 | 690.5 |
| 217 | 118.5 | 146.7 | 191.2 | 121 | 293.9 | 120.7 |
| 218 | 252.2 | 466.5 | 274.1 | 124.9 | 335.3 | 428.6 |
| 220 | 125.2 | 75.4 | 108.2 | 10561 | 77.9 | 131.8 |
| 221 | 139.2 | 264.1 | 452 | 146.5 | 699.3 | 143.9 |
| 223 | 415.6 | 845.6 | 362.8 | 477.7 | 165.6 | 689.8 |
| 227 | 314.8 | 2480.3 | 1008.1 | 182.6 | 1091.2 | 223.2 |
| 228 | 432.9 | 294.9 | 213.2 | 461.6 | 568.4 | 517.8 |
| 229 | 143.7 | 774 | 171.7 | 1810.6 | 305 | 446.6 |
| 230 | 179 | 63.3 | 79.4 | 202.4 | 63.6 | 37.6 |
| 231 | 136.2 | 113.7 | 127 | 180.7 | 207.4 | 205 |
| 232 | 45.3 | 272.3 | 86.5 | 8.9 | 63.8 | 337.4 |
| 233 | 1483 | 4545.5 | 6134.3 | 1579.1 | 7762.7 | 3649.3 |
| 235 | 85.7 | 443.4 | 423.8 | 66.6 | 211.8 | 323.6 |
| 236 | 110.2 | 202.4 | 106.8 | 196.1 | 123.9 | 257.5 |
| 237 | 1495.9 | 564.8 | 1641 | 1057.2 | 1533.6 | 704.4 |
| 238 | 744.2 | 349.2 | 899.4 | 244.1 | 1354 | 349.4 |
| 239 | 186.3 | 100.4 | 196.3 | 93 | 124 | 153.1 |
| 240 | 195.9 | 71.5 | 727.2 | 65.8 | 131.3 | 148 |
| 241 | 237.2 | 205.2 | 1034.3 | 94.1 | 300.5 | 145.6 |
| 242 | 5003.3 | 13867.1 | 2129.7 | 15742.7 | 4086.9 | 11985.6 |
| 243 | 803.1 | 288.9 | 174.5 | 134.2 | 226.2 | 143.1 |
| 244 | 97.8 | 194.6 | 282.9 | 8.3 | 256.1 | 109.7 |
| 246 | 359 | 276.8 | 388.3 | 169.2 | 115.4 | 448.4 |
| 247 | 170.8 | 140.7 | 109.7 | 303.9 | 118.1 | 139.1 |
| 248 | 245.6 | 109.7 | 217.3 | 113.2 | 196.9 | 135.7 |
| 249 | 96.3 | 216.6 | 223.8 | 135.9 | 48.3 | 585.6 |
| 251 | 187.9 | 61.1 | 115.5 | 59.8 | 112.5 | 93.9 |
| 253 | 260.8 | 275.7 | 327.1 | 165.6 | 468.1 | 240.6 |
| 254 | 63.2 | 64.3 | 465.3 | 121 | 2267.5 | 15.9 |
| 255 | 1923 | 352.4 | 648.8 | 811.1 | 1499.8 | 1126.3 |
| 256 | 563.4 | 448.2 | 228.9 | 490.4 | 82.7 | 573.9 |
| 257 | 378.8 | 648.4 | 475.3 | 161.3 | 185.7 | 145.8 |
| 258 | 582.1 | 257.2 | 5241.4 | 168 | 40.2 | 1024 |
| 259 | 30 | 73.9 | 532.4 | 28.9 | 44.5 | 13.3 |
| 260 | 209.5 | 228.1 | 128.5 | 237.3 | 114 | 618.7 |
| 261 | 135.7 | 834.3 | 320.6 | 115.8 | 259.6 | 134.1 |
| 263 | 320.6 | 673.5 | 537 | 470.6 | 473.9 | 2280.3 |

| | | | | | | |
|---|---|---|---|---|---|---|
| 263 | 316.2 | 103.4 | 161.5 | 369.4 | 123.6 | 137.2 |
| 265 | 106.3 | 287.4 | 601.3 | 317.4 | 934.8 | 119.6 |
| 266 | 216.8 | 145.8 | 179.8 | 955 | 181.8 | 470.8 |
| 256 | 147.4 | 89.7 | 97.7 | 81.8 | 124.2 | 119.5 |
| 268 | 304.8 | 19.7 | 60.1 | 175.6 | 2929.8 | 220.9 |
| 269 | 61.3 | 84.9 | 35 | 57.5 | 38 | 158.7 |
| 270 | 276.1 | 144.9 | 183.1 | 1394.4 | 176.9 | 726.2 |
| 271 | 139.7 | 191.4 | 249.6 | 243.4 | 292.7 | 190 |
| 272 | 1068.5 | 485.1 | 605.5 | 722 | 403 | 558.4 |
| 273 | 145.8 | 421.9 | 4104.1 | 1.3 | 1705.7 | 34.7 |
| 274 | 178.2 | 517.1 | 426.4 | 468.2 | 206.6 | 681.6 |
| 275 | 271.4 | 219.3 | 164 | 299.8 | 209.9 | 428.2 |
| 276 | 396.3 | 3030.8 | 977.3 | 188 | 1246.2 | 307.4 |
| 277 | 228.6 | 153.3 | 181.7 | 212.1 | 199 | 244.3 |
| 278 | 50.3 | 1164.1 | 1019 | 52 | 88.6 | 105.7 |
| 279 | 985.7 | 1407 | 2129.1 | 232.5 | 2794.6 | 1002 |
| 280 | 1510.7 | 2192.5 | 6373 | 286.6 | 3920.2 | 1434.5 |
| 281 | 24.5 | 1.2 | 32.8 | 20.6 | 66.1 | 40.3 |
| 282 | 4703.6 | 248.3 | 312.9 | 291 | 241.6 | 404.4 |
| 283 | 413.8 | 290.4 | 220 | 660.8 | 232 | 552.8 |
| 284 | 106.4 | 84.7 | 78.8 | 140.9 | 65.1 | 99.7 |
| 285 | 148.4 | 92.7 | 162.5 | 143.5 | 104.4 | 219 |
| 286 | 129 | 10.1 | 1134.4 | 36.1 | 1.3 | 510.6 |
| 287 | 127.8 | 111.9 | 67.9 | 468.2 | 86.5 | 147.5 |
| 288 | 267.1 | 129.5 | 153.3 | 256.4 | 171.7 | 231.2 |
| 289 | 91.1 | 390.9 | 271 | 465.5 | 214.2 | 1306.3 |
| 291 | 133.2 | 72.5 | 78.4 | 5.9 | 133.9 | 35.1 |
| 292 | 75.7 | 25.7 | 293.3 | 71.5 | 18.4 | 104.7 |
| 293 | 438 | 86.4 | 72.7 | 37.3 | 77.1 | 371 |
| 295 | 244.8 | 581.6 | 30.1 | 979.1 | 569.4 | 507.9 |
| 296 | 1349.4 | 504 | 1210.8 | 671.3 | 716.7 | 813.5 |
| 297 | 64.9 | 45.4 | 49.4 | 853.9 | 62 | 98.3 |
| 298 | 186.2 | 307.3 | 416.8 | 311.1 | 232.4 | 211.4 |
| 299 | 306.5 | 444.4 | 962 | 703.8 | 375.8 | 401.4 |
| 300 | 420.6 | 101.7 | 176.4 | 125.4 | 475.3 | 235.4 |
| 301 | 507.2 | 2222 | 808.6 | 528.9 | 1281.3 | 340.3 |
| 302 | 29.2 | 22.3 | 23.2 | 31.4 | 9.5 | 10.1 |
| 303 | 457.4 | 993.6 | 1360.4 | 930 | 836.6 | 1031.2 |
| 304 | 262.8 | 452.7 | 227.3 | 209.2 | 208.1 | 397.9 |
| 305 | 531.2 | 6335.1 | 145.3 | 300 | 1625.9 | 5308.7 |
| 306 | 262.8 | 287.6 | 155.9 | 258.3 | 126 | 317.5 |
| 307 | 150.7 | 121.6 | 150 | 167.9 | 118.6 | 139.3 |
| 308 | 1991.2 | 756 | 1163.4 | 4741.6 | 1375.7 | 4290.1 |
| 310 | 950.2 | 194.6 | 1093.2 | 1092.8 | 219.2 | 640.7 |
| 311 | 64.6 | 25.1 | 144.2 | 55.8 | 44.7 | 73.8 |
| 312 | 797.1 | 35.8 | 1289.1 | 5881.4 | 362.4 | 1192 |
| 313 | 542.5 | 719.4 | 122 | 119.1 | 139.3 | 161.9 |
| 314 | 938.2 | 209.6 | 551.3 | 68.6 | 147.2 | 345 |
| 315 | 123.7 | 274.1 | 155.2 | 218.6 | 299.1 | 52.8 |
| 316 | 289.8 | 413.1 | 415.9 | 205.6 | 84.3 | 704.2 |
| 317 | 202.4 | 156.1 | 133.7 | 210.8 | 138.1 | 206.3 |
| 318 | 126.8 | 259.1 | 109.3 | 26.5 | 16.2 | 157.6 |
| 319 | 362 | 168.9 | 161.6 | 713.5 | 195.9 | 1566.2 |
| 320 | 503.3 | 277.3 | 356.8 | 1176.5 | 487.2 | 297.3 |
| 321 | 218.1 | 470.4 | 426.1 | 337.2 | 517.9 | 711.3 |
| 322 | 375.3 | 251.1 | 292.4 | 504 | 186.1 | 609.6 |
| 323 | 553 | 949.4 | 2829.3 | 768.9 | 4431.8 | 1365.8 |
| 324 | 179.2 | 52.7 | 227.9 | 81.1 | 81.7 | 41.1 |

| | | | | | | |
|---|---|---|---|---|---|---|
| 325 | 335.3 | 242.7 | 242.8 | 170.7 | 198.5 | 313.4 |
| 326 | 1260.9 | 2604.5 | 4986.5 | 584.6 | 5373.5 | 643.7 |
| 327 | 29.1 | 14.2 | 26.9 | 21.4 | 61.9 | 2.8 |
| 328 | 58.8 | 218.5 | 113.1 | 63.9 | 223.4 | 73.3 |
| 329 | 191.7 | 61.4 | 40.3 | 147.8 | 62.3 | 144.2 |
| 330 | 134.1 | 100.9 | 58.4 | 36 | 18.7 | 37.3 |
| 331 | 445.5 | 369.7 | 271.4 | 981.9 | 227.2 | 893.2 |
| 333 | 76.9 | 473 | 115.9 | 230.1 | 287.1 | 484.1 |
| 334 | 172.8 | 136 | 152.4 | 100.5 | 143.3 | 120.5 |
| 335 | 122.9 | 126.9 | 113.2 | 212.1 | 846.9 | 658.3 |
| 336 | 308.8 | 716 | 987.8 | 236.4 | 665.2 | 600.4 |
| 337 | 43.9 | 98.3 | 1139.9 | 53.3 | 129.7 | 270.9 |
| 338 | 290.4 | 144.6 | 420.3 | 105.8 | 134.4 | 220.2 |
| 339 | 338.2 | 255.3 | 217.6 | 483.7 | 351.7 | 459.4 |
| 340 | 241.7 | 256.2 | 235.6 | 263.3 | 247.8 | 313.8 |
| 341 | 693.6 | 1174.2 | 706.5 | 545.1 | 590.9 | 506.5 |
| 343 | 2675.2 | 997.1 | 235.2 | 5179.8 | 686.5 | 3885.2 |
| 344 | 1600.8 | 650.5 | 866.5 | 3374.5 | 937.5 | 785.9 |
| 345 | 371 | 324 | 225.2 | 332.3 | 208.4 | 349.4 |
| 347 | 930.2 | 70.2 | 922.3 | 734.3 | 7238.6 | 705.8 |
| 348 | 2298.3 | 37.7 | 1946.1 | 988.1 | 10651.2 | 2867.2 |
| 349 | 208.2 | 402.1 | 215.3 | 174.1 | 296.4 | 343.8 |
| 350 | 2.9 | 34.4 | 518.4 | 27.6 | 143.5 | 191.8 |
| 351 | 188.2 | 69.4 | 94.8 | 40.4 | 46.1 | 744.7 |
| 352 | 269.7 | 230.4 | 500.5 | 390.8 | 298.9 | 319.5 |
| 353 | 757.9 | 154.1 | 248.3 | 481.8 | 1304.4 | 432.8 |

Table 4. 4-Gene signature performance in 98 training samples

| | Tumor | Benign |
|---|---|---|
| Positive | 48 | 18 |
| Negative | 4 | 28 |
| Sensitivity | 92% (0.82, 0.97) | |
| Specificity | 61% (0.46, 0.74) | |

B. Signature Identification using Linear Discrimination Analysis

We used a forward selection process that adds one gene at a time until the posterior error as evaluated by a linear discriminator is less than or equal to 0.1. A four-gene signature was discovered using this approach with the 322 genes. The identities of these 4 genes are listed in Tables 5 and 16 and their chip data are shown in Tables 6 and 7.

Table 5. 4-Gene Signature

| SEQ ID NO: | Gene Name |
|---|---|
| 354 | Chemokine (C-C motif) ligand 18 (pulmonary and activation-regulated) |
| 199 | Pulmonary surfactant-associated protein B (SP-B) |
| 207 | K+ channel beta subunit |
| 255 | Putative prostate cancer tumor suppressor |

Leave One Out Cross Validation (LOOCV) resulted in 92% sensitivity and 61% specificity, shown in Table 4. The ROC curve gave an AUC of 0.897, as shown in Figure 1.

Table 6

| | Signal | | | | | | |
|---|---|---|---|---|---|---|---|
| SEQ ID | PC_984TT | PC_986TT | FA_987TT | PC_988TT | PC_989TT | FA_992TT | FA_993TT |
| 12 | 3399.2 | 7041.3 | 5438 | 6376.7 | 2734.6 | 3569.5 | 4305.9 |
| 18 | 6550.8 | 5870.6 | 5815.9 | 4265.1 | 8856.4 | 3454.4 | 20405.3 |
| 29 | 4578.6 | 6455.8 | 2329.4 | 4259 | 2666.8 | 3694 | 7531.2 |
| | FA_994TT | FA_995TT | FA_996TT | FA_998TT | FA_999TT | FA_1001TT | FA_1002TT |
| 12 | 2545.7 | 2451.8 | 4418 | 3938.3 | 3648.5 | 6834.5 | 4882 |
| 18 | 12566.4 | 9889.5 | 4341.5 | 5210.1 | 6639.5 | 4794.8 | 3921.9 |
| 29 | 2239.8 | 1834.5 | 4607.1 | 6424.1 | 3069.6 | 4301.4 | 3164 |
| | FA_1004TT | FA_1005TT | FA_1006TT | FA_1010TT | FA_1013TT | FA_1014TT | FA_1017TT |
| 12 | 2274.9 | 2644.2 | 3978.8 | 2999.7 | 4012.5 | 1724 | 5005.8 |
| 18 | 10402 | 7376.3 | 9985.1 | 1330.3 | 4618.8 | 6132 | 4562.6 |
| 29 | 1688.9 | 4771.2 | 3159.1 | 3082.6 | 6861.7 | 1465 | 3384.7 |
| | FA_1018TT | FA_1020TT | FA_1023TT | FA_1024TT | FA_1026TT | FA_1027TT | FA_1028TT |
| 12 | 2847.6 | 7102 | 7513.5 | 2086.3 | 2431.3 | 1318.2 | 1231 |
| 18 | 6388.5 | 2640.8 | 1967.7 | 6853.3 | 5068.5 | 2006.4 | 1804.9 |
| 29 | 3829.5 | 4235 | 4272.5 | 924 | 1702.3 | 1691.5 | 1731.3 |
| | FA_1029TT | FA_1030TT | FA_10311TT | FA_1032TT | FA_1034TT | FA_1035TT | FC_1037TT |
| 12 | 4607.6 | 2416.1 | 2203.7 | 6489.9 | 2095.3 | 1547.9 | 1847.4 |
| 18 | 3507.3 | 2270.8 | 8058 | 11247.5 | 8258.1 | 7485.8 | 8309 |
| 29 | 1530.5 | 5641.4 | 3460.1 | 4802.1 | 1650.7 | 854.6 | 2418.4 |
| | PC_1039TT | PC_1040TT | PC_1041TT | PC_1042TT | PC_1043TT | PC_1044TT | PC_1045TT |
| 12 | 8204.9 | 2638.5 | 4795 | 2607.8 | 6725.4 | 5178.7 | 2111.1 |
| 18 | 6454.7 | 10129.3 | 7964 | 4952.6 | 3663.3 | 1887.7 | 19840.6 |
| 29 | 8858.5 | 4136.8 | 8434 | 2230.7 | 3434 | 2770 | 2243.1 |

(continued)

| | | | | | | | |
|---|---|---|---|---|---|---|---|
| | PC_1046TT | PC_1047TT | PC_1048TT | PC_1049TT | PC_1050TT | PC_1051TT | PC-FV_1052TT |
| 12 | 4403.6 | 9394.7 | 2262.5 | 4831.2 | 2826.9 | 1288.6 | 1953.2 |
| 18 | 2417.7 | 5678.4 | 6655.2 | 4325.2 | 1706 | 6904.8 | 4596.1 |
| 29 | 2319.8 | 20509.6 | 1176.1 | 5375.3 | 2053.1 | 4027.5 | 3332.3 |
| | PC_1053TT | PC_1054TT | PC_1055TT | PC_1059TT | PC_1060TT | PC_1061TT | PC_1062TT |
| 12 | 4150.4 | 6180.4 | 1835.7 | 2447.2 | 4201.3 | 4984.3 | 3399.3 |
| 18 | 5965.7 | 13847.6 | 3740.6 | 4167.7 | 14516.2 | 4896.7 | 8891.6 |
| 29 | 3718.1 | 4818.3 | 1728.8 | 2972.2 | 4851 | 3469 | 3718.6 |
| | PC_FV_1064TT | PC-FV_1065TT | PC-FV_1066TT | PC-FV_1067TT | PC-FV_1068TT | PC-FV_1069TT | PC-FV_1071 TT |
| 12 | 4221 | 4884.5 | 7256.6 | 640 | 4055 | 3208.2 | 6301.3 |
| 18 | 4095 | 6627.6 | 1427.1 | 5405.5 | 10722 | 11509.9 | 10314.3 |
| 29 | 5896.7 | 5461.2 | 9437.3 | 1634.4 | 1344.2 | 2186.4 | 1794.8 |
| | PC_FV_1072TT | FA_1073TT | FA_1074TT | FA_1075TT | FA_1076TT | FC_1077TT | FC_1078TT |
| 12 | 1390.3 | 3325.5 | 1430.8 | 1784.4 | 1563.9 | 1999.4 | 1830.7 |
| 18 | 5629 | 10010.5 | 3459.2 | 11454 | 10807.8 | 1384.8 | 11906.2 |
| 29 | 2974.5 | 7133.1 | 3009 | 4184.9 | 1673.9 | 1044.5 | 4264.1 |
| | FC_1079TT | PC-FV_1080TT | PC-FV_1081TT | FC_1082TT | pb1 | pb2 | pb3 |
| 12 | 2856.2 | 2437.9 | 3541.2 | 4439.8 | 11.4 | 9.1 | 9.9 |
| 18 | 22738 | 4344.1 | 4108.3. | 3312.3 | 94.2 | 50.7 | 67.6 |
| 29 | 1092.8 | 3052.8 | 3302.6 | 2383 | 8.7 | 21.6 | 30.5 |
| | pb4 | pb5’ | pb6’ | pb7’ | pb8’ | pd10 | pd11 |
| 12 | 33.8 | 8.3 | 6.6 | 18.8 | 61.4 | 38.2 | 10.7 |
| 18 | 74.9 | 29.7 | 53.6 | 37.8 | 31.8 | 68.8 | 117.5 |
| 29 | 66.1 | 64.6 | 36.4 | 51.5 | 25.1 | 17.2 | 17.2 |
| | pd12 | pd9 | | | | | |

(continued)

| | FC_1079TT | PC-FV_1080TT | PC-FV_1081TT | FC_1082TT | pb1 | pb2 | pb3 |
|---|---|---|---|---|---|---|---|
| 12 | 4.1 | 11.6 | | | | | |
| 18 | 70.8 | 23.4 | | | | | |
| 29 | 19.7 | 11 | | | | | |

Table 7

| | Signal | | | | | | |
|---|---|---|---|---|---|---|---|
| SEQ ID | FA_987TT | FA_992TT | FA_993TT | FA_994TT | FA_995TT | FA_996TT | FA_998TT |
| 354 | 50.9 | 100.4 | 63.7 | 14 | 699.7 | 181.7 | 11.9 |
| 199 | 81.7 | 18 | 43.3 | 130.3 | 461.9 | 469.4 | 135.6 |
| 207 | 3844.9 | 1325.1 | 77.9 | 397.2 | 518.3 | 807.5 | 1084.3 |
| 255 | 1374.4 | 2332.1 | 1631 | 611.1 | 1616.7 | 419.2 | 230.7 |
| | FA_999TT | FA_1001TT | FA_1002TT | FA_1004TT | FA_1005TT | FA_1006TT | FA_1010TT |
| 354 | 194.7 | 2397.3 | 50.6 | 205.2 | 9.6 | 29.1 | 341.5 |
| 199 | 124.1 | 574.9 | 206 | 158.5 | 423.6 | 121 | 2489.4 |
| 207 | 1861.5 | 1325.1 | 846.6 | 308.8 | 1765.9 | 598.9 | 1067.7 |
| 255 | 266.5 | 186.7 | 324.2 | 94 | 1071.3 | 1098.5 | 1294 |
| | FA_1013TT | FA_1014TT | FA_1017TT | FA_1018TT | FA_1020TT | FA_1023TT | FA_1024TT |
| 354 | 207.5 | 69 | 21.7 | 20.1 | 64.7 | 87.4 | 372.5 |
| 199 | 440.8 | 99.8 | 221.6 | 226.9 | 108.2 | 155.2 | 112.6 |
| 207 | 535.7 | 1910.9 | 1185.2 | 571.8 | 1552.7 | 2739.5 | 692.6 |
| 255 | 437.2 | 655.6 | 165.1 | 178.4 | 86.4 | 436.5 | 40.7 |
| | FA_1026TT | FA_1027TT | FA_1028TT | FA_1029TT | FA_1030TT | FA_1031TT | FA_1032TT |
| 354 | 21.5 | 18.1 | 3.8 | 13.3 | 66 | 15.1 | 38.8 |
| 199 | 70.9 | 46.1 | 561.3 | 67.7 | 35.8 | 48 | 107.3 |
| 207 | 1230.4 | 80.5 | 732 | 3216.4 | 2253.5 | 1989.9 | 2115.2 |
| 255 | 479.9 | 226.5 | 35.9 | 1403.4 | 1144.3 | 247.9 | 1989.4 |
| | FA_1034TT | FA_1035TT | FA_1073TT | FA_1074TT | FA_1075TT | FA_1076TT | PC_984TT |
| 354 | 61.3 | 25.3 | 1355.7 | 188.5 | 14.7 | 8.9 | 97.5 |
| 199 | 216.5 | 56.1 | 1980.6 | 454.1 | 86.2 | 173.8 | 154.2 |
| 207 | 421.8 | 1493.5 | 771.6 | 1291.4 | 116.5 | 1169.9 | 759.2 |
| 255 | 489.2 | 229.5 | 205.3 | 655 | 152.7 | 156.3 | 152 |

(continued)

| | | | | | | | |
|---|---|---|---|---|---|---|---|
| | PC_986TT | PC_988TT | PC_989TT | PC_1039TT | PC_1040T T | PC_1041TT | PC_1042T T |
| 354 | 966.6 | 619 | 12.9 | 2577 | 540.4 | 1229.7 | 320 |
| 199 | 103.9 | 142.5 | 288.5 | 1006.6 | 3990.7 | 17402.7 | 1023.2 |
| 207 | 400.5 | 2357.5 | 1213.8 | 627.5 | 62.6 | 133.6 | 981.5 |
| 255 | 299.7 | 2137.5 | 131.9 | 500.6 | 2412.4 | 2763.1 | 303.4 |
| | PC_1043TT | PC_1044TT | PC_1045TT | PC_1046TT | PC_1047T T | PC_1048TT | PC_1049T T |
| 354 | 444.3 | 170.8 | 817.6 | 1095.1 | 966.8 | 1263.5 | 800.7 |
| 199 | 604.3 | 222.7 | 22961.4 | 9488.3 | 10311.6 | 1702.7 | 1366 |
| 207 | 1680.3 | 594.2 | 136.5 | 673.2 | 755 | 20.8 | 254.8 |
| 255 | 407.6 | 4162.4 | 872.5 | 2472.3 | 1497.3 | 2944.8 | 2399.9 |
| | PC_1050TT | PC_1051TT | PC_1053TT | PC_1054TT | PC_1055T T | PC_1059TT | PC_1060T T |
| 354 | 292.9 | 614.4 | 1035.4 | 1545.3 | 139.5 | 500.2 | 37.1 |
| 199 | 117 | 29233.8 | 1435 | 8550.5 | 242.8 | 13914.6 | 2377.3 |
| 207 | 87.8 | 113.4 | 73.3 | 187.7 | 491.6 | 158.4 | 207.3 |
| 255 | 2331.8 | 917.2 | 1505.9 | 2186.4 | 3033.2 | 2191.1 | 215.4 |
| | PC_1061TT | PC_1062TT | FC_1037TT | FC_1077TT | FC_1078T | FC_1079TT | FC_1082T |
| 207 | 1088.5 | 1133.5 | 838.8 | 2012.3 | 24.9 | 196.8 | 1356.4 |
| 255 | 3084.8 | 1017.8 | 1355.3 | 199 | 1352.4 | 1578 | 1294.4 |
| | PC-FV_1052TT | PC-FV_1064TT | FV_1065TT | PC-FV_1066TT | PC-FV_1067TT | PC-FV_1068TT | PC-FV_1069TT |
| 354 | 394.8 | 131.4 | 143.7 | 281.9 | 302.1 | 973.6 | 44.9 |
| 199 | 4620.7 | 1931 | 1613.2 | 170.8 | 579.2 | 20066.3 | 96.9 |
| 207 | 88.2 | 1033.1 | 705 | 244.1 | 2559 | 8.8 | 12.3 |
| 255 | 2479.7 | 242.2 | 1322 | 641.9 | 1222.8 | 1914.8 | 93.2 |
| | PC-FV_1071TT | PC-FV_1072TT | PC-FV_1080TT | PC-FV_1081TT | | | |
| 354 | 109.5 | 127.2 | 205.7 | 12.4 | | | |
| 199 | 257.5 | 2302.7 | 320 | 206 | | | |

(continued)

| | PC-FV_1071TT | PC-FV_1072TT | PC-FV_1080TT | PC-FV_1081TT |
|---|---|---|---|---|
| 207 | 42.9 | 72.2 | 2795.9 | 145 |
| 255 | 1128.1 | 2320.8 | 1705.2 | 196.8 |

C. Manual Selection of Markers

Individual genes were selected with an aim to formulate a RT-PCR based assay. Comparison of gene expression profiles between thyroid cancers and non-cancer tissues has identified a five-gene signature from these 322 genes. The identities of these five genes are shown in Tables 8 and 16 and the chip data are shown in Table 9. The performance of this signature was assessed using LDA in the 98 samples, and the signature gives 92% sensitivity and 70% specificity, shown in Table 10. The ROC curve gave an AUC of 0.88, as shown in Figure 2.

Table 8. 5-Gene signature

| SEQ ID NO: | Gene Name |
|---|---|
| 53 | Cadherin 3, type 1 (P cadherin) |
| 242 | Fibronectin |
| 76 | Secretory granule, neuroendocrine protein 1 |
| 36 | Testican-1 |
| 211 | Thyroid Peroxidase (TPO) |

Table 9

| SEQ ID | BN_800TT | BN_801TT | BN_802TT | BN_804TT | BN_805TT | BN_806TT | BN_807TT |
|---|---|---|---|---|---|---|---|
| 36 | 908.3 | 247 | 118.9 | 349.7 | 425.1 | 229.8 | 227.7 |
| 53 | 48.6 | 25 | 24.1 | 34 | 17.8 | 32.2 | 16.7 |
| 76 | 252.3 | 272.9 | 75.2 | 260.6 | 169 | 587.1 | 189.8 |
| 242 | 22247.9 | 1204.2 | 676.7 | 2441.5 | 3900.1 | 2073.4 | 2060.1 |
| 211 | 24.2 | 16983.5 | 32579.5 | 20189.3 | 24480.5 | 14186.3 | 6488.1 |
| | BN_871TT | BN_913TT | BN_914TT | BN_915TT | FA_917TT | FA_918TT | FA_919TT |
| 36 | 897.2 | 435.1 | 442.3 | 508.7 | 169.6 | 184.3 | 213.3 |
| 53 | 192.4 | 28 | 76.1 | 44.2 | 67.7 | 135.7 | 25.7 |
| 76 | 2134.6 | 252.5 | 956.3 | 174.3 | 62 | 144 | 233.8 |
| 242 | 13722.5 | 10481.9 | 4172.9 | 1891.1 | 1654.2 | 11791 | 2788.1 |
| 211 | 6243.3 | 16339.1 | 14355 | 36350.1 | 29876.5 | 19395.7 | 15935.8 |
| | FA_818TT | FA_819TT | FA_820TT | FA_821TT | FA_822TT | FA_842TT | FA_862TT |
| 36 | 120.4 | 155.8 | 210.6 | 31.7 | 283.3 | 122.2 | 24.1 |
| 53 | 27.2 | 26.9 | 34 | 33.2 | 34.4 | 27.8 | 66.1 |
| 36 | 120.4 | 155.8 | 210.6 | 31.7 | 283.3 | 122.2 | 24.1 |
| 53 | 27.2 | 26.9 | 34 | 33.2 | 34.4 | 27.8 | 66.1 |
| 76 | 13 | 69.8 | 36.2 | 171.2 | 116.2 | 190.8 | 15.9 |
| 242 | 1433.3 | 2211.8 | 2714.7 | 325 | 2278 | 3959.5 | 1573.1 |
| 211 | 15976.6 | 19882.4 | 10029.6 | 29411.3 | 25179.3 | 12492.5 | 32721.4 |
| | FA_863TT | FA_864TT | FA_865TT | FA_866TT | FA_867TT | FA_869TT | FA_907TT |
| 36 | 955.1 | 173.1 | 400.5 | 230.8 | 233.5 | 2581.5 | 207.8 |
| 53 | 333.9 | 27.9 | 147.1 | 155 | 25 | 1224.9 | 52.8 |
| 76 | 2614.7 | 624.1 | 614.9 | 336.7 | 163.5 | 1921.3 | 38.5 |
| 242 | 930.4 | 1419.2 | 4708.4 | 2169.8 | 1081.9 | 3241.5 | 4555.3 |
| 211 | 19631.3 | 27382.8 | 29846 | 25011.6 | 30599.1 | 23680.6 | 35313.3 |

(continued)

| | FA_908TT | FA_920TT | FA_938TT | FA_9401T | FA_941TT | Fa_EA40374_921TT | Fa_EA40376_923TT |
|---|---|---|---|---|---|---|---|
| 36 | 329.6 | 165.4 | 331.5 | 298.8 | 267.4 | 211.3 | 188.6 |
| 53 | 35.8 | 28.5 | 21.7 | 291.4 | 45.3 | 23.8 | 62.8 |
| 76 | 253.8 | 64.7 | 3312.7 | 870.1 | 1038.3 | 153.3 | 236.1 |
| 242 | 1630.4 | 2452.4 | 6684 | 765 | 2229.5 | 1809.2 | 3314 |
| 211 | 21639.5 | 31897.6 | 26420.3 | 18786.4 | 31922.5 | 36025.3 | 15065.2 |
| | BN_EA40377_924TT | Fa_EA40378_92 5TT | Fa_EA40379_926TT | BN_EA40380_927TT | Fa_EA40387_955TT | Fa_EA40388_956TT | Fa_EA40389_957TT |
| 36 | 1409.1 | 215.1 | 228.5 | 833.3 | 94.7 | 259.7 | 786.2 |
| 53 | 2294.2 | 40.5 | 496.1 | 64.9 | 197.4 | 230.4 | 17.3 |
| 76 | 624.1 | 149.1 | 211.3 | 501.1 | 2225.4 | 1443.7 | 223.7 |
| 242 | 21699.6 | 1079.3 | 21653.5 | 3263.3 | 2194.8 | 989.6 | 2107.8 |
| 211 | 664.1 | 12583.3 | 13036.6 | 43191.4 | 9409.2 | 19630.9 | 4160.9 |
| | Fa_EA40390_959TT | Fa_EA40391_96 0TT | Fa_EA40392_961TT | Fa_EA40393_962TT | PC_829TT | PC_830TT | PC_831TT |
| 36 | 2496.1 | 175.3 | 58.2 | 1470.7 | 330.3 | 210.3 | 432.7 |
| 53 | 24.4 | 40.3 | 52.6 | 183 | 73 | 49.6 | 1916.2 |
| 76 | 2397 | 47.6 | 77.2 | 1048.1 | 758.4 | 576.2 | 394.3 |
| 242 | 1991.2 | 1042.5 | 1474.6 | 688.1 | 6760 | 6785 | 25733 |
| 211 | 38927.5 | 31764 | 34287.3 | 44314.7 | 25565 | 31930.4 | 138.4 |
| | PC_832TT | PC_834TT | PC_835TT | PC_836TT | PC_837TT | PC_838TT | PC_839TT |
| 36 | 1376.8 | 4076.8 | 1208.3 | 585 | 1015.5 | 1620.2 | 71 |
| 53 | 1909.1 | 2701 | 1178.5 | 710.7 | 1469.6 | 3009.9 | 41.2 |
| 76 | 433.6 | 665.5 | 654.3 | 2432.3 | 412.1 | 1946.7 | 327.2 |
| 242 | 9423.8 | 18037.6 | 23053.8 | 1044.8 | 22442.7 | 27313.9 | 3641 |
| 211 | 7174.2 | 1961.7 | 282.1 | 24646.9 | 15840.2 | 264.2 | 17049.3 |

(continued)

| | PC_879TT | PC_881TT | PC_882TT | PC_883TT | PC_884TT | PC_885TT | PC_886TT |
|---|---|---|---|---|---|---|---|
| 36 | 457.4 | 1779.4 | 561.2 | 722.1 | 1129.2 | 552.5 | 1244.9 |
| 53 | 2036.8 | 3121.5 | 2072.4 | 2753 | 2221.1 | 1703.8 | 1325.9 |
| 76 | 1168 | 1829.9 | 1210.6 | 2663.6 | 1076 | 1232.2 | 1866.8 |
| 242 | 46283.3 | 32477.3 | 33142.3 | 38026.7 | 42087.5 | 49249 | 39172.5 |
| 211 | 86.8 | 613.5 | 48.3 | 126.2 | 1565.2 | 120 | 1553 |
| | PC_890TT | PC_892TT | PC_893TT | PC_894TT | PC_903TT | PC_904TT | PC_928TT |
| 36 | 813.3 | 2211.4 | 1046.2 | 585.3 | 3395 | 767.9 | 246.6 |
| 53 | 4685.6 | 3536.8 | 1363.5 | 1244.4 | 3057.5 | 199.9 | 41.1 |
| 76 | 3926.2 | 2787 | 2359.3 | 1020.3 | 1564 | 203.6 | 421.5 |
| 242 | 36114.4 | 31599.6 | 34700.3 | 41193.8 | 18485.8 | 19181.5 | 3729.4 |
| 211 | 1569.2 | 1703.6 | 234.5 | 1594 | 4063.1 | 5354.1 | 18199.2 |
| | PC_932TT | PC_933TT | Pc_EA40375_ 922TT | Pc_EA40381 _945TT | Pc_EA4038 2_946TT | 3_947TT | Pc_EA40384 _948TT |
| 36 | 352.4 | 920.3 | 316.8 | 818.6 | 3078.8 | 532.1 | 311.1 |
| 53 | 3447.4 | 788.3 | 41.8 | 803.9 | 2330.1 | 1337.6 | 1556.2 |
| 76 | 850.2 | 865.6 | 211.2 | 237.9 | 1830 | 1279.7 | 598 |
| 242 | 21913.7 | 24536 | 1758.5 | 13301.5 | 22076.9 | 31987.1 | 27579.2 |
| 211 | 490.5 | 13171.6 | 30959.7 | 331 | 857.8 | 2007.7 | 556.5 |
| | FC_823TT | FC_824TT | FC_825TT | FC_827T | FC_828TT | FC_840TT | FC_896TT |
| 36 | 282.5 | 2413 | 1506.3 | 821.8 | 564.4 | 331.6 | 857.1 |
| 53 | 23.2 | 24.1 | 25.5 | 348.1 | 27.8 | 32.3 | 245.4 |
| 76 | 460.1 | 2833.9 | 1128.9 | 1421.6 | 705.3 | 341.8 | 358.9 |
| 242 | 12129 | 22000.8 | 874.8 | 2544.9 | 845.1 | 3459.7 | 1998.1 |
| 211 | 30520.5 | 496.2 | 7923.9 | 11508.2 | 33401.7 | 10882.8 | 1625.3 |

(continued)

| | FC_898TT | FC_899TT | FC_900TT | FC_901TT | FC_902TT | FC_909TT | FC_910TT |
|---|---|---|---|---|---|---|---|
| 36 | 827.5 | 1152.4 | 778.8 | 11199.3 | 578.7 | 713 | 132.4 |
| 53 | 38.1 2517.5 | 1949.7 | 2221 | 29.8 | 185.9 | 21.5 | 57.2 |
| 76 | 2517.5 | 1009.8 | 3523.8 | 28603.2 | 2201.1 | 195.6 | 4744.8 |
| 242 | 39786.8 | 38117 | 29127.6 | 4032.5 | 43673.6 | 3478.5 | 1273.2 |
| 211 | 1160.7 | 132.9 | 79.4 | 26.8 | 251.4 | 488 | 8979.7 |
| | Fc_EA40386_954TT | Fc_EA40394_96 7TT | Fc_EA40395_968TT | Fc_EA40396_969TT | Fc_EA40397_970TT | Fc_EA40405_982TT | Fc_EA40406_983TT |
| 36 | 619.3 | 412.7 | 134 | 218.6 | 463 | 1233.6 | 234.6 |
| 53 | 15.8 | 31 | 23.8 | 55.9 | 65.5 | 31.6 | 120.8 |
| 76 | 4136.1 | 682.5 | 41.2 | 18.4 | 594.7 | 931 | 2232 |
| 242 | 969.6 | 5003.3 | 13867.1 | 2129.7 | 15742.7 | 4086.9 | 11985.6 |
| 211 | 20581.7 | 1509.8 | 2603.1 | 15427.4 | 369.3 | 30161.2 | 19869.7 |

Table 10. 5-Gene signature performance in 98 training samples

| | Tumor | Benign |
|---|---|---|
| Positive | 48 | 14 |
| Negative | 4 | 32 |
| Sensitivity | 92% (0.82, 0.97) | |
| Specificity | 70% (0.55, 0.81) | |

D. Cross Validation with the 74 Independent Thyroid Samples

74 independent thyroid samples were processed and profiled with the U133a chip, and the chip data for these two signatures are shown in the Table 11. The performances of the 4-gene and the 5-gene signatures were assessed with LDA. Both signatures gave equivalent performance in these samples compared to the 98 training samples. The sensitivity and specificity for both signatures are shown in Table 12, and the ROC curves are demonstrated in Figure 3a and 3b.

Table 11

| | Signal | | | | | | |
|---|---|---|---|---|---|---|---|
| SEQ ID | FA_987TT | FA_992TT | FA_993TT | FA_994TT | FA_995TT | FA_996TT | FA_998TT |
| 36 | 92.8 | 437.8 | 640.5 | 4152.5 | 714.7 | 254.8 | 303.4 |
| 53 | 15.8 | 422.8 | 25.2 | 29.5 | 258.9 | 45.6 | 26.8 |
| 76 | 485.1 | 2536.5 | 1708.6 | 288.3 | 643.3 | 605.6 | 341 |
| 242 | 686.5 | 971.4 | 24532.8 | 895.7 | 6424.8 | 842.1 | 905.1 |
| 211 | 28269.8 | 14689.4 | 357.5 | 33711.6 | 34943.2 | 16131 | 22376.1 |
| | FA_999TT | FA_1001TT | FA_1002TT | FA_1004TT | FA_1005TT | FA_1006TT | FA_1010TT |
| 36 | 63.7 | 22.3 | 55.6 | 408.5 | 128.3 | 353 | 442 |
| 53 | 30.9 | 31.8 | 39.6 | 70.8 | 37.9 | 38.9 | 119.6 |
| 76 | 110.7 | 90.2 | 253.5 | 159.1 | 1559.6 | 413.7 | 1222.5 |
| 242 | 1550.6 | 1689.3 | 256.5 | 1668 | 3921.5 | 1013.2 | 1837.3 |
| 211 | 26779.6 | 15870.7 | 24525.5 | 43040.2 | 25392.9 | 32641.9 | 20140.7 |
| | FA_1013TT | FA_1014TT | A_1017TT | FA_1018TT | FA_1020TT | FA_1023TT | FA_1024TT |
| 36 | 453.5 | 333.7 | 179.6 | 146.6 | 417.3 | 75.1 | 129.2 |
| 53 | 39.8 | 21.4 | 99.9 | 23.7 | 26.8 | 62.3 | 22.2 |
| 76 | 333.7 | 1231.2 | 241.1 | 131 | 1435.3 | 1491.6 | 260.8 |
| 242 | 1459.4 | 415 | 1696.3 | 287.1 | 617.5 | 1932.6 | 1689.2 |
| 211 | 7911.3 | 19359.4 | 18089 | 18222.8 | 30038.8 | 9053 | 15611.2 |
| | FA_1026TT | FA_1027TT | FA_1028TT | FA_1029TT | FA_1030TT | FA_1031TT | FA_1032TT |
| 36 | 281.1 | 130.5 | 150.4 | 629.1 | 760.4 | 191.6 | 1671.5 |
| 53 | 125.5 | 62.3 | 118.2 | 230.7 | 420.5 | 27.7 | 519 |
| 76 | 77.4 | 111.9 | 184 | 2100.9 | 1741.3 | 338.1 | 3643.5 |
| 242 | 1091.6 | 754.3 | 705.9 | 684.7 | 372.4 | 1848.8 | 3094.7 |
| 211 | 21979.1 | 7890.5 | 26226 | 15344.1 | 10368 | 30439.9 | 23943.3 |

(continued)

| | | | | | | | |
|---|---|---|---|---|---|---|---|
| | FA_1034TT | FA_1035TT | FA_1073TT | FA_1074TT | FA_1075TT | FA_1076TT | PC_984TT |
| 36 | 24.8 | 119.9 | 111.7 | 1010.9 | 26 | 224.5 | 303 |
| 53 | 29.6 | 30.4 | 366.7 | 96.9 | 94.3 | 31 | 37.2 |
| 76 | 289.3 | 239.7 | 167.4 | 326.8 | 818.9 | 248.5 | 198.3 |
| 242 | 1482.1 | 590.9 | 4842.5 | 2815.5 | 2726.8 | 754.8 | 785.1 |
| 211 | 38135.4 | 30510.6 | 29406.4 | 31075.4 | 20833.9 | 34960.4 | 24938.2 |
| | PC_986TT | PC_988TT | PC_989TT | PC_1039TT | PC_1040TT | PC_1041T T | PC_1042T T |
| 36 | 676.3 | 391.7 | 264.9 | 735.7 | 524.7 | 769.9 | 1125 |
| 53 | 32.5 | 395.6 | 305.5 | 655 | 1153.9 | 2194.1 | 111.5 |
| 76 | 287.9 | 1384.8 | 888.3 | 1048.4 | 1381.1 | 412.6 | 1384.9 |
| 242 | 2450.7 | 2243.2 | 698.6 | 20366.4 | 32090.9 | 27480.9 | 1915.7 |
| 211 | 2689.5 | 19903.2 | 32075.9 | 9197.7 | 152.2 | 1862.2 | 10375.9 |
| | PC_1043TT | PC_1044TT | PC_1045TT | PC_1046TT | PC_1047TT | PC_1048T T | PC_1049T T |
| 36 | 1013.6 | 1023.8 | 809.8 | 1811.1 | 1546.2 | 481.8 | 2491.9 |
| 53 | 730.7 | 2965.3 | 2533.7 | 2052.8 | 756.1 | 988.3 | 2000.6 |
| 76 | 1977.4 | 3380.4 | 422 | 4343.9 | 3570.2 | 1786.8 | 1619.4 |
| 242 | 3907.5 | 20074.8 | 41761.2 | 33253.5 | 28124.7 | 30516 | 21324.5 |
| 211 | 7015.5 | 11.5 | 136.1 | 59.1 | 3841.7 | 31.1 | 1476 |
| | PC_1050TT | PC_1051TT | PC_1053TT | PC_1054TT | PC_1055TT | PC_1059T T | PC_1060T T |
| 36 | 443.9 | 669 | 477.1 | 952.8 | 645.3 | 660.2 | 648.5 |
| 53 | 4037.8 | 1290.7 | 933.9 | 1336.9 | 2123.2 | 1353.8 | 66.9 |
| 76 | 856.7 | 1039 | 463.2 | 1399.8 | 4385 | 365.8 | 160.7 |
| 242 | 1261.6 | 58258.5 | 26004.9 | 21219.8 | 12760.8 | 26084.4 | 3277.6 |
| 211 | 1150.2 | 375.9 | 73.2 | 2575.8 | 400.5 | 24.2 | 1637.7 |
| | PC_1061TT | PC_1062TT | FC_1037TT | FC_1077TT | FC_1078TT | FC_1079T T | FC_1082T T |
| 36 | 3523.2 | 1912.5 | 234.2 | 128.3 | 825.1 | 297.5 | 1447.6 |

(continued)

| | | | | | | | |
|---|---|---|---|---|---|---|---|
| | PC_1061TT | PC_1062TT | FC_1037TT | FC_1077TT | FC_1078TT | FC_1079T T | FC_1082T T |
| 53 | 2348.3 | 975.7 | 97.6 | 46.2 | 15.8 | 38.5 | 254.6 |
| 76 | 4994.9 | 3891.7 | 5559.8 | 252 | 1103.5 | 700 | 1754.7 |
| 242 | 15397.4 | 2006.1 | 1826 | 2407.5 | 22661.8 | 1595.5 | 713.7 |
| 211 | 3379.4 | 2201.9 | 41570 | 15804.5 | 3572.1 | 4377.9 | 16863.7 |
| | PC-FV_1052TT | PC-FV_1064TT | PC-FV_1065TT | PC-FV_1066TT | PC-FV_1067TT | PC-FV_1068TT | PC-FV_1069TT |
| 36 | 1993.3 | 230.6 | 1128.8 | 708.4 | 1348 | 331.4 | 33.2 |
| 53 | 2450.3 | 72.7 | 610.3 | 72.5 | 146.4 | 1073.7 | 21.5 |
| 76 | 2075 | 451.3 | 1350.2 | 922.5 | 1743.7 | 950.5 | 332.8 |
| 242 | 21494 | 7110.9 | 10228.8 | 2206.3 | 3319.2 | 28034.2 | 430.1 |
| 211 | 1581.5 | 29936.9 | 1594.7 | 5693.1 | 11288.8 | 573.6 | 12485.5 |
| | PC-FV_1071TT | PC-FV_1072TT | PC-FV_1080TT | PC-FV_1081TT | | | |
| 36 | 148.9 | 332.2 | 1288.7 | 672 | | | |
| 53 | 1836.9 | 1450.3 | 2221 | 22.9 | | | |
| 76 | 4053 | 1628.6 | 2275.9 | 62.6 | | | |
| 242 | 1810.6 | 26396.9 | 683.4 | 984 | | | |
| 211 | 8.9 | 24 | 3416.7 | 2630.8 | | | |

Table 12. 4-Gene and 5-gene signatures performance in 74 validation samples

4-Gene Signature

| | Tumor | Benign |
|---|---|---|
| Positive | 33 | 12 |
| Negative | 3 | 26 |
| Sensitivity | 92% (0.70, 0.97) | |
| Specificity | 68% (0.53, 0.81) | |

5-Gene Signature

| | Tumor | Benign |
|---|---|---|
| Positive | 33 | 9 |
| Negative | 3 | 29 |
| Sensitivity | 92% (0.78, 0.97) | |
| Specificity | 76% (0.61, 0.87) | |

E. Control Gene Marker Identification

With the 98 thyroid samples and 12 PBL samples we selected two groups of genes as sampling control. One group consists of genes that are expressed in thyroid but not in PBL, the second group includes genes that are expressed in PBL but not in thyroid. The full gene list and corresponding chip data are shown in Tables 13a and 13b. From these genes we selected six genes that are abundant and the differentiation between thyroid and PBL is relatively large. Their expression profile was validated in the 74 independent thyroid samples. The identities of these six genes are listed in Table 14 and their chip data are shown in Tables 15a and 15b.

Table 13a

13a1

| | ThyMixBen_......_Signal | | | | | |
|---|---|---|---|---|---|---|
| SEQ ID | 02014_40062_H 133A_800TT | 02014_40063_H 133A_801TT | 02014_40064_ H133A_802TT | 02014_40065_ H133A_804TT | 02014_40066_ H133A_805TT | 02014_40067_ H133A_806TT |
| 2 | 2789.6 | 2676.2 | 3009.2 | 2957.5 | 3644.5 | |
| 3 | 6346.1 | 794.7 | 623.9 | 772 | 2977.3 | 622.7 |
| 5 | 845.5 | 3039.1 | 4452.6 | 2982.5 | 8283.2 | 1627.6 |
| 6 | 2730.4 | 1108.5 | 1155.6 | 2201.2 | 1954.3 | 1750 |
| 9 | 2248.9 | 12094.5 | 7529.2 | 2827.3 | 7701.7 | 1548.3 |

| | | | | | | |
|---|---|---|---|---|---|---|
| 12 | 10625.3 | 3137.2 | 2382.7 | 5727.8 | 6238 | 4685.1 |
| 18 | 3171 | 5947.9 | 13241.7 | 10294.9 | 7849.3 | 4037.4 |
| 22 | 2715.2 | 9522.5 | 6429.7 | 2590.9 | 5294.3 | 6490.4 |
| 25 | 19159.3 | 1467.3 | 550.1 | 2838 | 11294.7 | 1374.2 |
| 28 | 722.7 | 835.1 | 727.1 | 568.1 | 1336.7 | 740.7 |
| 32 | 4160.6 | 8115.2 | 4061.8 | 6625.3 | 7348.9 | 3964.1 |
| 39 | 314.3 | 1243.1 | 1486.8 | 742.5 | 1027.7 | 1185.9 |
| 74 | 323.9 | 1227.6 | 1965.1 | 1281.5 | 760.1 | 505.4 |
| 78 | 250.8 | 677 | 1029.5 | 403.7 | 795.2 | 355 |
| 143 | 1337.3 | 1339.1 | 2607.9 | 1758.8 | 1933.5 | 1809 |
| 174 | 1263.1 | 4430.7 | 6041.6 | 3543.2 | 7346 | 3920.5 |
| 175 | 1996.3 | 1829.5 | 3632.7 | 1646.5 | 2847.5 | 3054.2 |
| 191 | 7108.4 | 848.3 | 2063 | 1133.9 | 1482.4 | 1149 |
| 212 | 258.2 | 1405.8 | 1196.9 | 882.4 | 1296.8 | 1790 |
| 222 | 20586.8 | 1371.2 | 2411.9 | 2573.9 | 1228.6 | 1342 |
| 233 | 1410.6 | 14126.8 | 3020.4 | 2186.1 | 8439.4 | 6024.4 |
| 234 | 5048.1 | 2183.3 | 3215.5 | 4103.9 | 4096.9 | 2220.2 |
| 237 | 905 | 3397.9 | 2525.1 | 2135.4 | 2822 | 2492.3 |
| 238 | 198 | 1937.7 | 1289.5 | 804.5 | 1538.6 | 1454.7 |
| 245 | 143.7 | 1598.5 | 1964.2 | 897.9 | 2276.6 | 1348.2 |
| 250 | 1300.9 | 777 | 1405.9 | 1466.4 | 1301.3 | 1865.3 |
| 252 | 945.6 | 1547.3 | 1210.2 | 1357 | 1437.1 | 1141.1 |
| 264 | 455.1 | 988.1 | 932.8 | 631.7 | 1253.9 | 845.4 |
| 290 | 345.6 | 327.2 | 533.1 | 470.2 | 700.5 | 231.9 |
| 294 | 1043 | 3655.8 | 4435.4 | 3563.9 | 4377.4 | 2734.8 |
| 296 | 845.5 | 1242.5 | 1068.6 | 1506.5 | 2423.4 | 1527.3 |
| 342 | 771.4 | 535.9 | 998.5 | 922.5 | 1108.1 | 605.4 |

13a2

| | Signal | | | | | |
|---|---|---|---|---|---|---|
| SEQ ID | ThyMixBen_02014_40068_H133A_807TT | 02014_40198_H133A_871TT | 02014_40321_H133A_913TT | 02014_40322_H133A_914TT | 02014_40323_H133A_915TT | 02014_40324_H133A_917TT |
| 2 | 1749.6 | 1571.1 | 2634.4 | 2419.2 | 2697.4 | 2901.1 |
| 3 | 377.2 | 508 | 2322.7 | 259.8 | 1123.4 | 875.2 |
| 5 | 2762.9 | 1795.8 | 6670.2 | 2094.7 | 11425.7 | 4957.2 |
| 6 | 1280.1 | 2033.3 | 1910.3 | 2543.7 | 2173.2 | 1358.7 |
| 9 | 3346.8 | 548.3 | 11398.5 | 953.4 | 2085.2 | 10684.4 |
| 12 | 6927.1 | 4060.5 | 6664.4 | 2730.5 | 4322.8 | 2616.2 |
| 18 | 7418.9 | 14860.4 | 3134.7 | 26576.3 | 9585.4 | 8588.9 |
| 22 | 1797.5 | 744.5 | 7334.3 | 317.9 | 5120.1 | 8611.7 |
| 25 | 1245.8 | 815.8 | 7201.1 | 253.7 | 2882.9 | 1053.4 |
| 28 | 301.1 | 640.8 | 1325.9 | 409.2 | 762.7 | 838.8 |
| 32 | 2992.4 | 3260.2 | 3588.1 | 792.5 | 5413.2 | 2581 |
| 39 | 435.8 | 958.1 | 1152.1 | 499.6 | 556.9 | 898.6 |
| 74 | 689.2 | 1453.7 | 491.7 | 736.6 | 986.9 | 1217 |
| 78 | 160.1 | 383 | 151.2 | 172.9 | 597.4 | 745.6 |
| 143 | 1338.8 | 1466.6 | 1069.9 | 2070.9 | 2377.2 | 1976.3 |
| 174 | 2464.3 | 1616.3 | 829.8 | 1365.2 | 4938 | 4804.3 |
| 175 | 1701 | 1511.5 | 1101.1 | 2325.4 | 2530.2 | 2237.6 |
| 191 | 763.6 | 2383.3 | 2013.2 | 1274.2 | 601.2 | 1100.2 |
| 212 | 481 | 1131.9 | 912.5 | 663.2 | 666.4 | 1081 |
| 222 | 280.8 | 1574 | 4944.1 | 342.2 | 1225.9 | 449 |

| | | | | | | |
|---|---|---|---|---|---|---|
| 233 | 1289.5 | 1272.4 | 6285.6 | 866.8 | 1839.3 | 5797.4 |
| 234 | 1645.4 | 3271.1 | 2263.3 | 1789.3 | 6497.4 | 3416 |
| 237 | 1732.6 | 879.6 | 3780 | 1345.3 | 2154.2 | 2802.8 |
| 238 | 493.5 | 893.1 | 1098.7 | 405.9 | 592.8 | 1326.4 |
| 245 | 661.9 | 1378.3 | 1244.1 | 645.4 | 1124.2 | 1171.1 |
| 250 | 736.7 | 1305.6 | 633 | 1409.4 | 1368.9 | 1146.7 |
| 252 | 1145.8 | 1986 | 1203.2 | 1323.5 | 1544.6 | 757 |
| 264 | 462.7 | 891.3 | 629.4 | 649.7 | 872.1 | 1319.5 |
| 290 | 295.8 | 697.1 | 987.8 | 819.9 | 440.6 | 268.7 |
| 294 | 1909.1 | 1128.4 | 924.1 | 1519.2 | 3225.3 | 2954.9 |
| 296 | 898.4 | 1232.2 | 925.1 | 1290.3 | 738.1 | 1069.6 |
| 342 | 482.4 | 712.6 | 476.1 | 694.8 | 801.2 | 605.7 |

13a3

| | Signal | | | | | |
|---|---|---|---|---|---|---|
| SEQ ID | 02014_40325_H133A_918TT | 02014_40326_H133A_919TT | ThyFolBen_02014_40079_H133A_818TT | ThyFolBen_02014_40080_H133A_819TT | ThyFolBen_02014_40081_H133A_820TT | ThyFolBen_02014_40082_H133A_821TT |
| 2 | 3457.7 | 2769.1 | 4234.5 | 5589.2 | 3035.3 | 2681.6 |
| 3 | 920.5 | 1289.3 | 627 | 690.9 | 585.4 | 610 |
| 5 | 1805.2 | 5184.1 | 1487.2 | 1942 | 2663.8 | 1231.8 |
| 6 | 1560.8 | 1519.3 | 996.2 | 1140.3 | 2093.4 | 841.6 |
| 9 | 11354.8 | 9921.9 | 3894 | 2165.4 | 7467 | 7685.6 |
| 12 | 7531.7 | 6887.2 | 1379.1 | 1292.4 | 5085.1 | 3585.9 |
| 18 | 8220.1 | 5452.2 | 7762.8 | 7277.4 | 5374.9 | 2379.1 |
| 22 | 4750.8 | 5739.1 | 5655.3 | 7618.6 | 6227.9 | 1551.9 |
| 25 | 1557.9 | 3017.5 | 1200.4 | 881.1 | 1279.8 | 406.4 |
| 28 | 886.6 | 1189.2 | 731.4 | 681.6 | 1225.7 | 473.9 |
| 32 | 4134.1 | 10964.4 | 927.6 | 3075.2 | 2775 | 608.8 |
| 39 | 1259.2 | 1022.1 | 1423.7 | 1632.9 | 899.5 | 688.9 |
| 74 | 741.8 | 948.9 | 770 | 674.6 | 1139.7 | 213 |
| 78 | 447.9 | 488.2 | 790.6 | 602.8 | 776.3 | 332.1 |
| 143 | 3165.1 | 1865.5 | 2330.7 | 1160.7 | 1676 | 1672.4 |
| 174 | 607.5 | 2151.8 | 4026.2 | 4702.6 | 10677.1 | 3826.8 |
| 175 | 5358.5 | 2402.7 | 3765.6 | 1231.9 | 2531.9 | 2307.7 |
| 191 | 2685.9 | 1183.6 | 1661.4 | 1657.1 | 1377.8 | 368.2 |
| 212 | 1306.3 | 1072.1 | 2181.8 | 2035.6 | 954.2 | 1663.4 |
| 222 | 2332.8 | 431.2 | 1015.6 | 1651.7 | 3755.4 | 1669.9 |
| 233 | 5181.8 | 12377 | 3400.9 | 4679.3 | 2476 | 1506.6 |
| 234 | 3760.2 | 1988.2 | 3527.4 | 3971.4 | 4526.5 | 997.6 |
| 237 | 2712 | 3073.8 | 1930.6 | 3521.8 | 2951.3 | 2673.5 |
| 238 | 1552.2 | 1636.1 | 1333.5 | 837.6 | 1069.7 | 1860.5 |
| 245 | 1722.2 | 1981.5 | 1978.8 | 2170 | 763.6 | 1392.9 |
| 250 | 1502.7 | 1094 | 1083.3 | 710.8 | 864.9 | 1823.2 |
| 252 | 1609.9 | 1679.3 | 1599.4 | 1463.9 | 1199.9 | 1019.2 |
| 264 | 860.1 | 835.6 | 1451.1 | 1315.6 | 1221.6 | 936.8 |
| 290 | 773.4 | 490.4 | 218.3 | 507.2 | 648.3 | 294.6 |
| 294 | 555.3 | 2490.7 | 1854.9 | 2997.4 | 7108.2 | 2160.7 |
| 296 | 1240.1 | 1863 | 1139.6 | 1245.4 | 1172.5 | 1318.3 |
| 342 | 738.7 | 509.3 | 1018.4 | 728.8 | 977.2 | 710.6 |

13a4

Signal

| SEQ ID | ThyFolBen_02014_40 083_H133A_822TT | fa__EA40173_V DX842TT | fa__EA40191_VDX862TT | fa__EA40192 _VDX863TT | fa__EA40193 _VDX864TT | fa__EA40194 _VDX865TT |
|---|---|---|---|---|---|---|
| 2 | 3403.4 | 3423.5 | 2246.6 | 2723.3 | 4130.4 | 2828.3 |
| 3 | 644.4 | 950.1 | 1313.2 | 344.4 | 1731.1 | 2576.9 |
| 5 | 3439.5 | 754.3 | 2119 | 3324.7 | 1663.9 | 4423 |
| 6 | 1373.7 | 987.7 | 834.7 | 1399.9 | 1115.4 | 1872.1 |
| 9 | 7457.5 | 2730.9 | 9307.8 | 7048.5 | 3775.7 | 6931.3 |
| 12 | 3978.9 | 9462.8 | 777.5 | 4280.8 | 4821 | 2268.2 |
| 18 | 6386.9 | 1591.2 | 9546.6 | 4979.5 | 5005.2 | 4409.5 |
| 22 | 2652.5 | 7124.7 | 11054.7 | 4669.9 | 4776.9 | 5782.5 |
| 25 | 1887.7 | 470.8 | 703.9 | 714.5 | 445.9 | 698.2 |
| 28 | 548.5 | 562.4 | 596.8 | 915.9 | 1194.1 | 1041 |
| 32 | 4425.5 | 2120.3 | 1084.1 | 2638.9 | 2593.1 | 1919.9 |
| 39 | 1103.1 | 1110.2 | 898.4 | 1375.1 | 1520.4 | 1744.1 |
| 74 | 285.9 | 895.8 | 532.1 | 841.2 | 1938.6 | 1109.5 |
| 78 | 643.1 | 346.6 | 533.3 | 680.5 | 621.5 | 385.8 |
| 143 | 1979.9 | 3302.9 | 1686.6 | 2406.6 | 2255.4 | 3419.1 |
| 174 | 5408.1 | 608.2 | 5072.8 | 5907.1 | 2956.1 | 2670.2 |
| 175 | 2650.3 | 6596.8 | 2600.8 | 4704.8 | 3123 | 5286.8 |
| 191 | 667.7 | 2665.7 | 1382.1 | 740.2 | 1133.3 | 3678.2 |
| 212 | 1357.8 | 2545.2 | 1590.2 | 1326.3 | 1135.1 | 1079 |
| 222 | 2239.9 | 471 | 548.8 | 584.1 | 899.4 | 1002.2 |
| 233 | 7318.7 | 11617.9 | 10868.4 | 6925.9 | 5416.9 | 14212.9 |
| 234 | 2395.9 | 2832.7 | 6005.3 | 2493.8 | 2128.8 | 6818.7 |
| 237 | 2275.3 | 2639.3 | 2406.6 | 2595.1 | 1857.2 | 2546.1 |
| 238 | 1181.8 | 2124.4 | 1586.7 | 2125.3 | 2039.5 | 1859.1 |
| 245 | 2082.5 | 2302.1 | 2846.4 | 2280.8 | 3973.8 | 1520.6 |
| 250 | 1684 | 1718 | 862.9 | 2782.6 | 1973.4 | 1594.3 |
| 252 | 1166.7 | 1579.6 | 590.4 | 1623.2 | 1735.2 | 1584.3 |
| 264 | 654.2 | 1604.2 | 3062.9 | 1460.8 | 1510.1 | 2018.9 |
| 290 | 342 | 431.8 | 458.8 | 602.3 | 745 | 977.6 |
| 294 | 2978.3 | 651.5 | 3533.7 | 4065.9 | 2720.4 | 2292.8 |
| 296 | 2132.4 | 1762.9 | 815.3 | 1040.3 | 1787.6 | 932.6 |
| 342 | 1058.5 | 488.3 | 475.4 | 759.3 | 658.3 | 851.2 |

13a5

| | | | Signal | | | |
|---|---|---|---|---|---|---|
| SEQ ID | fa__EA40195_V DX866TT | fa__EA40196_V DX867TT | fa__EA40197_VD X869TT | fa__EA40219 _VDX907TT | fa__EA40220 _VDX908TT | 02014_40327_ H133A_920TT |
| 2 | 3243.6 | 2969.9 | 3761.4 | 3352.1 | 2486.7 | 3109.7 |
| 3 | 728.9 | 548.2 | 531.1 | 796 | 1204.6 | 981.6 |
| 5 | 2392.7 | 6268.7 | 3870.9 | 10216 | 3635.2 | 11319.3 |
| 6 | 1534 | 965.4 | 2782.8 | 2638.6 | 1155.7 | 2100.9 |
| 9 | 8238.3 | 8829.9 | 4175.6 | 1650.4 | 7363.2 | 3172 |
| 12 | 3334.6 | 2752 | 3382.5 | 3589.1 | 6717.7 | 3191.1 |
| 18 | 9146.3 | 8150.5 | 7796.3 | 8021.5 | 8185 | 8907.1 |
| 22 | 4665.5 | 8163.5 | 3812 | 4001.7 | 5539.5 | 6346.7 |
| 25 | 555.6 | 524.6 | 1592.8 | 2057.7 | 4103.3 | 886.1 |
| 28 | 1115.3 | 604 | 918.9 | 1190.9 | 999.1 | 998.2 |
| 32 | 1089.3 | 1808.7 | 1245.6 | 4202 | 7454.6 | 1100.2 |
| 39 | 1606.8 | 1418.2 | 1392.4 | 1443.3 | 949.8 | 1388.7 |
| 74 | 638.2 | 2085.2 | 694.7 | 1778.1 | 557.7 | 1486.9 |
| 78 | 480.6 | 603.6 | 959.8 | 1000.3 | 476 | 721.1 |

| | | | | | | |
|---|---|---|---|---|---|---|
| 143 | 3003 | 2033.2 | 3441.7 | 3224.1 | 2461.3 | 2701.2 |
| 174 | 3322 | 4573.5 | 4168.2 | 5982 | 3022.2 | 5710.7 |
| 175 | 4747.7 | 2573.2 | 5834.5 | 4666.3 | 3540.8 | 3751 |
| 191 | 2724.1 | 726 | 831.2 | 1991.3 | 1556.8 | 643.4 |
| 212 | 2362.7 | 1705.9 | 2344.8 | 1259.8 | 1288.5 | 1033.6 |
| 222 | 271.7 | 2039.4 | 683.4 | 1370.8 | 728.8 | 1675.2 |
| 233 | 10892.9 | 6981.9 | 10048.5 | 6773.9 | 15803 | 3520.9 |
| 234 | 4015.4 | 3229.5 | 4734.4 | 7233.3 | 2712.8 | 8148.3 |
| 237 | 2159.9 | 2415.2 | 2194.8 | 2990.7 | 2565.8 | 2364 |
| 238 | 1451 | 1111.6 | 1037.4 | 914.9 | 1634.9 | 1078.9 |
| 245 | 2059.1 | 2548.6 | 2180.9 | 2093.2 | 1730 | 1329.4 |
| 250 | 3534.3 | 993.3 | 2513.3 | 878.9 | 686.3 | 980.4 |
| 252 | 1589.8 | 1334.1 | 2762.4 | 1267.3 | 1857.6 | 1608.2 |
| 264 | 1335.1 | 1560.1 | 1656.9 | 942.7 | 950.4 | 1076.1 |
| 290 | 496.3 | 626.2 | 584.9 | 549.5 | 309.7 | 613.4 |
| 294 | 2869.4 | 4842.4 | 2762.9 | 4119.2 | 2299.8 | 3497.8 |
| 296 | 1414.4 | 882.4 | 1284 | 1106.4 | 1493.4 | 888.5 |
| 342 | 595.6 | 660.6 | 1442.5 | 1050.3 | 607.5 | 1278.6 |

13a6

| | Signal | | | | | |
|---|---|---|---|---|---|---|
| SEQ ID | 02014_40332_ H133A_938TT | 02014_40334_ H133A_941TT | 02014_40335_ H133A_940TT | EA02014_40374 _H133A_921TT | EA02014_40376 _H133A_923TT | EA02014_40377 _H133A_924TT |
| 2 | 1963.7 | 2662.2 | 2416.5 | 3047.7 | 2043.6 | 3322 |
| 3 | 942.5 | 342 | 749.1 | 1300.6 | 549.3 | 1182 |
| 5 | 2218 | 3903.1 | 3011.1 | 1669.8 | 1508.3 | 1453.3 |
| 6 | 939.5 | 1239.9 | 1853.8 | 837.7 | 1477.8 | 1129.4 |
| 9 | 5245.3 | 1988.9 | 1165.8 | 4137 | 8735.7 | 4453.3 |
| 12 | 2500.4 | 2100 | 3597.7 | 5716.2 | 6694.6 | 3667.8 |
| 18 | 5929.7 | 7819.7 | 5335.4 | 4017.5 | 2899.7 | 5013.3 |
| 22 | 2265.8 | 6298.2 | 2112.7 | 3386.6 | 4519.5 | 4186.6 |
| 25 | 1160.2 | 916.5 | 614.7 | 1055.5 | 1184.4 | 828.4 |
| 28 | 617.6 | 408.5 | 740.7 | 477.3 | 701.3 | 550.8 |
| 32 | 2918.8 | 3242.3 | 3083.9 | 1411.4 | 4079.5 | 2063.5 |
| 39 | 1356.6 | 876.8 | 1855 | 1231.3 | 982.1 | 1248 |
| 74 | 1102.1 | 826.7 | 1384.4 | 296.9 | 632.9 | 787.7 |
| 78 | 361.5 | 383.5 | 482.7 | 578.5 | 351.8 | 389.9 |
| 143 | 1394.9 | 2889.2 | 1838.1 | 1776 | 1563 | 3881.9 |
| 174 | 2198.3 | 1887.5 | 3921.8 | 3076.4 | 1858.1 | 1967.3 |
| 175 | 1391.4 | 3677.8 | 2169.3 | 2169 | 2397.2 | 5896.8 |
| 191 | 1235.1 | 364.7 | 1283.5 | 616.4 | 1037.7 | 1957 |
| 212 | 1625.4 | 871.1 | 1083.8 | 1086.2 | 1450.7 | 981.1 |
| 222 | 503.4 | 638.5 | 320.1 | 1707.8 | 1089.4 | 640.8 |
| 233 | 8499.4 | 1999.9 | 4012.1 | 3345.8 | 10991.6 | 11095.5 |
| 234 | 2067.7 | 2973.7 | 2630.4 | 2027.1 | 1597 | 3240 |
| 237 | 2763.1 | 1501.1 | 2724 | 1997.9 | 2249.3 | 2941.1 |
| 238 | 1055.6 | 1055.7 | 1596.5 | 1035.6 | 1810.9 | 1257.5 |
| 245 | 751.4 | 1076.1 | 915.2 | 2714.4 | 2120.1 | 1553.6 |
| 250 | 1050.6 | 1474.3 | 2517.1 | 1428.1 | 1169.4 | 2857.1 |
| 252 | 987.8 | 1382.5 | 2070.7 | 881.9 | 1589.3 | 2217.2 |
| 264 | 1278.3 | 1261.1 | 826.2 | 865.3 | 1055.9 | 1034.7 |
| 290 | 786.4 | 936.8 | 952.1 | 312.4 | 385.7 | 1231.1 |
| 294 | 2197.5 | 2064.7 | 2836.2 | 2573.5 | 1835.4 | 2657.6 |

| | | | | | | |
|---|---|---|---|---|---|---|
| 296 | 635.2 | 572.8 | 700.9 | 1768.2 | 1426.3 | 2462.5 |
| 342 | 718.6 | 866.8 | 735.7 | 770.7 | 565.3 | 1695.1 |

13a7

| SEQ ID | 40378_H133A _925TT | 40379_H133A_ 926TT | EA02014_ 40380_H133A_9 27TT | Signal 40387_H133A_9 55TT | 40388_H133A_9 56TT | 40389_H133A_9 57TT |
|---|---|---|---|---|---|---|
| 2 | 3274 | 3025.4 | 2424.9 | 2302.6 | 2724.6 | 2641.3 |
| 3 | 1545.1 | 1151.1 | 687.6 | 424.7 | 420.6 | 609.2 |
| 5 | 3661.8 | 1323.2 | 7949.9 | 4860 | 4059.3 | 4895.3 |
| 6 | 1371.5 | 1220.8 | 1831 | 2330.9 | 1642.8 | 1963.8 |
| 9 | 10387.7 | 11670.1 | 6402.7 | 3105.3 | 4256.6 | 8744.8 |
| 12 | 5829.9 | 6903.8 | 4783.9 | 2874.5 | 3494.1 | 2451 |
| 18 | 6648 | 4053.1 | 7410.2 | 4600.2 | 3912.9 | 16235.3 |
| 22 | 6859.7 | 6621.2 | 3008.1 | 1369.9 | 2480.5 | 4685 |
| 25 | 1035.6 | 3448.2 | 1347.1 | 365.3 | 1587.2 | 816.8 |
| 28 | 1087 | 917.1 | 747.8 | 1057 | 1169.8 | 974.1 |
| 32 | 5212.9 | 6841.6 | 3282.5 | 2814.2 | 4083.1 | 8121.2 |
| 39 | 1228.7 | 1245.7 | 762.4 | 904.9 | 1136.2 | 1862.7 |
| 74 | 823.9 | 721.6 | 1460.4 | 2781.4 | 970.2 | 839 |
| 78 | 713.8 | 377.6 | 621.3 | 212.8 | 421.3 | 891.4 |
| 143 | 1953.5 | 2925.7 | 2861.8 | 1243.3 | 2335.1 | 2564.3 |
| 174 | 3029 | 1818.4 | 5264.1 | 3925.2 | 4789.1 | 4553.5 |
| 175 | 3229.1 | 4827.2 | 3821.1 | 1561.7 | 4603.8 | 4098.3 |
| 191 | 1112.4 | 5308.2 | 841 | 861.6 | 865.2 | 1964.9 |
| 212 | 890.9 | 915.6 | 729.1 | 1264.1 | 1494.2 | 1177.2 |
| 222 | 480.1 | 2895.3 | 605.6 | 145.2 | 425.3 | 1712.4 |
| 233 | 6680.7 | 9190.5 | 2236.9 | 441.5 | 3626.6 | 11326.8 |
| 234 | 1871.2 | 4194.1 | 4428.8 | 1260.8 | 1599 | 4627.7 |
| 237 | 2758.4 | 2108.7 | 2070.1 | 1655.9 | 2140.3 | 2820.6 |
| 238 | 1210.7 | 1289.5 | 774.3 | 1017.9 | 1645.6 | 1150.6 |
| 245 | 2369.7 | 1585.8 | 785.1 | 1808.4 | 2014.9 | 1185.6 |
| 250 | 971.8 | 1387.3 | 1193.5 | 2014.8 | 2033 | 608.6 |
| 252 | 1417.1 | 990.9 | 1186.9 | 1561.4 | 2006.1 | 1838 |
| 264 | 1089.9 | 538.3 | 758.6 | 1340.4 | 1171.9 | 933.1 |
| 290 | 565.1 | 601.8 | 485.3 | 597.5 | 511.7 | 852.7 |
| 294 | 2574.6 | 1541.3 | 3862.3 | 2639.9 | 3968.8 | 3955.7 |
| 296 | 1821.8 | 1115.3 | 1015.1 | 1379.4 | 1444.1 | 867.3 |
| 342 | 621 | 757.4 | 791.6 | 504.5 | 671.6 | 1320.3 |

13a8

| SEQ ID | EA02014_4039 0_H133A_959T T | EA02014_403 91_H133A_96 0TT | EA02014_40 392_H133A_ 961TT | Signal EA02014_403 93_H133A_96 2TT | ThyPapCan_0201 4_40090_H133A_ 829TT | ThyPapCan_02014_ 40091_H133A_830T T@IC |
|---|---|---|---|---|---|---|
| 2 | 1817.9 | 1937.6 | 2632.3 | 2613.7 | 2167.7 | 1372.7 |
| 3 | 605.6 | 546.8 | 697.5 | 480.4 | 1793.5 | 1236.1 |
| 5 | 8452.7 | 11532.9 | 5852.5 | 5194.8 | 3735.8 | 2822.7 |
| 6 | 2508.7 | 1035.1 | 1581.2 | 1561.5 | 1568.4 | 1723.3 |
| 9 | 1557.3 | 17159.3 | 4950.2 | 1272.3 | 3597.7 | 3243.8 |
| 12 | 8423.6 | 3364.4 | 3626.3 | 1198.7 | 5938.4 | 3878.6 |
| 18 | 9560.7 | 5478.7 | 8997.6 | 4601.2 | 6673.3 | 6322.9 |
| 22 | 5745.2 | 8584.6 | 5849.4 | 2275.2 | 4413.3 | 2539.9 |

| | | | | | | |
|---|---|---|---|---|---|---|
| 25 | 2003.7 | 619.6 | 797.7 | 887.6 | 2860.8 | 3257.2 |
| 28 | 1280.7 | 1023.3 | 810.5 | 604.5 | 703.2 | 592.2 |
| 32 | 5819.5 | 1430 | 6760.1 | 736.3 | 5057.7 | 6877.1 |
| 39 | 844.6 | 1193.7 | 2035 | 1643.1 | 906.4 | 977.6 |
| 74 | 1794.4 | 871.9 | 1342.7 | 1207.3 | 873.7 | 438.1 |
| 78 | 1290.5 | 705.2 | 582.1 | 644.6 | 514.2 | 463 |
| 143 | 3606.5 | 1206.5 | 2610 | 3369.8 | 2462.6 | 1861.9 |
| 174 | 5367.9 | 3197 | 2890.4 | 2641.3 | 4481.7 | 4198.6 |
| 175 | 6112 | 1232.1 | 3258.2 | 5085.3 | 3246.9 | 2442.8 |
| 191 | 628.6 | 681.7 | 1024.6 | 538.5 | 913.3 | 723.7 |
| 212 | 762 | 850.9 | 1201 | 690.9 | 797.9 | 784 |
| 222 | 3380.6 | 2257.2 | 1397.9 | 127.7 | 892.4 | 646.7 |
| 233 | 1273.2 | 3459.2 | 5807.2 | 1846.1 | 4170.6 | 4565.3 |
| 234 | 4522.8 | 4556.7 | 4306.9 | 2687.8 | 3024.6 | 3006.1 |
| 237 | 1813.8 | 2740.9 | 2387.6 | 1999.7 | 1879.2 | 1708 |
| 238 | 1451 | 897.7 | 1411.1 | 1416.3 | 966.6 | 803.6 |
| 245 | 841.7 | 1542 | 1434.2 | 1628.4 | 1415.8 | 1521.5 |
| 250 | 834.9 | 839.6 | 1111.7 | 2545.8 | 1268.4 | 993.6 |
| 252 | 1612 | 1068.9 | 1122.2 | 1770.9 | 1379.1 | 1201.1 |
| 264 | 1720.7 | 1090.3 | 766.9 | 838.8 | 728.4 | 1170.1 |
| 290 | 548.7 | 268.4 | 292.1 | 366.4 | 284.9 | 221.6 |
| 294 | 4894.2 | 2878.6 | 1768.4 | 2181.6 | 2628.2 | 1888.8 |
| 296 | 1074.9 | 850.9 | 729.8 | 975.7 | 1061.8 | 946.4 |
| 342 | 1199.4 | 802.1 | 874.5 | 1428.7 | 842.7 | 661 |

13a9

| | | | ThyPapCan_02014_....Signal | | | |
|---|---|---|---|---|---|---|
| SEQ ID | 40092_H133A_831TT | 40093_H133A_832TT | 40095_H133A_834TT | 40096_H133A_835TT | 40097_H133A_836TT | 40098_H133A_837TT@I2 |
| 2 | 2960.8 | 3724.8 | 3502.5 | 2185.5 | 2399.1 | 2900.3 |
| 3 | 1034.7 | 1002.2 | 539.6 | 891.3 | 452.9 | 1885.7 |
| 5 | 1648.6 | 2929.1 | 3651.5 | 1611.8 | 3731.1 | 3572.5 |
| 6 | 1182.8 | 1644.7 | 1603.7 | 966.3 | 2282.6 | 1818.1 |
| 9 | 1637.6 | 5720.9 | 3045.3 | 1451 | 5197.6 | 7768.2 |
| 12 | 4300.1 | 3654.1 | 3555.9 | 3946.6 | 2299 | 2877.5 |
| 18 | 6335.8 | 3922.6 | 3579.6 | 3282.2 | 3591.8 | 9567 |
| 22 | 3973 | 4533 | 5478.5 | 2546.7 | 2145.4 | 7779.3 |
| 25 | 7110.3 | 2680 | 2117.2 | 5212.6 | 869.3 | 8325.2 |
| 28 | 550.3 | 1205.7 | 660.4 | 730.8 | 642.1 | 734.4 |
| 32 | 3476.1 | 8075.3 | 2580 | 2524.9 | 2454.3 | 7864.8 |
| 39 | 973.9 | 1322.9 | 1283.6 | 847.1 | 1328.7 | 1793.3 |
| 74 | 611.8 | 1044.9 | 1510.9 | 831.5 | 519.6 | 513.4 |
| 78 | 179.8 | 404.5 | 344.8 | 133.7 | 582.7 | 482.5 |
| 143 | 2333.5 | 2803.8 | 3730.7 | 2073.1 | 2033.9 | 3437.3 |
| 174 | 2628.9 | 5254.6 | 2333.9 | 922.6 | 5487.5 | 2544 |
| 175 | 3237.9 | 5586.3 | 5010.7 | 2460.9 | 2800.9 | 4480.6 |
| 191 | 3519.9 | 1826.9 | 1761.9 | 1360.1 | 531.4 | 2426.7 |
| 212 | 957 | 1085.3 | 1218.4 | 764.9 | 1307.9 | 1161.5 |
| 222 | 3373.3 | 1016 | 723.7 | 3010.1 | 829.7 | 1642.5 |
| 233 | 11130.3 | 16096.3 | 16100.3 | 10534.6 | 2873.8 | 13024.1 |
| 234 | 2382.4 | 2253.4 | 2236.6 | 842.4 | 1767 | 3577 |
| 237 | 2141.4 | 2712.7 | 2749.7 | 2120.8 | 2269.8 | 2385.2 |
| 238 | 719.8 | 2489.2 | 1179.1 | 806.1 | 1516.4 | 1336.1 |

| | | | | | | |
|---|---|---|---|---|---|---|
| 245 | 873.3 | 1325.6 | 1693 | 870.8 | 1916.1 | 1869.5 |
| 250 | 2074.6 | 1295 | 2234.8 | 1654.7 | 2588.9 | 1104.7 |
| 252 | 1496.7 | 2444.6 | 2031.7 | 1605.8 | 1808.1 | 1897.2 |
| 264 | 931.2 | 1035.7 | 1188 | 746.7 | 1074.1 | 1170.2 |
| 290 | 499.8 | 667.3 | 663.2 | 626.9 | 634.4 | 625.8 |
| 294 | 2010 | 4135.1 | 2098.4 | 1207.1 | 3722.1 | 2055.4 |
| 296 | 2128.8 | 2051.6 | 2507.5 | 2156.5 | 1001 | 2034.3 |
| 342 | 835.3 | 1453.2 | 1071.6 | 615.5 | 1084 | 1209 |

13a10

| | Signal | | | | | |
|---|---|---|---|---|---|---|
| SEQ ID | ThyPapCan_02014_40 099_H133A_838TT@I2 | 02014_40317_ H133A_839TT | pc__EA40200 _VDX879TT | pc__EA40201 _VDX881TT | pc__EA40202 _VDX882TT | pc__EA40203_ VDX883TT |
| 2 | 3540.3 | 1657 | 3331 | 3253.1 | 3349.3 | 3642 |
| 3 | 1796.3 | 750.6 | 1334.9 | 1248.1 | 477.8 | 697.9 |
| 5 | 4954 | 2602 | 3011.2 | 2106.6 | 1504.9 | 2070.9 |
| 6 | 2558.4 | 1246.9 | 1008.5 | 1708.8 | 874.6 | 704.3 |
| 9 | 4538 | 3328.9 | 1097.2 | 1789.6 | 857.2 | 1327.3 |
| 12 | 2544 | 6420.6 | 5055.9 | 4641.7 | 3423.2 | 2283 |
| 18 | 9287.1 | 5085 | 6074.5 | 5157.6 | 7305.1 | 5996.2 |
| 22 | 5415.4 | 3850 | 3537 | 3100.8 | 2588.4 | 2125.9 |
| 25 | 12197.9 | 1296 | 3368.6 | 4941.8 | 1669.9 | 1166.3 |
| 28 | 915.5 | 631 | 842.7 | 869.9 | 606.5 | 459.6 |
| 32 | 4804.9 | 3883.4 | 3039.3 | 5722.3 | 3401.7 | 1964.8 |
| 39 | 1409.2 | 756 | 1855 | 1356 | 1421 | 1878.4 |
| 74 | 992.8 | 349.3 | 597.9 | 708.5 | 741.6 | 421.6 |
| 78 | 352 | 259.9 | 342.4 | 434.8 | 172.4 | 207.2 |
| 143 | 4199.6 | 1314.8 | 2573.1 | 5070.9 | 4302.9 | 3455.3 |
| 174 | 3924.5 | 1399.1 | 5622.6 | 4217.6 | 1231.4 | 1538.6 |
| 175 | 6697.7 | 1590 | 4211.2 | 7931.3 | 7786.3 | 5294.6 |
| 191 | 3745.9 | 816.2 | 4875.5 | 3853.9 | 2370.5 | 1807.8 |
| 212 | 1219.1 | 1064.8 | 1480.3 | 1571.7 | 1350.2 | 874.7 |
| 222 | 8593.9 | 434.6 | 1994.5 | 2235.1 | 980.8 | 748.4 |
| 233 | 20899.6 | 4657 | 37907.7 | 22959.2 | 12092.7 | 30504.2 |
| 234 | 3763.9 | 1496.4 | 3667.8 | 2431.2 | 1077.6 | 1447.8 |
| 237 | 2711.1 | 2046.9 | 1892.2 | 2919 | 2374.3 | 2011.2 |
| 238 | 994.1 | 1047.3 | 1092.4 | 929.6 | 877.7 | 776.8 |
| 245 | 1339.1 | 1019.4 | 2826.7 | 1538.1 | 1379.8 | 1254.9 |
| 250 | 1736 | 710.8 | 2570.5 | 1597.7 | 1899 | 1923.8 |
| 252 | 2933.1 | 1316.3 | 1849.2 | 2395.5 | 2621 | 1992.7 |
| 264 | 1426.6 | 987 | 2826.4 | 1272.7 | 2511.5 | 1495.4 |
| 290 | 666.3 | 368.1 | 487 | 665.4 | 692.1 | 706 |
| 294 | 3175.8 | 1237.3 | 4622.7 | 3449.7 | 976.8 | 1074.7 |
| 296 | 1837.4 | 899.1 | 2639 | 2217.5 | 2398.4 | 2049.3 |
| 342 | 1355.2 | 406.1 | 1662.9 | 803.3 | 1501.3 | 863 |

13a11

| | Signal | | | | | |
|---|---|---|---|---|---|---|
| SEQ ID | pc__EA40204_VD X884TT | pc__EA40205_ VDX885TT | pc__EA40206_V DX886TT | pc__EA40207_ VDX890TT | pc__EA40208 _VDX892TT | pc__EA40209_ VDX893TT |
| 2 | 2895.1 | 2994.7 | 3397.6 | 4328.1 | 4435.3 | 5980.3 |
| 3 | 1144 | 932.3 | 1277.6 | 3294.1 | 1361.2 | 475.7 |
| 5 | 1096.3 | 1151.4 | 4943.4 | 1741.8 | 3416.4 | 653.5 |

| | | | | | | |
|---|---|---|---|---|---|---|
| 6 | 916.1 | 945 | 1696.2 | 1593.6 | 1278.6 | 740.7 |
| 9 | 2396.9 | 3699.9 | 1671.3 | 1488.6 | 2591.9 | 1644.7 |
| 12 | 3762.2 | 3308.4 | 3095.3 | 2865.4 | 5996.8 | 1644.2 |
| 18 | 9468.3 | 6669.2 | 10475.1 | 18044.8 | 9220.2 | 11224.4 |
| 22 | 3823.4 | 2813.4 | 4326.9 | 7811.1 | 7837 | 1650.1 |
| 25 | 1322.8 | 496 | 4227.5 | 4437.5 | 1271.9 | 878.1 |
| 28 | 563.6 | 333.7 | 602.3 | 782.9 | 724.2 | 636.5 |
| 32 | 4985.4 | 854.7 | 1788.8 | 2423.6 | 2099.4 | 629.4 |
| 39 | 963 | 1562 | 1937.7 | 1973.7 | 1774.1 | 1387.6 |
| 74 | 417 | 443.9 | 383.6 | 600.6 | 814.2 | 578.6 |
| 78 | 169.9 | 266.2 | 346.4 | 207.7 | 507.4 | 117.7 |
| 143 | 2357.9 | 2936.4 | 2961 | 6472.6 | 4513.4 | 3395.7 |
| 174 | 1096.5 | 2227.9 | 1825 | 1268.5 | 5715 | 380.6 |
| 175 | 3559.1 | 4964.6 | 5395 | 12711.3 | 7961.8 | 5835.7 |
| 191 | 5320.2 | 8526.6 | 4668.1 | 4129.5 | 8005 | 4597.4 |
| 212 | 1168.2 | 1634.1 | 1396.4 | 1197.9 | 1161.5 | 1523.4 |
| 222 | 805.9 | 739.2 | 2330.2 | 3890.4 | 873.8 | 1137.4 |
| 233 | 25920.6 | 37919.3 | 33438 | 7762.1 | 23325.9 | 20940.7 |
| 234 | 1882.8 | 1513.3 | 2438.5 | 4528.9 | 2232.7 | 1253.9 |
| 237 | 2950.6 | 1089.9 | 1886.5 | 2930.8 | 2484.4 | 2082.4 |
| 238 | 1037.6 | 1240 | 818.8 | 709.9 | 1096.2 | 877.7 |
| 245 | 1151.5 | 1872.7 | 2007.7 | 1316.8 | 2042.3 | 1717.4 |
| 250 | 3324 | 2153.4 | 2363.7 | 1885.4 | 2787.9 | 3192.2 |
| 252 | 1641 | 1236.4 | 1558.6 | 2605.7 | 1944.3 | 2613.4 |
| 264 | 1120.1 | 2994.2 | 1776.5 | 1401.3 | 1517.1 | 2362.8 |
| 290 | 624.5 | 516.4 | 692.1 | 520.9 | 778.6 | 1209.7 |
| 294 | 1273.1 | 3050.2 | 1557 | 1442.8 | 3564.9 | 424.1 |
| 296 | 2601.3 | 2023.6 | 2244.2 | 2054.1 | 2777.3 | 4347 |
| 342 | 723.2 | 973.5 | 587 | 2028.3 | 679.8 | 746.2 |

13a12

| | Signal | | | | | |
|---|---|---|---|---|---|---|
| SEQ ID | pc__EA40210_VDX894TT | 02014_40319_H133A_903TT | 02014_40320_H133A_904TT | 02014_40328_H133A_928TT | 02014_40330_H133A_932TT | 02014_40331_H133A_933TT |
| 2 | 4563.8 | 3729.2 | 2859.9 | 2475.3 | 3977.2 | 3386.3 |
| 3 | 475.3 | 827.4 | 4626.7 | 246.3 | 2009.2 | 1481.9 |
| 5 | 1717.5 | 3130.1 | 1505.9 | 1737.4 | 1875.4 | 4763.8 |
| 6 | 698.9 | 1153.2 | 2668.3 | 1104 | 1295.6 | 1385.4 |
| 9 | 3017.4 | 3184.7 | 6316.2 | 1911.3 | 3801.9 | 2397.6 |
| 12 | 1662.3 | 4850 | 7880.2 | 3035 | 5874.2 | 3637.2 |
| 18 | 7186 | 6997.2 | 14099 | 11077.7 | 9724.6 | 10100.8 |
| 22 | 1195.2 | 3583.4 | 6058.2 | 4793.7 | 6322.5 | 4421.3 |
| 25 | 587.7 | 868.9 | 28372.6 | 667.2 | 3526 | 4180.2 |
| 28 | 728.8 | 621.5 | 1685.2 | 927.3 | 646.4 | 882.8 |
| 32 | 1503.2 | 2517.7 | 19668.7 | 3607.7 | 1986.9 | 6399.2 |
| 39 | 1609.6 | 1035.8 | 1140.8 | 1205 | 1028.3 | 1359.6 |
| 74 | 240.3 | 718.9 | 623.5 | 583.2 | 953 | 1021.1 |
| 78 | 196.7 | 180.1 | 370.1 | 543 | 465.1 | 373.7 |
| 143 | 1838.3 | 3062.8 | 1513.2 | 2903.2 | 3977.3 | 3056.1 |
| 174 | 1548.4 | 1810.3 | 1087.6 | 2910.3 | 3511.6 | 3939.4 |
| 175 | 2462.7 | 5545.8 | 1615.4 | 4518.2 | 7011.2 | 5184.6 |
| 191 | 3292.3 | 2497.1 | 3479.2 | 1085 | 5805.4 | 2286.3 |
| 212 | 1289.5 | 1230.1 | 645.1 | 1795.6 | 639 | 1130.8 |

| | | | | | | |
|---|---|---|---|---|---|---|
| 222 | 502.2 | 315.3 | 8484.1 | 1685.8 | 4996.9 | 611.8 |
| 233 | 40786.7 | 16562 | 17697.8 | 3636.9 | 19787.1 | 4648.9 |
| 234 | 1248.4 | 1443.8 | 3706.7 | 2565.5 | 2733.9 | 2463.9 |
| 237 | 1286.1 | 2852.7 | 2832.7 | 2610.7 | 2178.6 | 2681.6 |
| 238 | 1382.7 | 1243.2 | 990.6 | 1636.4 | 1423.2 | 1469.2 |
| 245 | 2174 | 1000.7 | 703.1 | 2029.7 | 1000.1 | 1302.2 |
| 250 | 2738.6 | 2792.3 | 1076.8 | 1439.3 | 3692.8 | 2113.9 |
| 252 | 1078.4 | 1615.7 | 2091.1 | 2366.6 | 1895.9 | 2343.2 |
| 264 | 2550.8 | 948.7 | 659.3 | 1351 | 811.7 | 1104.5 |
| 290 | 518.9 | 745.9 | 1057.1 | 320.1 | 924.2 | 1016.3 |
| 294 | 1936.2 | 1348.3 | 757.5 | 2287.3 | 2252.8 | 2351.2 |
| 296 | 2194.3 | 1614.6 | 1446.4 | 729.6 | 2955.6 | 1690.2 |
| 342 | 612.9 | 748.4 | 366.7 | 1061 | 1424.5 | 894.4 |

13a13

| | Signal | | | | | |
|---|---|---|---|---|---|---|
| SEQ ID | EA02014_40375_H133A_922TT | EA02014_40381_H133A_945TT | EA02014_40382_H133A_946TT | EA02014_40383_H133A_947TT | EA02014_40384_H133A_948TT | ThyFolCan_02014_40084_H133A_823TT |
| 2 | 1365.7 | 2250.1 | 3658.1 | 2299 | 3252.8 | 3923.8 |
| 3 | 935.6 | 1124.4 | 1023.9 | 2053.6 | 1187.9 | 1004.7 |
| 5 | 7421.5 | 656.7 | 3000.3 | 4151.2 | 1730.9 | 3450.3 |
| 6 | 1026.6 | 982.6 | 1628.3 | 1939.7 | 1023.9 | 1961.6 |
| 9 | 10992.7 | 1452.2 | 2478.2 | 4581.5 | 1567.6 | 3548.1 |
| 12 | 3007 | 2413.7 | 5402.5 | 7248.9 | 4382.6 | 6659.3 |
| 18 | 4106.5 | 1506.8 | 9107.3 | 6629.5 | 9741.9 | 7072.7 |
| 22 | 4766.3 | 5493.6 | 3034.7 | 4839.3 | 4984.8 | 4552 |
| 25 | 900.4 | 10351.1 | 1252.1 | 10112.4 | 3841.3 | 7385.3 |
| 28 | 685.8 | 1022.4 | 714.8 | 1191.6 | 656.5 | 625.4 |
| 32 | 3099 | 1984 | 3215.8 | 12202.3 | 3643.5 | 4762.1 |
| 39 | 1012.3 | 480.5 | 1173 | 1094.8 | 781.3 | 1099.1 |
| 74 | 987.5 | 61.1 | 894.2 | 877.1 | 629 | 887.2 |
| 78 | 418.8 | 111.9 | 529.1 | 188.7 | 371.9 | 650.2 |
| 143 | 2030.1 | 1411.5 | 3847.8 | 2492.6 | 2091.2 | 2930.6 |
| 174 | 2288.4 | 269.9 | 4741.5 | 2231.5 | 2488.1 | 7050.3 |
| 175 | 2412.3 | 2138.9 | 6427.7 | 3285.3 | 3691.4 | 4648.5 |
| 191 | 617.2 | 983.6 | 2182.4 | 2820.3 | 2081 | 1043.3 |
| 212 | 1018.3 | 208.9 | 1054.6 | 964.8 | 771.1 | 591.5 |
| 222 | 725.6 | 8103.4 | 443.9 | 4302.9 | 1433 | 10512.7 |
| 233 | 4450.9 | 1177.3 | 20110.2 | 19683.9 | 4061.3 | 2305.1 |
| 234 | 1926 | 2079.3 | 2095.5 | 2384.8 | 2482.7 | 6283.2 |
| 237 | 2547.1 | 2912.4 | 3040.2 | 2736.1 | 3005.4 | 2681.3 |
| 238 | 1822.6 | 264.6 | 1023.9 | 929.2 | 1088.1 | 1176.9 |
| 245 | 2699.8 | 113.4 | 1230.8 | 1359.8 | 887 | 1067.6 |
| 250 | 1198.1 | 249 | 1802.6 | 1764.8 | 2220.8 | 1112.4 |
| 252 | 1311.3 | 613.6 | 2434.6 | 2260.9 | 1332.7 | 1526.7 |
| 264 | 1364.1 | 382.4 | 1206.6 | 1095.1 | 1210.5 | 1451.3 |
| 290 | 463.8 | 1223.5 | 805.1 | 865.9 | 867.2 | 932.7 |
| 294 | 2024.9 | 340.9 | 4085.3 | 1859.8 | 2226.1 | 3796.8 |
| 296 | 1214 | 407.7 | 1761.4 | 1917.2 | 3476.1 | 1298.6 |
| 342 | 619 | 828.4 | 1154.4 | 639.7 | 934.8 | 1110 |

13a14

| SEQ ID | Signal ThyFolCan_02014_40085_H133A_824TT | ThyFolCan_02014_40086_H133A_825TT | ThyFolCan_02014_40088_H133A_827TT | ThyFolCan_02014_40089_H133A_828TT | 02014_40318_H133A_840TT | fc__EA40212_VDX896TT |
|---|---|---|---|---|---|---|
| 2 | 4103.9 | 1719.4 | 2824.5 | 3008.4 | 2542.7 | 2391.5 |
| 3 | 2480.6 | 144.5 | 880.5 | 497.6 | 1222.4 | 1069.4 |
| 5 | 2190.2 | 1199.7 | 4743.6 | 2936.8 | 3696.7 | 776.8 |
| 6 | 678.7 | 2589.2 | 2824.2 | 1605.5 | 1411.7 | 1403.4 |
| 9 | 6166.7 | 326.1 | 5428.5 | 2131.9 | 9187.9 | 10000.2 |
| 12 | 2872.9 | 1257.4 | 4087.8 | 3981.7 | 7045.4 | 3506.5 |
| 18 | 2329.3 | 4765.3 | 11522.2 | 7266.2 | 3045.4 | 4683.8 |
| 22 | 4848.8 | 1224.5 | 3611.1 | 6001.8 | 8784.3 | 1719.5 |
| 25 | 11319.1 | 234.5 | 3308.3 | 557.1 | 3685.8 | 383.8 |
| 28 | 772.7 | 206.8 | 830.6 | 1057.1 | 1256.5 | 1118.8 |
| 32 | 2272 | 1393.6 | 5410.1 | 3330.7 | 13825 | 869 |
| 39 | 892.8 | 541.9 | 838.5 | 941.3 | 1041 | 3461.7 |
| 74 | 126.8 | 2071.9 | 988.3 | 1357.4 | 696.8 | 1290.4 |
| 78 | 253.6 | 307.6 | 635.6 | 920.6 | 422.6 | 705.5 |
| 143 | 2718.2 | 1570.7 | 2322.8 | 2621.6 | 1038.8 | 4569.2 |
| 174 | 323 | 1399.8 | 2817 | 9345.5 | 876 | 8849.8 |
| 175 | 4437.4 | 1584 | 4411.6 | 4784.9 | 1387.4 | 7804.9 |
| 191 | 2214.1 | 575.3 | 641 | 1208.4 | 1370.9 | 4609.8 |
| 212 | 1667.2 | 662.2 | 577.7 | 1864 | 1229 | 590.6 |
| 222 | 13107.5 | 470.6 | 1030.1 | 3020.4 | 431.9 | 641.9 |
| 233 | 3196 | 1087.3 | 2932.3 | 7081 | 12626.7 | 4567.8 |
| 234 | 1659.8 | 875 | 2243.3 | 1839.4 | 1328 | 7454.4 |
| 237 | 4693.9 | 830.4 | 3700.2 | 2563.5 | 3040.5 | 1008.9 |
| 238 | 206.3 | 244.8 | 1695.9 | 2546.2 | 1614.2 | 1880.4 |
| 245 | 248.7 | 425 | 1273.9 | 1215.4 | 1333.6 | 2807.1 |
| 250 | 935.9 | 379.8 | 2284.4 | 894.4 | 867.2 | 844.3 |
| 252 | 1005.5 | 1023.3 | 1166.6 | 1884.3 | 1524.6 | 678.4 |
| 264 | 231.5 | 589 | 1480.9 | 1740.6 | 670.9 | 2459.5 |
| 290 | 638.4 | 233 | 961.8 | 668.6 | 430.5 | 996 |
| 294 | 253.4 | 1349.8 | 3087.1 | 7034.2 | 1137.3 | 7044.6 |
| 296 | 1530.8 | 1570.6 | 1591.5 | 1110.6 | 1647.7 | 1498.9 |
| 342 | 1457.8 | 712.8 | 1052.1 | 946.9 | 436.8 | 1893.8 |

13a15

| SEQ ID | Signal fc__EA40214_VDX898TT | fc__EA40215_VDX899TT | fc__EA40216_VDX900TT | fc__EA40217_VDX901TT | fc__EA40218_VDX902TT | fc__EA40221_VDX909TT |
|---|---|---|---|---|---|---|
| 2 | 2470.7 | 3328.2 | 4429.9 | 993.8 | 2649.9 | 4427.4 |
| 3 | 226.4 | 523.3 | 1216.2 | 230.6 | 417 | 2364.4 |
| 5 | 1037.2 | 3614.5 | 1440.6 | 3319 | 1487.4 | 9590 |
| 6 | 997 | 685.7 | 1641.1 | 967.8 | 2453.4 | 2537.2 |
| 9 | 384.9 | 1897.9 | 973.8 | 468.7 | 915.8 | 3339.3 |
| 12 | 9940.9 | 1131.5 | 4352.5 | 3202.2 | 4269.6 | 15203.8 |
| 18 | 4150.7 | 7715.2 | 5736.9 | 555.2 | 9139.4 | 7575.1 |
| 22 | 499.6 | 4466 | 2120.9 | 208.2 | 1563.8 | 1471.9 |
| 25 | 1721.2 | 1912 | 10485.2 | 826.1 | 1002 | 6640.8 |
| 28 | 232.1 | 706.8 | 501.4 | 122.6 | 779.4 | 645.5 |
| 32 | 2728.8 | 3753 | 2644.6 | 1820.8 | 3404.5 | 10045.3 |
| 39 | 1006.6 | 1103.3 | 1192.6 | 880.8 | 2310.8 | 346.3 |

| | | | | | | |
|---|---|---|---|---|---|---|
| 74 | 2642.5 | 794.9 | 374.8 | 1621.8 | 1057.9 | 294 |
| 78 | 294.6 | 280.8 | 131.2 | 136.4 | 344.4 | 391.3 |
| 143 | 2141.4 | 2616.6 | 5902.7 | 3882.4 | 3860.3 | 2140.3 |
| 174 | 407.1 | 2263.7 | 4270 | 2006.5 | 2076.1 | 4365.1 |
| 175 | 2416.2 | 3697.7 | 9897.3 | 6489.5 | 5706.6 | 2832.4 |
| 191 | 1436.8 | 2900.8 | 5153.2 | 165.9 | 963.5 | 512.1 |
| 212 | 839.5 | 1241.8 | 173.4 | 186.6 | 451.1 | 539.9 |
| 222 | 293.5 | 1318.1 | 6645.4 | 426.3 | 623.3 | 2727.5 |
| 233 | 17314.7 | 18164.1 | 2971.6 | 2885.8 | 2068.7 | 15099.5 |
| 234 | 2834.4 | 1575.6 | 3547 | 904.6 | 3783.5 | 6440.1 |
| 237 | 945.9 | 3105.6 | 1268.9 | 2192.9 | 893.1 | 2333.7 |
| 238 | 702.9 | 1189 | 744.7 | 554.4 | 383.3 | 908.6 |
| 245 | 914.6 | 1729.7 | 1038.5 | 839.4 | 784.2 | 459.1 |
| 250 | 845.9 | 2673.5 | 2175.8 | 702.9 | 1305.7 | 723.2 |
| 252 | 1300.4 | 2454.3 | 1377.7 | 1061.4 | 1235.8 | 2869.5 |
| 264 | 467.6 | 1277.4 | 638.7 | 2058 | 1076.7 | 569.3 |
| 290 | 573.7 | 617.2 | 993.4 | 767.4 | 1281.3 | 866.1 |
| 294 | 507.1 | 1922.1 | 2827.1 | 1753.3 | 1585.8 | 2926.6 |
| 296 | 2156.2 | 2563 | 1832.8 | 533.7 | 2130.3 | 2647.8 |
| 342 | 535.6 | 937.4 | 2261.2 | 1949.2 | 975.7 | 618.5 |

13a16

| | Signal | | | | | |
|---|---|---|---|---|---|---|
| SEQ ID | fc_EA40222 _VDX910TT | EA02014_40386 _H133A_954TT | EA02014_40394 _H133A_967TT | EA02014_40395 _H133A_968TT | EA02014_40396 _H133A_969TT | EA02014_40397 _H133A_970TT |
| 2 | 2362.1 | 1417 | 1834.4 | 2135.5 | 3006.2 | 1378.8 |
| 3 | 254.5 | 263.8 | 293.1 | 346.3 | 346.5 | 230.2 |
| 5 | 2789.1 | 8005.6 | 1358.8 | 637.3 | 1214.1 | 1214.7 |
| 6 | 1096.6 | 1050.7 | 1291.7 | 987.4 | 1223.8 | 2564.6 |
| 9 | 834.8 | 1052.4 | 2701.2 | 1171.4 | 6080 | 440.4 |
| 12 | 2031.6 | 4283.7 | 2805.1 | 4294.1 | 4966.4 | 1686.4 |
| 18 | 9498.5 | 18893.2 | 15454.8 | 2385.4 | 3575.2 | 14192.8 |
| 22 | 1449.5 | 1020 | 888.3 | 2585.4 | 3721.5 | 727.2 |
| 25 | 541.3 | 253.5 | 572.5 | 3378.9 | 709.2 | 372 |
| 28 | 502.3 | 443 | 868.6 | 378.9 | 964.1 | 341.9 |
| 32 | 1205.9 | 7122.3 | 811.1 | 2553.9 | 797 | 1753.4 |
| 39 | 1215.4 | 1031.1 | 511 | 722 | 2166.1 | 527.2 |
| 74 | 1385.4 | 1477.5 | 509.1 | 1159.3 | 1031.4 | 495.4 |
| 78 | 367.5 | 834.2 | 200.7 | 235.3 | 433.7 | 262.9 |
| 143 | 1956.7 | 1083.1 | 1486.7 | 2746 | 2538.4 | 1943.7 |
| 174 | 4466.4 | 2823.7 | 1156.3 | 3547.9 | 1541.5 | 2214.3 |
| 175 | 1799.1 | 1264 | 1758.7 | 4539.5 | 3663.3 | 2264.4 |
| 191 | 560.2 | 1494 | 607.9 | 2943.2 | 2727.4 | 1474.7 |
| 212 | 1046.1 | 307.8 | 548.8 | 340.4 | 982.9 | 647 |
| 222 | 248.3 | 533.1 | 1356.4 | 1194.2 | 1169.8 | 228.2 |
| 233 | 1183.6 | 612.7 | 1063.5 | 5528.1 | 6265.3 | 1076 |
| 234 | 1886.7 | 3460.3 | 1207.2 | 2384.1 | 3860.6 | 3322.7 |
| 237 | 934.6 | 933.5 | 2133.3 | 791 | 1890.7 | 1257.5 |
| 238 | 834.9 | 291.1 | 1034.1 | 380.8 | 946.4 | 415 |
| 245 | 1509.7 | 786.6 | 657.1 | 384.9 | 4773.4 | 447.6 |
| 250 | 2406.8 | 1245.7 | 1247.1 | 659 | 1647.1 | 595 |
| 252 | 1536.8 | 1182.4 | 1035.6 | 526.7 | 893 | 1276.7 |
| 264 | 1315.8 | 582.8 | 1373.3 | 958 | 1622.3 | 508.6 |

| | | | | | | |
|---|---|---|---|---|---|---|
| 290 | 732.4 | 835.3 | 683.8 | 1744.3 | 523 | 850.2 |
| 294 | 2817 | 2372.1 | 929.5 | 2611.8 | 1279.4 | 2097.3 |
| 296 | 1088.4 | 1541 | 2014.6 | 621.4 | 1368.3 | 981.8 |
| 342 | 763.6 | 866.9 | 722.2 | 559 | 594.3 | 445.9 |

13a17

| | Signal | | | | | | |
|---|---|---|---|---|---|---|---|
| SEQ ID | EA02014_40405_H133A_982TT | EA02014_40406_H133A_983TT | pb1 | pb2 | pb3 | pb4 | pb5' |
| 2 | 3615.6 | 2106 | 204.1 | 324.2 | 178.6 | 206.6 | 249.1 |
| 3 | 765 | 372.2 | 171.5 | 303.8 | 159.1 | 61.1 | 90.2 |
| 5 | 2446.1 | 394 | 107.9 | 19.9 | 68.8 | 74.6 | 69 |
| 6 | 1051.6 | 1176 | 211.6 | 214.4 | 318.6 | 218.8 | 355.4 |
| 9 | 2059.2 | 1081.4 | 18 | 18.8 | 29.2 | 9.7 | 134 |
| 12 | 3520 | 391.5 | 11.4 | 9.1 | 9.9 | 33.8 | 8.3 |
| 18 | 4633.4 | 13339 | 94.2 | 50.7 | 67.6 | 74.9 | 29.7 |
| 22 | 6678.8 | 403.5 | 80.1 | 8.8 | 58.9 | 6.8 | 5 |
| 25 | 1229.9 | 601.5 | 197.1 | 106.7 | 65.2 | 18.7 | 87.1 |
| 28 | 778.2 | 197.6 | 11.4 | 9 | 6.6 | 11.3 | 1.7 |
| 32 | 1302.9 | 4182.6 | 8.7 | 21.6 | 30.5 | 66.1 | 64.6 |
| 39 | 2104 | 699 | 246.6 | 121.1 | 42.8 | 122.8 | 171.7 |
| 74 | 1189.7 | 1142.3 | 59.9 | 94.4 | 70.3 | 29 | 5.2 |
| 78 | 833.6 | 80.9 | 48 | 45.5 | 66.9 | 49.5 | 119.7 |
| 143 | 2842.2 | 1778.2 | 290 | 291.2 | 190.4 | 65.6 | 203.5 |
| 174 | 3307.3 | 329.8 | 29.8 | 42.8 | 25.8 | 11.5 | 13.9 |
| 175 | 3878.9 | 1966.8 | 165.7 | 282.2 | 158.3 | 67.2 | 249.2 |
| 191 | 3952.2 | 5278 | 46.8 | 181.4 | 148.2 | 97.3 | 152.1 |
| 212 | 2201.7 | 1134.3 | 70.4 | 55.7 | 3.5 | 87 | 99.8 |
| 222 | 469 | 451.7 | 120.4 | 148.4 | 57.7 | 49.6 | 58 |
| 233 | 11069.2 | 5607 | 13 | 9.8 | 76.9 | 7.6 | 5.6 |
| 234 | 3590 | 3449.5 | 186 | 201.3 | 165.3 | 54.8 | 141.1 |
| 237 | 2057.6 | 740.2 | 135.3 | 150.7 | 16.6 | 14.5 | 19 |
| 238 | 1679.4 | 432.3 | 22.5 | 43.8 | 11.4 | 6.2 | 5.1 |
| 245 | 2301.6 | 1040.5 | 7.1 | 12.7 | 22.5 | 5.9 | 4.6 |
| 250 | 434.6 | 1079.4 | 97.8 | 138.9 | 33.7 | 71.3 | 46.8 |
| 252 | 844.6 | 1446.1 | 35.6 | 80.3 | 29.6 | 63.3 | 88.5 |
| 264 | 1125.7 | 1084.5 | 102.8 | 31.6 | 147.6 | 101.5 | 163.3 |
| 290 | 347.9 | 466.9 | 35.8 | 7.3 | 14.4 | 87.2 | 71.7 |
| 294 | 1601.7 | 575.2 | 105.1 | 94.5 | 41.5 | 31.9 | 35.3 |
| 296 | 872.8 | 1349.1 | 217.8 | 162.6 | 186.2 | 171.2 | 212.5 |
| 342 | 948.7 | 482.8 | 296.5 | 220.2 | 308.1 | 126.3 | 298.6 |

13a18

| | Signal | | | | | | |
|---|---|---|---|---|---|---|---|
| SEQ ID | pb6' | pb7' | pb8' | pd10 | pd11 | pd12 | pd9 |
| 2 | 167.6 | 233.9 | 232.6 | 116.9 | 204.5 | 293.2 | 79.6 |
| 3 | 110.3 | 88 | 221 | 90.7 | 74.9 | 90.9 | 129.5 |
| 5 | 29.7 | 70.2 | 182.5 | 32.6 | 34.5 | 130.5 | 12.9 |
| 6 | 226.5 | 440.1 | 207 | 219.7 | 275.2 | 134.1 | 193.1 |
| 9 | 28.9 | 26.8 | 30.9 | 32.4 | 5.3 | 25.7 | 20.3 |
| 12 | 6.6 | 18.8 | 61.4 | 38.2 | 10.7 | 4.1 | 11.6 |
| 18 | 53.6 | 37.8 | 31.8 | 68.8 | 117.5 | 70.8 | 23.4 |

| | | | | | | | |
|---|---|---|---|---|---|---|---|
| 22 | 16.9 | 10.6 | 32.1 | 6.4 | 29.2 | 21.2 | 3.1 |
| 25 | 127 | 123.7 | 118.7 | 9.7 | 10.6 | 64.9 | 6.6 |
| 28 | 13.2 | 4.9 | 43.3 | 8 | 8.1 | 12 | 26.6 |
| 32 | 36.4 | 51.5 | 25.1 | 17.2 | 17.2 | 19.7 | 11 |
| 39 | 326.5 | 182.6 | 27.3 | 126.2 | 151.9 | 26 | 182.7 |
| 74 | 48.2 | 29 | 39.8 | 55.3 | 35.3 | 76.9 | 6.2 |
| 78 | 92.1 | 20 | 93.1 | 26.9 | 11.9 | 56.6 | 104.8 |
| 143 | 176.4 | 224.5 | 248.5 | 126.2 | 184.6 | 162.8 | 249.6 |
| 174 | 40.8 | 149.8 | 24.2 | 10.6 | 15.6 | 81.4 | 16.8 |
| 175 | 262.5 | 355.4 | 207.8 | 21.6 | 213.9 | 175.8 | 193.7 |
| 191 | 151.2 | 310.7 | 93.3 | 65.7 | 108.4 | 95.9 | 79.3 |
| 212 | 93.6 | 121.7 | 141.7 | 30.8 | 42.6 | 108.4 | 93.2 |
| 222 | 83.5 | 82.8 | 60.7 | 16.4 | 50 | 71.1 | 66.9 |
| 233 | 144.7 | 64.1 | 16.5 | 7 | 6.4 | 70.8 | 8.1 |
| 234 | 40.4 | 248.1 | 43.8 | 45.5 | 143.3 | 30.9 | 77.2 |
| 237 | 223.8 | 141.1 | 129.5 | 57.6 | 21.9 | 11.5 | 18.7 |
| 238 | 32.1 | 27.6 | 66.5 | 10.5 | 33.4 | 7.7 | 61.6 |
| 245 | 19.8 | 7.2 | 4.8 | 2.4 | 11.2 | 4.7 | 6.7 |
| 250 | 75.3 | 109.2 | 65.3 | 85.7 | 119.9 | 118.7 | 66.4 |
| 252 | 103.8 | 91.2 | 94.8 | 17 | 69.3 | 76.1 | 22.5 |
| 264 | 151 | 224.6 | 129.9 | 122.8 | 99.8 | 61.5 | 24.8 |
| 290 | 21.8 | 27.6 | 50.7 | 49.3 | 6.2 | 2.7 | 3 |
| 294 | 91.1 | 58.4 | 40 | 24.8 | 52.6 | 67.8 | 31.7 |
| 296 | 282.4 | 299.2 | 143.7 | 149.7 | 197.9 | 205.2 | 147.7 |
| 342 | 304.5 | 437.8 | 327.4 | 90.9 | 247.6 | 224.4 | 268.6 |

Table 13b

13b1

| SEQ ID | ThyMixBen_02014_....._Signal 40062_H133A_800TT | 40063_H133A_801TT | 40064_H133A_802TT | 40065_H133A_804TT | 40066_H133A_805TT | 40067_H133A_806TT | 40068_H133A_807TT |
|---|---|---|---|---|---|---|---|
| 83 | 156.7 | 114.3 | 73.8 | 240.3 | 173 | 193.6 | 291.2 |
| 142 | 28.3 | 7.9 | 15 | 28.4 | 26.9 | 25.8 | 14.2 |
| 136 | 100.4 | 23.4 | 15.3 | 106.9 | 28.3 | 14.9 | 56 |
| 137 | 527.2 | 47 | 32.3 | 177.2 | 19.5 | 53.1 | 404.6 |
| 152 | 237.5 | 230.4 | 188.7 | 465.2 | 183 | 260.9 | 190.9 |
| 160 | 291.6 | 141.9 | 80.9 | 323.1 | 98.1 | 120.3 | 219.7 |
| 163 | 15.6 | 53.1 | 25.1 | 70.8 | 20.1 | 56.1 | 47.8 |
| 165 | 190.2 | 230.7 | 142.4 | 483.5 | 179.3 | 241.7 | 410.9 |
| 210 | 436.9 | 8.6 | 18.4 | 343.7 | 31.8 | 8 | 148.1 |
| 214 | 269.2 | 134.4 | 167.7 | 141.8 | 145.7 | 140.5 | 349.1 |
| 219 | 217 | 19.6 | 7.9 | 85.6 | 21.8 | 26.7 | 115.1 |
| 224 | 588.2 | 121.4 | 119.2 | 275.2 | 136.6 | 240.5 | 392.5 |
| 225 | 508.1 | 30.1 | 42.3 | 107.3 | 59.4 | 29.5 | 147.3 |
| 226 | 57.3 | 48.2 | 80.9 | 103.9 | 43.5 | 12.3 | 88.9 |
| 309 | 257.7 | 20.6 | 20.7 | 75.5 | 19.2 | 24 | 833.9 |
| 332 | 287.1 | 17 | 26.1 | 103.8 | 115.9 | 68.8 | 37.9 |
| 346 | 622.2 | 18.3 | 24 | 17.6 | 20.7 | 13.2 | 442.1 |

13b2

| SEQ | 02014_...._Signal 40198_H13 | 40321_H13 | 40322_H13 | 40323_H133 | 40324_H133A | 40325_H133A_9 | 40326_H133A_ |
|---|---|---|---|---|---|---|---|

| ID | 3A_871TT | 3A_913TT | 3A_914TT | A_915TT | _917TT | 18TT | 919TT |
|---|---|---|---|---|---|---|---|
| 83 | 39.5 | 90.2 | 76.4 | 255.8 | 150.8 | 109.7 | 142.6 |
| 142 | 25.3 | 22.5 | 12.9 | 34.4 | 8.5 | 20.6 | 67.7 |
| 136 | 9.8 | 31.8 | 16.8 | 132 | 115.4 | 130 | 7.2 |
| 137 | 227.8 | 53.6 | 68.7 | 60.1 | 29.6 | 133.2 | 29 |
| 152 | 184.4 | 214.3 | 253.2 | 294.7 | 313.8 | 251.3 | 294.2 |
| 160 | 267.5 | 250.7 | 206.5 | 328.8 | 191.1 | 220.8 | 114.4 |
| 163 | 44.3 | 56.4 | 23.9 | 98.5 | 35.2 | 68 | 41.8 |
| 165 | 174.3 | 221.8 | 185.5 | 266.3 | 104.6 | 217.1 | 159.9 |
| 210 | 25.8 | 153.6 | 42 | 55.5 | 8.2 | 152 | 49.1 |
| 214 | 43.2 | 83.6 | 110.6 | 213.7 | 132.6 | 89.3 | 138.7 |
| 219 | 56.6 | 53.5 | 45.2 | 122.1 | 24.4 | 124 | 41.3 |
| 224 | 269.7 | 357.4 | 260 | 191.5 | 168.8 | 330.8 | 169.8 |
| 225 | 204.8 | 322.6 | 114.2 | 55.6 | 121.8 | 317.6 | 95.8 |
| 226 | 11.9 | 57.4 | 69.9 | 22 | 67.5 | 33.6 | 19.3 |
| 309 | 37.9 | 37 | 28.2 | 53 | 10.2 | 32.6 | 24.2 |
| 332 | 9.9 | 59.2 | 66.7 | 57.8 | 13 | 40.5 | 9 |
| 346 | 28.6 | 263.8 | 11.1 | 27.2 | 32.5 | 153.4 | 61.1 |

13b3

| SEQ | ThyFolBen_02014_...._Signal | | | | |
|---|---|---|---|---|---|
| ID | 40079_H133A_818TT | 40080_H133A_819TT | 40081_H133A_820TT | 40082_H133A_821TT | 40083_H133A_822TT |
| 83 | 63.4 | 151.8 | 104.1 | 27.5 | 138.9 |
| 142 | 15 | 20.2 | 18.8 | 18.4 | 23 |
| 136 | 17.6 | 135.4 | 77.1 | 31.7 | 17.7 |
| 137 | 24.7 | 36.1 | 18 | 28.4 | 42.6 |
| 152 | 62.1 | 176.7 | 146.4 | 273.3 | 328.2 |
| 160 | 153 | 138.1 | 131 | 192.4 | 124.1 |
| 163 | 6.9 | 21.4 | 35 | 63.5 | 13.4 |
| 165 | 28.7 | 155.3 | 112.6 | 135.2 | 222.6 |
| 210 | 27.5 | 10.4 | 8.1 | 9.9 | 32.7 |
| 214 | 211.7 | 210.8 | 83.9 | 166.4 | 160.2 |
| 219 | 18.7 | 111.8 | 45.7 | 10.9 | 29.6 |
| 224 | 114.7 | 241.8 | 100 | 160.9 | 242.4 |
| 225 | 69.4 | 63.8 | 145.4 | 110.9 | 126.1 |
| 226 | 92.1 | 95.5 | 36.3 | 74.5 | 67.2 |
| 309 | 16.8 | 17.1 | 12.1 | 18.8 | 27.3 |
| 332 | 40 | 20.5 | 21 | 11 | 6.6 |
| 346 | 7.2 | 27.1 | 18.6 | 29.3 | 26.7 |

13b4

| | fa__EA40...._Signal | | | | | | | | |
|---|---|---|---|---|---|---|---|---|---|
| SEQ ID | 191_VDX 862TT | 192_VDX 863TT | 193_VDX 864TT | 194_VDX 865TT | 195_VDX 866TT | 196_VDX 867TT | 197_VDX 869TT | 219_VDX 907TT | 220_VDX 908TT |
| 83 | 26.9 | 24.4 | 56.7 | 155.1 | 22.3 | 94.8 | 63 | 41.9 | 256 |
| 142 | 16.1 | 22.4 | 14.5 | 54.8 | 17.1 | 15.8 | 15 | 21.9 | 23.9 |
| 136 | 23 | 59 | 20.4 | 121.4 | 12.5 | 88.4 | 77.1 | 47.1 | 36.8 |
| 137 | 26.7 | 32.4 | 19.4 | 45.2 | 32.8 | 30.8 | 55.2 | 97 | 63.4 |
| 152 | 75.7 | 189.4 | 211.4 | 320.4 | 194 | 75.5 | 314 | 114.8 | 260 |
| 160 | 239.5 | 101.4 | 91.3 | 292.1 | 55.8 | 86.8 | 53.7 | 47.4 | 145.4 |
| 163 | 48.5 | 47.6 | 23.1 | 168.3 | 35.4 | 13.4 | 51.5 | 13.5 | 60.4 |

| | | | | | | | | | |
|---|---|---|---|---|---|---|---|---|---|
| 165 | 26.5 | 94.3 | 81.1 | 197.9 | 112.9 | 164.6 | 155.9 | 29.7 | 250.4 |
| 210 | 32.2 | 16.2 | 23.8 | 81.4 | 25.3 | 22.7 | 140.5 | 15.5 | 200.8 |
| 214 | 119.9 | 130.9 | 141.7 | 235.4 | 263.7 | 30.4 | 124.9 | 57 | 241 |
| 219 | 80.6 | 7.6 | 15.9 | 20.9 | 25 | 10 | 16.5 | 23.4 | 20.1 |
| 224 | 50.9 | 25.8 | 50 | 80 | 32.6 | 99.7 | 136.4 | 137.5 | 167 |
| 225 | 66.7 | 41.4 | 64.4 | 69.5 | 18.1 | 94.7 | 51.1 | 51.6 | 149.8 |
| 226 | 76 | 2.8 | 3.5 | 99.1 | 88.6 | 18.3 | 60.6 | 6.4 | 11.6 |
| 309 | 6.7 | 15.2 | 9.4 | 62.6 | 20.2 | 18.4 | 15.9 | 10.2 | 33.3 |
| 332 | 9.6 | 36.9 | 10.8 | 91.1 | 103 | 30.1 | 12.3 | 19.7 | 36.5 |
| 346 | 25.1 | 16.5 | 20.8 | 89.5 | 34.2 | 20.9 | 18.3 | 11.3 | 114.6 |

13b5

| | 02014_...._Signal | | | |
|---|---|---|---|---|
| SEQ ID | 40327_H133A_920TT | 40332_H133A_938TT | 40334_H133A_941TT | 40335_H133A_940TT |
| 83 | 216.8 | 145.1 | 58.1 | 102.1 |
| 142 | 22.2 | 7.9 | 6.6 | 14.5 |
| 136 | 71.7 | 30.1 | 47.9 | 57.1 |
| 137 | 47 | 48.1 | 69.1 | 57.4 |
| 152 | 252.7 | 189 | 184.9 | 138.7 |
| 160 | 153.6 | 179.9 | 121.9 | 66.6 |
| 163 | 53.3 | 13 | 69.1 | 38.6 |
| 165 | 146.9 | 146.7 | 85.1 | 120 |
| 210 | 24 | 59.1 | 21.5 | 23.8 |
| 214 | 90.5 | 224.7 | 178.7 | 139.3 |
| 219 | 36.4 | 17.5 | 14.9 | 63.3 |
| 224 | 180.2 | 126.6 | 154.2 | 161 |
| 225 | 59.7 | 197.5 | 83.8 | 115.7 |
| 226 | 31.9 | 58.1 | 33.7 | 34.5 |
| 309 | 27.1 | 7.7 | 6.2 | 15.6 |
| 332 | 83.8 | 7.3 | 40.7 | 47.6 |
| 346 | 17 | 18.8 | 27.6 | 24.4 |

13b6

| | EA02014_...._Signal | | | | | |
|---|---|---|---|---|---|---|
| SEQ ID | 40374_H133A_921TT | 40376_H133A_923TT | 40377_H133A_924TT | 40378_H133A_925TT | 40379_H133A_926TT | 40380_H133A_927TT |
| 83 | 174.4 | 32.7 | 69.1 | 86.6 | 67.7 | 204.9 |
| 142 | 13.7 | 19.8 | 16.5 | 57.7 | 22.9 | 18.9 |
| 136 | 24.2 | 9.3 | 12.1 | 25.3 | 190.4 | 16.3 |
| 137 | 38.3 | 143.4 | 28.2 | 77.5 | 156.1 | 255.2 |
| 152 | 178.2 | 230.8 | 155 | 237.4 | 235.7 | 70 |
| 160 | 125.2 | 133.5 | 74.9 | 197 | 170.1 | 234 |
| 163 | 54.4 | 4.9 | 35.5 | 46.1 | 65.2 | 59.9 |
| 165 | 218.9 | 155.1 | 206 | 85.6 | 158.1 | 532.5 |
| 210 | 36.8 | 25 | 20.6 | 25.8 | 19.8 | 551.3 |
| 214 | 126.7 | 186.1 | 167.2 | 114.1 | 191.9 | 161.4 |
| 219 | 32.3 | 25.5 | 15.3 | 10.2 | 95.6 | 18.5 |
| 224 | 220.8 | 301.9 | 129.7 | 178.8 | 308.2 | 270.9 |
| 225 | 158 | 221.8 | 127.9 | 65.6 | 269.4 | 60.6 |
| 226 | 37.5 | 33.4 | 9.6 | 57.2 | 80.8 | 18.1 |
| 309 | 26.1 | 16 | 17.8 | 31.8 | 32.6 | 49.2 |

| | | | | | | |
|---|---|---|---|---|---|---|
| 332 | 26.3 | 38.5 | 6.7 | 11.6 | 73.5 | 21.6 |
| 346 | 20.7 | 151.4 | 21.6 | 19.2 | 179.4 | 53.6 |

13b7

| | EA02014_...._Signal | | | | | | |
|---|---|---|---|---|---|---|---|
| SEQ ID | 40387_H133A_955TT | 40388_H133A_956TT | 40389_H133A_957TT | 40390_H133A_959TT | 40391_H133A_960TT | 40392_H133A_961TT | 40393_H133A_962TT |
| 83 | 29.1 | 121.5 | 70.7 | 28.8 | 121.6 | 120.5 | 34 |
| 142 | 16.7 | 14.2 | 10.8 | 21.4 | 18.6 | 16.5 | 21.9 |
| 136 | 15.7 | 27.8 | 67.7 | 45.9 | 15.4 | 8.2 | 43 |
| 137 | 28.5 | 44.8 | 34.5 | 50 | 31.3 | 162.2 | 18.5 |
| 152 | 85.4 | 163 | 183.4 | 127.1 | 298.3 | 193 | 221.2 |
| 160 | 121.9 | 51.9 | 175 | 137.4 | 208.5 | 125.9 | 162.9 |
| 163 | 47 | 27.7 | 43.6 | 80.1 | 29.7 | 33.8 | 44.4 |
| 165 | 42.2 | 122.9 | 103.8 | 150.6 | 253.3 | 106.9 | 221.6 |
| 210 | 27.1 | 5.2 | 20.4 | 73.7 | 29.4 | 38.7 | 85 |
| 214 | 36.4 | 68.3 | 165.9 | 25.3 | 129.8 | 165.9 | 100.9 |
| 219 | 23.3 | 51 | 29.1 | 25.7 | 67 | 20.8 | 21 |
| 224 | 143.8 | 95.1 | 128.8 | 78.7 | 108.8 | 152 | 109 |
| 225 | 140.5 | 83.1 | 34.3 | 54 | 62.8 | 114.7 | 94.3 |
| 226 | 4.3 | 24.7 | 84.1 | 47.7 | 33.3 | 66.1 | 13.3 |
| 309 | 18.7 | 11.4 | 26.7 | 44.2 | 20.6 | 34.1 | 35.2 |
| 332 | 11.1 | 4 | 37.9 | 14.1 | 17.8 | 13.1 | 11.5 |
| 346 | 21.8 | 22.1 | 12.8 | 35.7 | 38.7 | 29.6 | 10.1 |

13b8

| | ThyPapCan_02014_...._Signal | | | | | | | | |
|---|---|---|---|---|---|---|---|---|---|
| SEQ ID | 40090_H133A_829TT | 40091_H133A_830TT@IC | 40092_H133A_831TT | 40093_H133A_832TT | 40095_H133A_834TT | 40096_H133A_835TT | 40097_H133A_836TT | 40098_H133A_837TT@I2 | 40099_H133A_838TT@I2 |
| 83 | 304.3 | 199.4 | 258.5 | 171.4 | 112.3 | 186.5 | 28.5 | 137.1 | 85.9 |
| 142 | 26 | 34.7 | 22.4 | 19.1 | 16.4 | 25.8 | 12.1 | 22.5 | 15.4 |
| 136 | 122.2 | 116.6 | 21.7 | 27.3 | 30.7 | 110.2 | 143.8 | 14.1 | 140.7 |
| 137 | 40.7 | 72 | 23.8 | 36.8 | 19.5 | 31.5 | 29.6 | 29.4 | 32.5 |
| 152 | 611.5 | 682.6 | 236.2 | 170.5 | 200.6 | 104 | 136.4 | 118 | 155.9 |
| 160 | 218.1 | 244.7 | 225.4 | 68.9 | 76.4 | 137.3 | 83.2 | 171.7 | 66.7 |
| 163 | 17.8 | 119.7 | 38.9 | 51.8 | 52.9 | 42.7 | 25.5 | 57.2 | 23.6 |
| 165 | 370.6 | 263.3 | 353.8 | 154.1 | 165.1 | 233.2 | 100.4 | 227.9 | 213.3 |
| 210 | 223.2 | 249.8 | 303 | 9.7 | 30.2 | 94.5 | 21.9 | 42.1 | 23.1 |
| 214 | 178.4 | 246.7 | 155.4 | 188.3 | 138.6 | 133.7 | 69 | 298.6 | 90.8 |
| 219 | 36.8 | 136.4 | 64.9 | 24.3 | 19.1 | 25.9 | 11.7 | 13.6 | 23.8 |
| 224 | 364.7 | 427.6 | 180 | 191.1 | 163.6 | 211.5 | 36.2 | 303.4 | 164.5 |
| 225 | 333.9 | 213.5 | 221 | 124.6 | 137.2 | 195.1 | 46.9 | 145.6 | 34.3 |
| 226 | 179.2 | 142 | 57 | 15.4 | 13.7 | 91.8 | 33.5 | 132.6 | 38 |
| 309 | 88.8 | 136.6 | 48.8 | 22.1 | 23.6 | 40.5 | 16.5 | 33.9 | 24.7 |
| 332 | 92 | 56.9 | 42.4 | 61.1 | 34.4 | 70.6 | 14.5 | 108.1 | 60.8 |
| 346 | 58.4 | 61 | 197.1 | 38 | 29.1 | 281.5 | 37.5 | 30.8 | 33 |

13b9

| | Signal | | | | | |
|---|---|---|---|---|---|---|
| SEQ ID | 02014_40317_H133A_839TT | pc__EA40200_VDX879TT | pc__EA40201_VDX881TT | pc__EA40202_VDX882TT | pc__EA40203_VDX883TT | pc__EA40204_VDX884TT |

| | | | | | | |
|---|---|---|---|---|---|---|
| 83 | 288.1 | 25.7 | 32.9 | 29.2 | 58.6 | 184.4 |
| 142 | 16.1 | 21.2 | 23.5 | 7.6 | 21.5 | 30.6 |
| 136 | 62.8 | 160.5 | 141.2 | 8.7 | 13.2 | 19.4 |
| 137 | 45.4 | 155.5 | 35.5 | 63.9 | 41.5 | 31.1 |
| 152 | 383.4 | 197 | 90.6 | 123.7 | 188 | 240 |
| 160 | 142.9 | 79.7 | 181.6 | 205.4 | 157.5 | 137.8 |
| 163 | 60.7 | 102.6 | 138.6 | 54.2 | 51.3 | 9.1 |
| 165 | 238.4 | 123.6 | 227.1 | 113.8 | 224.6 | 241.9 |
| 210 | 134.1 | 184.4 | 28.4 | 42.7 | 12.1 | 10 |
| 214 | 189.2 | 173 | 50 | 164.7 | 109.2 | 250.7 |
| 219 | 26.8 | 30.4 | 13.3 | 78.6 | 34.3 | 34.8 |
| 224 | 215.8 | 139.5 | 255.3 | 96.9 | 181.3 | 163.2 |
| 225 | 167.5 | 35.2 | 98.1 | 32.4 | 221.4 | 54.3 |
| 226 | 49.4 | 43.4 | 84.3 | 16.4 | 141.3 | 27.2 |
| 309 | 340.8 | 33.7 | 32 | 22.8 | 29.8 | 45.9 |
| 332 | 15.9 | 39.9 | 67.1 | 14.3 | 88.5 | 25.2 |
| 346 | 14.3 | 37.9 | 36.5 | 33.5 | 28.7 | 37.4 |

13b10

| | pc__EA40....Signal | | | | | |
|---|---|---|---|---|---|---|
| SEQ ID | 205_VDX885TT | 206_VDX886TT | 207_VDX890TT | 208_VDX892TT | 209_VDX893TT | 210_VDX894TT |
| 83 | 22.9 | 81.5 | 34.1 | 159.2 | 88.7 | 43.6 |
| 142 | 26.4 | 22.6 | 8.7 | 10.8 | 18.5 | 23.6 |
| 136 | 28.1 | 18.2 | 9.6 | 14.6 | 17.3 | 22.5 |
| 137 | 207.7 | 94.4 | 68.8 | 83.7 | 17.8 | 140.4 |
| 152 | 77.3 | 225.5 | 227.5 | 95.5 | 301.1 | 59.9 |
| 160 | 294.5 | 164.6 | 110.3 | 84.5 | 105.6 | 163 |
| 163 | 140.9 | 34 | 25.9 | 9.3 | 41.9 | 83.5 |
| 165 | 241.8 | 101.4 | 193.6 | 161.9 | 112.8 | 190.6 |
| 210 | 416.8 | 24.1 | 45.5 | 15.1 | 26.9 | 15.6 |
| 214 | 323.3 | 139.5 | 148.2 | 174.1 | 104.5 | 142.1 |
| 219 | 131.8 | 12 | 25.9 | 48.9 | 14.4 | 96.2 |
| 224 | 215.3 | 136.2 | 126 | 193.8 | 195.4 | 67.1 |
| 225 | 49.5 | 105 | 54.2 | 97.4 | 33 | 38.8 |
| 226 | 181.1 | 55.2 | 34.1 | 4 | 15 | 125.1 |
| 309 | 89.7 | 47.4 | 58.6 | 17.8 | 23.5 | 72.3 |
| 332 | 73 | 66.8 | 48.2 | 27.5 | 41.1 | 25.3 |
| 346 | 13.6 | 107.7 | 145.3 | 12.7 | 18.3 | 20 |

13b11

| | 02014_40...._Signal | | | | |
|---|---|---|---|---|---|
| SEQ ID | 319_H133A_903TT | 320_H133A_904TT | 328_H133A_928TT | 330_H133A_932TT | 331_H133A_933TT |
| 83 | 58.3 | 226.6 | 92.7 | 79.2 | 106.8 |
| 142 | 14.5 | 118.3 | 7.4 | 8 | 13 |
| 136 | 18.9 | 87 | 29.4 | 86.9 | 9.3 |
| 137 | 24.4 | 174.5 | 25.7 | 135.9 | 31.8 |
| 152 | 178.1 | 137.4 | 176.6 | 151.1 | 166.2 |
| 160 | 83.2 | 205 | 93.5 | 103.6 | 73.3 |
| 163 | 31.5 | 100.2 | 19.2 | 40.5 | 33.4 |
| 165 | 223.6 | 235.9 | 161.5 | 132.8 | 132 |
| 210 | 35.5 | 99.9 | 16.4 | 124.9 | 17 |

| | | | | | |
|---|---|---|---|---|---|
| 214 | 118.8 | 178 | 25.6 | 115.6 | 117 |
| 219 | 15.1 | 80.2 | 27.7 | 54.2 | 38.2 |
| 224 | 174.5 | 323.7 | 223.3 | 203 | 185.3 |
| 225 | 170.3 | 200.3 | 180.3 | 157.3 | 157.2 |
| 226 | 65.2 | 26.9 | 25.2 | 4.6 | 35.7 |
| 309 | 11.1 | 270.7 | 15.9 | 91.5 | 18.6 |
| 332 | 12 | 77.4 | 15.1 | 7.2 | 7.3 |
| 346 | 30.4 | 387.2 | 25.4 | 204.4 | 14.7 |

13b12

| | EA02014_403...._Signal | | | | |
|---|---|---|---|---|---|
| SEQ ID | 75_H133A_922TT | 381_H133A_945TT | 382_H133A_946TT | 383_H133A_947TT | 384_H133A_948TT |
| 83 | 88.1 | 22.1 | 193.5 | 166.9 | 73.7 |
| 142 | 12 | 19.6 | 13.2 | 21.6 | 16.9 |
| 136 | 11.8 | 12.3 | 52 | 46.7 | 107.9 |
| 137 | 51.3 | 182.2 | 43.4 | 23.9 | 14.5 |
| 152 | 341 | 139 | 248.1 | 299.5 | 84.2 |
| 160 | 95.6 | 102 | 99.5 | 184.6 | 90.4 |
| 163 | 77.6 | 41.6 | 25.9 | 51.7 | 4.9 |
| 165 | 254.8 | 63.5 | 172.6 | 209.2 | 81.1 |
| 210 | 8.8 | 78.8 | 28.8 | 44 | 92.5 |
| 214 | 79 | 94.5 | 148.7 | 101.7 | 82.7 |
| 219 | 22.7 | 31.8 | 63.9 | 23.8 | 18.1 |
| 224 | 190.5 | 194.7 | 212 | 278.9 | 201.1 |
| 225 | 48.8 | 135.5 | 46.8 | 274.4 | 97.5 |
| 226 | 20 | 24 | 90.8 | 45.3 | 8.8 |
| 309 | 30.5 | 195 | 29.7 | 53.4 | 37.9 |
| 332 | 10.4 | 4.2 | 9.2 | 75 | 44 |
| 346 | 19.1 | 298.1 | 35.4 | 251.2 | 37.2 |

13b13

| | | | ThyFolCan_02014_400...._Signal | | |
|---|---|---|---|---|---|
| SEQ ID | 84_H133A_823TT | 85_H133A_824TT | 86_H133A_825TT | 88_H133A_827TT | 89_H133A_828TT |
| 83 | 242 | 70.1 | 71.2 | 24.8 | 145.4 |
| 142 | 19.8 | 12.5 | 35.7 | 17.7 | 19.6 |
| 136 | 35.7 | 21 | 59.4 | 20.3 | 9 |
| 137 | 86.1 | 23.7 | 116.3 | 18.2 | 48.6 |
| 152 | 271.9 | 199.6 | 213.1 | 140.9 | 202.5 |
| 160 | 134.5 | 134.1 | 415.1 | 162.4 | 154 |
| 163 | 64.5 | 46.1 | 82.1 | 15.5 | 57.8 |
| 165 | 189.3 | 222.6 | 239 | 223.8 | 145.3 |
| 210 | 8.8 | 236.3 | 50 | 21.8 | 33.8 |
| 214 | 33.1 | 71.3 | 121.6 | 157.3 | 100.8 |
| 219 | 24.8 | 64.8 | 82.1 | 14.1 | 60.2 |
| 224 | 213 | 96.4 | 371.4 | 159.1 | 92.8 |
| 225 | 164.9 | 103.3 | 224.4 | 145.2 | 98.3 |
| 226 | 10.3 | 5 | 24 | 30.5 | 21.6 |
| 309 | 15 | 33.3 | 38.4 | 14.9 | 19.5 |
| 332 | 77.6 | 55.7 | 124.1 | 10.6 | 84.5 |
| 346 | 39 | 124.6 | 31.5 | 29.5 | 21.3 |

13b14

| SEQ ID | Signal 02014_40318_H133A_840TT | fc__EA40212_VDX896TT | fc__EA40214_VDX898TT | fc__EA40215_VDX899TT | fc__EA40216_VDX900TT | fc__EA40217_VDX901TT | fc__EA40218_VDX902TT |
|---|---|---|---|---|---|---|---|
| 83 | 189.9 | 110.7 | 51.4 | 35.8 | 104.6 | 22.8 | 44.7 |
| 142 | 18.9 | 20.5 | 246.2 | 18.5 | 20.7 | 25.8 | 28.6 |
| 136 | 22.6 | 105.2 | 21.3 | 20.3 | 33.6 | 24.5 | 24.4 |
| 137 | 32.3 | 40.6 | 46.3 | 33.8 | 23.9 | 90.6 | 48.5 |
| 152 | 306 | 216.1 | 317.8 | 169.4 | 154.6 | 169.3 | 374.5 |
| 160 | 99.5 | 197.5 | 240.4 | 68.7 | 146.7 | 210.5 | 196.7 |
| 163 | 52.1 | 29.7 | 70.7 | 75.3 | 13.6 | 25.1 | 59.3 |
| 165 | 236.7 | 142.2 | 147 | 168 | 174.2 | 28.5 | 89.4 |
| 210 | 26.5 | 108.8 | 39.9 | 25.5 | 42.2 | 11 | 24.4 |
| 214 | 115.1 | 63.9 | 59 | 129.9 | 150.1 | 194.1 | 218.2 |
| 219 | 16.2 | 46.2 | 62.7 | 39.4 | 42.1 | 92.3 | 39.4 |
| 224 | 203.9 | 194.6 | 321.6 | 225.2 | 238 | 212.5 | 187.3 |
| 225 | 198 | 53.6 | 82.8 | 52.5 | 250.7 | 72 | 53.5 |
| 226 | 31 | 57.3 | 111.5 | 66.7 | 24.8 | 41 | 79.9 |
| 309 | 17.2 | 40.2 | 44.3 | 43 | 20.2 | 21.3 | 63.8 |
| 332 | 51.4 | 26.5 | 129.1 | 7.8 | 5.6 | 37.8 | 31.6 |
| 346 | 11 | 27.8 | 21.6 | 19.4 | 320.2 | 24.3 | 210.6 |

13b15

| SEQ ID | Signal fc__EA40221_VDX909TT | fc__EA40222_VDX910TT | EA02014_40386_H133A_954TT | EA02014_40394_H133A_967TT | EA02014_40395_H133A_968TT | EA02014_40396_H133A_969TT | EA02014_40397_H133A_970TT |
|---|---|---|---|---|---|---|---|
| 83 | 312.2 | 30.6 | 105.2 | 42.2 | 47.9 | 24.4 | 46.5 |
| 142 | 17.8 | 9.9 | 41.7 | 30.5 | 11.8 | 16.7 | 25.7 |
| 136 | 63.1 | 87.5 | 38 | 29.7 | 206.6 | 10.1 | 99.2 |
| 137 | 170.4 | 111.7 | 78.6 | 32.1 | 20.5 | 29.4 | 18.2 |
| 152 | 624.7 | 121 | 215.4 | 212.7 | 160.9 | 216.7 | 214.3 |
| 160 | 257.9 | 30.5 | 247.6 | 161.6 | 192.1 | 87.9 | 538.8 |
| 163 | 21.6 | 27 | 43.5 | 50.7 | 56.9 | 72.4 | 48.7 |
| 165 | 251.9 | 105 | 92.7 | 136.3 | 166.5 | 123.8 | 180.7 |
| 210 | 34 | 37.1 | 49 | 43.9 | 21.1 | 7.4 | 167.3 |
| 214 | 257.9 | 176.9 | 168.4 | 69.9 | 113.6 | 140.1 | 14 |
| 219 | 117.7 | 25.9 | 43.2 | 42.1 | 24 | 12.6 | 57.9 |
| 224 | 55.1 | 150 | 176.9 | 280.1 | 184.5 | 194.4 | 374.4 |
| 225 | 205.8 | 42.3 | 66.7 | 320.3 | 27 | 66.2 | 54.6 |
| 226 | 84.9 | 14.8 | 148.6 | 26 | 47.6 | 36.9 | 21.7 |
| 309 | 745.6 | 9.7 | 53.6 | 42 | 23.5 | 14.6 | 71 |
| 332 | 15.9 | 7.6 | 69.9 | 10.3 | 63.6 | 13.3 | 26.8 |
| 346 | 48 | 25 | 54.7 | 142.6 | 73.6 | 16.6 | 79.7 |

13b16

| SEQ ID | Signal EA02014_40405_H133A_982TT | EA02014_40406_H133A_983TT | pb1 | pb2 | pb3 | pb4 | pb5' |
|---|---|---|---|---|---|---|---|
| 83 | 58.1 | 37.8 | 524.7 | 768.1 | 855.6 | 1163.9 | 876.3 |
| 142 | 19.4 | 37.4 | 1289.3 | 1088.9 | 659.7 | 732.6 | 1415 |

| | | | | | | | |
|---|---|---|---|---|---|---|---|
| 136 | 94.2 | 11 | 1770.1 | 1622.7 | 523 | 979.3 | 617.3 |
| 137 | 17.6 | 111.2 | 1871 | 2554 | 1313.7 | 781.1 | 2087.1 |
| 152 | 255.8 | 37.6 | 9696.4 | 9404.8 | 1099.8 | 9900.7 | 7060 |
| 160 | 54.7 | 347.1 | 1273.2 | 1171.6 | 907 | 937.4 | 926.2 |
| 163 | 32.9 | 59.7 | 833 | 682.5 | 559.6 | 358.6 | 467.8 |
| 165 | 71.8 | 232.2 | 1217.8 | 986.3 | 1278.9 | 2537.1 | 1464.3 |
| 210 | 5.2 | 33.2 | 3401 | 2546.6 | 756.3 | 4893 | 1770.8 |
| 214 | 212.6 | 165 | 1384.3 | 1540 | 636.7 | 656.5 | 1213.9 |
| 219 | 14.1 | 43.6 | 1692.6 | 1911.7 | 912 | 2112.2 | 1417.9 |
| 224 | 137 | 228.2 | 2481.3 | 3104.2 | 1905.4 | 2292.4 | 2242.7 |
| 225 | 109.6 | 153.4 | 2367.8 | 2860.8 | 1887.6 | 2052.6 | 2116.6 |
| 226 | 41.6 | 11 | 797.2 | 969.9 | 617.1 | 626.6 | 920 |
| 309 | 20.7 | 30.6 | 5357.5 | 5904 | 3572.6 | 6641.9 | 3580.9 |
| 332 | 6.8 | 60.2 | 258.8 | 304.2 | 110.1 | 255.3 | 363.3 |
| 346 | 27 | 57.3 | 1584.2 | 1705.7 | 1507.5 | 1626.3 | 1587.1 |

13b17

Signal

| SEQ ID | pb6' | pb7' | pb8' | pd10 | pd11 | pd12 | pd9 |
|---|---|---|---|---|---|---|---|
| 83 | 818.3 | 333.4 | 692.1 | 709.8 | 699.7 | 926.2 | 534.7 |
| 142 | 1287.9 | 565.2 | 2004.2 | 821.9 | 904.8 | 1619.6 | 282.5 |
| 136 | 1039 | 962.2 | 1013.5 | 1147.9 | 840.9 | 599.8 | 284 |
| 137 | 1881.4 | 667.8 | 2058.1 | 2182.1 | 1678.9 | 1632.9 | 912.1 |
| 152 | 3657.7 | 2025 | 2993.5 | 11129.5 | 11824.2 | 11963.5 | 1607 |
| 160 | 1154.5 | 301.4 | 473.2 | 1849.5 | 1424.7 | 660 | 242 |
| 163 | 628.5 | 6534.3 | 1863.4 | 148.7 | 247.4 | 230.2 | 4365.5 |
| 165 | 1485.2 | 889 | 1904.7 | 1404.2 | 1560.4 | 1123.6 | 1919.6 |
| 210 | 2729.4 | 595.1 | 5286.8 | 7026.5 | 3861.8 | 1028.8 | 1724.8 |
| 214 | 871.1 | 442.3 | 644.5 | 936.1 | 939.1 | 852 | 347.7 |
| 219 | 1216.7 | 411.6 | 959.7 | 1085.9 | 1974.4 | 1580.2 | 245.9 |
| 224 | 2039.1 | 1488.3 | 1844.2 | 2280.1 | 2823.4 | 1252.3 | 1246.6 |
| 225 | 2214.8 | 1494.6 | 2186 | 1961.1 | 2628.3 | 1184.1 | 1230.7 |
| 226 | 822.2 | 437.3 | 684.5 | 540.5 | 839.5 | 1062.8 | 273.7 |
| 309 | 3787.2 | 1401.5 | 3537 | 3522.1 | 4518.1 | 5118.1 | 931.7 |
| 332 | 218.7 | 630.6 | 462.4 | 168.4 | 220.4 | 242.2 | 322.8 |
| 346 | 1682.4 | 909 | 2317.6 | 1193.5 | 1748.9 | 2159 | 622.7 |

Table 14 Control Genes

| SEQ ID NO: | Gene Name |
|---|---|
| Control Genes for Blood | |
| 142 | Bone marrow stromal cell antigen 1 (BST1) |
| 219 | Leucocyte immunoglobulin-like receptor-6b (LIR-6) |
| 309 | Bridging integrator 2 (BIN2) |
| Control Genes for Thyroid | |
| 9 | Cysteine-rich, angiogenic inducer, 61 (CYR61) |
| 12 | Selenoprotein P, plasma, 1 (SEPP1) |
| 18 | Insulin-like growth factor-binding protein 4 (IGFBP4) |

Table 15a

| SEQ ID | BN_800TT | BN_801TT | BN_802TT | BN_804TT | BN_805TT | BN_806TT | BN_807TT |
|---|---|---|---|---|---|---|---|
| 142 | 28.3 | 7.9 | 15 | 28.4 | 26.9 | 25.8 | 14.2 |
| 219 | 217 | 19.6 | 7.9 | 85.6 | 21.8 | 26.7 | 115.1 |
| 309 | 257.7 | 20.6 | 20.7 | 75.5 | 19.2 | 24 | 833.9 |
| | BN_871TT | BN_913TT | BN_914TT | BN_915TT | FA_917TT | FA_918TT | FA_919TT |
| 142 | 25.3 | 22.5 | 12.9 | 34.4 | 8.5 | 20.6 | 67.7 |
| 219 | 56.6 | 53.5 | 45.2 | 122.1 | 24.4 | 124 | 41.3 |
| 309 | 37.9 | 37 | 28.2 | 53 | 10.2 | 32.6 | 24.2 |
| | FA_818TT | FA_819TT | FA_820TT | FA_821TT | FA_822TT | FA_842TT | FA_862TT |
| 142 | 15 | 20.2 | 18.8 | 18.4 | 23 | 10 | 16.1 |
| 219 | 18.7 | 111.8 | 45.7 | 10.9 | 29.6 | 28.9 | 80.6 |
| 309 | 16.8 | 17.1 | 12.1 | 18.8 | 27.3 | 15.2 | 6.7 |
| | FA_863TT | FA_864TT | FA_865TT | FA_866TT | FA_867TT | FA_869TT | FA_907TT |
| 142 | 22.4 | 14.5 | 54.8 | 17.1 | 15.8 | 15 | 21.9 |
| 219 | 7.6 | 15.9 | 20.9 | 25 | 10 | 16.5 | 23.4 |
| 309 | 15.2 | 9.4 | 62.6 | 20.2 | 18.4 | 15.9 | 10.2 |
| | FA_908TT | FA_920TT | FA_938TT | FA_940TT | FA_941TT | Fa_EA40374 _921TT | Fa_EA40376 _923TT |
| 142 | 23.9 | 22.2 | 7.9 | 6.6 | 14.5 | 13.7 | 19.8 |
| 219 | 20.1 | 36.4 | 17.5 | 14.9 | 63.3 | 32.3 | 25.5 |
| 309 | 33.3 | 27.1 | 7.7 | 6.2 | 15.6 | 26.1 | 16 |
| | BN_EA40377 _924TT | Fa_EA40378 _925TT | Fa_EA40379 _926TT | BN_EA40380 _927TT | Fa_EA40387 _955TT | Fa_EA40388 _956TT | Fa_EA40389 _957TT |
| 142 | 16.5 | 57.7 | 22.9 | 18.9 | 16.7 | 14.2 | 10.8 |
| 219 | 15.3 | 10.2 | 95.6 | 18.5 | 23.3 | 51 | 29.1 |
| 309 | 17.8 | 31.8 | 32.6 | 49.2 | 18.7 | 11.4 | 26.7 |
| | Fa_EA40390_959TT | Fa_EA40391 _960TT | Fa_EA40392 _961TT | Fa_EA40393_962TT | PC_829TT | PC_830TT | PC_831TT |
| 142 | 21.4 | 18.6 | 16.5 | 21.9 | 26 | 34.7 | 22.4 |
| 219 | 25.7 | 67 | 20.8 | 21 | 36.8 | 136.4 | 64.9 |
| 309 | 44.2 | 20.6 | 34.1 | 35.2 | 88.8 | 136.6 | 48.8 |
| | PC_832TT | PC_834TT | PC_835TT | PC_836TT | PC_837TT | PC_838TT | PC_839TT |
| 142 | 19.1 | 16.4 | 25.8 | 12.1 | 22.5 | 15.4 | 16.1 |
| 219 | 24.3 | 19.1 | 25.9 | 11.7 | 13.6 | 23.8 | 26.8 |
| 309 | 22.1 | 23.6 | 40.5 | 16.5 | 33.9 | 24.7 | 340.8 |

(continued)

| SEQ ID | BN_800TT | BN_801TT | BN_802TT | BN_804TT | BN_805TT | BN_806TT | BN_807TT |
|---|---|---|---|---|---|---|---|
| | PC_879TT | PC_881TT | PC_882TT | PC_883TT | PC_884TT | PC_885TT | PC_886TT |
| 142 | 21.2 | 23.5 | 7.6 | 21.5 | 30.6 | 26.4 | 22.6 |
| 219 | 30.4 | 13.3 | 78.6 | 34.3 | 34.8 | 131.8 | 12 |
| 309 | 33.7 | 32 | 22.8 | 29.8 | 45.9 | 89.7 | 47.4 |
| | PC_890TT | PC_892TT | PC_893TT | PC_894TT | PC_903TT | PC_904TT | PC_928TT |
| 142 | 8.7 | 10.8 | 18.5 | 23.6 | 14.5 | 118.3 | 7.4 |
| 219 | 25.9 | 48.9 | 14.4 | 96.2 | 15.1 | 80.2 | 27.7 |
| 309 | 58.6 | 17.8 | 23.5 | 72.3 | 11.1 | 270.7 | 15.9 |
| | PC_932TT | PC_933TT | Pc_EA40375 _922TT | Pc_EA40381_945TT | Pc_EA40382 _946TT | Pc_EA40383 _947TT | Pc_EA40384 _948TT |
| 142 | 8 | 13 | 12 | 19.6 | 13.2 | 21.6 | 16.9 |
| 219 | 54.2 | 38.2 | 22.7 | 31.8 | 63.9 | 23.8 | 18.1 |
| 309 | 91.5 | 18.6 | 30.5 | 195 | 29.7 | 53.4 | 37.9 |
| | FC_823TT | FC_824TT | FC_825TT | FC_827TT | FC_828TT | FC_840TT | FC_896TT |
| 142 | 19.8 | 12.5 | 35.7 | 17.7 | 19.6 | 18.9 | 20.5 |
| 219 | 24.8 | 64.8 | 82.1 | 14.1 | 60.2 | 16.2 | 46.2 |
| 309 | 15 | 33.3 | 38.4 | 14.9 | 19.5 | 17.2 | 40.2 |
| | FC_898TT | FC_899TT | FC_900TT | FC_901TT | FC_902TT | FC_909TT | FC_910TT |
| 142 | 246.2 | 18.5 | 20.7 | 25.8 | 28.6 | 17.8 | 9.9 |
| 219 | 62.7 | 39.4 | 42.1 | 92.3 | 39.4 | 117.7 | 25.9 |
| 309 | 44.3 | 43 | 20.2 | 21.3 | 63.8 | 745.6 | 9.7 |
| | Fc_EA40386_954TT | Fc_EA40394 _967TT | Fc_EA40395 _968TT | Fc_EA40396_969TT | Fc_EA40397 _970TT | Fc_EA40405 _982TT | Fc_EA40406 _983TT |
| 142 | 41.7 | 30.5 | 11.8 | 16.7 | 25.7 | 19.4 | 37.4 |
| 219 | 43.2 | 42.1 | 24 | 12.6 | 57.9 | 14.1 | 43.6 |
| 309 | 53.6 | 42 | 23.5 | 14.6 | 71 | 20.7 | 30.6 |
| | pb1_Signal | pb2_Signal | pb3_Signal | pb4_Signal | pb5'_Signal | pb6'_Signal | pb7'_Signal |
| 142 | 1289.3 | 1088.9 | 659.7 | 732.6 | 1415 | 1287.9 | 565.2 |
| 219 | 1692.6 | 1911.7 | 912 | 2112.2 | 1417.9 | 1216.7 | 411.6 |
| 309 | 5357.5 | 5904 | 3572.6 | 6641.9 | 3580.9 | 3787.2 | 1401.5 |
| | pb8'_Signal | pd10_Signal | pd11_Signal | pd12_Signal | pd9_Signal | | |
| 142 | 2004.2 | 821.9 | 904.8 | 1619.6 | 282.5 | | |
| 219 | 959.7 | 1085.9 | 1974.4 | 1580.2 | 245.9 | | |

(continued)

| SEQ ID | BN_800TT | BN_801TT | BN_802TT | BN_804TT | BN_805TT | BN_806TT | BN_807TT |
|---|---|---|---|---|---|---|---|
| 309 | 3537 | 3522.1 | 4518.1 | 5118.1 | 931.7 | | |

Table 15b

| | Signal | | | | | | |
|---|---|---|---|---|---|---|---|
| SEQ ID | PC_984TT | PC_986TT | FA_987TT | PC_988TT | PC_989TT | FA_992TT | FA_993TT |
| 142 | 25.1 | 125.8 | 18.5 | 11.4 | 13.7 | 11.8 | 113.6 |
| 219 | 17.5 | 25.7 | 22.6 | 11.3 | 32.4 | 61.9 | 25.2 |
| 309 | 27.6 | 661.8 | 15.7 | 47.4 | 29.8 | 33.2 | 28.4 |
| | FA_994TT | FA_995TT | FA_996TT | FA_998TT | FA_999TT | FA_1001TT | FA_1002TT |
| 142 | 20.6 | 25.2 | 34.1 | 21.8 | 31.4 | 12.7 | 74.6 |
| 219 | 25.9 | 36.3 | 41.5 | 24.9 | 16.4 | 26.5 | 25.5 |
| 309 | 16.8 | 26.3 | 19.4 | 22.5 | 14.6 | 32.3 | 47.7 |
| | FA_1004TT | FA_1005TT | FA_1006TT | FA_1010TT | FA_1013TT | FA_1014TT | FA_1017TT |
| 142 | 24.7 | 20.1 | 67.7 | 14.9 | 48.9 | 29.8 | 26.9 |
| 219 | 27.5 | 11.7 | 53.3 | 112.9 | 35.1 | 39.5 | 27.2 |
| 309 | 39.5 | 10.3 | 34.6 | 83.2 | 46.8 | 20 | 25.7 |
| | FA_1018TT | FA_1020TT | FA_1023TT | FA_1024TT | FA_1026TT | FA_1027TT | FA_1028TT |
| 142 | 25.1 | 25.5 | 35.6 | 26.9 | 27.9 | 15.2 | 24.5 |
| 219 | 66.3 | 26 | 6.1 | 26 | 59.3 | 18.8 | 31.7 |
| 309 | 31 | 37.4 | 20.6 | 46.9 | 51.4 | 8.8 | 33.3 |
| | FA_1029TT | FA_1030TT | FA_1031TT | FA_1032TT | FA_1034TT | FA_1035TT | FC_1037TT |
| 142 | 10.6 | 14 | 20 | 21.6 | 31.9 | 17.1 | 17.8 |
| 219 | 12.5 | 33.5 | 15.1 | 15 | 68.5 | 25.6 | 63.5 |
| 309 | 20.7 | 13 | 21.5 | 32.2 | 52.6 | 24.6 | 26.2 |
| | PC_1039TT | PC_1040TT | PC_1041TT | PC_1042TT | PC_1043TT | PC_1044TT | PC_1045TT |
| 142 | 16.1 | 22.7 | 99.5 | 129.8 | 20.7 | 79.2 | 16.4 |
| 219 | 27.9 | 29.5 | 23.6 | 29 | 21.5 | 19.8 | 38.4 |
| 309 | 585.3 | 37.6 | 48.7 | 41 | 32.8 | 59.2 | 112.3 |
| | PC_1046TT | PC_1047TT | PC_1048TT | PC_1049TT | PC_1050TT | PC_1051TT | PC-FV_1052TT |
| 142 | 22.4 | 81.2 | 24.7 | 37.5 | 25.9 | 26.6 | 26.4 |
| 219 | 11.1 | 97.5 | 61.8 | 14.9 | 14.7 | 109.1 | 30.6 |
| 309 | 22.5 | 207.8 | 35.4 | 64 | 39.5 | 56.7 | 28.2 |
| | PC_1053TT | PC_1054TT | PC_1055TT | PC_1059TT | PC_1060TT | PC_1061TT | PC_1062TT |
| 142 | 12.8 | 18.5 | 24.4 | 22.7 | 24.6 | 15.8 | 25.6 |
| 219 | 12.6 | 19.6 | 10.2 | 24.5 | 82.6 | 53.7 | 20.7 |
| 309 | 20.4 | 22.1 | 24 | 18.9 | 32.3 | 54.5 | 29.1 |

(continued)

| | | | | | | | |
|---|---|---|---|---|---|---|---|
| | PC-FV_1064TT | PC-FV_1065TT | PC-FV_1066TT | PC-FV_1067TT | PC-FV_1068TT | PC-FV_1069TT | PC-FV_1071 TT |
| 142 | 16.7 | 11.3 | 25.5 | 17.4 | 17.5 | 14 | 16.7 |
| 219 | 174.4 | 20.3 | 132.4 | 24.8 | 12.3 | 14.8 | 18.8 |
| 309 | 30.4 | 169.6 | 49.4 | 16.9 | 135.5 | 18.1 | 7.6 |
| | PC-FV_1072TT | FA_1073TT | FA_1074TT | FA_1075TT | FA_1076TT | FC_1077TT | FC_1078TT |
| 142 | 14 | 25.5 | 22.7 | 27 | 16.4 | 15.8 | 38.9 |
| 219 | 8.7 | 73.2 | 57 | 54.3 | 69.9 | 20 | 17.1 |
| 309 | 29.9 | 145.4 | 21 | 53 | 24.3 | 9.1 | 32.9 |
| | FC_1079TT | PC-FV_1080TT | PC-FV_1081TT | FC_1082TT | pb1 | pb2 | pb3 |
| 142 | 31.9 | 10.2 | 22 | 19.5 | 1289.3 | 1088.9 | 659.7 |
| 219 | 40.1 | 17.4 | 38.3 | 88.1 | 1692.6 | 1911.7 | 912 |
| 309 | 27.1 | 23.4 | 40.8 | 12.9 | 5357.5 | 5904 | 3572.6 |
| | pb4 | pb5' | pb6' | pb7' | pb8' | pd10 | pd11 |
| 142 | 732.6 | 1415 | 1287.9 | 565.2 | 2004.2 | 821.9 | 904.8 |
| 219 | 2112.2 | 1417.9 | 1216.7 | 411.6 | 959.7 | 1085.9 | 1974.4 |
| 309 | 6641.9 | 3580.9 | 3787.2 | 1401.5 | 3537 | 3522.1 | 4518.1 |
| | pd12 | pd9 | | | | | |
| 142 | 1619.6 | 282.5 | | | | | |
| 219 | 1580.2 | 245.9 | | | | | |
| 309 | 5118.1 | 931.7 | | | | | |

F. Signature Normalized to Control Genes

We further examined our 5-gene and 4-gene signatures by normalizing these genes to the three selected thyroid control genes as an algorithm for gene chip data normalization. The average fluorescent intensities of the three thyroid control genes were used for signature gene signal normalization. The performance of both signatures improved slightly when these two signatures were normalized. The sensitivity and specificity of the two signatures are listed in Table 16, and the ROC curves are shown in Figure 4a and 4b.

Table 16. The performances of the 4-gene and 5-gene signatures normalized to control genes

| | 4-Gene Signature | |
|---|---|---|
| | Tumor | Benign |
| Positive | 33 | 11 |
| Negative | 3 | 27 |
| Sensitivity | 92% (0.78, 0.97) | |
| Specificity | 71% (0.55, 0.83) | |
| | 5-Gene Signature | |
| | Tumor | Benign |
| Positive | 33 | 8 |
| Negative | 3 | 30 |
| Sensitivity | 92% (0.78, 0.97) | |
| Specificity | 79% (0.64, 0.89) | |

G. Signature Validation with Two-Round Amplified Probes

To determine if the FNA samples lack sufficienTThyroid cells to provide enough probe material for hybridizing to the Affymetrix U133a gene chips after one round of amplification, two-round amplification of the target RNAs we performed two-round amplification with 47 samples that are among the 74 independent validation sample set. The data obtained show thaTThe performances of the 5-gene and 4-gene signatures are identical with either one-round or two-round amplifications. The ROC curves of the two gene signatures with two different target preparations are shown in Figures 5a, 5b, 5c, and 5d.

**Example 4**

**Cross validation with independent samples**

A. Cross Validation with the 83 Independent Fresh Frozen Thyroid Samples

83 independenTThyroid samples were processed and profiled with the U133a chip. The number of samples in each category is list in Table 17.

Table 17. Fresh Frozen sample collection for signature validation

| **Sample Type** | **Number of Samples** |
|---|---|
| Follicular Adenoma | 18 |
| Follicular Thyroid Carcinoma | 1 |
| Papillary Carcinoma, Follicular Variant | 18 |
| Papillary Thyroid Carcinoma | 11 |
| Adenomatoid Nodules | 18 |
| Adenomatoid Nodules w/Hashimoto | 1 |
| Multinodular Hyperplasia | 16 |

The performance of the 4-gene signature and the 5-gene signature was assessed with LDA using the same cut-off value as in the training set. Both signatures gave equivalent performance in these samples, and they are comparable with the performance in the 98 training set. The sensitivity and specificity for both signatures are shown in Table 18, and the ROC curves are demonstrated in Figures 6a and 6b.

Table 18. 4-Gene and 5-gene signatures performance in 83 validation samples

| | 4-Gene Signature | |
|---|---|---|
| | Tumor | Benign |
| Positive | 20 | 11 |
| Negative | 10 | 42 |
| Sensitivity | 67% (0.49, 0.81) | |
| Specificity | 79% (0.67,0.88) | |
| | 5-Gene Signature | |
| | Tumor | Benign |
| Positive | 24 | 24 |
| Negative | 6 | 29 |
| Sensitivity | 80% (0.63, 0.90) | |
| Specificity | 55% (0.41, 0.67) | |

B. Signature Validation with the 47 Fine Needle Aspirate (FNA) Thyroid Samples

47 thyroid FNA samples were processed and profiled with the U133a chip. The number of samples in each category is list in Table 19.

Table 19. FNA sample collection for signature validation

| **Sample Type** | **Number of Samples** |
|---|---|
| Follicular Adenoma | 10 |
| Follicular Thyroid Carcinoma | 2 |
| Papillary Carcinoma, Follicular Variant | 3 |
| Papillary Thyroid Carcinoma | 13 |
| Adenomatoid Nodules | 10 |
| Adenomatoid Nodules w/Hashimoto | 1 |
| Multinodular Hyperplasia | 8 |

The performance of the 4-gene signature and the 5-gene signature was assessed with LDA model. Both signatures gave equivalent performance in the FNA samples, and they are comparable with the performance in the 98 training set. The sensitivity and specificity for both signatures are shown in Table 20, and the ROC curves are demonstrated in Figures 7a and 7b.

Table 20. 4-Gene and 5-gene signatures performance in 47 FNA samples

| | 4-Gene Signature | |
|---|---|---|
| | Tumor | Benign |
| Positive | 15 | 4 |
| Negative | 3 | 25 |
| Sensitivity | 94% (0.74, 0.99) | |
| Specificity | 62% (0.44, 0.77) | |
| | 5-Gene Signature | |
| | Tumor | Benign |
| Positive | 17 | 10 |
| Negative | 1 | 19 |
| Sensitivity | 94% (0.74, 0.99 | |
| Specificity | 66% (0.47, 0.80) | |

C. Signature Performance in 28 Paired Fresh Frozen and FNA Thyroid Samples

Within the 83 fresh frozen and the 47 FNA sample collections there are 28 samples that were from the same patient. The direct comparison of our signatures in these paired samples demonstrates how well the signature will translate into the final molecular assay. The performance of the 4-gene signature and the 5-gene signature was assessed with the LDA model. Both signatures gave equivalent performance in the fresh frozen and FNA samples. These results demonstrated that our 4-gene and 5-gene signatures can perform equally well in both sample types, and proved the approach using fresh frozen samples for gene/signature identification is valid. The sensitivity and specificity for both signatures are shown in Table 21, and the ROC curves are demonstrated in Figures 8a and 8b.

Table 21. 4-Gene and 5-gene signatures performance in 28 matched fresh frozen and FNA samples

| | 4-Gene Signature | | | |
|---|---|---|---|---|
| | Fresh Frozen | | FNA | |
| | Tumor | Benign | Tumor | Benign |
| Positive | 10 | 4 | 12 | 7 |
| Negative | 3 | 11 | 1 | 8 |
| Sensitivity | 77% (0.50 - 0.92) | | 92% (0.67 - 0.99) | |
| Specificity | 73% (0.48 - 0.89) | | 53% (0.30 - 0.75) | |
| | 5-Gene Signature | | | |
| | Fresh Frozen | | FNA | |
| | Tumor | Benign | Tumor | Benign |
| Positive | 11 | 7 | 12 | 7 |
| Negative | 2 | | 1 | 8 |
| Sensitivity | 85% (0.58 - 0.96) | | 92% (0.67 - 0.99) | |
| Specificity | 53% (0.30 - 0.75) | | 53% (0.30 - 0.75) | |

Example 5

Real-time quantitative RT-PCR

Sample Acquisition:

In order to determine whether a subset of the gene profiles and/or controls would give adequate specificity and sensitivity with RT-PCR, the following experiment was performed. The following has the advantage of requiring only one round of RNA amplification.

A total of 107 thyroid biopsies were analyzed in our study: 26 follicular adenoma, 23 follicular carcinoma, 38 papillary carcinoma, 5 normal, 3 papillary carcinoma follicular variant, 3 Hashimoto thyroiditis, 2 microfollicular adenoma, 1 diffuse goiter, 1 goiter with papillary hyperplasia, 1 Hurthle cell adenoma, 1 hyperplasia with papillary structure, 1 multinodular goiter, 1 oncocytic hyperplasia, 1 thyroiditis. Total RNA isolation was extracted by using the Trizol reagent according to the manufacturer's instructions. RNA concentrations were determined by absorbance readings at 260 nm with the Gene-Spec (Hitachi) spectrophotometer. The isolated RNA was stored in RNase-free water at -80°C until use.

**Primer and Probe Design:**

The primers and hydrolysis probes were designed using Oligo 6.0 and the Genebank sequences for thyroid cancer status markers (Table 22). These primers and probe sets were designed such thaTThe annealing temperature of the primers was 62°C and the probes 5-10°C higher and amplicon size ranges from 100-150bp. Genomic DNA amplification was excluded by designing our primers around exon-intron splicing sites. Hydrolysis probes were labeled aTThe 5' nucleotide with FAM as the reporter dye and at 3' nucleotide with TAMRA as the quenching dye.

*Table 22*

| Target | accession # | Forward Primer (SEQ ID NO:) | Reverse Primer (SEQ ID NO:) | Probe Sequence (SEQ ID NO:) | product Length |
|---|---|---|---|---|---|
| SGENE | NM_003020 | gaaagcggaqgagtgtcaatcca (364) | ggttttcgtctagcatcttctcttta (365) | atctacaaggacagagactggataatgttg (366) | 130 |
| TESTICAN1 | AF231124 | gtgagctgtgaagaggagcaggaa(367) | ctttggtcccagctcccgttca (368) | cctcaggggattttggcagtggtgggtccg (369) | 102 |
| GABRE | NM_004961 | taaactccgccatcctcgtatcaa (370) | cagtggtgacaatctggcacacaa (371) | ccgtgcccatgcccgtacccgtgca (372) | 113 |
| CDH3 | NM_001793 | ctgaagcaggatacatatgacgtgca (373) | agggtccagggcaggtttcgaca (374) | catggcagtcgcacacagtggccctga (375) | 124 |
| FN1 | NM_002026 | tggtgccatgacaatggtgtgaacta (376) | catcatcgtaanangttgcctcatga (377) | aagattggagagaagtgggaccgtcaggga (378) | 148 |
| TPO-1 | NM_000547 | catctgtgacaacactggcctca (379) | gccacacttgtcgcttgaggaa (380) | caaattccccgaagactttgagtcttgtgacagc (381) | 148 |
| TPO-2 | NM_000547 | aacctgcgtaaactccggaggc (382) | gccagtgcgtgtccacctgca (383) | ctcgggtgacttggatctccatgtcgctgg (384) | 124 |
| KCNAB1-1 | NM_003471 | tgaaagttccagggcttcactgaa (385) | agctgaggtagtgtcatcccaga (386) | ctaccagtggttgaaagaaagaattgtaagtgaag (387) | 138 |
| KCNAB1-2 | NM_003471 | tagctgttgcgtggtgcctgagaa (388) | tgatgtcatctttgggagaacctgaa (389) | aaggtgtgagttctgtgctcctgggatcat (390) | 119 |
| FABP4-1 | NM_001442 | gaaagaagtaggagtgggctttgcca (391) | ggcccagtatgaaggaaatctcagta (392) | aaaggtactttcagatttaatggtgatcacatcc (393) | 143 |
| FABP4-2 | NM_001442 | aggaaagtcaagagcaccataacctta (394) | gacgcattccaccaccagtttatca (395) | ttgattttccatcccatttctgcacatgta (396) | 123 |
| DOI1-1 | NM_000792 | gggtaaatctggcccttggaactaca (397) | cccgttggtcacctagaattgaggta (398) | cccagaggaagttcgtgctgttctggaaaa (399) | 106 |
| DOI1-2 | NM_000792 | tgcatcagatggctgggctttta (400) | gctctggttctgcatggtgtcca (401) | catcagaaatcaccagaaccttcaggatcg (402) | 142 |
| B-ACTIN | NM_001101 | tcacccacactgtgcccatctacga (403) | cagcgpaaccgctcattgccaatgg (404) | atgccctcccccatgccatcctgcgt (405) | 295 |
| GOLGIN67 | NM_015003 | gcatggtgatctttgtgaggcga (406) | ctcctggtggtcctgcatctca (407) | ctcaccaacagcgtggagcctgcacaagga (408) | 139 |
| PAX8 | BC001060 | actccagcttgctgagttcccca (409) | actccagcttgctgagttcccca (410) | attattacagttccacatcaaggccgagtgca (411) | 125 |
| HERC | NM_003922 | gggtgcttttcatgaggtttgtgtca (412) | taaggtaggcagactgtcgtaaggcc (413) | ccaacactgctgacatttctcagagatttcaaat (414) | 219 |
| DDIT3 | NM_004083 | cctggaaatgaagaggaagaatcaa (415) | agctctgactggaatctggagagtga (416) | aatcttcaccactcttgaccctgcttctctgg (417) | 142 |
| ITM1 | NM_152713 | tggctggtcaggatatacaaggtaaa (418) | tcaacgtcctaaatgtgatgtgctca (419) | cctggataatcgaggcttgtcaaggacataaa (420) | 117 |
| C1OF2 | NM_052966 | tctgaaagtgataaaggaagctgcta (421) | ctttagatctgttaagctggacacac (422) | cttgaagaaacacaacttatttgaagataacatg (423) | 103 |
| MOT8 | NM_018836 | acggcctataacgagaccctgca (424) | gaagaggagggtcggtttccattaa (425) | ttctcacgagtgggtcagcrgcatctgtgc (426) | 133 |
| ARG2 | NM_00172 | atttgaccctacactggctccagc (427) | gatccagtgctgatagcaaccctgta (428) | actcctgttgtcgggggactaacctatcgaga (429) | 119 |

**Real-Time Quantitative RT-PCR:**

Gene specific real-time quantitative RT-PCR amplification of 21 thyroid cancer status genes and a housekeeping gene was performed using the TaqMan One-Step RT-PCR Master Mix (2x) (Applied Biosystems) and the ABI Prism 7900HT sequence detection system (Applied Biosystems). In a 25μl one-step reaction total RNA (10ng) was added to a mix that contained: 1x RT-PCR Master Mix, 0.25U/μl Multiscribe Enzyme, 0.6uM primers and 0.25μM probe. Cycling parameters were 48°C for 30min and 95°C 10min, followed by 40 cycles of 95°C 15sec and 62°C 1min. Real-time PCR monitoring was achieved by measuring fluorescent signal aTThe end of the annealing phase for each cycle. The number of cycles to reach the fluorescence threshold was defined as the cycle threshold (Ct value). To minimize the errors arising from the integrity of the RNA in the samples β-actin mRNA was amplified as an internal reference. External standards were prepared by 10-fold serial dilutions of known thyroid cancer positive RNA and used to ensure linearity throughout our assays. Results were expressed in mean Ct value and samples were excluded that had a standard deviation greater then one. The results are provided in Tables 23a, 23b, 23c, 24a and 24b.

The data show thaTThe two gene signature shown in Table 23b is not as sensitive and specific as the four-gene signature from which it was derived. Table 24 shows that use of the PAX8 gene in an RT-PCR reaction as a control for thyroid-specific tissue is effective in an RT-PCR reaction.

Table 23a

| | | | | Thyroid markers | | | | | | | | | | | | | | | |
|---|---|---|---|---|---|---|---|---|---|---|---|---|---|---|---|---|---|---|---|
| | | | | CDH3 | FN1 | GAB | TEST | SGE NE | KCN12 57 | KCN1 120 | TPO2 166 | TPO2 592 | FABP 4;110 | FABP 4-2 | DIO1; 480 | DIO1; 687 | DDIT 3 | C1OF 24 | ltm1 |
| | | % total | | 44.86 | 44.86 | 48.60 | 58.88 | 57.01 | 62.62 | 48.60 | 41.12 | 30.84 | 38.32 | 42.06 | 39.25 | 56.07 | 44.86 | 45.79 | 42.99 |
| | | % false positives | | | | | | | | | | | | | | | | | |
| [redacted] | | | | | | | | | | | | | | | | | | | |
| Outliers | | | | | | | | | | | | | | | | | | | |
| Markers with best performance | | | | | | | | | | | | | | | | | | | |
| | | | Bactin | CDH3 | FN1 | GAB | TEST | SGE NE | KCN12 57 | KCN1 120 | TPO2 166 | TPO25 92 | FABP 4;110 | FABP 4-2 | DIO1; 480 | DIO1; 687 | DDIT 3 | C1OF 24 | ltm1 |
| 70 | 915 | ben DG | 26.69 | 34.23 | 29.35 | 31.94 | 32.00 | 33.87 | [redacted] | 32.46 | [redacted] | [redacted] | [redacted] | [redacted] | [redacted] | [redacted] | 29.54 | 31.01 | 26.46 |
| 7 | 818 | ben FA | [illegible] | 50.00 | 50.00 | 50.00 | 50.00 | 50.00 | 50.00 | 50.00 | 50.00 | 50.00 | 50.00 | 50.00 | 50.00 | 50.00 | 50.00 | 50.00 | 50.00 |
| 24 | 842 | ben FA | 25.92 | 31.74 | 29.97 | 30.96 | 32.98 | 32.54 | [redacted] | [redacted] | 23.88 | 24.44 | 32.08 | 34.09 | 27.82 | 27.42 | 29.76 | 33.81 | 27.09 |
| 28 | 862 | ben FA | 26.86 | 33.35 | 30.38 | 30.92 | 41.29 | 50.00 | 33.60 | 32.95 | [redacted] | [redacted] | 32.02 | 32.16 | [redacted] | [redacted] | 28.44 | 38.86 | 27.25 |
| 29 | 863 | ben FA | 24.56 | 30.03 | 29.91 | [redacted] | [redacted] | [redacted] | [redacted] | [redacted] | [redacted] | [redacted] | [redacted] | [redacted] | [redacted] | [redacted] | [redacted] | 30.25 | [redacted] |
| 30 | 864 | ben FA | 26.15 | 32.18 | 30.74 | 31.97 | 34.52 | 30.22 | [redacted] | [redacted] | [redacted] | [redacted] | 30.06 | 30.15 | [redacted] | [redacted] | 28.23 | 31.93 | 25.59 |
| 31 | 865 | ben FA | 26.21 | 32.94 | 27.23 | 31.29 | 32.07 | 30.73 | [redacted] | [redacted] | [redacted] | [redacted] | 31.25 | 31.31 | [redacted] | [redacted] | 30.19 | 47.61 | 26.83 |
| 32 | 866 | ben FA | 27.00 | 33.10 | 29.02 | 32.26 | 34.44 | 32.77 | [redacted] | [redacted] | [redacted] | 23.43 | 33.18 | 33.81 | [redacted] | [redacted] | 29.26 | 31.60 | 26.44 |
| 33 | 867 | ben FA | 24.89 | 31.72 | 30.52 | 31.88 | 32.29 | 33.85 | [redacted] | [redacted] | [redacted] | [redacted] | [redacted] | [redacted] | [redacted] | [redacted] | 29.33 | 31.53 | 26.71 |
| 34 | 869 | ben FA | 24.99 | [redacted] | 28.94 | [redacted] | [redacted] | [redacted] | [redacted] | [redacted] | [redacted] | [redacted] | 30.69 | 30.08 | [redacted] | [redacted] | [redacted] | 29.46 | [redacted] |
| 64 | 907 | ben FA | 25.09 | 31.42 | 26.64 | [redacted] | 33.83 | 35.86 | [redacted] | [redacted] | [redacted] | [redacted] | 31.21 | 50.00 | [redacted] | [redacted] | 29.63 | 30.46 | 25.79 |
| 73 | 920 | ben FA | 25.37 | 34.29 | 28.40 | 30.93 | 33.48 | 50.00 | 31.87 | 32.83 | [redacted] | [redacted] | 30.14 | [redacted] | [redacted] | [redacted] | 29.53 | [redacted] | [redacted] |
| 83 | 937 | ben FA | 23.32 | 33.55 | 28.06 | 32.56 | 34.45 | 36.65 | [redacted] | [redacted] | [redacted] | [redacted] | [redacted] | [redacted] | [redacted] | [redacted] | [redacted] | 32.90 | [redacted] |
| 84 | 938 | ben FA | 23.39 | 31.45 | [redacted] | 31.58 | [redacted] | [redacted] | [redacted] | [redacted] | [redacted] | [redacted] | [redacted] | [redacted] | [redacted] | [redacted] | [redacted] | 29.88 | [redacted] |
| 85 | 939 | ben FA | 25.58 | 34.57 | 29.28 | 31.87 | 32.01 | 34.13 | 32.02 | 32.95 | [redacted] | 23.85 | 31.29 | [redacted] | 27.69 | 27.32 | 30.66 | 31.70 | 28.01 |
| 86 | 940 | ben FA | 25.33 | 34.29 | 27.70 | 31.38 | 31.19 | [redacted] | [redacted] | [redacted] | [redacted] | [redacted] | 33.44 | 31.36 | [redacted] | [redacted] | 28.28 | 31.78 | [redacted] |
| 87 | 941 | ben FA | 26.84 | 31.44 | 31.05 | 33.27 | 31.66 | 31.12 | [redacted] | [redacted] | 23.57 | 24.30 | 32.70 | 30.48 | 26.36 | [redacted] | 30.37 | 40.24 | 26.75 |
| 88 | 957 | ben FA | 24.37 | 31.53 | 27.42 | [redacted] | [redacted] | 31.34 | [redacted] | [redacted] | 24.86 | 25.62 | [redacted] | [redacted] | [redacted] | [redacted] | 28.77 | 31.38 | [redacted] |
| 90 | 923 | ben FA | 23.12 | 30.71 | 26.38 | [redacted] | [redacted] | 30.94 | [redacted] | [redacted] | [illegible] | [illegible] | [redacted] | [redacted] | [redacted] | [redacted] | [redacted] | 30.17 | [redacted] |
| 91 | 926 | ben FA | 22.89 | [redacted] | [redacted] | [redacted] | [redacted] | 30.63 | [redacted] | [redacted] | [illegible] | [illegible] | 30.71 | [redacted] | [redacted] | [redacted] | 28.06 | [redacted] | [redacted] |
| 92 | 955 | ben FA | 26.97 | 31.90 | 28.78 | 31.72 | 33.70 | [redacted] | [redacted] | [redacted] | 24.36 | 25.76 | 35.04 | 36.25 | 28.43 | 27.07 | 28.34 | 31.37 | 25.77 |
| 93 | 956 | ben FA | 27.07 | 31.06 | 29.52 | 30.12 | 32.02 | 30.01 | [redacted] | [redacted] | [redacted] | 24.09 | 31.88 | 30.98 | 27.17 | [redacted] | 28.79 | 32.24 | 25.83 |
| 94 | 959 | ben FA | [illegible] | 50.00 | 50.00 | 50.00 | 50.00 | 50.00 | 50.00 | 50.00 | 50.00 | 50.00 | 50.00 | 50.00 | 50.00 | 50.00 | 50.00 | 50.00 | 46.83 |
| 95 | 960 | ben FA | 25.12 | 32.35 | 27.30 | [redacted] | [redacted] | [redacted] | [redacted] | [redacted] | [redacted] | [redacted] | [redacted] | [redacted] | [redacted] | [redacted] | 28.19 | 30.38 | 25.70 |
| 96 | 921 | ben FA | 22.10 | [redacted] | [redacted] | [redacted] | 31.95 | 31.03 | [redacted] | [redacted] | [redacted] | [redacted] | [redacted] | [redacted] | [redacted] | [redacted] | [redacted] | 34.76 | [redacted] |
| 97 | 961 | ben FA | [illegible] | 32.16 | 30.98 | 31.66 | [redacted] | 35.61 | [redacted] | [redacted] | [redacted] | 24.61 | 31.93 | 30.46 | 26.99 | [redacted] | 30.93 | 31.46 | 27.40 |
| 98 | 962 | ben FA | 23.81 | [redacted] | 28.66 | [redacted] | [redacted] | [redacted] | [redacted] | [redacted] | [redacted] | [redacted] | [redacted] | [redacted] | [redacted] | [redacted] | [redacted] | 29.61 | [redacted] |
| 106 | 924 | ben GPH | 21.48 | [redacted] | [redacted] | [redacted] | [redacted] | [redacted] | [redacted] | [redacted] | 24.88 | 26.58 | 31.30 | [redacted] | 26.64 | [redacted] | 41.06 | 50.00 | 34.50 |
| 35 | 871 | ben | 23.42 | 31.13 | [redacted] | 31.80 | [redacted] | [redacted] | [redacted] | 32.50 | 23.62 | 24.19 | 30.68 | 31.30 | [redacted] | [redacted] | [redacted] | [redacted] | [redacted] |

| | | HCA | | | | | | | | | | | | | | | | | |
|---|---|---|---|---|---|---|---|---|---|---|---|---|---|---|---|---|---|---|---|
| 2 | 805 | ben HPS | 26.84 | 33.76 | 28.86 | 31.08 | 31.63 | 33.82 | [redacted] | [redacted] | [redacted] | [redacted] | [redacted] | [redacted] | [redacted] | [redacted] | [redacted] | 29.63 | 26.20 |
| 1 | 804 | ben HT | 24.43 | 32.77 | 29.24 | 30.49 | 32.51 | 31.61 | [redacted] | [redacted] | [redacted] | [redacted] | [redacted] | [redacted] | 27.07 | [redacted] | [redacted] | [redacted] | 25.55 |
| 107 | 927 | ben HT | 23.24 | 30.81 | [redacted] | 30.09 | [redacted] | [redacted] | [redacted] | [redacted] | [redacted] | [redacted] | [redacted] | [redacted] | [redacted] | [redacted] | [redacted] | [redacted] | [redacted] |
| 71 | 917 | ben MA | [redacted] | 33.78 | 32.31 | 32.93 | 46.96 | 37.89 | [redacted] | 33.73 | 23.92 | 24.95 | 31.34 | 30.82 | 27.29 | [redacted] | 31.83 | 33.95 | 28.04 |
| 72 | 918 | ben MA | 26.91 | 32.97 | 28.17 | 33.26 | 41.60 | 38.14 | 31.18 | 33.17 | 24.49 | 25.37 | 33.96 | 33.85 | 27.51 | [redacted] | 31.16 | 33.08 | 28.81 |
| 3 | 806 | ben MG | 25.34 | 31.47 | 29.22 | 30.09 | 32.70 | [redacted] | [redacted] | [redacted] | [redacted] | 23.58 | [redacted] | [redacted] | [redacted] | [redacted] | [redacted] | 29.36 | 25.78 |
| 69 | 914 | ben OH | 24.00 | 30.88 | 26.46 | 31.98 | 31.46 | [redacted] | 33.41 | 36.09 | 23.70 | 23.41 | [redacted] | [redacted] | 29.92 | 30.02 | [redacted] | [redacted] | 26.38 |
| 68 | 913 | ben T | 25.53 | 32.08 | 26.07 | 31.03 | 31.68 | 33.27 | [redacted] | 32.61 | 23.95 | 24.88 | 31.69 | 31.43 | 26.95 | [redacted] | 28.53 | 31.91 | 27.72 |
| 4 | 807 | Benign HT | 24.58 | 30.66 | 29.49 | 30.94 | 32.66 | 31.29 | [redacted] | 31.69 | 24.08 | 24.85 | [redacted] | 30.38 | 27.11 | [redacted] | 28.68 | [redacted] | 25.83 |
| 8 | 823 | mal FC | 23.53 | [redacted] | [redacted] | [redacted] | [redacted] | [redacted] | [redacted] | 32.36 | [redacted] | [redacted] | [redacted] | [redacted] | [redacted] | [redacted] | [redacted] | [redacted] | [redacted] |
| 9 | 825 | mal FC | 25.84 | 31.66 | 31.99 | 32.23 | [redacted] | [redacted] | [redacted] | 33.25 | 24.22 | 24.59 | [redacted] | 30.60 | [redacted] | [redacted] | [redacted] | [redacted] | 25.85 |
| 10 | 826 | mal FC | 24.61 | 31.98 | 29.17 | 31.38 | 33.08 | 31.99 | [redacted] | 31.99 | 24.64 | 25.74 | [redacted] | [redacted] | 26.18 | [redacted] | 30.48 | [redacted] | 26.32 |
| 11 | 827 | mal FC | 24.99 | 30.42 | 28.44 | [redacted] | [redacted] | [redacted] | [redacted] | [redacted] | [redacted] | [redacted] | [redacted] | [redacted] | 27.49 | 27.27 | [redacted] | [redacted] | [redacted] |
| 12 | 828 | mal FC | 24.15 | [redacted] | 30.34 | [redacted] | [redacted] | [redacted] | [redacted] | [redacted] | [redacted] | [redacted] | [redacted] | [redacted] | [redacted] | [redacted] | [redacted] | 29.42 | 25.68 |
| 13 | 830 | mal FC | 23.53 | [redacted] | 30.58 | [redacted] | [redacted] | [redacted] | [redacted] | [redacted] | 24.97 | 25.54 | [redacted] | [redacted] | [redacted] | [redacted] | [redacted] | 29.30 | [redacted] |
| 22 | 840 | mal FC | 26.21 | 33.44 | 29.31 | 30.23 | 32.41 | 31.76 | [redacted] | [redacted] | 23.63 | 24.82 | [redacted] | [redacted] | 27.43 | 27.04 | 29.36 | 29.31 | 26.05 |
| 52 | 895 | mal FC | 24.03 | 31.04 | 26.30 | 31.86 | 34.24 | [redacted] | [redacted] | 32.00 | [redacted] | [redacted] | 31.13 | 30.80 | [redacted] | [redacted] | [redacted] | 32.15 | 25.87 |
| 53 | 896 | mal FC | 24.42 | 30.91 | 26.39 | [redacted] | [redacted] | 30.21 | 31.46 | 33.38 | 25.11 | 26.15 | 31.99 | 31.29 | [redacted] | [redacted] | 30.13 | 30.06 | 26.60 |
| 54 | 897 | mal FC | 24.87 | [redacted] | 26.02 | [redacted] | [redacted] | [redacted] | [redacted] | [redacted] | [redacted] | [redacted] | [redacted] | [redacted] | [redacted] | [redacted] | [redacted] | [redacted] | [redacted] |
| 55 | 898 | mal FC | 23.17 | [redacted] | [redacted] | [redacted] | [redacted] | [redacted] | 33.27 | 36.19 | 24.82 | 26.33 | 31.06 | 31.76 | [redacted] | [redacted] | [redacted] | [redacted] | [redacted] |
| 56 | 899 | mal FC | 26.16 | [redacted] | [redacted] | [redacted] | [redacted] | [redacted] | 33.33 | 36.57 | 29.63 | 30.55 | 31.44 | 33.12 | 29.39 | 30.45 | 28.85 | 29.38 | 27.21 |
| 57 | 900 | mal FC | 22.66 | [redacted] | [redacted] | 30.42 | [redacted] | [redacted] | 33.08 | 35.24 | 32.84 | 32.09 | 32.51 | 32.58 | 30.58 | 31.09 | [redacted] | [redacted] | [redacted] |
| 58 | 901 | mal FC | 24.63 | 31.68 | 26.95 | [redacted] | [redacted] | [redacted] | 34.01 | 37.22 | 50.00 | 45.31 | 30.02 | 30.53 | 26.03 | [redacted] | 29.16 | 30.77 | [redacted] |
| 59 | 902 | mal FC | 22.48 | [redacted] | [redacted] | [redacted] | [redacted] | [redacted] | 33.19 | 35.81 | 27.04 | 28.18 | [redacted] | 30.53 | 26.82 | [redacted] | [redacted] | [redacted] | [redacted] |
| 65 | 910 | mal FC | 24.98 | [redacted] | 28.65 | 30.68 | 32.34 | [redacted] | [redacted] | [redacted] | [redacted] | 24.76 | 31.28 | 30.76 | [redacted] | [redacted] | [redacted] | [redacted] | [redacted] |
| 82 | 936 | mal FC | 22.15 | [redacted] | [redacted] | [redacted] | [redacted] | [redacted] | 31.43 | 32.16 | [redacted] | 28.37 | 32.59 | 31.01 | [redacted] | [redacted] | [redacted] | [redacted] | [redacted] |
| 99 | 954 | mal FC | 21.76 | 31.46 | 27.47 | [redacted] | [redacted] | [redacted] | [redacted] | [redacted] | [redacted] | [redacted] | [redacted] | [redacted] | [redacted] | [redacted] | [redacted] | [redacted] | [redacted] |
| 100 | 968 | mal FC | [redacted] | 34.38 | [redacted] | 34.81 | 35.43 | 49.41 | 34.83 | 35.75 | 25.93 | 27.17 | 34.49 | 33.11 | [redacted] | [redacted] | 28.62 | 33.17 | 26.20 |
| 101 | 969 | mal FC | 25.80 | 31.58 | 27.15 | 30.36 | 36.99 | 41.12 | [redacted] | [redacted] | [redacted] | 23.59 | 32.18 | 31.16 | 26.90 | 25.60 | [redacted] | 45.43 | [redacted] |
| 102 | 970 | mal FC | 27.36 | 32.81 | [redacted] | 34.70 | 31.77 | 30.95 | 34.85 | 36.34 | 28.96 | 30.88 | 33.85 | 31.88 | [redacted] | [redacted] | [redacted] | 29.19 | 27.33 |
| 103 | 982 | mal FC | 26.16 | 32.15 | 26.80 | 31.90 | [redacted] | [redacted] | [redacted] | [redacted] | [redacted] | [redacted] | 30.50 | [redacted] | [redacted] | [redacted] | 28.32 | 35.41 | [redacted] |
| 104 | 983 | mal FC | 22.95 | [redacted] | [redacted] | 31.18 | 33.32 | [redacted] | [redacted] | [redacted] | [redacted] | [redacted] | 32.37 | 33.25 | [redacted] | [redacted] | [redacted] | [redacted] | [redacted] |
| 105 | 967 | mal FC | 23.84 | 30.25 | [redacted] | [redacted] | 31.34 | [redacted] | [redacted] | 31.58 | 24.77 | 25.88 | 31.53 | 31.48 | [redacted] | [redacted] | [redacted] | [redacted] | [redacted] |
| 14 | 831 | mal PC | 22.92 | [redacted] | [redacted] | [redacted] | [redacted] | [redacted] | 33.96 | 34.32 | 29.49 | 29.59 | 32.39 | 32.51 | 30.97 | 31.19 | [redacted] | [redacted] | [redacted] |
| 15 | 832 | mal PC | 25.88 | [redacted] | 27.56 | [redacted] | [redacted] | 30.92 | [redacted] | [redacted] | 24.72 | 25.60 | 30.07 | [redacted] | 29.54 | 29.10 | 29.21 | 29.25 | 26.29 |
| 16 | 834 | mal PC | 24.30 | [redacted] | [redacted] | [redacted] | [redacted] | [redacted] | [redacted] | [redacted] | 25.02 | 25.33 | 32.68 | 31.88 | 29.54 | 28.66 | [redacted] | [redacted] | [redacted] |

| | | | | | | | | | | | | | | | | | | | |
|---|---|---|---|---|---|---|---|---|---|---|---|---|---|---|---|---|---|---|---|
| 17 | 835 | mal PC | 23.68 | | | | | | 32.71 | 35.57 | 28.42 | 29.43 | 32.36 | 32.27 | 31.11 | 30.86 | 28.05 | | |
| 18 | 836 | mal PC | 26.05 | 30.43 | 30.76 | 30.39 | | | | | | 23.30 | | | 26.19 | | | 30.99 | 26.41 |
| 19 | 837 | mal PC | 23.03 | | | | | | | | | | | | | | | | |
| 20 | 838 | mal PC | 27.10 | | | | | | 36.39 | 35.07 | 30.58 | 31.04 | 30.61 | 30.29 | 34.41 | 33.13 | 29.76 | 29.34 | 27.12 |
| 23 | 841 | mal PC | 24.61 | | | | | | 50.00 | 35.53 | 35.72 | 32.06 | | | 29.02 | 28.83 | | | |
| 36 | 874 | mal PC | 23.75 | | | 30.73 | | | 50.00 | 31.62 | 50.00 | 33.93 | 34.19 | 35.64 | 31.97 | 32.29 | | | |
| 37 | 875 | mal PC | 22.97 | | | | | | | 32.27 | 24.54 | 25.32 | 31.28 | 30.30 | 29.08 | 27.87 | | | |
| 38 | 876 | mal PC | 24.02 | | | | | | 32.69 | 34.43 | 28.51 | 30.22 | 32.28 | 33.43 | 29.70 | 29.26 | | | |
| 39 | 879 | mal PC | 24.09 | | | | 31.07 | | 35.70 | 37.76 | 30.62 | 33.05 | 33.28 | 36.11 | 32.62 | 31.46 | | | 26.14 |
| 40 | 880 | mal PC | 23.73 | | | | | | | | 28.60 | 29.22 | 30.21 | 30.42 | 26.16 | 29.98 | 28.76 | | |
| 41 | 881 | mal PC | 25.76 | | | | | | | 33.33 | 27.44 | 28.12 | 30.53 | 30.82 | 29.93 | 29.51 | 28.45 | | 26.21 |
| 42 | 882 | mal PC | 23.60 | | | | | | 33.48 | 34.90 | 30.58 | 30.32 | 32.26 | 33.33 | 29.19 | 28.54 | | | |
| 43 | 883 | mal PC | 27.24 | | | 30.46 | | | 32.77 | 34.87 | 39.38 | 34.10 | 33.16 | 33.40 | 33.41 | 32.65 | 29.74 | 31.77 | 26.98 |
| 44 | 884 | mal PC | 24.78 | | | | | | 32.34 | 34.96 | 26.63 | 25.94 | 30.08 | 32.03 | 28.51 | 27.64 | 28.64 | | 25.54 |
| 45 | 885 | mal PC | 24.40 | | | | 31.08 | | 33.18 | 35.97 | 30.78 | 30.49 | 32.27 | 34.75 | 29.74 | 28.90 | | | 25.53 |
| 46 | 886 | mal PC | 26.66 | | | 30.22 | | | | | 26.11 | 27.57 | 32.02 | 32.31 | 31.81 | 31.08 | 30.42 | 29.99 | 26.66 |
| 47 | 887 | mal PC | 26.37 | | | 30.61 | | 30.80 | 35.66 | 37.77 | 28.38 | 30.00 | 33.81 | 36.74 | 30.28 | 30.19 | 28.77 | 29.03 | 26.52 |
| 48 | 890 | mal PC | 23.01 | | | | | | | | 24.36 | 25.53 | 31.50 | 32.64 | 28.77 | 27.54 | | | |
| 49 | 892 | mal PC | 25.50 | | | | | | | 31.68 | 25.29 | 26.38 | 32.91 | 33.10 | 30.31 | 30.66 | 28.89 | | |
| 50 | 893 | mal PC | 22.27 | | | | | | 35.53 | 37.83 | 28.40 | 28.93 | | 34.42 | 30.51 | 30.32 | | | |
| 51 | 894 | mal PC | 25.06 | | | | | | 34.74 | 37.83 | 26.40 | 27.92 | 35.91 | 36.95 | 32.53 | 31.97 | 28.57 | | 26.58 |
| 60 | 903 | mal PC | 24.67 | | | | | | | | 24.33 | 25.56 | 31.52 | 30.92 | 27.66 | 27.01 | 29.10 | 29.55 | 25.84 |
| 61 | 904 | mal PC | 25.60 | 30.50 | | | | 31.91 | | 32.92 | 25.37 | 25.87 | | | 28.43 | 28.28 | 31.19 | 29.39 | 27.30 |
| 62 | 905 | mal PC | 23.52 | | | | | 30.18 | 37.22 | 37.87 | 50.00 | 32.35 | 32.89 | 33.96 | 32.32 | 31.44 | 29.11 | | 26.43 |
| 63 | 906 | mal PC | 24.75 | | | 30.82 | | | 36.14 | 38.43 | 50.00 | 39.67 | 36.00 | 36.36 | 33.75 | 33.85 | | | 25.78 |
| 67 | 912 | mal PC | 26.06 | 32.23 | 28.67 | 30.31 | 31.91 | 34.61 | 31.43 | 33.19 | 27.54 | 28.77 | | | 31.15 | 29.76 | 32.22 | | 28.95 |
| 74 | 928 | mal PC | 24.74 | 31.29 | 26.35 | | 31.21 | 30.42 | 28.56 | | | | | | | | | 29.72 | |
| 75 | 929 | mal PC | 25.88 | | | | | | 33.19 | 33.95 | 30.24 | 30.77 | | | 33.94 | 35.60 | 31.53 | | 26.20 |
| 76 | 930 | mal PC | 23.24 | | | | | | 27.63 | | 27.51 | 28.42 | | | 32.01 | 40.98 | 29.63 | | |
| 77 | 931 | mal PC | 24.56 | | 28.87 | 30.21 | | | 25.10 | | 29.61 | 30.19 | 36.74 | 37.03 | | | 28.83 | 31.99 | 25.73 |
| 78 | 932 | mal PC | 23.10 | | | | | | 33.42 | 35.50 | 26.75 | 27.96 | 35.90 | 35.13 | 32.08 | 50.00 | 29.96 | | |
| 79 | 933 | mal PC | 26.28 | 30.24 | | 31.17 | | 30.03 | 30.71 | | 23.90 | 24.64 | 31.53 | 30.49 | 27.61 | 27.30 | 30.83 | 29.28 | 25.92 |
| 80 | 934 | mal PC | 24.62 | 30.08 | | | | | 33.71 | 35.30 | 27.25 | 28.02 | 32.43 | 31.58 | 31.25 | 32.87 | 30.27 | | 25.52 |
| 81 | 935 | mal PC | 22.89 | | | 30.39 | | | 33.18 | 35.02 | 27.75 | 29.46 | 35.17 | 33.71 | 34.23 | 50.00 | | | |
| 21 | 839 | mal PC FV | 24.92 | 31.46 | 28.20 | 30.32 | 32.12 | | 29.58 | | | | | | 26.31 | | 29.67 | | 26.05 |
| 66 | 911 | mal PC FV | 30.42 | 31.06 | 27.26 | 31.99 | | 30.23 | 32.08 | 34.06 | 29.75 | 30.24 | | | 31.83 | 31.21 | 33.24 | 31.23 | 29.47 |
| 89 | 981 | mal PC FV | 25.69 | | 27.55 | | | | 26.79 | | | | 30.24 | | 26.74 | | | 30.03 | |
| 5 | 809 | normal | 26.05 | 32.31 | 30.82 | 30.44 | 32.57 | 32.53 | 30.19 | 31.61 | | 23.74 | | | 27.08 | | 29.19 | | 26.19 |

| | | | | | | | | | | | | | | | | | | | |
|---|---|---|---|---|---|---|---|---|---|---|---|---|---|---|---|---|---|---|---|
| 6 | 817 | normal | 26.92 | 32.62 | 31.69 | 31.12 | 32.11 | 33.50 | 29.04 | | | 23.44 | | | 26.61 | | 29.39 | 30.18 | 26.14 |
| 25 | 844 | normal | 26.61 | 33.39 | 32.33 | 32.40 | 32.36 | 34.60 | 31.34 | 33.27 | 24.60 | 24.75 | 30.80 | | 28.03 | 27.35 | 30.12 | 31.79 | 28.13 |
| 26 | 845 | normal | 25.08 | 31.37 | 31.11 | 31.87 | 33.50 | 30.13 | 31.27 | 32.54 | | | | | 28.26 | 27.38 | | | 25.65 |
| 27 | 851 | normal | 25.92 | 31.32 | 30.18 | | | 31.08 | | | 24.11 | 24.94 | | | 27.48 | 27.16 | 29.29 | 29.50 | 26.05 |
| | | | 28.00 | 30.00 | 26.00 | 30.00 | 31.00 | 30.00 | 31.00 | 31.50 | 23.50 | 23.25 | 30.00 | 30.00 | 26.00 | 27.00 | 28.00 | 29.00 | 25.50 |

Table 23b

| | | | Thyroid UR Markers | | | | | | | | | |
|---|---|---|---|---|---|---|---|---|---|---|---|---|
| | | CDH3 | FN1 | GAB | SGENE | DDIT3 | C10f24 | ITM1 | TEST | KCN1257 | TPO2166 | TPO2592 |
| % Sensitivity | | 76.56 | 71.88 | 39.06 | 23.44 | 23.44 | 78.13 | 43.75 | 57.81 | 48.44 | 71.88 | 51.56 |
| % Specificity | | 83.33 | 80.56 | 80.56 | 94.44 | 86.11 | 77.78 | 63.89 | 77.78 | 86.11 | 88.89 | 97.22 |
| | | | | | | | | | | | | |
| | | CDH3/KCN | CDH3/TPO-1 | CDH3/TPO-2 | SGENE/GAB | | | | FN1/KCN | FN1/TPO-1 | FN1/TPO-2 | TEST/KCN |
| | Sensitivity% | 85.94 | 89.06 | 84.38 | 96.88 | | | | 76.56 | 84.38 | 78.13 | 81.25 |
| | Specificity% | 69.44 | 75.00 | 83.33 | 5.56 | | | | 66.67 | 72.22 | 80.56 | 63.89 |
| | | | CDH3/FN1/TEST/TPO2166 | | | | FN1/SGENE/TPO2166 | | | | | |
| | Sensitivity% | | 95.31 | | | | 89.06 | | | | | |
| | Specificity% | | 61.11 | | | | 72.22 | | | | | |

| | | Thyroid DR Markers | |
|---|---|---|---|
| | | DIO1;480 | DIO1;687 |
| | | 54.69 | 53.13 |
| % Sensitivity | | 86.11 | 97.22 |
| % Specificity | | | |

Positive

Outliers

Markers with Best Performance

| | | BACTIN | CDH3 | FN1 | GAB | SGENE | DDIT3 | C10f24 | ITM1 | TEST | KCN1257 | TPO2166 | TPO2592 | FABP4;110 | FABP4-2 | DIO1;480 | DIO1;687 |
|---|---|---|---|---|---|---|---|---|---|---|---|---|---|---|---|---|---|
| 915TT | benign DG | 26.69 | 34.23 | 30.09 | 31.94 | 33.60 | 29.54 | 31.01 | 26.46 | 32.00 | 30.70 | 20.69 | 21.83 | 29.87 | 28.76 | 24.78 | 24.09 |
| 924TT | benign GPH | 21.48 | | | | 27.96 | 41.06 | 50.00 | 34.50 | | 27.04 | | | 31.30 | 29.97 | 26.64 | 25.82 |
| 871TT | benign HCA | 23.42 | 31.13 | | 31.80 | | | | | | 30.89 | 23.62 | 24.19 | 30.68 | | 24.86 | 24.70 |
| 805TT | benign HPS | 26.84 | 33.76 | 28.61 | 31.08 | 33.72 | 27.57 | 29.63 | 26.20 | 31.63 | 27.13 | 22.12 | 22.53 | 28.81 | 28.65 | 25.93 | 25.17 |
| 804TT | benign HT | 24.43 | 32.77 | 28.98 | 30.49 | 31.62 | 26.62 | | 25.55 | 32.51 | 29.05 | 22.27 | 23.13 | 27.21 | 27.10 | 27.07 | 26.58 |

| | | | | | | | | | | | | | | | | | |
|---|---|---|---|---|---|---|---|---|---|---|---|---|---|---|---|---|---|
| 927TT | benign HT | 23.24 | 30.81 | | 30.09 | 28.23 | | | | | 28.49 | 18.52 | 19.83 | 28.34 | 27.17 | 23.11 | 22.07 |
| 917TT | benign MA | 28.22 | 33.78 | 33.13 | 32.93 | 37.79 | 31.83 | 33.95 | 28.04 | 46.96 | 30.89 | 23.92 | 24.95 | 31.34 | | 27.29 | 26.77 |
| 918TT | benign MA | 26.91 | 32.97 | 28.74 | 33.26 | 38.32 | 31.16 | 33.08 | 28.81 | 41.60 | | | 25.37 | | | | 26.76 |
| 806TT | benign MG | 25.34 | 31.47 | 28.77 | 30.09 | 29.84 | 27.14 | | 25.78 | 32.70 | 27.05 | 23.30 | 23.58 | 28.29 | 27.73 | 25.79 | 25.61 |
| 914TT | benign OH | 24.00 | 30.88 | 27.01 | 31.98 | 28.33 | | | 26.38 | 31.46 | | 23.70 | 23.41 | 29.62 | 29.06 | | |
| 913TT | benign T | 25.53 | 32.08 | | 31.03 | 34.07 | 28.53 | 31.91 | 27.72 | 31.68 | 30.28 | 23.95 | 24.88 | 31.69 | | 26.95 | 26.52 |
| 807TT | benignHT | 24.58 | 30.66 | 28.98 | 30.94 | 31.35 | 28.68 | | 25.83 | 32.66 | 29.94 | 24.08 | 24.85 | 29.48 | 30.38 | 27.11 | 26.54 |
| 842TT | benign FA | 25.92 | 31.74 | 29.53 | 30.96 | 32.71 | 29.76 | 33.81 | 27.09 | 32.98 | 27.45 | 23.88 | 24.44 | | | | 27.42 |
| 862TT | benign FA | 26.86 | 33.35 | 29.92 | 30.92 | 50.00 | 28.44 | 38.86 | 27.25 | 41.29 | | 22.50 | 22.54 | | | 25.63 | 25.24 |
| 863TT | benign FA | 24.56 | | 29.48 | 29.85 | 27.28 | 27.71 | 30.25 | | | 25.73 | 22.03 | 22.89 | 29.05 | 28.82 | 24.74 | 24.42 |
| 864TT | benign FA | 26.15 | 32.18 | 30.08 | 31.97 | 30.48 | 28.23 | 31.93 | 25.59 | 34.52 | 26.65 | 21.76 | 22.65 | 30.06 | 30.15 | 25.67 | 25.30 |
| 865TT | benign FA | 26.21 | 32.94 | 26.92 | 31.29 | 30.93 | 30.19 | 47.61 | 26.83 | 32.07 | 29.70 | 21.87 | 22.37 | 31.25 | | 24.25 | 23.83 |
| 866TT | benign FA | 27.00 | 33.10 | 28.77 | 32.26 | 32.61 | 29.26 | 31.60 | 26.44 | 34.44 | 29.59 | 23.32 | 23.43 | | | 25.94 | 25.57 |
| 867TT | benign FA | 24.89 | 31.72 | 30.37 | 31.88 | 33.81 | 29.33 | 31.53 | 26.71 | 32.29 | 29.50 | 21.88 | 21.95 | 27.71 | 27.69 | 24.90 | 24.73 |
| 869TT | benign FA | 24.99 | | 28.58 | 29.99 | 28.11 | 27.39 | 29.46 | | | 27.32 | 21.24 | 22.14 | 30.69 | 30.08 | 25.88 | 25.39 |
| 907TT | benign FA | 25.09 | 31.42 | 27.00 | 29.49 | 35.86 | 29.63 | 30.46 | 25.79 | 33.83 | 29.98 | 20.92 | 21.81 | 31.21 | | 22.49 | 22.55 |
| 920TT | benign FA | 25.37 | 34.29 | 28.37 | 30.93 | 48.63 | 29.53 | | | 33.48 | | 21.43 | 21.83 | 30.14 | 28.91 | 22.40 | 23.30 |
| 937TT | benign FA | 23.32 | 33.55 | 28.00 | 32.56 | 36.65 | 27.15 | 32.90 | | 34.45 | 25.48 | 19.17 | 20.06 | 27.50 | 25.18 | 24.69 | 23.50 |
| 938TT | benign FA | 23.39 | 31.45 | | 31.58 | | 27.77 | 29.88 | | 29.82 | 27.98 | 20.84 | 21.34 | 29.53 | 27.24 | 22.26 | 22.03 |
| 939TT | benign FA | 25.58 | 34.57 | 29.13 | 31.87 | 34.24 | 30.66 | 31.70 | 28.01 | 32.01 | | 23.17 | 23.85 | 31.29 | 29.78 | | 27.32 |
| 940TT | benign FA | 25.33 | 34.29 | 27.51 | 31.38 | 29.38 | 28.28 | 31.78 | | 31.19 | 28.87 | 21.47 | 21.28 | | | 24.78 | 23.85 |
| 941TT | benign FA | 26.84 | 31.44 | 30.89 | 33.27 | 31.13 | 30.37 | 40.24 | 26.75 | 31.66 | 27.82 | 23.57 | 24.30 | | 30.48 | 26.36 | 25.46 |
| 957TT | benign FA | 24.37 | 31.53 | 27.49 | | 31.29 | 28.77 | 31.38 | | | 30.23 | | 25.62 | 29.60 | 28.10 | 22.90 | 21.85 |
| 923TT | benign FA | 23.12 | 30.71 | 26.83 | | 31.48 | 26.80 | 30.17 | | 30.31 | 26.76 | 21.47 | 22.43 | 29.22 | 28.27 | 24.34 | 23.24 |
| 926TT | benign FA | 22.89 | | | | 30.84 | 28.06 | | | 30.01 | 27.07 | 21.86 | 22.86 | 30.71 | 29.07 | 23.50 | 22.23 |
| 955TT | benign FA | 26.97 | 31.90 | 29.65 | 31.72 | 29.22 | 28.34 | 31.37 | 25.77 | 33.70 | 28.93 | | 25.76 | | | | 27.07 |
| 956TT | benign FA | 27.07 | 31.06 | 29.58 | 30.12 | 30.07 | 28.79 | 32.24 | 25.83 | 32.02 | 29.24 | 23.30 | 24.09 | 31.88 | | 27.17 | 25.99 |
| 960TT | benign FA | 25.12 | 32.35 | 27.33 | | 27.89 | 28.19 | 30.38 | 25.70 | | 26.04 | 19.93 | 20.86 | 28.25 | 26.54 | 23.59 | 23.03 |
| 921TT | benign FA | 22.10 | | | | 30.89 | | 34.76 | | 31.95 | 24.57 | 18.33 | 19.18 | 26.27 | 24.77 | 24.04 | 22.51 |
| 961TT | benign FA | 28.34 | 32.16 | 31.04 | 31.66 | 35.11 | 30.93 | 31.46 | 27.40 | 30.29 | 29.39 | 23.34 | 24.61 | 31.93 | 30.46 | 26.99 | 26.64 |
| 962TT | benign FA | 23.81 | | 28.78 | | 27.51 | | 29.61 | | | 25.81 | 19.33 | 20.25 | 29.59 | 29.19 | 23.12 | 22.37 |
| 323TT | malignant FC | 23.53 | | | | 29.23 | 27.45 | | | 30.51 | 29.60 | 20.43 | 20.63 | 27.38 | 27.02 | 24.02 | 23.51 |
| 325TT | malignant FC | 25.84 | 31.66 | 31.36 | 32.23 | 28.74 | 26.58 | | 25.85 | | 30.39 | | 24.59 | 29.69 | | 25.40 | 24.94 |
| 326TT | malignant FC | 24.61 | 31.98 | 28.66 | 31.38 | 32.18 | 30.48 | | 26.32 | 33.08 | 29.75 | | 25.74 | 28.87 | 28.78 | 26.18 | 25.35 |
| 327TT | malignant FC | 24.99 | | 28.10 | 29.30 | 28.53 | 27.98 | | | 29.64 | 29.90 | 22.95 | 23.06 | 29.02 | 29.01 | 27.49 | 27.27 |
| 328TT | malignant FC | 24.15 | | 29.96 | 29.43 | 28.86 | 27.15 | | 25.68 | | 25.14 | 19.74 | 20.43 | 25.99 | 26.55 | 22.44 | 22.20 |
| 330TT | malignant FC | 23.53 | | 30.09 | | 27.51 | 26.81 | | | | 26.34 | | 25.54 | 28.12 | 27.60 | 23.23 | 23.27 |

| | | | | | | | | | | | | | | | | | |
|---|---|---|---|---|---|---|---|---|---|---|---|---|---|---|---|---|---|
| 840TT | malignant FC | 26.21 | 33.44 | 28.95 | 30.23 | 31.79 | 29.36 | [illegible] | 26.05 | 32.41 | 29.77 | 23.63 | 24.82 | 28.00 | 27.53 | 27.43 | 27.04 |
| 395TT | malignant FC | 24.03 | 31.04 | [illegible] | 31.86 | 29.67 | 27.59 | 32.15 | 25.87 | 34.24 | 29.80 | 21.45 | 22.96 | 31.13 | [illegible] | 22.47 | 21.66 |
| 396TT | malignant FC | 24.42 | 30.91 | [illegible] | [illegible] | 30.77 | 30.13 | 30.06 | 26.60 | [illegible] | [illegible] | [illegible] | 26.15 | 31.99 | [illegible] | 25.37 | 24.28 |
| 397TT | malignant FC | 24.87 | [illegible] | [illegible] | [illegible] | [illegible] | 27.14 | [illegible] | [illegible] | [illegible] | | 26.89 20.75 | 22.10 | 27.59 | 27.46 | 25.72 | 24.59 |
| 398TT | malignant FC | 23.17 | [illegible] | [illegible] | 29.43 | [illegible] | [illegible] | [illegible] | [illegible] | [illegible] | [illegible] | [illegible] | 26.33 | 31.06 | [illegible] | 24.16 | 24.79 |
| 399TT | malignant FC | 26.16 | [illegible] | [illegible] | [illegible] | 29.92 | 28.85 | [illegible] | 27.21 | [illegible] | [illegible] | [illegible] | [illegible] | 31.44 | [illegible] | 29.39 | 30.45 |
| 900TT | malignant FC | 22.66 | [illegible] | [illegible] | 30.42 | [illegible] | 27.76 | [illegible] | [illegible] | [illegible] | [illegible] | [illegible] | [illegible] | [illegible] | [illegible] | 30.58 | [illegible] |
| 901TT | malignant FC | 24.63 | 31.68 | 27.38 | [illegible] | [illegible] | 29.16 | 30.77 | [illegible] | [illegible] | [illegible] | [illegible] | [illegible] | 30.02 | [illegible] | 26.03 | 26.82 |
| 902TT | malignant FC | 22.48 | [illegible] | [illegible] | [illegible] | [illegible] | 25.24 | 25.71 | [illegible] | [illegible] | [illegible] | [illegible] | [illegible] | 29.44 | [illegible] | 26.82 | 25.63 |
| 910TT | malignant FC | 24.98 | [illegible] | 28.71 | 30.68 | [illegible] | [illegible] | [illegible] | [illegible] | 32.34 | 28.00 | 23.29 | 24.76 | 31.28 | [illegible] | 25.86 | 25.58 |
| 936TT | malignant FC | 22.15 | [illegible] | [illegible] | 29.63 | [illegible] | 24.33 | 25.07 | [illegible] | [illegible] | [illegible] | 22.84 | [illegible] | [illegible] | [illegible] | 25.96 | 24.27 |
| 954TT | malignant FC | 21.76 | 31.46 | 28.01 | 29.92 | [illegible] | 23.99 | 25.51 | 22.72 | [illegible] | 30.58 | 19.16 | 20.78 | 27.09 | 25.74 | 23.98 | 23.30 |
| 968TT | malignant FC | 28.23 | 34.38 | [illegible] | 34.81 | 46.83 | 28.62 | 33.17 | 26.20 | 35.43 | [illegible] | [illegible] | [illegible] | [illegible] | [illegible] | 25.13 | 23.80 |
| 969TT | malignant FC | 25.80 | 31.58 | 27.84 | 30.36 | 42.77 | 27.72 | 45.43 | 25.30 | 36.99 | 28.36 | 22.94 | 23.59 | 32.18 | 31.16 | 26.90 | 25.60 |
| 970TT | malignant FC | 27.36 | 32.81 | [illegible] | 34.70 | 30.90 | [illegible] | [illegible] | 27.33 | 31.77 | [illegible] | [illegible] | [illegible] | [illegible] | [illegible] | 25.34 | 25.47 |
| 982TT | malignant FC | 26.16 | 32.15 | 27.30 | 31.90 | 29.32 | 28.32 | 35.41 | 25.41 | [illegible] | 26.34 | 20.54 | 22.21 | 30.50 | 29.66 | 24.51 | 23.58 |
| 983TT | malignant FC | 22.95 | [illegible] | [illegible] | 31.18 | [illegible] | [illegible] | [illegible] | [illegible] | 33.32 | 29.51 | 20.34 | 20.87 | [illegible] | [illegible] | 22.14 | 20.88 |
| 967TT | malignant FC | 23.84 | [illegible] | [illegible] | 29.45 | 28.46 | [illegible] | [illegible] | [illegible] | 31.34 | 29.90 | [illegible] | 25.88 | 31.53 | [illegible] | 25.77 | 25.25 |
| 831TT | malignant PC | 22.92 | [illegible] | [illegible] | 29.29 | 28.98 | 27.66 | [illegible] | [illegible] | 30.14 | [illegible] | [illegible] | [illegible] | [illegible] | [illegible] | 30.97 | [illegible] |
| 832TT | malignant PC | 25.88 | [illegible] | 26.78 | 29.72 | 30.99 | 29.21 | [illegible] | 26.29 | [illegible] | 30.43 | [illegible] | 25.60 | 30.07 | 29.22 | 29.54 | [illegible] |
| 834TT | malignant PC | 24.30 | [illegible] | [illegible] | [illegible] | 28.67 | 27.67 | [illegible] | [illegible] | [illegible] | 29.84 | [illegible] | 25.33 | [illegible] | 31.88 | 29.54 | [illegible] |
| 835TT | malignant PC | 23.68 | [illegible] | [illegible] | [illegible] | 29.06 | 28.05 | [illegible] | 25.36 | [illegible] | [illegible] | [illegible] | [illegible] | [illegible] | 32.27 | 31.11 | [illegible] |
| 836TT | malignant PC | 26.05 | [illegible] | 30.35 | 30.39 | 28.40 | 27.82 | 30.99 | 26.41 | 30.26 | 27.47 | 22.99 | 23.30 | 29.85 | 29.63 | 26.19 | 25.62 |
| 837TT | malignant PC | 23.03 | [illegible] | [illegible] | [illegible] | 28.84 | 27.43 | [illegible] | [illegible] | [illegible] | 27.94 | 21.85 | 22.10 | 28.94 | 29.14 | 25.99 | 25.43 |
| 838TT | malignant PC | 27.10 | [illegible] | [illegible] | [illegible] | 29.67 | 29.76 | [illegible] | 27.12 | 29.61 | [illegible] | [illegible] | [illegible] | 30.61 | 30.29 | [illegible] | [illegible] |
| 841TT | malignant PC | 24.61 | [illegible] | [illegible] | 29.59 | [illegible] | [illegible] | [illegible] | [illegible] | [illegible] | [illegible] | 35.72 | [illegible] | 29.05 | 28.91 | [illegible] | [illegible] |
| 874TT | malignant PC | 23.75 | [illegible] | [illegible] | 30.73 | 27.01 | [illegible] | [illegible] | [illegible] | 29.68 | [illegible] | 50.00 | [illegible] | [illegible] | [illegible] | [illegible] | [illegible] |
| 875TT | malignant PC | 22.97 | [illegible] | [illegible] | [illegible] | 27.93 | 27.26 | [illegible] | [illegible] | [illegible] | 29.84 | 24.54 | 25.32 | 31.28 | 30.30 | 29.08 | [illegible] |
| 876TT | malignant PC | 24.02 | [illegible] | [illegible] | [illegible] | 27.87 | 26.74 | [illegible] | [illegible] | 29.60 | [illegible] | 28.51 | 30.22 | 32.28 | 33.43 | 29.70 | [illegible] |
| 879TT | malignant PC | 24.09 | [illegible] | [illegible] | [illegible] | 29.49 | 27.90 | [illegible] | 26.14 | 31.07 | [illegible] | 30.62 | 33.05 | [illegible] | [illegible] | [illegible] | [illegible] |
| 880TT | malignant PC | 23.73 | [illegible] | [illegible] | [illegible] | [illegible] | 28.76 | [illegible] | [illegible] | [illegible] | 28.45 | [illegible] | [illegible] | 30.21 | 30.42 | 26.16 | [illegible] |
| 881TT | malignant PC | 25.76 | [illegible] | [illegible] | 29.43 | 29.01 | 28.45 | [illegible] | 26.21 | [illegible] | 30.89 | [illegible] | [illegible] | 30.53 | [illegible] | [illegible] | [illegible] |
| 882TT | malignant PC | 23.60 | [illegible] | [illegible] | [illegible] | [illegible] | 26.52 | [illegible] | [illegible] | [illegible] | [illegible] | [illegible] | [illegible] | [illegible] | [illegible] | [illegible] | [illegible] |
| 883TT | malignant PC | 27.24 | [illegible] | [illegible] | 30.46 | 28.08 | 29.74 | 31.77 | 26.98 | 30.28 | [illegible] | [illegible] | [illegible] | [illegible] | [illegible] | [illegible] | [illegible] |
| 884TT | malignant PC | 24.78 | [illegible] | [illegible] | 29.17 | 28.48 | 28.64 | [illegible] | 25.54 | [illegible] | [illegible] | [illegible] | 25.94 | 30.08 | [illegible] | [illegible] | [illegible] |
| 885TT | malignant PC | 24.40 | [illegible] | [illegible] | [illegible] | 27.45 | 27.90 | [illegible] | 25.53 | 31.08 | [illegible] | [illegible] | [illegible] | [illegible] | [illegible] | [illegible] | [illegible] |
| 886TT | malignant PC | 26.66 | [illegible] | [illegible] | 30.22 | 28.93 | 30.42 | 29.99 | 26.66 | [illegible] | 29.64 | [illegible] | 27.57 | 32.07 | [illegible] | [illegible] | [illegible] |

| | | | | | | | | | | | | | | | | | |
|---|---|---|---|---|---|---|---|---|---|---|---|---|---|---|---|---|---|
| 887TT | malignant PC | 26.37 | [illegible] | [illegible] | 30.61 | 30.98 | 28.77 | [illegible] | 26.52 | 30.46 | [illegible] | [illegible] | [illegible] | [illegible] | [illegible] | [illegible] | [illegible] |
| 890TT | malignant PC | 23.01 | [illegible] | [illegible] | [illegible] | [illegible] | 27.82 | [illegible] | [illegible] | [illegible] | 27.45 | [illegible] | 25.53 | 31.50 | [illegible] | [illegible] | [illegible] |
| 892TT | malignant PC | 25.50 | [illegible] | [illegible] | [illegible] | 27.93 | 28.89 | [illegible] | 25.49 | [illegible] | 29.38 | [illegible] | 26.38 | [illegible] | [illegible] | 30.31 | [illegible] |
| 893TT | malignant PC | 22.27 | [illegible] | [illegible] | [illegible] | [illegible] | [illegible] | [illegible] | [illegible] | [illegible] | [illegible] | [illegible] | [illegible] | 29.47 | [illegible] | 30.51 | [illegible] |
| 894TT | malignant PC | 25.06 | [illegible] | [illegible] | 29.72 | 29.12 | 28.57 | [illegible] | 26.58 | 30.81 | [illegible] | [illegible] | [illegible] | [illegible] | [illegible] | [illegible] | [illegible] |
| 903TT | malignant PC | 24.67 | [illegible] | [illegible] | 29.80 | 28.03 | 29.10 | 29.55 | 25.84 | [illegible] | 28.93 | [illegible] | 25.56 | 31.52 | [illegible] | [illegible] | 27.01 |
| 904TT | malignant PC | 25.60 | [illegible] | [illegible] | 29.32 | 32.31 | 31.19 | [illegible] | 27.30 | [illegible] | 30.64 | [illegible] | 25.87 | 29.77 | 29.40 | [illegible] | [illegible] |
| 905TT | malignant PC | 23.52 | [illegible] | [illegible] | 29.38 | 30.32 | 29.11 | [illegible] | 26.43 | [illegible] | [illegible] | [illegible] | [illegible] | [illegible] | [illegible] | [illegible] | [illegible] |
| 906TT | malignant PC | 24.75 | [illegible] | [illegible] | 30.82 | 27.25 | [illegible] | [illegible] | 25.78 | 30.53 | [illegible] | [illegible] | [illegible] | [illegible] | [illegible] | [illegible] | [illegible] |
| 912TT | malignant PC | 26.06 | 32.23 | 28.74 | 30.31 | 35.11 | 32.22 | [illegible] | 28.95 | 31.91 | [illegible] | [illegible] | [illegible] | 29.96 | 29.40 | [illegible] | [illegible] |
| 928TT | malignant PC | 24.74 | 31.29 | [illegible] | 29.75 | 30.63 | [illegible] | 29.72 | [illegible] | 31.21 | 28.56 | 20.69 | 22.43 | 29.21 | 27.50 | 22.88 | 22.28 |
| 929TT | malignant PC | 25.88 | [illegible] | [illegible] | [illegible] | 29.79 | 31.53 | [illegible] | 26.20 | [illegible] | [illegible] | [illegible] | [illegible] | 29.15 | 28.05 | [illegible] | [illegible] |
| 930TT | malignant PC | 23.24 | [illegible] | [illegible] | [illegible] | [illegible] | 29.63 | [illegible] | [illegible] | [illegible] | 27.63 | [illegible] | [illegible] | 29.55 | 28.48 | [illegible] | [illegible] |
| 931TT | malignant PC | 24.56 | [illegible] | 29.48 | 30.21 | 27.22 | 28.83 | 31.99 | 25.73 | [illegible] | 25.10 | [illegible] | 30.19 | 36.74 | [illegible] | 24.27 | 23.53 |
| 932TT | malignant PC | 23.10 | [illegible] | [illegible] | [illegible] | 29.13 | 29.96 | [illegible] | 25.37 | [illegible] | [illegible] | [illegible] | [illegible] | [illegible] | [illegible] | [illegible] | [illegible] |
| 933TT | malignant PC | 26.28 | [illegible] | [illegible] | 31.17 | 30.25 | 30.83 | [illegible] | 25.92 | [illegible] | 30.71 | 23.90 | 24.64 | 31.53 | 30.49 | [illegible] | 27.30 |
| 934TT | malignant PC | 24.62 | [illegible] | [illegible] | [illegible] | 29.63 | 30.27 | [illegible] | 25.52 | 30.07 | [illegible] | [illegible] | [illegible] | [illegible] | [illegible] | [illegible] | [illegible] |
| 935TT | malignant PC | 22.89 | [illegible] | [illegible] | 30.39 | 28.59 | [illegible] | [illegible] | [illegible] | [illegible] | [illegible] | [illegible] | [illegible] | [illegible] | [illegible] | [illegible] | [illegible] |
| 839TT | mal PCFV | 24.92 | 31.46 | 27.85 | 30.32 | 31.81 | 29.67 | [illegible] | 26.05 | 32.12 | 29.58 | 22.79 | 23.13 | 29.37 | 29.10 | 26.31 | 26.14 |
| 911TT | mal PCFV | 30.42 | 31.06 | 27.58 | 31.99 | 30.59 | 33.24 | 31.23 | 29.47 | 30.06 | [illegible] | [illegible] | [illegible] | 29.33 | 29.38 | [illegible] | [illegible] |
| 981TT | mal PCFV | 25.69 | [illegible] | 27.55 | [illegible] | [illegible] | [illegible] | 30.03 | [illegible] | [illegible] | 26.79 | 21.80 | 22.90 | 30.24 | 29.47 | 26.74 | 26.57 |
| | | 28.00 | 30.50 | 26.58 | 29.15 | 27.00 | 26.50 | 29.42 | 25.30 | 29.60 | 31.00 | 24.20 | 26.45 | 32.00 | 30.50 | 27.50 | 27.50 |

Table 23c

| | FN1/SGENE/C1of24/TPO2592 |
|---|---|
| Sensitivity | 95.31% |
| Specificity | 66.67% |

| | % Within +/- .5 of Cutoff | | | |
|---|---|---|---|---|
| | FN1 | SGENE | C1of24 | TPO2592 |
| Benign | 16.67% | 8.33% | 13.90% | 5.56% |
| Cancer | 12.50% | 15.60% | 20.30% | 10.93% |

| | |
|---|---|
| (dark) | Positive |
| (white) | Outliers |

Markers with Best Performance

| | | BACTIN | FN1 | SGENE | C10f24 | TPO2592 |
|---|---|---|---|---|---|---|
| 915TT | benign DG | 26.69 | 30.09 | 33.60 | 31.01 | 21.83 |
| 924TT | benign GPH | 21.48 | [redacted] | 27.96 | 50.00 | [redacted] |
| 871TT | benign HCA | 23.42 | [redacted] | [redacted] | [redacted] | 24.19 |
| 805TT | benign HPS | 26.84 | 28.61 | 33.72 | 29.63 | 22.53 |
| 804TT | benign HT | 24.43 | 28.98 | 31.62 | [redacted] | 23.13 |
| 927TT | benign HT | 23.24 | [redacted] | 28.23 | [redacted] | 19.83 |
| 917TT | benign MA | 28.22 | 33.13 | 37.79 | 33.95 | 24.95 |
| 918TT | benign MA | 26.91 | 28.74 | 38.32 | 33.08 | 25.37 |
| 806TT | benign MG | 25.34 | 28.77 | 29.84 | 29.36 | 23.58 |
| 914TT | benign OH | 24.00 | 27.01 | 28.33 | 26.56 | 23.41 |
| 913TT | benign T | 25.53 | [redacted] | 34.07 | 31.91 | 24.88 |
| 807TT | benignHT | 24.58 | 28.98 | 31.35 | [redacted] | 24.85 |
| 842TT | benign FA | 25.92 | 29.53 | 32.71 | 33.81 | 24.44 |
| 862TT | benign FA | 26.86 | 29.92 | 50.00 | 38.86 | 22.54 |
| 863TT | benign FA | 24.56 | 29.48 | 27.28 | 30.25 | 22.89 |
| 864TT | benign FA | 26.15 | 30.08 | 30.48 | 31.93 | 22.65 |
| 865TT | benign FA | 26.21 | 26.92 | 30.93 | 47.61 | 22.37 |
| 866TT | benign FA | 27.00 | 28.77 | 32.61 | 31.60 | 23.43 |
| 867TT | benign FA | 24.89 | 30.37 | 33.81 | 31.53 | 21.95 |
| 869TT | benign FA | 24.99 | 28.58 | 28.11 | 29.46 | 22.14 |
| 907TT | benign FA | 25.09 | 27.00 | 35.86 | 30.46 | 21.81 |
| 920TT | benign FA | 25.37 | 28.37 | 48.63 | [redacted] | 21.83 |
| 937TT | benign FA | 23.32 | 28.00 | 36.65 | 32.90 | 20.06 |
| 938TT | benign FA | 23.39 | [redacted] | [redacted] | 29.88 | 21.34 |
| 939TT | benign FA | 25.58 | 29.13 | 34.24 | 31.70 | 23.85 |
| 940TT | benign FA | 25.33 | 27.51 | 29.38 | 31.78 | 21.28 |
| 941TT | benign FA | 26.84 | 30.89 | 31.13 | 40.24 | 24.30 |
| 957TT | benign FA | 24.37 | 27.49 | 31.29 | 31.38 | 25.62 |
| 923TT | benign FA | 23.12 | 26.83 | 31.48 | 30.17 | 22.43 |
| 926TT | benign FA | 22.89 | [redacted] | 30.84 | [redacted] | 22.86 |
| 955TT | benign FA | 26.97 | 29.65 | 29.22 | 31.37 | 25.76 |
| 956TT | benign FA | 27.07 | 29.58 | 30.07 | 32.24 | 24.09 |
| 960TT | benign FA | 25.12 | 27.33 | 27.89 | 30.38 | 20.86 |
| 921TT | benign FA | 22.10 | [redacted] | 30.89 | 34.76 | 19.18 |

| | | | | | | |
|---|---|---|---|---|---|---|
| 961TT | benign FA | 28.34 | 31.04 | 35.11 | 31.46 | 24.61 |
| 962TT | benign FA | 23.81 | 28.78 | 27.51 | 29.61 | 20.25 |
| 823TT | malignant FC | 23.53 | [illegible] | 29.23 | [illegible] | 20.63 |
| 825TT | malignant FC | 25.84 | 31.36 | 28.74 | [illegible] | 24.59 |
| 826TT | malignant FC | 24.61 | 28.66 | 32.18 | [illegible] | 25.74 |
| 827TT | malignant FC | 24.99 | 28.10 | 28.53 | [illegible] | 23.06 |
| 828TT | malignant FC | 24.15 | 29.96 | 28.86 | [illegible] | 20.43 |
| 830TT | malignant FC | 23.53 | 30.09 | 27.51 | [illegible] | 25.54 |
| 840TT | malignant FC | 26.21 | 28.95 | 31.79 | [illegible] | 24.82 |
| 895TT | malignant FC | 24.03 | [illegible] | 29.67 | 32.15 | 22.96 |
| 896TT | malignant FC | 24.42 | [illegible] | 30.77 | 30.06 | [illegible] |
| 897TT | malignant FC | 24.87 | [illegible] | [illegible] | [illegible] | 22.10 |
| 898TT | malignant FC | 23.17 | 20.35 | [illegible] | 26.05 | [illegible] |
| 899TT | malignant FC | 26.16 | 21.34 | 29.92 | 29.38 | 30.55 |
| 900TT | malignant FC | 22.66 | [illegible] | [illegible] | [illegible] | 32.09 |
| 901TT | malignant FC | 24.63 | 27.38 | 21.76 | 30.77 | 45.31 |
| 902TT | malignant FC | 22.48 | [illegible] | 26.11 | [illegible] | 28.18 |
| 910TT | malignant FC | 24.98 | 28.71 | 26.34 | 28.49 | 24.76 |
| 936TT | malignant FC | 22.15 | [illegible] | 26.83 | 25.07 | [illegible] |
| 954TT | malignant FC | 21.76 | 28.01 | [illegible] | [illegible] | 20.78 |
| 968TT | malignant FC | 28.23 | [illegible] | 46.83 | 33.17 | [illegible] |
| 969TT | malignant FC | 25.80 | 27.84 | 42.77 | 45.43 | 23.59 |
| 970TT | malignant FC | 27.36 | [illegible] | 30.90 | [illegible] | [illegible] |
| 982TT | malignant FC | 26.16 | 27.30 | 29.32 | 35.41 | 22.21 |
| 983TT | malignant FC | 22.95 | [illegible] | [illegible] | 24.70 | 20.87 |
| 967TT | malignant FC | 23.84 | 25.99 | 28.46 | 25.78 | 25.88 |
| 831TT | malignant PC | 22.92 | [illegible] | 28.98 | 26.16 | [illegible] |
| 832TT | malignant PC | 25.88 | 26.78 | 30.99 | 29.25 | 25.60 |
| 834TT | malignant PC | 24.30 | [illegible] | 28.67 | 26.69 | 25.33 |
| 835TT | malignant PC | 23.68 | [illegible] | 29.06 | [illegible] | [illegible] |
| 836TT | malignant PC | 26.05 | 30.35 | 28.40 | 30.99 | 23.30 |
| 837TT | malignant PC | 23.03 | [illegible] | 28.84 | [illegible] | 22.10 |
| 838TT | malignant PC | 27.10 | 23.25 | 29.67 | [illegible] | [illegible] |
| 841TT | malignant PC | 24.61 | 25.17 | [illegible] | [illegible] | [illegible] |
| 874TT | malignant PC | 23.75 | [illegible] | [illegible] | [illegible] | [illegible] |
| 875TT | malignant PC | 22.97 | [illegible] | 27.93 | [illegible] | 25.32 |

| | | | | | | |
|---|---|---|---|---|---|---|
| 876TT | *malignant PC* | 24.02 | 19.37 | 27.87 | 25.46 | 30.22 |
| 879TT | malignant PC | 24.09 | [illegible] | 29.49 | 28.76 | [illegible] |
| 880TT | malignant PC | 23.73 | [illegible] | [illegible] | 28.46 | [illegible] |
| 881TT | malignant PC | 25.76 | [illegible] | 29.01 | 28.45 | [illegible] |
| 882TT | malignant PC | 23.60 | [illegible] | [illegible] | [illegible] | 30.32 |
| 883TT | *malignant PC* | 27.24 | [illegible] | 28.08 | 31.77 | [illegible] |
| 884TT | malignant PC | 24.78 | [illegible] | 28.48 | [illegible] | 25.94 |
| 885TT | malignant PC | 24.40 | [illegible] | 27.45 | [illegible] | [illegible] |
| 886TT | malignant PC | 26.66 | [illegible] | 28.93 | 29.99 | 27.57 |
| 887TT | malignant PC | 26.37 | [illegible] | 30.98 | [illegible] | [illegible] |
| 890TT | *malignant PC* | 23.01 | [illegible] | [illegible] | 27.50 | 25.53 |
| 892TT | malignant PC | 25.50 | 21.43 | 27.93 | 28.93 | [illegible] |
| 893TT | malignant PC | 22.27 | 18.03 | [illegible] | 25.24 | 28.93 |
| 894TT | malignant PC | 25.06 | [illegible] | 29.12 | [illegible] | [illegible] |
| 903TT | malignant PC | 24.67 | 23.24 | 28.03 | 29.55 | 25.56 |
| 904TT | *malignant PC* | 25.60 | 24.86 | 32.31 | 29.39 | 25.87 |
| 905TT | malignant PC | 23.52 | 20.64 | 30.32 | 27.66 | [illegible] |
| 906TT | malignant PC | 24.75 | [illegible] | 27.25 | 25.31 | 39.67 |
| 912TT | malignant PC | 26.06 | 28.74 | 35.11 | [illegible] | [illegible] |
| 928TT | malignant PC | 24.74 | [illegible] | 30.63 | 29.72 | 22.43 |
| 929TT | *malignant PC* | 25.88 | 21.77 | 29.79 | 28.31 | 30.77 |
| 930TT | malignant PC | 23.24 | 22.84 | [illegible] | 28.36 | 28.42 |
| 931TT | malignant PC | 24.56 | 29.48 | 27.22 | 31.99 | 30.19 |
| 932TT | malignant PC | 23.10 | 21.26 | 29.13 | 28.07 | [illegible] |
| 933TT | malignant PC | 26.28 | 24.46 | 30.25 | 29.28 | 24.64 |
| 934TT | *malignant PC* | 24.62 | 26.38 | 29.63 | 28.02 | 28.02 |
| 935TT | malignant PC | 22.89 | [illegible] | 28.59 | 24.60 | 29.46 |
| 839TT | malignant PCFV | 24.92 | 27.85 | 31.81 | [illegible] | 23.13 |
| 911TT | malignant PCFV | 30.42 | 27.58 | 30.59 | 31.23 | [illegible] |
| 981TT | malignant PCFV | 25.69 | 27.55 | [illegible] | 30.03 | 22.90 |
| | | 28.00 | 26.58 | 27.16 | 29.42 | 26.14 |

Table 24a

| | Thyroid Markers | | |
|---|---|---|---|
| | GLOGIN67 | HERC | PAX8 |
| % Total | 46.73% | 79.44% | 93.46% |

Positive Outliers

| | | | BACTIN | GLOGIN67 | HERC | PAX8 |
|---|---|---|---|---|---|---|
| 70 | 915TT | benign DG | 26.69 | 26.62 | 28.56 | [illegible] |
| 7 | 818TT | benign FA | 50.00 | 46.96 | 50.00 | 50.00 |
| 24 | 842TT | benign FA | 25.92 | 24.15 | 26.50 | 23.98 |
| 28 | 862TT | benign FA | 26.86 | [illegible] | 27.23 | 23.35 |
| 29 | 863TT | benign FA | 24.56 | 23.66 | 26.08 | 22.59 |
| 30 | 864TT | benign FA | 26.15 | [illegible] | 26.54 | 23.32 |
| 31 | 865TT | benign FA | 26.21 | 25.67 | 27.24 | 23.05 |
| 32 | 866TT | benign FA | 27.00 | 24.33 | 27.24 | 23.73 |
| 33 | 867TT | benign FA | 24.89 | 25.28 | 26.99 | 23.16 |
| 34 | 869TT | benign FA | 24.99 | [illegible] | 26.19 | 22.20 |
| 64 | 907TT | benign FA | 25.09 | 25.28 | 27.11 | 22.07 |
| 73 | 920TT | benign FA | 25.37 | 25.55 | 27.85 | 20.73 |
| 83 | 937TT | benign FA | 23.32 | [illegible] | 26.11 | 21.40 |
| 84 | 938TT | benign FA | 23.39 | 24.95 | 26.31 | 21.50 |
| 85 | 939TT | benign FA | 25.58 | 28.18 | 29.45 | 25.14 |
| 86 | 940TT | benign FA | 25.33 | 26.11 | [illegible] | 22.96 |
| 87 | 941TT | benign FA | 26.84 | 26.10 | 28.84 | 25.31 |
| 88 | 957TT | benign FA | 24.37 | 26.24 | 27.00 | 21.79 |
| 90 | 923TT | benign FA | 23.12 | [illegible] | [illegible] | 21.11 |
| 91 | 926TT | benign FA | 22.89 | 21.73 | [illegible] | 21.34 |
| 92 | 955TT | benign FA | 26.97 | [illegible] | 28.14 | 24.78 |
| 93 | 956TT | benign FA | 27.07 | 24.84 | [illegible] | [illegible] |
| 94 | 959TT | benign FA | 50.00 | 50.00 | 50.00 | 50.00 |
| 95 | 960TT | benign FA | 25.12 | 25.29 | [illegible] | [illegible] |
| 96 | 921TT | benign FA | 22.10 | [illegible] | [illegible] | [illegible] |
| 97 | 961TT | benign FA | 28.34 | 26.42 | 29.37 | [illegible] |
| 98 | 962TT | benign FA | 23.81 | [illegible] | [illegible] | [illegible] |
| 106 | 924TT | benign GPH | 21.48 | [illegible] | [illegible] | [illegible] |
| 35 | 871TT | benign HCA | 23.42 | 24.65 | 26.21 | [illegible] |
| 2 | 805TT | benign HPS | 26.84 | 24.49 | 26.94 | [illegible] |
| 1 | 804TT | benign HT | 24.43 | 24.27 | 26.79 | [illegible] |
| 107 | 927TT | benign HT | 23.24 | [illegible] | [illegible] | 21.50 |
| 71 | 917TT | benign MA | 28.22 | 34.84 | 30.68 | [illegible] |

| | | | | | | |
|---|---|---|---|---|---|---|
| 72 | 918TT | benign MA | 26.91 | 29.71 | 29.63 | [illegible] |
| 3 | 806TT | benign MG | 25.34 | 24.33 | [illegible] | [illegible] |
| 69 | 914TT | benign OH | 24.00 | 24.85 | 28.46 | [illegible] |
| 68 | 913TT | benign T | 25.53 | 26.79 | 28.47 | [illegible] |
| 4 | 807TT | benignHT | 24.58 | 25.72 | [illegible] | [illegible] |
| 8 | 823TT | malignant FC | 23.53 | 24.32 | [illegible] | [illegible] |
| 9 | 825TT | malignant FC | 25.84 | 26.16 | [illegible] | [illegible] |
| 10 | 826TT | malignant FC | 24.61 | 25.14 | [illegible] | [illegible] |
| 11 | 827TT | malignant FC | 24.99 | [illegible] | [illegible] | [illegible] |
| 12 | 828TT | malignant FC | 24.15 | 23.94 | [illegible] | [illegible] |
| 13 | 830TT | malignant FC | 23.53 | [illegible] | 25.01 | [illegible] |
| 22 | 840TT | malignant FC | 26.21 | 25.45 | 27.05 | [illegible] |
| 52 | 895TT | malignant FC | 24.03 | [illegible] | 26.40 | 20.15 |
| 53 | 896TT | malignant FC | 24.42 | 24.49 | 26.14 | 22.46 |
| 54 | 897TT | malignant FC | 24.87 | [illegible] | 26.67 | 20.56 |
| 55 | 898TT | malignant FC | 23.17 | 23.11 | [illegible] | 22.13 |
| 56 | 899TT | malignant FC | 26.16 | 23.13 | 28.13 | 24.19 |
| 57 | 900TT | malignant FC | 22.66 | 24.75 | [illegible] | [illegible] |
| 58 | 901TT | malignant FC | 24.63 | 34.54 | 25.44 | 50.00 |
| 59 | 902TT | malignant FC | 22.48 | [illegible] | 25.29 | [illegible] |
| 65 | 910TT | malignant FC | 24.98 | 24.23 | 26.69 | 22.61 |
| 82 | 936TT | malignant FC | 22.15 | 24.20 | 25.30 | 23.02 |
| 99 | 954TT | malignant FC | 21.76 | [illegible] | [illegible] | 21.25 |
| 100 | 968TT | malignant FC | 28.23 | 27.18 | 28.85 | 24.69 |
| 101 | 969TT | malignant FC | 25.80 | [illegible] | [illegible] | 22.97 |
| 102 | 970TT | malignant FC | 27.36 | 26.46 | 28.56 | 25.99 |
| 103 | 982TT | malignant FC | 26.16 | 24.58 | [illegible] | 22.11 |
| 104 | 983TT | malignant FC | 22.95 | [illegible] | 25.20 | [illegible] |
| 105 | 967TT | malignant FC | 23.84 | 21.66 | 25.76 | [illegible] |
| 14 | 831TT | malignant PC | 22.92 | [illegible] | 26.22 | [illegible] |
| 15 | 832TT | malignant PC | 25.88 | 24.82 | 26.66 | [illegible] |
| 16 | 834TT | malignant PC | 24.30 | [illegible] | 25.39 | [illegible] |
| 17 | 835TT | malignant PC | 23.68 | 23.83 | 26.43 | [illegible] |
| 18 | 836TT | malignant PC | 26.05 | 23.63 | 27.23 | [illegible] |
| 19 | 837TT | malignant PC | 23.03 | 21.96 | [illegible] | [illegible] |
| 20 | 838TT | malignant PC | 27.10 | 23.89 | 28.01 | [illegible] |
| 23 | 841TT | malignant PC | 24.61 | 21.73 | [illegible] | 24.13 |

| | | | | | | |
|---|---|---|---|---|---|---|
| 36 | 874TT | malignant PC | 23.75 | 24.09 | [illegible] | [illegible] |
| 37 | 875TT | malignant PC | 22.97 | [illegible] | [illegible] | [illegible] |
| 38 | 876TT | malignant PC | 24.02 | 22.27 | [illegible] | [illegible] |
| 39 | 879TT | malignant PC | 24.09 | 22.84 | 26.03 | [illegible] |
| 40 | 880TT | malignant PC | 23.73 | 23.63 | 26.14 | [illegible] |
| 41 | 881TT | malignant PC | 25.76 | 23.74 | 27.61 | [illegible] |
| 42 | 882TT | malignant PC | 23.60 | 22.39 | 25.12 | [illegible] |
| 43 | 883TT | malignant PC | 27.24 | 22.74 | 28.54 | [illegible] |
| 44 | 884TT | malignant PC | 24.78 | 23.40 | 27.50 | [illegible] |
| 45 | 885TT | malignant PC | 24.40 | [illegible] | [illegible] | [illegible] |
| 46 | 886TT | malignant PC | 26.66 | 24.24 | 28.20 | 24.30 |
| 47 | 887TT | malignant PC | 26.37 | 24.60 | [illegible] | 24.56 |
| 48 | 890TT | malignant PC | 23.01 | [illegible] | 26.20 | 23.04 |
| 49 | 892TT | malignant PC | 25.50 | 25.21 | 26.30 | 23.42 |
| 50 | 893TT | malignant PC | 22.27 | [illegible] | 25.76 | 22.25 |
| 51 | 894TT | malignant PC | 25.06 | 24.39 | 27.38 | 24.22 |
| 60 | 903TT | malignant PC | 24.67 | 24.29 | 26.64 | 23.20 |
| 61 | 904TT | malignant PC | 25.60 | 26.45 | 28.57 | 25.53 |
| 62 | 905TT | malignant PC | 23.52 | 23.33 | 27.22 | [illegible] |
| 63 | 906TT | malignant PC | 24.75 | [illegible] | [illegible] | 27.56 |
| 67 | 912TT | malignant PC | 26.06 | 25.15 | 28.99 | 26.56 |
| 74 | 928TT | malignant PC | 24.74 | [illegible] | [illegible] | [illegible] |
| 75 | 929TT | malignant PC | 25.88 | 24.40 | 27.97 | 25.41 |
| 76 | 930TT | malignant PC | 23.24 | 23.43 | 26.78 | 23.78 |
| 77 | 931TT | malignant PC | 24.56 | 23.61 | 27.11 | 22.39 |
| 78 | 932TT | malignant PC | 23.10 | [illegible] | 25.82 | 22.39 |
| 79 | 933TT | malignant PC | 26.28 | 24.09 | 27.35 | 23.29 |
| 80 | 934TT | malignant PC | 24.62 | 30.20 | 28.82 | 27.39 |
| 81 | 935TT | malignant PC | 22.89 | [illegible] | 26.11 | 23.15 |
| 21 | 839TT | malignant PCFV | 24.92 | 24.22 | 26.85 | [illegible] |
| 66 | 911TT | malignant PCFV | 30.42 | 25.66 | 30.30 | 27.22 |
| 89 | 981TT | malignant PCFV | 25.69 | [illegible] | [illegible] | [illegible] |
| 5 | 809TT | normal | 26.05 | 24.93 | 27.35 | [illegible] |
| 6 | 817TT | normal | 26.92 | 25.30 | [illegible] | [illegible] |
| 25 | 844TT | normal | 26.61 | 26.30 | 28.34 | [illegible] |
| 26 | 845TT | normal | 25.08 | 25.10 | [illegible] | [illegible] |
| 27 | 851TT | normal | 25.92 | 24.42 | 26.81 | 23.16 |

| 28.00 | 24.00 | 28.00 | 26.00 |
|---|---|---|---|

Table 24b

| | [illegible] | [illegible] | [illegible] |
|---|---|---|---|
| Sensitivity | 66.00% | 22.00% | 99.00% |
| Specificity | 50.00% | 44.74% | 100.00% |

Positive
Outliers
Markers with Best Performance

| | | BACTIN | GOLGIN67 | HERC | PAX8 |
|---|---|---|---|---|---|
| 915TT | benign DG | 26.69 | 26.62 | 28.56 | 24.37 |
| 924TT | benign GPH | 21.48 | [illegible] | [illegible] | 20.45 |
| 871TT | benign HCA | 23.42 | 24.65 | 26.21 | 23.21 |
| 805TT | benign HPS | 26.84 | [illegible] | 26.94 | 23.37 |
| 804TT | benign HT | 24.43 | 24.27 | 26.79 | 23.92 |
| 927TT | benign HT | 23.24 | [illegible] | [illegible] | 21.50 |
| 917TT | benign MA | 28.22 | 34.84 | 30.68 | 25.79 |
| 918TT | benign MA | 26.91 | 29.71 | 29.63 | 25.71 |
| 806TT | benign MG | 25.34 | [illegible] | 26.04 | 22.91 |
| 914TT | benign OH | 24.00 | 24.85 | 28.46 | 24.56 |
| 913TT | benign T | 25.53 | 26.79 | 28.47 | 24.46 |
| 807TT | benignHT | 24.58 | 25.72 | 26.37 | 24.73 |
| 842TT | benign FA | 25.92 | [illegible] | 26.50 | 23.98 |
| 862TT | benign FA | 26.86 | 23.89 | 27.23 | 23.35 |
| 863TT | benign FA | 24.56 | 23.66 | 26.08 | 22.59 |
| 864TT | benign FA | 26.15 | [illegible] | 26.54 | 23.32 |
| 865TT | benign FA | 26.21 | 25.67 | 27.24 | 23.05 |
| 866TT | benign FA | 27.00 | [illegible] | 27.24 | 23.73 |
| 867TT | benign FA | 24.89 | 25.28 | 26.99 | 23.16 |
| 869TT | benign FA | 24.99 | [illegible] | 26.19 | 22.20 |
| 907TT | benign FA | 25.09 | 25.28 | 27.11 | 22.07 |
| 920TT | benign FA | 25.37 | 25.55 | 27.85 | 20.73 |
| 937TT | benign FA | 23.32 | [illegible] | 26.11 | 21.40 |
| 938TT | benign FA | 23.39 | 24.95 | 26.31 | [illegible] |

| | | | | | |
|---|---|---|---|---|---|
| 939TT | benign FA | 25.58 | 28.18 | 29.45 | [illegible] |
| 940TT | benign FA | 25.33 | 26.11 | 27.22 | [illegible] |
| 941TT | benign FA | 26.84 | 26.10 | 28.84 | [illegible] |
| 957TT | benign FA | 24.37 | 26.24 | 27.00 | [illegible] |
| 923TT | benign FA | 23.12 | [illegible] | [illegible] | [illegible] |
| 926TT | benign FA | 22.89 | [illegible] | [illegible] | 21.34 |
| 955TT | benign FA | 26.97 | 23.11 | 28.14 | [illegible] |
| 956TT | benign FA | 27.07 | 24.84 | 27.67 | [illegible] |
| 960TT | benign FA | 25.12 | 25.29 | 27.11 | [illegible] |
| 921TT | benign FA | 22.10 | [illegible] | [illegible] | 20.24 |
| 961TT | benign FA | 28.34 | 26.42 | 29.37 | 24.98 |
| 962TT | benign FA | 23.81 | [illegible] | [illegible] | 21.14 |
| 823TT | malignant FC | 23.53 | [illegible] | [illegible] | 21.25 |
| 825TT | malignant FC | 25.84 | 26.16 | 27.73 | 23.30 |
| 826TT | malignant FC | 24.61 | 25.14 | 26.32 | 23.91 |
| 827TT | malignant FC | 24.99 | 23.18 | 26.52 | 23.29 |
| 828TT | malignant FC | 24.15 | 23.94 | [illegible] | 21.23 |
| 830TT | malignant FC | 23.53 | [illegible] | [illegible] | 22.05 |
| 840TT | malignant FC | 26.21 | 25.45 | 27.05 | 23.34 |
| 895TT | malignant FC | 24.03 | [illegible] | 26.40 | 20.15 |
| 896TT | malignant FC | 24.42 | 24.49 | 26.14 | 22.46 |
| 897TT | malignant FC | 24.87 | 22.14 | 26.67 | 20.56 |
| 898TT | malignant FC | 23.17 | 23.11 | [illegible] | 22.13 |
| 899TT | malignant FC | 26.16 | [illegible] | 28.13 | 24.19 |
| 900TT | malignant FC | 22.66 | 24.75 | 26.81 | [illegible] |
| 901TT | malignant FC | 24.63 | 34.54 | 25.44 | 50.00 |
| 902TT | malignant FC | 22.48 | [illegible] | 25.29 | [illegible] |
| 910TT | malignant FC | 24.98 | 24.23 | 26.69 | 22.61 |
| 936TT | malignant FC | 22.15 | 24.20 | 25.30 | 23.02 |
| 954TT | malignant FC | 21.76 | [illegible] | [illegible] | [illegible] |
| 968TT | malignant FC | 28.23 | 27.18 | 28.85 | 24.60 |
| 969TT | malignant FC | 25.80 | [illegible] | 26.95 | [illegible] |
| 970TT | malignant FC | 27.36 | 26.46 | 28.56 | [illegible] |
| 982TT | malignant FC | 26.16 | 24.58 | 26.80 | [illegible] |
| 983TT | malignant FC | 22.95 | [illegible] | [illegible] | 19.72 |
| 967TT | malignant FC | 23.84 | [illegible] | [illegible] | [illegible] |

| | | | | | |
|---|---|---|---|---|---|
| 831TT | malignant PC | 22.92 | [illegible] | 26.22 | [illegible] |
| 832TT | malignant PC | 25.88 | 24.82 | 26.66 | [illegible] |
| 834TT | malignant PC | 24.30 | [illegible] | [illegible] | [illegible] |
| 835TT | malignant PC | 23.68 | [illegible] | 26.43 | [illegible] |
| 836TT | malignant PC | 26.05 | [illegible] | 27.23 | [illegible] |
| 837TT | malignant PC | 23.03 | [illegible] | [illegible] | [illegible] |
| 838TT | malignant PC | 27.10 | [illegible] | 28.01 | [illegible] |
| 841TT | malignant PC | 24.61 | [illegible] | 26.09 | [illegible] |
| 874TT | malignant PC | 23.75 | [illegible] | 26.07 | [illegible] |
| 875TT | malignant PC | 22.97 | [illegible] | 26.39 | [illegible] |
| 876TT | malignant PC | 24.02 | [illegible] | [illegible] | 22.64 |
| 879TT | malignant PC | 24.09 | 22.84 | 26.63 | 23.56 |
| 880TT | malignant PC | 23.73 | [illegible] | 26.14 | 23.77 |
| 881TT | malignant PC | 25.76 | [illegible] | 27.61 | 24.12 |
| 882TT | malignant PC | 23.60 | [illegible] | [illegible] | 22.19 |
| 883TT | malignant PC | 27.24 | [illegible] | 28.54 | 24.87 |
| 884TT | malignant PC | 24.78 | 23.40 | 27.50 | 22.92 |
| 885TT | malignant PC | 24.40 | [illegible] | 26.89 | 22.62 |
| 886TT | malignant PC | 26.66 | [illegible] | 28.20 | 24.30 |
| 887TT | malignant PC | 26.37 | 24.60 | 27.77 | 24.56 |
| 890TT | malignant PC | 23.01 | [illegible] | 26.20 | 23.04 |
| 892TT | malignant PC | 25.50 | 25.21 | 26.30 | 23.42 |
| 893TT | malignant PC | 22.27 | [illegible] | [illegible] | 22.25 |
| 894TT | malignant PC | 25.06 | 24.39 | 27.38 | 24.22 |
| 903TT | malignant PC | 24.67 | 24.29 | 26.64 | 23.20 |
| 904TT | malignant PC | 25.60 | 26.45 | 28.57 | 25.53 |
| 905TT | malignant PC | 23.52 | 23.33 | 27.22 | 24.11 |
| 906TT | malignant PC | 24.75 | [illegible] | 27.28 | 27.56 |
| 912TT | malignant PC | 26.06 | 25.15 | 28.99 | [illegible] |
| 928TT | malignant PC | 24.74 | [illegible] | 27.06 | 22.00 |
| 929TT | malignant PC | 25.88 | 24.40 | 27.97 | [illegible] |
| 930TT | malignant PC | 23.24 | 23.43 | 26.78 | [illegible] |
| 931TT | malignant PC | 24.56 | 23.61 | 27.11 | [illegible] |
| 932TT | malignant PC | 23.10 | 23.17 | [illegible] | [illegible] |
| 933TT | malignant PC | 26.28 | 24.09 | 27.35 | [illegible] |
| 934TT | malignant PC | 24.62 | 30.20 | 28.82 | [illegible] |
| 935TT | malignant PC | 22.89 | [illegible] | 26.11 | [illegible] |

| | | | | | |
|---|---|---|---|---|---|
| 839TT | malignant PCFV | 24.92 | [illegible] | 26.85 | [illegible] |
| 911TT | malignant PCFV | 30.42 | 25.66 | 30.30 | [illegible] |
| 981TT | malignant PCFV | 25.69 | [illegible] | 26.67 | [illegible] |
| S1 | SkinN | 20.34 | [illegible] | [illegible] | 49.98 |
| S2 | SkinN | 22.37 | [illegible] | 26.28 | 50.00 |
| S3 | SkinN | 20.02 | [illegible] | [illegible] | 50.00 |
| S4 | SkinN | 23.90 | 26.08 | 28.22 | 33.80 |
| S5 | SkinN | 21.64 | [illegible] | 26.22 | 33.76 |
| S6 | SkinN | 21.57 | [illegible] | 26.45 | 50.00 |
| S7 | SkinN | 20.88 | [illegible] | [illegible] | 38.50 |
| S8 | SkinN | 21.56 | 23.95 | [illegible] | 50.00 |
| S9 | SkinN | 21.27 | 22.88 | [illegible] | 50.00 |
| S10 | SkinN | 21.15 | [illegible] | [illegible] | 50.00 |
| Mu1 | MuscleN | 23.31 | 27.76 | 27.24 | 50.00 |
| Mu2 | MuscleN | 25.07 | 43.49 | 28.72 | 50.00 |
| Mu3 | MuscleN | 24.56 | [illegible] | [illegible] | 50.00 |
| Mu4 | MuscleN | 25.07 | 29.17 | 26.64 | 50.00 |
| Mu5 | MuscleN | 24.57 | 32.11 | [illegible] | 50.00 |
| Mu6 | MuscleN | 23.11 | 25.30 | [illegible] | 50.00 |
| M1 | Blood | 21.38 | 25.62 | 26.20 | 50.00 |
| M2 | Blood | 21.02 | [illegible] | [illegible] | 37.18 |
| M3 | Blood | 21.44 | 25.60 | 26.33 | 50.00 |
| M4 | Blood | 21.04 | [illegible] | 26.15 | 50.00 |
| M5 | Blood | 20.41 | [illegible] | 25.33 | 50.00 |
| M6 | Blood | 20.43 | 25.72 | 25.54 | 38.36 |
| M7 | Blood | 20.76 | [illegible] | 25.79 | 50.00 |
| M8 | Blood | 20.91 | 25.23 | 25.66 | 50.00 |
| M9 | Blood | 21.31 | 24.69 | [illegible] | 50.00 |
| M10 | Blood | 21.84 | 28.56 | 26.55 | 50.00 |
| M12 | Blood | 20.84 | [illegible] | [illegible] | 50.00 |
| M14 | Blood | 21.45 | 25.02 | [illegible] | 50.00 |
| M15 | Blood | 21.10 | 24.80 | 26.01 | 50.00 |
| M16 | Blood | 22.13 | 25.11 | 26.17 | 50.00 |
| M17 | Blood | 20.85 | [illegible] | [illegible] | 50.00 |
| M18 | Blood | 20.97 | 25.33 | 26.14 | 50.00 |
| M19 | Blood | 21.97 | 25.22 | 26.45 | 50.00 |
| M20 | Blood | 20.52 | [illegible] | [illegible] | 38.40 |

| | | | | | |
|---|---|---|---|---|---|
| M21 | Blood | 22.29 | 24.91 | 26.41 | 50.00 |
| M22 | Blood | 22.10 | 25.56 | 26.86 | 50.00 |
| M23 | Blood | 20.92 | | | 50.00 |
| M24 | Blood | 21.30 | | | 50.00 |
| | | 28.00 | 24.50 | 26.00 | 28.00 |

Table 25

| Sequence identifications | | | | |
|---|---|---|---|---|
| SEQ NO: | psids | Name | Accession No. | Description |
| 1 | 200635_s_at | | AU145351 | Hs.75216 proTTyrosine phosphatase, rec. type, F |
| 2 | 200771_at | | NM_002293 | laminin, gamma 1 |

(continued)

| Sequence identifications | | | | |
|---|---|---|---|---|
| SEQ NO: | psids | Name | Accession No. | Description |
| 3 | 201069_at | | NM_304530 | matrix metalloproteinase 2 |
| 4 | 201117_s_at | CPE | NM_001873 | carboxypeptidase E |
| 5 | 201150_s_at | | NM_000362 | tissue inhibitor of metalloproteinase 3 |
| 6 | 201185_at | | NM_002775 | protease, serine, 11 |
| 7 | 201203_s_at | | AI921320 | ribosome binding protein 1 |
| 8 | 201212_at | | NM_005606 | cysteine protease |
| 9 | 201289_at | CYR61 | NM_001554 | cysteine-rich, angiogenic inducer, 61 |
| 10 | 201292_at | | NM_001067 | topoisomerase (DNA) II alpha |
| 11 | 201418_s_at | SOX4 | NM_003107 | SRY (sex determining region Y)-box 4 |
| 12 | 201427_s_at | SEPP1 | NM_005410 | Selenoprotein P, plasma, 1 |
| 13 | 201430_s_at | DPYSL3 | NM_001387 | dihydropvrimidinase-like 3 |
| 14 | 201431_s_at | DPYSL3 | NM_001387 | dihvdropyrimidinase-like 3 |
| 15 | 201438_at | COL6A3 | NM_004369 | collagen, type VI, alpha 3 |
| 16 | 201474_s_at | ITGA3 | NM_002204 | integrin, $\alpha$ 3 transcript variant a |
| 17 | 201505_at | LAMB1 | NM_002291 | laminin, beta 1 |
| 18 | 201508_at | IGFBP4 | NM_001552 | Insulin-like growth factor-binding protein 4 |
| 19 | 201525_at | APOD | NM_001647 | apolipoprotein D |
| 20 | 201645_at | HXB | NM_002160 | hexabrachion (tenascin C, cytotactin) |
| 21 | 201650_at | KRT19 | NM_002276 | keratin 19 |
| 22 | 201667_at | GJA1 | NM_000165 | gap junction protein, a 1, 43kD (connexin 43) |
| 23 | 201744_s_at | LUM | NM_002345 | lumican |
| 24 | 201792_at | AEBP1 | NM_001129 | AE-binding protein 1 |
| 25 | 201852_x_at | | AI813758 | Collagen, type III, alpha 1 |
| 26 | 201893_x_at | | AF138300 | decorin variant A |
| 27 | 201983_s_at | | AW157070 | epidermal growth factor receptor |
| 28 | 202133_at | | AA081084 | Transcriptional co-activator w PDZ-binding motif |
| 29 | 202219_at | SLC6A8 | NM_005629 | solute carrier family 6, member 8 |
| 30 | 202237_at | NNMT | No_006169 | nicotinamide N-methyltransferase |
| 31 | 202286_s_at | | NM_002353 | tumor-associated calcium siqnal transducer 2 |
| 32 | 202291-s-at | | No_000900 | matrix Gla protein |
| 33 | 202310_s_at | | NM_000088 | proalpha 1 (1) chain of type I procollagen |
| 34 | 202350_s_at | MATN2 | No_002380 | matrilin 2 precursor, transcript variant 1 |
| 35 | 202357 s at | BF | NM_001710 | B-factor, properdin |
| 36 | 202363_at | | AF231124 | testican-1 |
| 37 | 202376_at | SERPINA3 | No_001085 | Ser (or Cys) proteinase inhib clade A mem 3 |
| 38 | 202404_s_at | COL1A2 | NM_000089 | collagen, type I, alpha 2 |

(continued)

| Sequence identifications | | | | |
|---|---|---|---|---|
| SEQ NO: | psids | Name | Accession No. | Description |
| 39 | 202440_s_at | | NM_005418 | suppression of tumorigenicity 5 |
| 40 | 202504_at | ATDC | NM_012101 | ataxia-telangiectasia group D-assoc. protein |
| 41 | 202575_at | CRABP2 | NM_001878 | cellular retinoic acid-binding protein 2 |
| 42 | 202588_at | AK1 | NM_000476 | adenylate kinase 1 |
| 43 | 202712_s_at | CKMT1 | NM_020990 | creatine kinase, mitochondrial 1 |
| 44 | 202796_at | KIAA1029 | NM_007286 | synaptopodin |
| 45 | 202826_at | SPINT1 | NM_003710 | serine protease inhibitor, Kunitz type 1 |
| 46 | 202834_at | SERPINA8 | NM_000029 | Ser (or Cys) proteinase inhib, clade A mem 8 |
| 47 | 202898_at | KIAA0468 | NM_014654 | KIAA0468 gene product |
| 48 | 202992_at | C7 | NM_000587 | complement component 7 |
| 49 | 203021_at | SLPI | NM_003064 | secretory leukocyte protease inhibitor |
| 50 | 203083_at | THBS2 | NM_003247 | thrombospondin 2 |
| 51 | 203180_at | ALDH1A3 | NM_000693 | aldehyde dehydrogenase 1 family, mem. A3 |
| 52 | 203228_at | PAFAH1B3 | NM_002573 | platelet-activating factor acetylhydrolase, isoform Ib, $\gamma$ sub |
| 53 | 203256_at | CDH3 | NM_001793 | cadherin 3, type 1, P-cadherin (placental) |
| 54 | 203349_s_at | ETV5 | NM_004454 | ets variant gene 5 (ets-related molecule) |
| 55 | 203352_at | ORC4L | NM_002552 | origin recognition complex, subunit 4-like |
| 56 | 203354_s_at | | NM_015310 | |
| 57 | 203381_s_at | | NM_000041 | |
| 58 | 203382_s_at | APOE | NM_000041 | apolipoprotein E |
| 59 | 203407_at | PPL | NM_002705 | periplakin |
| 60 | 203417_at | MAP2 | NM_017459 | microfibrillar-associated protein 2, tran var 1 |
| 61 | 203438_at | | NM_003714 | stanniocalcin 2 |
| 62 | 203453_at | SCNN1A | NM_001038 | sodium channel, nonvoltage-gated 1 $\alpha$ |
| 63 | 203499_at | EPHA2 | NM_004431 | EphA2 |
| 64 | 203548_s_at | | NM_000237 | lipoprotein lipase |
| 65 | 203570_at | LOXL1 | NM_005576 | lysyl oxidase-like 1 |
| 66 | 203632_s_at | GPRC5B | NM_016235 | G protein-coupled rec, fam C, group 5, mem B |
| 67 | 203673_at | TG | NM_003235 | thyroglobulin |
| 68 | 203699_s_at | | NM_013989 | type II iodothyronine deiodinase |
| 69 | 203700_s_at | DIO2 | NM_013989 | deiodinase, iodothyronine, type II, tran var 1 |
| 70 | 203786_s_at | TPD52L1 | NM_003287 | tumor protein D52-like 1 |
| 71 | 203851_at | IGFBP6 | NM_002178 | insulin-like growth factor binding protein 6 |
| 72 | 203854_at | IF | NM_000204 | I factor (complement) |
| 73 | 203859_s_at | PALM | NM_002579 | paralemmin |

(continued)

| Sequence identifications | | | | |
|---|---|---|---|---|
| SEQ NO: | psids | Name | Accession No. | Description |
| 74 | 203875_at | | NM_003069 | SWISNF related, matrix assoc, actin dep regulator of chromatin subfam a mem 1 |
| 75 | 203881_s_at | | NM_004010 | dystrophin, includes DXS142, DXS164, DXS206, DXS230, DXS239, DXS268, DXS269, DXS270, DXS272 (DMD), transcript variant Dp427p2 |
| 76 | 203889_at | SGNE1 | NM_003020 | secretory granule, neuroendocrine protein 1 |
| 77 | 203911_at | RAP1GA1 | NM_002885 | RAP1, GTPase activating protein 1 |
| 78 | 203934_at | KDR | NM_002253 | kinase insert domain receptor |
| 79 | 203986_at | GENX-3414 | NM_003943 | genethonin 1 |
| 80 | 204105_s_at | NRCAM | NM_005010 | neuronal cell adhesion molecule |
| 81 | 204124_at | NaPi-IIb | AF146796 | Na dependent phosphate transporter isoform |
| 82 | 204149_s_at | GSTM4 | NM_000850 | glutathione S-transferase M4 |
| 83 | 204152_s_at | | AI738965 | manic fringe (Drosophila) homolog |
| 84 | 204154_at | CDO1 | NM_001801 | cysteine dioxygenase, type I |
| 85 | 204259_at | MMP7 | NM_002423 | matrix metalloproteinase 7 (matrilysin, uterine) |
| 86 | 204260_at | CHGB | NM_001819 | chromogranin B (secretogranin 1) |
| 87 | 204268_at | S100A2 | NM_005978 | S100 calcium-binding protein A2 |
| 88 | 204288_s_at | ARGBP2 | NM_021069 | ArpAbl-interacting prot ArgBP2, trans var 2 |
| 89 | 204298_s_at | LOX | NM_002317 | lysyl oxidase |
| 90 | 204337_at | | NM_005613 | regulator of G-protein signalling 4 |
| 91 | 204416_x_at | APOC1 | NM_001645 | apolipoprotein C-I |
| 92 | 204424_s_at | | NM_018640 | neuronal specific transcription factor DAT1 |
| 93 | 204433_s_at | | NM_006038 | spermatogenesis associated PD1 |
| 94 | 204442_x_at | LTBP4 | NM_003573 | latenTTransforming GFβ binding protein 4 |
| 95 | 204452_s_at | | NM_003505 | frizzled 1 |
| 96 | 204476_s_at | PC | NM_022172 | pyruvate carboxylase |
| 97 | 204503_at | EVPL | NM_001988 | envoplakin |
| 98 | 204591_at | CHL1 | NM_006614 | cell adhesion molecule w/ homology to L1CAM homolog L1 |
| 99 | 204600_at | EPHB3 | NM_004443 | EphB3 |
| 100 | 204623_at | TFF3 | NM_003226 | trefoil factor 3 (intestinal) |
| 101 | 204625_s_at | | NM_000212 | integrin, β3 (platelet glycoprotein IIIa, CD61) |
| 102 | 204697_s_at | CHGA | NM_001275 | chromogranin A |
| 103 | 204741_at | BICD1 | NM_001714 | Bicaudal D (Drosophila) homolog 1 |
| 104 | 204753_s_at | HLF | NM_002126 | hepatic leukemia factor |
| 105 | 204754_at | HLF | NM_002126 | hepatic leukemia factor |
| 106 | 204755_x_at | HLF | NM_002126 | hepatic leukemia factor |

(continued)

| Sequence identifications | | | | |
|---|---|---|---|---|
| SEQ NO: | psids | Name | Accession No. | Description |
| 107 | 204787_at | Z39IG | NM_007268 | Ig superfamily protein |
| 108 | 204797_s_at | EMAPL | NM_004434 | echinoderm microtubute-associated pro-like |
| 109 | 204869_at | | AL031664 | DNA seq RP4-531H16 chrom 20p11.22-12 |
| 110 | 204870_s_at | PCSK2 | NM_002594 | proprotein convertase subtilisinkexin type 2 |
| 111 | 204933_s_at | TNFRSF11B | NM_002546 | TNFR superfamily, member 11 b |
| 112 | 204934_s_at | HPN | NM_002151 | hepsin (transmembrane protease, serine 1) |
| 113 | 204944_at | PTPRG | NM_002841 | protein tyrosine phosphatase receptor type G |
| 114 | 204964_s_at | SSPN | NM_005086 | sarcospan (Kras oncopene-associated gene) |
| 115 | 204975_at | EMP2 | No_001424 | ithelial membrane protein 2 |
| 116 | 204990_s_at | ITGB4 | NM_000213 | intearin, beta 4 |
| 117 | 205051_s_at | KIT | NM_000222 | v-kit Hardy-Zuckerman 4 feline sarcoma viral oncogene homolog |
| 118 | 205110_s_at | FGF13 | NM_004114 | fibroblast growth factor 13 |
| 119 | 205153_s_at | TNFRSF5 | NM_001250 | TNFR superfamily, mem 5 |
| 120 | 205168_at | DDR2 | NM_006182 | discoidin domain receptor amity, member 2 |
| 121 | 205258_at | INHBB | NM_002193 | inhibin, β B (activin AB β polypeptide) |
| 122 | 205286_at | | NM_003222 | transcription factor AP-2 gamma |
| 123 | 205325_at | KIAA0273 | NM_014759 | KIAA0273 gene product |
| 124 | 205336_at | PVALB | NM_002854 | parvalbumin |
| 125 | 205402_x_at | PRSS2 | NM_002770 | protease, serine, 2 (trypsin 2) |
| 126 | 205413_at | C11ORF8 | NM_001584 | chromosome 11 open reading frame 8 |
| 127 | 205455_at | MST1R | NM_002447 | macrophape stimulating 1 receptor |
| 128 | 205470_s_at | KLK11 | NM_006853 | kallikrein 11 |
| 129 | 205479_s_at | PLAU | NM_002658 | plasminogen activator, urokinase |
| 130 | 205481_at | ADORA1 | NM_000674 | adenosine A1 receptor |
| 131 | 205485_at | RYR1 | NM_000540 | ryanodine receptor 1 (skeletal) |
| 132 | 205490_x_at | connexin 31 | NM_024009 | gap junction protein, beta 3, 31 kD |
| 133 | 205531_s_at | GA | NM_013267 | breast cell glutaminase |
| 134 | 205593_s_at | PDE9A | NM_002606 | phosphodiesterase 9A |
| 135 | 205614_x_at | MST1 | NM_020998 | macrophage stimulating 1 |
| 136 | 205627_at | | NM_001785 | cytidine deaminase |
| 137 | 205639_at | | NM_001637 | acyloxyacyl hydrolase |
| 138 | 205683_x_at | TPSB1 | NM_003294 | tryptase beta 1 |
| 139 | 205689_at | KIAA0435 | NM_014801 | KIAA0435 gene product |
| 140 | 205700_at | RODH | NM_003725 | oxidative 3 α hydroxysteroid dehydrogenase; retinol dehydrogenase; 3-hydroxysteroid epimerase |

(continued)

| Sequence identifications | | | | |
|---|---|---|---|---|
| SEQ NO: | psids | Name | Accession No. | Description |
| 141 | 205710_at | LRP2 | NM_004525 | low density lipoprotein-related protein 2 |
| 142 | 205715_at | BST1 | NM_004334 | bone marrow stromal cell antigen 1 |
| 143 | 205717_x_at | | NM_002588 | protocadherin gamma subfamily C, 3 |
| 144 | 205728_at | | AL022718 | DNA seq from clone 1052M9 on chrom Xq25 |
| 145 | 205747_at | CBLN1 | NM_004352 | cerebellin 1 *precursor* |
| 146 | 205778_at | KLK7 | NM_005046 | kallikrein 7 (chymotryptic, stratum corneum) |
| 147 | 205858_at | NGFR | NM_002507 | nerve growth factor receptor |
| 148 | 205927_s_at | CTSE | NM_001910 | cathepsin E |
| 149 | 205980_s_at | ARHGAP8 | NM_015366 | Rho GTPase activating protein 8 |
| 150 | 206002_at | GPR64 | NM_005756 | G protein-coupled receptor 64 |
| 151 | 206114_at | EPHA4 | NM_004438 | EphA4 |
| 152 | 206390_x_at | | NM_002619 | platelet factor 4 |
| 153 | 206594_at | KIAA0135 | NM_015148 | KIAA0135 protein |
| 154 | 206595_at | CST6 | NM_001323 | cystatin EM |
| 155 | 206714_at | ALOX15B | NM_001141 | arachidonate 15-lipoxygenase, second type |
| 156 | 206757_at | PDE5A | NM_001083 | phosphodiesterase 5A, cGMP-specific |
| 157 | 206866_at | CDH4 | NM_001794 | cadherin 4, type 1, R-cadherin (retinal) |
| 158 | 206884_s_at | SCEL | NM_003843 | sciellin |
| 159 | 206912_at | FOXE1 | NM_004473 | forkhead box E1 (thyroid transcription factor2) |
| 160 | 207111_at | | NM_001974 | egf-like module cont., mucin-like, hormone rec-like seq 1 |
| 161 | 207144_s_at | CITED1 | NM_004143 | Cbpp300-interacting transactivator, with GluAsp-rich carboxy-terminal domain, 1 |
| 162 | 207173_x_at | | NM_001797 | OB-cadherin-1 |
| 163 | 207674_at | FCAR | NM_002000 | Fc fragment of IgA |
| 164 | 207695_s_at | IGSF1 | NM_001555 | immunoglobulin superfamily, member 1 |
| 165 | 207795_s_at | CD94 | AB009597 | |
| 166 | 207826_s_at | ID3 | NM_002167 | inhibitor of DNA binding 3, dominant negative helix-loop-helix protein |
| 167 | 207923_x_at | PAX8 | NM_013953 | paired box gene 8 |
| 168 | 208396_s_at | PDE1A | NM_005019 | phosphodiesterase 1A, calmodulin-dependent |
| 169 | 208451_s_at | C4B | NM_000592 | complement component 4B |
| 170 | 208712_at | PRAD1 | M73554 | cyclinD1 |
| 171 | 208747_s_at | | NM_001734 | subcomponent C1s, $\alpha$- and $\beta$-chains |
| 172 | 209021_x_at | | BC001331 | Similar to KIAA0652 gene product |
| 173 | 209035_at | | NM_002391 | midkine |
| 174 | 209071_s_at | | AF159570 | regulator of G-protein signalling 5 |

(continued)

| Sequence identifications | | | | |
|---|---|---|---|---|
| SEQ NO: | psids | Name | Accession No. | Description |
| 175 | 209079_x_at | | AF152318 | protocadherin gamma A1 |
| 176 | 209173_at | | NM_006408 | putative secreted protein XAG |
| 177 | 209208_at | | NM_004870 | clone 015e11 My008 protein |
| 178 | 209228_x_at | | NM_006765 | Putative prostate cancer tumor suppressor |
| 179 | 209270_at | LAMB3 | NM_000228 | laminin S B3 chain |
| 180 | 209280_at | NM_006039 | NM_006039 | chromosome 17 unknown product mRNA |
| 181 | 209291_at | | NM_001546 | inhibitor of DNA binding 4, dominant negative helix-loop-helix protein |
| 182 | 209297_at | ITSN | AF114488 | intersectin short isoform |
| 183 | 209335_at | | AI281593 | |
| 184 | 209365_s_at | ECM1 | NM_004425 | extracellular matrix protein 1 |
| 185 | 209386_at | | AI346835 | transmembrane 4 superfamily member 1 |
| 186 | 209485_s_at | ORP1 | AF274714 | oxysterol-binding protein-related protein |
| 187 | 209496_at | | BC000069 | retinoic acid receptor responder 2 |
| 188 | 209505_at | | NM_005654 | nuclear receptor subfam 2, group F, mem 1 |
| 189 | 209506_s_at | | BC004154 | nuclear receptor subfam 2, group F, mem 1 |
| 190 | 209529_at | | AF047760 | phosphatidic acid phosphohydrolase type-2c |
| 191 | 209596_at | | AF245505 | adlican |
| 192 | 209598_at | KIAA0883 | AB020690 | KIAA0883 protein |
| 193 | 209652_s_at | | B001422 | Similar to placental growth factor, vascular endothelial growth factor-related protein |
| 194 | 209691_s_at | | BC003541 | hypothetical protein FLJ10488 |
| 195 | 209739_s_at | | AI814551 | GS2 gene |
| 196 | 209772_s_at | | X69397 | cell surface antigen |
| 197 | 209781_s_at | T-Star | AF069681 | T-Star |
| 198 | 209792s_at | | BC002710 | kallikrein 10 |
| 199 | 209810_at | SP-B | J02761 | pulmonary surfactant-associated protein B |
| 200 | 209897_s_at | | AF055585 | neurogenic extracellular slit protein Slit2 |
| 201 | 209924_at | | AB000221 | small inducible cytokine subfamily A (Cys-Cys), mem18, pulmonary/activation-reg |
| 202 | 209946_at | | U58111 | FLT4 ligand |
| 203 | 209990_s_at | | NM_005458 | GABA-B receptor |
| 204 | 210051_at | cAMP-GEFI | U78168 | cAMP-regulated guanine nucleotide exchange factor I |
| 205 | 210055_at | | NM_000369 | thyroid stimulating hormone receptor |
| 206 | 210072_at | | NM_006274 | beta chemokine Exodus-3 |
| 207 | 210078_s_at | | L39833 | K+ channel beta subunit |

(continued)

| Sequence identifications | | | | |
|---|---|---|---|---|
| SEQ NO: | psids | Name | Accession No. | Description |
| 208 | 210096_at | | NM_000779 | lung cytochrome P450 (IV subfamily) BI |
| 209 | 210298_x_at | FHL1 | AF098518 | four and 1/2 LIM domainsl protein isoform B |
| 210 | 210321_a_t | | M36118 | cytotoxin serine protease-C |
| 211 | 210342_s_at | TPO | NM-000547 | thyroid peroxidase |
| 212 | 210372_s_at | TPD52L2 | AF208012 | tumor protein D52-like 2 |
| 213 | 210397_a_t | | U73945 | beta-defensin-1 |
| 214 | 210401_at | | U45448 | P2x1 receptor |
| 215 | 210471_s_at | | U33428 | K+ channel β 1a subunit mRNA, alt spliced |
| 216 | 210473_s_at | p58GTA | M37712 | galactosyltransferase assoc protein kinase |
| 217 | 210605_s_at | | BC003610 | Similar to milk fat globule-EGF factor 8 |
| 218 | 210640_s_at | GPCR-Br | U63917 | G protein coupled receptor |
| 219 | 210660_at | LIR-6 | AF025529 | leucocyte immunoglobulin-like receptor-6b |
| 220 | 210727_a_t | | NM_001741 | Calcitonin, calcitonincalcitonin-rel polypeptide, α |
| 221 | 210762_s_at | HP | NM_006094 | HP protein |
| 222 | 210809_s_at | | D13665 | osf-2 mRNA for osteoblast specific factor 2 |
| 223 | 210827_s_at | ESE-1 | U73844 | epithelial-specific transcription factor ESE-1a |
| 224 | 211100_x_at | | U82278 | immunoglobulin-like transcript 1c |
| 225 | 211101_x_at | | U82276 | immunoglobulin-like transcript 1a |
| 226 | 211102_s_at | | U82277 | immunoglobulin-like transcript 1b |
| 227 | 211161_s_at | | AF130082 | collagen, type III, alpha 1 |
| 228 | 211217_s_at | | AF051426 | slow delayed rectifier channel subunit |
| 229 | 211538_s_at | | U56725 | heat shock 70kD protein 2 |
| 230 | 211564_s_at | | BC003096 | Similar to LIM domain protein |
| 231 | 211679_x_at | GABBR2 | AF095784 | GABA-B receptor R2 |
| 232 | 211813_x_at | | AF138303 | decorin D |
| 233 | 211959_at | IGFBP5 | AW007532 | insulin-like growth factor binding protein 5 |
| 234 | 211964_at | | AK025912 | type IV collagen alpha (2) chain |
| 235 | 212067_s_at | | AL573058 | complement component 1, r subcomponent |
| 236 | 212253_x_at | KIAA0728 | BG253119 | KIAA0728 protein |
| 237 | 212294_at | | BG111761 | DKFZp586B0918 |
| 238 | 212328_at | KIAA1102 | AB029025 | KIAA1102 protein |
| 239 | 212344_at | KIAA1077 | AW043713 | KIAA1077 protein |
| 240 | 212353_at | KIAA1077 | A479175 | KIAA1077 protein |
| 241 | 212354_at | KIAA1077 | BE500977 | KIAA1077 protein |
| 242 | 212464_s_at | FN | X02761 | fibronectin precursor |
| 243 | 212724_at | | NM_005168 | ras homolog gene family, member E |

(continued)

| Sequence identifications | | | | |
|---|---|---|---|---|
| SEQ NO: | psids | Name | Accession No. | Description |
| 244 | 212738_at | | AV717623 | |
| 245 | 212775_at | | AI978623 | KIAA0657 protein |
| 246 | 212803_at | | NM_005967 | NGFI-A binding protein 2 |
| 247 | 212806_at | KIAA0367 | AL138349 | KIAA0367 protein |
| 248 | 212850_s_at | | AA584297 | low density lipoprotein receptor-rel protein 4 |
| 249 | 212865_s_at | | BF449063 | collagen, type XIV, alpha 1 (undulin) |
| 250 | 212912_at | | AI992251 | Ribosomal pro S6 kinase, 90 kDa, polypep 2 |
| 251 | 212992_at | | AI935123 | |
| 252 | 213029_at | | AL110126 | DKFZp564H1916 |
| 253 | 213306_at | | AA917899 | multiple PDZ domain protein |
| 254 | 213381_at | | N91149 | DKFZp586M2022 |
| 255 | 213423_x_at | AI884858 | AI884858 | Putative prostate cancer tumor suppressor |
| 256 | 213553_x_at | | W79394 | apolipoprotein C-I |
| 257 | 213668_s_at | | AI989477 | SRY (sex determining region Y)-box 4 |
| 258 | 213693_s_at | | AI610869 | mucin 1, transmembrane |
| 259 | 213800_at | | X04697 | complement factor H 38-kDa N-term frag |
| 260 | 213904_at | | AL390170 | DKFZp547E184 |
| 261 | 213924_at | FLJ11585 | BF476502 | hypothetical protein FLJ11585 |
| 262 | 214023_x_at | | AL533838 | tubulin, beta polypeptide |
| 263 | 214175_x_at | 214175_x_at | AI254547 | LIM domain protein |
| 264 | 21239_x_at | Mel-18 | AI560455 | Zinc finger protein 144 |
| 265 | 214307_at | | AI478172 | homogentisate 1,2-dioxygenase |
| 266 | 214434_at | KIAA0417 | AB007877 | KIAA0417 gene product |
| 267 | 214632_at | | NM_003872 | neuropilin 2 |
| 268 | 214680_at | | BF674712 | neurotrophic tyrosine kinase receptor 2 |
| 269 | 214702_at | MSF-FN70 | AJ276395 | migration stimulation factor FN70 |
| 270 | 214763_at | FLJ13875 | AK023937 | FLJ13875 |
| 271 | 214803_at | | BF344237 | DKFZp564N1116 |
| 272 | 214955_at | | AI912086 | DNA seq clone 1170K4 chrom 22q12.2-13.1 |
| 273 | 214977_at | FLJ13790 | AK023852 | FLJ13790 |
| 274 | 215016_x_at | KIAA0728 | BC004912 | KIAA0728 protein |
| 275 | 215034_s_at | FLJ13302 | AI189753 | FLJ13302 |
| 276 | 215076_s_at | FLJ11428 | AU144167 | FLJ11428 |
| 277 | 215243_s_at | GJB3 | AF099730 | connexin 31 |
| 278 | 215388_s_at | | X56210 | complement Factor H-related protein 1 |
| 279 | 215442_s_at | | BE740743 | thyroid stimulating hormone receptor |

(continued)

| Sequence identifications | | | | |
|---|---|---|---|---|
| SEQ NO: | psids | Name | Accession No. | Description |
| 280 | 215443_at | | BE740743 | thyroid stimulating hormone receptor |
| 281 | 215506_s_at | | AK021882 | highly sim to putative tumor sup NOEY2 |
| 282 | 215536_at | | X87344 | DMA, DMB, HLA-Z1, IPP2, LMP2, TAP1, LMP7, TAP2, DOB, DQB2 and RING8, 9, 13, 14 genes |
| 283 | 216356_x_at | KIAA0734 | AB018277 | KIAA0734 protein |
| 284 | 216470_x_at | | AF009664 | T cell receptor β locus, 3 trypsinogen repeats |
| 285 | 216569_at | FABP3-ps | U72237 | fatty acid-binding protein pseudogene |
| 286 | 217546_at | | R06655 | |
| 287 | 217561_at | | BF447272 | |
| 288 | 217592_at | | AV684859 | |
| 289 | 217767_at | | NM_000064 | |
| 290 | 217820_s_at | FLJ10773 | NM_018212 | hypothetical protein FLJ10773 |
| 291 | 217875_s_at | TMEPAI | NM_020182 | transmem, prostate androgen induced RNA |
| 292 | 218002_s_at | SCYB14 | NM_004887 | small inducible cytokine subfam B (Cys-X-Cys), mem 14 (BRAK) |
| 293 | 218182_s_at | CLDN1 | NM_021101 | claudin 1 |
| 294 | 218353_at | | NM_025226 | MSTP032 protein |
| 295 | 218368_s_at | FN14 | NM_016639 | type I transmembrane protein Fn14 |
| 296 | 218418_s_at | | NM_015493 | DKFZP434N161 |
| 297 | 218469_at | CKTSF1B1 | NM_013372 | Cys knot superfamily 1, BMP antagonist 1 |
| 298 | 218537_at | FLJ20568 | NM_017885 | hypothetical protein FLJ20568 |
| 299 | 218546_at | FLJ14146 | NM_024709 | hypothetical protein FLJ14146 |
| 300 | 218613_at | | NM_018422 | hypothetical protein DKFZp761 K1423 |
| 301 | 218653_at | SLC25A15 | NM_014252 | solute carrier family 25 (mitochondrial carrier; ornithine transporter) member 15 |
| 302 | 218691_s_at | RIL | NM_003687 | reversion-induced LIM protein |
| 303 | 218844_at | FLJ20920 | NM_025149 | hypothetical protein FLJ20920 |
| 304 | 218856_at | LOC51323 | NM_016629 | hypothetical protein LOC51323 |
| 305 | 218952_at | SAAS | NM_013271 | granin-like neuroendocrine peptide precursor |
| 306 | 218960_at | TMPRSS4 | NM_016425 | transmembrane protease, serine 4 |
| 307 | 219010_at | FLJ10901 | NM_018265 | hypothetical protein FLJ10901 |
| 308 | 219127_at | MGC11242 | NM_024320 | hypothetical protein MGC11242 |
| 309 | 219191_s_at | BIN2 | NM_016293 | bridging integrator 2 |
| 310 | 219195_at | PPARGC1 | NM_013261 | peroxisome proliferative activated receptor, y, coactivator 1 |
| 311 | 219211_at | USP18 | NM_017414 | ubiquitin specific protease 18 |
| 312 | 219277_s_at | FLJ10851 | NM_018245 | hypothetical protein FLJ10851 |

(continued)

| Sequence identifications | | | | |
|---|---|---|---|---|
| SEQ NO: | psids | Name | Accession No. | Description |
| 313 | 219331_s_at | FLJ10748 | NM_018203 | hypothetical protein FLJ10748 |
| 314 | 219416_at | CSR1 | NM_016240 | CSR1 protein |
| 315 | 219436_s_at | LOC51705 | NM_016242 | endomucin-2 |
| 316 | 219440_at | RAI2 | NM_021785 | retinoic acid induced 2 |
| 317 | 219463_at | HS1119D91 | NM_012261 | sim to S68401 (cattle) glucose induced gene |
| 318 | 219476_at | MGC4309 | NM_024115 | hypothetical protein MGC4309 |
| 319 | 219525_at | FLJ10847 | NM_018242 | hypothetical protein FLJ10847 |
| 320 | 219527_at | FLJ20605 | NM_017898 | hypothetical protein FLJ20605 |
| 321 | 219561_at | LOC51226 | NM_016429 | COPZ2 for nonclathrin coat protein zeta-COP |
| 322 | 219596_at | LOC56906 | NM_020147 | hyp protein from EUROIMAGE 511235 |
| 323 | 219597_s_at | DUOX1 | NM_017434 | dual oxidase 1 |
| 324 | 219743_at | HEY2 | NM_012259 | hairyenhancer-of-split rel with YRPW motif 2 |
| 325 | 219749_at | FLJ20967 | NM_022071 | hypothetical protein FLJ20967 |
| 326 | 219836_at | MGC10796 | NM_024508 | hypothetical protein MGC10796 |
| 327 | 219855_at | FLJ10628 | NM_018159 | hypothetical protein FLJ10628 |
| 328 | 219856_at | MGC2742 | NM_023938 | hypothetical protein MGC2742 |
| 329 | 219926_at | POP3 | NM_022361 | popeye protein 3 |
| 330 | 219932_at | VLCS-H1 | NM_014031 | v long-chain acyl-CoA synthetase homolog 1 |
| 331 | 219958_at | FLJ11190 | NM_018354 | hypothetical protein FLJ11190 |
| 332 | 220034_at | | NM_007199 | interleukin-1 receptor-associated kinase M |
| 333 | 220108_at | GNA14 | NM_004297 | guanine nucl binding protein (G protein), $\alpha$ 14 |
| 334 | 220332_at | CLDN16 | NM_006580 | claudin 16 |
| 335 | 220595_at | DKFZp434B0 417 | NM_013377 | hypothetical protein DKFZp434B0417 |
| 336 | 220751_s_at | C5ORF4 | NM_016348 | chromosome 5 open reading frame 4 |
| 337 | 221009_s_at | PGAR | NM_016109 | PPAR(gamma) angiopoietin related protein |
| 338 | 221073_s__at | NOD1 | NM_006092 | caspase recruitment domain 4 |
| 339 | 221147_x_at | WWOX | NM_018560 | WW domain-containing oxidoreductase |
| 340 | 221266_s_at | LOC81501 | NM_030788 | DC-specific transmembrane protein |
| 341 | 221270_s_at | TGT | NM_031209 | tRNA-guanine transglycosylase |
| 342 | 221489_s_at | | AF227517 | sprouty (Drosophila) homolog 4 |
| 343 | 221577_x_at | | AF003934 | prostate differentiation factor |
| 344 | 221636_s_at | | AL136931 | DKFZp586G2122 |
| 345 | 221701_s_at | | AF352728 | STRA6 isoform 1 mRNA, alternatively spliced |
| 346 | 221724_s_at | | AF200738 | C-type lectin DDB27 short form |
| 347 | 221795_at | | AI346341 | Similar to hypothetical protein FLJ20093 |
| 348 | 221796_at | | AA707199 | Similar to hypothetical protein FLJ20093 |

(continued)

| Sequence identifications | | | | |
|---|---|---|---|---|
| SEQ NO: | psids | Name | Accession No. | Description |
| 349 | 221799_at | KIAA1402 | AB037823 | KIAA1402 protein |
| 350 | 221870_at | | AI417917 | FLJ22356 fis |
| 351 | 221900_at | | AI806793 | collagen, type VIII, alpha 2 |
| 352 | 221928_at | | AI057637 | Weakly sim to 2109260A B cell growth factor |
| 353 | 221959_at | | BE672313 | highly sim to HSU79298 Human clone 23803 |
| 354 | 32128_at | | Y13710 | alternative activated macrophage specific CC chemokine 1 |
| 355 | 37004_at | SP-B | J02761 | pulmonary surfactant-associated protein B |
| 356 | 37117_at | | Z83838 | GTPase-activating protein sim to rhoGAP protein. ribosomal protein L6 pseudogene |
| 357 | 37152_at | | L07592 | peroxisome proliferator activated receptor |
| 358 | 37408_at | KIAA0709 | AB014609 | KIAA0709 protein |
| 359 | 38691_s_at | SP5 | J03553 | pulmonary surfactant protein |
| 360 | 45297_at | | AI417917 | |
| 361 | 63305_at | | D81792 | |
| 362 | 823_at | | HSU84487 | CX3C chemokine precursor, alt spliced |
| 363 | 91920_at | | AI205180 | |
| 364 | | | | SGENE forward primer |
| 365 | | | | SGENE reverse primer |
| 366 | | | | SGENE probe |
| 367 | | | | TESTICAN1 forward primer |
| 368 | | | | TESTICAN1 reverse primer |
| 369 | | | | TESTICAN1 probe |
| 370 | | | | GABRE forward primer |
| 371 | | | | GABRE reverse primer |
| 372 | | | | GABRE probe |
| 373 | | | | CDH3 forward primer |
| 374 | | | | CDH3 reverse primer |
| 375 | | | | CDH3 probe |
| 376 | | | | FN1 forward primer |
| 377 | | | | FN1 reverse primer |
| 378 | | | | FN1 probe |
| 379 | | | | TPO-1 forward primer |
| 380 | | | | TPO-1 reverse primer |
| 381 | | | | TPO-1 probe |
| 382 | | | | TPO-2 forward primer |
| 383 | | | | TPO-2 reverse primer |

(continued)

| Sequence identifications | | | | |
|---|---|---|---|---|
| SEQ NO: | psids | Name | Accession No. | Description |
| 384 | | | | TPO-2 probe |
| 385 | | | | KCNAB1-1 forward primer |
| 386 | | | | KCNAB1-1 reverse primer |
| 387 | | | | KCNAB1-1 probe |
| 388 | | | | KCNAB1-2 forward primer |
| 389 | | | | KCNAB1-2 reverse primer |
| 390 | | | | KCNAB1-2 probe |
| 391 | | | | FABP4-1 forward primer |
| 392 | | | | FABP4-1 reverse primer |
| 393 | | | | FABP4-1 probe |
| 394 | | | | FABP4-2 forward primer |
| 395 | | | | FABP4-2 reverse primer |
| 396 | | | | FABP4-2 probe |
| 397 | | | | DOI1-1 forward primer |
| 398 | | | | DOI1-1 reverse primer |
| 399 | | | | DOI1-1 probe |
| 400 | | | | DOI1-2 forward primer |
| 401 | | | | DOI1-2 reverse primer |
| 402 | | | | DOI1-2 probe |
| 403 | | | | B-ACTIN forward primer |
| 404 | | | | B-ACTIN reverse primer |
| 405 | | | | B-ACTIN probe |
| 406 | | | | GOLGIN67 forward primer |
| 407 | | | | GOLGIN67 reverse primer |
| 408 | | | | GOLGIN67 probe |
| 409 | | | | PAX8 forward primer |
| 410 | | | | PAX8 reverse primer |
| 411 | | | | PAX8 probe |
| 412 | | | | HERC forward primer |
| 413 | | | | HERC reverse primer |
| 414 | | | | HERC probe |
| 415 | | | | DDIT3 forward primer |
| 416 | | | | DDIT3 reverse primer |
| 417 | | | | DDIT3 probe |
| 418 | | | | ITM1 forward primer |
| 419 | | | | ITM1 reverse primer |

(continued)

| Sequence identifications | | | | |
|---|---|---|---|---|
| SEQ NO: | psids | Name | Accession No. | Description |
| 420 | | | | ITM1 probe |
| 421 | | | | C1OF24 forward primer |
| 422 | | | | C1OF24 reverse primer |
| 423 | | | | C1OF24 probe |
| 424 | | | | MOT8 forward primer |
| 425 | | | | MOT8 reverse primer |
| 426 | | | | MOT8 probe |
| 427 | | | | ARG2 forward primer |
| 428 | | | | ARG2 reverse primer |
| 429 | | | | ARG2 probe |

## Claims

1. A method of diagnosing thyroid cancer in a biological sample obtained from a patient comprising measuring the expression levels of genes corresponding to SEQ ID NOs: 36, 53, 76, 211 and 242; wherein the gene expression levels above or below pre-determined cut-off levels are indicative of thyroid cancer; wherein gene expression is (a) measured on a microarray or gene chip; or (b) determined by nucleic acid amplification conducted by polymerase chain reaction (PCR) of RNA extracted from the sample.

2. The method of claim 1 wherein the sample was prepared by a method selected from the group consisting of fine needle aspiration, bulk tissue preparation and laser capture microdissection.

3. The method of claim 2 wherein the bulk tissue preparation was obtained from a biopsy or a surgical specimen.

4. The method of claim 1 further comprising measuring the expression level of at least one gene encoding mRNA corresponding to SEQ ID NOs: 142, 219, 309, 9, 12 and/or 18.

5. The method of claim 1 further comprising measuring the expression level of at least one gene constitutively expressed in the sample.

6. The method of claim 1 wherein the comparison of expression patterns is conducted with pattern recognition methods.

7. The method of claim 6 wherein the pattern recognition methods include the use of a Cox proportional hazards analysis.

8. The method of claim 1 wherein the pre-determined cut-off levels are at least about 1.5-fold over- or under- expression in the sample relative to benign cells or normal tissue.

9. The method of claim 1 wherein the microarray is a cDNA array or an oligonucleotide array.

10. The method of claim 9 wherein the microarray or gene chip further comprises one or more internal control reagents.

11. The method of claim 1 wherein said PCR is reverse transcription polymerase chain reaction (RT-PCR).

12. The method of claim 11, wherein the RT-PCR further comprises one or more internal controls.

13. The method of claim 12, wherein the internal control is a method of detecting PAX8 gene expression

14. The method of claim 13, wherein PAX8 gene expression is measured using SEQ ID NOs: 409-411.

15. A composition consisting of one probe set consisting of: SEQ ID NOs: 36, 53, 76, 211 and 242.

16. A kit for use in the method of claim 1, consisting of materials for detecting isolated nucleic acid sequences, their complements, or portions thereof of a combination of genes encoding mRNA corresponding to SEQ ID NOs: 36, 53, 76, 211 and 242.

17. The use of a microarray or gene chip for performing the method of claim 1.

18. The use of claim 17 wherein the microarray comprises isolated nucleic acid sequences, complements, or portions thereof of a combination of genes encoding mRNA corresponding to SEQ ID NOs: 36, 53, 76, 211 and 242.

19. The use of claim 18 wherein the measurement or characterization is at least about 1.5-fold over- or under-expression.

20. The use of claim 18 wherein the microarray comprises a cDNA array or an oligonucleotide array.

21. The use of claim 18 wherein the microarray further comprises or more internal control reagents.

**Patentansprüche**

1. Verfahren zur Diagnose von Schilddrüsenkrebs in einer von einem Patienten erhaltenen biologischen Probe, wobei man die Expressionsniveaus von SEQ ID NO: 36, 53, 76, 211 und 242 entsprechenden Genen misst; wobei die Genexpressionsniveaus ober- oder unterhalb vorbestimmter Ausschlussniveaus Schilddrüsenkrebs anzeigen; wobei die Genexpression (a) auf einem Mikroarray oder Gen-Chip gemessen; oder (b) über mittels Polymerasekettenreaktion (PCR) ausgeführter Nukleinsäureamplifikation von aus der Probe extrahierter RNA bestimmt wird.

2. Verfahren nach AnsprucH1, wobei die Probe mit einem aus der aus Feinnadelbiopsie, Gesamtgewebepräparation und LCM (Laser Capture Microdissection) bestehenden Gruppe ausgewählten Verfahren hergestellt wurde.

3. Verfahren nach Anspruch 2, wobei die Gesamtgewebepräparation von einer Biopsie oder einem chirurgischen Präparat erhalten wurde.

4. Verfahren nach AnsprucH1, bei dem man ferner das Expressionsniveau wenigstens eines SEQ ID NO: 142, 219, 309, 9, 12 und/oder 18 entsprechende mRNA codierenden Gens misst.

5. Verfahren nach AnsprucH1, bei dem man ferner das Expressionsniveau wenigstens eines konstitutiv in der Probe exprimierten Gens misst.

6. Verfahren nach AnsprucH1, wobei der Vergleich von Expressionsmustern mit Mustererkennungsmethoden ausgeführt wird.

7. Verfahren nach Anspruch 6, wobei die Mustererkennungsmethoden die Verwendung einer Cox-Regressionsanalyse (Cox Proportional Hazards Analysis) beinhalten.

8. Verfahren nach AnsprucH1, wobei es sich bei den vorbestimmten Ausschlussniveaus um wenigstens etwa 1,5-fache Über- oder Unterexpression in der Probe relativ zu gutartigen Zellen oder Normalgewebe handelt.

9. Verfahren nach AnsprucH1, wobei es sich bei dem Mikroarray um ein cDNA-Array oder ein Oligonukleotid-Array handelt.

10. Verfahren nach Anspruch 9, wobei das Mikroarray bzw. der Gen-Chip ferner ein oder mehrere interne Kontrollreagentien enthält.

11. Verfahren nach AnsprucH1, wobei es sich bei der PCR um Reverse-Transkription-Polymerasekettenreaktion (RT-PCR) handelt.

12. Verfahren nach AnsprucH11, wobei die RT-PCR ferner eine oder mehrere interne Kontrollen umfasst.

13. Verfahren nach AnsprucH12, wobei die interne Kontrolle ein Verfahren zum Nachweis von PAX8-Genexpression ist.

14. Verfahren nach AnsprucH13, wobei die PAX8-Genexpression unter Verwendung von SEQ ID NO: 409-411 gemessen wird.

**15.** Zusammensetzung bestehend aus einem Sondensatz, bestehend aus: SEQ ID NO: 36, 53, 76, 211 und 242.

**16.** Kit zur Verwendung im Verfahren nach AnsprucH1, bestehend aus Materialien zum Nachweisen isolierter Nukleinsäuresequenzen, ihrer Komplemente oder von Teilen davon einer Kombination von SEQ ID NO: 36, 53, 76, 211 und 242 entsprechende mRNA codierenden Genen.

**17.** Verwendung eines Mikroarrays oder Gen-Chips zur Durchführung des Verfahrens nach AnsprucH1.

**18.** Verwendung nach AnsprucH17, wobei das Mikroarray isolierte Nukleinsäuresequenzen, Komplemente oder Teile davon einer Kombination von SEQ ID NO: 36, 53, 76, 211 und 242 entsprechende mRNA codierenden Genen umfasst.

**19.** Verwendung nach AnsprucH18, wobei es sich bei der Messung oder Charakterisierung um wenigstens etwa 1,5-fache Über- oder Unterexpression handelt.

**20.** Verwendung nach AnsprucH18, wobei das Mikroarray ein cDNA-Array oder ein Oligonukleotid-Array umfasst.

**21.** Verwendung nach AnsprucH18, wobei das Mikroarray ferner ein oder mehrere interne Kontrollreagentien umfasst.

**Revendications**

**1.** Procédé de diagnostic du cancer de la thyroïde dans un échantillon biologique obtenu à partir d'un patient, comprenant la mesure des taux d'expression de gènes correspondant aux SEQ ID NO: 36, 53, 76, 211 et 242 ; dans lequel les taux d'expression génique au-dessus ou au-dessous de seuils de coupure prédéterminés sont indicatifs d'un cancer de la thyroïde; dans lequel l'expression génique est (a) mesurée sur une micromatrice ou une puce à ADN ; ou (b) déterminée par amplification d'acide nucléique conduite par réaction de polymérase en chaîne (PCR) d'ARN extrait de l'échantillon.

**2.** Procédé de la revendication 1 dans lequel l'échantillon est préparé par un procédé choisi dans le groupe constitué de l'aspiration à l'aiguille fine, la préparation de tissu en bloc et la microdissection par capture laser.

**3.** Procédé de la revendication 2, dans lequel la préparation de tissu en bloc a été obtenue d'une biopsie ou d'un spécimen chirurgical.

**4.** Procédé de la revendication 1 comprenant en outre la mesure des taux d'expression d'au moins un gène codant pour un ARNm correspondant à SEQ ID NO: 142, 219, 309, 9, 12 et/ou 18.

**5.** Procédé de la revendication 1 comprenant en outre la mesure des taux d'expression d'au moins un gène constitutivement exprimé dans l'échantillon.

**6.** Procédé de la revendication 1, dans lequel la comparaison de profils d'expression est conduite par des procédés de reconnaissance de profil.

**7.** Procédé de la revendication 6, dans lequel les procédés de reconnaissance de profil comprennent l'utilisation d'une analyse des risques proportionnels de Cox.

**8.** Procédé selon la revendication 1, dans lequel les taux seuils prédéterminés sont au moins environ 1,5 fois surexprimés ou sous-exprimés dans l'échantillon par rapport à des cellules bénignes ou des tissus normaux.

**9.** Procédé de la revendication 1, dans lequel la micromatrice est une matrice d'ADNc ou une matrice d'oligonucléotides.

**10.** Procédé de la revendication 9, dans lequel la micromatrice ou puce à ADN comprend en outre un ou plusieurs réactifs témoins internes.

**11.** Procédé de la revendication 1, dans lequel ladite PCR est la réaction de polymérase en chaîne par transcription inverse (RT-PCR).

**12.** Procédé de la revendication 11, dans lequel la RT-PCR comprend en outre un ou plusieurs témoins internes.

**13.** Procédé de la revendication 12, dans lequel le témoin interne est un procédé de détection de l'expression génique de PAX8.

**14.** Procédé de la revendication 13, dans lequel la détection de l'expression génique de PAX8 est mesurée en utilisant SEQ ID NO: 409-411.

**15.** Composition constituée d'un ensemble de sondes constitué de SEQ ID NO: 36, 53, 76, 211 et 242.

**16.** Kit pour l'utilisation dans le procédé de la revendication 1, constitué de matériaux pour détecter des séquences d'acide nucléique isolées, leurs compléments, ou des parties de celles-ci d'une combinaison de gènes codant pour un ARNm correspondant à SEQ ID NO: 36, 53, 76, 211 et 242.

**17.** Utilisation d'une micromatrice ou puce à ADN pour conduire le procédé de la revendication 1.

**18.** Utilisation de la revendication 17, dans laquelle la micromatrice comprend des séquences d'acide nucléique isolées, des compléments, ou des parties de celles-ci d'une combinaison de gènes codant pour un ARNm correspondant à SEQ ID NO : 36, 53, 76, 211 et 242.

**19.** Utilisation de la revendication 18, dans laquelle la mesure ou caractérisation est une surexpression ou sous-expression d'au moins environ 1,5 fois.

**20.** Utilisation de la revendication 18, dans laquelle la micromatrice comprend une matrice d'ADNc ou une matrice d'oligonucléotides.

**21.** Utilisation de la revendication 18, dans laquelle la micromatrice comprend un ou plusieurs réactifs témoins internes.

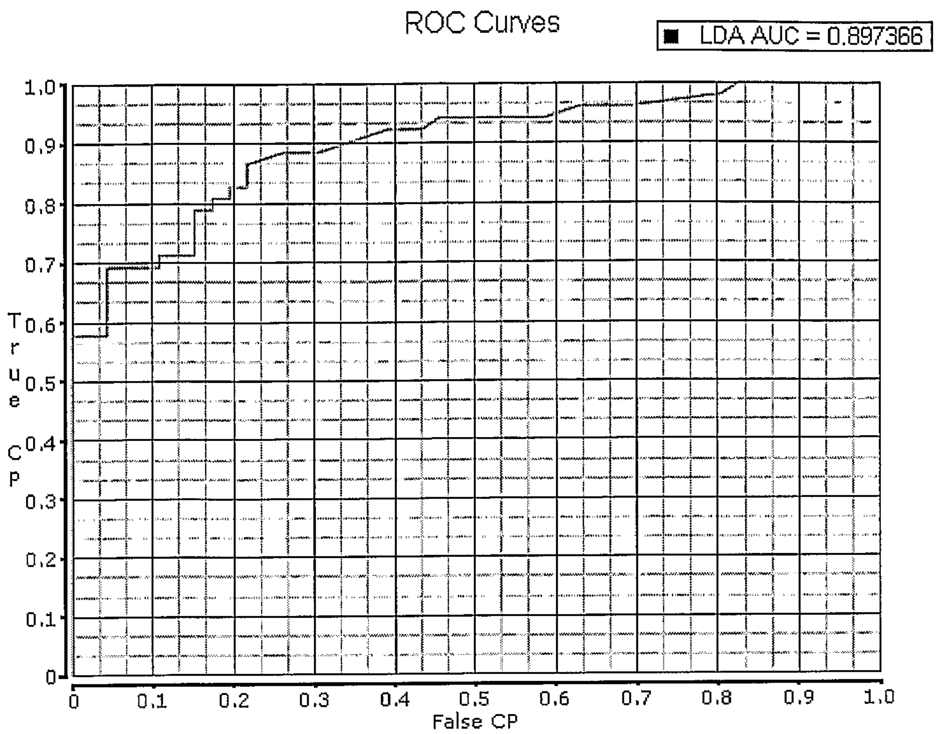


Figure 1

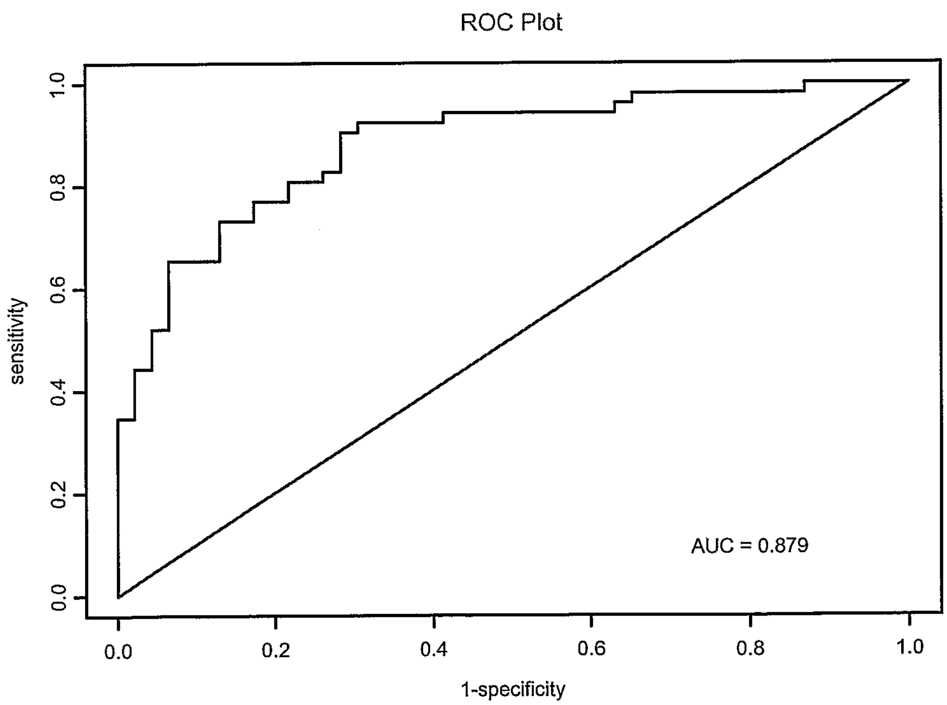


Figure 2

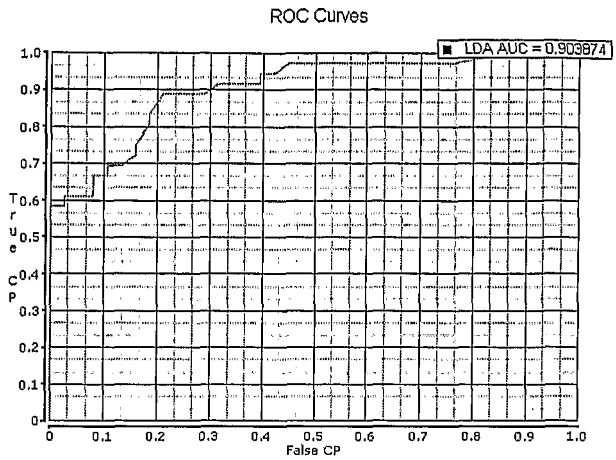


Figure 3a

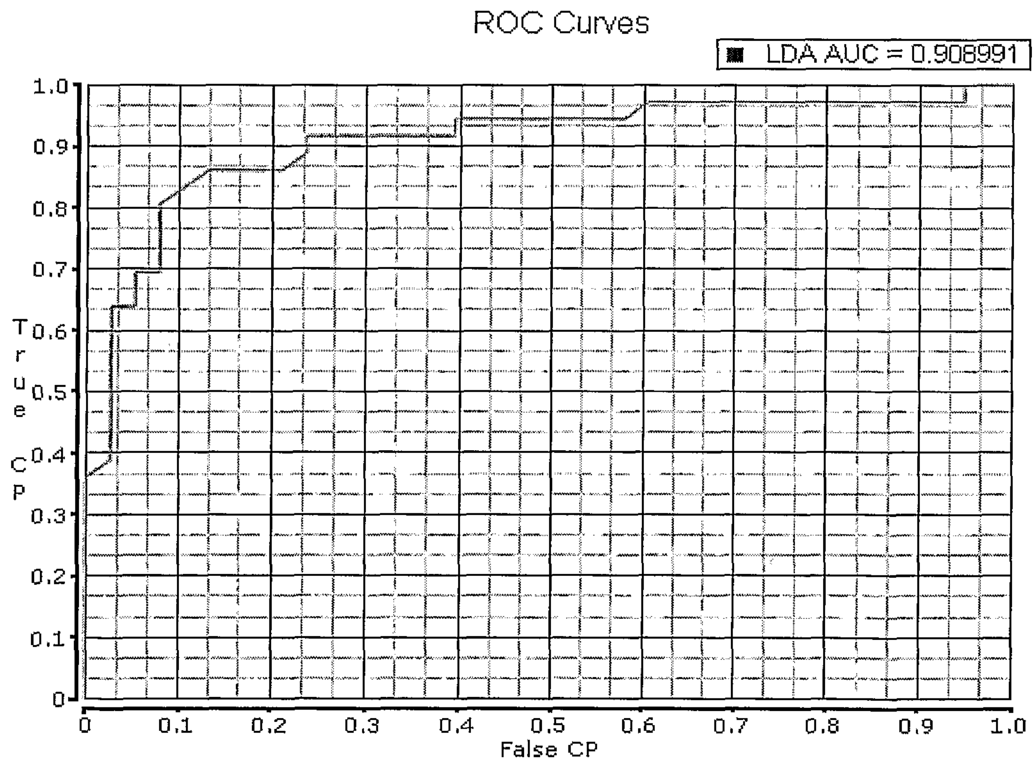


Figure 3b

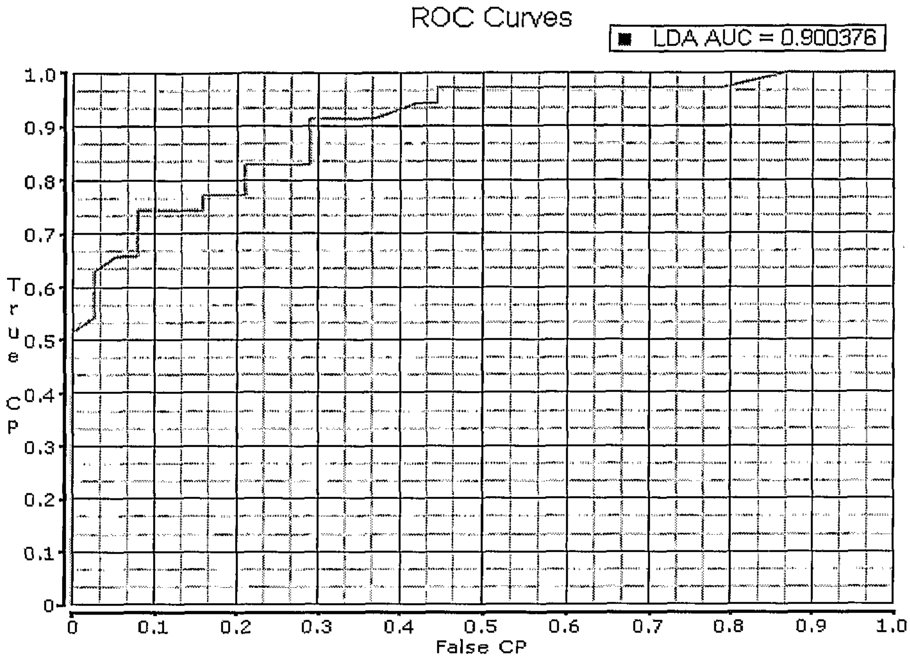


Figure 4a

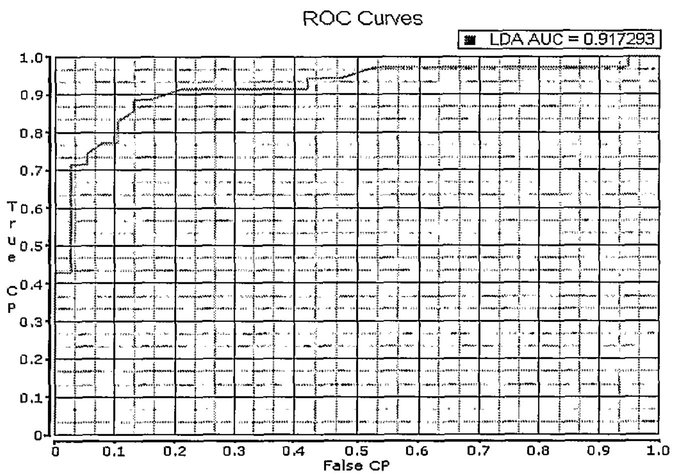


Figure 4b

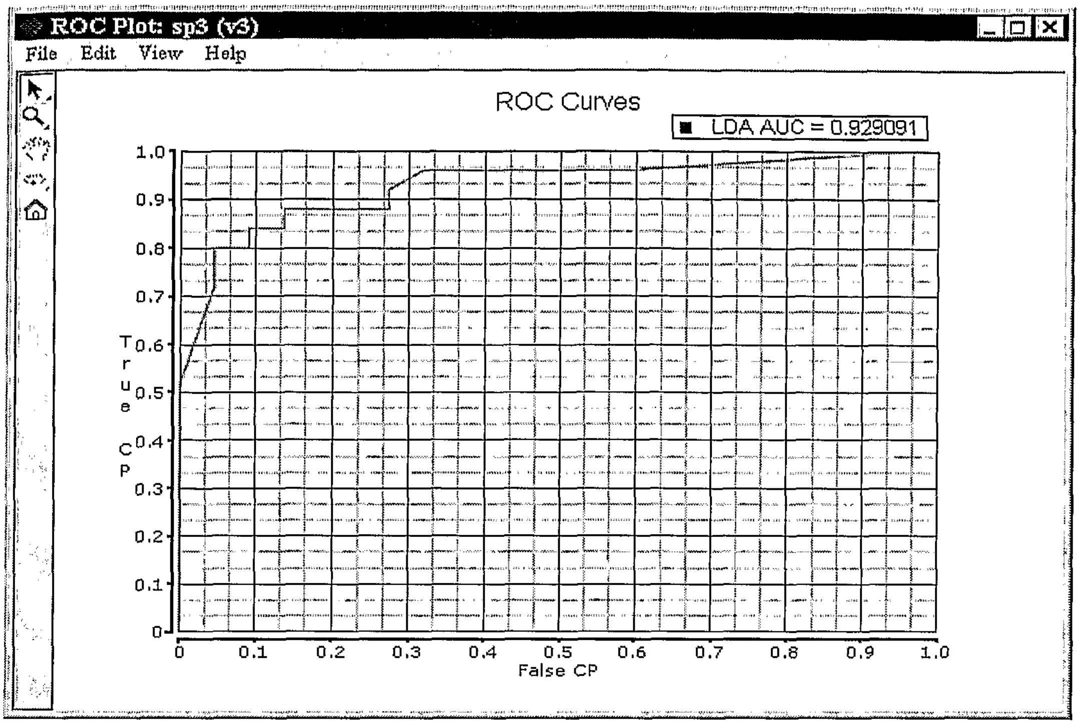


Figure 5a

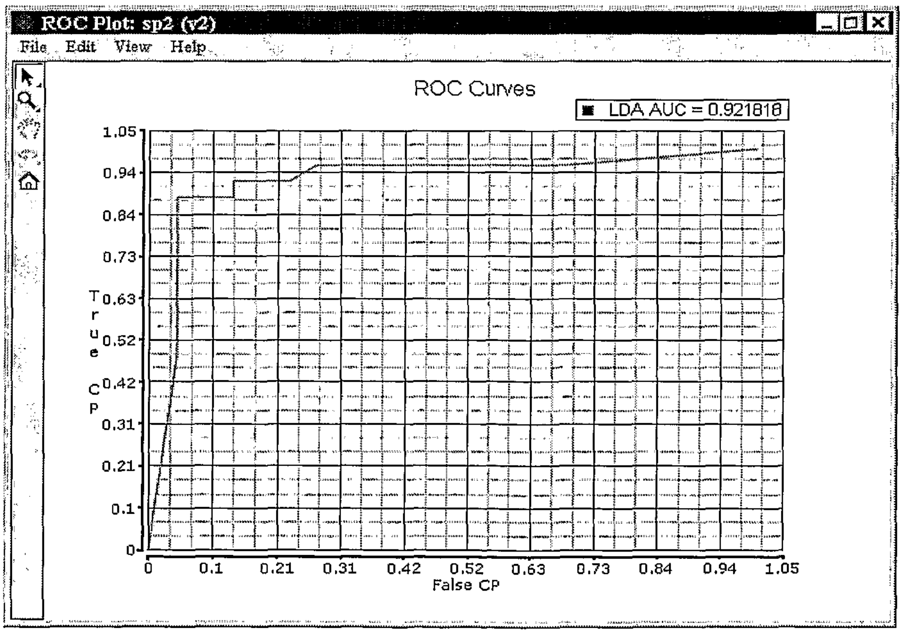


Figure 5b

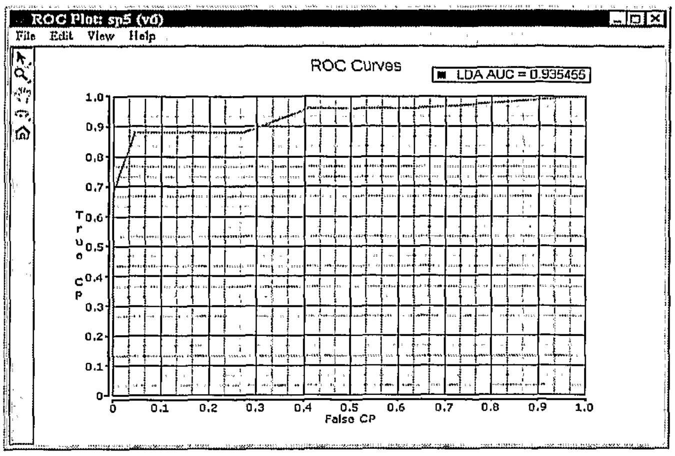


Figure 5c

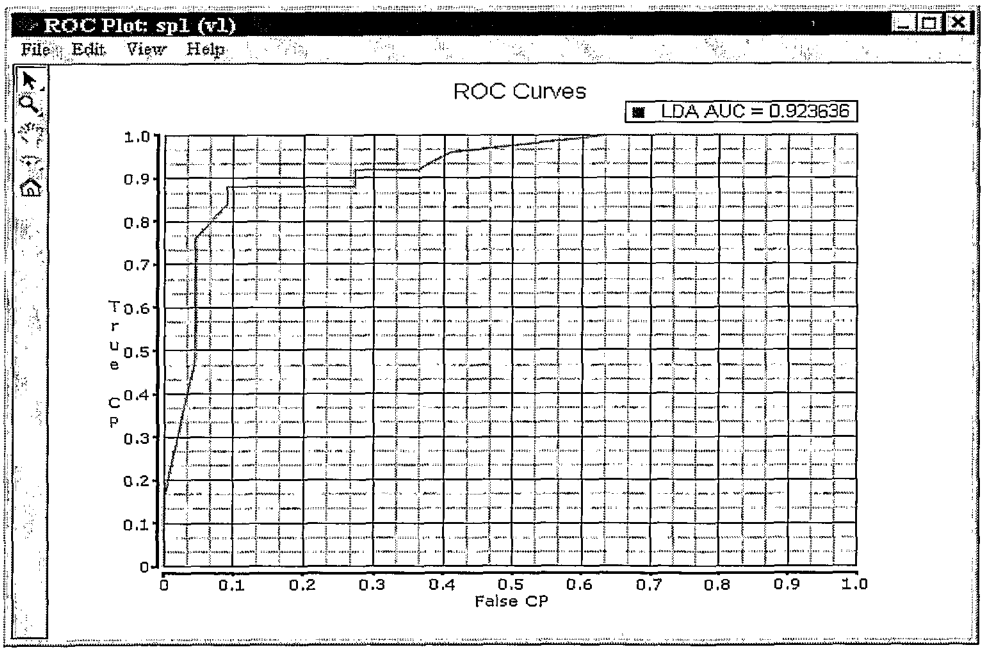


Figure 5d

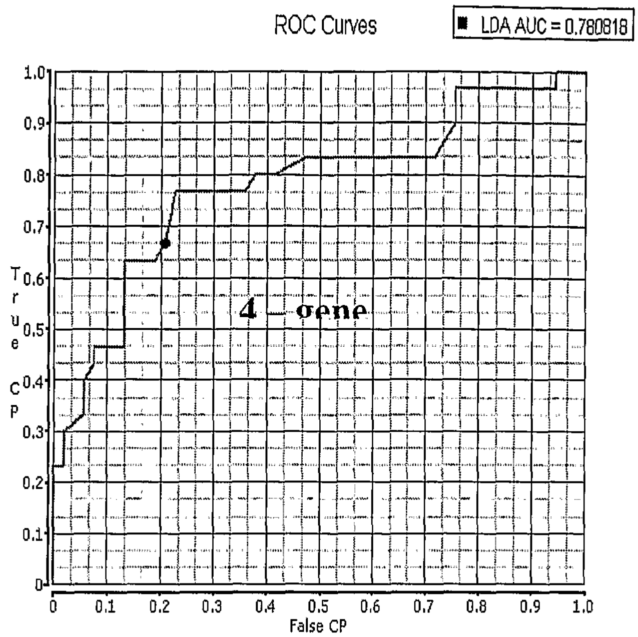


**Figure 6a**

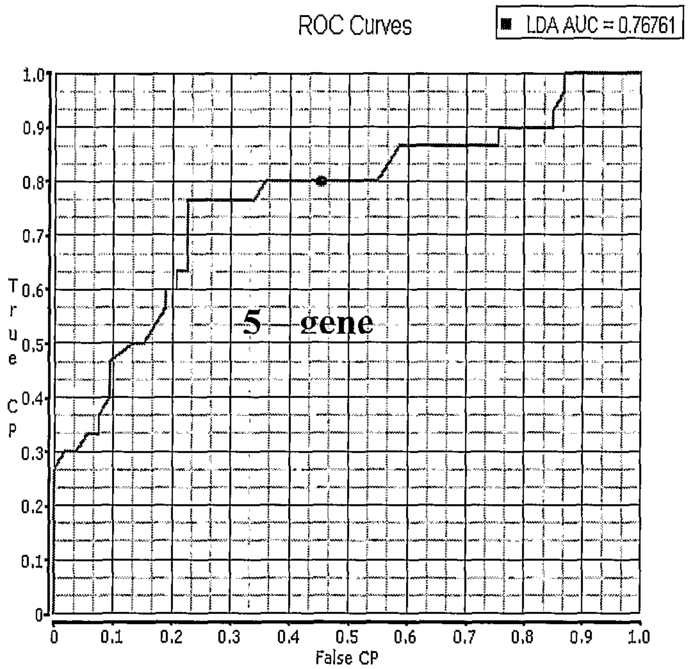


**Figure 6b**

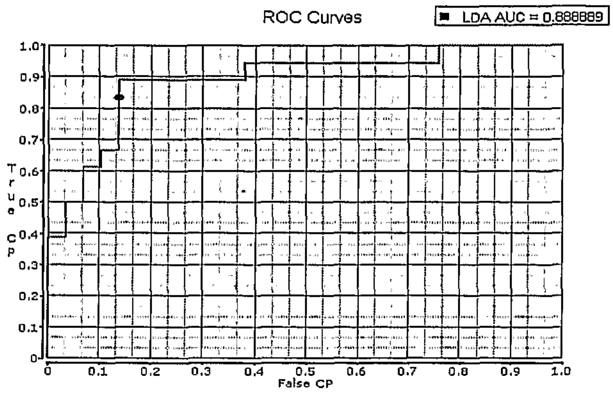


**Figure 7a**

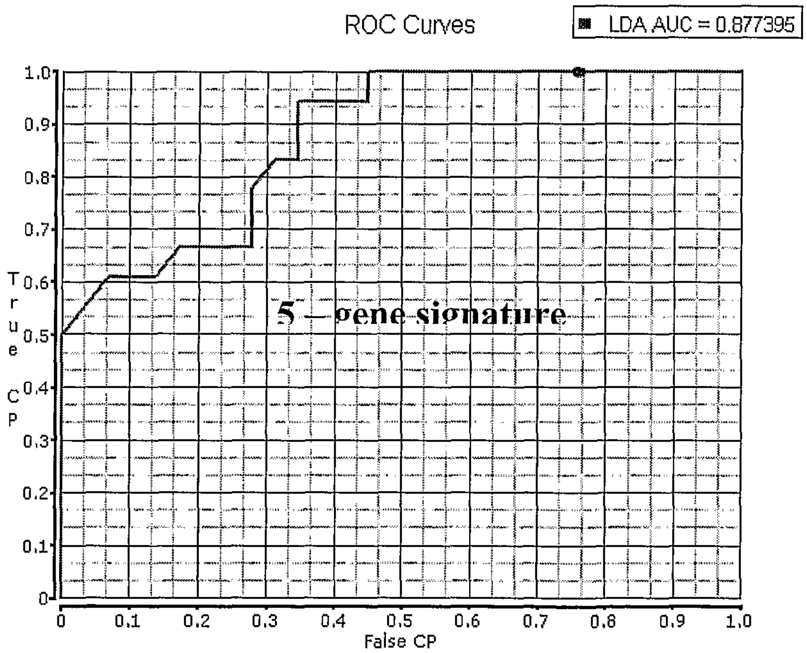


**Figure 7b**

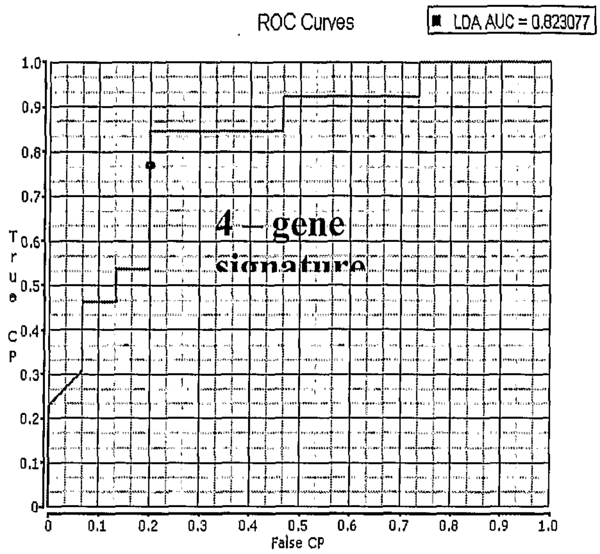
ROC Curves
LDA AUC = 0.823077
4 – gene
signature
True CP
False CP
1.0
0.9
0.8
0.7
0.6
0.5
0.4
0.3
0.2
0.1
0
0
0.1
0.2
0.3
0.4
0.5
0.6
0.7
0.8
0.9
1.0

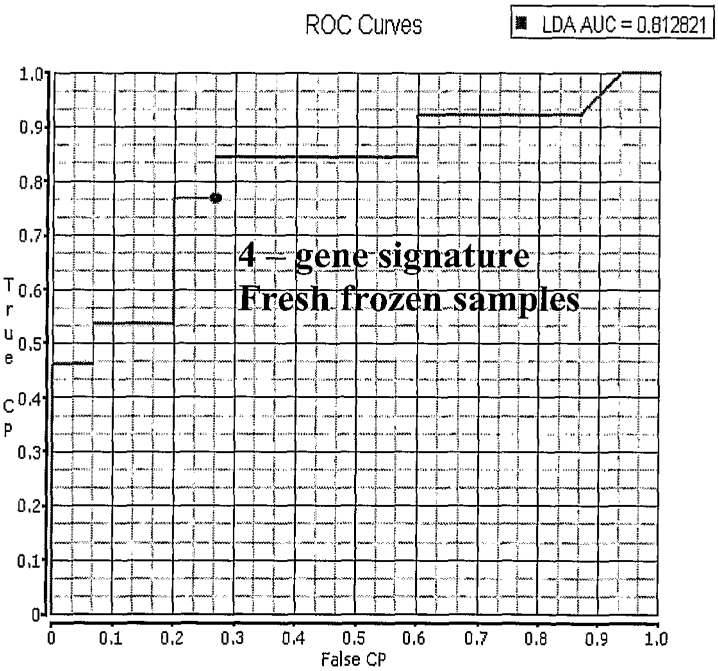
ROC Curves
LDA AUC = 0.812821
4 – gene signature
Fresh frozen samples
True CP
False CP
1.0
0.9
0.8
0.7
0.6
0.5
0.4
0.3
0.2
0.1
0
0
0.1
0.2
0.3
0.4
0.5
0.6
0.7
0.8
0.9
1.0

Figure 8a

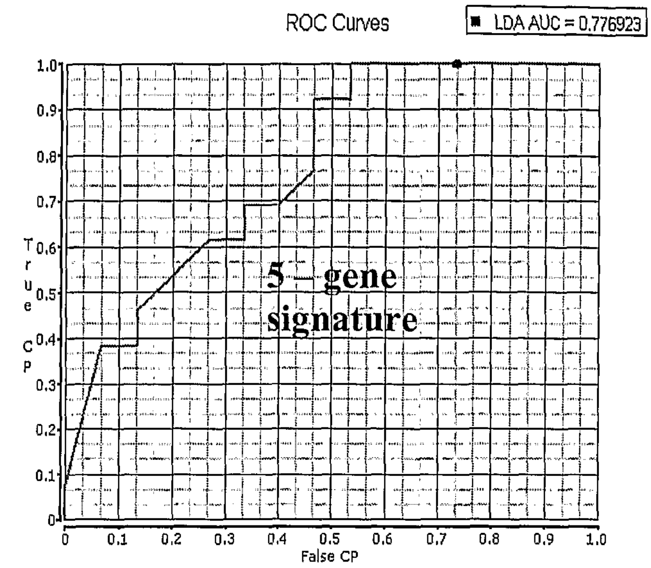
ROC Curves
LDA AUC = 0.776923
True CP
False CP
5 – gene signature
0
0.1
0.2
0.3
0.4
0.5
0.6
0.7
0.8
0.9
1.0

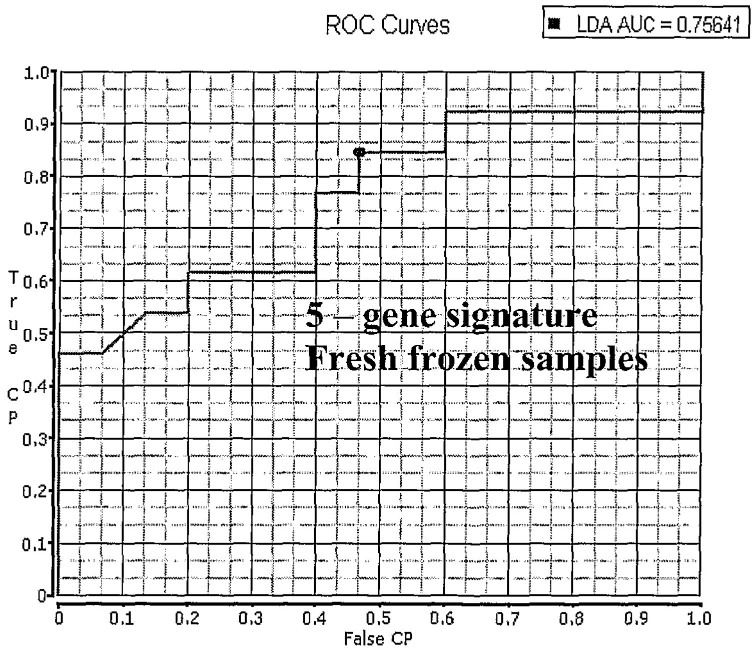
ROC Curves
LDA AUC = 0.75641
True CP
False CP
5 – gene signature
Fresh frozen samples
0
0.1
0.2
0.3
0.4
0.5
0.6
0.7
0.8
0.9
1.0

**Figure 8b**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 6136182 A
- US 20030194406 A
- US 20050037439 A
- US 20040137539 A
- US 6436642 B
- US 20030104419 A
- US 20030232350 A
- US 20040002067 A
- US 20030108963 A
- US 20050037463 A
- US 6066449 A
- US 20030118553 A
- US 20030054571 A
- WO 9102061 A
- WO 9856953 A
- WO 2005005601 A
- US 20020114806 A
- US 20030219760 A
- US 20030096782 A
- US 20020168638 A
- WO 2005020784 A
- WO 2004040014 A
- US 20030060614 A
- EP 1393776 A
- WO 02057414 A
- WO 0116158 A
- US 6831063 B
- WO 2004030615 A
- WO 9733995 A
- US 20040241653 A
- WO 2005015236 A
- WO 9203469 A
- WO 9203152 A
- EP 0546053 A
- US 5445934 A
- US 5532128 A
- US 5556752 A
- US 5242974 A
- US 5384261 A
- US 5405783 A
- US 5412087 A
- US 5424186 A
- US 5429807 A
- US 5436327 A
- US 5472672 A
- US 5527681 A
- US 5529756 A
- US 5545531 A
- US 5554501 A
- US 5561071 A
- US 5571639 A
- US 5593839 A
- US 5599695 A
- US 5624711 A
- US 5658734 A
- US 5700637 A
- US 6271002 B
- US 6218122 B
- US 6218114 B
- US 6004755 B
- US 20030194734 A
- EP 06770766 A
- EP 60683173 A


### Non-patent literature cited in the description


- **KHOLOVA et al.** *Anticancer Res.,* March 2003, vol. 23 (2A), 871-5
- **MAZZANTI et al.** *Cancer Res.,* 15 April 2004, vol. 64 (8), 2898-903
- **JARZAB et al.** *Cancer Res.,* 15 February 2005, vol. 65 (4), 1587-97
- **FINLEY et al.** *Ann Surg.,* September 2004, vol. 240 (3), 425-36, 436-7
- **TAKANO et al.** *Br J Cancer.,* December 2000, vol. 83 (11), 1495-1502
- **GOLUB et al.** *Weighted Voting,* 1999
- **SU et al.** *Support Vector Machines,* 2001
- **RAMASWAMY et al.** *K-nearest Neighbors: Ramaswamy,* 2001
- **VAN'T VEER et al.** *Correlation Coefficients,* 2002

SEQUENCE LISTING - AMENDMENT

<110> JANSSEN DIAGNOSTICS, LLC

<120> THYROID FINE NEEDLE ASPIRATION MOLECULAR ASSAY

<130> P048898EP

<140> EP06770766.1
<141> 2006-05-18

<150> 60/683,173
<151> 2005-05-20

<160> 429

<170> PatentIn version 3.3

<210> 1

<400> 1
000

<210> 2

<400> 2
000

<210> 3

<400> 3
000

<210> 4

<400> 4
000

<210> 5

<400> 5
000

<210> 6

<400> 6
000

<210> 7

<400> 7
000

<210> 8

<400> 8
000

<210> 9
<211> 1887
<212> DNA
<213> human

<400> 9

```
gcgcacggcc tgtccgctgc acaccagctt gttggcgtct tcgtcgccgc gctcgccccg     60
ggctactcct gcgcgccaca atgagctccc gcatcgccag ggcgctcgcc ttagtcgtca    120
cccttctcca cttgaccagg ctggcgctct ccacctgccc cgctgcctgc cactgccccc    180
tggaggcgcc caagtgcgcg ccgggagtcg ggctggtccg ggacggctgc ggctgctgta    240
aggtctgcgc caagcagctc aacgaggact gcagcaaaac gcagccctgc gaccacacca    300
aggggctgga atgcaacttc ggcgccagct ccaccgctct gaaggggatc tgcagagctc    360
agtcagaggg cagaccctgt gaatataact ccagaatcta ccaaaacggg gaaagtttcc    420
agcccaactg taaacatcag tgcacatgta ttgatggcgc cgtgggctgc attcctctgt    480
gtccccaaga actatctctc cccaacttgg gctgtcccaa ccctcggctg gtcaaagtta    540
ccgggcagtg ctgcgaggag tgggtctgtg acgaggatag tatcaaggac cccatggagg    600
accaggacgg cctccttggt aaggagctgg gattcgatgc ctccgaggtg gagttgacga    660
gaaacaatga attgattgca gttggaaaag gcagctcact gaagcggatc cctgtttttg    720
gaatggagcc tcgcatccga tacaaccctt tacaaggcca gaaatgtatt gttcaaacaa    780
cttcatggtc ccagtgctca aagacctgtg gaactggtat ctccacacga gttaccaatg    840
acaaccctga gtgccgcctt gtgaaagaaa cccggatttg tgaggtgcgg ccttgtggac    900
agccagtgta cagcagcctg aaaaagggca agaaatgcag caagaccaag aaatcccccg    960
aaccagtcag gtttacttac gctggatgtt tgagtgtgaa gaaataccgg cccaagtact   1020
gcggttcctg cgtggacggc cgatgctgca cgccccagct gaccaggact gtgaagatgc   1080
ggttccgctg cgaagatggg gagacatttt ccaagaacgt catgatgatc cagtcctgca   1140
aatgcaacta caactgcccg catgccaatg aagcagcgtt tcccttctac aggctgttca   1200
atgacattca caaatttagg gactaaatgc tacctgggtt tccagggcac acctagacaa   1260
acaagggaga agagtgtcag aatcagaatc atggagaaaa tgggcggggg tggtgtgggt   1320
gatgggactc attgtagaaa ggaagccttg ctcattcttg aggagcatta aggtatttcg   1380
```

```
aaactgccaa gggtgctggt gcggatggac actaatgcag ccacgattgg agaatacttt   1440
gcttcatagt attggagcac atgttactgc ttcattttgg agcttgtgga gttgatgact   1500
ttctgttttc tgtttgtaaa ttatttgcta agcatatttt ctctaggctt ttttcctttt   1560
ggggttctac agtcgtaaaa gagataataa gattagttgg acagtttaaa gcttttattc   1620
gtcctttgac aaaagtaaat gggagggcat tccatccctt cctgaagggg gacactccat   1680
gagtgtctgt gagaggcagc tatctgcact ctaaactgca aacagaaatc aggtgtttta   1740
agactgaatg ttttatttat caaaatgtag cttttgggga gggaggggaa atgtaatact   1800
ggaataattt gtaaatgatt ttaattttat attcagtgaa aagattttat ttatggaatt   1860
aaccatttaa taaagaaata tttacct                                       1887
```

<210> 10

<400> 10
000

<210> 11

<400> 11
000

<210> 12
<211> 362
<212> DNA
<213> human

<400> 12

```
gatccagaaa tacttaacac gtgaatattt tgctaaaaaa gcatatataa ctattttaaa     60
tatccattta tcttttgtat atctaagact catcctgatt tttactatca cacatgaata    120
aaggcctttg tatctttctt tctctaatgt tgtatcatac tcttctaaaa cttgagtggc    180
tgtcttaaaa gatataaggg gaaagataat attgtctgtc tctatattgc ttagtaagta    240
tttccatagt caatgatggt ttaataggta aaccaaaccc tataaacctg acctccttta    300
tggttaatac tattaagcaa gaatgcagta cagaattgga tacagtacgg atttgtccaa    360
at                                                                   362
```

<210> 13

<400> 13
000

<210> 14

<400> 14
000

<210> 15

<400> 15
000

<210> 16

<400> 16
000

<210> 17

<400> 17
000

<210> 18
<211> 552
<212> DNA
<213> human

<400> 18

```
agagacatgt accttgacca tcgtccttcc tctcaagcta gcccagaggg tgggagccta     60
aggaagcgtg gggtagcaga tggagtaatg gtcacgaggt ccagacccac tcccaaagct    120
cagacttgcc aggctccctt tctcttcttc cccaggtcct tcctttaggt ctggttgttg    180
caccatctgc ttggttggct ggcagctgag agccctgctg tgggagagcg aagggggtca    240
aaggaagact tgaagcacag agggctaggg aggtggggta catttctctg agcagtcagg    300
gtgggaagaa agaatgcaag agtggactga atgtgcctaa tggagaagac ccacgtgcta    360
ggggatgagg ggcttcctgg gtcctgttcc cctaccccat ttgtggtcac agccatgaag    420
tcaccgggat gaacctatcc ttccagtggc tcgctccctg tagctctgcc tccctctcca    480
tatctccttc ccctacacct ccctccccac acctccctac tcccctgggc atcttctggc    540
ttgactggat gg                                                        552
```

<210> 19

<400> 19
000

<210> 20

<400> 20
000

<210> 21

<400> 21
000

<210> 22

<400> 22
000

<210> 23

<400> 23
000

<210> 24

<400> 24
000

<210> 25

<400> 25
000

<210> 26

<400> 26
000

<210> 27

<400> 27
000

<210> 28

<400> 28
000

<210> 29

<400> 29
000

<210> 30

<400> 30
000

<210> 31

<400> 31
000

<210> 32

<400> 32
000

<210> 33

<400> 33
000

<210> 34

<400> 34
000

<210> 35

<400> 35
000

<210> 36
<211> 4695
<212> DNA
<213> human

<400> 36

```
ggacccgggc cggcccccaa gatgccggcg atcgcggtat tggcggcggc cgccgcggcg     60
tggtgcttcc tccaagtcga gagccggcac ctggacgcgc tcgccggagg cgcgggcccc    120
aaccacggca atttcctaga caatgaccag tggctgagca ccgtctccca gtacgaccgg    180
gacaagtact ggaaccgctt tcgagacgat gattatttca gaaactggaa tcccaacaag    240
ccctttgacc aagccctgga cccatccaag gacccctgcc tgaaggtaaa atgcagccct    300
cacaaagtgt gtgtgaccca ggactaccag accgccctgt gtgtcagccg caagcacctg    360
```

ctccccaggc aaaagaaggg gaacgtggcc cagaaacact gggttggacc ttcgaatttg 420

gtcaagtgca agccctgtcc cgtggcacag tcagccatgg tctgcggctc agatggccac 480

tcctacacat ccaagtgcaa attggagttc catgcttgtt ctactggcaa aagcctcgcc 540

accctctgtg atgggccctg tccctgtctc ccagagcctg agccaccaaa gcacaaggca 600

gaaaggagtg cctgcacaga caaggagttg cggaaccttg cctcccggct gaaggattgg 660

tttggagctc tccacgagga tgcgaacaga gtcatcaagc ccaccagctc caacacagcc 720

caaggcaggt ttgacactag catcctgccc atctgcaagg actccctggg ctggatgttc 780

aacaagttgg acatgaacta tgacctcctg cttgaccctt cagagatcaa tgccatctac 840

ctggataagt acgagccctg tatcaagcct cttttcaact cgtgtgactc cttcaaggat 900

ggcaagcttt ctaacaatga gtggtgctac tgcttccaga agcctggagg tctcccttgc 960

cagaatgaaa tgaacagaat tcagaagctg agtaagggga aaagcctgtt gggggccttc 1020

atacctcggt gtaatgagga gggctattac aaagccacac agtgccacgg cagcacgggg 1080

cagtgctggt gtgtggacaa atatgggaat gagttggctg gctccaggaa acagggtgct 1140

gtgagctgtg aagaggagca ggaaacctca ggggattttg gcagtggtgg gtccgtggtc 1200

ctgctggatg acctagaata tgaacgggag ctgggaccaa aggacaaaga ggggaagctg 1260

agggtgcaca cccgagccgt gacagaggat gatgaggatg aggatgatga caaagaggat 1320

gaggtcgggt acatatggta gtgcccacaa gaaagaggac acaagttttg cacaaaattg 1380

caagtcactt cctattcctg catttgtatc taagactcca aggcaccaag gtctcttctc 1440

cattgttgct ctctataccc gacctaaggt ttggaagaca actgcttgtt cccagaggat 1500

tctgattttg catatgtttg tatgggagaa agggtgttgt gttttttttt ttgttgttgt 1560

ttattttttg gatagggaag tcattggctt aattagagcc tccttccttt ctgtgagatt 1620

tttccaacaa gcatgtgatt tacgtggaat tctgacagtg cagggagccc ccaccctctt 1680

aaatgtcaaa gacccttttt gattacccac actggtggtt attacagcat ggttcccagc 1740

cttacagtgt ctaagtgctt ctcttgtgtc ctgtagatgt tgtgaaaaag aaaaaaacaa 1800

aaaatacacc acactgtact ttttccccct gcccccgcta ctgccggtga ttattattaa 1860

aaattagttt ttttcacatc attatatctg gcttcctata aacaacagcc ttaattcagt 1920

caagactccc tttgggaatt cattttatta aaaattggtg tctggatact tccctgtaca 1980

tgcataaata tgcatgcatg tacaggaaga ctgtatgtgt gtgccttgca cacacaccca 2040

tacctctcag aaaaagtggg tatattaaaa actcgaaaaa caatgataaa tttctcagct 2100

tgtccagacc tggaacaaaa tttctggaat aagaaatttg tattaaagtc ctttttttgca 2160

ctaacagttg gctcttgtag cctgcaggct gaggaagtct cttctctgtg catcagcaga 2220

gttactgaaa gcctctgatt gagaaaaaac ctccgtctgc ctaaatcact tttctcgcag 2280
aagccatgcg actcccacac gacacgggca gcttcacaag ccatctcttt catttctgct 2340
tgaagccccc ttggctgcag caatcctgtc tgccataggt ttcttccttc cttacctact 2400
caagggcttt ttctaaggca tgcacacata tctcctgttc tctgagagta ccatggtgtt 2460
ccttaaaaga agaaaatttc taattctgaa ctcaatgttt tgcttttact ccctttctac 2520
tgacaaatca tgataagggc acaaaagctg tacagatttt ttttttaac cactcaatcc 2580
caaatggagg cctacaaaga acatcgtaat aacacatgga agcaaaccct gggtttttaa 2640
gagcaaattc tgtccccccc tcactccccc aagtgacaag atactaatga agaaagttct 2700
tcaccatagt gtttgtttta actaaactca ttggagtcta gttccaaatt tggtagggtc 2760
atcatctcta cattccttag gatttctctc cctatcaagc tggcccagat acaagtacca 2820
aacagtagtc tctgaagttc cccatttcc ttcagtacca gtctataagc tactgtccgc 2880
cactgatttt catctatcag ggtgtcctaa tcagaatcag ccacccaagc aagcctctct 2940
ggcccacata tctatctctt gccttccccc atgaacttca gcctgtccac acaaaagcca 3000
cataaactca agcaagaaat atgttcagcc aaaacatgat tatagtggca gctgaccaat 3060
cccccatccc aaattgacca tttagatgta ccaactcacc ttaaattagc atgttccaat 3120
ccagtcggca ttgcctgaat acagtagcat catacctata gttggtctta gataagaaat 3180
gaactacttg atatagcaaa gtcctttggc ttcgtaaata accctgaggt tttgtactta 3240
ctttccccat aggaagacag accataggca aactctgttt tgggatctca actccatcac 3300
ctttgtttca atatttttt tctctcttga acaaaactga gataatttag aaaacaggtg 3360
cttaattgca ataaaattac tatgaagtat attaaaaatc acgacattgt aaaatctcac 3420
tttagatcat caaagaaaac cattgttact atctcctttg agcttaggaa aatgtacaag 3480
agaacaaatt aaaattgaaa aattgatttc acttagaaaa acttctagga acagggtgaa 3540
ccactgattt taatttgcct aattatctta tgacaagtat caaattaaga tgacacttaa 3600
agatccttag cattaactta atgatggaga agagtgctca acagacagtt cccagtaagg 3660
taatgagatg ccattttccg agacattcta agaagatatt ttgattcatt aaaacattaa 3720
ataaaaagcc ctcctcagat tggaaccccc aaatcgatgg agtcacatta ataatacttt 3780
tcatgcctca ctttgacatg acagcattcg attttttaa agatctttaa tactttccat 3840
gagtactaaa gattgtaatg agttacctta tccttagaag tagaatgttt gctttcttct 3900
tcttggaaat agtcctccaa aaagtccact tgtaccagtg acaagaagtc acttgtatag 3960
tgaccaagta cacataaaat accgattata aaaatattgt aataaaccac ttcctcattt 4020
gatactggta ttaagctgaa tcctcactaa ttcacttttg aaaagttctt aatgaacagt 4080
tttattctta taccatgaac agctctttta atacaatttc cttggtatcc aacttaaatc 4140

```
ccagaatttt gttccaccat ggttagttat ttgccatatg aatgtattct tgtttctacc   4200
aatattctag gcatgagaat accttaatac tgagttggag attttgtatc tgtttctcct   4260
cctttacgcc cattttccta cccacagcag caaatgacaa cgtgtctgtc caggtctgtc   4320
cccctgctca tcccaggatg ccactcacat ttttttcttc tttgttaccc ttgacccacg   4380
ctgtacaagt aacatccaag agcccattct acaagtgggt ggttttggtc tttttataac   4440
tttttctcaa agtcactgat gtttgttcct gttaaatgta tagcattgta atgagagccc   4500
atcaaatcct gagtgtcagt ttgttgtccc tattgtagat gaaatagtga tgtagcaaaa   4560
acctagtaaa ttctgaatgc ttttccacgt agacttatct ggaatgtgaa cacaactctt   4620
tggttaatag taaatgctta actgtagtcc tgagtaggtg catttctgtc tgtctcaata   4680
aattttactt tgtcg                                                    4695
```

<210> 37

<400> 37
000

<210> 38

<400> 38
000

<210> 39

<400> 39
000

<210> 40

<400> 40
000

<210> 41

<400> 41
000

<210> 42

<400> 42
000

<210> 43

<400> 43
000

<210> 44

<400> 44
000

<210> 45

<400> 45
000

<210> 46

<400> 46
000

<210> 47

<400> 47
000

<210> 48

<400> 48
000

<210> 49

<400> 49
000

<210> 50

<400> 50
000

<210> 51

<400> 51
000

<210> 52

<400> 52
000

<210> 53
<211> 3171
<212> DNA
<213> human

<400> 53

gcggaacacc ggcccgccgt cgcggcagct gcttcacccc tctctctgca gccatggggc 60
tccctcgtgg acctctcgcg tctctcctcc ttctccaggt ttgctggctg cagtgcgcgg 120
cctccgagcc gtgccgggcg gtcttcaggg aggctgaagt gaccttggag gcgggaggcg 180
cggagcagga gcccggccag gcgctgggga aagtattcat gggctgccct gggcaagagc 240
cagctctgtt tagcactgat aatgatgact tcactgtgcg gaatggcgag acagtccagg 300
aaagaaggtc actgaaggaa aggaatccat tgaagatctt cccatccaaa cgtatcttac 360
gaagacacaa gagagattgg gtggttgctc caatatctgt ccctgaaaat ggcaagggtc 420
ccttccccca gagactgaat cagctcaagt ctaataaaga tagagacacc aagattttct 480
acagcatcac ggggccgggg gcagacagcc cccctgaggg tgtcttcgct gtagagaagg 540
agacaggctg gttgttgttg aataagccac tggaccggga ggagattgcc aagtatgagc 600
tctttggcca cgctgtgtca gagaatggtg cctcagtgga ggaccccatg aacatctcca 660
tcatcgtgac cgaccagaat gaccacaagc ccaagtttac ccaggacacc ttccgaggga 720
gtgtcttaga gggagtccta ccaggtactt ctgtgatgca ggtgacagcc acagatgagg 780
atgatgccat ctacacctac aatggggtgg ttgcttactc catccatagc caagaaccaa 840
aggacccaca cgacctcatg ttcacaattc accggagcac aggcaccatc agcgtcatct 900
ccagtggcct ggaccgggaa aaagtccctg agtacacact gaccatccag gccacagaca 960
tggatgggga cggctccacc accacggcag tggcagtagt ggagatcctt gatgccaatg 1020
acaatgctcc catgtttgac ccccagaagt acgaggccca tgtgcctgag aatgcagtgg 1080
gccatgaggt gcagaggctg acggtcactg atctggacgc ccccaactca ccagcgtggc 1140
gtgccaccta ccttatcatg ggcggtgacg acggggacca ttttaccatc accacccacc 1200
ctgagagcaa ccagggcatc ctgacaacca ggaagggttt ggattttgag gccaaaaacc 1260
agcacaccct gtacgttgaa gtgaccaacg aggccccttt tgtgctgaag ctcccaacct 1320
ccacagccac catagtggtc cacgtggagg atgtgaatga ggcacctgtg tttgtcccac 1380
cctccaaagt cgttgaggtc caggagggca tccccactgg ggagcctgtg tgtgtctaca 1440
ctgcagaaga ccctgacaag gagaatcaaa agatcagcta ccgcatcctg agagacccag 1500
cagggtggct agccatggac ccagacagtg ggcaggtcac agctgtgggc accctcgacc 1560

gtgaggatga gcagtttgtg aggaacaaca tctatgaagt catggtcttg gccatggaca 1620

atggaagccc tcccaccact ggcacgggaa cccttctgct aacactgatt gatgtcaacg 1680

accatggccc agtccctgag ccccgtcaga tcaccatctg caaccaaagc cctgtgcgcc 1740

acgtgctgaa catcacggac aaggacctgt ctccccacac ctcccctttc caggcccagc 1800

tcacagatga ctcagacatc tactggacgg cagaggtcaa cgaggaaggt gacacagtgg 1860

tcttgtccct gaagaagttc ctgaagcagg atacatatga cgtgcacctt tctctgtctg 1920

accatggcaa caaagagcag ctgacggtga tcagggccac tgtgtgcgac tgccatggcc 1980

atgtcgaaac ctgccctgga ccctggaaag gaggtttcat cctccctgtg ctgggggctg 2040

tcctggctct gctgttcctc ctgctggtgc tgcttttgtt ggtgagaaag aagcggaaga 2100

tcaaggagcc cctcctactc ccagaagatg acacccgtga caacgtcttc tactatggcg 2160

aagagggggg tggcgaagag gaccaggact atgacatcac ccagctccac cgaggtctgg 2220

aggccaggcc ggaggtggtt ctccgcaatg acgtggcacc aaccatcatc ccgacaccca 2280

tgtaccgtcc taggccagcc aacccagatg aaatcggcaa ctttataatt gagaacctga 2340

aggcggctaa cacagacccc acagccccgc cctacgacac cctcttggtg ttcgactatg 2400

agggcagcgg ctccgacgcc gcgtccctga gctccctcac ctcctccgcc tccgaccaag 2460

accaagatta cgattatctg aacgagtggg gcagccgctt caagaagctg gcagacatgt 2520

acggtggcgg ggaggacgac taggcggcct gcctgcaggg ctggggacca aacgtcaggc 2580

cacagagcat ctccaagggg tctcagttcc cccttcagct gaggacttcg gagcttgtca 2640

ggaagtggcc gtagcaactt ggcggagaca ggctatgagt ctgacgttag agtggttgct 2700

tccttagcct ttcaggatgg aggaatgtgg gcagtttgac ttcagcactg aaaacctctc 2760

cacctgggcc agggttgcct cagaggccaa gtttccagaa gcctcttacc tgccgtaaaa 2820

tgctcaaccc tgtgtcctgg gcctgggcct gctgtgactg acctacagtg gactttctct 2880

ctggaatgga accttcttag gcctcctggt gcaacttaat tttttttttt aatgctatct 2940

tcaaaacgtt agagaaagtt cttcaaaagt gcagcccaga gctgctgggc ccactggccg 3000

tcctgcattt ctggtttcca gaccccaatg cctcccattc ggatggatct ctgcgttttt 3060

atactgagtg tgcctaggtt gccccttatt ttttattttc cctgttgcgt tgctatagat 3120

gaagggtgag gacaatcgtg tatatgtact agaacttttt tattaaagaa a 3171

<210> 54

<400> 54
000

<210> 55

<400> 55
000

<210> 56

<400> 56
000

<210> 57

<400> 57
000

<210> 58

<400> 58
000

<210> 59

<400> 59
000

<210> 60

<400> 60
000

<210> 61

<400> 61
000

<210> 62

<400> 62
000

<210> 63

<400> 63
000

<210> 64

<400> 64
000

<210> 65

<400> 65
000

<210> 66

<400> 66
000

<210> 67

<400> 67
000

<210> 68

<400> 68
000

<210> 69

<400> 69
000

<210> 70

<400> 70
000

<210> 71

<400> 71
000

<210> 72

<400> 72
000

<210> 73

<400> 73
000

<210> 74

<400> 74
000

<210> 75

<400> 75
000

<210> 76
<211> 1152
<212> DNA
<213> human

<400> 76

cgctcctcgg gctgcccctc ggttgacaat ggtctccagg atggtctcta ccatgctatc 60
tggcctactg ttttggctgg catctggatg gactccagca tttgcttaca gcccccggac 120
ccctgaccgg gtctcagaag cagatatcca gaggctgctt catggtgtta tggagcaatt 180
gggcattgcc aggccccgag tggaatatcc agctcaccag gccatgaatc ttgtgggccc 240
ccagagcatt gaaggtggag ctcatgaagg acttcagcat ttgggtcctt ttggcaacat 300
ccccaacatc gtggcagagt tgactggaga caacattcct aaggacttta gtgaggatca 360
ggggtaccca gaccctccaa atccctgtcc tgttggaaaa acagatgatg gatgtctaga 420
aaacacccct gacactgcag agttcagtcg agagttccag ttgcaccagc atctctttga 480
tccggaacat gactatccag gcttgggcaa gtggaacaag aaactccttt acgagaagat 540
gaagggagga gagagacgaa agcggaggag tgtcaatcca tatctacaag gacagagact 600
ggataatgtt gttgcaaaga agtctgtccc ccatttttca gatgaggata aggatccaga 660
gtaaagagaa gatgctagac gaaaacccac attacctgtt aggcctcagc atggcttatg 720
tgcacgtgta aatggagtcc ctgtgaatga cagcatgttt cttacataga taattatgga 780
tacaaagcag ctgtatgtag atagtgtatt gtcttcacac cgatgattct gctttttgct 840
aaattagaat aagagctttt ttgtttcttg ggtttttaaa atgtgaatct gcaatgatca 900
taaaaattaa aatgtgaatg tcaacaataa aaagcaagac tatgaaaggc tcagatttct 960
tgcagtttaa aatggtgtct gaggttgtac tattttggcc aagtctgtag aaagctgtca 1020
tttgattttg attatgtagt tcatccagcc cttgggcatt gttatacacc agtaaagaag 1080
gctgtactca agaggaggag ctgacacatt tcacttggct gcgtcttaat aaacatgaat 1140
gcaagcattg gc 1152

<210> 77

<400> 77
000

<210> 78

<400> 78
000

<210> 79

<400> 79
000

<210> 80

<400> 80
000

<210> 81

<400> 81
000

<210> 82

<400> 82
000

<210> 83

<400> 83
000

<210> 84

<400> 84
000

<210> 85

<400> 85
000

<210> 86

<400> 86
000

<210> 87

<400> 87
000

<210> 88

<400> 88
000

<210> 89

<400> 89
000

<210> 90

<400> 90
000

<210> 91

<400> 91
000

<210> 92

<400> 92

000

<210> 93

<400> 93
000

<210> 94

<400> 94
000

<210> 95

<400> 95
000

<210> 96

<400> 96
000

<210> 97

<400> 97
000

<210> 98

<400> 98
000

<210> 99

<400> 99
000

<210> 100

<400> 100
000

<210> 101

<400> 101
000

<210> 102

<400> 102
000

<210> 103

<400> 103
000

<210> 104

<400> 104
000

<210> 105

<400> 105
000

<210> 106

<400> 106
000

<210> 107

<400> 107
000

<210> 108

<400> 108
000

<210> 109

<400> 109
000

<210> 110

<400> 110
000

<210> 111

<400> 111
000

<210> 112

<400> 112
000

<210> 113

<400> 113
000

<210> 114

<400> 114
000

<210> 115

<400> 115
000

<210> 116

<400> 116
000

<210> 117

<400> 117
000

<210> 118

<400> 118
000

<210> 119

<400> 119
000

<210> 120

<400> 120
000

<210> 121

<400> 121
000

<210> 122

<400> 122
000

<210> 123

<400> 123
000

<210> 124

<400> 124
000

<210> 125

<400> 125
000

<210> 126

<400> 126
000

<210> 127

<400> 127

000

<210> 128

<400> 128
000

<210> 129

<400> 129
000

<210> 130

<400> 130
000

<210> 131

<400> 131
000

<210> 132

<400> 132
000

<210> 133

<400> 133
000

<210> 134

<400> 134
000

<210> 135

<400> 135
000

<210> 136

<400> 136
000

<210> 137

<400> 137
000

<210> 138

<400> 138
000

<210> 139

<400> 139
000

<210> 140

<400> 140
000

<210> 141

<400> 141
000

<210> 142
<211> 493
<212> DNA
<213> human

<400> 142

```
aggacatggg gttccagtac agctgtatta atgattaccg accagtgaag ctcttacagt     60
gcgtggacca cagcacccat cctgactgtg ccttaaagtc ggcagcagcc gctactcaaa    120
gaaaagcccc aagtctttat acagaacaaa gggcgggtct tatcattccc ctctttctgg    180
tgctggcttc ccggactcaa ctgtaactgg aaactgtgtt gctctaaccc tcctccagcc    240
ctgcagcctc cccttgcagt catcattcgt gttctgtgta taccaaatga ttctgttatc    300
taaagaagct ttttgctggg aaaacgatgt cctgaaaatg gtatttcaat gaggcatatg    360
ttcaggattt cagaaacaag aagttagttc tatttagcag gttaaaaaat gctgcattag    420
aattaaagca agttattttc ttatttgtat aatgacacaa agcattggga gtcagactgc    480
ttgtatatta tca                                                       493
```

<210> 143

<400> 143
000

<210> 144

<400> 144
000

<210> 145

<400> 145
000

<210> 146

<400> 146
000

<210> 147

<400> 147
000

<210> 148

<400> 148
000

<210> 149

<400> 149
000

<210> 150

<400> 150
000

<210> 151

<400> 151
000

<210> 152

<400> 152
000

<210> 153

<400> 153
000

<210> 154

<400> 154
000

<210> 155

<400> 155
000

<210> 156

<400> 156
000

<210> 157

<400> 157
000

<210> 158

<400> 158

000

<210> 159

<400> 159
000

<210> 160

<400> 160
000

<210> 161

<400> 161
000

<210> 162

<400> 162
000

<210> 163

<400> 163
000

<210> 164

<400> 164
000

<210> 165

<400> 165
000

<210> 166

<400> 166
000

<210> 167

<400> 167
000

<210> 168

<400> 168
000

<210> 169

<400> 169
000

<210> 170

<400> 170
000

<210> 171

<400> 171
000

<210> 172

<400> 172
000

<210> 173

<400> 173
000

<210> 174

<400> 174
000

<210> 175

<400> 175
000

<210> 176

<400> 176
000

<210> 177

<400> 177
000

<210> 178

<400> 178
000

<210> 179

<400> 179
000

<210> 180

<400> 180
000

<210> 181

<400> 181
000

<210> 182

<400> 182
000

<210> 183

<400> 183
000

<210> 184

<400> 184
000

<210> 185

<400> 185
000

<210> 186

<400> 186
000

<210> 187

<400> 187
000

<210> 188

<400> 188
000

<210> 189

<400> 189
000

<210> 190

<400> 190
000

<210> 191

<400> 191
000

<210> 192

<400> 192
000

<210> 193

<400> 193

000

<210> 194

<400> 194
000

<210> 195

<400> 195
000

<210> 196

<400> 196
000

<210> 197

<400> 197
000

<210> 198

<400> 198
000

<210> 199

<400> 199
000

<210> 200

<400> 200
000

<210> 201

<400> 201
000

<210> 202

<400> 202
000

<210> 203

<400> 203
000

<210> 204

<400> 204
000

<210> 205

<400> 205
000

<210> 206

<400> 206
000

<210> 207

<400> 207
000

<210> 208

<400> 208
000

<210> 209

<400> 209
000

<210> 210

<400> 210
000

<210> 211
<211> 3060
<212> DNA
<213> human

<400> 211

gaggcaattg aggcgcccat ttcagaagag ttacagccgt gaaaattact cagcagtgca 60
gttggctgag aagaggaaaa aagaatgaga gcgctggctg tgctgtctgt cacgctggtt 120
atggcctgca cagaagcctt cttccccttc atctcgagag ggaaagaact cctttgggga 180
aagcctgagg agtctcgtgt ctctagcgtc ttggaggaaa gcaagcgcct ggtggacacc 240
gccatgtacg ccacgatgca gagaaacctc aagaaaagag gaatcctttc tggagctcag 300
cttctgtctt tttccaaact tcctgagcca acaagcggag tgattgcccg agcagcagag 360
ataatggaaa catcaataca agcgatgaaa agaaaagtca acctgaaaac tcaacaatca 420
cagcatccaa cggatgcttt atcagaagat ctgctgagca tcattgcaaa catgtctgga 480
tgtctccctt acatgctgcc cccaaaatgc ccaaacactt gcctggcgaa caaatacagg 540
cccatcacag gagcttgcaa caacagagac caccccagat ggggcgcctc caacacggcc 600
ctggcacgat ggctccctcc agtctatgag gacggcttca gtcagccccg aggctggaac 660
cccggcttct tgtacaacgg gttcccactg cccccggtcc gggaggtgac aagacatgtc 720
attcaagttt caaatgaggt tgtcacagat gatgaccgct attctgacct cctgatggca 780
tggggacaat acatcgacca cgacatcgcg ttcacaccac agagcaccag caaagctgcc 840
ttcgggggag ggtctgactg ccagatgact tgtgagaacc aaaacccatg ttttcccata 900
caactcccgg aggaggcccg gccggccgcg ggcaccgcct gtctgccctt ctaccgctct 960
tcggccgcct gcggcaccgg ggaccaaggc gcgctctttg ggaacctgtc cacggccaac 1020
ccgaggcagc agatgaacgg gttgacctcg ttcctggacg cgtccaccgt gtatggcagc 1080
tccccggccc tagagaggca gctgcggaac tggaccagtg ccgaagggct gctccgcgtc 1140
cacggccgcc tccgggactc cggccgcgcc tacctgccct tcgtgccgcc acgcgcgcct 1200
gcggcctgtg cgcccgagcc cggcaacccc ggagagaccc gcgggccctg cttcctggcc 1260
ggagacggcc gcgccagcga ggtcccctcc ctgacggcac tgcacacgct gtggctgcgc 1320

gagcacaacc gcctggccgc ggcgctcaag gccctcaatg cgcactggag cgcggacgcc 1380
gtgtaccagg aggcgcgcaa ggtcgtgggc gctctgcacc agatcatcac cctgagggat 1440
tacatcccca ggatcctggg acccgaggcc ttccagcagt acgtgggtcc ctatgaaggc 1500
tatgactcca ccgccaaccc cactgtgtcc aacgtgttct ccacagccgc cttccgcttc 1560
ggccatgcca cgatccaccc gctggtgagg aggctggacg ccagcttcca ggagcacccc 1620
gacctgcccg ggctgtggct gcaccaggct ttcttcagcc catggacatt actccgtgga 1680
ggtggtttgg acccactaat acgaggcctt cttgcaagac cagccaaact gcaggtgcag 1740
gatcagctga tgaacgagga gctgacggaa aggctctttg tgctgtccaa ttccagcacc 1800
ttggatctgg cgtccatcaa cctgcagagg ggccgggacc acgggctgcc aggttacaat 1860
gagtggaggg agttctgcgg cctgcctcgc ctggagaccc ccgctgacct gagcacagcc 1920
atcgccagca ggagcgtggc cgacaagatc ctggacttgt acaagcatcc tgacaacatc 1980
gatgtctggc tgggaggctt agctgaaaac ttcctcccca gggctcggac agggcccctg 2040
tttgcctgtc tcattgggaa gcagatgaag gctctgcggg acggtgactg gttttggtgg 2100
gagaacagcc acgtcttcac ggatgcacag aggcgtgagc tggagaagca ctccctgtct 2160
cgggtcatct gtgacaacac tggcctcacc agggtgccca tggatgcctt ccaagtcggc 2220
aaattccccg aagactttga gtcttgtgac agcatcactg gcatgaacct ggaggcctgg 2280
agggaaacct ttcctcaaga cgacaagtgt ggcttcccag agagcgtgga gaatggggac 2340
tttgtgcact gtgaggagtc tgggaggcgc gtgctggtgt attcctgccg gcacgggtat 2400
gagctccaag gccgggagca gctcacttgc acccaggaag gatgggattt ccagcctccc 2460
ctctgcaaag atgtgaacga gtgtgcagac ggtgcccacc ccccctgcca cgcctctgcg 2520
aggtgcagaa acaccaaagg cggcttccag tgtctctgcg cggacccccta cgagttagga 2580
gacgatggga gaacctgcgt agactccggg aggctccctc gggtgacttg gatctccatg 2640
tcgctggctg ctctgctgat cggaggcttc gcaggtctca cctcgacggt gatttgcagg 2700
tggacacgca ctggcactaa atccacactg cccatctcgg agacaggcgg aggaactccc 2760
gagctgagat gcggaaagca ccaggccgta gggacctcac cgcagcgggc cgcagctcag 2820
gactcggagc aggagagtgc tgggatggaa ggccgggata ctcacaggct gccgagagcc 2880
ctctgagggc aaagtggcag gacactgcag aacagcttca tgttcccaaa atcaccgtac 2940
gactcttttc caaacacagg caaatcggaa atcagcagga cgactgtttt cccaacacgg 3000
gtaaatctag taccatgtcg tagttactct caggcatgga tgaataaatg ttatagctgc 3060

<210> 212

<400> 212

000

<210> 213

<400> 213
000

<210> 214

<400> 214
000

<210> 215

<400> 215
000

<210> 216

<400> 216
000

<210> 217

<400> 217
000

<210> 218

<400> 218
000

<210> 219
<211> 503
<212> DNA
<213> human

<400> 219

```
ggcatttgca gtgtgttggt gatccacgaa aggaaaatca cggaagcagg atagaaatcc     60
agctgcagac aagacctcag gtcgatgaat cttgacaagc agttgagctg tttttttcta    120
ctcacctagg acagtcaggc agaagtatgc aaaatgactg gggctgattc ttttctgaat    180
tgtcgcaaac agcaagagga cttgagtcct agcattaaag agttcaacat gtctaggtcc    240
aagaccactg ttgtgtttga aggatgtaaa accctgctgc ataggatgga atatttggag    300
ggaggatcct gaaaaacatg agggatcaaa tagtcctcaa ctttctagga caaagggagc    360
agctatttgc catctaccct ccagaataaa gaaatcttat cattcaccat ctaccctcta    420
gaataaagaa atcttatcat tcgccatcta ccctgtagaa taaagaaatc ttatcattca    480
ccgtctaccc tctagagtaa aca                                            503
```

<210> 220

<400> 220
000

<210> 221

<400> 221
000

<210> 222

<400> 222
000

<210> 223

<400> 223
000

<210> 224

<400> 224
000

<210> 225

<400> 225
000

<210> 226

<400> 226
000

<210> 227

<400> 227
000

<210> 228

<400> 228
000

<210> 229

<400> 229
000

<210> 230

<400> 230
000

<210> 231

<400> 231
000

<210> 232

<400> 232
000

<210> 233

<400> 233
000

<210> 234

<400> 234
000

<210> 235

<400> 235
000

<210> 236

<400> 236
000

<210> 237

<400> 237
000

<210> 238

<400> 238
000

<210> 239

<400> 239
000

<210> 240

<400> 240
000

<210> 241

<400> 241
000

<210> 242
<211> 7680
<212> DNA
<213> human

<400> 242

gaagagcaag aggcaggctc agcaaatggt tcagccccag tccccggtgg ctgtcagtca 60

aagcaagccc ggttgttatg acaatggaaa acactatcag ataaatcaac agtgggagcg 120

gacctaccta ggtaatgtgt tggtttgtac ttgttatgga ggaagccgag gttttaactg 180

cgaaagtaaa cctgaagctg aagagacttg ctttgacaag tacactggga acacttaccg 240

agtgggtgac acttatgagc gtcctaaaga ctccatgatc tgggactgta cctgcatcgg 300

ggctgggcga gggagaataa gctgtaccat cgcaaaccgc tgccatgaag gggtcagtc 360

ctacaagatt ggtgacacct ggaggagacc acatgagact ggtggttaca tgttagagtg 420

tgtgtgtctt ggtaatggaa aaggagaatg gacctgcaag cccatagctg agaagtgttt 480

tgatcatgct gctgggactt cctatgtggt cggagaaacg tgggagaagc cctaccaagg 540

ctggatgatg gtagattgta cttgcctggg agaaggcagc ggacgcatca cttgcacttc 600

tagaaataga tgcaacgatc aggacacaag gacatcctat agaattggag acacctggag 660

caagaaggat aatcgaggaa acctgctcca gtgcatctgc acaggcaacg gccgaggaga 720

gtggaagtgt gagaggcaca cctctgtgca gaccacatcg agcggatctg gccccttcac 780

cgatgttcgt gcagctgttt accaaccgca gcctcacccc cagcctcctc cctatggcca 840

ctgtgtcaca gacagtggtg tggtctactc tgtggggatg cagtggttga agacacaagg 900

aaataagcaa atgctttgca cgtgcctggg caacggagtc agctgccaag agacagctgt 960

aacccagact tacggtggca acttaaatgg agagccatgt gtcttaccat tcacctacaa 1020

tggcaggacg ttctactcct gcaccacgga agggcgacag gacggacatc tttggtgcag 1080

cacaacttcg aattatgagc aggaccagaa atactctttc tgcacagacc acactgtttt 1140

ggttcagact caaggaggaa attccaatgg tgccttgtgc cacttcccct tcctatacaa 1200

caaccacaat tacactgatt gcacttctga gggcagaaga gacaacatga agtggtgtgg 1260

gaccacacag aactatgatg ccgaccagaa gtttgggttc tgccccatgg ctgcccacga 1320

ggaaatctgc acaaccaatg aaggggtcat gtaccgcatt ggagatcagt gggataagca 1380

gcatgacatg ggtcacatga tgaggtgcac gtgtgttggg aatggtcgtg gggaatggac 1440

atgcattgcc tactcgcaac ttcgagatca gtgcattgtt gatgacatca cttacaatgt 1500

gaacgacaca ttccacaagc gtcatgaaga ggggcacatg ctgaactgta catgcttcgg 1560

tcagggtcgg ggcaggtgga agtgtgatcc cgtcgaccaa tgccaggatt cagagactgg 1620

gacgttttat caaattggag attcatggga gaagtatgtg catggtgtca gataccagtg 1680

ctactgctat ggccgtggca ttggggagtg gcattgccaa cctttacaga cctatccaag 1740

ctcaagtggt cctgtcgaag tatttatcac tgagactccg agtcagccca actcccaccc 1800

catccagtgg aatgcaccac agccatctca catttccaag tacattctca ggtggagacc 1860

taaaaattct gtaggccgtt ggaaggaagc taccatacca ggccacttaa actcctacac 1920

catcaaaggc ctgaagcctg gtgtggtata cgagggccag ctcatcagca tccagcagta 1980

cggccaccaa gaagtgactc gctttgactt caccaccacc agcaccagca cacctgtgac 2040

cagcaacacc gtgacaggag agacgactcc cttttctcct cttgtggcca cttctgaatc 2100

tgtgaccgaa atcacagcca gtagctttgt ggtctcctgg gtctcagctt ccgacaccgt 2160

gtcgggattc cgggtggaat atgagctgag tgaggaggga gatgagccac agtacctgga 2220

tcttccaagc acagccactt ctgtgaacat ccctgacctg cttcctggcc gaaaatacat 2280

tgtaaatgtc tatcagatat ctgaggatgg ggagcagagt ttgatcctgt ctacttcaca 2340

aacaacagcg cctgatgccc ctcctgaccc gactgtggac caagttgatg acacctcaat 2400

tgttgttcgc tggagcagac cccaggctcc catcacaggg tacagaatag tctattcgcc 2460

atcagtagaa ggtagcagca cagaactcaa ccttcctgaa actgcaaact ccgtcaccct 2520

cagtgacttg caacctggtg ttcagtataa catcactatc tatgctgtgg aagaaaatca 2580

agaaagtaca cctgttgtca ttcaacaaga aaccactggc accccacgct cagatacagt 2640
gccctctccc agggacctgc agtttgtgga agtgacagac gtgaaggtca ccatcatgtg 2700
gacaccgcct gagagtgcag tgaccggcta ccgtgtggat gtgatccccg tcaacctgcc 2760
tggcgagcac gggcagaggc tgcccatcag caggaacacc tttgcagaag tcaccgggct 2820
gtcccctggg gtcacctatt acttcaaagt ctttgcagtg agccatggga gggagagcaa 2880
gcctctgact gctcaacaga caaccaaact ggatgctccc actaacctcc agtttgtcaa 2940
tgaaactgat tctactgtcc tggtgagatg gactccacct cgggcccaga taacaggata 3000
ccgactgacc gtgggcctta cccgaagagg ccagcccagg cagtacaatg tgggtccctc 3060
tgtctccaag taccccctga ggaatctgca gcctgcatct gagtacaccg tatccctcgt 3120
ggccataaag ggcaaccaag agagccccaa agccactgga gtctttacca cactgcagcc 3180
tgggagctct attccacctt acaacaccga ggtgactgag accaccatcg tgatcacatg 3240
gacgcctgct ccaagaattg gttttaagct gggtgtacga ccaagccagg gaggagaggc 3300
accacgagaa gtgacttcag actcaggaag catcgttgtg tccggcttga ctccaggagt 3360
agaatacgtc tacaccatcc aagtcctgag agatggacag gaaagagatg cgccaattgt 3420
aaacaaagtg gtgacaccat tgtctccacc aacaaacttg catctggagg caaaccctga 3480
cactggagtg ctcacagtct cctgggagag gagcaccacc ccagacatta ctggttatag 3540
aattaccaca acccctacaa acggccagca gggaaattct ttggaagaag tggtccatgc 3600
tgatcagagc tcctgcactt ttgataacct gagtcccggc ctggagtaca atgtcagtgt 3660
ttacactgtc aaggatgaca aggaaagtgt ccctatctct gataccatca tcccagctgt 3720
tcctcctccc actgacctgc gattcaccaa cattggtcca gacaccatgc gtgtcacctg 3780
ggctccaccc ccatccattg atttaaccaa cttcctggtg cgttactcac ctgtgaaaaa 3840
tgaggaagat gttgcagagt tgtcaatttc tccttcagac aatgcagtgg tcttaacaaa 3900
tctcctgcct ggtacagaat atgtagtgag tgtctccagt gtctacgaac aacatgagag 3960
cacacctctt agaggaagac agaaaacagg tcttgattcc ccaactggca ttgacttttc 4020
tgatattact gccaactctt ttactgtgca ctggattgct cctcgagcca ccatcactgg 4080
ctacaggatc cgccatcatc ccgagcactt cagtgggaga cctcgagaag atcgggtgcc 4140
ccactctcgg aattccatca ccctcaccaa cctcactcca ggcacagagt atgtggtcag 4200
catcgttgct cttaatggca gagaggaaag tcccttattg attggccaac aatcaacagt 4260
ttctgatgtt ccgagggacc tggaagttgt tgctgcgacc cccaccagcc tactgatcag 4320
ctgggatgct cctgctgtca cagtgagata ttacaggatc acttacggag aaacaggagg 4380
aaatagccct gtccaggagt tcactgtgcc tgggagcaag tctacagcta ccatcagcgg 4440

ccttaaacct ggagttgatt ataccatcac tgtgtatgct gtcactggcc gtggagacag 4500
ccccgcaagc agcaagccaa tttccattaa ttaccgaaca gaaattgaca aaccatccca 4560
gatgcaagtg accgatgttc aggacaacag cattagtgtc aagtggctgc cttcaagttc 4620
ccctgttact ggttacagag taaccaccac tcccaaaaat ggaccaggac caacaaaaac 4680
taaaactgca ggtccagatc aaacagaaat gactattgaa ggcttgcagc ccacagtgga 4740
gtatgtggtt agtgtctatg ctcagaatcc aagcggagag agtcagcctc tggttcagac 4800
tgcagtaacc aacattgatc gccctaaagg actggcattc actgatgtgg atgtcgattc 4860
catcaaaatt gcttgggaaa gcccacaggg gcaagtttcc aggtacaggg tgacctactc 4920
gagccctgag gatggaatcc atgagctatt ccctgcacct gatggtgaag aagacactgc 4980
agagctgcaa ggcctcagac cgggttctga gtacacagtc agtgtggttg ccttgcacga 5040
tgatatggag agccagcccc tgattggaac ccagtccaca gctattcctg caccaactga 5100
cctgaagttc actcaggtca cacccacaag cctgagcgcc cagtggacac cacccaatgt 5160
tcagctcact ggatatcgag tgcgggtgac ccccaaggag aagaccggac caatgaaaga 5220
aatcaacctt gctcctgaca gctcatccgt ggttgtatca ggacttatgg tggccaccaa 5280
atatgaagtg agtgtctatg ctcttaagga cactttgaca agcagaccag ctcagggtgt 5340
tgtcaccact ctggagaatg tcagcccacc aagaagggct cgtgtgacag atgctactga 5400
gaccaccatc accattagct ggagaaccaa gactgagacg atcactggct tccaagttga 5460
tgccgttcca gccaatggcc agactccaat ccagagaacc atcaagccag atgtcagaag 5520
ctacaccatc acaggtttac aaccaggcac tgactacaag atctacctgt acaccttgaa 5580
tgacaatgct cggagctccc ctgtggtcat cgacgcctcc actgccattg atgcaccatc 5640
caacctgcgt ttcctggcca ccacacccaa ttccttgctg gtatcatggc agccgccacg 5700
tgccaggatt accggctaca tcatcaagta tgagaagcct gggtctcctc ccagagaagt 5760
ggtccctcgg ccccgccctg gtgtcacaga ggctactatt actggcctgg aaccgggaac 5820
cgaatataca atttatgtca ttgccctgaa gaataatcag aagagcgagc cctgattgg 5880
aaggaaaaag acagacgagc ttccccaact ggtaaccctt ccacacccca atcttcatgg 5940
accagagatc ttggatgttc cttccacagt tcaaaagacc cctttcgtca cccaccctgg 6000
gtatgacact ggaaatggta ttcagcttcc tggcacttct ggtcagcaac ccagtgttgg 6060
gcaacaaatg atctttgagg aacatggttt taggcggacc acaccgccca caacggccac 6120
ccccataagg cataggccaa gaccataccc gccgaatgta ggacaagaag ctctctctca 6180
gacaaccatc tcatgggccc cattccagga cacttctgag tacatcattt catgtcatcc 6240
tgttggcact gatgaagaac ccttacagtt caggttcct ggaacttcta ccagtgccac 6300
tctgacaggc ctcaccagag gtgccaccta caacatcata gtggaggcac tgaaagacca 6360

gcagaggcat aaggttcggg aagaggttgt taccgtgggc aactctgtca acgaaggctt 6420

gaaccaacct acggatgact cgtgctttga cccctacaca gtttcccatt atgccgttgg 6480

agatgagtgg gaacgaatgt ctgaatcagg ctttaaactg ttgtgccagt gcttaggctt 6540

tggaagtggt catttcagat gtgattcatc tagatggtgc catgacaatg gtgtgaacta 6600

caagattgga gagaagtggg accgtcaggg agaaaatggc cagatgatga gctgcacatg 6660

tcttgggaac ggaaaaggag aattcaagtg tgaccctcat gaggcaacgt gttacgatga 6720

tgggaagaca taccacgtag gagaacagtg gcagaaggaa tatctcggtg ccatttgctc 6780

ctgcacatgc tttggaggcc agcggggctg gcgctgtgac aactgccgca gacctggggg 6840

tgaacccagt cccgaaggca ctactggcca gtcctacaac cagtattctc agagatacca 6900

tcagagaaca aacactaatg ttaattgccc aattgagtgc ttcatgcctt tagatgtaca 6960

ggctgacaga gaagattccc gagagtaaat catctttcca atccagagga acaagcatgt 7020

ctctctgcca agatccatct aaactggagt gatgttagca gacccagctt agagttcttc 7080

tttctttctt aagccctttg ctctggagga agttctccag cttcagctca actcacagct 7140

tctccaagca tcaccctggg agtttcctga gggttttctc ataaatgagg gctgcacatt 7200

gcctgttctg cttcgaagta ttcaataccg ctcagtattt taaatgaagt gattctaaga 7260

tttggtttgg gatcaatagg aaagcatatg cagccaacca agatgcaaat gttttgaaat 7320

gatatgacca aaattttaag taggaaagtc acccaaacac ttctgctttc acttaagtgt 7380

ctggcccgca atactgtagg aacaagcatg atcttgttac tgtgatattt taaatatcca 7440

cagtactcac tttttccaaa tgatcctagt aattgcctag aaatatcttt ctcttacctg 7500

ttatttatca atttttccca gtatttttat acggaaaaaa ttgtattgaa aacacttagt 7560

atgcagttga taagaggaat ttggtataat tatggtgggt gattattttt tatactgtat 7620

gtgccaaagc tttactactg tggaaagaca actgttttaa taaaagattt acattccaca 7680

<210> 243

<400> 243
000

<210> 244

<400> 244
000

<210> 245

<400> 245
000

<210> 246

<400> 246
000

<210> 247

<400> 247
000

<210> 248

<400> 248
000

<210> 249

<400> 249
000

<210> 250

<400> 250
000

<210> 251

<400> 251
000

<210> 252

<400> 252
000

<210> 253

<400> 253
000

<210> 254

<400> 254
000

<210> 255

<400> 255
000

<210> 256

<400> 256
000

<210> 257

<400> 257
000

<210> 258

<400> 258
000

<210> 259

<400> 259
000

<210> 260

<400> 260
000

<210> 261

<400> 261
000

<210> 262

<400> 262
000

<210> 263

<400> 263
000

<210> 264

<400> 264
000

<210> 265

<400> 265
000

<210> 266

<400> 266
000

<210> 267

<400> 267
000

<210> 268

<400> 268
000

<210> 269

<400> 269

000

<210> 270

<400> 270
000

<210> 271

<400> 271
000

<210> 272

<400> 272
000

<210> 273

<400> 273
000

<210> 274

<400> 274
000

<210> 275

<400> 275
000

<210> 276

<400> 276
000

<210> 277

<400> 277
000

<210> 278

<400> 278
000

<210> 279

<400> 279
000

<210> 280

<400> 280
000

<210> 281

<400> 281
000

<210> 282

<400> 282
000

<210> 283

<400> 283
000

<210> 284

<400> 284
000

<210> 285

<400> 285
000

<210> 286

<400> 286
000

<210> 287

<400> 287
000

<210> 288

<400> 288
000

<210> 289

<400> 289
000

<210> 290

<400> 290
000

<210> 291

<400> 291
000

<210> 292

<400> 292
000

<210> 293

<400> 293
000

<210> 294

<400> 294
000

<210> 295

<400> 295
000

<210> 296

<400> 296
000

<210> 297

<400> 297
000

<210> 298

<400> 298
000

<210> 299

<400> 299
000

<210> 300

<400> 300
000

<210> 301

<400> 301
000

<210> 302

<400> 302
000

<210> 303

<400> 303
000

<210> 304

<400> 304

000

<210> 305

<400> 305
000

<210> 306

<400> 306
000

<210> 307

<400> 307
000

<210> 308

<400> 308
000

<210> 309
<211> 534
<212> DNA
<213> human

<400> 309

```
ataagcttat ctcagctgac tcctcggagg gccaagacca gcttcaagtc tccatggtac     60
cagaaaacaa caacctcaca gcacctgaac ctcaagaaga ggtatccaca agtgaaaatc    120
cacaactctg aagagaaact accaagactc ctcctgcccc aaacctcgcc agagaagctc    180
ttcaaccaga gggtataggt cagagggata taagagccag catccatccc tgggttctca    240
gtaggaatgc tggtgctgtc taaagacctg gcattaatgg aggcggagga gcagccttac    300
gggagggatg gagggaggca ggctggggag aagagaacat tagactcagg gaatatttaa    360
ttctggtttt agcattatta gaataagact ttatacatta actaaagtgg agctttaatc    420
actataaaaa gcaaaagtat ctatagacac agacacttgc ctatacagag acataaccac    480
acacactcag aggatagtga acaaatctgt ctttgactta cgacccattt tgca          534
```

<210> 310

<400> 310
000

<210> 311

<400> 311
000

<210> 312

<400> 312
000

<210> 313

<400> 313
000

<210> 314

<400> 314
000

<210> 315

<400> 315
000

<210> 316

<400> 316
000

<210> 317

<400> 317
000

<210> 318

<400> 318
000

<210> 319

<400> 319
000

<210> 320

<400> 320
000

<210> 321

<400> 321
000

<210> 322

<400> 322
000

<210> 323

<400> 323
000

<210> 324

<400> 324
000

<210> 325

<400> 325
000

<210> 326

<400> 326
000

<210> 327

<400> 327
000

<210> 328

<400> 328
000

<210> 329

<400> 329
000

<210> 330

<400> 330
000

<210> 331

<400> 331
000

<210> 332

<400> 332
000

<210> 333

<400> 333
000

<210> 334

<400> 334
000

<210> 335

<400> 335

000

<210> 336

<400> 336
000

<210> 337

<400> 337
000

<210> 338

<400> 338
000

<210> 339

<400> 339
000

<210> 340

<400> 340
000

<210> 341

<400> 341
000

<210> 342

<400> 342
000

<210> 343

<400> 343
000

<210> 344

<400> 344
000

<210> 345

<400> 345
000

<210> 346

<400> 346
000

<210> 347

<400> 347
000

<210> 348

<400> 348
000

<210> 349

<400> 349
000

<210> 350

<400> 350
000

<210> 351

<400> 351
000

<210> 352

<400> 352
000

<210> 353

<400> 353
000

<210> 354

<400> 354
000

<210> 355

<400> 355
000

<210> 356

<400> 356
000

<210> 357

<400> 357
000

<210> 358

<400> 358
000

<210> 359

<400> 359
000

<210> 360

<400> 360
000

<210> 361

<400> 361
000

<210> 362

<400> 362
000

<210> 363

<400> 363
000

<210> 364

<400> 364
000

<210> 365

<400> 365
000

<210> 366

<400> 366
000

<210> 367

<400> 367
000

<210> 368

<400> 368
000

<210> 369

<400> 369
000

<210> 370

<400> 370

000

<210> 371

<400> 371
000

<210> 372

<400> 372
000

<210> 373

<400> 373
000

<210> 374

<400> 374
000

<210> 375

<400> 375
000

<210> 376

<400> 376
000

<210> 377

<400> 377
000

<210> 378

<400> 378
000

<210> 379

<400> 379
000

<210> 380

<400> 380
000

<210> 381

<400> 381
000

<210> 382

<400> 382
000

<210> 383

<400> 383
000

<210> 384

<400> 384
000

<210> 385

<400> 385
000

<210> 386

<400> 386
000

<210> 387

<400> 387
000

<210> 388

<400> 388
000

<210> 389

<400> 389
000

<210> 390

<400> 390
000

<210> 391

<400> 391
000

<210> 392

<400> 392
000

<210> 393

<400> 393
000

<210> 394

<400> 394
000

<210> 395

<400> 395
000

<210> 396

<400> 396
000

<210> 397

<400> 397
000

<210> 398

<400> 398
000

<210> 399

<400> 399
000

<210> 400

<400> 400
000

<210> 401

<400> 401
000

<210> 402

<400> 402
000

<210> 403

<400> 403
000

<210> 404

<400> 404
000

<210> 405

<400> 405

000

<210> 406

<400> 406
000

<210> 407

<400> 407
000

<210> 408

<400> 408
000

<210> 409
<211> 23
<212> DNA
<213> human

<400> 409
actccagctt gctgagttcc cca 23

<210> 410
<211> 23
<212> DNA
<213> human

<400> 410
actccagctt gctgagttcc cca 23

<210> 411
<211> 32
<212> DNA
<213> human

<400> 411
attattacag ttccacatca aggccgagtg ca 32

<210> 412

<400> 412
000

<210> 413

<400> 413
000

<210> 414

<400> 414
000

<210> 415

<400> 415
000

<210> 416

<400> 416
000

<210> 417

<400> 417
000

<210> 418

<400> 418
000

<210> 419

<400> 419
000

<210> 420

<400> 420
000

<210> 421

<400> 421
000

<210> 422

<400> 422
000

<210> 423

<400> 423
000

<210> 424

<400> 424
000

<210> 425

<400> 425
000

<210> 426

<400> 426
000

<210> 427

<400> 427
000

<210> 428

<400> 428
000

<210> 429

<400> 429
000